(19) Europäisches Patentamt
European Patent Office
Office européen des brevets

(11) EP 4 644 390 A1

(12) # EUROPEAN PATENT APPLICATION
published in accordance with Art. 153(4) EPC

(43) Date of publication:
**05.11.2025 Bulletin 2025/45**

(21) Application number: **23910768.3**

(22) Date of filing: **27.12.2023**

(51) International Patent Classification (IPC):
***C07D 417/12*** *(2006.01)* ***A61K 31/425*** *(2006.01)*
***A61P 35/00*** *(2006.01)*

(52) Cooperative Patent Classification (CPC):
**A61K 31/425; A61P 35/00; C07D 417/12**

(86) International application number:
**PCT/CN2023/142499**

(87) International publication number:
**WO 2024/140850 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 CN 202211695647**

(71) Applicant: **Beyang Therapeutics Co., Ltd.**
**Suzhou, Jiangsu 215127 (CN)**

(72) Inventors:
- **HU, Qiyue**
  **Suzhou, Jiangsu 215127 (CN)**
- **HU, Weimin**
  **Suzhou, Jiangsu 215127 (CN)**
- **LIU, Suxing**
  **Suzhou, Jiangsu 215127 (CN)**

(74) Representative: **HGF**
**HGF Limited**
**1 City Walk**
**Leeds LS11 9DX (GB)**

(54) **PROTEIN TYROSINE KINASE INHIBITOR AND MEDICAL USE THEREOF**

(57) The present invention relates to a protein tyrosine kinase inhibitor and uses thereof in the prevention and/or treatment of protein tyrosine kinase-mediated hyperproliferative diseases, for example ocular diseases and malignant tumors accompanied by pathologic neovascularization. The protein tyrosine kinase inhibitor of the invention effectively antagonizes VEGFR receptor tyrosine kinase activity, and also exhibits improved pharmacokinetic properties as well as high selectivity for inhibiting an "offtarget" EGFR tyrosine kinase activity, thereby reducing or avoiding adverse effects, particularly ocular side effects.

EP 4 644 390 A1

## Description

### FIELD OF THE INTVENTION

The present invention relates to the field of pharmaceutical technology, and specifically to a protein tyrosine kinase inhibitor and pharmaceutical use thereof, particularly in the prevention and/or treatment of protein tyrosine kinase-mediated hyperproliferative diseases.

### BACKGROUND OF THE INVENTION

Receptor tyrosine kinases (RTKs) play crucial roles in developmental biology, tissue homeostasis, and cancer biology. RTKs consist of an extracellular ligand-binding domain, a transmembrane domain, and an intracellular catalytic domain. Dimerization of two RTKs following ligand binding induces autophosphorylation of tyrosine residues within the intracellular catalytic domains, resulting in an active conformation and subsequent activation of intracellular signal transduction cascades. The tyrosine kinases are highly regulated due to their significant effects on cells. When these kinases are constitutively activated through mutations or overexpression and are no longer ligand-independent, many diseases (such as cancer or ophthalmic diseases like diabetic retinopathy) may develop via unregulated cell proliferation and other mechanisms. For this reason, tyrosine kinase inhibitors (TKIs) can provide pharmacological intervention for certain diseases by disrupting these dysregulated processes. The development of inhibitors (RTKi) targeting pro-angiogenic receptor tyrosine kinases (primarily the vascular endothelial growth factor receptor (VEGFR) family) has significantly improved outcomes in certain cancers, such as renal cell carcinoma, hepatocellular carcinoma, and colorectal cancer, and has become an effective therapy for tumor-associated angiogenesis.

Diabetic retinopathy (DR) and age-related macular degeneration (AMD) are leading causes of blindness across the world. These pathological changes are associated with neovascularization in the posterior eye segment. In particular, DR is preferentially characterized by changes in neovascularization at the retinal level, while wet AMD is marked by neovascularization from the choroidal microvascular beds and invasion into the subretinal space. Both DR and AMD are characterized by the proliferation and migration of endothelial cells (ECs), increased vascular permeability, and inflammation. In these processes, vascular endothelial growth factors-A (VEGFs-A) and its corresponding receptors (VEGFRs) play critical roles. Many proliferative disorders, such as ocular diseases, tumors, and cancers, involve the overexpression or upregulation of RTK activity. Receptor tyrosine kinases are a class of kinase enzymes that modify proteins by chemically adding phosphate groups (phosphorylation). Phosphorylation typically leads to functional changes in target proteins by altering enzyme activity, cellular localization, or binding to other proteins. It is known that kinases have been used to regulate most cellular pathways, particularly those involved in signal transduction. To date, one of the approaches to inhibit the VEGF pathway is to inhibit the RTK activity. In the case of treating ocular diseases such as DR and AMD, the protein tyrosine kinase inhibitor therapy is aimed at canceling pathological neovascularization and disease progression to prevent vision impairment. Meanwhile, as a pro-angiogenic inducer, VEGFR is crucial in tumor growth, invasion, and extravasation, which makes it an excellent therapeutic target for various cancers. However, existing VEGFR receptor tyrosine kinase inhibitors (RTKis) often inhibit EGFR activity. Such a cross-reactivity can lead to adverse side effects by inhibiting biological functions associated with one or more of these off-target receptors. This greatly affects the application of VEGFR RTKis to the treatment of ophthalmic diseases. To date, the U.S. FDA has not yet approved small molecule tyrosine kinase inhibitors for the treatment of DR and neovascular AMD.

### SUMMARY OF THE INVENTION

The present invention provides a compound for treating protein tyrosine kinase-mediated hyperproliferative disorders or symptoms, for example ocular diseases and malignant tumors accompanied by pathologic neovascularization, by selectively inhibiting VEGFR receptor tyrosine kinase activity while exhibiting selectivity in inhibiting EGFR receptor tyrosine kinase, so as to reduce side effects, particularly adverse ocular side effects.

To overcome the defects in the prior art, the present invention provides a protein tyrosine kinase inhibitor and pharmaceutical use thereof.

In a first aspect of the present invention, a compound is provided. The compound has a structure of:

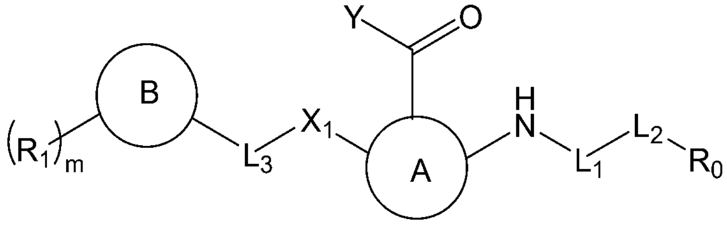

(I)

wherein,

Ring A is a 5-7 membered heteroaromatic ring;

Ring B is a $C_6$-$C_{10}$ aromatic ring or a 5-10 membered heterocyclic ring; optionally, said $C_6$-$C_{10}$ aromatic ring or 5-10 membered heterocyclic ring can be fused with a $C_6$-$C_{10}$ aromatic ring, a $C_5$-$C_8$ aliphatic ring, or a 5-10 membered heterocyclic ring;

$X_1$ is O or S;

Y is -N($C_0$-$C_{10}$ alkyl)($C_0$-$C_{10}$ alkyl) or -O($C_0$-$C_{10}$ alkyl);

$L_1$ is selected from: a single bond, -C(O)-, -C(O)O-, -C(O)$NR_3$-, -C(O)N($R_3$)O-, -$S(O)_2$-, - $S(O)_2NR_3$-, -S(O)-, -S(O)$NR_3$-, -Cy-; -Cy- is selected from: substituted or unsubstituted cycloalkylene, substituted or unsubstituted arylene, and substituted or unsubstituted heterocyclylene;

$L_2$ is a single bond or alkylene, wherein one or more methylene units in alkylene are optionally and independently substituted by a group selected from:

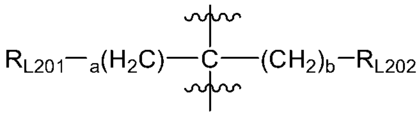

,

-N($R_4$)-, -N($R_4$)C(O)-, -C(O)N($R_4$)-, -N($R_4$)$S(O)_2$-, - $S(O)_2$N($R_4$)-, -O-, -C(O)-, -OC(O)-, -C(O)O-, -S-, -S(O)- or -$S(O)_2$; wherein, a and b are independently integers from 0 to 10, and $R_{L201}$ and $R_{L202}$ are independently selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, -$OR_{L203}$, -C(O)$R_{L203}$, -C(O)$OR_{L203}$, -$NR_{L204}$C(O)$OR_{L203}$, - OC(O)$R_{L203}$, -$NR_{L204}SO_2R_{L203}$, -$SO_2NR_{L203}R_{L204}$, -$NR_{L204}$C(O)$R_{L203}$, -C(O)$NR_{L203}R_{L204}$, - $NR_{L203}R_{L204}$, -$SR_{L203}$, -S(O)$R_{L203}$, -$S(O)_2R_{L203}$, -$SO_3$H, $C_3$-$C_6$ cycloalkyl, cycloalkyl alkyl, heterocyclyl, heterocyclylalkyl; wherein, $R_{L203}$ and $R_{L204}$ are independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl; $L_3$ is alkylene, wherein one or more methylene units in alkylene are optionally and independently substituted by a group selected from:

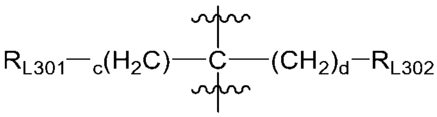

, -

N($R_5$)-, -N($R_5$)C(O)-, -C(O)N($R_5$)-, -N($R_5$)$S(O)_2$-, -$S(O)_2$N($R_5$)-, -O-, -C(O)-, -OC(O)-, - C(O)O-, -S-, -S(O)- or -$S(O)_2$; wherein, c and d are independently integers from 0 to 10, and $R_{L301}$ and $R_{L302}$ are independently selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, -$OR_{L303}$, -C(O)$R_{L303}$, -C(O)$OR_{L303}$, -$NR_{L304}$C(O)$OR_{L303}$, -OC(O)$R_{L303}$, -$NR_{L304}SO_2R_{L303}$, -$SO_2NR_{L303}R_{L304}$, - $NR_{L304}$C(O)$R_{L303}$, -C(O)$NR_{L303}R_{L304}$, -$NR_{L303}R_{L304}$, -$SR_{L303}$, -S(O)$R_{L303}$, -$S(O)_2R_{L303}$, -$SO_3$H, $C_3$-$C_6$ cycloalkyl, cycloalkyl alkyl, heterocyclyl, heterocyclylalkyl; wherein, $R_{L303}$ and $R_{L304}$ are independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl;

$R_3$ to $R_5$ are each independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl, wherein said alkyl, cycloalkyl, or heterocyclyl is optionally substituted by a group selected from: halogen, cyano, nitro, azido, -OR', -C(O)R', -C(O)OR', -OC(O)R', -NR'C(O)OR", - NR'$SO_2$R", -$SO_2$NR'R", -NR'C(O)R", -C(O)NR'R", -NR'R", -SR', -SOR', -$SO_2$R', -$SO_3$H, $C_1$-$C_{10}$ alkyl, $C_1$-$C_{10}$ haloalkyl,

$C_1$-$C_{10}$ silyl, $C_3$-$C_{10}$ cycloalkyl, phenyl, 4-10 membered heterocyclyl;

$R_1$ is one or more independent substituents on Ring B, each $R_1$ is independently selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, $-OR_{101}$, $-C(O)R_{101}$, $-C(O)OR_{101}$, $-NR_{102}C(O)OR_{101}$, $-OC(O)R_{101}$, $-NR_{102}SO_2R_{101}$, $-SO_2NR_{101}R_{102}$, $-NR_{102}C(O)R_{101}$, $-C(O)NR_{101}R_{102}$, $-NR_{101}R_{102}$, $-S(O)_jR_{101}$, where j is an integer from 0 to 2, $-SO_3H$, $-NR_{102}(CR_{103}R_{104})_tOR_{101}$, $-(CH_2)_t(C_6$-$C_{10}$ aryl), $-SO_2(CH_2)_t(C_6$-$C_{10}$ aryl), $-S(CH_2)_t(C_6$-$C_{10}$ aryl), $-O(CH_2)_t(C_6$-$C_{10}$ aryl), $-(CH_2)_t$(4-10 membered heterocyclyl), $-SO_2(CH_2)_t$(4-10 membered heterocyclyl), $-S(CH_2)_t$(4-10 membered heterocyclyl), $-O(CH_2)_t$(4-10 membered heterocyclyl), $-(CH_2)_t(C_3$-$C_{10}$ cycloalkyl), $-SO_2(CH_2)_t(C_3$-$C_{10}$ cycloalkyl), $-S(CH_2)_t(C_3$-$C_{10}$ cycloalkyl), $-O(CH_2)_t(C_3$-$C_{10}$ cycloalkyl), where t is an integer from 0 to 5; wherein, said $C_1$-$C_{10}$ alkyl, $C_6$-$C_{10}$ aryl, or 4-10 membered heterocyclyl is optionally substituted by a group selected from: halogen, cyano, nitro, azido, -OR', -C(O)R', -C(O)OR', -OC(O)R', -NR'C(O)OR", -NR'SO$_2$R", -SO$_2$NR'R", -NR'C(O)R", -C(O)NR'R", -NR'R", -SR', -SOR', -SO$_2$R', $-SO_3H$, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl;

$R_{101}$ to $R_{104}$ are each independently selected from: H, substituted or unsubstituted alkyl, substituted or unsubstituted cycloalkyl, substituted or unsubstituted cycloalkyl alkyl, substituted or unsubstituted heterocyclyl, substituted or unsubstituted heterocyclylalkyl, and $C_1$-$C_{10}$ silyl; wherein said alkyl, cycloalkyl, or heterocyclyl is optionally substituted by a group selected from: halogen, cyano, nitro, azido, -OR', -C(O)R', -C(O)OR', -OC(O)R', - NR'C(O)OR", -NR'SO$_2$R", -SO$_2$NR'R", -NR'C(O)R", -C(O)NR'R", -NR'R", -SR', -SOR', - SO$_2$R', $-SO_3H$, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl;

m is an integer from 1 to 5 (for example, 1, 2, 3, 4, or 5, where valency permits);

$R_0$ is selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, $-OR_{001}$, $-C(O)R_{001}$, $-C(O)OR_{001}$, $-NR_{002}C(O)OR_{001}$, $-OC(O)R_{001}$, $-NR_{02}SO_2R_{001}$, $-SO_2NR_{001}R_{002}$, $-NR_{002}C(O)R_{001}$, $-C(O)NR_{001}R_{002}$, $-NR_{001}R_{002}$, $-S(O)_iR_{002}$ (where i is an integer from 0 to 2), $-SO_3H$, $-NR_{002}(CR_{003}R_{004})_tOR_{001}$, and

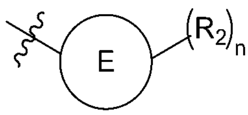

;

wherein, Ring E is a $C_6$-$C_{10}$ aromatic ring or a 4-10 membered heterocyclic ring; optionally, said $C_6$-$C_{10}$ aromatic ring or 4-10 membered heterocyclic ring can be fused with a $C_6$-$C_{10}$ aromatic ring, a $C_5$-$C_8$ aliphatic ring, or a 4-10 membered heterocyclic ring;

$R_2$ is selected from: H, =O, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, $-OR_{201}$, $-C(O)R_{201}$, $-C(O)OR_{201}$, $-NR_{202}C(O)OR_{201}$, $-OC(O)R_{201}$, $-NR_{202}SO_2R_{201}$, $-SO_2NR_{201}R_{202}$, $-NR_{202}C(O)R_{201}$, $-C(O)NR_{201}R_{202}$, $-NR_{201}R_{202}$, $-S(O)_iR_{201}$, where i is an integer from 0 to 2, $-SO_3H$, $-(CH_2)_j(C_6$-$C_{10}$ aryl), $-SO_2(CH_2)_j(C_6$-$C_{10}$ aryl), $-S(CH_2)_j(C_6$-$C_{10}$ aryl), $-O(CH_2)_j(C_6$-$C_{10}$ aryl), $-(CH_2)_j$(4-10 membered heterocyclyl), $-SO_2(CH_2)_j$(4-10 membered heterocyclyl), $-S(CH_2)_j$(4-10 membered heterocyclyl), $-O(CH_2)_j$(4-10 membered heterocyclyl), $-(CH_2)_j(C_3$-$C_{10}$ cycloalkyl), $-SO_2(CH_2)_j(C_3$-$C_{10}$ cycloalkyl), $-S(CH_2)_j(C_3$-$C_{10}$ cycloalkyl), and $-O(CH_2)_j(C_3$-$C_{10}$ cycloalkyl), where j is an integer from 0 to 5; wherein, said $C_1$-$C_{10}$ alkyl, $C_6$-$C_{10}$ aryl, or 4-10 membered heterocyclyl is optionally substituted by a group selected from: halogen, cyano, nitro, azido, -OR', -C(O)R', -C(O)OR', -OC(O)R', -NR'C(O)OR", -NR'SO$_2$R", -SO$_2$NR'R", - NR'C(O)R", -C(O)NR'R", -NR'R", -SR', -SOR', -SO$_2$R', $-SO_3H$, $C_3$-$C_{10}$ cycloalkyl, phenyl, and 4-10 membered heterocyclyl;

n is an integer from 1 to 5 (for example, 1, 2, 3, 4, or 5, where valency permits);

$R_{001}$ to $R_{004}$ are each independently selected from: H, substituted or unsubstituted alkyl, substituted or unsubstituted cycloalkyl, substituted or unsubstituted cycloalkyl alkyl, substituted or unsubstituted heterocyclyl, substituted or unsubstituted heterocyclylalkyl, and $C_1$-$C_{10}$ silyl, wherein said alkyl, cycloalkyl, or heterocyclyl is optionally substituted by a group selected from: halogen, cyano, nitro, azido, -OR', -C(O)R', -C(O)OR', -OC(O)R', - NR'C(O)OR", -NR'SO$_2$R", -SO$_2$NR'R", -NR'C(O)R", -C(O)NR'R", -NR'R", -SR', -SOR', - SO$_2$R', $-SO_3H$, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl; and

$R_{201}$ to $R_{204}$ are each independently selected from: H, substituted or unsubstituted alkyl, substituted or unsubstituted cycloalkyl, substituted or unsubstituted cycloalkyl alkyl, substituted or unsubstituted heterocyclyl, substituted or unsubstituted heterocyclylalkyl, and $C_1$-$C_{10}$ silyl, wherein said alkyl, cycloalkyl, or heterocyclyl is optionally substituted by a group selected from: halogen, cyano, nitro, azido, -OR', -C(O)R', -C(O)OR', -OC(O)R', - NR'C(O)OR", -NR'SO$_2$R", -SO$_2$NR'R", -NR'C(O)R", -C(O)NR'R", -NR'R", -SR', -SOR', - SO$_2$R', $-SO_3H$, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl; and

R' and R" are independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl.

In some embodiments of the present invention, Y is -N($C_0$-$C_{10}$ alkyl)($C_0$-$C_{10}$ alkyl), such as -$NH_2$ and $C_1$-$C_3$ alkylamino.

In some embodiments of the present invention, Y is -O($C_0$-$C_{10}$ alkyl), such as -OH and $C_1$-$C_3$ alkoxy.

Specifically, Ring A is a five-membered or six-membered aromatic ring or heteroaromatic ring, for example, Ring A is

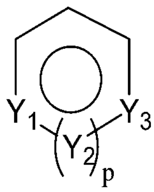,

wherein, $Y_1$, $Y_2$, $Y_3$ is independently selected from: O, S, N, and C($R^A$), $R^A$ is selected from: H, halogen, substituted or unsubstituted alkyl, hydroxyl, alkoxy, amino, alkylamino, cyano, and nitro; p is 0 or 1; and in some embodiments of the present invention, Ring A is

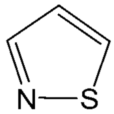, 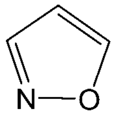, 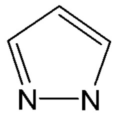, 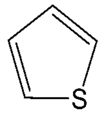, 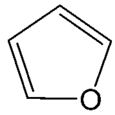,

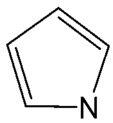, 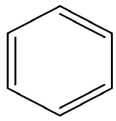, 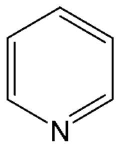, 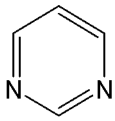, or 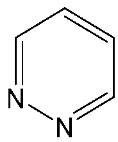,

particularly

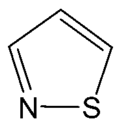.

In an embodiment of the present invention, the

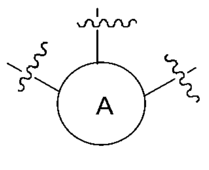

moiety has a structure of:

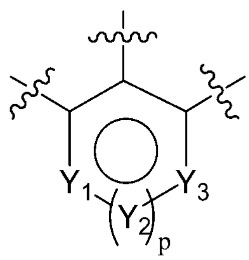.

Specifically, $R^A$ is selected from: H, halogen, $C_1$-$C_3$ alkyl, hydroxyl, $C_1$-$C_3$alkoxy, amino, $C_1$-$C_3$ alkylamino, cyano, and nitro; and in some embodiments of the present invention, $R^A$ is H.

In some embodiments of the present invention, the

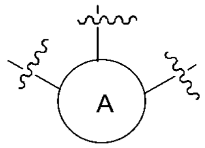

moiety has a structure of:

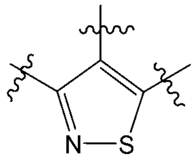, 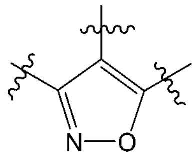, 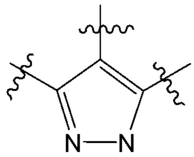, 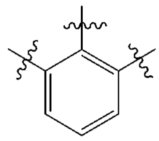, 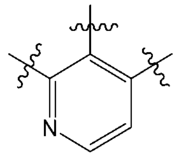, 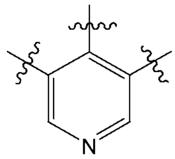,

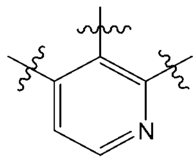, 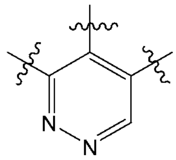, or 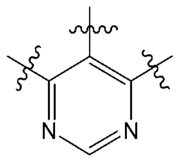,

particularly

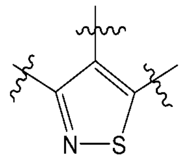.

Specifically, Ring B is a benzene ring, or a 5-6 membered monocyclic heterocyclic ring, optionally, the benzene ring or the 5-6 membered monocyclic heterocyclic ring may be fused with a benzene ring, a $C_5$-$C_8$ aliphatic ring, or a 5-6 membered monocyclic heterocyclic ring. In some embodiments of the present invention, Ring B is a benzene ring, a benzo-fused aliphatic ring, or a heterocyclic ring (including monocyclic heterocyclic rings (particularly 5-6 membered monocyclic heterocyclic rings), bicyclic heterocyclic rings (particularly 9-11 membered bicyclic fused heterocyclic rings)). In some embodiments of the present invention, the 5-6 membered monocyclic heterocyclic ring has a structure of:

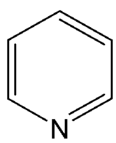, 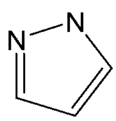,

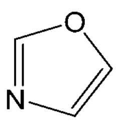, or 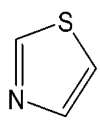.

In some embodiments of the present invention, the benzo-fused aliphatic ring has a structure of:

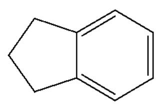, or 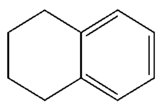.

In some embodiments of the present invention, the bicyclic heterocyclic ring has a structure of:

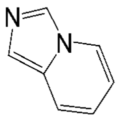, 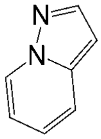,

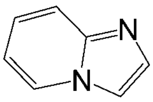, 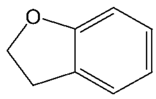, 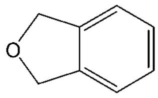, 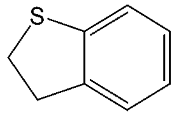, 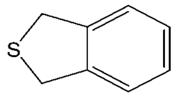, 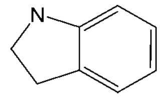,

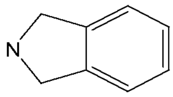, 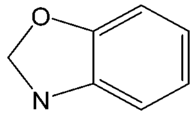, 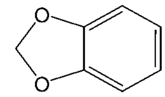, 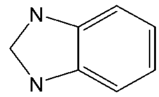, 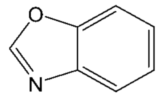, 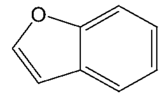,

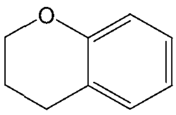, 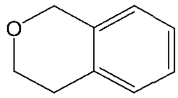, or 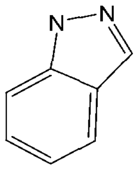.

Specifically, the

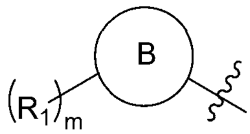

moiety may have a structure of:

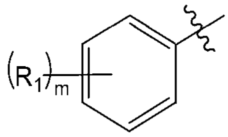,

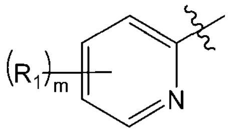, 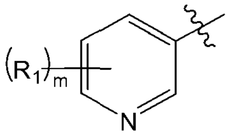, 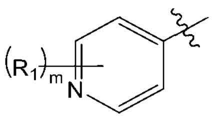, 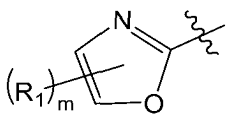, 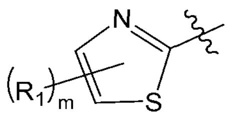,

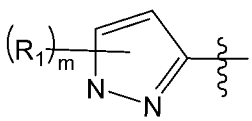, 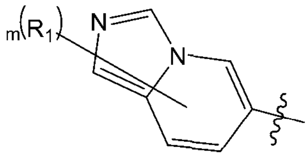, 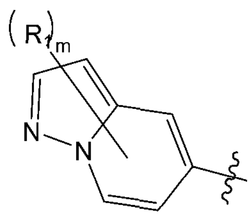, 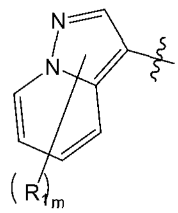,

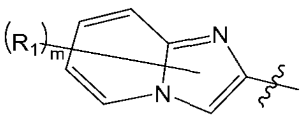, 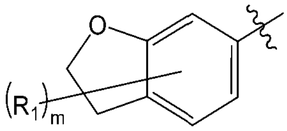, 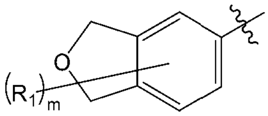, 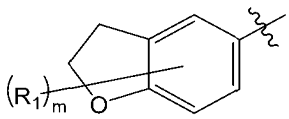,

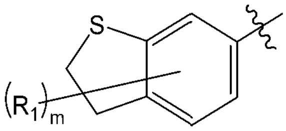, 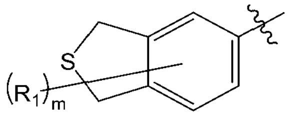, 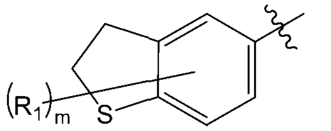,

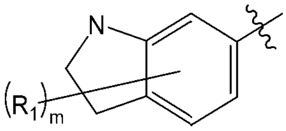, 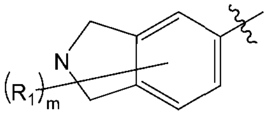, 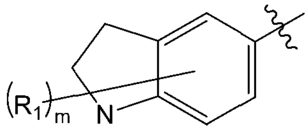, 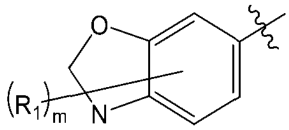,

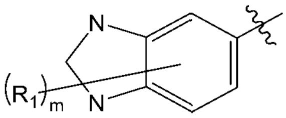, 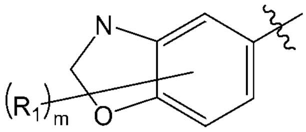, 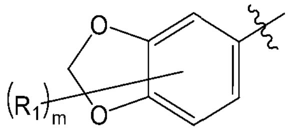, 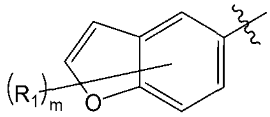,

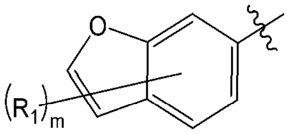, 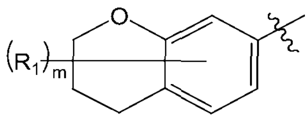, 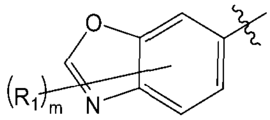, 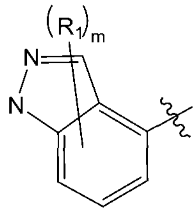,

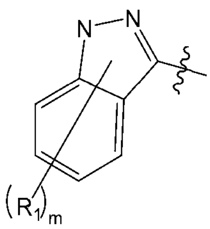, 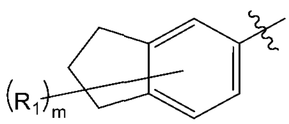, or 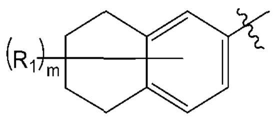.

In some embodiments of the present invention, Ring B is a benzene ring, for example, the

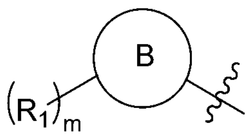

moiety is

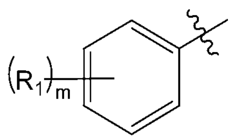.

In other embodiments of the present invention, Ring B is a monocyclic heterocyclic ring, particularly a 5-6 membered monocyclic heterocyclic ring, for example, the

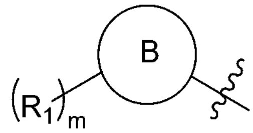

moiety may be

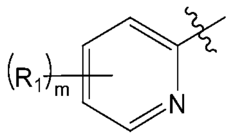 , 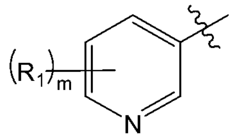 , 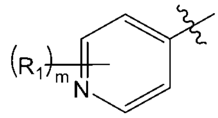 ,

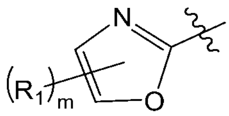 , 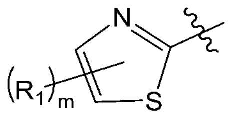 , or 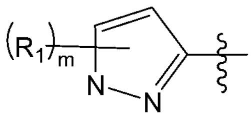 .

In some embodiments of the present invention, the

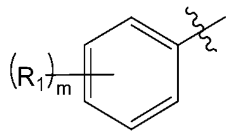

moiety has a structure of:

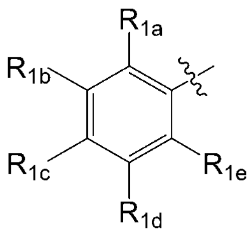 ,

wherein, $R_{1b}$ is selected from: $C_1$-$C_6$ haloalkyl, cyano, nitro, azido, - $OR_{101}$, $-C(O)R_{101}$, $-C(O)OR_{101}$, $-NHC(O)OR_{101}$, $-OC(O)R_{101}$, $-NHSO_2R_{101}$, $-SO_2NR_{101}R_{102}$, - $NHC(O)R_{101}$, $-C(O)NR_{101}R_{102}$, $-NR_{101}R_{102}$, $-SR_{101}$, $-S(O)_2R_{101}$, $-SO_3H$, $-(CH_2)_t$(phenyl), - $(CH_2)_t$(4-10 membered heterocyclyl), and $-(CH_2)_t(C_3$-$C_{10}$ cycloalkyl); more specifically, $R_{1b}$ is selected from: $C_1$-$C_6$ haloalkyl, cyano, nitro, azido, $-OR_{101}$, $-C(O)R_{101}$, $-NHC(O)R_{101}$, - $C(O)NHR_{101}$, $-NHR_{101}$, $-SR_{101}$, $-(CH_2)_t$(4-8 membered heterocyclyl), and $-(CH_2)_t(C_3$-$C_6$ cycloalkyl), where t is an integer from 0 to 5;

$R_{1c}$ is selected from: H, F, $C_1$-$C_6$ alkyl, cyano, nitro, azido, $-OR_{101}$, $-C(O)R_{101}$, $-C(O)OR_{101}$, $-NHC(O)OR_{101}$, $-OC(O)R_{101}$, $-NHSO_2R_{101}$, $-SO_2NR_{101}R_{102}$, $-NHC(O)R_{101}$, $-C(O)NR_{101}R_{102}$, $-NR_{101}R_{102}$, $-SR_{101}$, $-S(O)_2R_{101}$, $-SO_3H$, $-(CH_2)_t$(phenyl), $-(CH_2)_t$(4-10 membered heterocyclyl), and $-(CH_2)_t(C_3$-$C_{10}$ cycloalkyl);

$R_{1a}$, $R_{1d}$, and $R_{1e}$ are selected from: H, $C_1$-$C_6$ alkyl (for example, methyl, ethyl, n-propyl, or isopropyl), halogen (for example, F, Cl, Br, or I), $C_1$-$C_6$ haloalkyl, cyano, nitro, azido, $-OR_{101}$, $-C(O)R_{101}$, $-C(O)OR_{101}$, $-NHC(O)OR_{101}$, $-OC(O)R_{101}$, $-NHSO_2R_{101}$, $-SO_2NR_{101}R_{102}$, - $NHC(O)R_{101}$, $-C(O)NR_{101}R_{102}$, $-NR_{101}R_{102}$, $-SR_{101}$, $-S(O)_2R_{101}$, $-SO_3H$, $-(CH_2)_t$(phenyl), - $(CH_2)_t$(4-10 membered heterocyclyl), and $-(CH_2)_t(C_3$-$C_{10}$ cycloalkyl); and

$R_{101}$ and $R_{102}$ are each defined as above.

Further, $R_{1b}$ may be selected from: $-CF_3$, $-CHF_2$, $-CH_2F$,

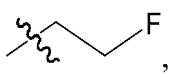 , 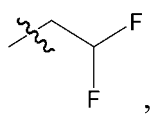 , 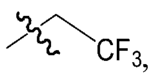

cyano, nitro, azido, -OH,

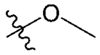, 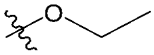, 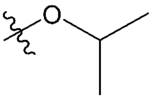, 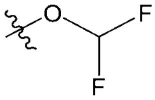, 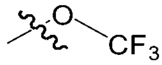,

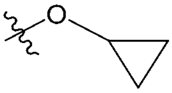, 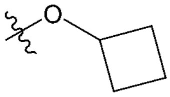, 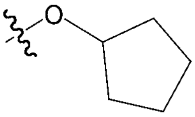, 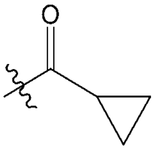, 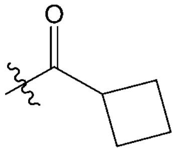, 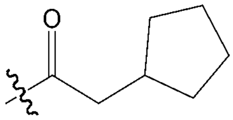,

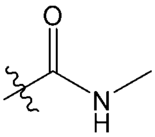, 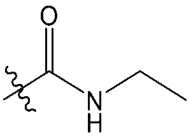, 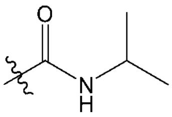, 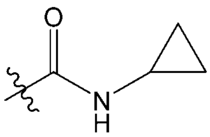, 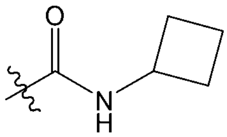,

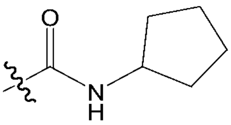, 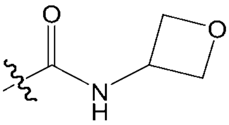, 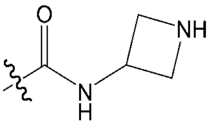, 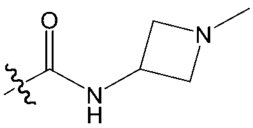, 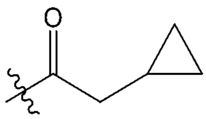,

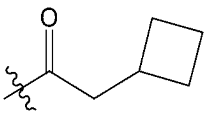, 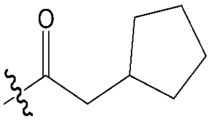, 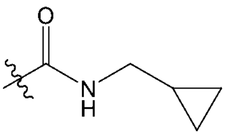, 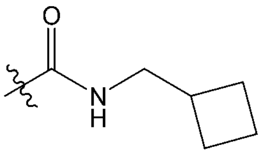,

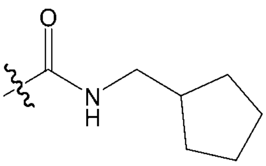, 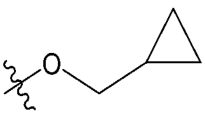, 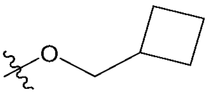, 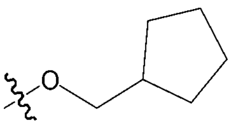, 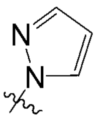, 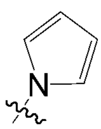,

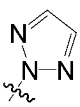, 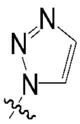, 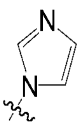, 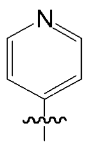, 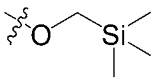, 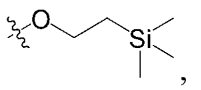, 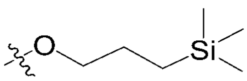, 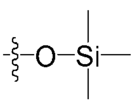,

and

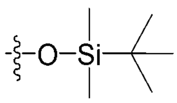.

In some preferred embodiments of the present invention, $R_{1b}$ is -C(O)$NR_{101}R_{102}$, wherein, $R_{101}$ and $R_{102}$ are independently selected from: H, $C_1$-$C_6$ alkyl, $C_3$-$C_6$ cycloalkyl, and $C_4$-$C_{10}$ cycloalkyl alkyl.

Further, $R_{1a}$ and $R_{1e}$ may be independently selected from: H, halogen (for example, F, or Cl), and $C_1$-$C_3$ alkyl (for example, methyl).

Preferably, $R_{1c}$ may be selected from: H, and F.

Further, $R_{1d}$ may be selected from: H, halogen (for example, F or Cl), and $C_1$-$C_3$ alkyl (for example, methyl).

In some preferred embodiments of the present invention, the

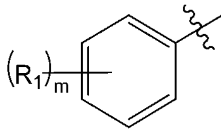

moiety has a structure of:

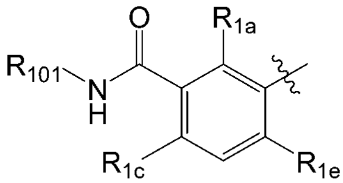

In other embodiments of the present invention, Ring B is a bicyclic fused heterocyclic ring, particularly a 9-11 membered bicyclic fused heterocyclic ring, for example,

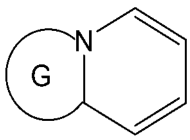

,

wherein, the G ring is a 5-6 membered heterocyclic ring, for example, the

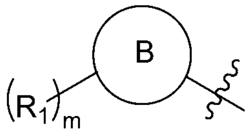

moiety may be

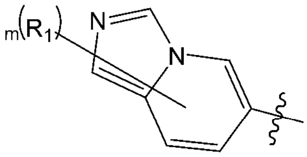

,

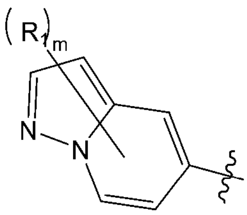

,

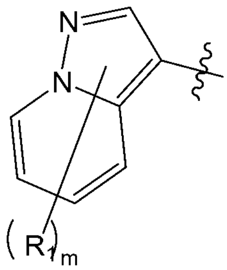

,

or

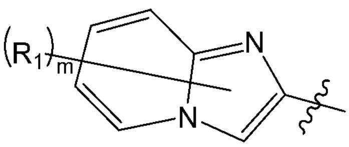

.

In other embodiments of the present invention, Ring B is a fused bicyclic ring, particularly a 9-11 membered fused bicyclic ring, for example,

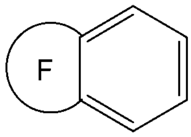,

wherein, Ring F is a 5-6 membered carboatomic ring or a 5-6 membered heterocyclic ring, for example the

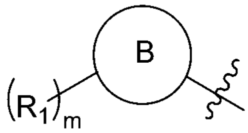

moiety may be

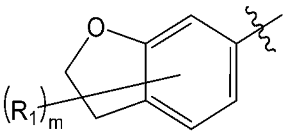, 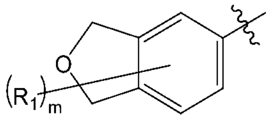,

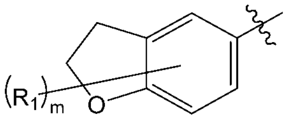, 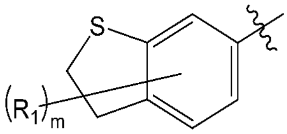, 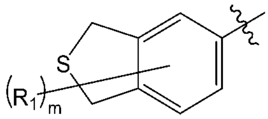, 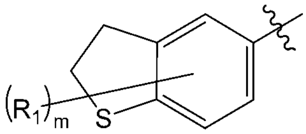,

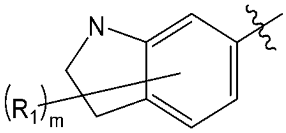, 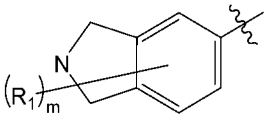, 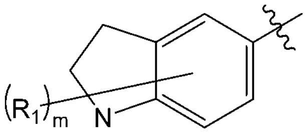, 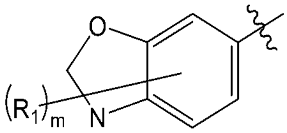,

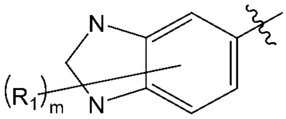, 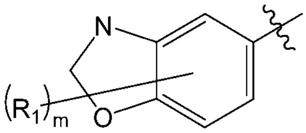, 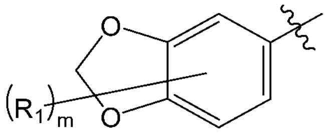,

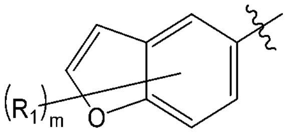, 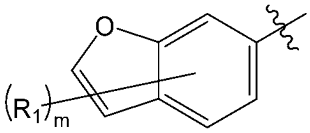, 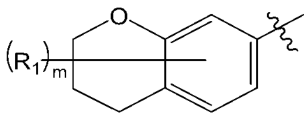,

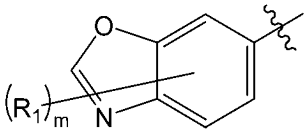, 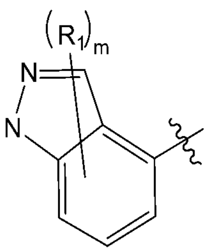, 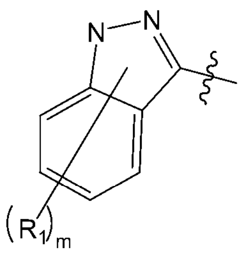, 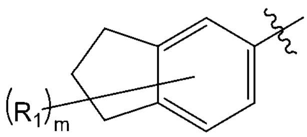,

or

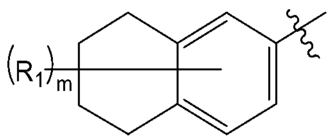.

Specifically, $R_{101}$ to $R_{104}$ may be each independently selected from: H, $C_1$-$C_6$ alkyl (for example, $-CH_3$,

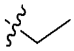, 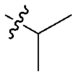, 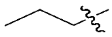, 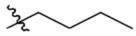, or 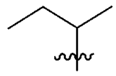

), $C_1$-$C_6$ haloalkyl (for example, $-CHF_2$, $-CH_2F$, $-CF_3$, $-CH_2$-$CH_2F$, $-CH_2$-$CHF_2$, $-CH_2$-$CF_3$, $-CH_2CH_2$-$CF_3$, and $-CH_2CH_2CH_2$-$CF_3$), $C_1$-$C_6$ hydroxyl-substituted alkyl (for example,

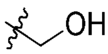, 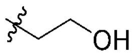,

or

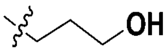),

$C_1$-$C_6$ alkoxy-substituted alkyl (for example,

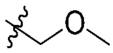, 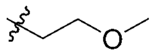,

or

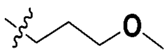),

$C_1$-$C_6$ amino-substituted alkyl (for example,

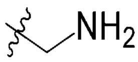, 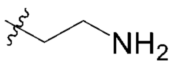,

or

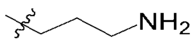),

$C_1$-$C_6$ alkylamino-substituted alkyl (for example,

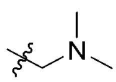, 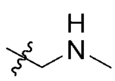,

or

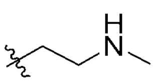),

$C_3$-$C_{10}$ cycloalkyl (for example,

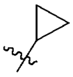, 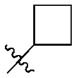, 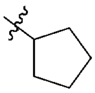, 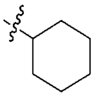, 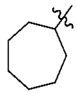,

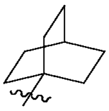, 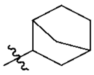, 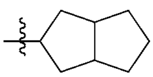, 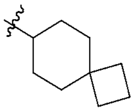, 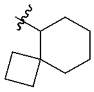, or 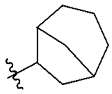),

$C_4$-$C_{10}$ cycloalkyl alkyl (for example,

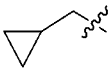, 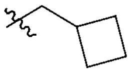, 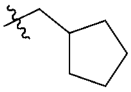, 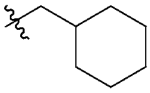, or 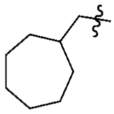

), substituted or unsubstituted 4-10 membered heterocyclyl (for example,

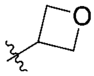, 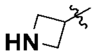, 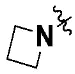, 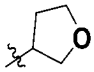,

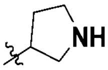, 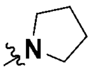, 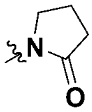, 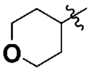, 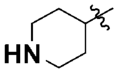, 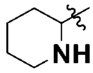, 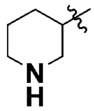, 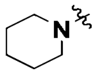, 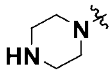,

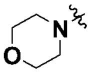, 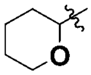, 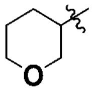, 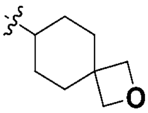, 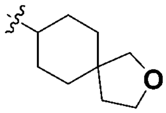, 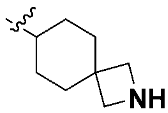, 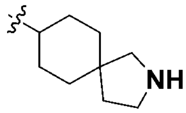,

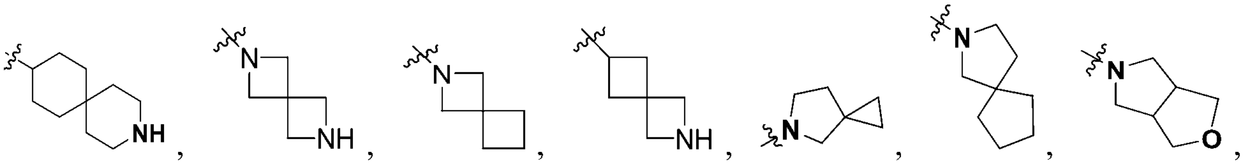

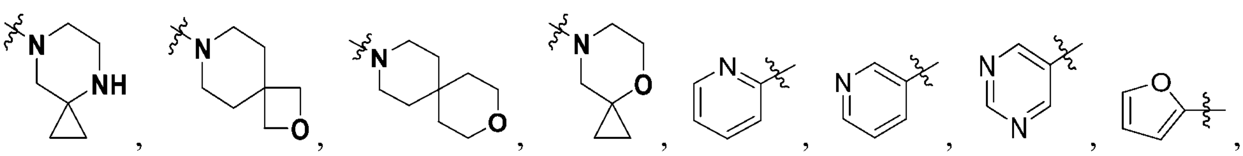

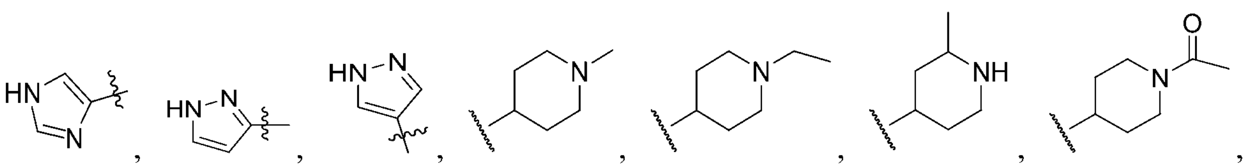

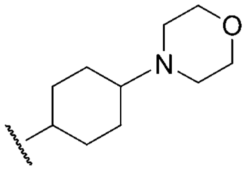, 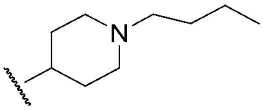, 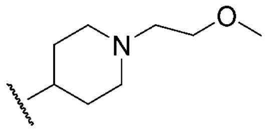, 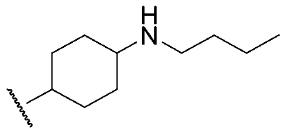,

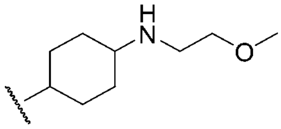, 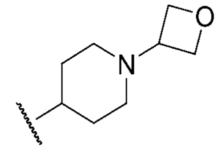, 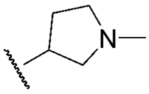, 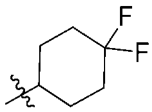, 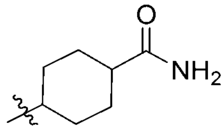,

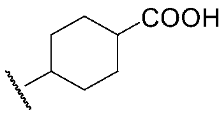, 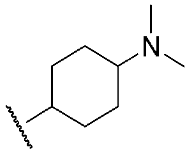, 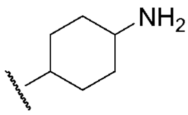, 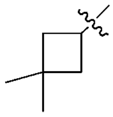, 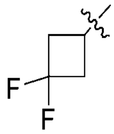, 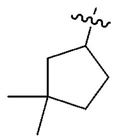, 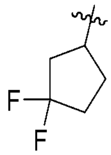,

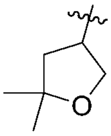, or 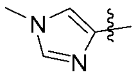), 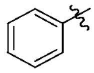, 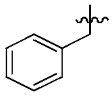,

$C_1$-$C_{10}$ silyl-substituted alkyl (for example,

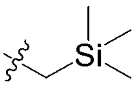, 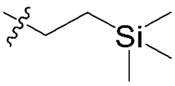, or 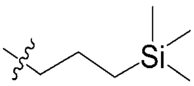

), and $C_1$-$C_{10}$ silyl (for example,

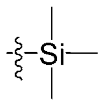,

or

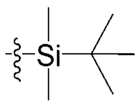

).

More specifically, $R_{101}$ to $R_{104}$ are each independently selected from: H, methyl, ethyl, n-propyl, isopropyl, $-CF_3$, $-CHF_2$, $-CH_2F$,

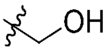, 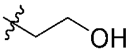, 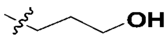, 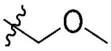,

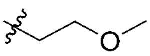, 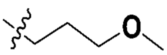, 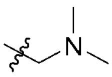, 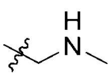, 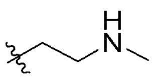, 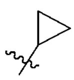, 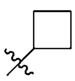, 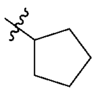,

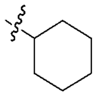, 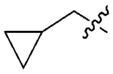, 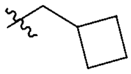, 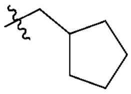, 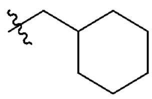, 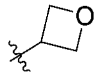, 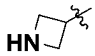, 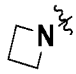, 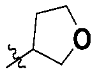,

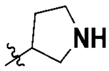, 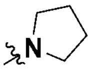, 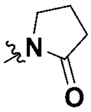, 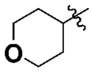, 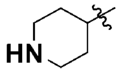, 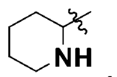, 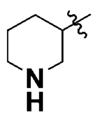, 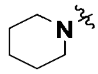, 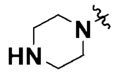,

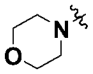, 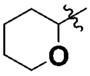, 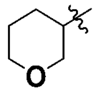 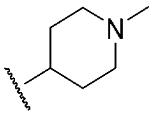, 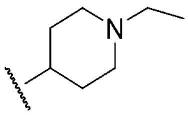, 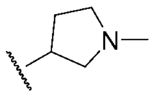, 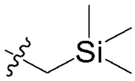,

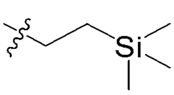, 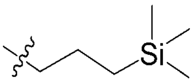, 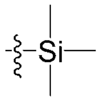, and 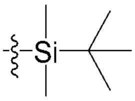.

Specifically, each $R_1$ may be independently selected from: $C_1$-$C_6$ alkyl (e.g., methyl, ethyl, n-propyl, and isopropyl), halogen (e.g., F, Cl, Br, and I), $C_1$-$C_6$ haloalkyl (e.g., $-CHF_2$, $-CH_2F$, $-CF_3$, $-CH_2$-$CH_2F$, $-CH_2$-$CHF_2$, $-CH_2$-$CF_3$, $-CH_2CH_2$-$CF_3$, and $-CH_2CH_2CH_2$-$CF_3$), cyano, nitro, azido, $-OR_{101}$, $-C(O)R_{101}$, $-C(O)OR_{101}$, $-NHC(O)OR_{101}$, $-OC(O)R_{101}$, $-NHSO_2R_{101}$, $-SO_2NR_{101}R_{102}$, $-NHC(O)R_{101}$, $-C(O)NR_{101}R_{102}$, $-NR_{191}R_{102}$, $-SR_{101}$, $-S(O)_2R_{101}$, $-SO_3H$, $-(CH_2)_t$(phenyl), $-(CH_2)_t$(4-10 membered heterocyclyl), and $-(CH_2)_t$($C_3$-$C_{10}$ cycloalkyl), where t is an integer from 0 to 5; specifically, the 4-10 membered heterocyclic ring is a 4-6 membered heterocyclic ring, for example,

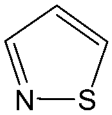, 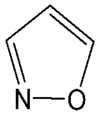, 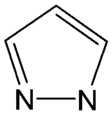, 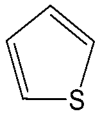, 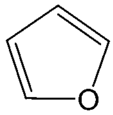,

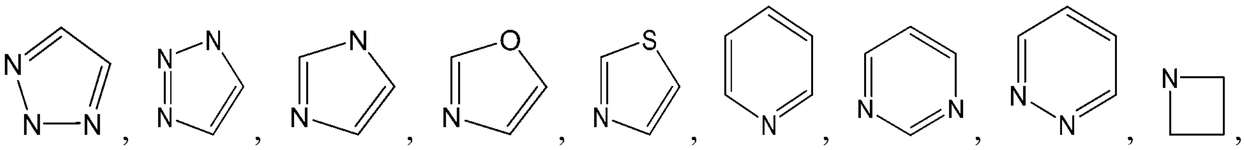

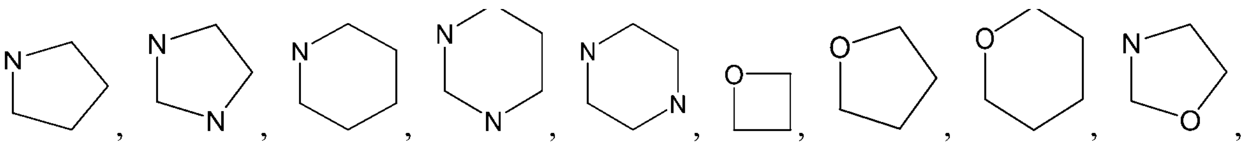

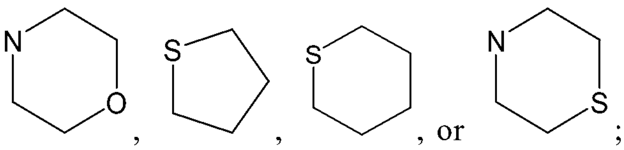

and specifically, the $C_3$-$C_8$ cycloalkyl is $C_3$-$C_6$ cycloalkyl, for example,

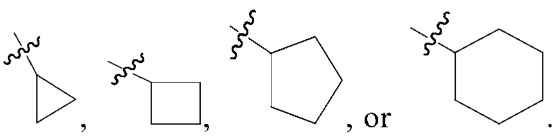

In some embodiments of the present invention, $R_{102}$ is H.

More specifically, each $R_1$ may be independently selected from: $C_1$-$C_6$ alkyl, halogen, $C_1$-$C_6$ haloalkyl, cyano, nitro, azido, -$OR_{101}$, -C(O)$R_{101}$, -NHC(O)$R_{101}$, -C(O)NH$R_{101}$, -NH$R_{101}$, - S$R_{101}$, -$(CH_2)_t$(4-8 membered heterocyclyl), and -$(CH_2)_t$($C_3$-$C_6$ cycloalkyl), where t is an integer from 0 to 5, and $R_{101}$ may be selected from: $C_1$-$C_6$ alkyl (e.g., methyl, ethyl, n-propyl, and isopropyl), $C_1$-$C_6$ haloalkyl (e.g., -$CHF_2$, -$CH_2F$, -$CF_3$, -$CH_2$-$CH_2F$, -$CH_2$-$CHF_2$, -$CH_2$-$CF_3$, -$CH_2CH_2$-$CF_3$, and -$CH_2CH_2CH_2$-$CF_3$), $C_3$-$C_6$ cycloalkyl (e.g.,

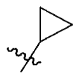, 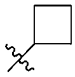, 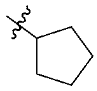,

and

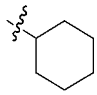

), and $C_4$-$C_{10}$ cycloalkyl alkyl (

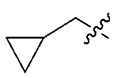, 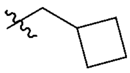, 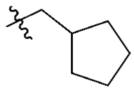, 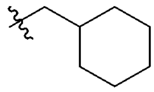,

and

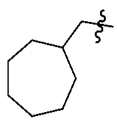

).

In some embodiments of the present invention, each $R_1$ is independently selected from: H, methyl, ethyl, n-propyl, isopropyl, -$CF_3$, -$CHF_2$, -$CH_2F$,

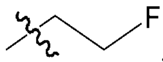, 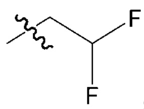, 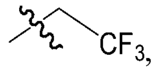

F, Cl, Br, I, cyano, nitro, azido, -OH,

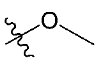, 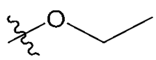, 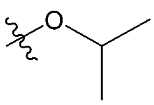, 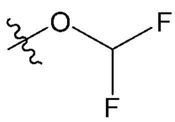,

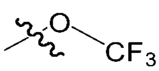, 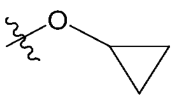, 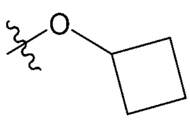, 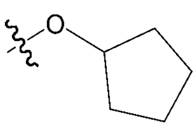, 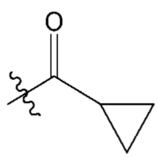, 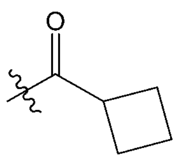,

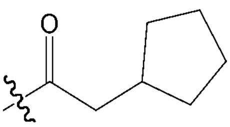, 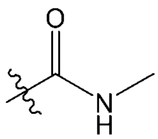, 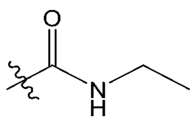, 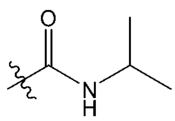, 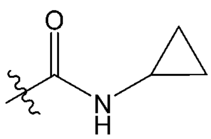,

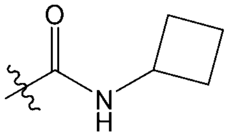, 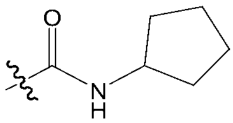, 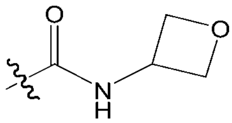, 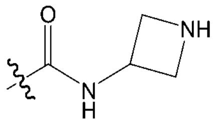,

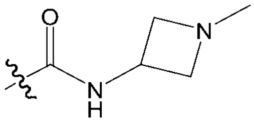, 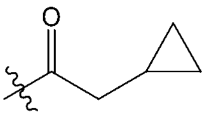, 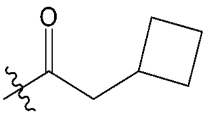, 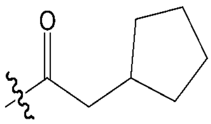, 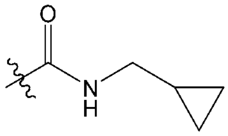,

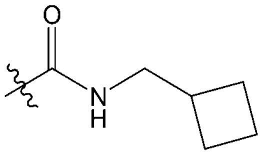, 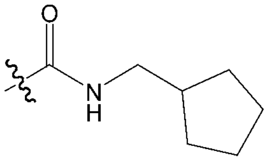, 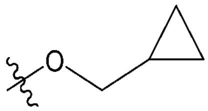, 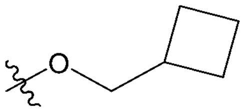,

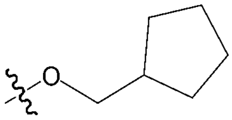, 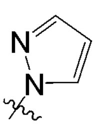, 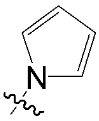, 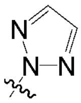, 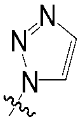, 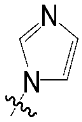, 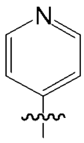, 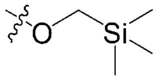,

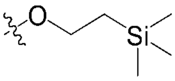, 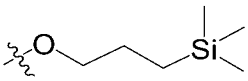, 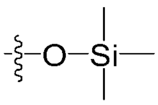, and 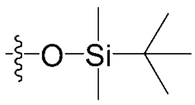.

In an embodiment of the present invention, $R_0$ is

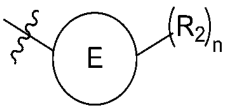.

In some embodiments of the present invention, Ring E is a 4-10 membered (e.g., 4, 5, 6, 7, 8, 9, or 10 membered ) heterocyclic ring, particularly a 4-8 membered saturated heterocyclic ring (including monocyclic rings, and polycyclic rings, for example fused, spiro or bridged polycyclic rings), for example,

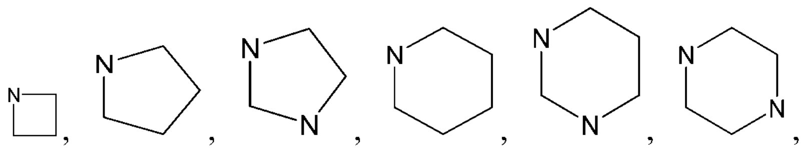

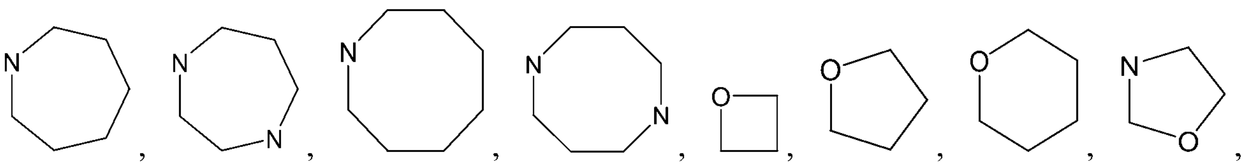

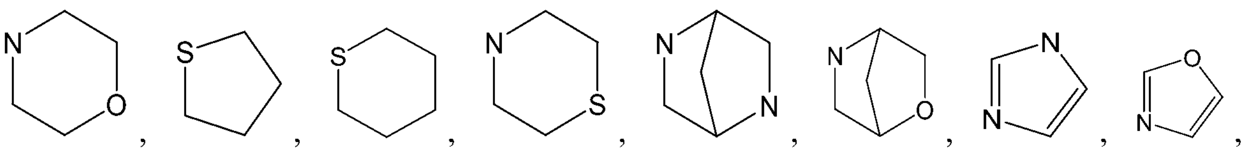

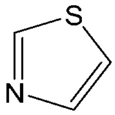, 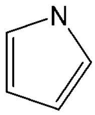, 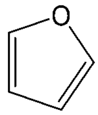, or 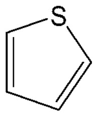;

particularly a 5-7 membered nitrogenous heterocyclic ring, for example,

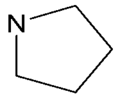, 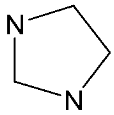, 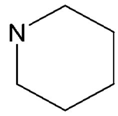, 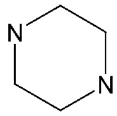, 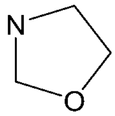, 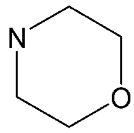,

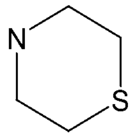, 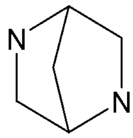, or 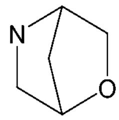.

In some embodiments of the present invention, the

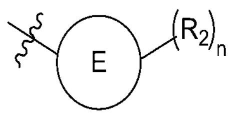

moiety is

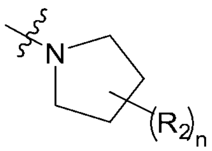, 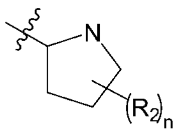, 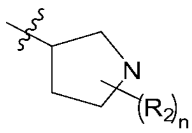, 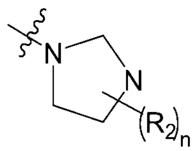, 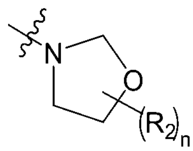,

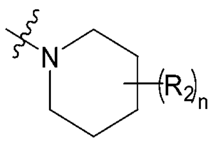, 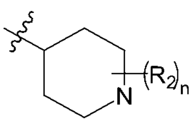, 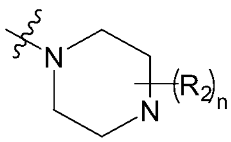, 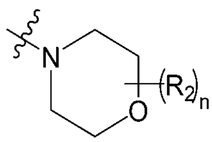, or 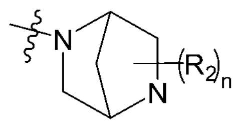.

Specifically, $R_{201}$ to $R_{204}$ may be each independently selected from: H, $C_1$-$C_6$ alkyl (for example, $-CH_3$,

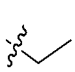, 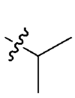, 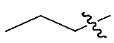, 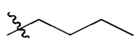, or 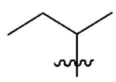

), $C_1$-$C_6$ haloalkyl (for example, $-CHF_2$, $-CH_2F$, $-CF_3$, $-CH_2$-$CH_2F$, $-CH_2$-$CHF_2$, $-CH_2$-$CF_3$, $-CH_2CH_2$-$CF_3$, and -$CH_2CH_2CH_2$-$CF_3$), $C_1$-$C_6$ hydroxyl-substituted alkyl (for example,

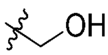, 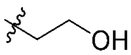,

or

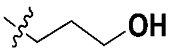

), $C_1$-$C_6$ alkoxy-substituted alkyl (for example,

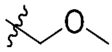, 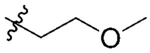,

or

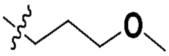

), $C_1$-$C_6$ amino-substituted alkyl (for example,

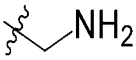, 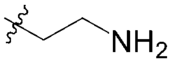,

or

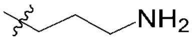

), $C_1$-$C_6$ alkylamino-substituted alkyl (for example,

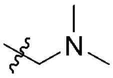, 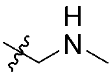,

or

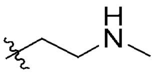

), $C_3$-$C_{10}$ cycloalkyl (for example,

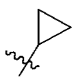, 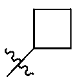, 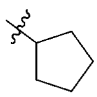, 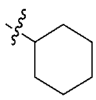, 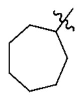,

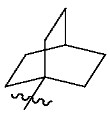, 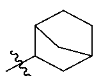, 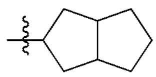, 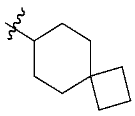, 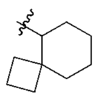, or 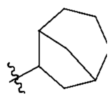

), $C_4$-$C_{10}$ cycloalkyl alkyl (for example,

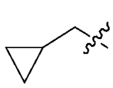, 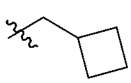, 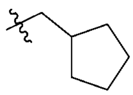, 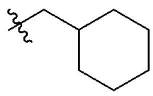, or 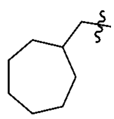

), and substituted or unsubstituted 4-10 membered heterocyclyl (for example,

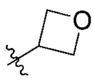, 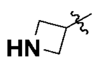, 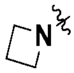,

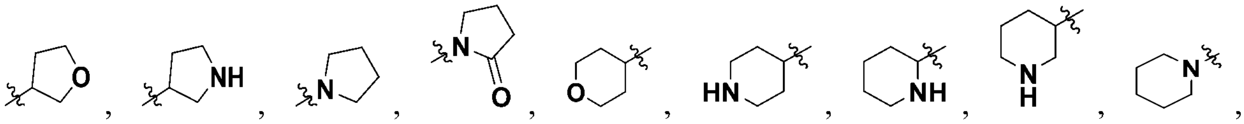
, , , , , , , , ,

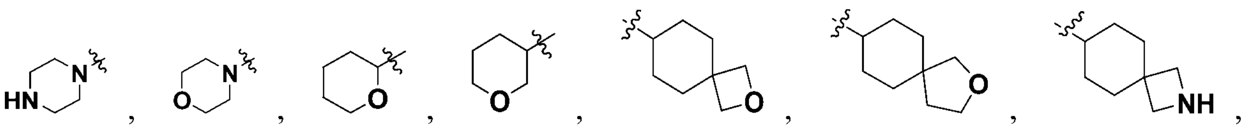
, , , , , , ,

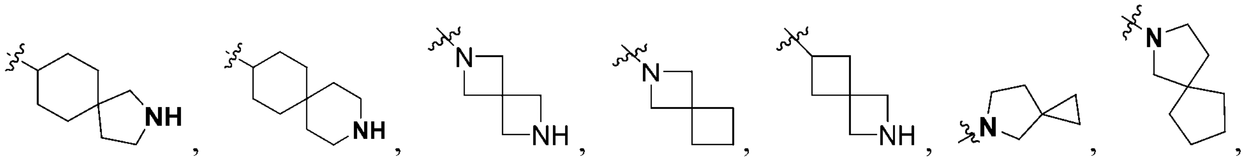
, , , , , , ,

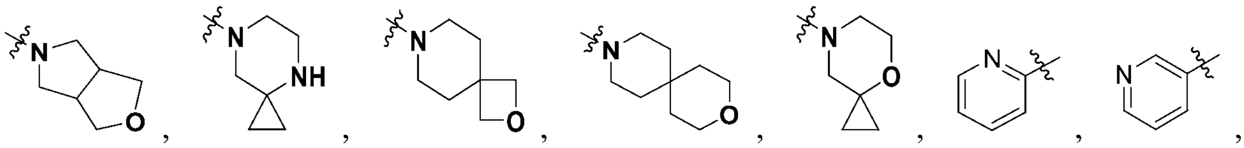
, , , , , , ,

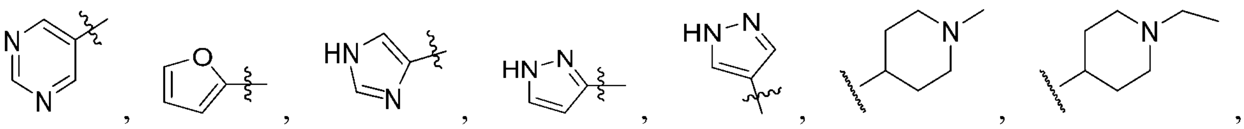
, , , , , , ,

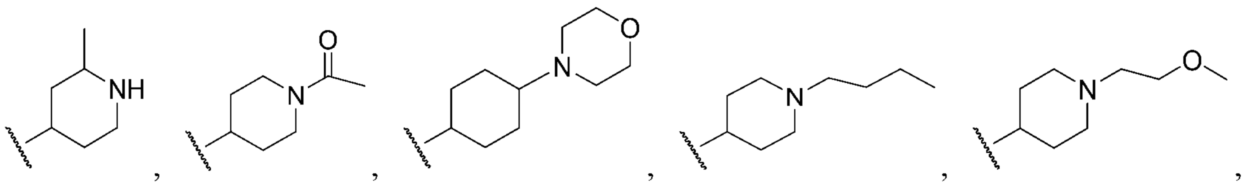
, , , , ,

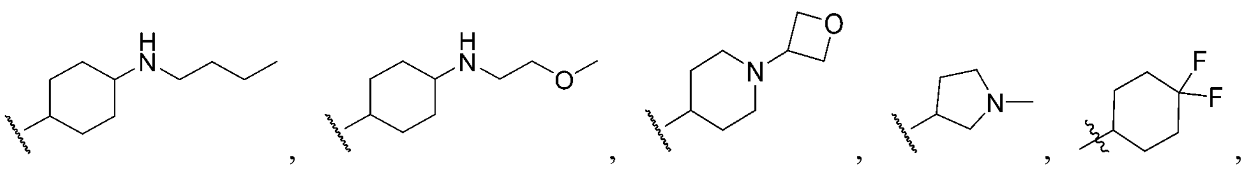
, , , , ,

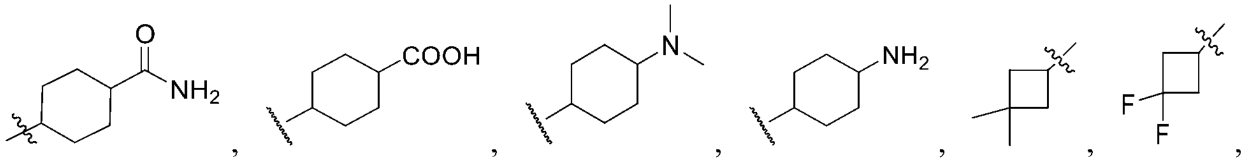
, , , , , ,

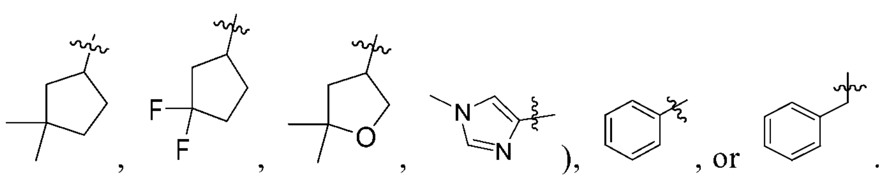
, , , ), , or .

More specifically, $R_{201}$ to $R_{204}$ may be each independently selected from: H, methyl, ethyl, n-propyl, isopropyl, $-CF_3$, $-CHF_2$, $-CH_2F$,

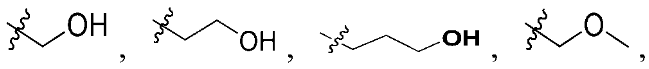

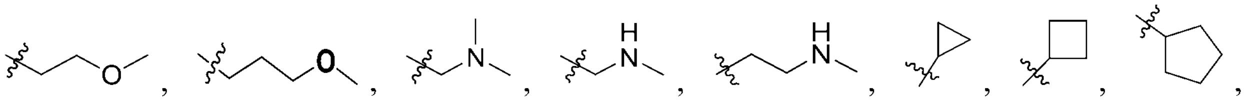

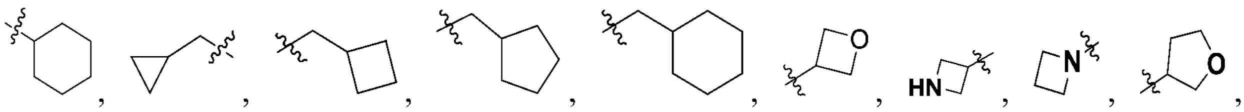

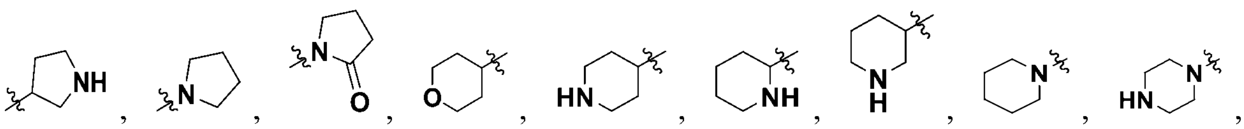

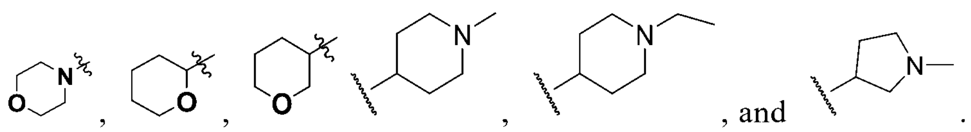

Specifically, $R_2$ may be selected from: H, =O, $C_1$-$C_6$ alkyl, $C_1$-$C_6$ haloalkyl, halogen, cyano, nitro, azido, $-OR_{201}$, $-C(O)R_{201}$, $-C(O)OR_{201}$, $-NHC(O)OR_{201}$, $-OC(O)R_{201}$, $-NHSO_2R_{201}$, $-SO_2NR_{201}R_{202}$, $-NHC(O)R_{201}$, $-C(O)NR_{201}R_{202}$, $-NR_{201}R_{202}$, $-SR_{201}$, $-S(O)_2R_{201}$, $-SO_3H$, $-(CH_2)_j$(phenyl), $-(CH_2)_j$(4-10 membered heterocyclyl), and $-(CH_2)_j(C_3$-$C_{10}$ cycloalkyl), where j is an integer from 0 to 5; specifically, the 4-10 membered heterocyclic ring is a 4-6 membered heterocyclic ring, for example,

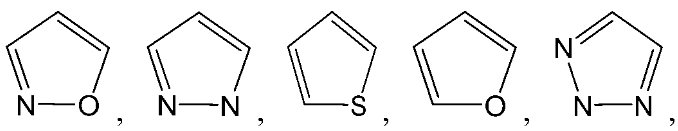

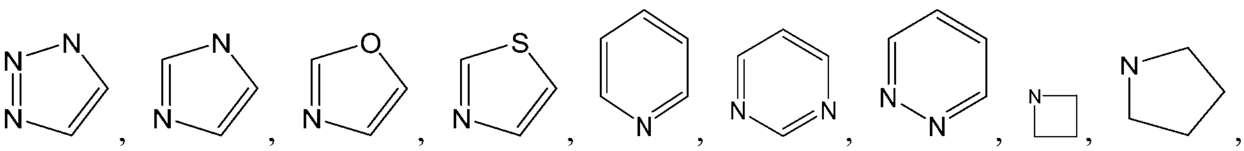

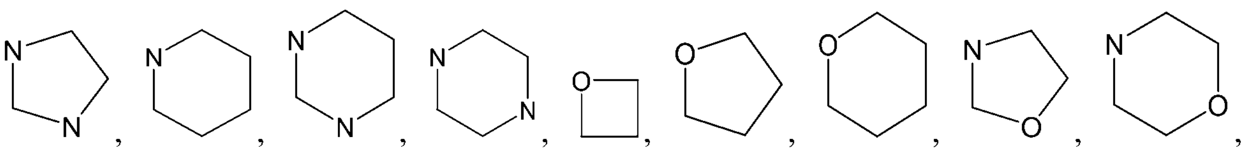

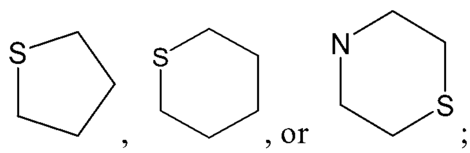

specifically, the $C_3$-$C_{10}$ cycloalkyl is $C_3$-$C_6$ cycloalkyl, for example,

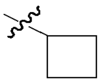, 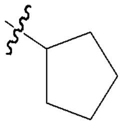, and 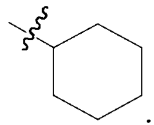.

More specifically, $R_2$ may be selected from: H, =O, $C_1$-$C_6$ alkyl, $C_1$-$C_6$ haloalkyl, halogen, halogen, cyano, nitro, azido, $C_1$-$C_6$alkoxy, $C_3$-$C_6$ cycloalkyl, and $C_4$-$C_{10}$ cycloalkyl alkyl.

In some embodiments of the present invention, $R_2$ is selected from: H, methyl, ethyl, n-propyl, isopropyl, $-CF_3$, $-CHF_2$, $-CH_2F$,

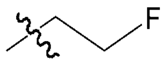, 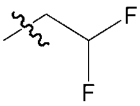, 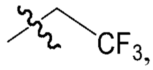

F, Cl, Br, I, cyano, nitro, azido, hydroxyl, methoxy, ethoxy,

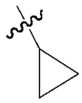, 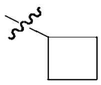, 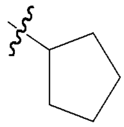, 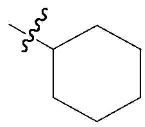,

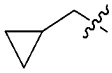, 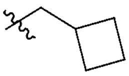, 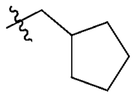, 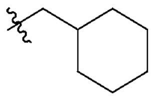, 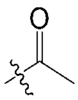, 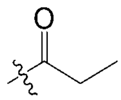, and 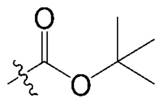.

In some embodiments of the present invention, $R_0$ is selected from:

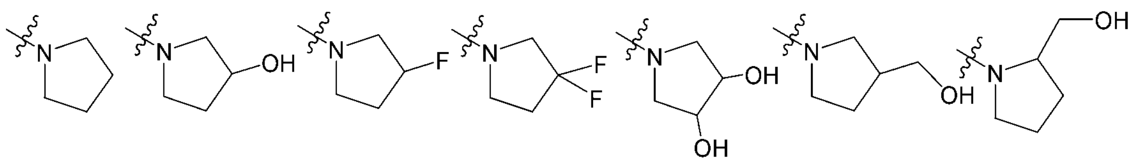

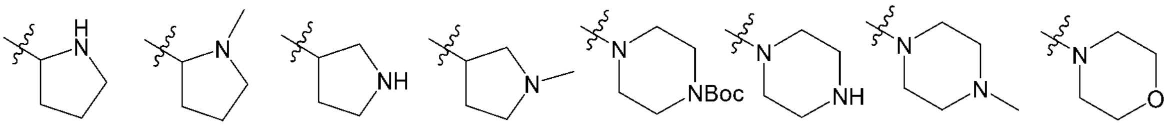

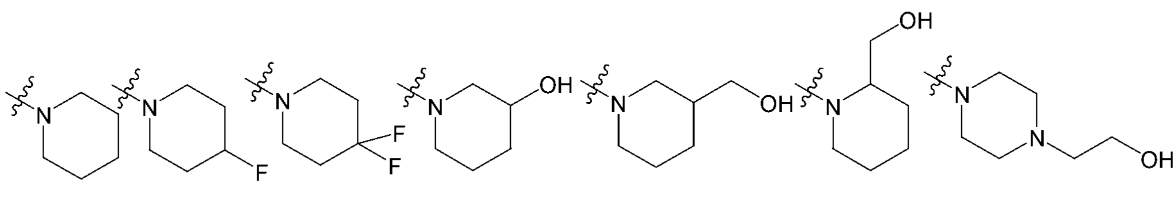

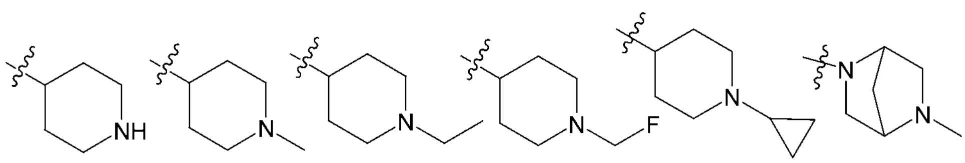

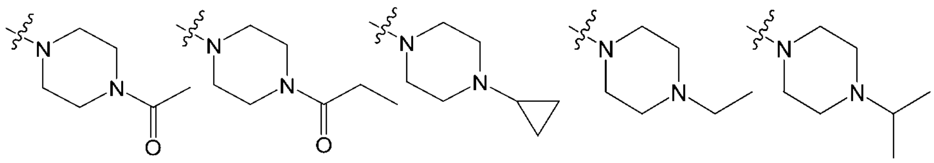.

In an embodiment of the present invention, $R_0$ is $-NR_{001}R_{002}$, wherein $R_{001}$ and $R_{002}$ are independently selected from: H, $C_1$-$C_6$ alkyl, $C_1$-$C_6$ haloalkyl, $C_1$-$C_6$ hydroxyl-substituted alkyl, $C_1$-$C_6$ alkoxy-substituted alkyl, $C_1$-$C_6$ amino-substituted alkyl, $C_1$-$C_6$ alkylamino-substituted alkyl, $C_3$-$C_6$ cycloalkyl, and $C_4$-$C_{10}$ cycloalkyl alkyl.

Specifically, $R_{001}$ and $R_{002}$ are independently selected from: H, methyl, ethyl, n-propyl, isopropyl, $-CF_3$, $-CHF_2$, $-CH_2F$,

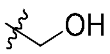, 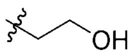, 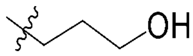, 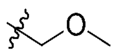,

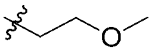, 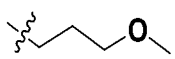, 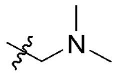, 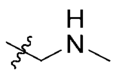, 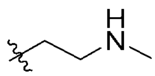, 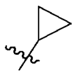, 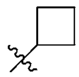, 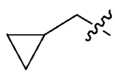,

and

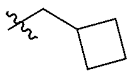.

In some embodiments of the present invention, $R_0$ is selected from:

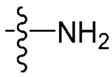, 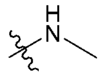,

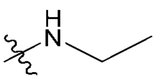, 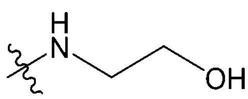, 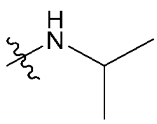, 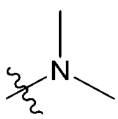, 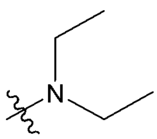, 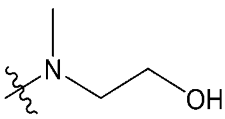,

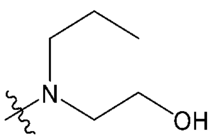, and 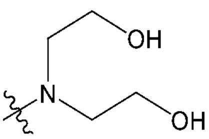.

In other embodiments of the present invention, $R_0$ is selected from: H, cyano, -O($C_0$-$C_{10}$ alkyl), -O($C_1$-$C_{10}$ silyl), -S($C_0$-$C_{10}$ alkyl), -C(O)($C_0$-$C_{10}$ alkyl), -C(O)O($C_0$-$C_{10}$ alkyl), - OC(O)($C_0$-$C_{10}$ alkyl), -N($C_0$-$C_{10}$ alkyl)$SO_2$($C_0$-$C_{10}$ alkyl), $-SO_2N$($C_0$-$C_{10}$ alkyl)($C_0$-$C_{10}$ alkyl), -N($C_0$-$C_{10}$ alkyl)C(O)($C_0$-$C_{10}$ alkyl), -C(O)N($C_0$-$C_{10}$ alkyl)($C_0$-$C_{10}$ alkyl), and $-SO_2$ ($C_0$-$C_{10}$ alkyl), wherein the alkyl is optionally substituted by a group selected from: halogen, cyano, hydroxyl, amino, $C_1$-$C_6$ alkoxy, $C_1$-$C_6$ alkylamino, and $C_3$-$C_6$ cycloalkyl; for example, $R_0$ is selected from: H, cyano, -OH,

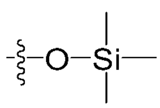, 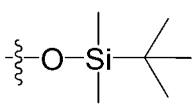, 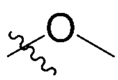, 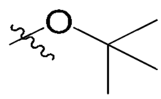,

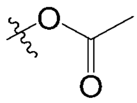, 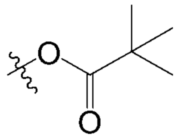,

-COOH, and

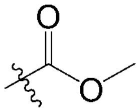.

Specifically, $R_3$ to $R_5$ are independently selected from: H, $C_1$-$C_6$ alkyl, $C_1$-$C_6$ haloalkyl, $C_1$-$C_6$ alkoxy-substituted alkyl, $C_1$-$C_6$ alkylamino-substituted alkyl, $C_3$-$C_6$ cycloalkyl, and $C_4$-$C_{10}$ cycloalkyl alkyl; more specifically, $R_3$ to $R_5$ are independently selected from: H, methyl, ethyl, n-propyl, isopropyl, cyclopropyl, and cyclobutyl.

In some embodiments of the present invention, $R_3$ is H.

In some embodiments of the present invention, $R_4$ is H.

In some embodiments of the present invention, $R_5$ is H.

Specifically, -Cy- may be selected from:

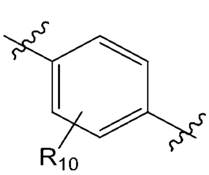, 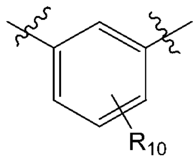, 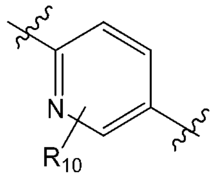,

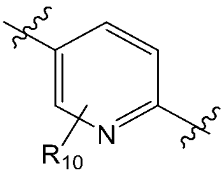, 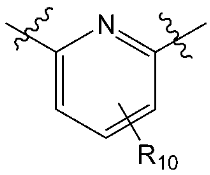, 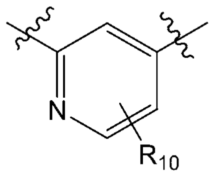, 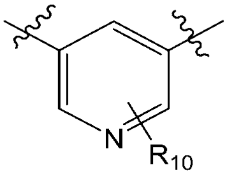, 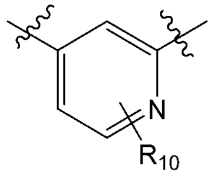,

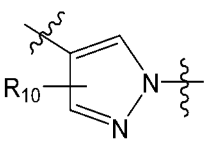, 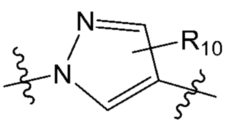, 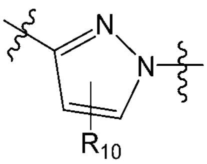, 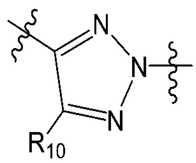, 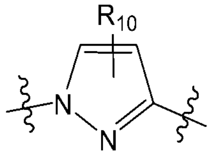,

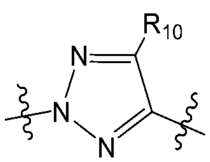, 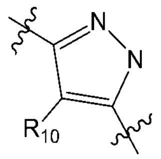, 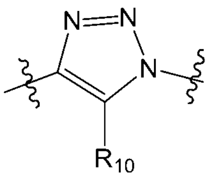, 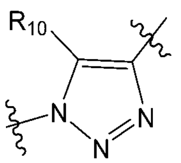, 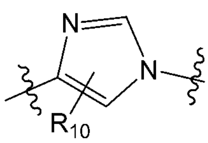,

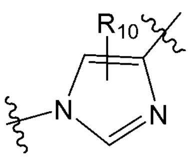, 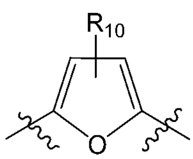, 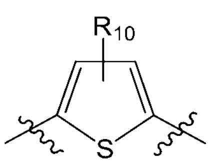, 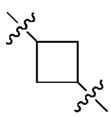, 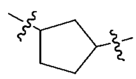, 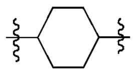,

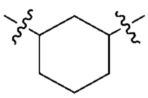, 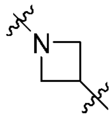, 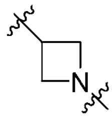, 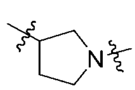, 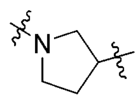, 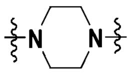, 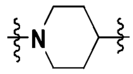, 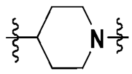,

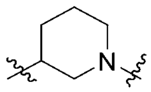;

Each $R_{10}$ is independently selected from: H, halogen, $C_1$-$C_6$ alkyl, $C_1$-$C_6$ haloalkyl, $C_1$-$C_6$ alkoxy-substituted alkyl, $C_1$-$C_6$ alkylamino-substituted alkyl, $C_3$-$C_6$ cycloalkyl, $C_4$-$C_{10}$ cycloalkyl alkyl, and 4-10 membered heterocyclyl; more specifically, each $R_{10}$ is independently selected from: H, methyl, ethyl, n-propyl, isopropyl, cyclopropyl, and cyclobutyl.

In an embodiment of the present invention, $L_1$ is -C(O)N$R_3$-, wherein $R_3$ is as defined above in the present invention.

In some embodiments of the present invention, $L_1$ is -C(O)NH-.

In another embodiment of the present invention, $L_1$ is -Cy-, wherein -Cy- is as defined above in the present invention.

In some embodiments of the present invention, -Cy- is selected from:

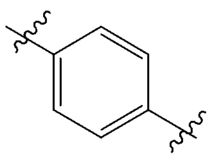,

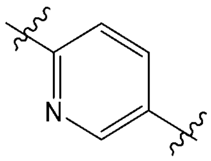, 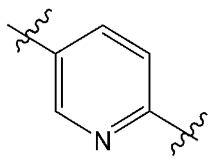, 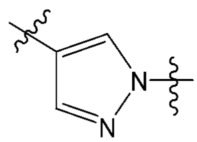, 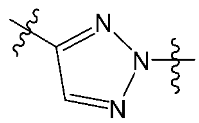, 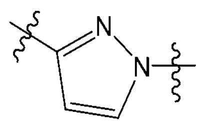,

and

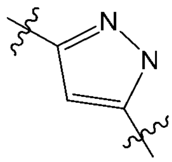.

In another embodiment of the present invention, $L_1$ is a single bond.

In another embodiment of the present invention, $L_1$ is -C(O)-.

Specifically, for the definition of $L_2$, a and b are independently selected from: 0, 1, 2, 3, 4, and 5.

Specifically, for the definition of $L_2$, $R_{L203}$ and $R_{L204}$ may be independently selected from: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, and $C_4$-$C_{10}$ cycloalkyl alkyl.

Specifically, $L_2$ is a single bond or $C_2$-$C_{10}$ alkylene, wherein one or more methylene units in alkylene are optionally and independently substituted by a group selected from: -N($R_4$)-, - N($R_4$)C(O)-, -C(O)N($R_4$)-, -N($R_4$)S(O)$_2$-, -S(O)$_2$N($R_4$)-, -O-, -C(O)-, -OC(O)-, -C(O)O-, -S-, -S(O)- or -S(O)$_2$, wherein one or more H atoms in the alkylene are optionally and independently substituted by the following groups: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, and $C_4$-$C_{10}$ cycloalkyl alkyl; more specifically, $L_2$ is $C_2$-$C_6$ alkylene, wherein one or more metylene units in the alkylene are optionally and independently substituted by a group selected from: -NH-, -O-, -C(O)-, -OC(O)-, -C(O)O-, and -S-.

In some embodiments of the present invention, $L_2$ is selected from: a single bond,

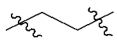,

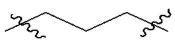, 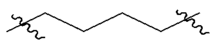, 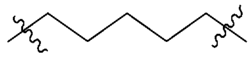, 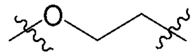, 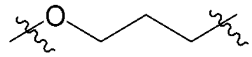,

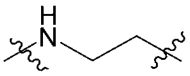, 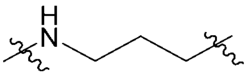, 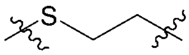, and 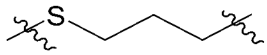.

Specifically, for the definition of $L_3$, c and d are independently selected from: 0, 1, 2, 3, 4, and 5.

Specifically, for the definition of $L_3$, $R_{L303}$ and $R_{L304}$ may be independently selected from: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, and $C_4$-$C_{10}$ cycloalkyl alkyl.

Specifically, $L_3$ is $C_1$-$C_6$ alkylene, wherein one or more methylene units in alkylene are optionally and independently substituted by a group selected from: -NH-, -O-, -C(O)-, - OC(O)-, -C(O)O-, -S-, wherein one or more H atoms in alkylene are optionally and independently substituted by the following groups: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, or $C_4$-$C_{10}$ cycloalkyl alkyl; and in some embodiments of the present invention, $L_3$ is methylene ($-CH_2-$).

In an embodiment of the present invention, the compound has a structure of:

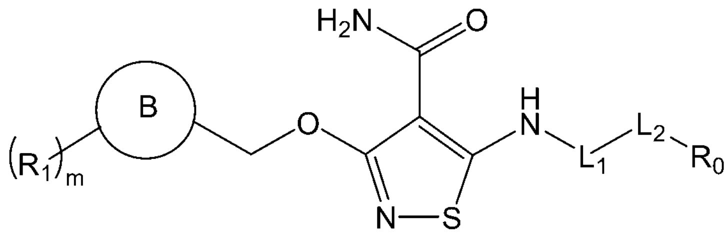

(II)

In an embodiment of the present invention, the compound has a structure of:

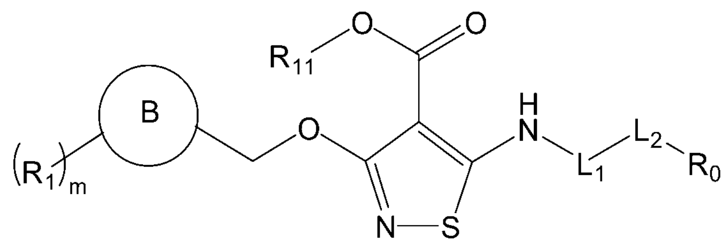

(III)

wherein $R_{11}$ is $C_1$-$C_{10}$ alkyl.

Specifically, $R_{11}$ is $C_1$-$C_6$ alkyl, particularly $C_1$-$C_3$ alkyl, for example, methyl and ethyl.

In some embodiments of the present invention, the compound has a structure of:

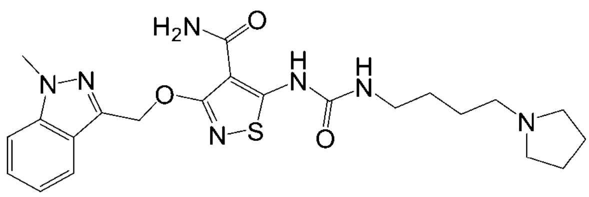

**T002**

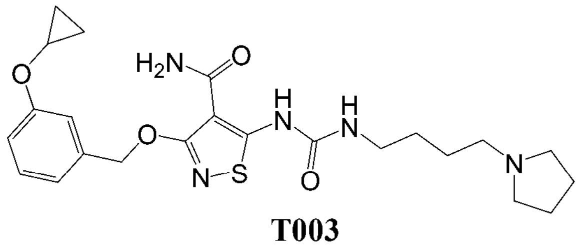

**T003**

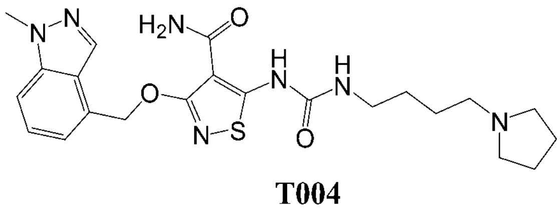

**T004**

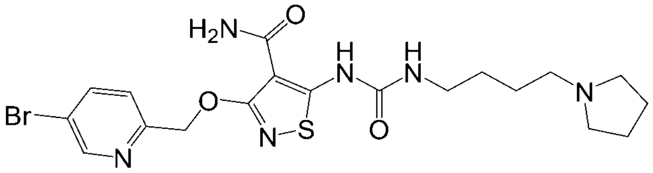

**T005**

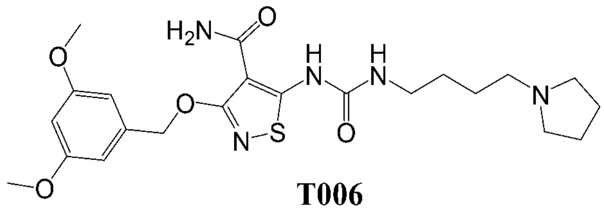

**T006**

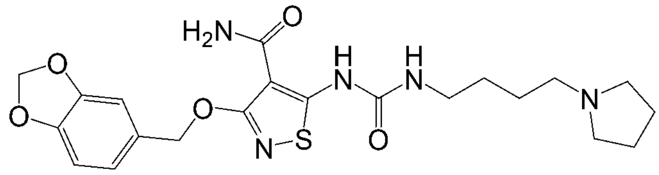

**T007**

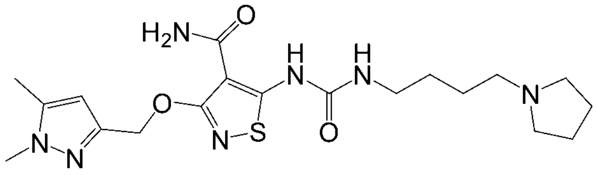

**T009**

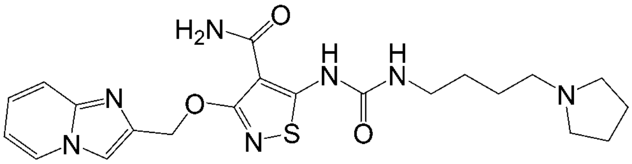

**T013**

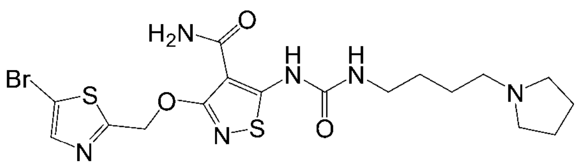

**T015**

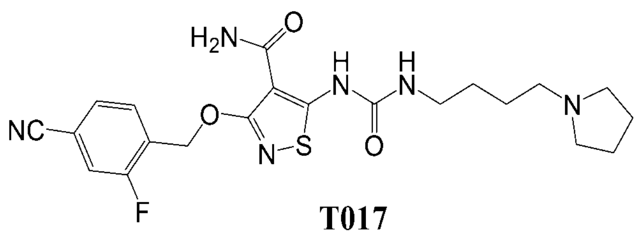

**T017**

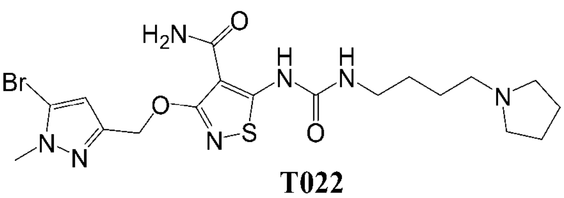

**T022**

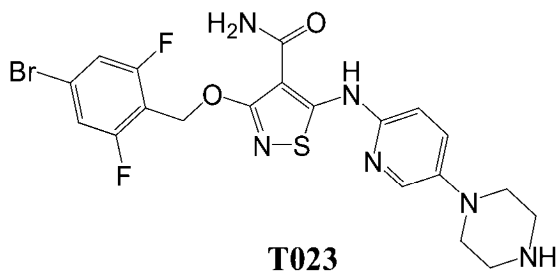

**T023**

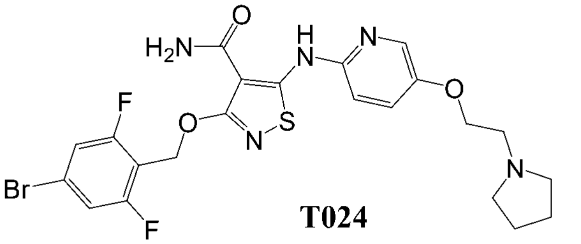

**T024**

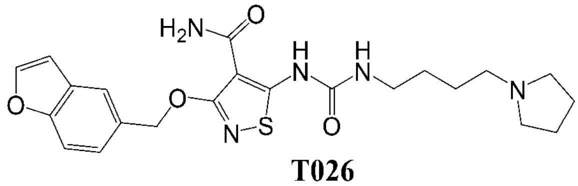

**T026**

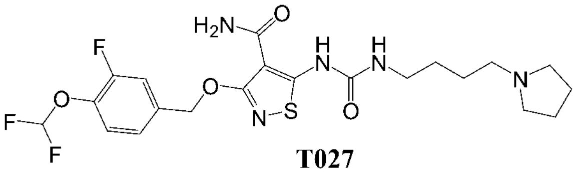

**T027**

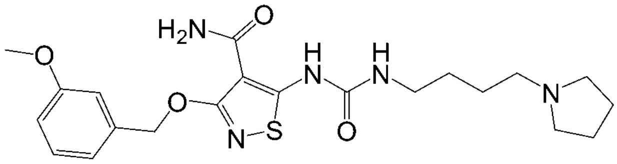

**T028**

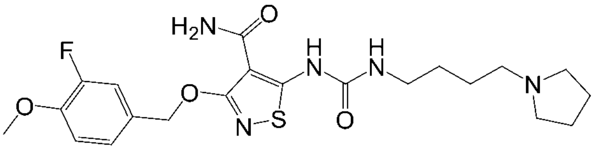

**T029**

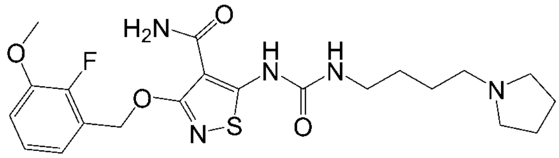

**T030**

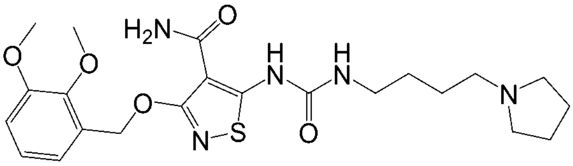

**T032**

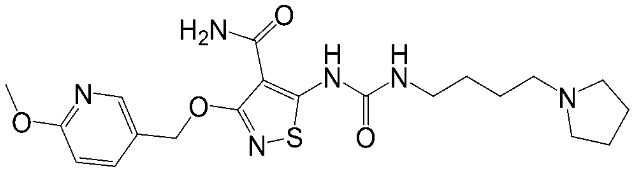

**T033**

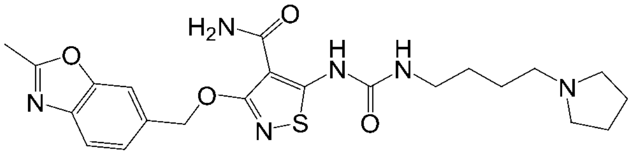

**T035**

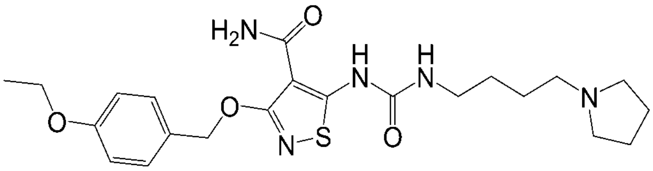

**T036**

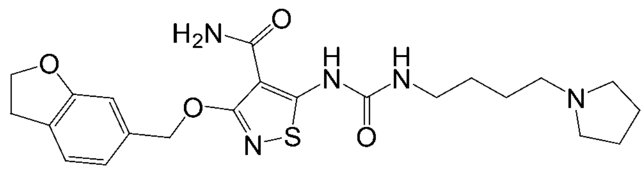

**T042**

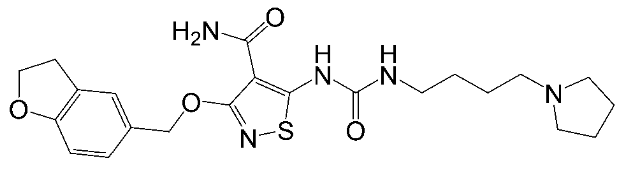

T044

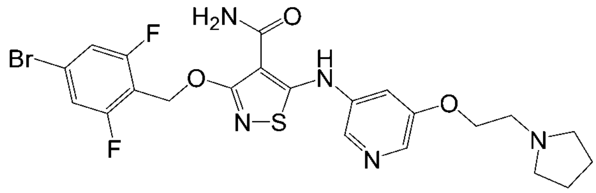

T045

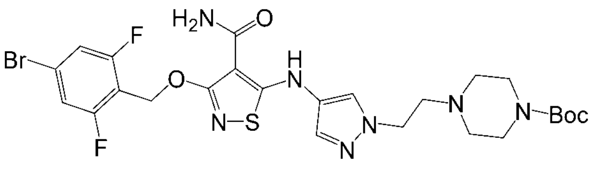

T046

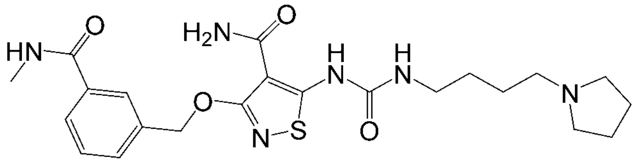

T049

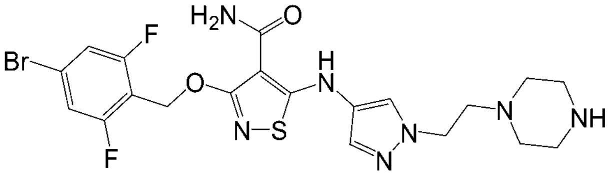

T050

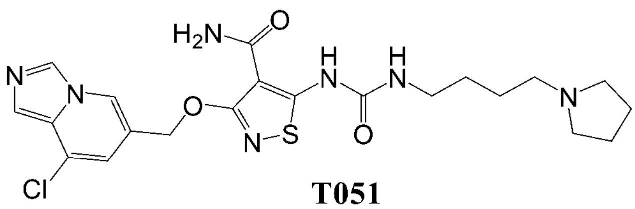

T051

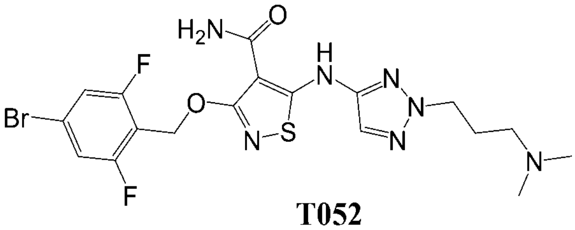

T052

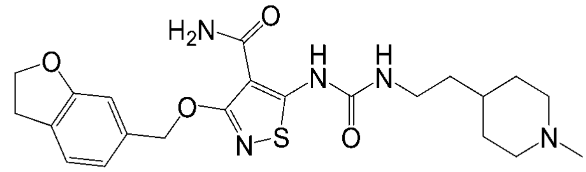

T053

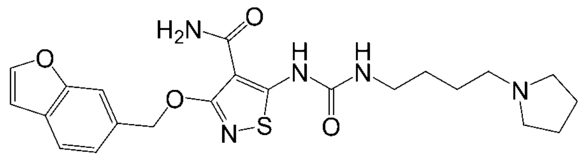

T054

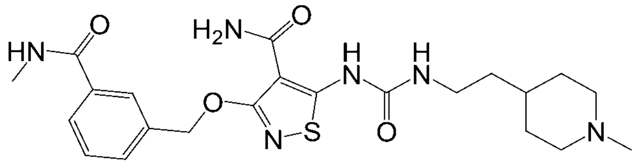

T055

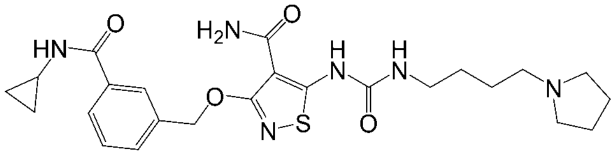

T056

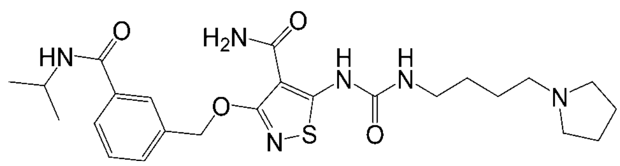

T057

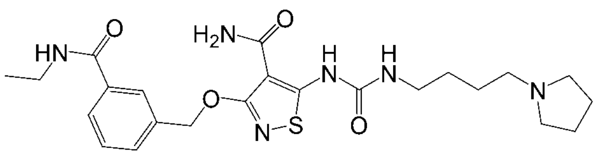

T058

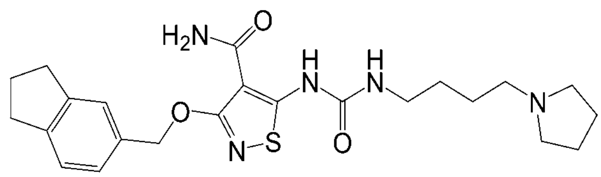

T061

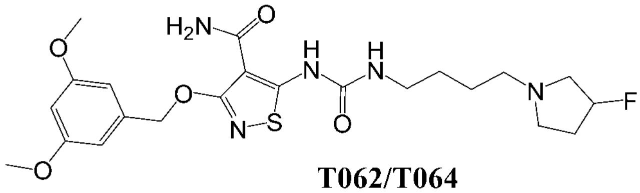

**T062/T064**

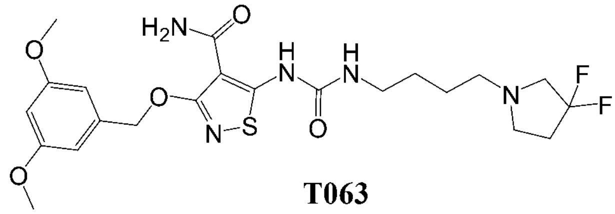

**T063**

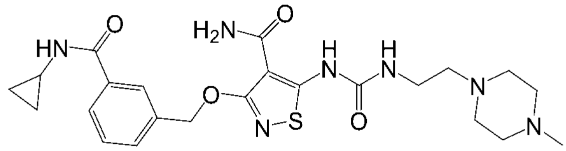

**T065**

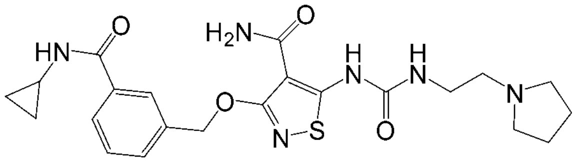

**T066**

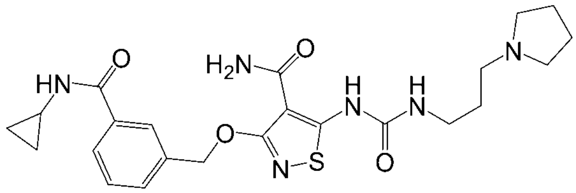

**T067**

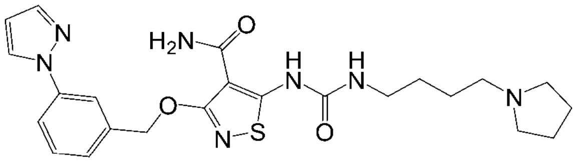

**T068**

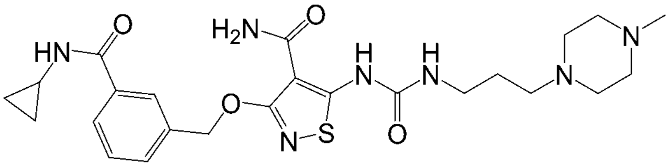

**T069**

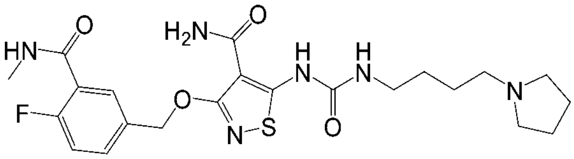

**T070**

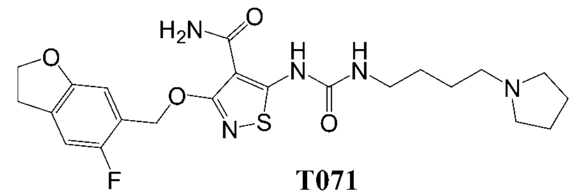

**T071**

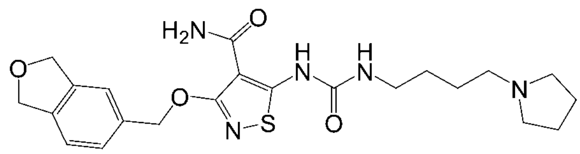

**T072**

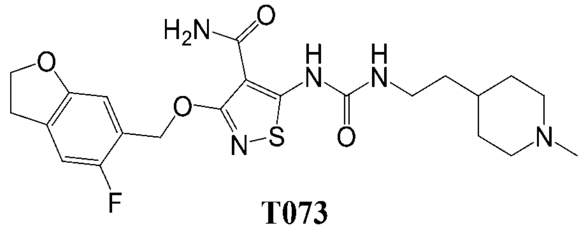

**T073**

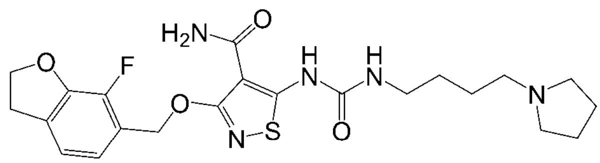

**T074**

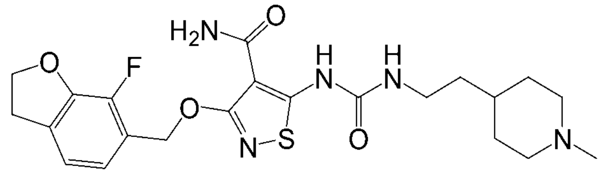

**T075**

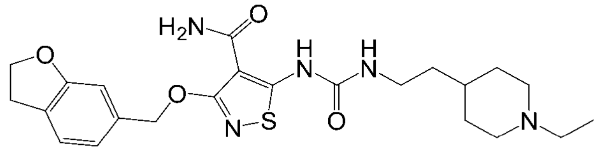

**T076**

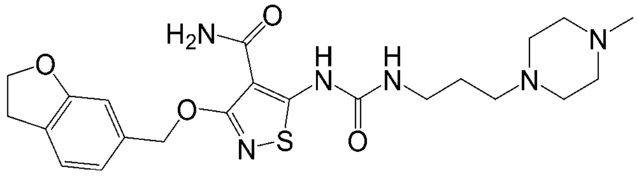

**T077**

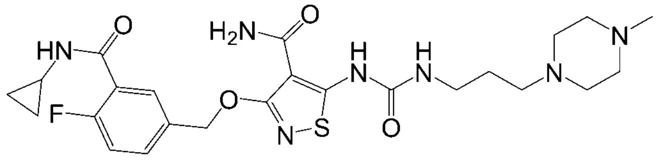

**T078**

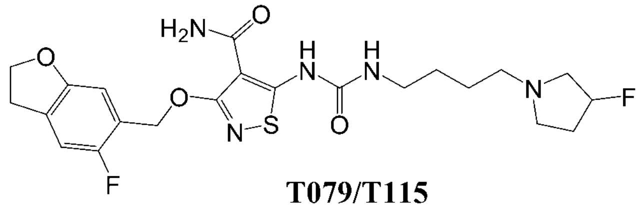

**T079/T115**

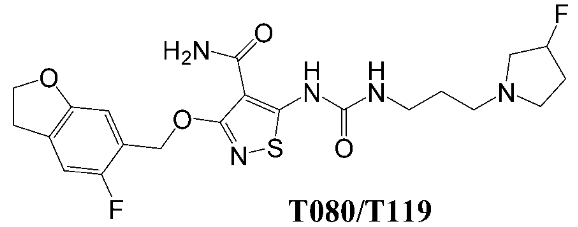

**T080/T119**

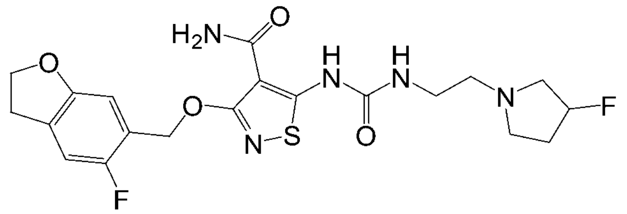

**T081**

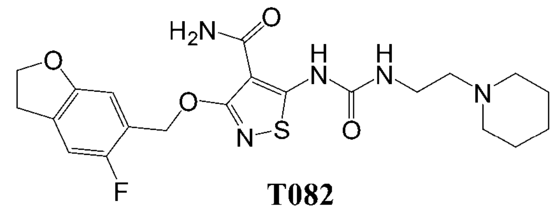

**T082**

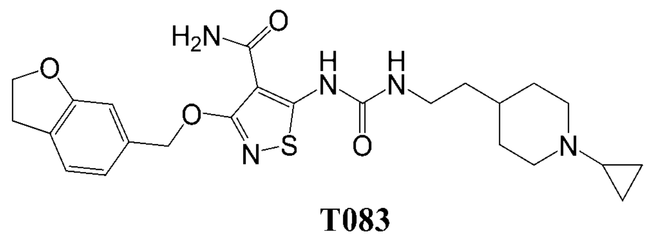

**T083**

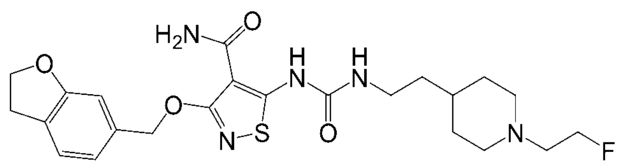

**T084**

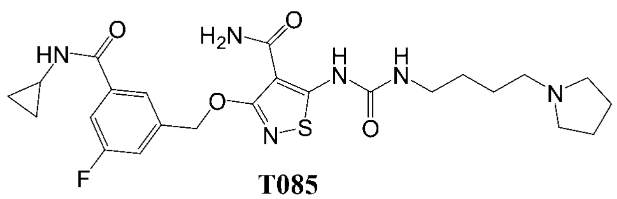

**T085**

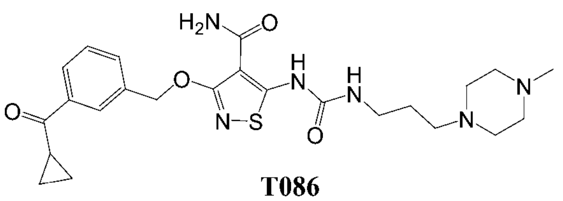

**T086**

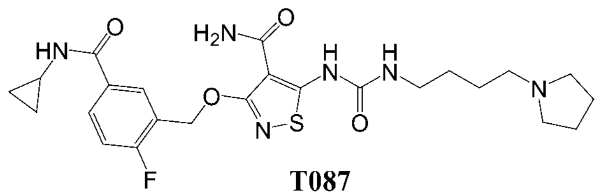

**T087**

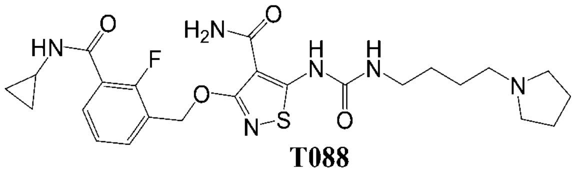

**T088**

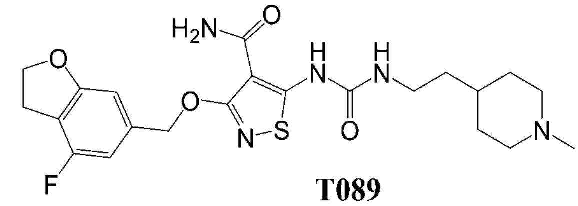

**T089**

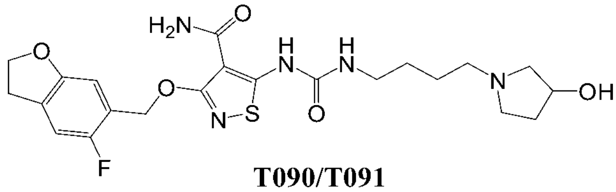

**T090/T091**

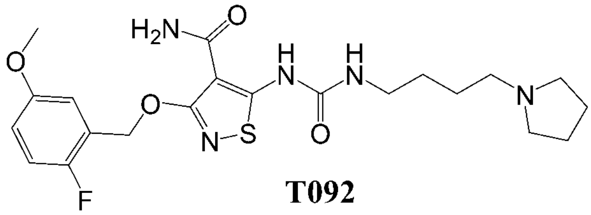

**T092**

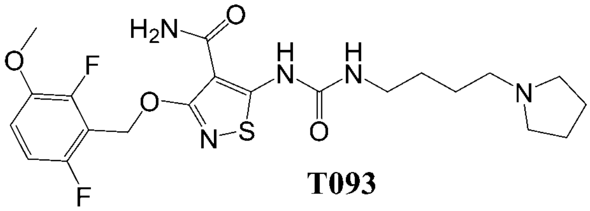

**T093**

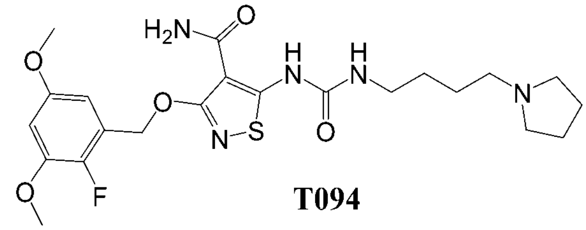

**T094**

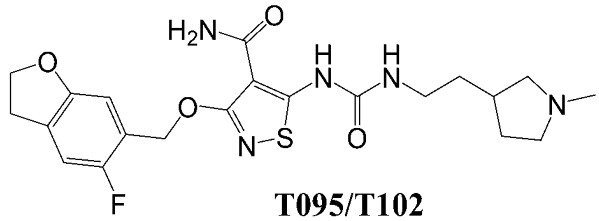

**T095/T102**

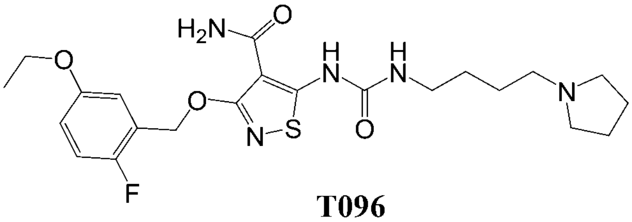

**T096**

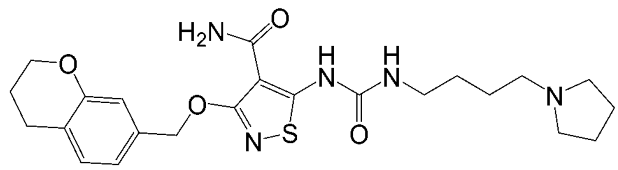

**T097**

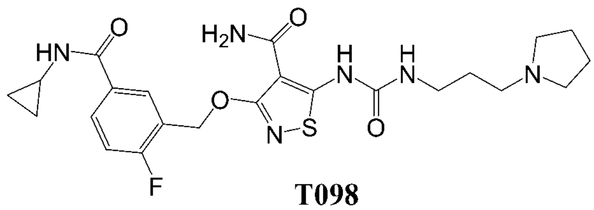

**T098**

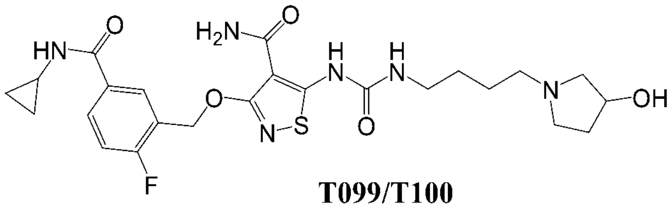

**T099/T100**

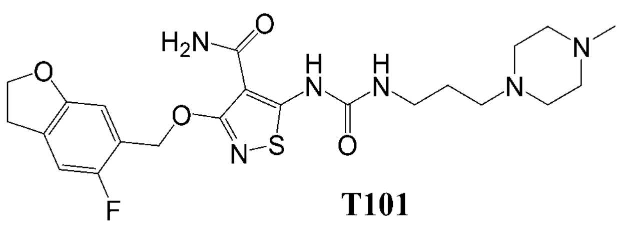

**T101**

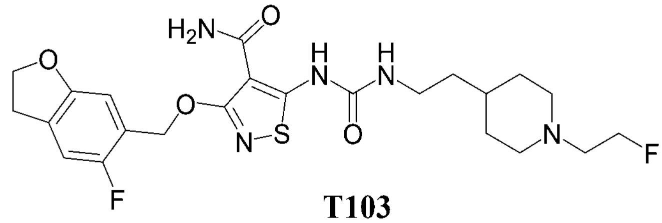

**T103**

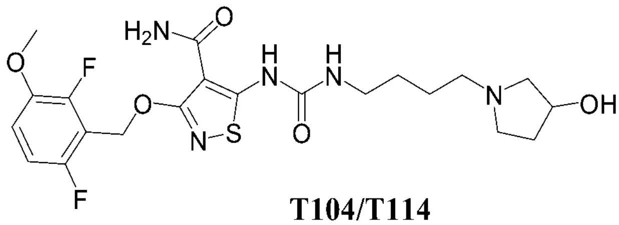

**T104/T114**

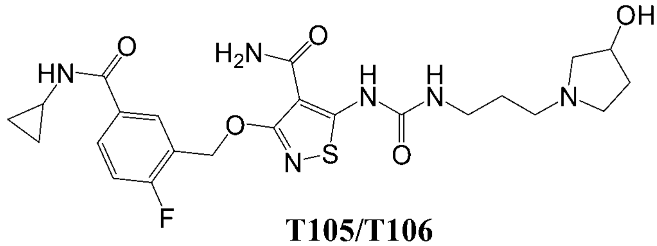

**T105/T106**

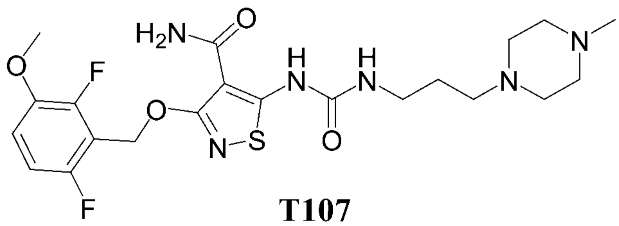

**T107**

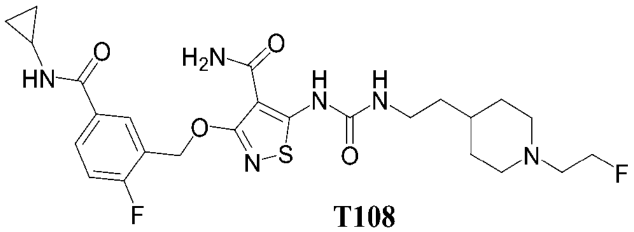

**T108**

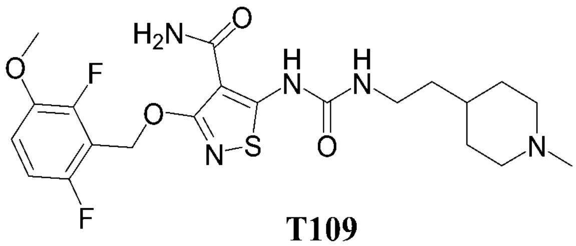

**T109**

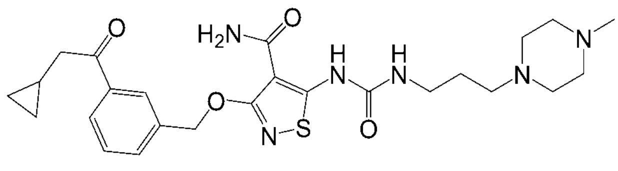

**T110**

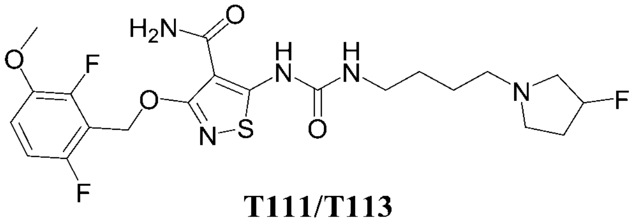

**T111/T113**

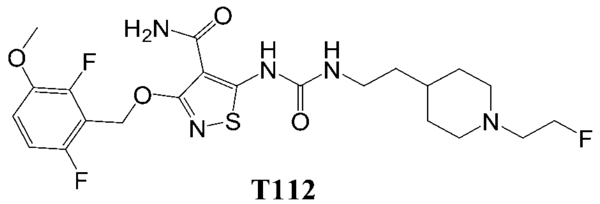

**T112**

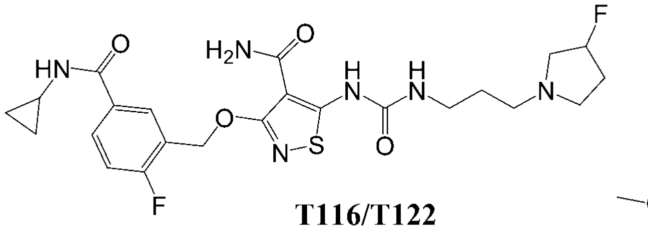

**T116/T122**

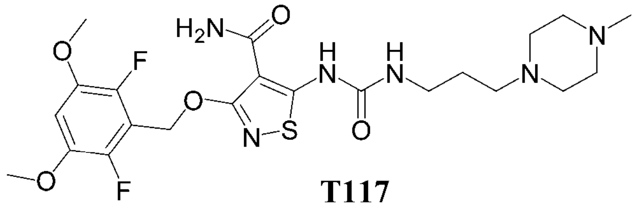

**T117**

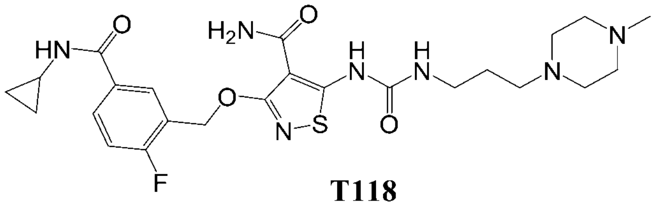

**T118**

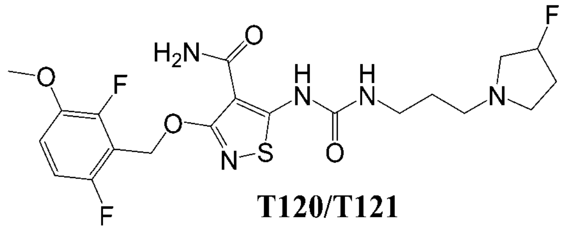

**T120/T121**

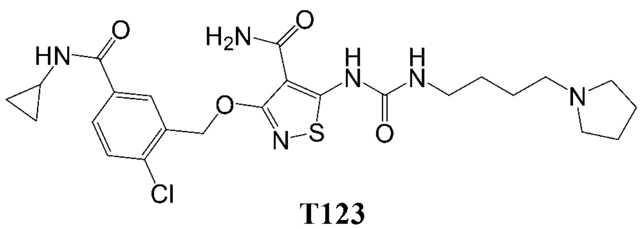

**T123**

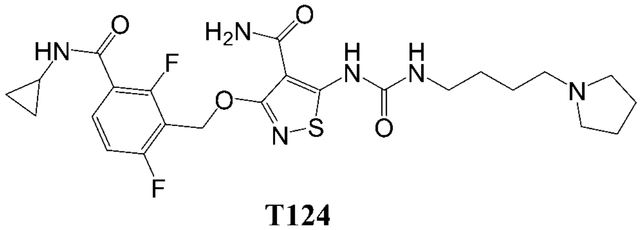

**T124**

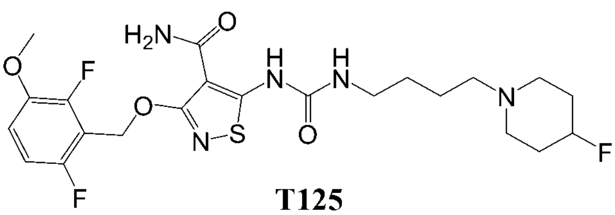

**T125**

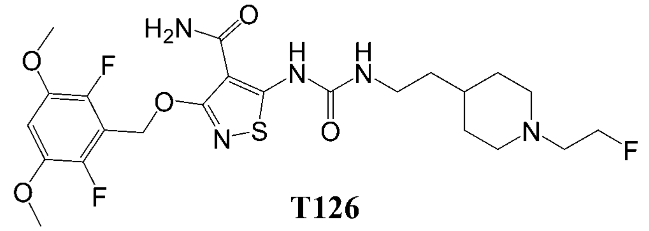

**T126**

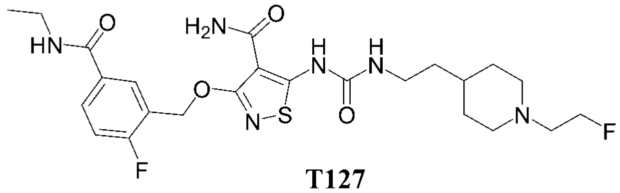

**T127**

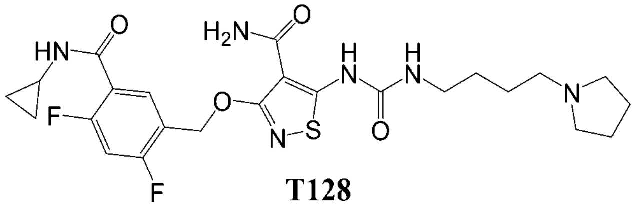

**T128**

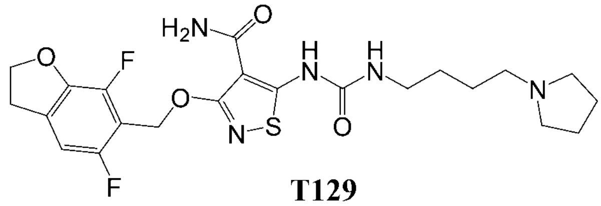

**T129**

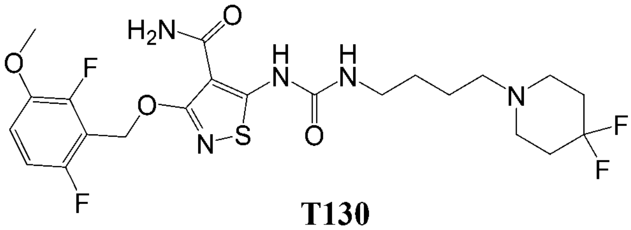

**T130**

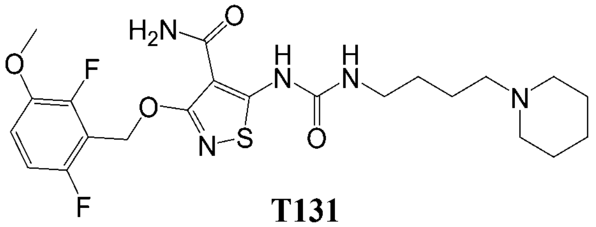

**T131**

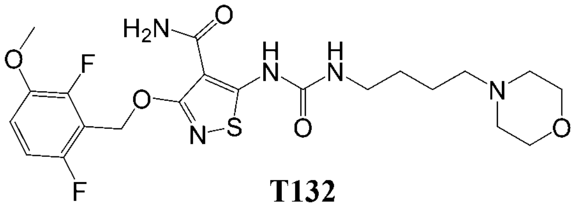

**T132**

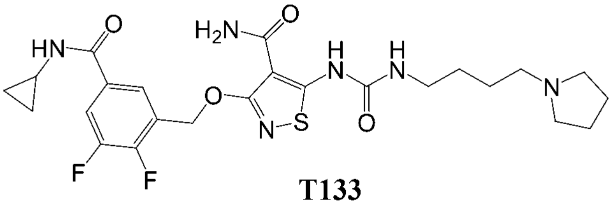

**T133**

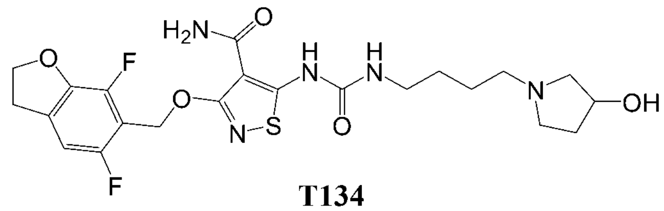

**T134**

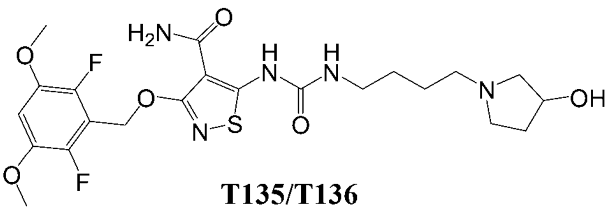

**T135/T136**

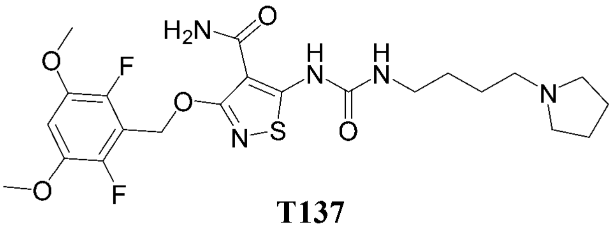

**T137**

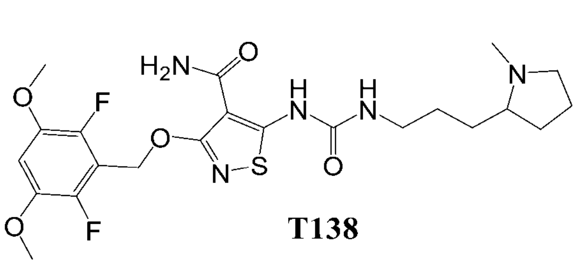
T138

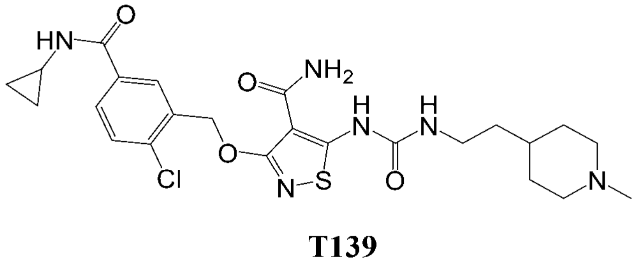
T139

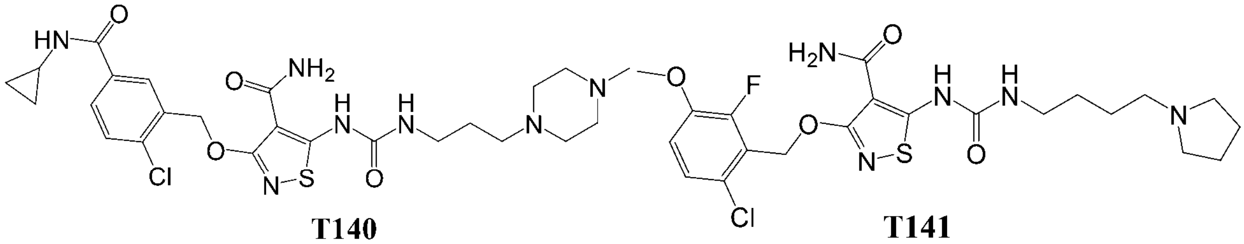
T140

T141

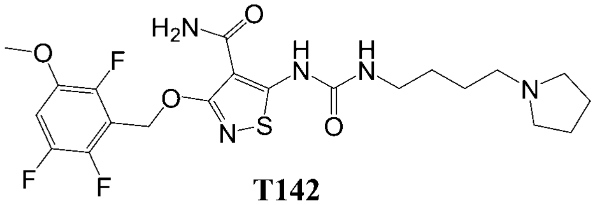
T142

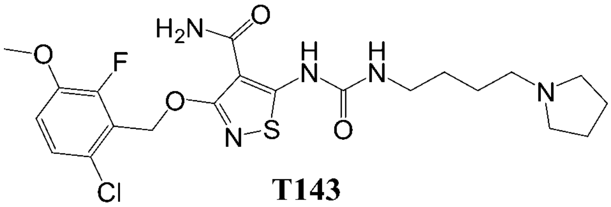
T143

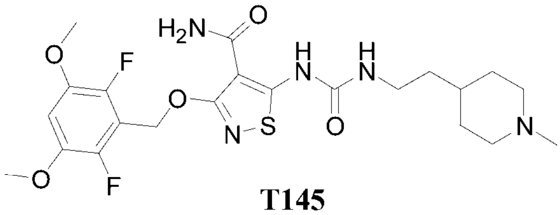
T145

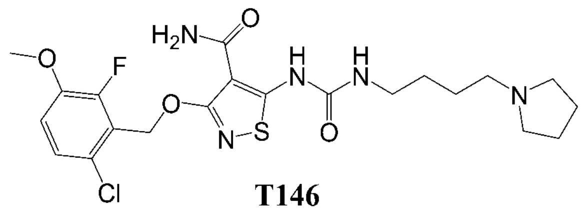
T146

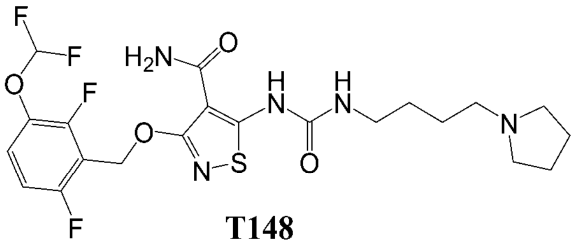
T148

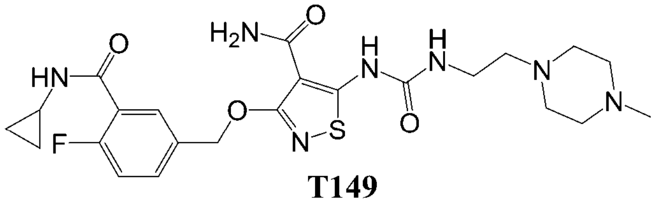
T149

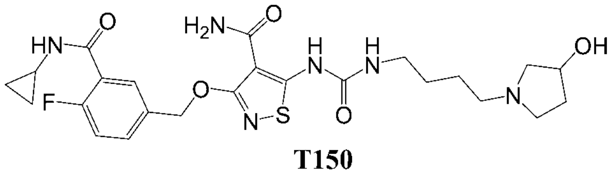
T150

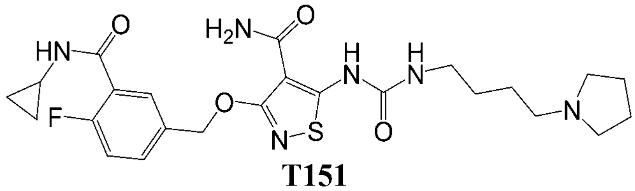
T151

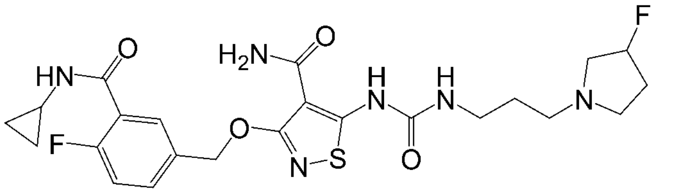

**T152**

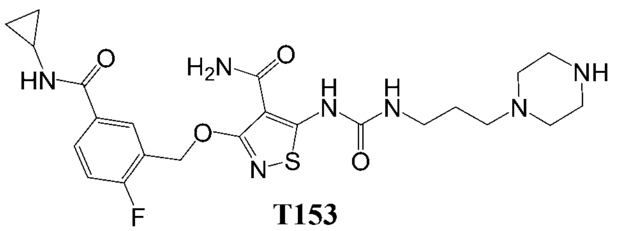

**T153**

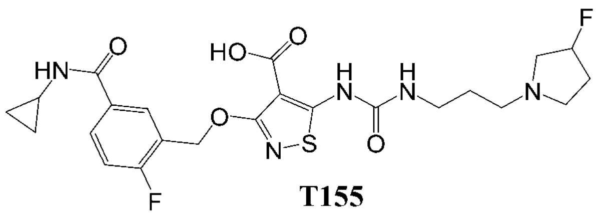

**T155**

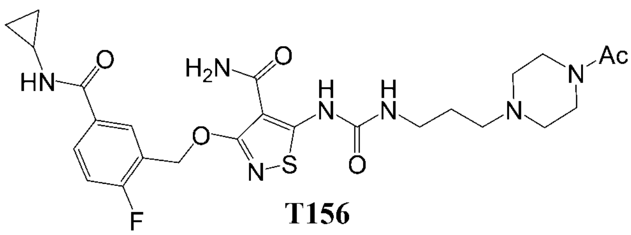

**T156**

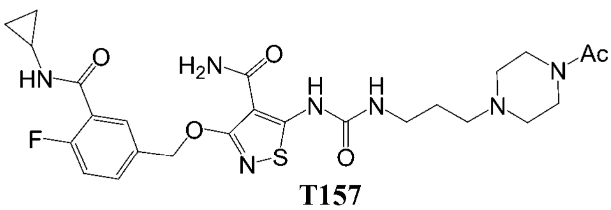

**T157**

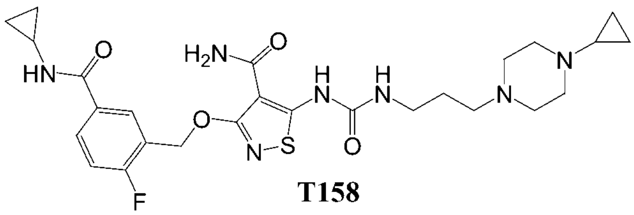

**T158**

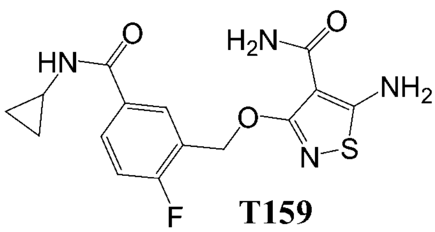

**T159**

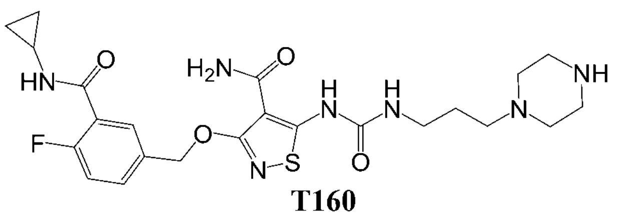

**T160**

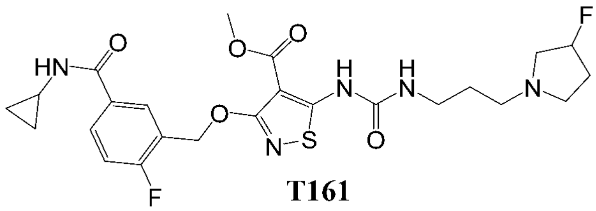

**T161**

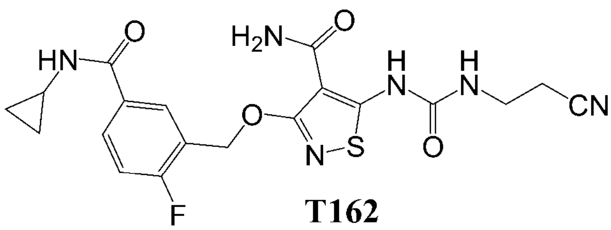

**T162**

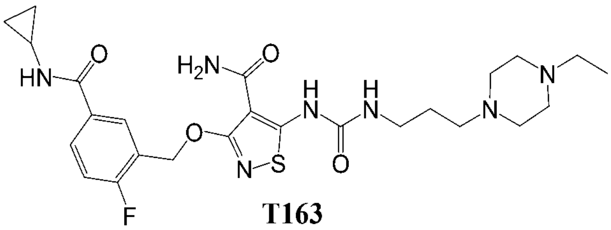

**T163**

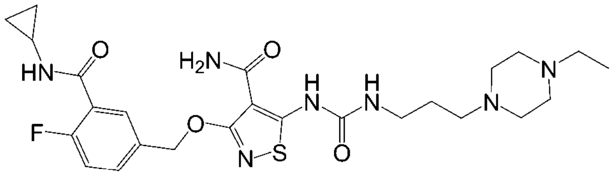

**T164**

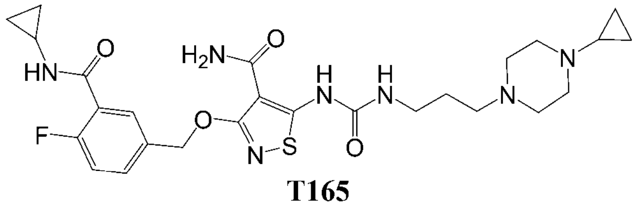

**T165**

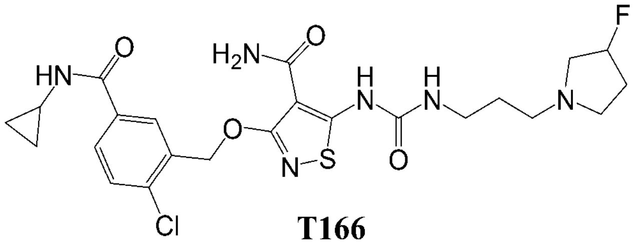

**T166**

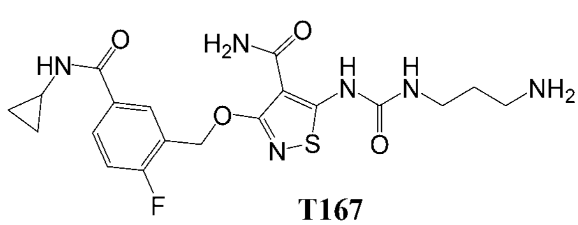

**T167**

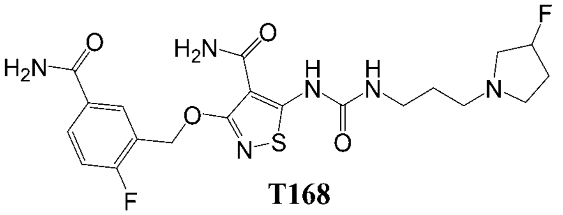

**T168**

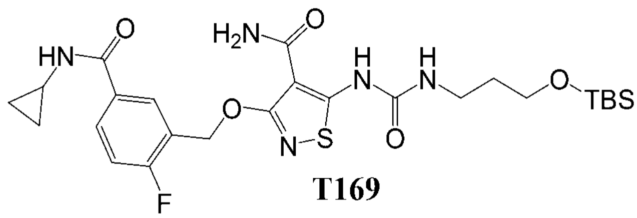

**T169**

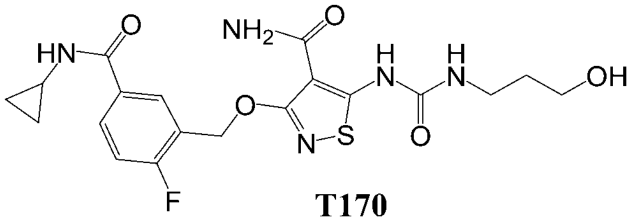

**T170**

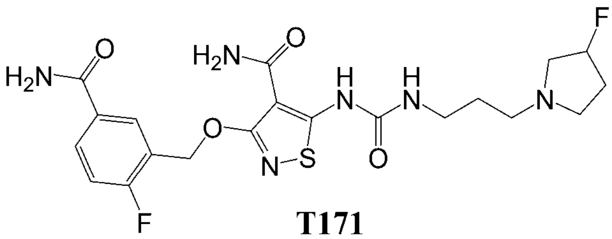

**T171**

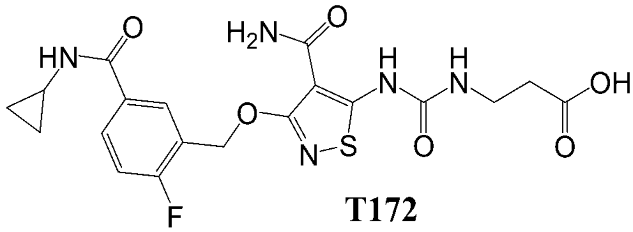

**T172**

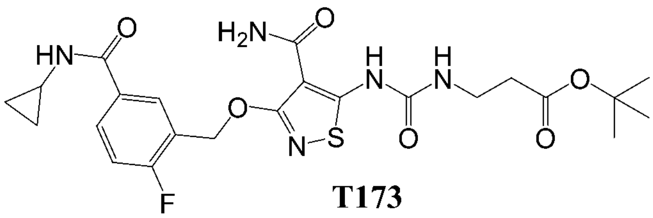

**T173**

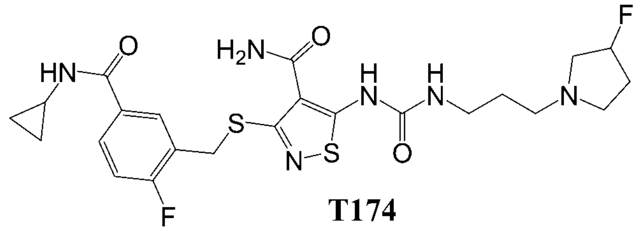

**T174**

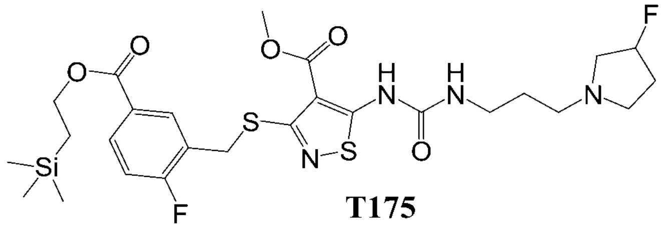

**T175**

.

In a second aspect of the present invention, a pharmaceutically acceptable salt, stereoisomer, ester, prodrug, solvate, or deuterated compound of the compound according to the first aspect is provided.

In some embodiments of the present invention, the stereoisomer has a structure of:

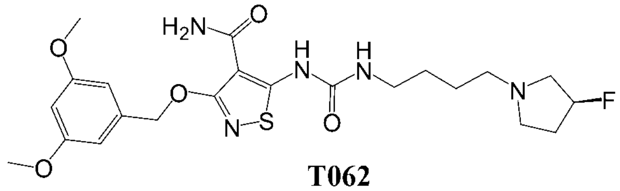

**T062**

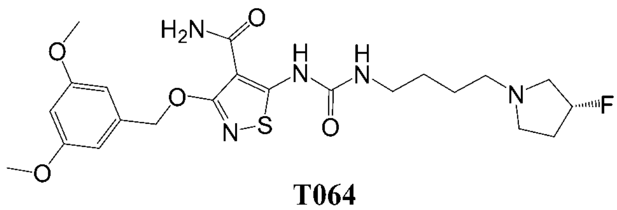

**T064**

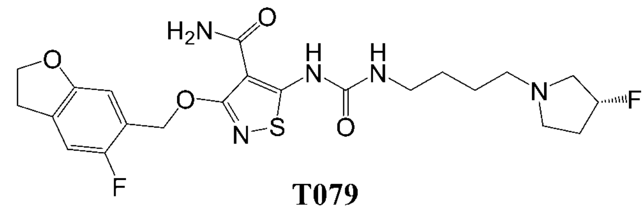

**T079**

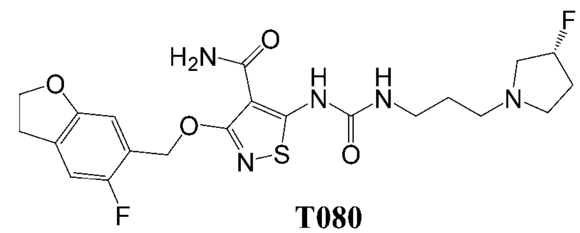

**T080**

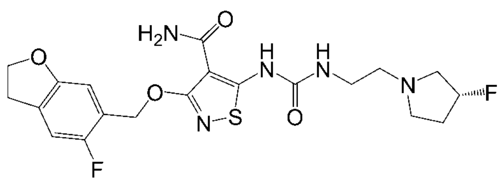

**T081**

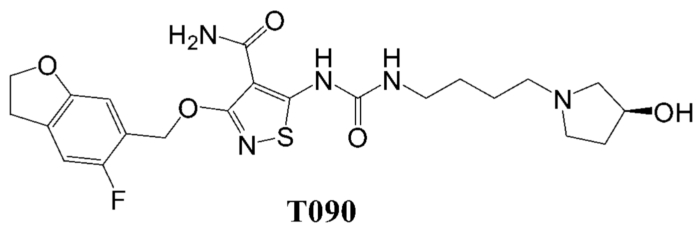

**T090**

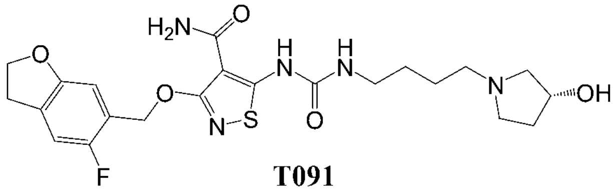

**T091**

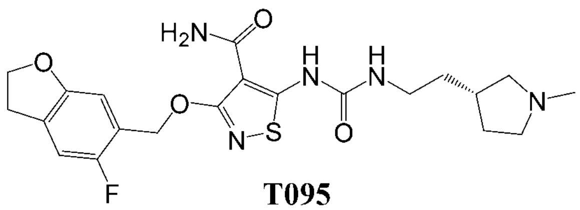

**T095**

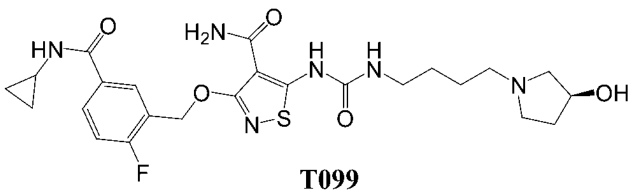

**T099**

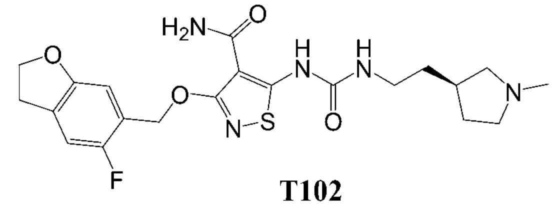

**T102**

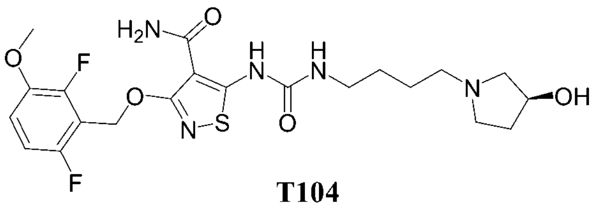

**T104**

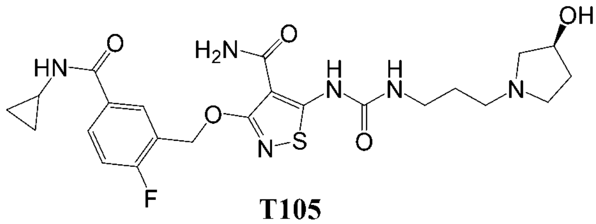

**T105**

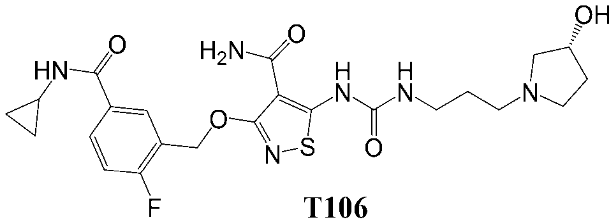

**T106**

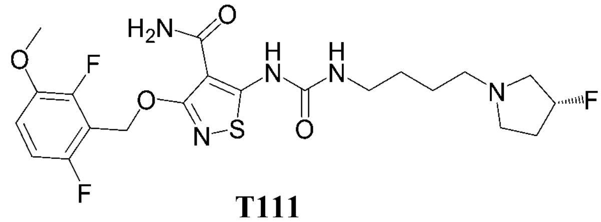

**T111**

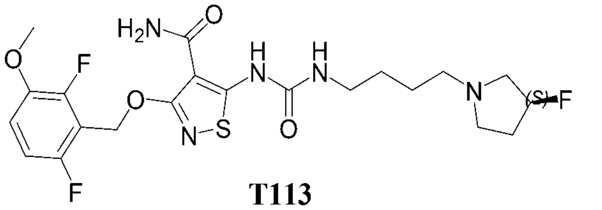

**T113**

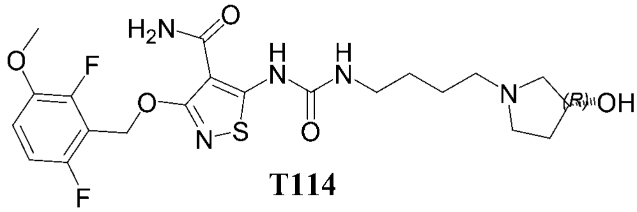

**T114**

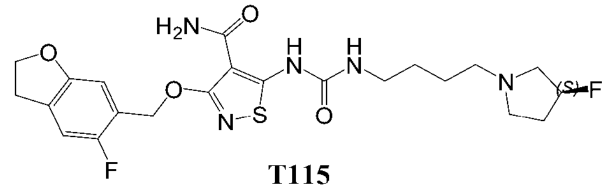

**T115**

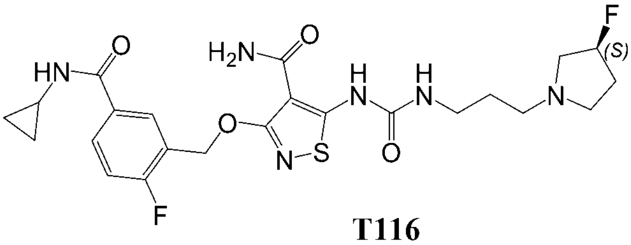

**T116**

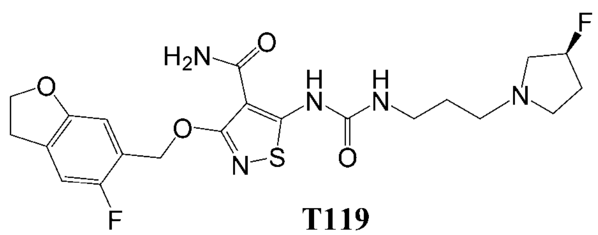

**T119**

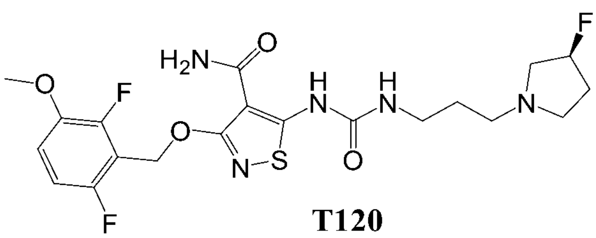

**T120**

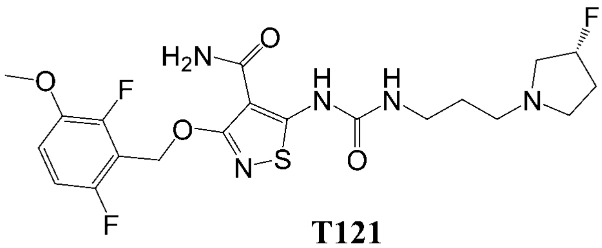

T121

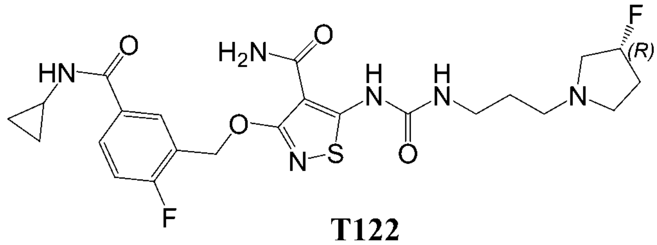

T122

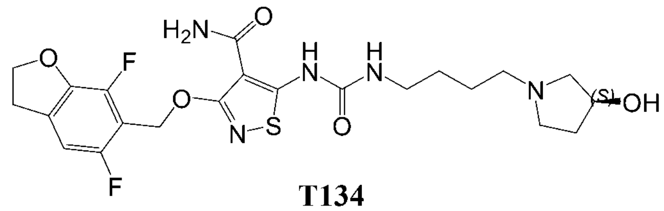

T134

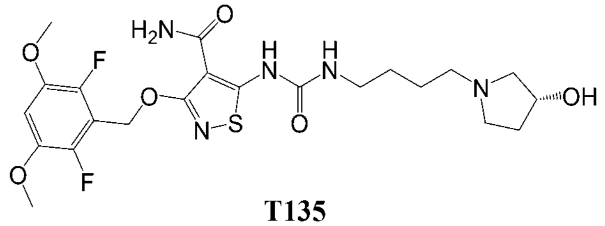

T135

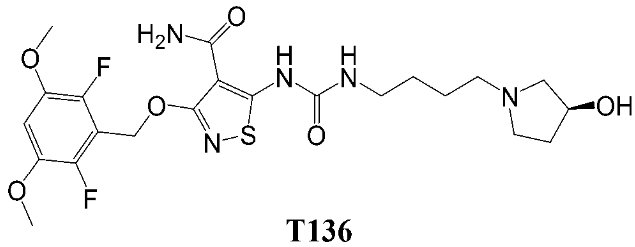

T136

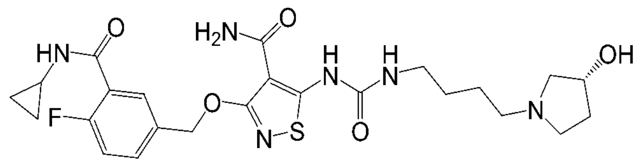

T150

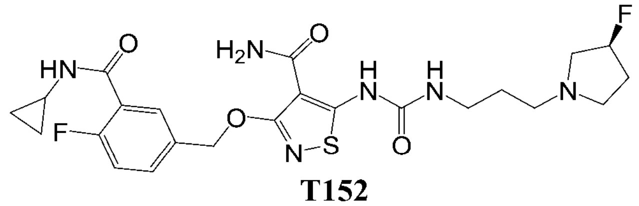

T152

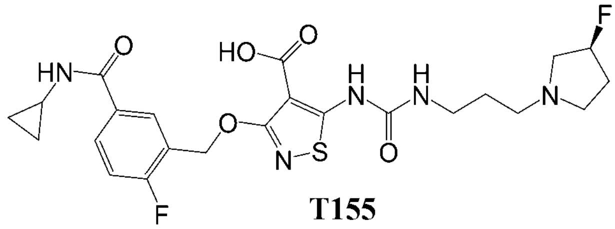

T155

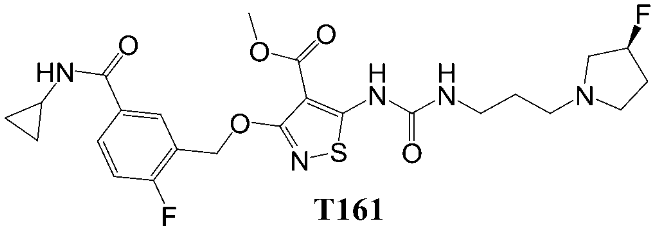

T161

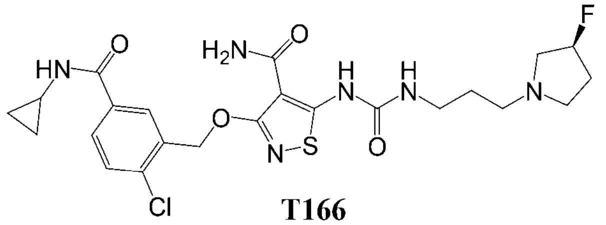

T166

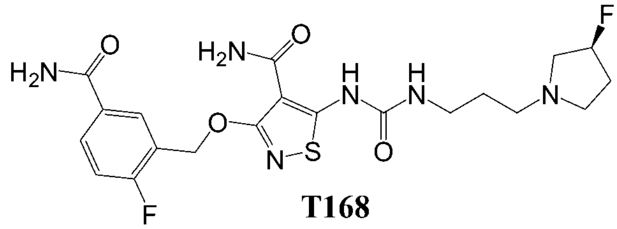

T168

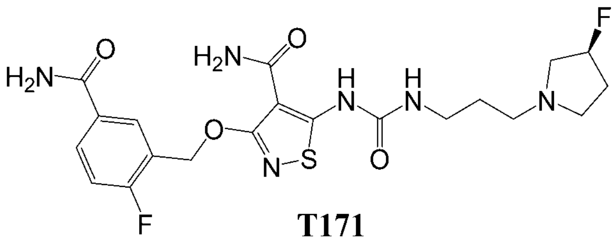

T171

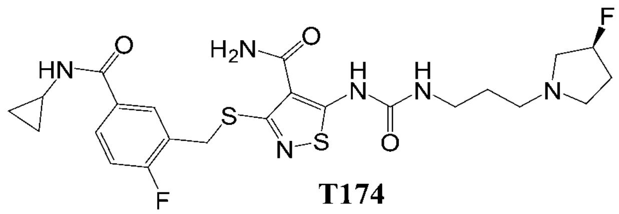

T174

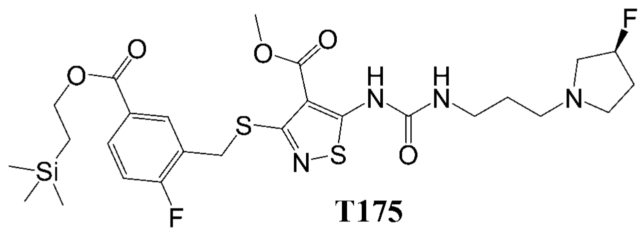

T175

.

In a third aspect of the present invention, an intermediate compound is provided for use in preparation of the compound with Y being -N($C_0$-$C_{10}$ alkyl)($C_0$-$C_{10}$ alkyl) according to the first aspect of the present invention (e.g., the compound of Formula II). The intermediate compound has a structure of:

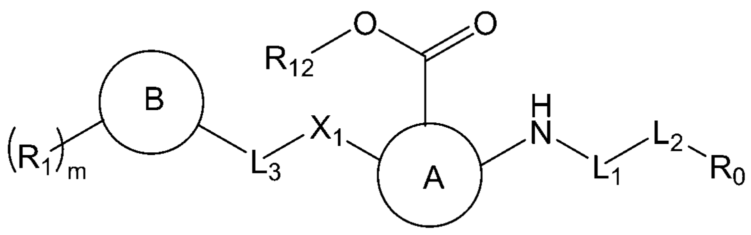

(IV)

wherein, $R_{12}$ is alkyl;

The A ring, Ring B, $X_1$, $L_1$, $L_2$, $L_3$, $R_1$, $R_0$, and m are defined as in the first aspect of the present invention.

Specifically, $R_{12}$ is $C_1$-$C_6$ alkyl, particularly $C_1$-$C_3$ alkyl, for example, methyl and ethyl.

In an embodiment of the present invention, the intermediate compound has a structure of:

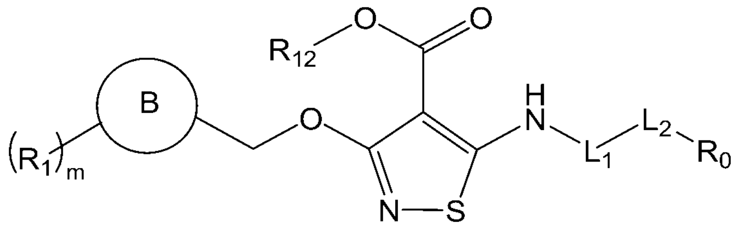

(V)

Specifically, the compound of Formula II can be obtained by one-step ammonolysis of the intermediate compound of Formula V (e.g., by reacting with ammonia in alcoholic solution or with ammonia water).

In a fourth aspect of the present invention, a pharmaceutical composition is provided. The pharmaceutical composition comprises the compound according to the first aspect or the pharmaceutically acceptable salt, stereoisomer, ester, prodrug, solvate, and deuterated compound thereof, and one or more pharmaceutically acceptable excipients.

Specifically, the pharmaceutically acceptable excipients may be selected from one or more of: disintegrants, binders, lubricants, suspending agents, stabilizers, fillers, absorption promoters, surfactants, flavoring agents, antioxidants, preservatives, etc.

Specifically, in this pharmaceutical composition, the compound according to the first aspect, or the pharmaceutically acceptable salt, stereoisomer, ester, prodrug, solvate, and deuterated compound thereof, may be used alone, or in combination with other types of active ingredients.

Specifically, the pharmaceutical composition may be delivered via any suitable route of administration, such as gastrointestinal administration (e.g., oral, sublingual, rectal) or non-gastrointestinal administration (e.g., intravenous, intramuscular, intranasal, intraocular, intracranial, vaginal, intraperitoneal, transdermal, subcutaneous, intradermal, or respiratory administration). In some embodiments of the present invention, the pharmaceutical composition is administered via intraocular routes (e.g., eye drops, ophthalmic ointments, subconjunctival injections, intraocular injections).

Specifically, the pharmaceutical composition can take any suitable dosage form, for example: gastrointestinal dosage forms, for example, including but not limited to, tablets, pills, powders, granules, capsules, troches, syrups, liquids, emulsions, suspensions, etc.; and non-gastrointestinal dosage forms, such as injectable dosage forms (e.g., for subcutaneous, intravenous, intramuscular, intraperitoneal injection), respiratory dosage forms (e.g., sprays, aerosols, and powder aerosols), dermal dosage forms (e.g., topical solutions, lotions, ointments, plasters, pastes, and patches), mucosal dosage forms (e.g., eye drops, ophthalmic ointments, nasal drops, gargles, and sublingual tablets), and cavity dosage forms (e.g., suppositories, aerosols, effervescent tablets, drops, pills) for the rectum, vagina, urethra, nose, ear canal, etc.

In some embodiments of the present invention, the above-mentioned pharmaceutical composition is an ophthalmic formulation, such as eye drops or ophthalmic ointments.

Specifically, various dosage forms of the pharmaceutical composition may be prepared according to the conventional production methods in the field of pharmacy. For example, an active ingredient is mixed with one or more pharmaceutically acceptable excipients to prepare a desired dosage form.

Specifically, in the pharmaceutical composition, the compound according to the first aspect, or the pharmaceutically acceptable salt, stereoisomer, ester, prodrug, solvate, and deuterated compound thereof may have a weight percentage of 0.1-99.5%, for example, 0.5%, 1%, 5%, 10%, 20%, 30%, 40%, 50%, 60%, 70%, 80%, 90%, 95%, or 99%, particularly 1-30%. In a fifth aspect of the present invention, use of the compound according to the first aspect or the pharmaceutically acceptable salt, stereoisomer, ester, prodrug, solvate, and deuterated compound thereof in preparation of drugs for inhibiting the activity of protein tyrosine kinases is provided.

Specifically, the protein tyrosine kinase is VEGFR, EGFR, or TIE2; and particularly, the compound inhibits VEGFR activity.

Specifically, the drugs are selective VEGFR inhibitors.

In a sixth aspect of the present invention, use of the compound according to the first aspect or the pharmaceutically acceptable salt, stereoisomer, ester, prodrug, solvate, and deuterated compound thereof in preparation of a medicament for preventing and/or treating a protein tyrosine kinase-medicated proliferative disease is provided.

In an embodiment of the present invention, the disease cancer (malignant tumor), including but not limited to breast cancer, lung cancer (especially non-small cell lung cancer), adenocarcinoma, colorectal cancer, renal cancer, liver cancer, pancreatic cancer, ovarian cancer, prostate cancer, glioma, glioblastoma, myeloma, agnogenic myeloid metaplasia, mesothelioma, myelodysplastic syndrome, and hematologic malignancy.

Specifically, hematologic malignancy includes: leukemia, lymphoma, and multiple myeloma (MM).

Specifically, the leukemia may include chronic lymphocytic leukemia (CLL), chronic myeloid leukemia (CML), acute lymphoblastic leukemia (ALL), acute myeloid leukemia (AML), and acute monocytic leukemia.

Specifically, the lymphoma may include Hodgkin lymphoma (HL) and non-Hodgkin lymphoma (NHL), for example, diffuse large cell lymphoma (DLCL) (e.g., diffuse large B-cell lymphoma), follicular lymphoma, chronic lymphocytic leukemia/small lymphocytic lymphoma (CLL/SLL), mantle cell lymphoma (MCL), marginal zone B-cell lymphoma, primary mediastinal B-cell lymphoma, Burkitt's lymphoma, lymphoplasmacytic lymphoma, hairy cell leukemia (HCL), immunoblastic large cell lymphoma, precursor B lymphoblastic lymphoma and primary central nervous system (CNS) lymphoma and T-cell non-Hodgkin lymphoma (NHL), such as precursor T lymphoblastic lymphoma/leukemia, peripheral T-cell lymphoma (PTCL), angioimmunoblastic T-cell lymphoma, extranodal natural killer (NK)/T-cell lymphoma, enteropathy-type T-cell lymphoma, subcutaneous panniculitis-like T-cell lymphoma, and anaplastic large cell lymphoma, NK/T-cell lymphoma, particularly diffuse large B-cell lymphoma (DLBCL). Specifically, in these uses, the treatment includes inducing tumor cell death, and inhibiting metastasis and suppressing micrometastatic growth.

In an embodiment of the present invention, the disease is an ocular disease, including but not limited to DR (including simple (background) DR, proliferative DR, and diabetic macular edema); AMD (including neovascular (wet/exudative) AMD, dry AMD, and geographic atrophy); pathological CNV originating from any pathological mechanism (i.e., high myopia, trauma, sickle cell anemia; ocular histoplasmosis, angioid streaks, traumatic choroidal rupture, optic nerve drusen, and certain retinal dystrophies); pathological retinal neovascularization originating from any pathological mechanism (i.e., sickle cell retinopathy, Eales disease (retinal periphlebitis), ocular ischemic syndrome, carotid cavernous sinus fistula, familial exudative vitreoretinopathy, hyperviscosity syndrome, idiopathic occlusive arteritis, birdshot retinochoroidopathy, retinal vasculitis, sarcoidosis, or toxoplasmosis); uveitis; retinal vein occlusion (central or branch); ocular trauma; postsurgical edema; postsurgical neovascularization; cystoid macular edema; ocular ischemia; retinopathy of prematurity; Coat's disease; sickle cell retinopathy and/or neovascular glaucoma.

In some embodiments of the present invention, the disease is DR, including simple (background) DR, proliferative DR, and diabetic macular edema.

In other embodiments of the present invention, the disease is AMD, including neovascular (wet/exudative) AMD, dry AMD, and geographic atrophy.

In a seventh aspect of the present invention, a method for inhibiting protein tyrosine kinase activity is provided. The method comprises the step of: administering the compound according to the first aspect, or the pharmaceutically acceptable salt, stereoisomer, ester, prodrug, solvate, and deuterated compound thereof, or the pharmaceutical composition according to the fourth aspect to a subject in need thereof.

Specifically, the protein tyrosine kinase may be VEGFR (e.g., VEGFR1, VEGFR2, or VEGFR3), EGFR, TIE2, FGFR (e.g., FGFR1, FGFR2, FGFR3, or FGFR4), or PDGFR (e.g., PDGFRA, or PDGFRB); particularly, the compound inhibits VEGFR activity, especially VEGFR2 activity.

Specifically, the method is the one selectively inhibiting VEGFR.

Specifically, the subject may be a mammal, particularly a human.

Specifically, the method is carried out in vivo or in vitro.

In an eighth aspect of the present invention, a method for preventing and/or treating protein tyrosine kinase-mediated proliferative diseases is provided. The method comprises the step of: administering the compound according to the first aspect, or the pharmaceutically acceptable salt, stereoisomer, ester, prodrug, solvate, and deuterated compound thereof, or the pharmaceutical composition according to the fourth aspect to a subject in need thereof.

Specifically, the diseases are as described as in the sixth aspect of the present invention. Specifically, the subject may be a mammal, particularly a human.

In a ninth aspect of the present invention, a method for inhibiting ocular angiogenesis and retinal vascular leakage is provided. The method comprises the step of: administering the compound according to the first aspect, or the pharmaceutically acceptable salt, stereoisomer, ester, prodrug, solvate, and deuterated compound thereof, or the pharmaceutical composition according to the fourth aspect to a subject in need thereof.

Specifically, the administration may be done through any suitable route of administration, particularly intraocular administration, for example, administration via eye drops, ophthalmic ointments, subconjunctival injection, and intraocular injection, especially eye drops.

Specifically, the subject may be a mammal, particularly a human.

The present invention provides a series of compounds capable of inhibiting anti-angiogenic tyrosine kinases. In addition to effective antagonism against the activities of VEGFR1, VEGFR2, and VEGFR3 tyrosine kinases, these compounds exhibit high selectivity for inhibiting the activities of EGFR tyrosine kinases, thereby effectively reducing or even avoiding the side effects, particularly ocular side effects (e.g., epithelial degeneration and defects, ulcers, corneal epithelial thinning, erosions and/or corneal edema, keratitis). These compounds possess significant applicational and research value.

## DETAILED DESCRIPTION OF THE INVENTION

Unless otherwise defined, all scientific and technical terms used in the present invention have the same

meanings as those generally understood by those of ordinary skill in the art which is involved in the present invention.

In the present invention, the term "aliphatic group" refers to a linear or branched hydrocarbon chain that is completely saturated or contains one or more unsaturated units, or a cyclic hydrocarbon group (also referred to herein as "aliphatic ring" or "cycloalkyl") that is completely saturated or contains one or more unsaturated units, which is linked to the remainder of the molecule through a single bond. Suitable aliphatic groups include, but are not limited to, linear or branched and substituted or unsubstituted alkyl, alkenyl, alkynyl, and their mixtures, for example, (cycloalkyl)alkyl, (cycloalkenyl)alkyl, (cycloalkyl)alkenyl, etc. A typical aliphatic group contains 1 to 10 (e.g., 1, 2, 3, 4, 5, 6, 7, 8, 9, or 10) carbon atoms, preferably 1 to 6 carbon atoms.

The term "carbon ring" consists entirely of carbon atoms, and is classified into an aliphatic ring or an aromatic ring.

The term "alkyl" refers to a linear or branched hydrocarbon chain radical that does not contain unsaturated bonds, and the hydrocarbon chain radical is attached to the rest of the molecule via a single bond. A typical alkyl group contains 1 to 10 (e.g., 1, 2, 3, 4, 5, 6, 7, 8, 9, or 10) carbon atoms, preferably 1 to 6 carbon atoms, such as methyl, ethyl, n-propyl, isopropyl, n-butyl, isobutyl, tert-butyl, n-pentyl, isopentyl, neopentyl, tert-pentyl, n-hexyl, isohexyl, etc. If alkyl is substituted by cycloalkyl, then "cycloalkyl alkyl" is derived correspondingly, such as cyclopropyl methyl, cyclopropyl ethyl, cyclobutyl methyl, cyclopentyl methyl, cyclohexyl methyl, etc. If alkyl is substituted by aryl, then "arylalkyl" is derived correspondingly, such as benzyl, diphenylmethyl, or phenylethyl. If alkyl is substituted by heterocyclyl, then "heterocyclylalkyl" is derived correspondingly. In the present invention, $C_0$ alkyl refers to H, that is, $C_{0-10}$ alkyl includes H and C1-10 alkyl.

The term "alkylene" refers to a hydrocarbon group (divalent alkyl group) derived from an alkane molecule by removing two hydrogen atoms, and it may be either linear or branched and is attached to the rest of the molecule via a single bond. A typical alkylene group herein contains 1 to 10 (e.g., 1, 2, 3, 4, 5, 6, 7, 8, 9, or 10) carbon atoms, preferably 1 to 6 carbon atoms, such as methylene (-CH2-), ethylene, propylene, butylene, etc. In the present invention, $C_0$ alkylene refers to a single bond, that is, $C_{0-10}$ alkylene includes a single bond and $C_{1-10}$ alkylene.

The term "cycloalkyl" refers to an aliphatic cyclic hydrocarbon, including for example 1 to 4 monocyclic and/or fused rings containing 3-18 carbon atoms, preferably 3-10 (e.g., 3, 4, 5, 6, 7, 8, 9, or 10) carbon atoms, such as cyclopropyl, cyclobutyl, cyclopentyl, cyclohexyl, or adamantyl. The term "alkoxy" refers to the substituent derived from a hydroxyl group by substituting the hydrogen with an alkyl group, for example, an alkoxy group containing 1-10 carbon atoms, such as methoxy, ethoxy, propoxy, butoxy, etc.

The term "alkylamino" refers to the substituent derived from an amino group (-NH2) by substituting one or two hydrogens with an alkyl group(s), for example, an alkylamine group containing 1-10 carbon atoms, such as

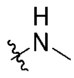, 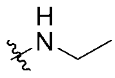, 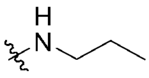, 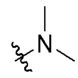, 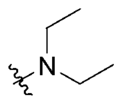,

and

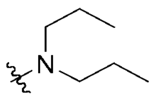.

The term "halogen" means fluorine, chlorine, bromine or iodine.

The term "haloalkyl" refers to the group derived from an alkyl group by substituting one or more hydrogens with a halogen atom(s) (e.g., fluorine, chlorine, bromine, or iodine), such as $-CHF_2$, $-CH_2F$, $-CF_3$, $-CH_2-CH_2F$, $-CH_2-CHF_2$, $-CH_2-CF_3$, $-CH_2CH_2-CF_3$, and $-CH_2CH_2CH_2-CF_3$.

The term "aryl" refers to a monocyclic or polycyclic radical, including a polycyclic radical containing a single aryl group and/or a fused aryl group, for example, containing 1-3 monocyclic or fused rings and 6-18 (e.g., 6, 8, 10, 12, 14, 16, or 18) carboatomic ring atoms. In the present invention, C6-C12 aryl refers to an aryl group containing 6-12 carboatomic ring atoms, such as phenyl, naphthyl, biphenyl, indenyl, etc.

The term "heterocyclyl" refers to a 3-18 membered cyclyl group containing 2 to 17 carbon atoms and 1 to 10 heteroatoms selected from N, O, or S. The heterocyclyl group may be a monocyclic, bicyclic, tricyclic, or tetracyclic system, or an additional polycyclic system, which may include a fused (two ring atoms shared by two rings), spiro (one ring atom shared by two rings), or bridged (more than three ring atoms shared by two rings) ring system (excluding the case of linked rings). The heterocyclyl group can be partially saturated (heteroaryl) or fully saturated (heterocycloalkyl). In the compound of the present invention, suitable heteroaryl contains 1, 2 or 3 types of heteroatoms selected from N, O or S atoms, and includes, for example, coumarin (including 8-coumarin), quinolyl (including 8-quinolyl), isoquinolyl, pyridinyl, pyrazinyl, pyrazolyl, pyrimidinyl, furanyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl,

triazinyl, cinnolinyl, benzimidazolyl, benzofuranyl, benzofurazanyl, thienyl, benzothiazolyl, benzoxazolyl, quinazolinyl, quinoxalinyl, naphthyridinyl and furopyridinyl. In the compound of the present invention, suitable heterocycloalkyl contains 1, 2 or 3 types of heteroatoms selected from N, O or S atoms, and includes, for example, pyrrolidinyl, tetrahydrofuranyl, dihydrofuran, tetrahydrothienyl, tetrahydrothiopyranyl, piperidinyl, morpholinyl, thiomorpholinyl, oxathiacyclohexanyl, piperazinyl, azacyclobutanyl, oxacyclobutanyl, thiacyclobutanyl, homopiperidinyl, oxacyclopropanyl, thiacyclopropanyl, azepinyl, oxazacycloheptyl, diazepinyl, triazepinyl, 1,2,3,6-tetrahydropyridyl, 2-pyrrolinyl, 3-pyrrolinyl, dihydroindolyl, 2H-pyranyl, 4H-pyranyl, dioxacyclohexanyl, 1,3-dioxolanyl, pyrazolinyl, dithanyl, dithiolanyl, dihydropyranyl, dihydrothienyl, pyrazolidinyl, imidazolinyl, imidazolidinyl, 3-azabicyclo[3.1.0]hexyl,3-azabicyclo[4.1.0]heptyl, 3H-indolyl, and quinazinyl.

The term "optionally substituted" group may be halogen, -CN, $-NO_2$, -OR’, -NR’R", -S(O)t-R’, - S(O)t-NR’R", -COR’, -C(O)OR’, -C(O)NR’R", -C(O)N(R’)OR", -OC(O)R’, -OC(O)NR’R", - NR’C(O)R", -N(R’)C(O)NR’R’, -N(R’)C(NR’)NR’R’, -NR’-S(O)t-R’, -NR’-S(O)t-NR’R’, - N=S(O)R’R", -S(NR’)(O)R", -N(R’)CN, -P(O)(R’)NR’R", -P(O)(R’)OR"or -P(O)R’R", $C_{1-6}$ alkyl, $C_{1-6}$ haloalkyl, $C_{1-6}$ alkenyl, $C_{1-6}$ alkynyl, $C_{3-6}$ cycloalkyl, $C_{4-10}$ cycloalkyl alkyl, $C_{6-10}$ aryl, $C_{6-10}$ arylalkyl, $C_{3-8}$ heterocyclyl, or $C_{3-8}$ heterocyclyl alkyl; t is 0, 1 or 2; R’ and R" are each independently selected from: H, halogen, -CN, $-NO_2$, alkyl, haloalkyl, cycloalkyl, cycloalkyl alkyl, aryl, arylalkyl, heterocyclyl, heterocyclylalkyl; or, R’ and R", which are connected to the same nitrogen, form a heterocyclic ring together with this nitrogen atom.

The term "pharmaceutically acceptable salt" includes acid addition salts and alkali addition salts. The term "acid addition salts" includes, but is not limited to, salts derived from inorganic acids (such as hydrochloric acid, nitric acid, phosphoric acid, sulfuric acid, hydrobromic acid, hydroiodic acid, and phosphonic acid), as well as salts derived from organic acids (such as aliphatic monocarboxylic and dicarboxylic acids, phenyl-substituted alkanic acid, hydroxyalkanoic acid, dialkanic acid, aromatic acid, and aliphatic and aromatic sulfonic acid), for example, acetate, salicylate, decanoate, stearate, oleate, caproate, malate, glycolate, ethanesulfonate, isethionate, etc. Therefore, these salts include, but are not limited to, sulfate, pyrosulfate, bisulfate, sulfite, bisulfite, nitrate, phosphate, monohydrogen phosphate, dihydrogen phosphate, metaphosphate, pyrophosphate, hydrochloride, hydrobromide, iodate, acetate, propionate, caprylate, isobutyrate, oxalate, malonate, succinate, suberate, sebacate, fumarate, maleate, amygdalate, benzoate, chlorobenzoate, methylbenzoate, dinitrobenzoate, phthalate, benzenesulfonate, tosylate, phenylacetate, citrate, lactate, tartrate and methanesulfonate, and further include salts of amino acids, such as arginine, gluconate, galacturonate, aspartate, glutamate, etc. The acid addition salts can be prepared by bringing free alkali into contact with a sufficient amount of the desired acid in a conventional way. The free alkali can be regenerated by bringing a salt into contact with an alkali, and this free alkali can be isolated in a conventional way.

The term "alkali addition salts" refer to salts formed with metals or amines (such as hydroxides of alkali metals and alkaline earth metals), or with organic amines. Examples of metals used as cations include, but are not limited to, sodium, potassium, magnesium, and calcium. Examples of suitable amines include, but are not limited to, N,N’-dibenzylethylenediamine, chloroprocaine, choline, diethanolamine, ethylenediamine(ethane-1,2-diamine), N-methylglucosamine, and procaine. The alkali addition salts can be prepared by bringing free acid into contact with a sufficient amount of the desired alkali in a conventional way. The free acid can be regenerated by bringing a salt into contact with an acid, and the free acid can be isolated in a conventional way. The term "stereoisomer" includes forms such as enantiomers, diastereomers, and geometric isomers. Some compounds of the present invention have a cyclic hydrocarbon group, which can be substituted on more than one carbon atom, in which case all their geometric forms (including cis and trans forms), and their mixtures, shall fall within the scope of the present invention.

The term "solvate" refers to the physical binding of the compound of the present invention to one or more solvent molecules. This physical bonding includes ionic and covalent bonding to various degrees, including hydrogen bonding. In some cases, the solvate can be isolated, for example, where one or more solvent molecules are incorporated into the crystal lattice of a crystalline solid. The solvate includes solution phases and isolatable solvates. Representative solvates include alcoholate, methylate, etc.

The term "deuterated compound" refers to a compound in which one or more hydrogen atoms (e.g., 1, 2, 3, 4, or 5 hydrogen atoms) are substituted by deuterium atoms (D).

It should be recognized that, depending on the source of the chemical material used in synthesis, the abundance of natural isotopes varies in the synthesized compounds. Therefore, the compound of the present invention will inherently contain a small amount of deuterated isotopologues. Despite this variation, the concentrations of stable hydrogen and carbon isotopes of this natural abundance are still low and insignificant compared to the degree of stable isotope substitution in the compound of the present invention. See, for example, Wada, E et al., Seikagaku, 1994, 66: 15; Gannes, LZ et al., Comp Biochem Physiol Mol Integr Physiol, 1998, 119: 725.

In the compound of the present invention, any atom that is not specified as deuterium exists in its natural isotope abundance. Unless otherwise indicated, when a position is specifically specified as "H" or "hydrogen", this position shall be understood to have hydrogen in accordance with its natural abundance isotope composition. Similarly, unless otherwise indicated, when a position is specifically specified as "D" or "Deuterium", this position shall be understood to have deuterium with the abundance of at least 3000 times greater than the natural abundance of deuterium (which is 0.015%) (i.e., at least 45% deuterium incorporated).

The term "isotope enrichment coefficient" as used herein refers to the ratio of the isotopic abundance of a particular isotope to its natural abundance.

In other embodiments, the isotope enrichment coefficient of the compound of the present invention for each specified deuterium atom is at least 3500 (52.5% deuterium incorporated at each specified deuterium atom), at least 4000 (60% deuterium incorporated), at least 4500 (67.5% deuterium incorporated), at least 5000 (75% deuterium incorporated), at least 5500 (82.5% deuterium incorporated), at least 6000 (90% deuterium incorporated), at least 6333.3 (95% deuterium incorporated), at least 6466.7 (97% deuterium incorporated), at least 6600 (99% deuterium incorporated) or at least 6633.3 (99.5% deuterium incorporated).

The term "isotopologue" refers to a substance with a chemical structure differing from the specific compound of the present invention only in isotopic composition.

The term "prodrug" refers to the compound forms of formula I, including acetal, ester and zwitterionic forms, which are suitable for administration to patients without excessive toxicity, irritation, or allergic reactions while remaining effective for its intended purpose. The prodrug undergoes in vivo conversion, for example, by hydrolysis in the blood, to obtain a parent compound.

The terms "patient" or "subject" are used interchangeably herein and refer to any animals or their cells treated by the methods described, whether in vitro or in situ. Specifically, such animals include mammals, such as rats, mice, guinea pigs, rabbits, dogs, monkeys, and humans, particularly humans.

The term "treatment" refers to the prevention, curing, reversal, attenuation, alleviation, minimization, inhibition, suppressing and/or halting of one or more clinical symptoms of a disease after its onset.

The term "prevention" refers to therapeutic intervention prior to disease onset to avoid, minimize, or impede disease occurrence or progression.

The term "tumor" refers to an abnormal tissue mass whose growth exceeds and is uncoordinated with that of normal tissues. Tumors may be "benign" or "malignant", depending on the following characteristics: degree of cell differentiation (in terms of morphology and function), growth rate, local invasion, and metastasis. "Benign tumors" are usually well differentiated, characterized by slower growth than malignant tumors and confinement to their site of origin. In addition, benign tumors lack the ability to infiltrate, invade, or metastasize to distant sites.. In some cases, some "benign" tumors may later lead to malignant tumors, which may be due to additional genetic alterations in the neoplastic cell subsets of the tumors. These tumors are known as "precancerous tumors." Malignant tumors are typically poorly differentiated (anaplastic) and exhibit characteristic rapid growth, accompanied by progressive infiltration, invasion, and destruction of surrounding tissues. In addition, malignant tumors are often capable of metastasizing to distant sites.

The term "cancer" refers to a malignant tumor (Stedman's Medical Dictionary, 25th ed.; Hensyl ed.; Williams & Wilkins: Philadelphia, 1990).

The term "protein tyrosine kinase inhibitor" refers to a molecule that reduces, inhibits, or otherwise decreases one or more of the biological activities of protein tyrosine kinases. Inhibition by a protein tyrosine kinase inhibitor does not necessarily indicate complete elimination of the activities of the protein tyrosine kinases. In contrast with controls, the activities of the protein tyrosine kinases can be significantly reduced by a significant amount, for example, by at least about 5%, 10%, 15%, 20%, 25%, 30%, 35%, 40%, 45%, 50%, 55%, 60%, 65%, 70%, 75%, 80%, 85%, 90%, 95%, or 100%.

Unless otherwise indicated, the range of values expressed in the form "from x to y" or "x-y" shall be understood to include x and y. When several preferred ranges are described in the form of "from x to y" or "x-y" for a specific feature, it should be understood that all ranges combining different endpoints can also be considered.

The disclosures of all the publications, patents and published patent specifications cited herein are incorporated herein by reference in their entireties.

Vascular endothelial growth factor (VEGF) and its receptors (VEGFRs) are known as the most potent vascular permeabilizers and endothelial cell-specific mitogens, playing critical roles in the proliferation, migration, and angiogenesis of endothelial cells. Angiogenesis is an important mechanism in many physiological and pathological processes and is involved in the proliferation, migration, and survival of endothelial cells, which in turn leads to further blood capillary formation and ultimately promotes vascularization. VEGF and its receptors (VEGFRs) play significant roles in pathological angiogenesis associated with conditions such as tumor development and ocular neovascular diseases. For example, the expression level of VEGF is significantly positively correlated with the degree of vascularization in tumor tissues. VEGF acts on the VEGFR receptors to activate the phosphorylation of the VEGFR receptor tyrosine kinase and lead to the signal transduction of abnormal cells, thereby promoting the proliferation of endothelial cells and neovascularization. VEGF is a key participant in many different cancers and ocular diseases associated with pathological neovascularization. However, despite the efforts to design VEGFR receptor-specific molecules, it is inevitable to have some cross-reactivity with other "off-target" receptors, for example, the inhibition of activities of TIE2 receptors (i.e., TEK tyrosine kinases), or EGFR receptors. It has been clinically observed that the receptor tyrosine kinase inhibitors inhibit both EGFR and EGFR, which affects EGFR-mediated corneal epithelial wound healing, leading to adverse side effects on the eyes. In addition, TIE2 is crucial in maintaining vascular integrity. The inhibition of TIE2 leads to weakened endothelial cell junctions, which expedites fluid leakage and eye edema and impairs blood flow and oxygen delivery in the retina, possibly resulting in vision

loss. The compound of the present invention has significantly improved selectivity for the inhibition of TIE2 and EGFR. It not only increases the antagonism against the tyrosine kinase activities of all VEGFR receptors (VEGFR1, VEGFR2, and VEGFR3), but also significantly improves the selectivity for the inhibition of activities of TIE2 and EGFR receptor (as illustrated in the test example).

VEGFR2 is the primary receptor for VEGF-induced endothelial cell signaling. During development and/or after tissue damage, the ligand VEGF binds to the receptor, leading to the autophosphorylation and activation of VEGFR2, which induces angiogenesis and bypasses blocked vessels. The clinical treatment of vascular endothelial growth factor VEGF-A/VEGFR2 signaling pathway has been demonstrated be an effective way to treat ocular neovascular diseases such as wet AMD. The compound of the present application has a significant inhibitory effect against VEGF-induced VEGFR2 autophosphorylation (pVEGFR2) in human endothelial cells to block the signal transduction of abnormal cells, thereby inhibiting neovascularization (as illustrated in the test example). VEGFR receptor signal transduction mainly functions to promote the proliferation of endothelial cells and neovascularization. The compound of the present application shows the ability to inhibit the VEGF-induced proliferation of human endothelial cells at a nanomolar concentration level (as illustrated in the test example). In summary, the compound of the present application is a novel tyrosine kinase inhibitor. In addition to the application to the treatment of neovascular AMD and DR, this novel tyrosine kinase inhibitor can also be used in therapeutic indications for tumors to block neovascularization in tumors and interrupt the blood and nutrient supply necessary for tumor growth, resulting in the death of tumor cells.

The U.S. Food and Drug Administration (FDA) approved aflibercept (VEGF Trap-Eye) for the treatment of neovascular AMD and DR. However, aflibercept is a 115 kDa fully human recombinant protein and needs to be administered by intravitreal injection. Frequent intravitreal injections are less convenient for both clinicians and patients and carry rare but serious injection-related risks (retinal detachment, endophthalmitis, intraocular inflammation, cataracts, etc.), leading to poor efficacy in many patients who are not administered on time. The compound of the present invention (small molecule VEGFR tyrosine kinase inhibitor) represents another method to directly target VEGF, as an alternative to VEGF antibody biologics. The good clinical results of VEGF antibody biologics have validated the effect of the VEGF pathway in neovascular AMD and DR. Small molecule targeted VEGFR tyrosine kinase inhibitors have many advantages over monoclonal antibodies. With the ability to inhibit all members of the VEGFR family, these inhibitors can effectively inhibit VEGF signaling. They can be formulated as eye drops, thus avoiding intravitreal injections. Small molecule eye drops are capable of crossing cell membranes and directly interacting with the cytoplasmic domains of RTK. Moreover, small-molecule eye drops are economically inexpensive as compared with monoclonal antibodies. It is challenging to develop small molecule tyrosine kinase inhibitors for clinical use in AMD and DR. One of the most critical challenges is to overcome the risk of "targeted" toxicity. In a healthy vascular system, inhibiting VEGFR may potentially lead to serious adverse events, such as hypertension, haemorrhage, and thrombus. Despite a number of clinical successes in oncological indications, the safety profile of oral VEGFR-2 inhibitors may be the main reason for its limited clinical use and/or for the limitation in development of its clinical use for patients with AMD and DR. Therefore, compared with oral VEGFR-2 inhibitors, topical eye drops can offer an effective therapy that limits systemic exposure and avoids problems of targeted toxicity.

While topical ocular administration has proven to be a successful strategy for treating diseases related to the anterior segment of the eye (such as glaucoma), there are currently no FDA-approved topical therapies for ocular diseases associated with posterior ocular tissues (such as retina and choroid), including neovascular AMD and DR. This is largely due to the anatomical and physiological barriers that the human eye has evolved to protect itself from exogenous substances. The tear film is one of the first barriers to overcome. Compounds in the anterior segment of the eye can be quickly washed away by the tear film, leading to nasolacrimal duct drainage, which means that compounds need to be rapidly absorbed after local instillation. However, absorption/permeation into ocular tissues can also be challenging. One pathway for absorption involves the permeability of the cornea, which consists of an epithelium with tight junctions and alternating lipophilic and hydrophilic layers. The other pathway for absorption is penetration into the conjunctiva and subsequent diffusion into the sclera. The sclera is relatively more permeable compared to other ocular tissues; however, drugs entering the conjunctiva tend to be "lost" to the systemic circulation due to the highly vascularized nature of this tissue. Compounds exposed in the sclera may potentially diffuse into the choroid, which is the primary target tissue for neovascular AMD. The diffusion from the choroid to the retina (the target tissue for neovascular AMD) is further diminished by the blood-retinal barrier (BRB). The BRB functions similarly to the blood-brain barrier and may pose a significant obstacle to compound diffusion. Due to these anatomical and physiological barriers, it is estimated that only less than 5% of the locally administered dose reaches the posterior ocular tissues. Despite these challenges associated with local administration, this present invention focuses on developing structure-activity relationships (SAR) related to ocular and blood exposure. Through the compound-containing eye drop formulation, effective delivery to the posterior tissues (choroid and retina) of the eye can be achieved (as illustrated in the test example). It has observed that the compound degrades rapidly in the blood plasma, indicating that, in one aspect, the exposure level of the compound of the present invention in the posterior tissues of the eye is distributed through the instillation site, rather than from the systemic blood. In another aspect, the low exposure of these compounds in the blood plasma or in the whole body is beneficial for avoiding

systemic targeted toxicity. Moreover, it has observed a considerable amount of exposure to the scleral tissues, indicating that these compounds are effectively delivered to the posterior tissues of the eye (choroid and retina) mainly through the sclera. The compound also shows a certain amount of exposure in the posterior tissues of the eye (choroid and retina) 8 hours after administration. The present invention provides a novel compound that achieves sufficient drug concentration in the posterior segment of the eye (such as the choroid and retina) to bind to relevant receptors in the target eye, thereby increasing the bioavailability in the posterior segment and addressing the issues encountered in the ocular delivery of existing topical therapeutic agents.

One of the goals in medicinal chemistry is to enhance the bioavailability and stability of compounds to improve their efficacy. Bioavailability refers to the rate and extent to which a therapeutic agent is absorbed from its dosage form and becomes available at the site of action. Existing tyrosine kinase (e.g., VEGFR1, VEGFR2, and VEGFR3) inhibitors have issues like low solubility and/or low kinase inhibitory activity, which significantly affect their bioavailability and thus reduce their efficacy. The present invention provides a compound with the advantage of increased solubility and/or significant kinase inhibition activity (as illustrated in the test example). In addition, the test results further provide the percentage of the free drug of the compound of the present invention (which does not bind to the melanin and is able to interact with receptors in the eye tissues) (as illustrated in the test example). Melanin-containing cells in the eye are located in the retinal pigment epithelium and choroid of the posterior segment, and the ciliary body and iris of the anterior segment. The binding of the compound to melanin may affect ocular pharmacokinetics after topical administration. The present invention provides a compound-containing eye drop formulation aimed at treating AMD and DR. Both AMD and DR are diseases of the posterior segment of the eye, and the compound-containing eye drop formulation targets effective delivery to the tissues at the posterior segment of the eye. In such cases, the compound may bind to melanin tissues in the posterior segment (retinal pigment epithelium, choroid) or anterior segment (ciliary body, iris) of the eye. Many clinical drugs binds to melanin, thereby affecting their ocular pharmacokinetics. The binding rate between the compound and melanin is an important factor in ocular pharmacokinetics and pharmacodynamics, and must be considered during drug discovery and development.

Because of the limited permeability of many ocular drops to the corneal and conjunctival barriers, a major limitation of ocular drops may be the need for high concentrations of compounds in ocular formulations in order to achieve therapeutically effective doses in the posterior ocular tissues. Depending on the compound (the molecule itself or its high concentration), ocular formulations may have side effects on anterior ocular tissues (including the conjunctiva, cornea, and/or lens), leading to various ocular surface injuries, such as corneal epithelial defects and erosions. In particular, it has been clinically observed that treatment with EGFR antibody drugs may cause ocular side effects, such as epithelial degeneration and defects, ulcers, corneal epithelial thinning, erosion, and/or corneal edema and keratitis. EGFR is a major factor in the wound healing of human corneal epithelial cells. Therefore, it is essential to select compounds for topical ocular formulations that avoid inhibiting EGFR activity. The compound of the present invention exhibits high selectivity for inhibiting EGFR tyrosine kinase activity in addition to its effective antagonism against the activities of VEGFR1, VEGFR2, and VEGFR3 tyrosine kinases (as illustrated in the test example). The inventors used male black-banded Dutch rabbits to evaluate the maximum tolerated dose (MTD) of the compound administered as eye drops, in order to help select the dose with the least toxicity and the highest possible efficacy to be used in in vivo animal efficacy experiments. The results showed that the MTD:PAN90806 was 100 μg/eye on the third day after eye drop administration; and T093 was 250 μg/eye or higher. The inventors proceeded to administer eye drops to rabbits for six consecutive days, to evaluate the risk of ocular toxicity of T093 and PAN90806. The results showed that no abnormalities were observed in the eyes of all rabbits in the T093 (250 μg/eye) group, while the MTD of the positive compound PAN90806 was reduced to 50 μg/eye. T078 and T116 were administered as eye drops at a dose of 1000 μg/eye for three consecutive days, and the results of toxicity risk assessment showed that no abnormalities were observed in the eyes of all rabbits. Therefore, the MTDs of T078 and T116 on the fourth day of eye drop administration were 1000 μg/eye or higher.

The present invention provides a compound of Formula I, in particular a compound of Formula II or III, and medical uses thereof.

In an embodiment (1) of the present invention, in Formula I, in particular Forumula II or III, $L_1$ is -C(O)$NR_3$- or a single bond, and the

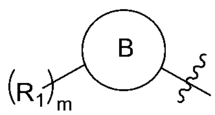

moiety is

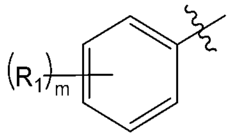,

wherein, $R_1$ comprises at least one of the following groups: $C_1$-$C_6$ alkyl, $C_1$-$C_6$ haloalkyl, cyano, nitro, azido, $-OR_{101}$, $-C(O)R_{101}$, $-C(O)OR_{101}$, $-NHC(O)OR_{101}$, $-OC(O)R_{101}$, $-NHSO_2R_{101}$, $-SO_2NR_{101}R_{102}$, $-NHC(O)R_{101}$, $-C(O)NR_{101}R_{102}$, $-NR_{101}R_{102}$, $-SR_{101}$, $-S(O)_2R_{101}$, $-SO_3H$, $-(CH_2)_t$(phenyl), $-(CH_2)_t$(4-10 membered heterocyclyl), and $-(CH_2)_t$($C_3$-$C_{10}$ cycloalkyl), where t is an integer form 0 to 5, and the E ring and $R_2$ being correspondingly as defined above in the present invention.

Specifically, $R_{101}$ and $R_{102}$ may be each independently selected from: H, $C_1$-$C_6$ alkyl, $C_1$-$C_6$ haloalkyl, $C_1$-$C_6$ hydroxyl-substituted alkyl, $C_1$-$C_6$ alkoxy-substituted alkyl, $C_1$-$C_6$ amino-substituted alkyl, $C_1$-$C_6$ alkylamino-substituted alkyl, $C_3$-$C_{10}$ cycloalkyl, $C_4$-$C_{10}$ cycloalkyl alkyl, substituted or unsubstituted 4-10 membered heterocyclyl,

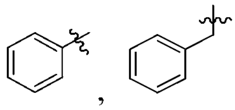, ,

$C_1$-$C_{10}$ silyl-substituted alkyl, and $C_1$-$C_{10}$ silyl.

More specifically, $R_{101}$ and $R_{102}$ may be each independently selected from: H, methyl, ethyl, n-propyl, isopropyl, $-CF_3$, $-CHF_2$, $-CH_2F$,

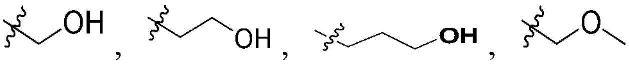, , , ,

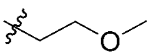, 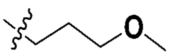, 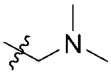, 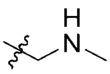, 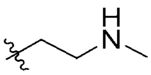, 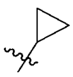, 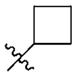, 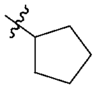,

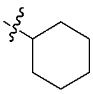, 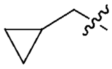, 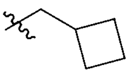, 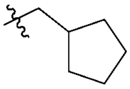, 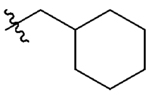, 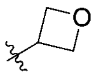, 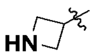, 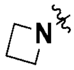, 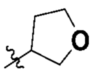,

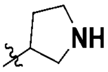, 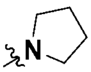, 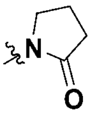, 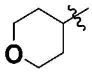, 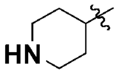, 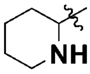, 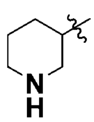, 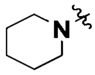, 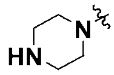,

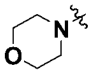, 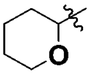, 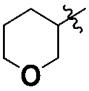 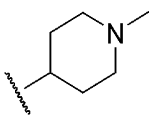, 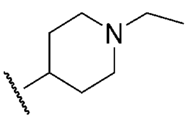, 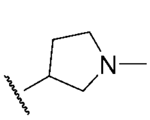, 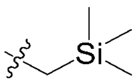,

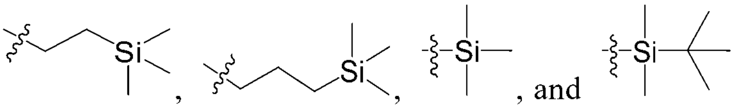, , , and .

In some embodiments of the present invention, $R_{101}$ and $R_{102}$ are each independently selected from: H, methyl, ethyl, n-propyl, isopropyl, $-CF_3$, $-CHF_2$, $-CH_2F$,

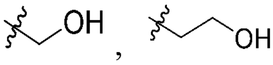, ,

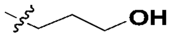, 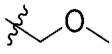, 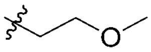, 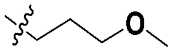, 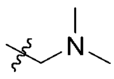, 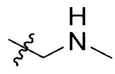, 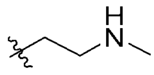,

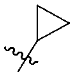, 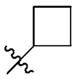, 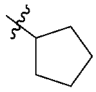, 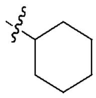, 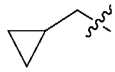, 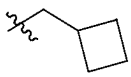, 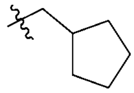, 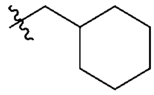, 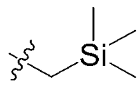,

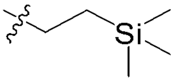, 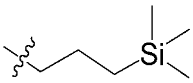, and 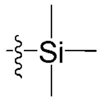, 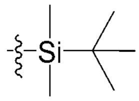.

In some embodiments of the present invention, $R_{102}$ is H.

In some embodiments of the present invention, $R_{101}$ is selected from: methyl, ethyl, n-propyl, isopropyl, $-CF_3$, $-CHF_2$, $-CH_2F$,

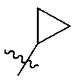, 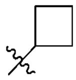, 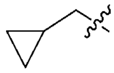, 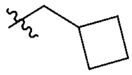, 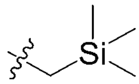,

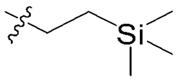, and 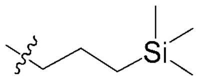.

More specifically, $R_1$ comprises at least one of the following groups: $C_1$-$C_6$ haloalkyl, cyano, nitro, azido, $-OR_{101}$, $-C(O)R_{101}$, $-NHC(O)R_{101}$, $-C(O)NHR_{101}$, $-NHR_{101}$, $-SR_{101}$, $-(CH_2)_t$(4-8 membered heterocyclyl), and $-(CH_2)_t(C_3$-$C_6$ cycloalkyl), where t is an integer from 0 to 5.

More specifically, $R_1$ may further comprise a group selected from: $C_1$-$C_6$ alkyl (e.g., methyl, ethyl, n-propyl, and isopropyl), and halogen (e.g., F, Cl, Br, and I). In some embodiments of the present invention, $L_1$ is $-C(O)NR_3$-or a single bond, the

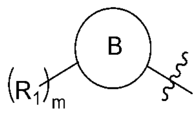

moiety is

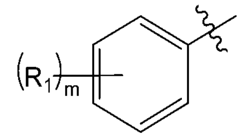,

wherein, $R_1$ comprises at least one of the following groups: $-CF_3$, $-CHF_2$, $-CH_2F$,

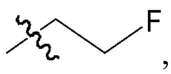, 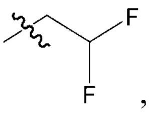, 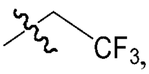,

cyano, nitro, azido, -OH,

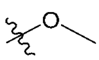, 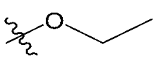,

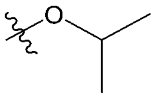, 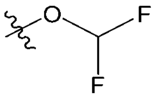, 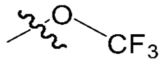, 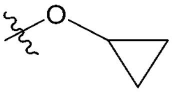, 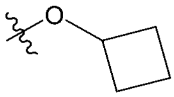, 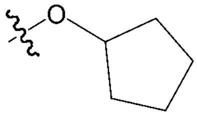,

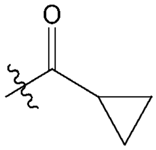, 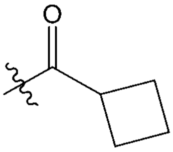, 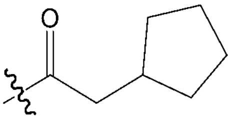, 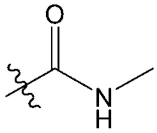, 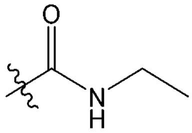, 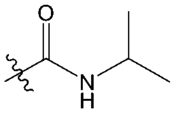,

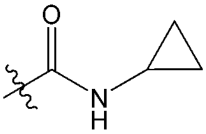, 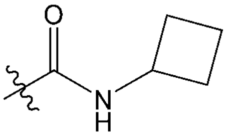, 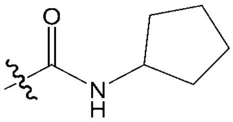, 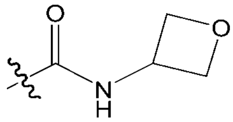, 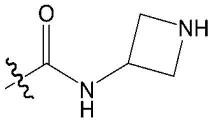,

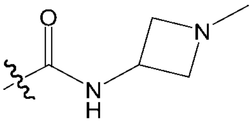, 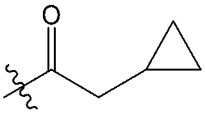, 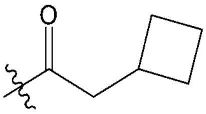, 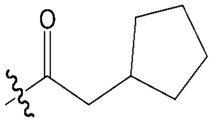, 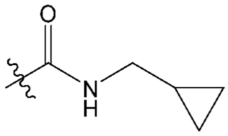,

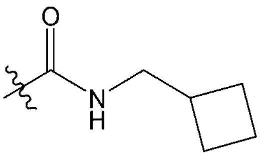, 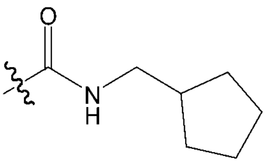, 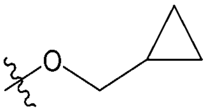, 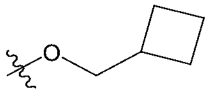,

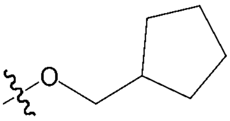, 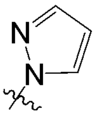, 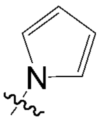, 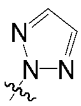, 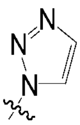, 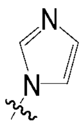, 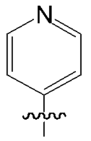, 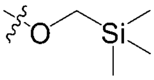,

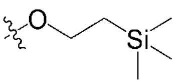, 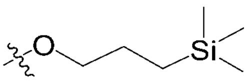, 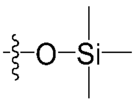, and 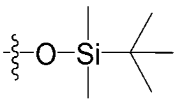;

more specifically, $R_1$ may further comprise a group selected from: $C_1$-$C_6$ alkyl (e.g., methyl, ethyl, n-propyl, and isopropyl), and halogen (e.g., F, Cl, Br, and I).

In some embodiments of the present invention, the

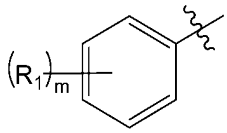

moiety has a structure of:

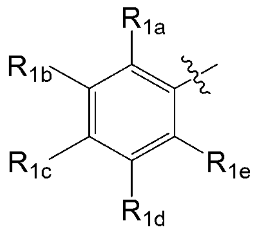,

wherein, $R_{1b}$ is selected from: $C_1$-$C_6$ haloalkyl, cyano, nitro, azido, - $OR_{101}$, -$C(O)R_{101}$, -$C(O)OR_{101}$, -$NHC(O)OR_{101}$, -$OC(O)R_{101}$, -$NHSO_2R_{101}$, -$SO_2NR_{101}R_{102}$, - $NHC(O)R_{101}$, -$C(O)NR_{101}R_{102}$, -$NR_{101}R_{102}$, -$SR_{101}$, -$S(O)_2R_{101}$, -$SO_3H$, -$(CH_2)_t$(phenyl), - $(CH_2)_t$(4-10 membered heterocyclyl), and -$(CH_2)_t$($C_3$-$C_{10}$ cycloalkyl); more specifically, $R_{1b}$ is selected from: $C_1$-$C_6$ haloalkyl, cyano, nitro, azido, -$OR_{101}$, -$C(O)R_{101}$, -$NHC(O)R_{101}$, - $C(O)NHR_{101}$, -$NHR_{101}$, -$SR_{101}$, -$(CH_2)_t$(4-8 membered heterocyclyl), and -$(CH_2)_t$($C_3$-$C_6$ cycloalkyl), where t is an integer from 0 to 5;

$R_{1c}$ is selected from: H, F, $C_1$-$C_6$ alkyl, cyano, nitro, azido, -$OR_{101}$, -$C(O)R_{101}$, -$C(O)OR_{101}$, -$NHC(O)OR_{101}$, -$OC(O)R_{101}$, -$NHSO_2R_{101}$, -$SO_2NR_{101}R_{102}$, -$NHC(O)R_{101}$, -$C(O)NR_{101}R_{102}$, -$NR_{101}R_{102}$, -$SR_{101}$, -$S(O)_2R_{101}$, -$SO_3H$, -$(CH_2)_t$(phenyl), -$(CH_2)_t$(4-10 membered heterocyclyl), and -$(CH_2)_t$($C_3$-$C_{10}$ cycloalkyl);

$R_{1a}$, $R_{1d}$, and $R_{1e}$ are selected from: H, $C_1$-$C_6$ alkyl (for example, methyl, ethyl, n-propyl, or isopropyl), halogen (for example, F, Cl, Br, or I), $C_1$-$C_6$ haloalkyl, cyano, nitro, azido, -$OR_{101}$, -$C(O)R_{101}$, -$C(O)OR_{101}$, -$NHC(O)OR_{101}$, -$OC(O)R_{101}$, -$NHSO_2R_{101}$, -$SO_2NR_{101}R_{102}$, - $NHC(O)R_{101}$, -$C(O)NR_{101}R_{102}$, -$NR_{101}R_{102}$, -$SR_{101}$, -$S(O)_2R_{101}$, -$SO_3H$, -$(CH_2)_t$(phenyl), - $(CH_2)_t$(4-10 membered heterocyclyl), and -$(CH_2)_t$($C_3$-$C_{10}$ cycloalkyl); and

$R_{101}$ and $R_{102}$ are each defined as above.

Further, $R_{1b}$ may be selected from: -$CF_3$, -$CHF_2$, -$CH_2F$,

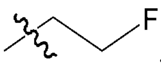, 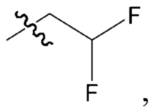, 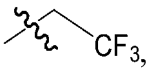

cyano, nitro, azido, -OH,

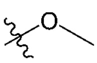, 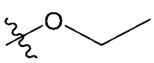, 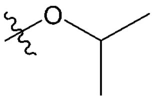, 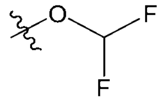, 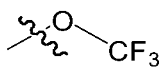

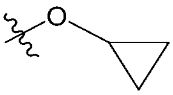, 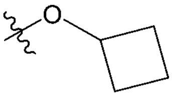, 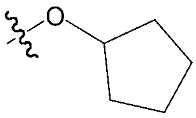, 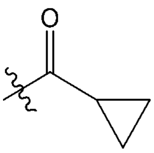, 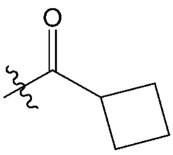, 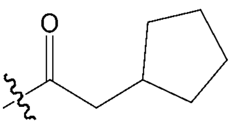,

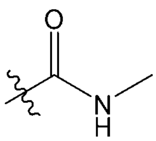, 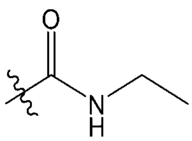, 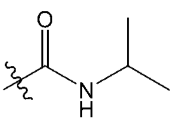, 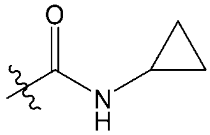, 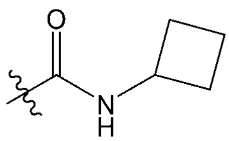,

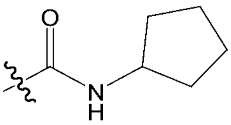, 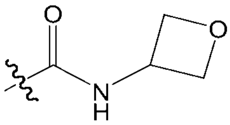, 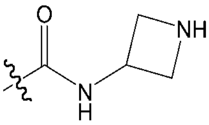, 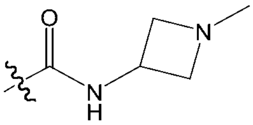, 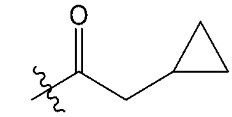,

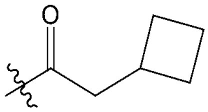, 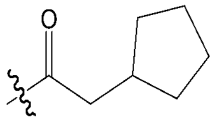, 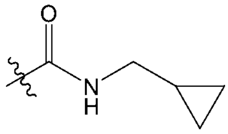, 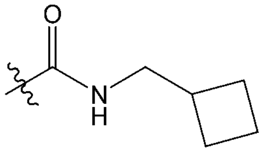,

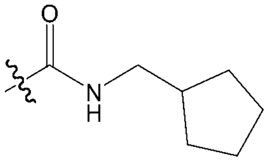, 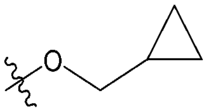, 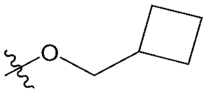, 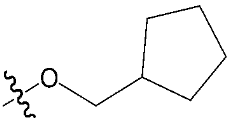, 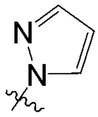, 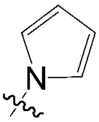,

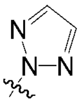, 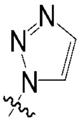, 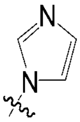, 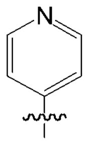, 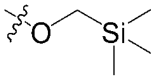, 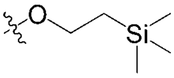, 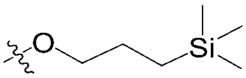, 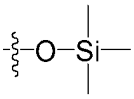,

and

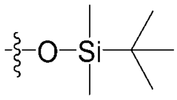.

In some embodiments of the present invention, $R_{1b}$ is -C(O)NR$_{101}$R$_{102}$, wherein, $R_{101}$ and $R_{102}$ are independently selected from: H, $C_1$-$C_6$ alkyl, $C_3$-$C_6$ cycloalkyl, and $C_4$-$C_{10}$ cycloalkyl alkyl.

Further, $R_{1a}$ and $R_{1e}$ may be independently selected from: H, halogen (for example, F, or Cl), and $C_1$-$C_3$ alkyl (for example, methyl).

Further, $R_{1c}$ may be selected from: H and F.

Further, $R_{1d}$ may be selected from: H, halogen (for example, F or Cl), and $C_1$-$C_3$ alkyl (for example, methyl).

In some embodiments of the present invention, the

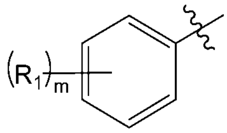

moiety has a structure of:

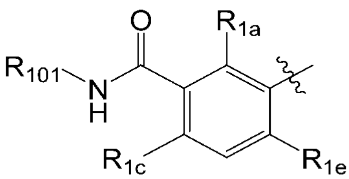.

In some embodiments of the present invention, $L_1$ is -C(O)NR$_3$-, and $R_0$ is

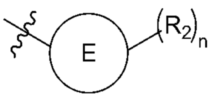.

In some embodiments of the present invention, $R_0$ is selected from: H, cyano, -O($C_0$-$C_{10}$ alkyl), -O($C_1$-$C_{10}$ silyl), -N($C_0$-$C_{10}$ alkyl)($C_0$-$C_{10}$ alkyl), -S($C_0$-$C_{10}$ alkyl), -C(O)($C_0$-$C_{10}$ alkyl), -C(O)O($C_0$-$C_{10}$ alkyl), -OC(O)($C_0$-$C_{10}$ alkyl), -N($C_0$-$C_{10}$ alkyl)SO$_2$($C_0$-$C_{10}$ alkyl), - SO$_2$N($C_0$-$C_{10}$ alkyl)($C_0$-$C_{10}$ alkyl), -N($C_0$-$C_{10}$ alkyl)C(O)($C_0$-$C_{10}$ alkyl), -C(O)N($C_0$-$C_{10}$ alkyl)($C_0$-$C_{10}$ alkyl), and -SO$_2$($C_0$-$C_{10}$ alkyl), wherein alkyl is optionally substituted by a group selected from:

halogen, cyano, hydroxyl, amino, $C_1$-$C_6$ alkoxy, $C_1$-$C_6$ alkylamino, and $C_3$-$C_6$ cycloalkyl; more specifically, $R_0$ is selected from:

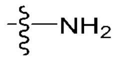, 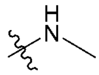, 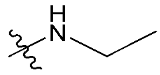,

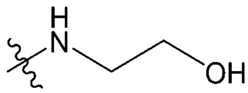, 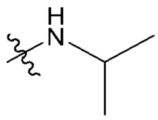, 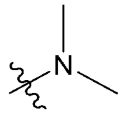, 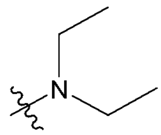, 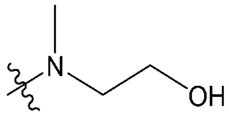,

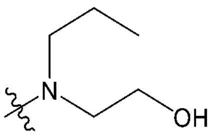, 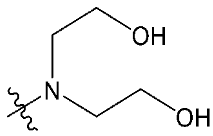,

H, cyano, -OH,

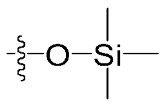, 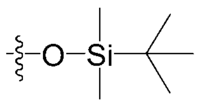, 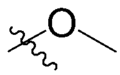,

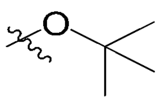, 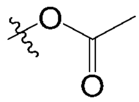, 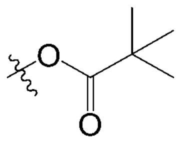,

-COOH, and

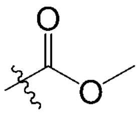.

In some embodiments of the present invention, $L_2$ is $C_0$-$C_{10}$ alkylene, wherein one or more H atoms in alkylene are optionally and independently substituted by a group selected from: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, or $C_4$-$C_{10}$ cycloalkyl alkyl; and more specifically, $L_2$ is $C_0$-$C_6$ alkylene.

In some embodiments of the present invention, $L_1$ is a single bond, $L_2$ is a single bond, and $R_0$ is H. That is,, the compound has a structure of:

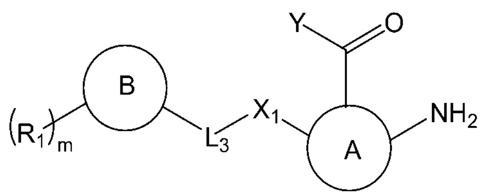,

in particular

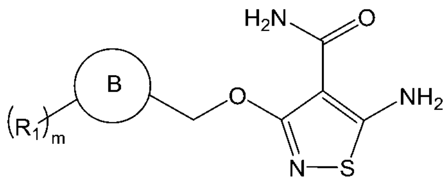 or 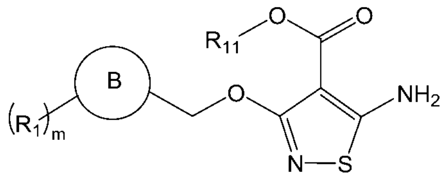 ,

and

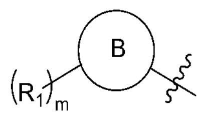

is defined as above.

In some embodiments of the present invention, the compound has a structure of:

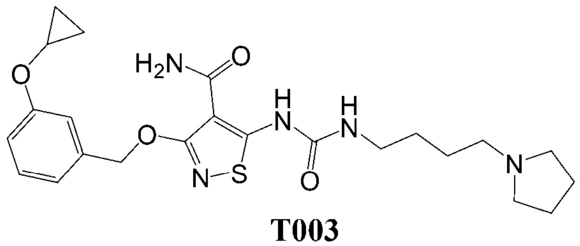

T003

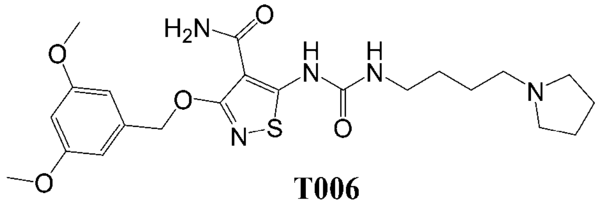

T006

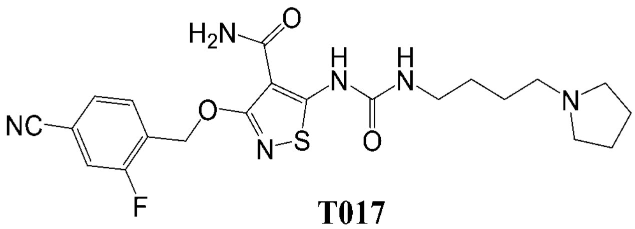

T017

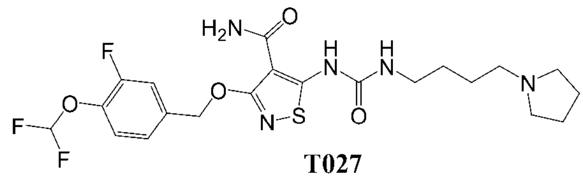

T027

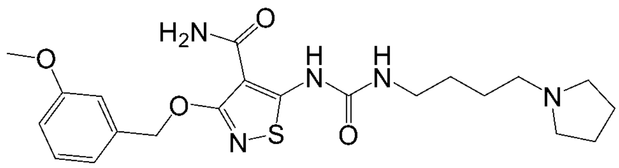

T028

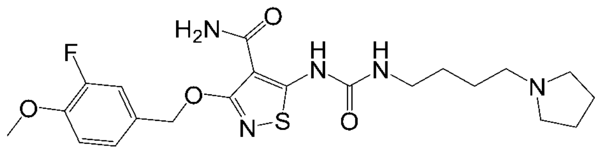

T029

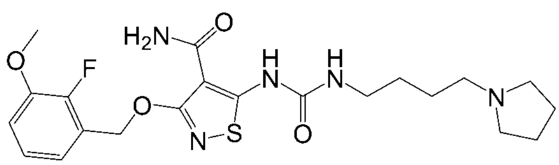

T030

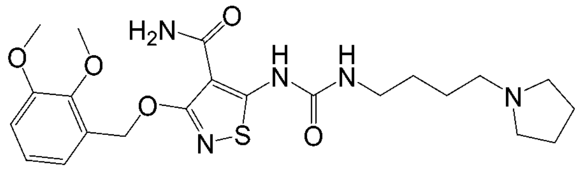

T032

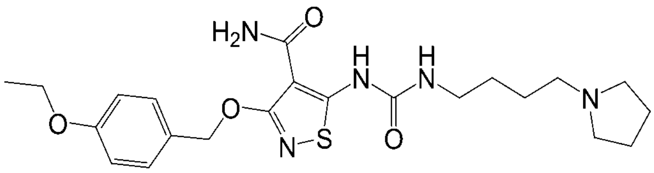

T036

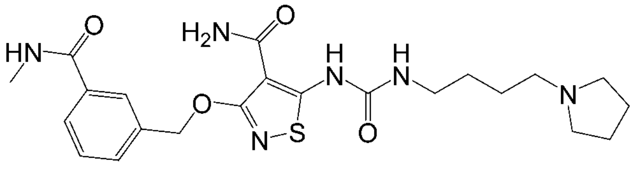

**T049**

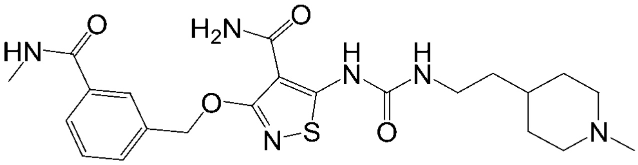

**T055**

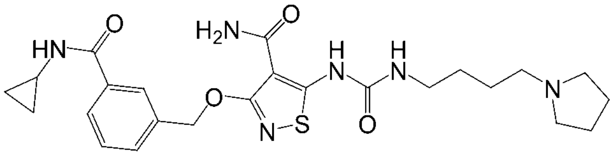

**T056**

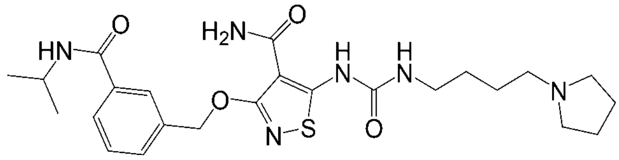

**T057**

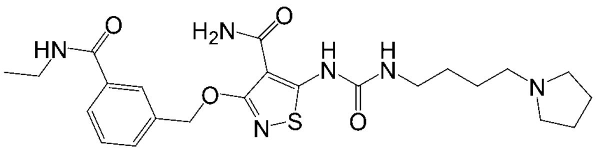

**T058**

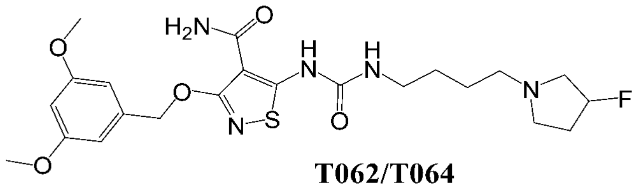

**T062/T064**

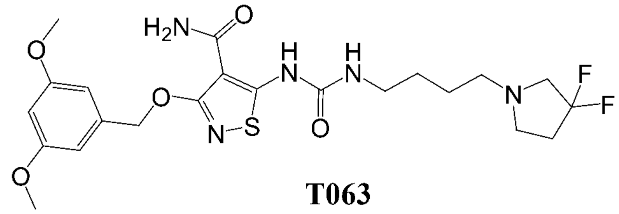

**T063**

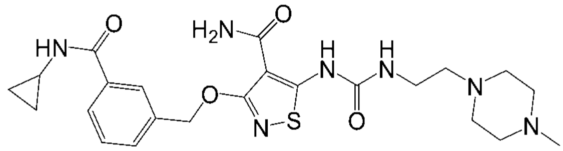

**T065**

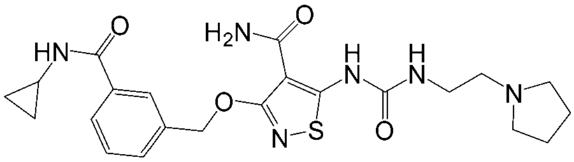

**T066**

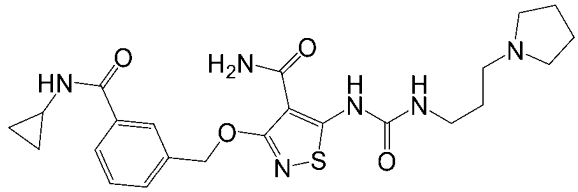

**T067**

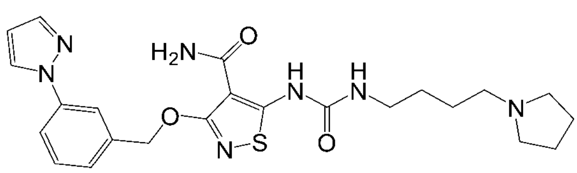

**T068**

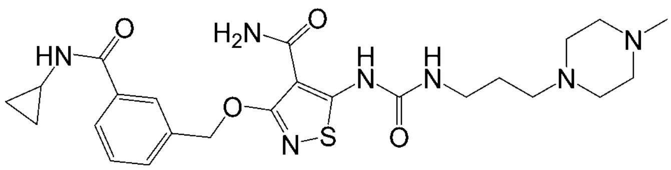

**T069**

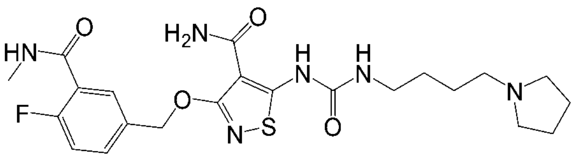

T070

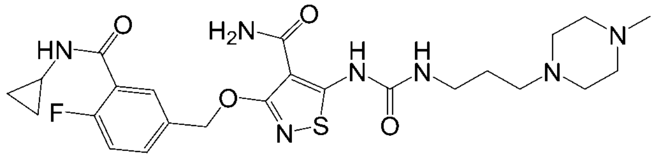

T078

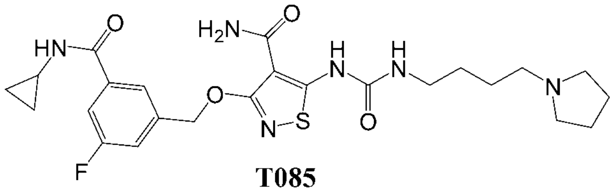

T085

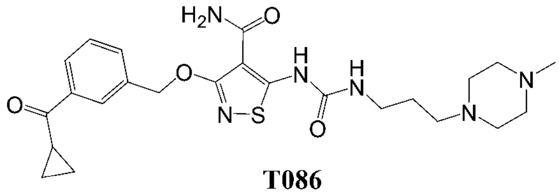

T086

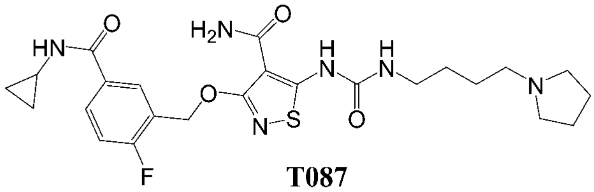

T087

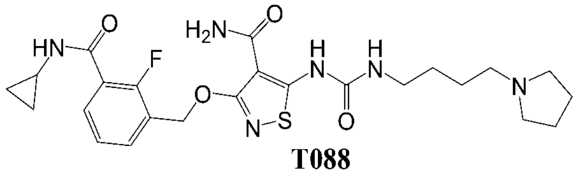

T088

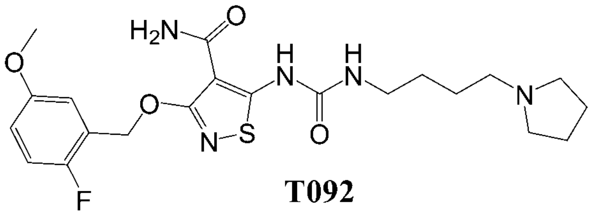

T092

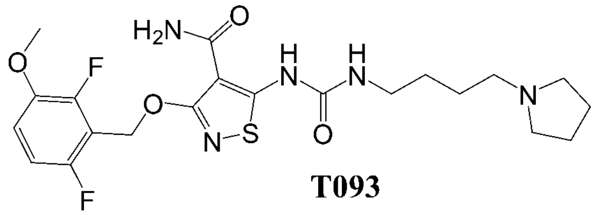

T093

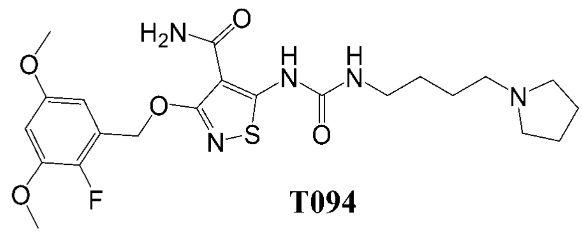

T094

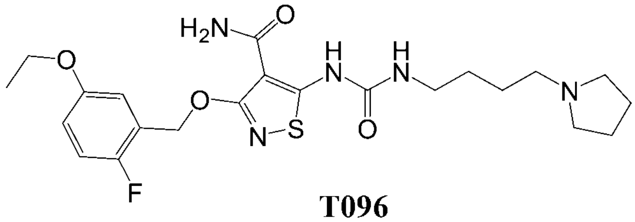

T096

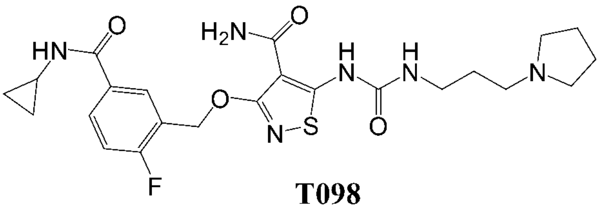

T098

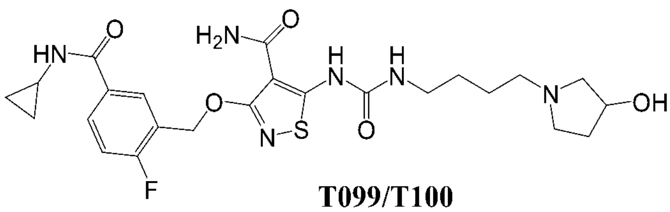

T099/T100

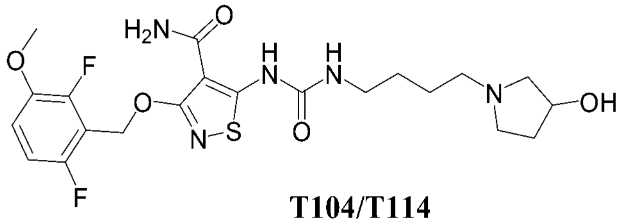

**T104/T114**

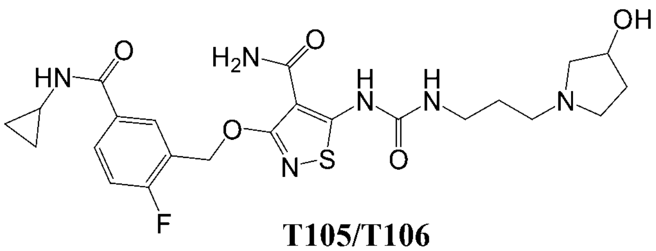

**T105/T106**

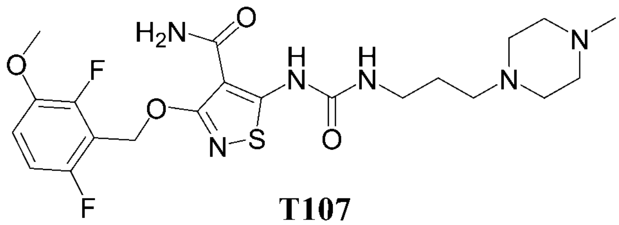

**T107**

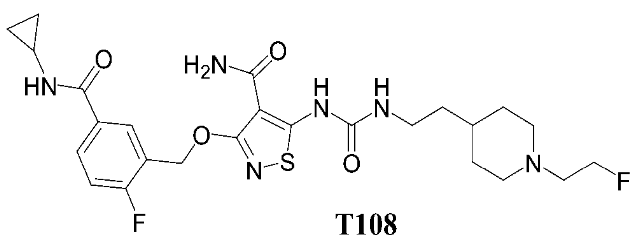

**T108**

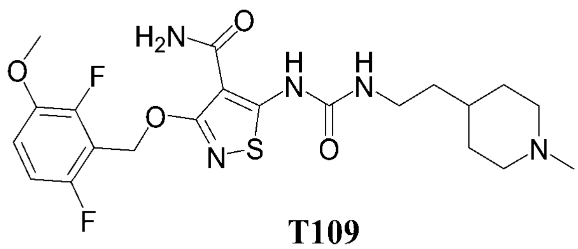

**T109**

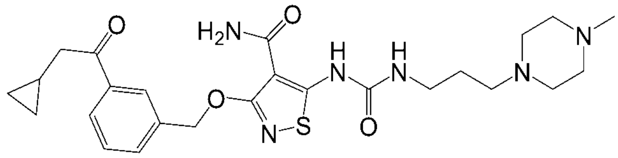

**T110**

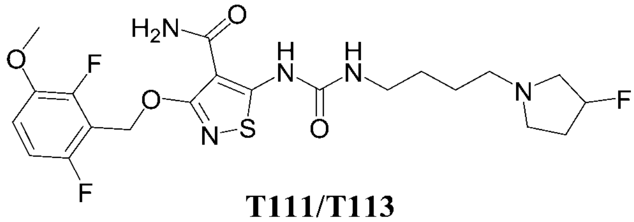

**T111/T113**

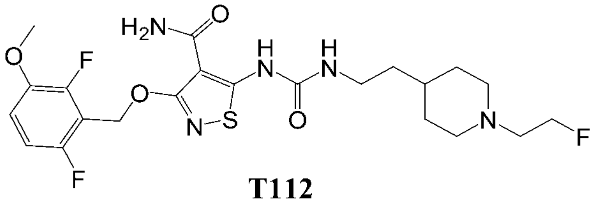

**T112**

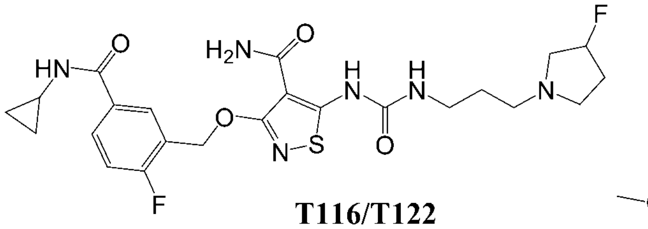

**T116/T122**

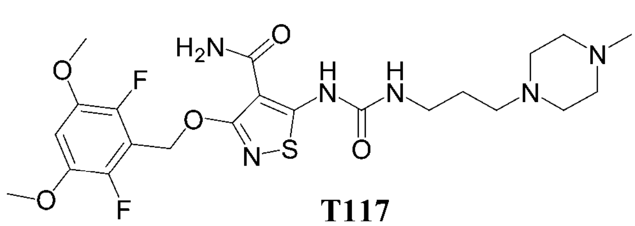

**T117**

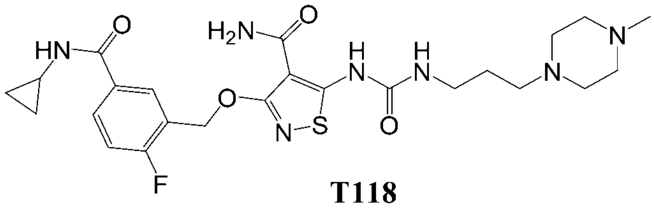

**T118**

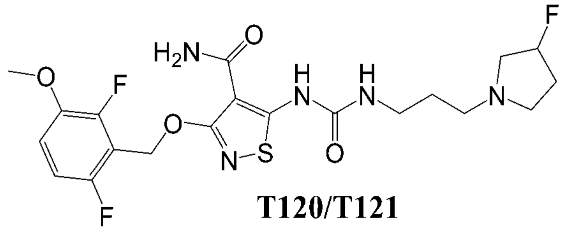

**T120/T121**

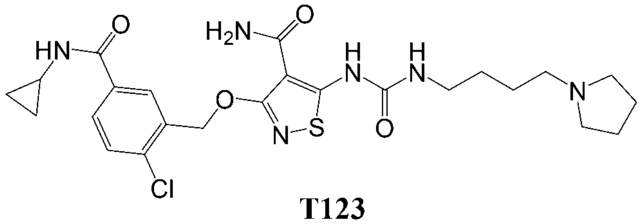

**T123**

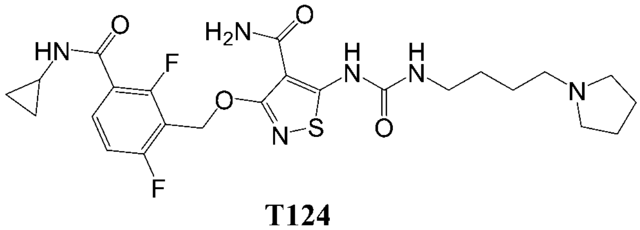

**T124**

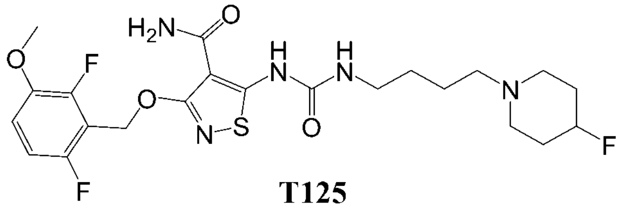

**T125**

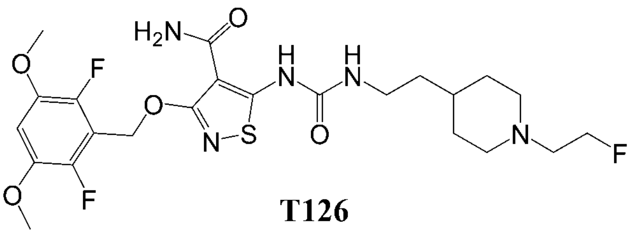

**T126**

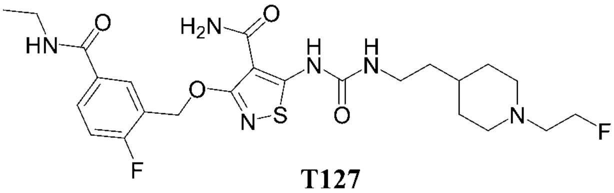

**T127**

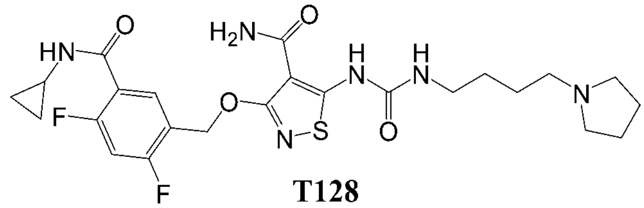

**T128**

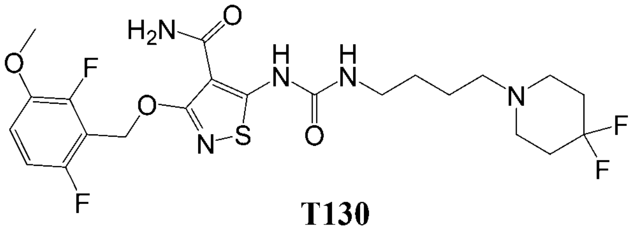

**T130**

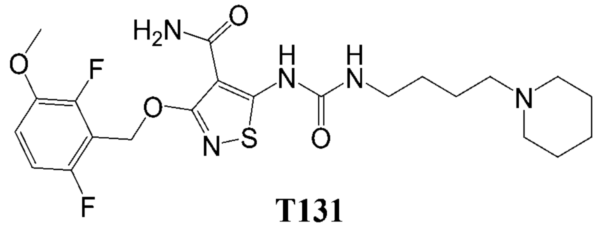

**T131**

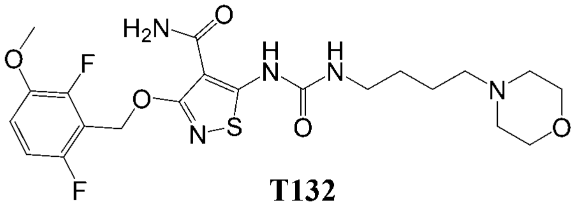

**T132**

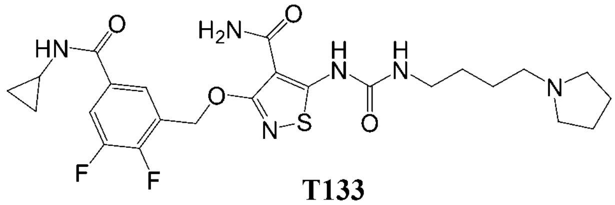

**T133**

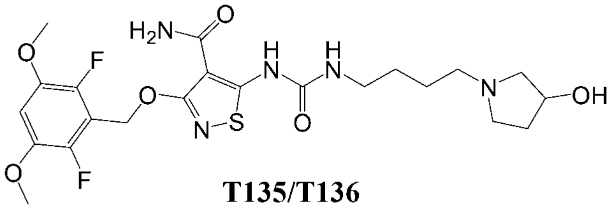

**T135/T136**

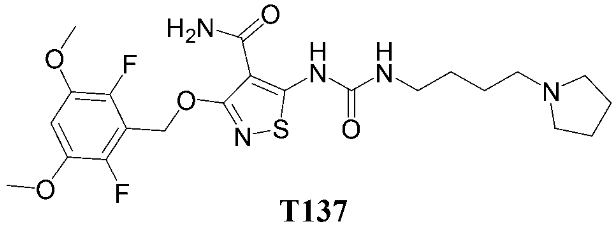

**T137**

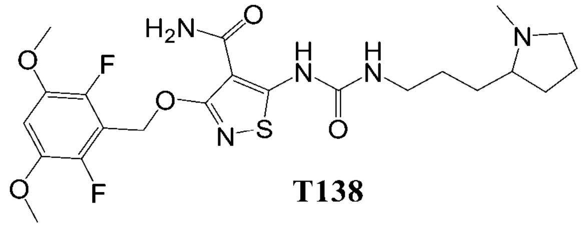

**T138**

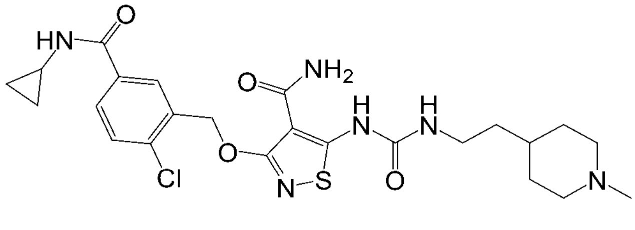

**T139**

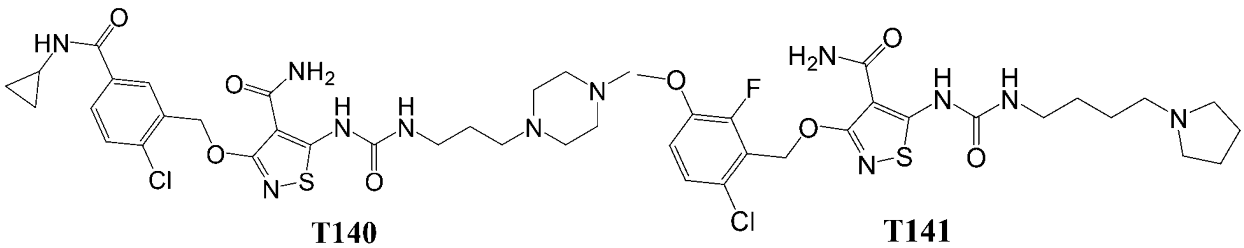

**T140**

**T141**

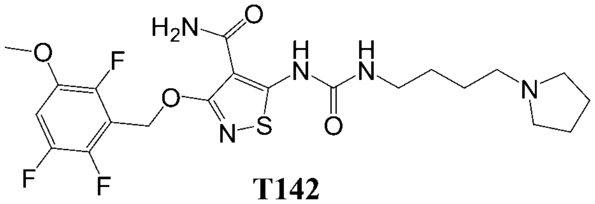
T142

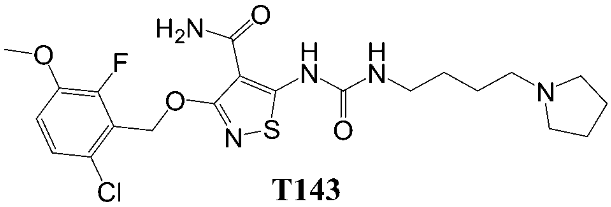
T143

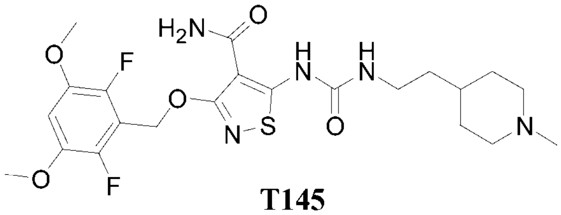
T145

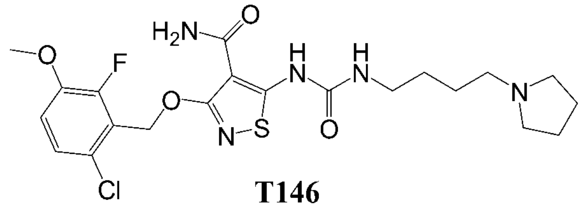
T146

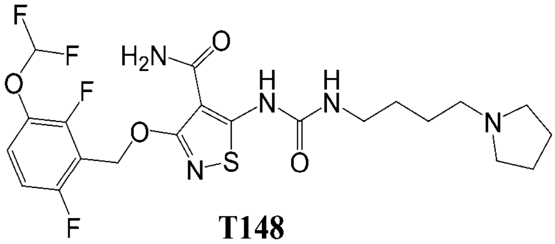
T148

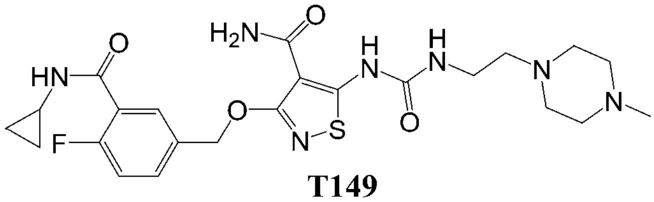
T149

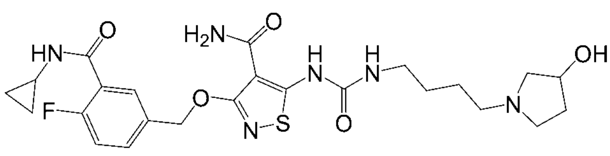
T150

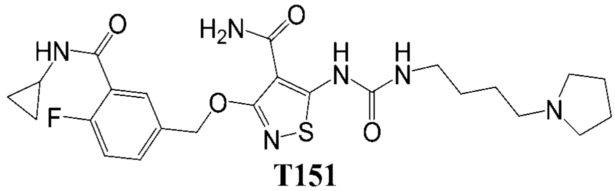
T151

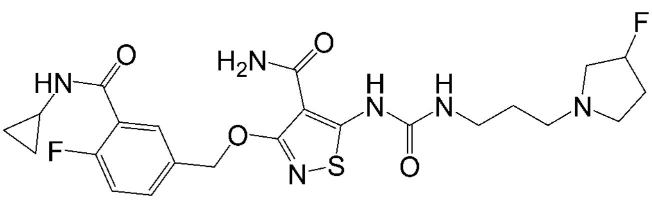
T152

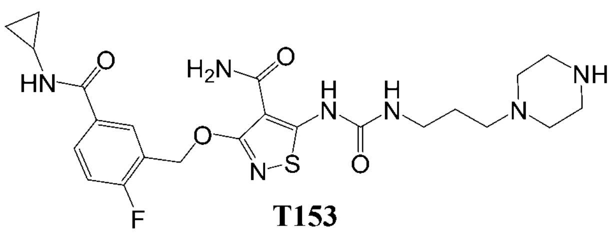
T153

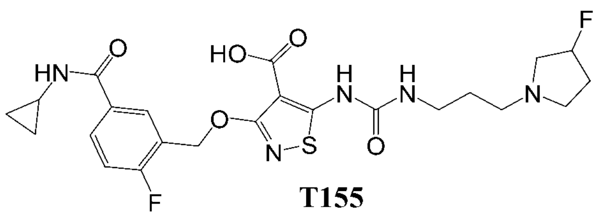
T155

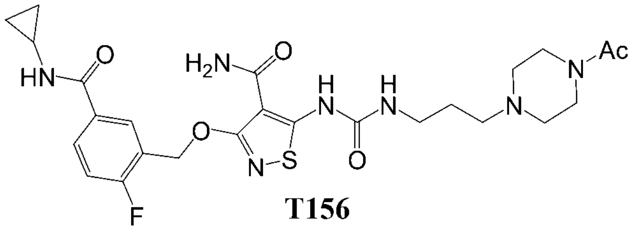
T156

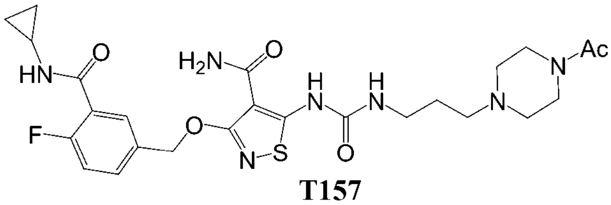
T157

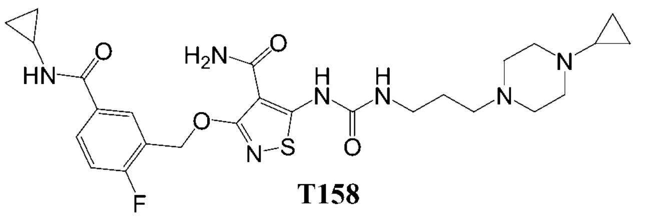
**T158**

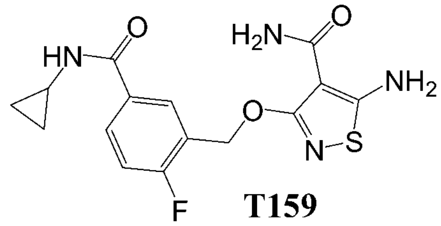
**T159**

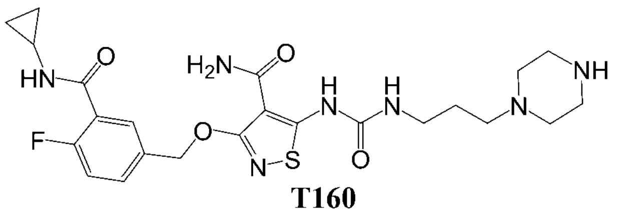
**T160**

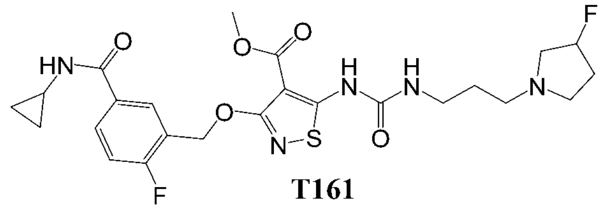
**T161**

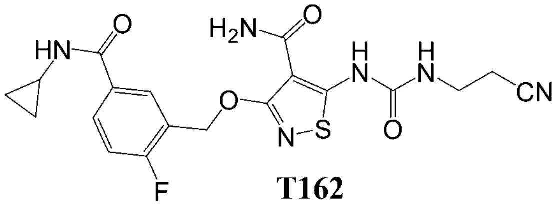
**T162**

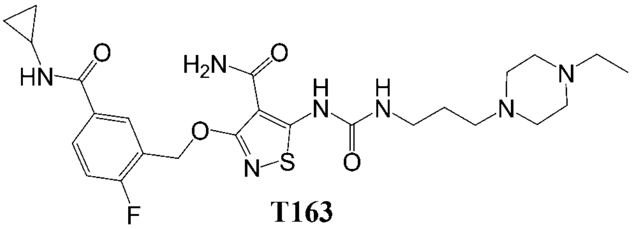
**T163**

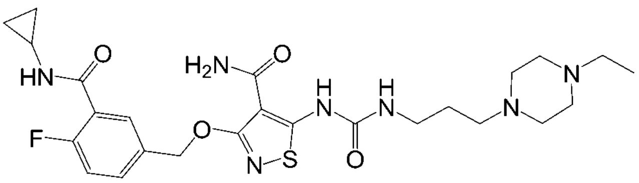
**T164**

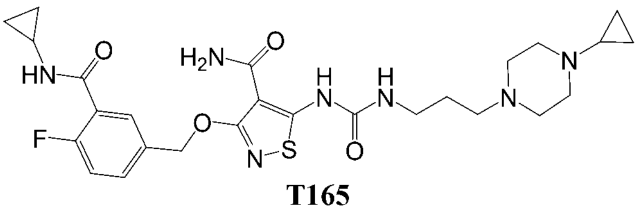
**T165**

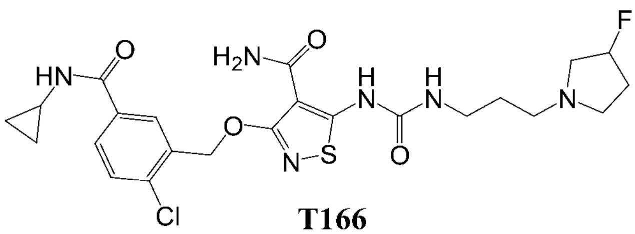
**T166**

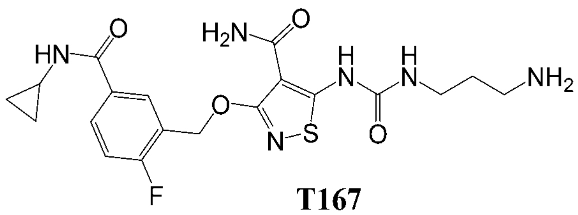
**T167**

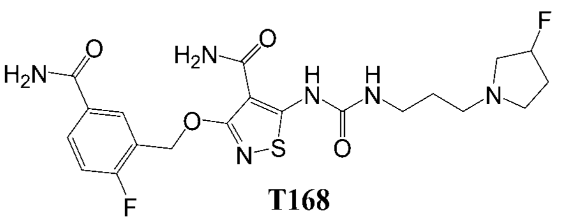
**T168**

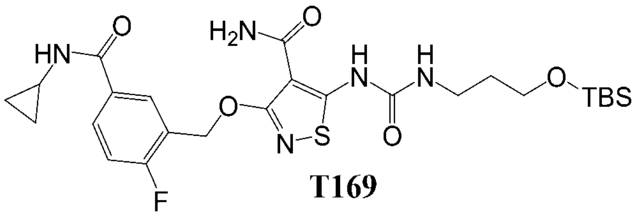
**T169**

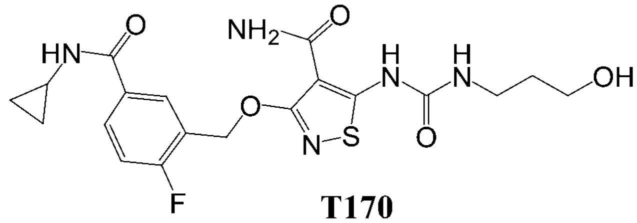

T170

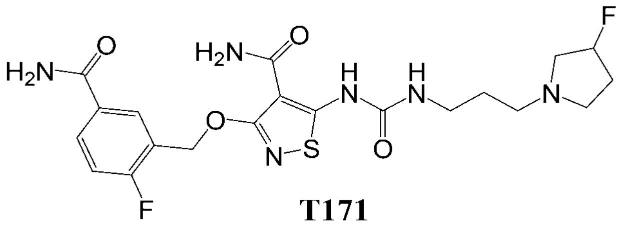

T171

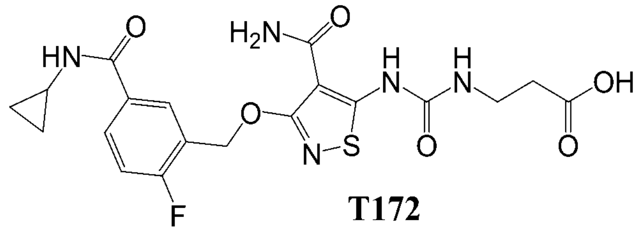

T172

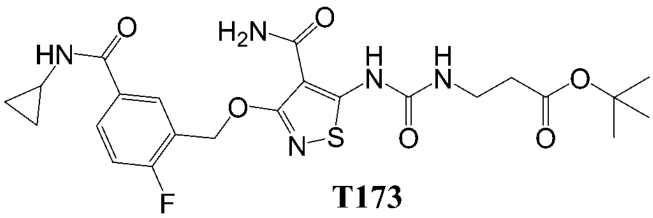

T173

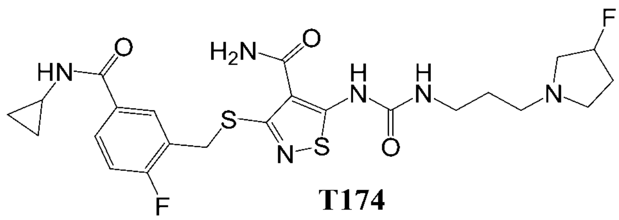

T174

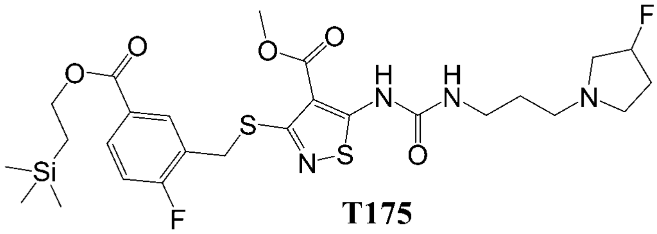

T175

In an embodiment (2) of the present invention, in Formula I, in particular Formula II or III, $L_1$ is $-C(O)NR_3-$, the B ring is a monoheterocyclic ring, in particular a 5-6 membered monoheterocyclic ring, for example the

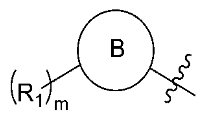

moiety may be

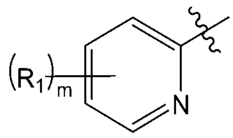,

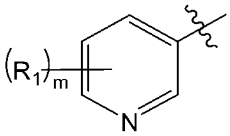, 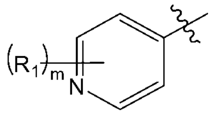, 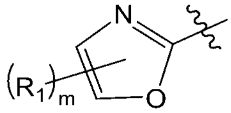, 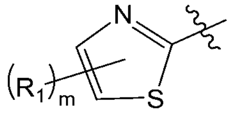,

or

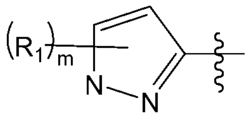,

with $R_1$ as defined above in the present invention.

Specifically, each $R_1$ may be independently selected from: H, $C_1$-$C_6$ alkyl, $C_1$-$C_6$ haloalkyl, halogen, cyano, nitro, azido, $-OR_{101}$, $-C(O)R_{101}$, $-C(O)OR_{101}$, $-NHC(O)OR_{101}$, $-OC(O)R_{101}$, - $NHSO_2R_{101}$, $-SO_2NR_{101}R_{102}$, $-NHC(O)R_{101}$, $-C(O)NR_{101}R_{102}$, $-NR_{101}R_{102}$, $-SR_{101}$, $-S(O)_2R_{101}$, $-SO_3H$, $-(CH_2)_t$(phenyl), $-(CH_2)_t$(4-10 membered heterocyclyl), and $-(CH_2)_t(C_3$-$C_{10}$ cycloalkyl), where t is an integer form 0 to 5.

In some embodiments of the present invention, each $R_1$ may be independently selected from: H, $C_1$-$C_6$ alkyl, $C_1$-$C_6$ haloalkyl, halogen, -OH, and $C_1$-$C_6$ alkoxy.

More specifically, each $R_1$ may be independently selected from: H, methyl, ethyl, n-propyl, isopropyl, $-CF_3$, $-CHF_2$, $-CH_2F$,

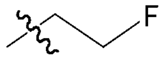, 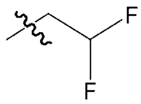, 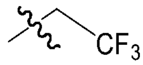,

F, Cl, Br, I, cyano, nitro, azido, -OH,

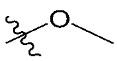, 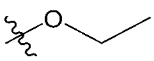, 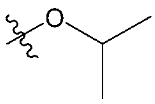, 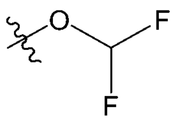, 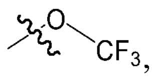, 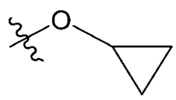,

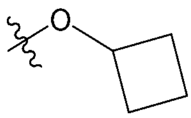, 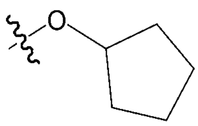, 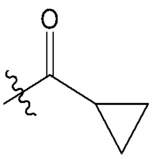, 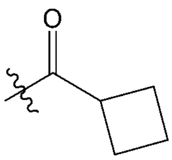, 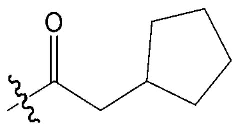, 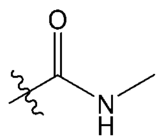,

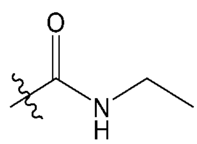, 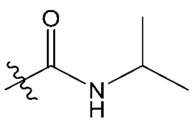, 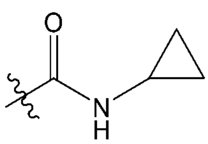, 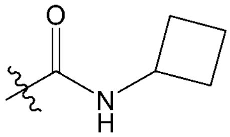, 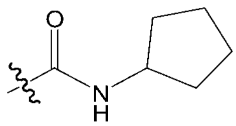,

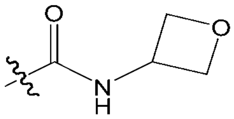, 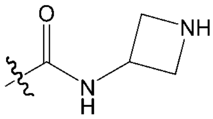, 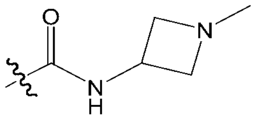, 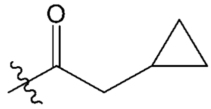, 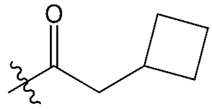,

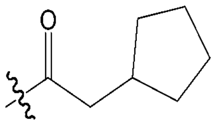, 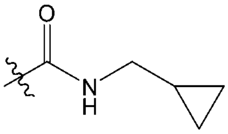, 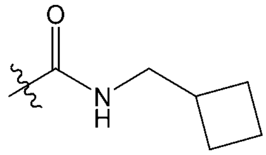, 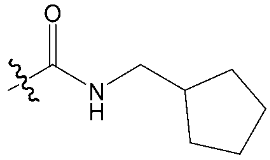,

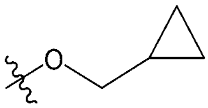, 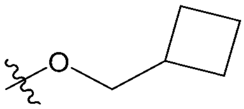, 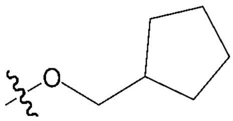, 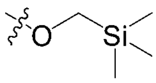, 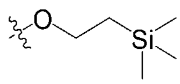,

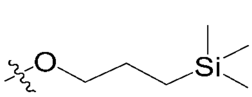, 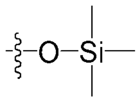, and 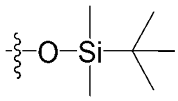.

In some embodiments of the present invention, $L_1$ is -C(O)$NR_3$-, and $R_0$ is

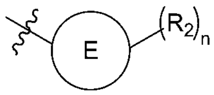.

In some embodiments of the present invention, $R_0$ is selected from: H, cyano, -O($C_0$-$C_{10}$ alkyl), -O($C_1$-$C_{10}$ silyl), -N($C_0$-$C_{10}$ alkyl)($C_0$-$C_{10}$ alkyl), -S($C_0$-$C_{10}$ alkyl), -C(O)($C_0$-$C_{10}$ alkyl), -C(O)O($C_0$-$C_{10}$ alkyl), -OC(O)($C_0$-$C_{10}$ alkyl), -N($C_0$-$C_{10}$ alkyl)$SO_2$($C_0$-$C_{10}$ alkyl), - $SO_2$N($C_0$-$C_{10}$ alkyl)($C_0$-$C_{10}$ alkyl), -N($C_0$-$C_{10}$ alkyl)C(O)($C_0$-$C_{10}$ alkyl), -C(O)N($C_0$-$C_{10}$ alkyl)($C_0$-$C_{10}$ alkyl), and -$SO_2$($C_0$-$C_{10}$ alkyl), wherein alkyl is optionally substituted by a group selected from: halogen, cyano, hydroxyl, amino, $C_1$-$C_6$ alkoxy, $C_1$-$C_6$ alkylamino, and $C_3$-$C_6$ cycloalkyl; more specifically, $R_0$ is selected from:

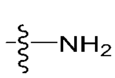, 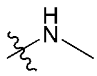, 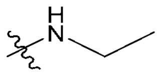,

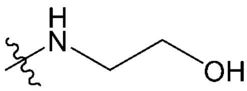, 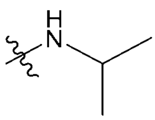, 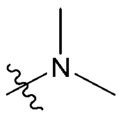, 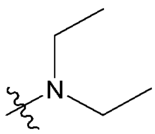, 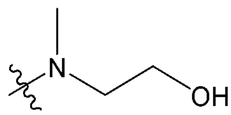,

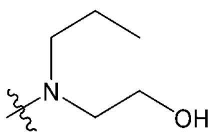, 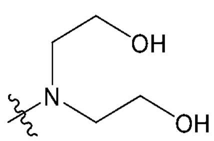,

H, cyano, -OH,

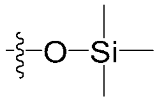, 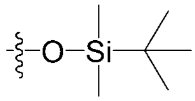, 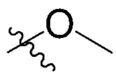,

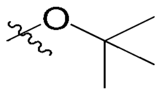, 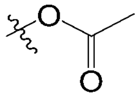, 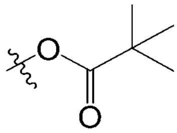,

-COOH, and

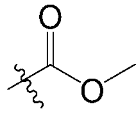.

In some embodiments of the present invention, $L_2$ is $C_0$-$C_{10}$ alkylene, wherein one or more H atoms in alkylene are optionally and independently substituted by a group selected from: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, or $C_4$-$C_{10}$ cycloalkyl alkyl; and more specifically, $L_2$ is $C_0$-$C_6$ alkylene.

In some embodiments of the present invention, the compound has a structure of:

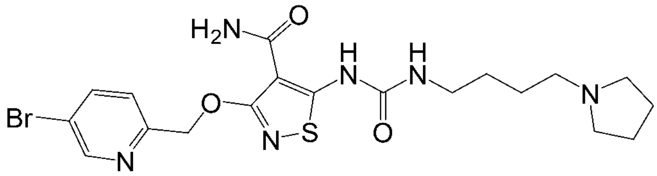

**T005**

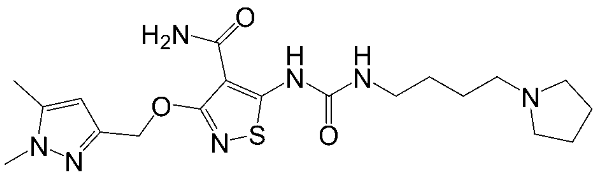

**T009**

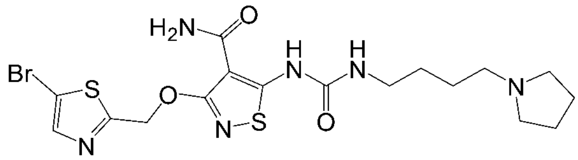

**T015**

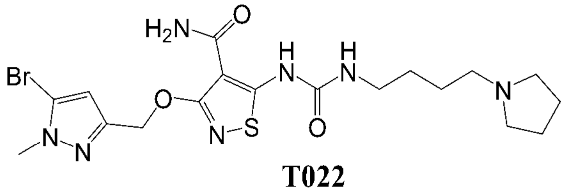

**T022**

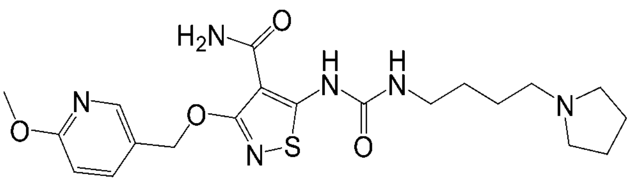

**T033**

.

In an embodiment (3) of the present invention, in Formula I, in particular Formula II or III, $L_1$ is -C(O)$NR_3$-, the B ring is a bicyclic ring, in particular a 9-11 membered fused bicyclic ring, for example, the

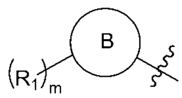

moiety may be

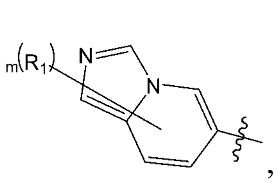, 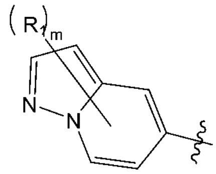,

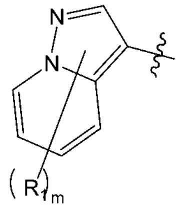, 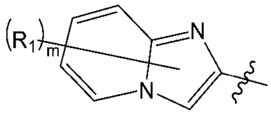, 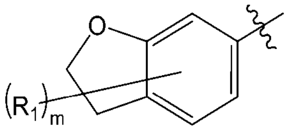, 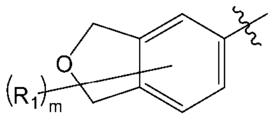,

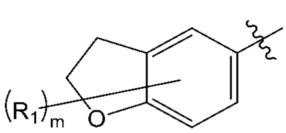, 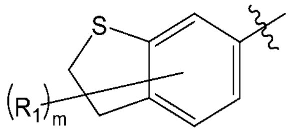, 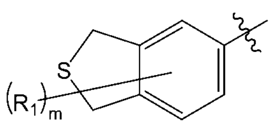, 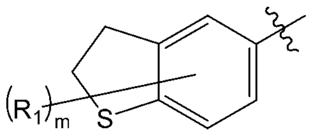,

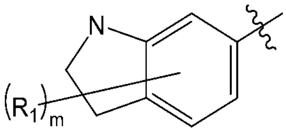, 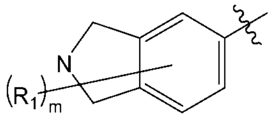, 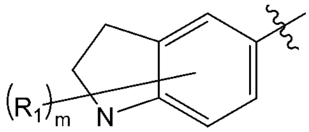, 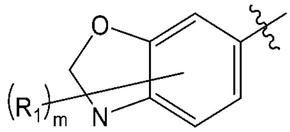,

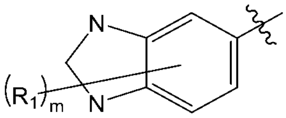, 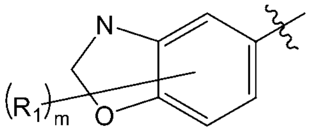, 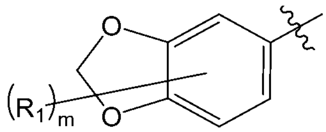,

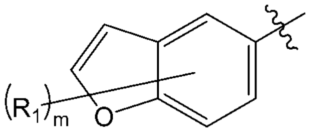, 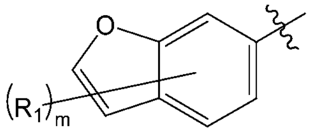, 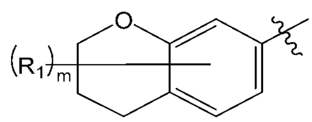,

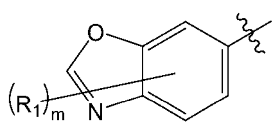, 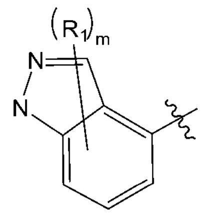, 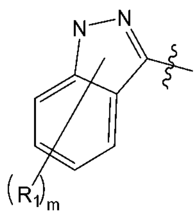, 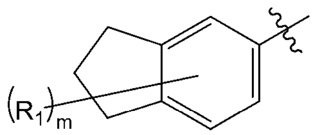,

or

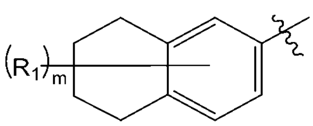,

with $R_1$ as defined above in the present invention.

Specifically, each $R_1$ may be independently selected from: H, $C_1$-$C_6$ alkyl, $C_1$-$C_6$ haloalkyl, halogen, cyano, nitro, azido, $-OR_{101}$, $-C(O)R_{101}$, $-C(O)OR_{101}$, $-NHC(O)OR_{101}$, $-OC(O)R_{101}$, - $NHSO_2R_{101}$, $-SO_2NR_{101}R_{102}$, $-NHC(O)R_{101}$,

-$C(O)NR_{101}R_{102}$, -$NR_{101}R_{102}$, -$SR_{101}$, -$S(O)_2R_{101}$, -$SO_3H$, -$(CH_2)_t$(phenyl), -$(CH_2)_t$(4-10 membered heterocyclyl), and -$(CH_2)_t(C_3$-$C_{10}$ cycloalkyl), where t is an integer form 0 to 5.

In some embodiments of the present invention, each $R_1$ may be independently selected from: H, $C_1$-$C_6$ alkyl, $C_1$-$C_6$ haloalkyl, halogen, -OH, and $C_1$-$C_6$ alkoxy.

More specifically, each $R_1$ is independently selected from: H, methyl, ethyl, n-propyl, isopropyl, -$CF_3$, -$CHF_2$, -$CH_2F$,

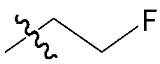, 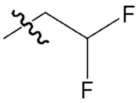, 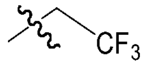,

F, Cl, Br, I, cyano, nitro, azido, -OH,

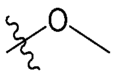, 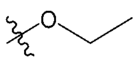, 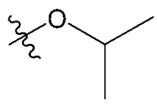, 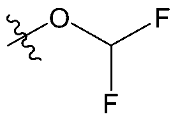, 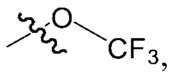, 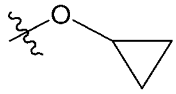,

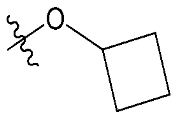, 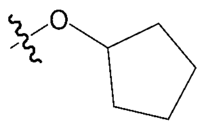, 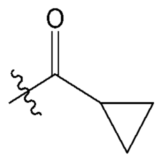, 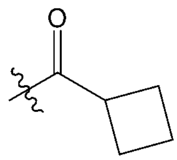, 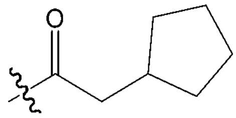, 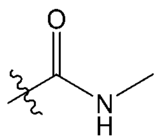,

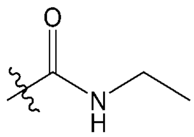, 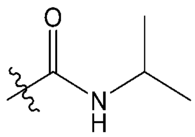, 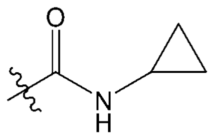, 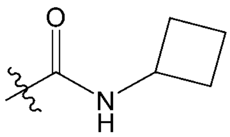, 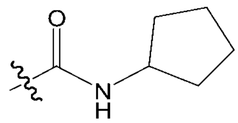,

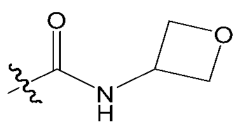, 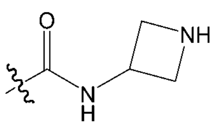, 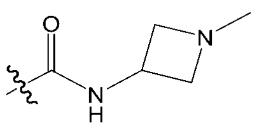, 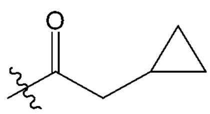, 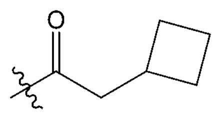,

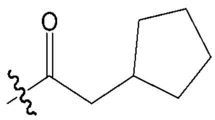, 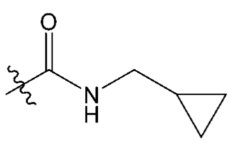, 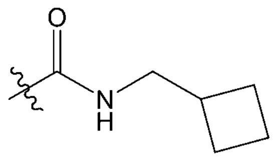, 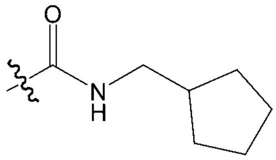,

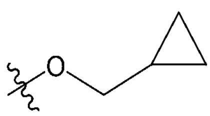, 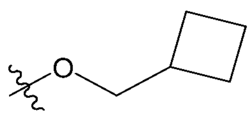, 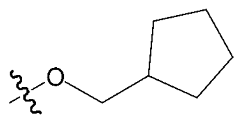, 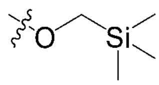, 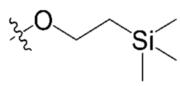,

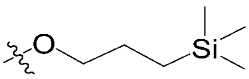, 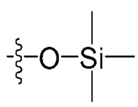, and 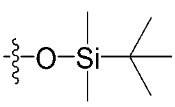;

and in some embodiments of the present invention, each $R_1$ is independently selected from: H, methyl, ethyl, -$CF_3$, -$CHF_2$, -$CH_2F$, F, Cl, Br, I, cyano, nitro, azido, and -OH.

In some embodiments of the present invention, $L_1$ is -$C(O)NR_3$-, and $R_0$ is

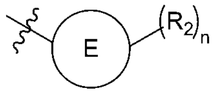.

In some embodiments of the present invention, $R_0$ is selected from: H, cyano, -O($C_0$-$C_{10}$ alkyl), -O($C_1$-$C_{10}$ silyl), -N($C_0$-$C_{10}$ alkyl)($C_0$-$C_{10}$ alkyl), -S($C_0$-$C_{10}$ alkyl), -C(O)($C_0$-$C_{10}$ alkyl), -C(O)O($C_0$-$C_{10}$ alkyl), -OC(O)($C_0$-$C_{10}$ alkyl), -N($C_0$-$C_{10}$ alkyl)$SO_2$($C_0$-$C_{10}$ alkyl), - $SO_2$N($C_0$-$C_{10}$ alkyl)($C_0$-$C_{10}$ alkyl), -N($C_0$-$C_{10}$ alkyl)C(O)($C_0$-$C_{10}$ alkyl), -C(O)N($C_0$-$C_{10}$ alkyl)($C_0$-$C_{10}$ alkyl), and -$SO_2$($C_0$-$C_{10}$ alkyl), wherein alkyl is optionally substituted by a group selected from: halogen, cyano, hydroxyl, amino, $C_1$-$C_6$ alkoxy, $C_1$-$C_6$ alkylamino, and $C_3$-$C_6$ cycloalkyl; more specifically, $R_0$ is selected from:

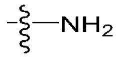, 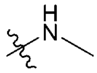, 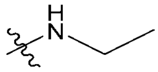,

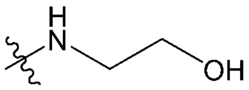, 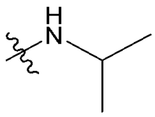, 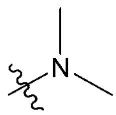, 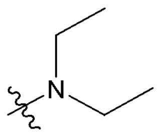, 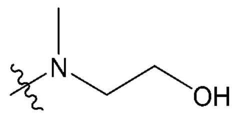,

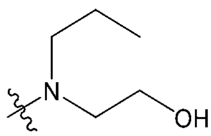, 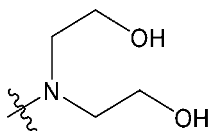,

H, cyano, -OH,

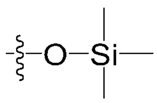, 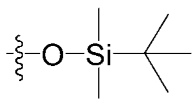, 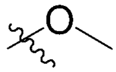,

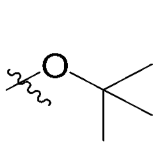, 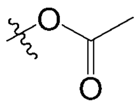, 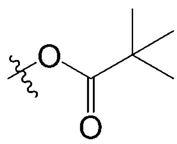,

-COOH, and

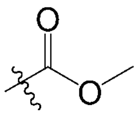.

In some embodiments of the present invention, $L_2$ is $C_0$-$C_{10}$ alkylene, wherein one or more H atoms in alkylene are optionally and independently substituted by a group selected from: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, or $C_4$-$C_{10}$ cycloalkyl alkyl; and more specifically, $L_2$ is $C_1$-$C_6$ alkylene.

In some embodiments of the present invention, the compound has a structure of:

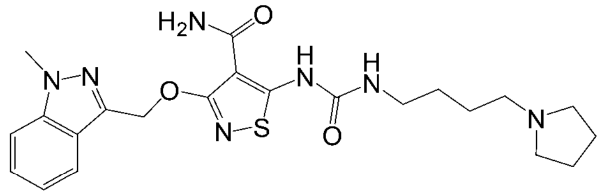

T002

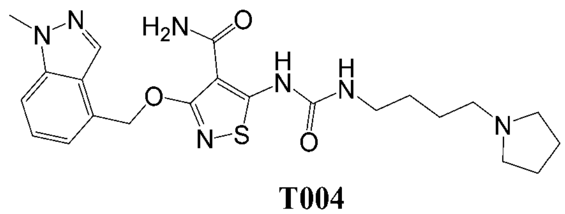

T004

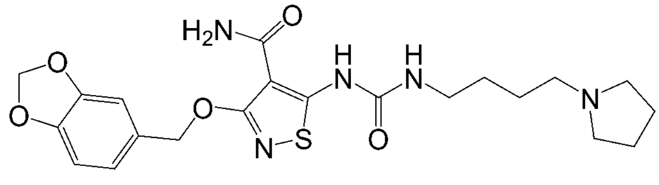

T007

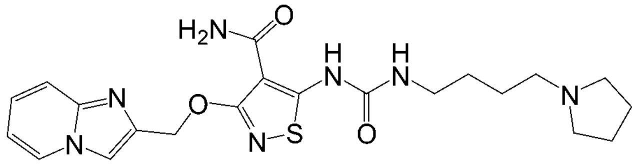

T013

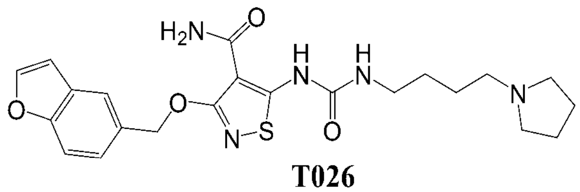

T026

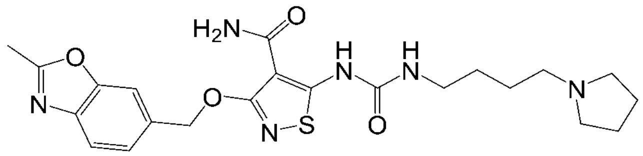

T035

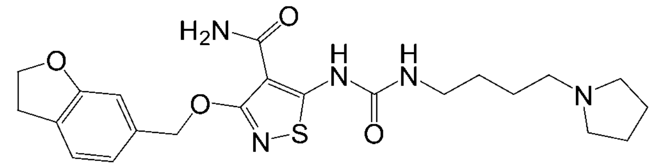

T042

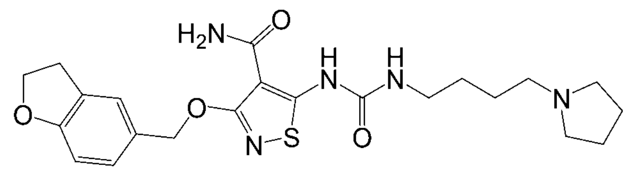

T044

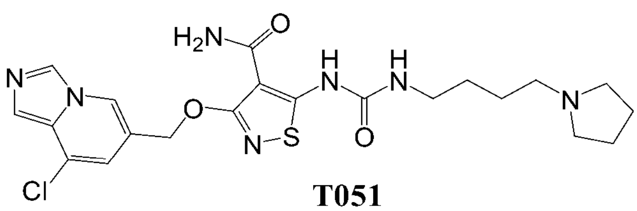

T051

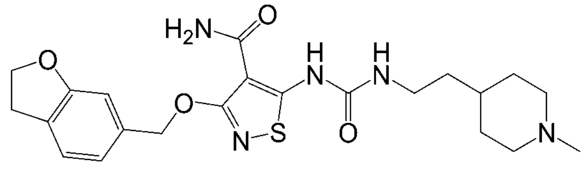

T053

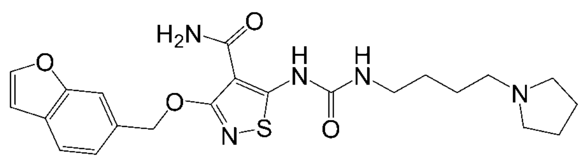

T054

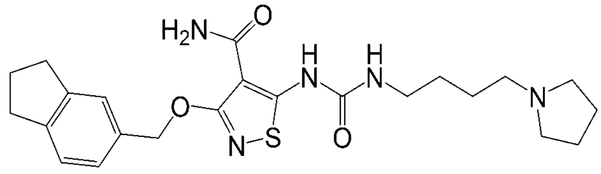

T061

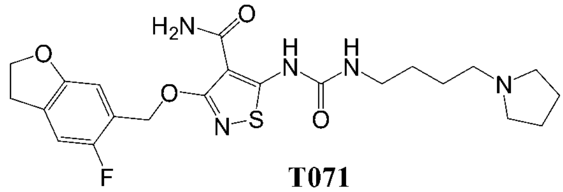

T071

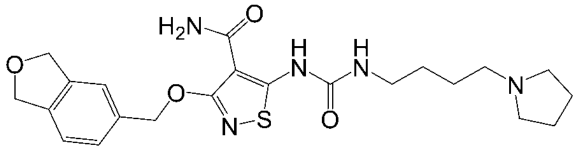

T072

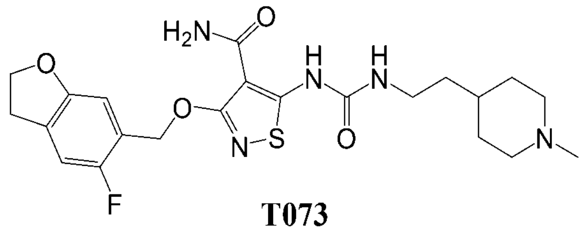

T073

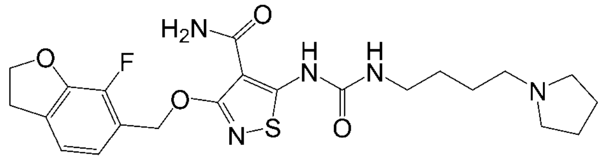

T074

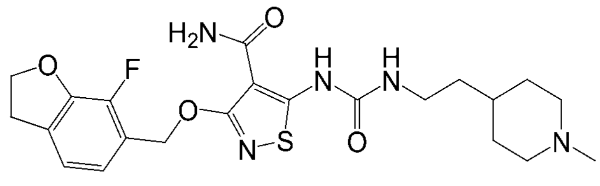

T075

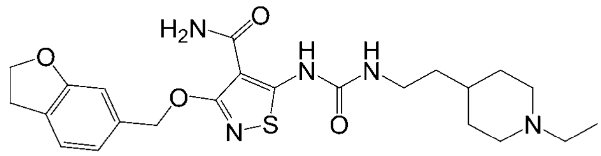

T076

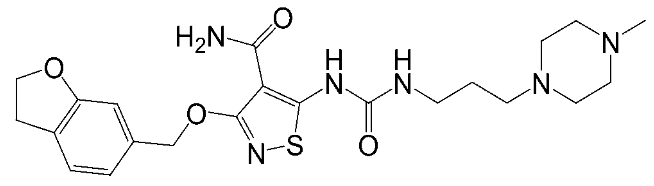

T077

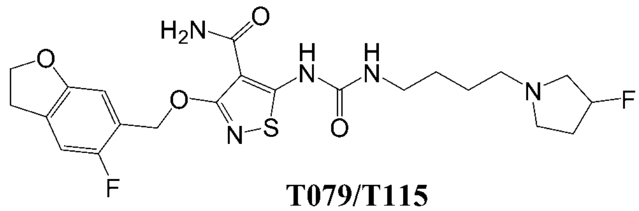

T079/T115

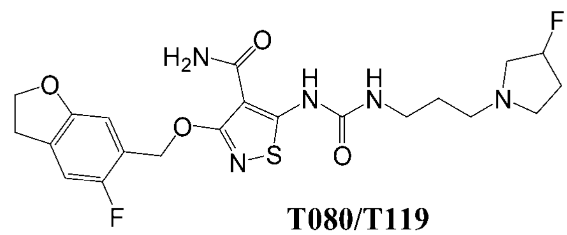

T080/T119

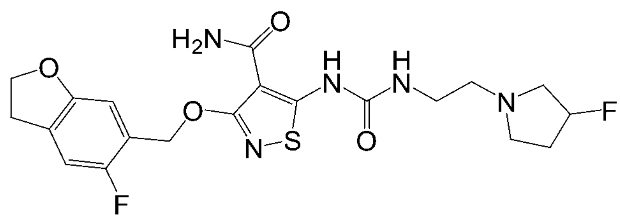

T081

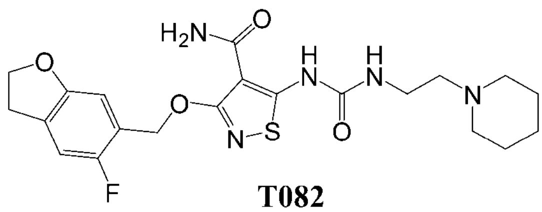

T082

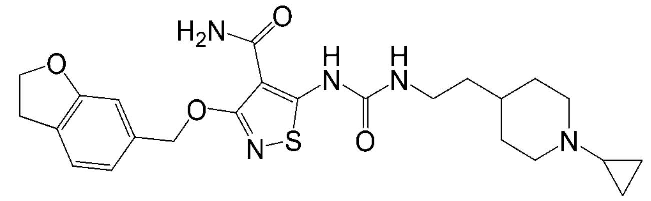

**T083**

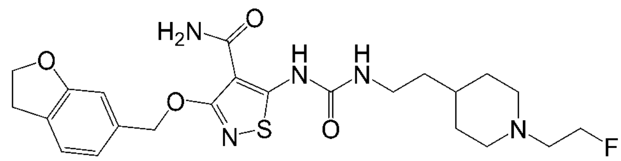

**T084**

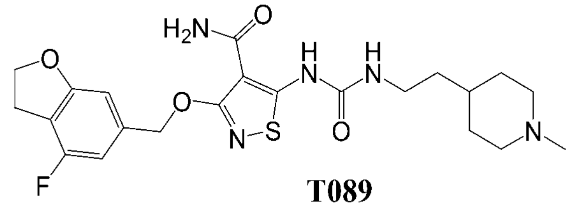

**T089**

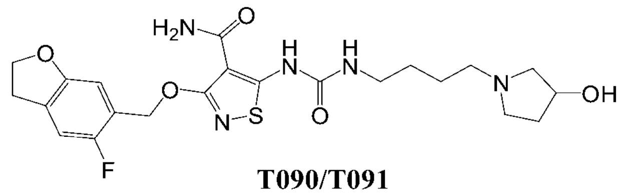

**T090/T091**

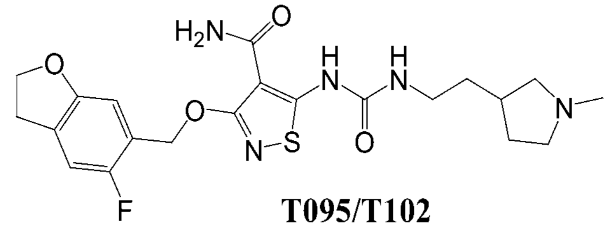

**T095/T102**

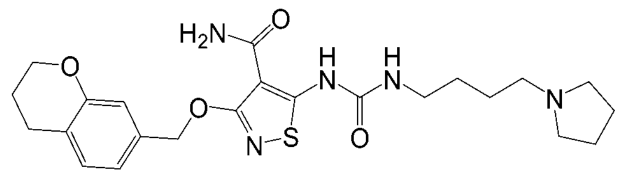

**T097**

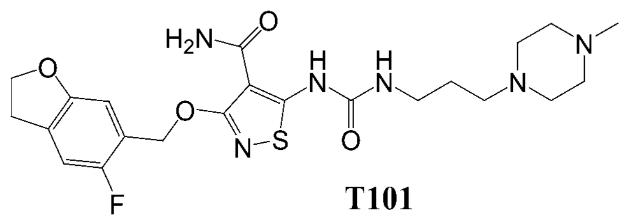

**T101**

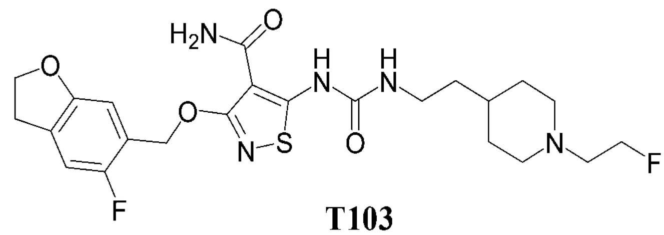

**T103**

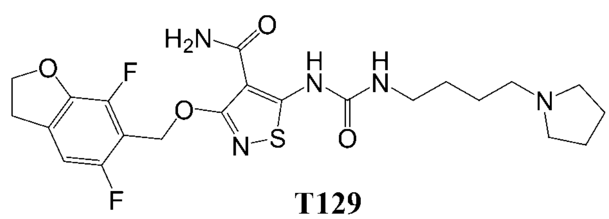

**T129**

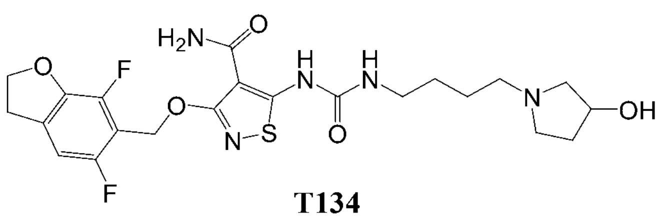

**T134**

.

In an embodiment (4) of the present invention, in Formula I, in particular Formula II or III, $L_1$ is -Cy-, and the B ring and $R_0$ are as defined above in the present invention.

In some embodiments of the present invention, $L_1$ is -Cy-, and $R_0$ is

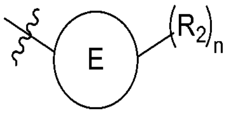.

In some embodiments of the present invention, $L_1$ is -Cy-, and $R_0$ is-$NR_{001}R_{002}$.

In some embodiments of the present invention, -Cy- is selected from:

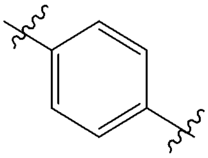,

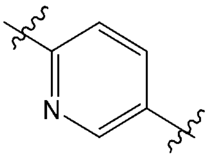, 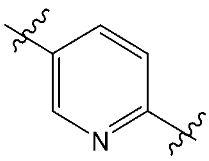, 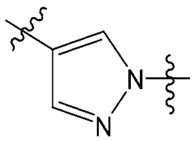, 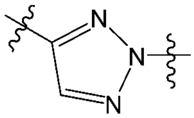, 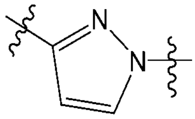,

and

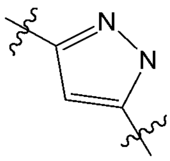.

In some embodiments of the present invention, $L_2$ is a single bond or $C_2$-$C_{10}$ alkylene, wherein one or more methylene units in alkylene are optionally and independently substituted by a group selected from: -N($R_4$)-, -N($R_4$)C(O)-, -C(O)N($R_4$)-, -N($R_4$)S(O)$_2$-, - S(O)$_2$N($R_4$)-, -O-, -C(O)-, -OC(O)-, -C(O)O-, -S-, -S(O)- or -S(O)$_2$, wherein one or more H atoms in alkylene are optionally and independently substituted by the following groups: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, and $C_4$-$C_{10}$ cycloalkyl alkyl; more specifically, $L_2$ is $C_2$-$C_6$ alkylene, wherein one or more metylene units in alkylene are optionally and independently substituted by a group selected from: -NH-, -O-, -C(O)-, -OC(O)-, -C(O)O-, and -S-. Specifically, $L_2$ may be selected from: a single bond,

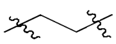, 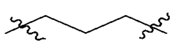,

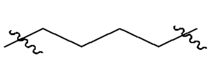, 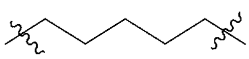, 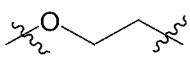, 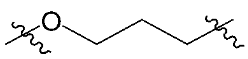, 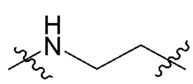,

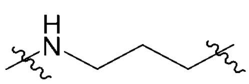, 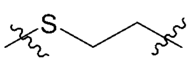, and 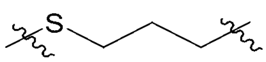.

Specifically, the

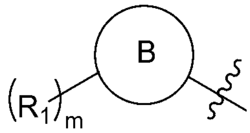

moiety may have a structure of:

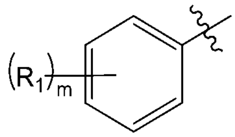,

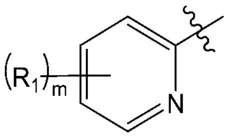, 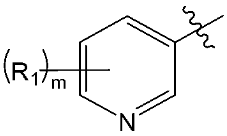, 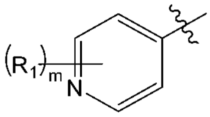, 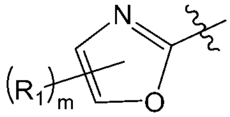, 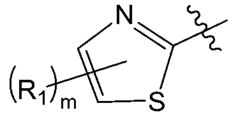,

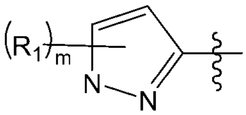, 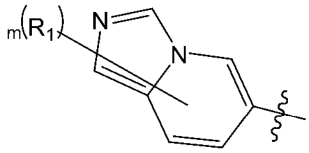, 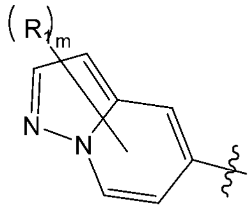, 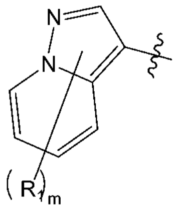,

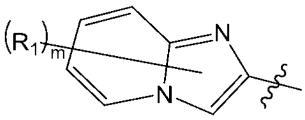, 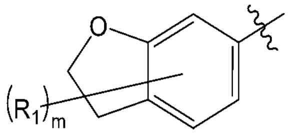, 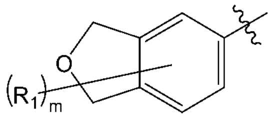, 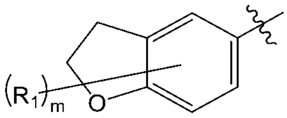,

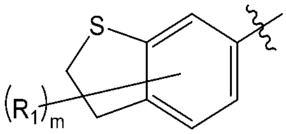, 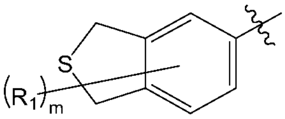, 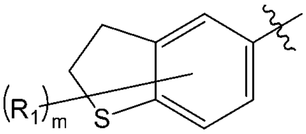,

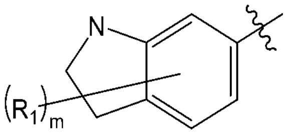, 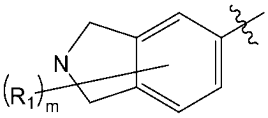, 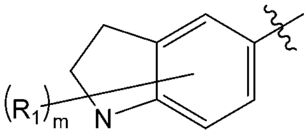, 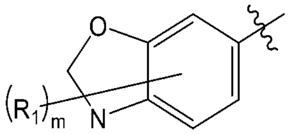,

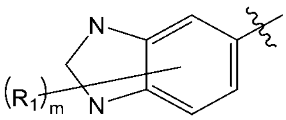, 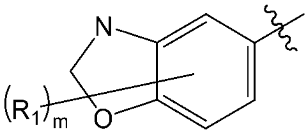, 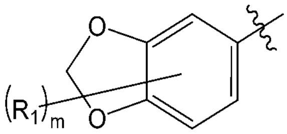, 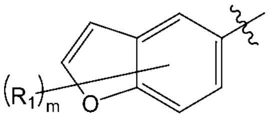,

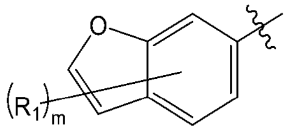, 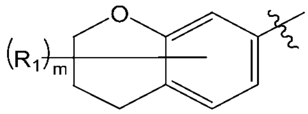, 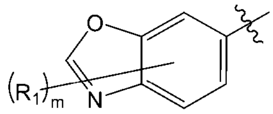, 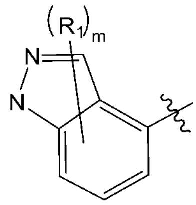,

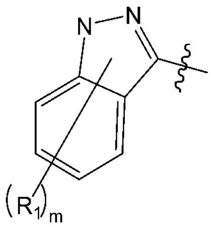, 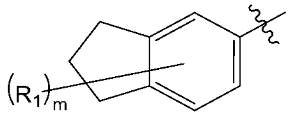, or 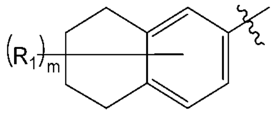.

In some embodiments of the present invention, the

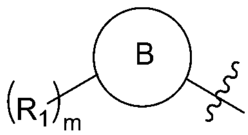

moiety has a structure of:

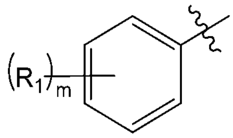;

more specifically, $R_1$ is selected from: $C_1$-$C_6$ alkyl, halogen, $C_1$-$C_6$ haloalkyl, cyano, nitro, azido, -O($C_0$-$C_{10}$ alkyl), -S($C_0$-$C_{19}$ alkyl), -C(O)($C_0$-$C_{10}$ alkyl), -C(O)O($C_0$-$C_{10}$ alkyl), -OC(O)($C_0$-$C_{10}$ alkyl), -N($C_0$-$C_{10}$ alkyl)$SO_2$($C_0$-$C_{10}$ alkyl), -$SO_2$N($C_0$-$C_{10}$ alkyl)($C_0$-$C_{10}$ alkyl), -N($C_0$-$C_{10}$ alkyl)C(O)($C_0$-$C_{10}$ alkyl), -C(O)N($C_0$-$C_{10}$ alkyl)($C_0$-$C_{10}$ alkyl), and -$SO_2$ ($C_0$-$C_{10}$ alkyl), wherein the alkyl is optionally substituted by a group selected from: halogen, cyano, hydroxyl, amino, $C_1$-$C_6$alkoxy, $C_1$-$C_6$ alkylamino, and $C_3$-$C_6$ cycloalkyl; more specifically, $R_1$ is selected from: halogen (e.g., F, Cl, and Br). In an embodiment of the present invention, the

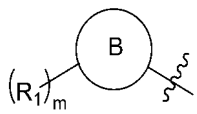

moiety is

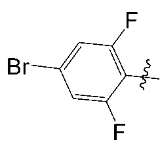.

In some embodiments of the present invention, the compound has a structure of:

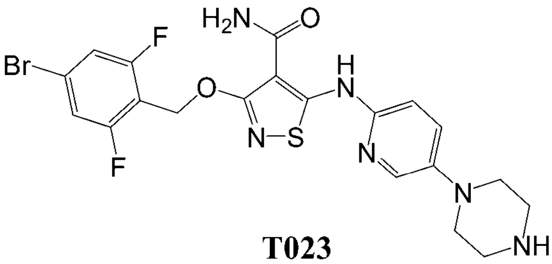

T023

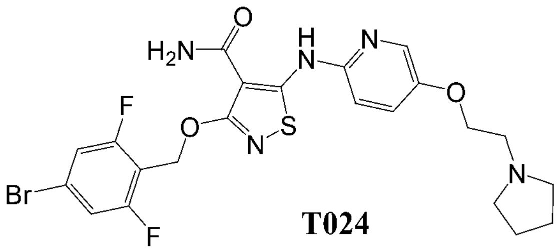

T024

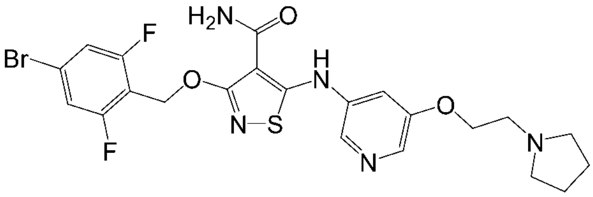

T045

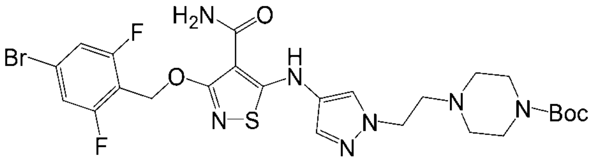

T046

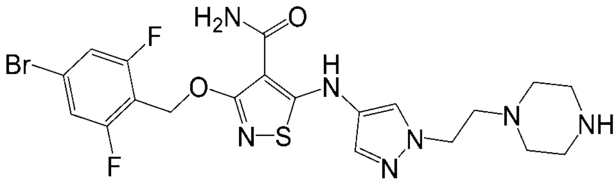

T050

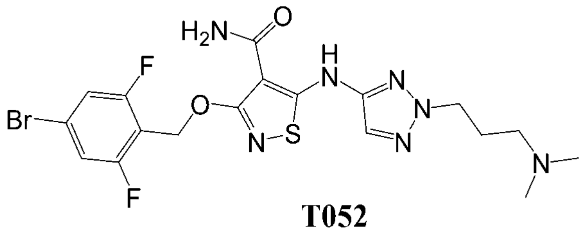

T052

The technical solutions of the present invention will be described clearly and completely below in conjunction with the embodiments of the present invention. Obviously, the embodiments described are only part of rather than all of the embodiments of the present invention. Based on the embodiments in the present invention, all other embodiments obtained by those ordinarily skilled in the art without making inventive efforts shall fall within the protection scope of the present invention.

Synthesis Examples:

Example 1: Synthesis of Compound T002

Synthesis route:

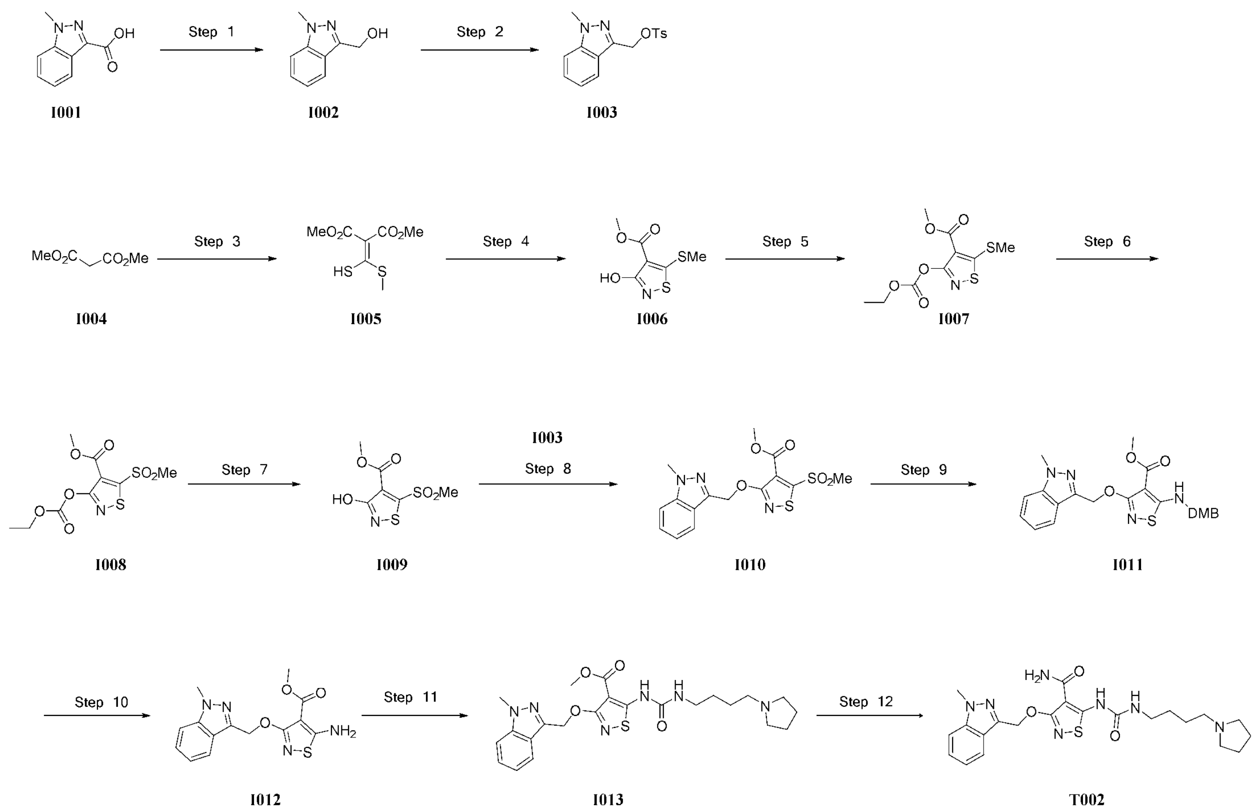


I001 I002 I003

I004 I005 I006 I007

I008 I009 I010 I011

I012 I013 T002

Step 1

(1-methyl-1H-indazol-3-yl)methanol

1-methyl-1H-indazol-3-carboxylic acid I001 (2.0 g, 11.4 mmol) was dissolved in anhydrous THF (20 mL), and cooled to -78°C under the protection of $N_2$, DIBAL-H (22.8 mL, 1M in hexane, 22.8 mmol) was added dropwise, and the reaction system was slowly warmed to room temperature and stirred for 1 hour. The reaction system was cooled to 0°C and quenched with 1M HCl aqueous solution (30 mL), the mixture was then subjected to liquid separation and extraction with ethyl acetate (30 mL x 2), and the organic phases were washed with saturated brine (50 mL), dried with anhydrous sodium sulfate, and concentrated under reduced pressure to obtain (1-methyl-1H-indazol-3-yl)methanol I002 (1.2 g, yellow liquid), with the yield of 65%.

For MS-ESI, the calculated value was $[M+H]^+$ 163.1, and the measurement was 162.9.

Step 2

(1-methyl-1H-indazol-3-yl)methyl 4-methylbenzenesulfonate

(1-Methyl-1H-indazol-3-yl)methanol I002 (1.2 g, 7.4 mmol), triethylamine (1.5 g, 14.8 mmol) and DMAP (90 mg, 0.7 mmol) were dissolved in DCM (30 mL) and then cooled to 0°C under the protection of $N_2$ (g), followed by the addition of TsCl(1.6 g, 8.2 mmol), and the reaction system was stirred at 0°C to react for 2 hours. The reaction mixture was sequentially washed with water (20 mL) and saturated brine (20 mL), dried with anhydrous sodium sulfate, and concentrated under reduced pressure to obtain a crude product, and the crude product was purified by chromatographic columns (PE:EA = 10:1) to obtain methyl (1-methyl-1H-indazol-3-yl)4-methyl benzenesulfonate I003 (400 mg, white solid), with the yield of 23%.

Step 3

Dimethyl 2-(mercapto(methylthio)methylene)malonate Under the protection of $N_2$, DBU(9.2 g, 60.6 mmol) was dissolved in ACN(30 mL), followed by the dropwise addition of dimethyl malonate I004 (4.0 g, 30.3 mmol) in the ice water bath, the mixture was then stirred in the ice water bath for 30 minutes, followed by the dropwise addition of carbon disulfide (2.3 g, 30.3 mmol), the mixture was stirred in the ice water bath to react for 1 hour, followed by the addition of dimethyl

sulfate (3.8 g, 30.3 mmol), and the reaction mixture was stirred at room temperature to react overnight. The reaction mixture was warmed to 25°C, and stirred to react for 2 hours to obtain a crude product of dimethyl 2-(mercapto(methylthio) methylene)malonate I005, which was then directly used in the next step.

Step 4

Methyl 3-hydroxy-5-(methylthio)isothiazole-4-carboxylate

Under the protection of $N_2$, sodium bicarbonate (3.0 g, 15.2 mmol) was dissolved in water (30 mL), followed by the dropwise addition of hydroxylamine-O-sulfonic acid (4.1 g, 36.4 mmol) in the ice water bath, the mixture was stirred for 30 minutes in the ice water bath, followed by the dropwise addition of the crude product of dimethyl 2-(mercapto(methylthio) methylene)malonate I005, and the reaction mixture was stirred at 25°C to react overnight. Concentration was carried out under reduced pressure to remove acetonitrile in the reaction system, which was then regulated with concentrated hydrochloric acid to PH = 1 and filtered by suction to obtain solids, and the solids were washed with water (20 mL) and EA/PE(10:1, 20 mL), and dried to obtain methyl 3-hydroxyl-5-(methylthio)isothiazol-4-carboxylate I006 (4.7 g, yellow solid), with hte two-step yield of 76%.

For MS-ESI, the calculated value was $[M+H]^+$ 206.3, and the measurement was 205.9.

$^1$H NMR(400 MHz, DMSO-$d_6$) $\delta$ = 11.90 (brs, 1H), 3.76 (s, 3H), 2.56 (s, 3H)

Step 5

Methyl 3-((ethoxycarbonyl)oxy)-5-(methylthio)isothiazole-4-carboxylate

Methyl 3-hydroxyl-5-(methylthio)isothiazol-4-carboxylate I006 (3.7 g, 18.0 mmol) and triethylamine (2.4 g, 23.5 mmol) were dissolved in DCM (30 mL), and cooled to 0°C under the protection of $N_2$, followed by the dropwise addition of ethyl chloroformate (2.3 g, 21.6 mmol), and the reaction mixture was stirred at room temperature to react for 2 hours. The reaction mixture was sequentially washed with water (20 mL) and saturated brine (20 mL), and dried with anhydrous sodium sulfate, followed by the addition of anhydrous acetonitrile (40 mL), and the reaction mixture was then concentrated under reduced pressure to remove dichloromethane, to obtain the solution of methyl 3-((ethoxycarbonyl) oxy)-5-(methylthio)isothiazol-4-carboxylate I007 in acetonitrile, which was directly used in the next step.

Step 6

Methyl 3-((ethoxycarbonyl)oxy)-5-(methylsulfonyl)isothiazole-4-carboxylate

Under the protection of $N_2$, the solution of methyl 3-((ethoxycarbonyl)oxy)-5-(methylthio)isothiazol-4-carboxylate I007 in acetonitrile was cooled to 0°C, followed by the addition of urea peroxide (4.6 g, 50.5 mmol) and then the dropwise addition of trifluoroacetic anhydride (10.6 g, 50.5 mmol), the reaction mixture was stirred at 0°C to react for 30 minutes, and the reaction mixture was quenched with sodium hydrogen sulfite (3.8 g, 36.1 mmol) and water (40 mL). The reaction mixture was concentrated under elevated pressure to remove acetonitrile, the aqueous solution was subjected to liquid separation and extraction with dichloromethane (30 mL x 2), the organic phases were sequentially washed with saturated brine (50 mL) and dried with anhydrous sodium sulfate, methanol (50 mL) was added, and the mixture was concentrated under reduced pressure to remove dichloromethane, to obtain the solution of methyl 3-((ethoxycarbonyl) oxy)-5-(methylsulfonyl)isothiazol-4-carboxylate I008 inmethanol, which was then directly used in the next step.

Step 7

Methyl 3-hydroxy-5-(methylsulfonyl)isothiazole-4-carboxylate

The solution of methyl 3-((ethoxycarbonyl)oxy)-5-(methylsulfonyl)isothiazol-4-carboxylate I008 in methanol was cooled to 0 °C, followed by the dropwise addition of 98% concentrated sulfuric acid (20 mL) in water (40 mL) to the reaction system, and the reaction mixture was stirred at 60 °C to react overnight. The reaction mixture was concentrated under reduced pressure to remove methanol, and was then subjected to liquid separation and extraction with dichloromethane (40 mL x 2), and the organic phases were sequentially washed with saturated brine (50 mL), dried with anhydrous sodium sulfate, and concentrated under reduced pressure to obtain a crude product, which was pulped using n-hexane (50 mL) to obtain methyl 3-hydroxyl-5-(methylsulfonyl)isothiazol-4-carboxylate I009 (3.1 g, yellow solid), with the three-step yield of 74%.

For MS-ESI, the calculated value was $[M+H]^+$ 238.0, and the measurement was 237.8.

$^1H$ NMR(400 MHz, DMSO-$d_6$) $\delta$ = 13.07 (brs, 1H), 3.86 (s, 3H), 3.57 (s, 3H).

Step 8

Methyl 3-((1-methyl-1H-indazol-3-yl)methoxy)-5-(methylsulfonyl)isothiazole-4-carboxylate

Methyl 3-hydroxyl-5-(methylsulfonyl)isothiazol-4-carboxylate I009 (400 mg, 1.7 mmol) and potassium carbonate (345 mg, 2.5 mmol) were dissolved in DMSO (10 mL), followed by the addition of methyl (1-methyl-1H-indazol-3-yl)4-methyl benzenesulfonate I003 (537 mg, 1.7 mmol), and the reaction system was stirred at 25 °C to react overnight. Water (50 mL) was added to the reaction mixture, which was then subjected to liquid separation and extraction with ethyl acetate (50 mL x 2), the organic phases were sequentially washed with saturated brine (60 mL), dried with anhydrous sodium sulfate, and concentrated under reduced pressure to obtain a crude product, and the crude product was purified by chromatographic columns (PE:EA = 1:1) to obtain methyl 3-((1-methyl-1H-indazol-3-yl)methoxy)-5-(methylsulfonyl) isothiazol-4-carboxylate I010 (350 mg, white solid), with the yield of 54%.
For MS-ESI, the calculated value was $[M+H]^+$ 382.1, and the measurement was 381.9.
$^1H$ NMR(400 MHz, DMSO-$d_6$) $\delta$ = 7.91 (d, *J=10.8* Hz, 1H), 7.49-7.44 (m, 1H), 7.91 (t, J=9.6 Hz, 1H), 5.83 (s, 2H), 4.08 (s,3H), 3.85 (s, 3H), 3.63 (s, 3H).

Step 9

Methyl 5-((2,4-dimethoxybenzyl)amino)-3-((1-methyl-1H-indazol-3-yl)methoxy)isothiazole-4-carboxylate

Methyl 3-((1-methyl-1H-indazol-3-yl)methoxy)-5-(methylsulfonyl)isothiazol-4-carboxylate I010 (270 mg, 0.7 mmol) was dissolved in THF(10 mL), followed by the addition of 2,4-dimethoxybenzylamine (1.2 g, 7.1 mmol), and the reaction system was stirred at 65 °C to react overnight. The reaction mixture was directly concentrated under reduced pressure to obtain a crude product, and the crude product was purified by chromatographic columns (PE:EA = 1:1) to obtain methyl 5-((2,4-dimethoxybenzyl)amino)-3-((1-methyl-1H-indazol-3-yl)methoxy)isothiazol-4-carboxylate I011 (310 mg, yellow solid), with the yield of 93%.
For MS-ESI, the calculated value was $[M+H]^+$ 469.2, and the measurement was 469.0.
$^1H$ NMR(400 MHz, DMSO-$d_6$) $\delta$ = 8.17 (t, J=5.4 Hz, 1H), 8.01 (d, J=8.4 Hz, 1H), 7.44-7.39 (m, 2H), 7.21-7.16 (m, 2H), 6.50-6.46 (m, 2H), 5.80 (s, 2H), 4.31(d, *J=6.0* Hz, 2H), 4.10 (s, 3H), 3.88 (s,3H), 3.84 (s, 3H), 3.78 (s, 3H).

Step 10

Methyl 5-amino-3-((1-methyl-1H-indazol-3-yl)methoxy)isothiazole-4-carboxylate

Methyl 5-((2,4-dimethoxybenzyl)amino)-3-((1-methyl-1H-indazol-3-yl)methoxy)isothiazol-4-carboxylate I011 (230 mg, 0.5 mmol) was dissolved in DCM/$H_2O$ (10/2 mL), followed by the addition of DDQ (446 mg, 2.0 mmol), and the reaction system was stirred at 0 °C to react for 30 minutes. The reaction mixture was sequentially washed with saturated sodium bicarbonate (10 mL) and saturated brine (10 mL), dried with anhydrous sodium sulfate, and concentrated under reduced pressure to obtain a crude product, and the crude product was purified by chromatographic columns to obtain a product methyl 5-amino-3-((1-methyl-1H-indazol-3-yl)methoxy)isothiazol-4-carboxylate 1012 (150 mg, yellow solid), with the yield of 96%.
For MS-ESI, the calculated value was $[M+H]^+$ 319.1, and the measurement was 319.0.

Step 11

Methyl 3-((1-methyl-1H-indazol-3-yl)methoxy)-5-(3-(4-(pyrrolidin-1-yl)butyl)ureido)isothiazole-4-carboxylate

4-(Pyrrolidin-1-yl)butyl-1-amine (100 mg, 0.7 mmol) was dissolved in anhydrous THF (5 mL), and then cooled to 0°C under the protection of $N_2$, followed by the addition of CDI(113 mg, 0.7 mmol), the mixture was stirred at 0°C to react for 40 minutes and then stirred at 25°C to react for 30 minutes, followed by the addition of DMSO (5 mL), the pressure was then reduced to remove THF, followed by the addition of methyl 5-amino-3-((1-methyl-1H-indazol-3-yl)methoxy)isothiazol-4-carboxylate I012 (150 mg, 0.5 mmol) and potassium carbonate (318 mg, 1.0 mmol), and the reaction system was stirred at 25 °C to react overnight. Water (20 mL) was added to the reaction mixture, which was then subjected to liquid separation and extraction with ethyl acetate (20 mL x 2), the organic phases were sequentially washed with saturated brine (60 mL), dried with anhydrous sodium sulfate, and concentrated under reduced pressure to obtain a crude product, and the crude product was purified by chromatographic columns (DCM:MeOH = 10:1) to obtain methyl 3-((1-methyl-1H-indazol-3-yl)methoxy)-5-(3-(4-(pyrrolidin-1-yl)butyl)ureido)isothiazol-4-carboxylate I013 (170 mg, yellow solid), with the yield of 70%.

For MS-ESI, the calculated value was $[M+H]^+$ 487.2, and the measurement was 487.0.

$^1H$ NMR(400 MHz, DMSO-$d_6$) δ = 10.40 (brs, 1H), 8.32 (t, J=4.2 Hz, 1H), 7.89 (d, J=8.0 Hz, 1H), 7.63 (d, J=8.8 Hz, 1H), 7.44-7.39 (m, 1H), 7.16 (d, J=7.6 Hz, 1H), 5.65 (s, 2H), 4.03 (s, 3H), 3.74 (s,3H), 3.57-3.40 (m, 2H), 3.19-3.14 (m, 2H), 3.12-3.02 (m, 2H), 2.90-2.87 (m, 2H), 2.06-1.80 (m, 4H), 1.66-1.62 (m, 2H), 1.54-1.47 (m, 2H).

Step 12

3-((1-methyl-1H-indazol-3-yl)methoxy)-5-(3-(4-(pyrrolidin-1-yl)butyl)ureido)isothiazole-4-carboxamide

In a microwave tube, methyl 3-((1-methyl-1H-indazol-3-yl)methoxy)-5-(3-(4-(pyrrolidin-1-yl)butyl)ureido)isothiazol-4-carboxylate I013 (170 mg, 0.35 mmol) was dissolved in anhydrous THF (1 mL), followed by the addition of ammonia methanol (7N, 4 mL), and the mixture was stirred at 50 °C to react for 96 hours. The reaction mixture was concentrated under reduced pressure to obtain a crude product, and the crude product was purified by the high-performance preparative chromatography ($NaHCO_3$) to obtain 3-((1-methyl-1H-indazol-3-yl)methoxy)-5-(3-(4-(pyrrolidin-1-yl)butyl)ureido)isothiazol-4-formamide T002 (80 mg, white solid), with the yield of 49%.

For MS-ESI, the calculated value was $[M+H]^+$ 472.2, and the measurement was 472.2.

$^1H$ NMR(400 MHz, DMSO-$d_6$) δ = 10.99 (s, 1H), 8.18 (t, J=4.4 Hz, 1H), 7.85 (d, J=8.4 Hz, 1H), 7.65 (d, J=8.8 Hz, 1H), 7.55 (s, 1H), 7.43 (t, J=8.4 Hz, 1H), 7.17 (t, J=8.0 Hz, 1H), 6.94 (s, 1H), 5.73 (s, 2H), 4.05 (s, 3H), 3.13-3.09 (m, 2H), 2.39-2.31 (m, 6H), 1.67-1.60 (m, 4H), 1.46-1.42 (m, 4H).

Example 2: Synthesis of Compound T003

Synthesis Route:

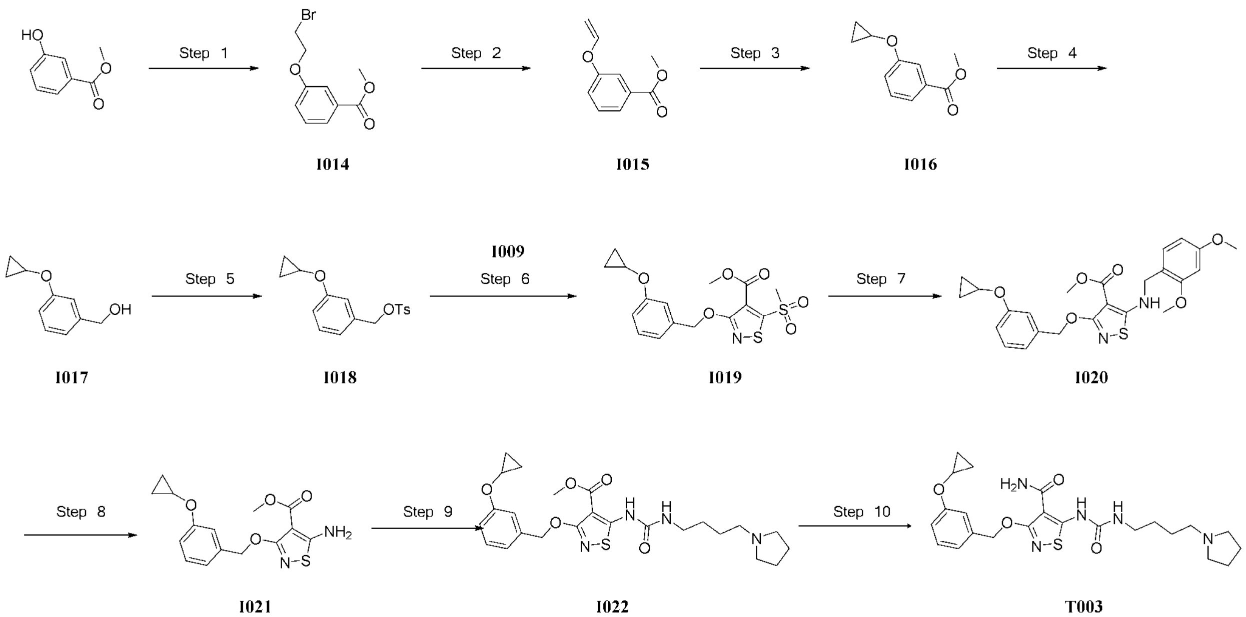


Step 1

Methyl 3-(2-bromoethoxy)benzoate

Under the protection of $N_2$, 3-hydroxylmethyl benzoate(10.0 g, 65.8 mmol) was dissolved in acetone (150 mL), followed by the addition of 1,2-dibromoethane (74.2 g, 0.39 mol) and potassium carbonate (18.2 g, 0.13 mol), and the mixture was stirred at 80°C to react overnight. Water (150 mL) was added to the reaction mixture, which was then subjected to liquid separation and extraction with ethyl acetate (150 mL x 2), the organic phases were sequentially washed with saturated brine (200 mL), dried with anhydrous sodium sulfate, and concentrated under reduced pressure to obtain a crude product, and the crude product was purified by chromatographic columns (PE:EA = 10:1) to obtain 3-(2-bromoethoxy)methyl benzoate I014 (6.1 g, colorless liquid, with the yield of 36%.

$^1H$ NMR(400 MHz, $CDCl_3$) δ = 7.59 (d, J=7.2 Hz, 1H), 7.49 (dd, J=2.0, 2.8 Hz, 1H), 7.28 (t, J=8.4 Hz, 1H), 7.06-7.04 (m, 1H), 4.26 (t, *J=6.0* Hz, 2H), 3.84 (s, 3H), 3.58 (t, J=6.0 Hz, 2H).

Step 2

Methyl 3-(vinyloxy)benzoate

Under the protection of $N_2$, methyl 3-(2-bromoethoxy)benzoate 1014(3.0 g, 11.6 mmol) was dissolved in anhydrous THF (20 mL), followed by the addition of 1M potassium tert-butoxide tetrahydrofuran solution (23 mL, 23.2 mmol), and the mixture was stirred at room temperature to react overnight. Water (50 mL) was added to the reaction mixture, which was then subjected to liquid separation and extraction with ethyl acetate (50 mL x 2), and the organic phases were sequentially washed with saturated brine (50 mL), dried with anhydrous sodium sulfate, and concentrated under reduced pressure to obtain 3-(vinyloxy)methyl benzoate I015( 1.7 g, colorless liquid, with the yield of 82%.

Step 3

Methyl 3-cyclopropoxybenzoate

Under the protection of $N_2$, methyl 3-(vinyloxy)benzoate I015 (1.7 g, 9.6 mmol) was dissolved in anhydrous DCM (20 mL), followed by the addition of diiodomethane (10.2 g, 38.2 mmol), the mixture was cooled to 0°C, followed by the dropwise addition of diethyl zinc (19 mL, 19.1 mmol), and the mixture was stirred at room temperature to react overnight. 1N dilute hydrochloric acid (20 mL) was added to the reaction mixture, which was then stratified, the aqueous phase was subjected to liquid separation and extraction with DCM (20 mL x 2), and the organic phases were combined, and then sequentially washed with saturated brine (50 mL), dried with anhydrous sodium sulfate, and concentrated under reduced pressure to obtain 3-cyclopropoxymethyl benzoate 1016(1.1 g of crude product, colorless liquid, which was then directly used in the next step.

Step 4

(3-cyclopropoxyphenyl)methanol

Under the protection of $N_2$, methyl 3-cyclopropoxy benzoate I016 (1.1 g, 6.8 mmol) was dissolved in anhydrous THF (10 mL), followed by the addition of LAH(327 mg, 8.6 mmol) in the ice water bath, and the mixture was stirred in the ice water bath to react for 30 minutes. The reaction mixture was quenched with water (0.3 mL), 15% sodium hydroxide (0.3 mL) and water (0.9 mL) were sequentially added, the mixture was dried with magnesium sulfate and filtered with diatomite, the solids were washed with ethyl acetate (20 mL), the filtrate was concentrated under reduced pressure to obtain a crude product, and the crude product was purified by chromatographic columns (PE:EA = 8:1) to obtain (3-cyclopropylphenyl) methanol I017 (870 mg, colorless liquid, with the yield of 92%.

For MS-ESI, the calculated value was $[M-OH]^+$ 147.2, and the measurement was 147.0.

$^1H$ NMR(400 MHz, $CDCl_3$) δ =7.26-7.24 (m, 1H), 7.06 (d, J=2.0 Hz, 1H), 6.98-6.94 (m, 2H), 4.67 (s, 2H), 3.76-3.71 (m, 1H), 0.81-0.76 (m, 4H).

Step 5

3-cyclopropoxybenzyl 4-methylbenzenesulfonate

(3-Cyclopropylphenyl)methanol I017(870 mg, 5.3 mmol), triethylamine (1.1 g, 10.6 mmol) and DMAP(65 mg, 0.5 mmol) were dissolved in DCM (10 mL) and then cooled to 0°C under the protection of $N_2$, followed by the addition of TsCl(1.3 g, 6.8 mmol), and the reaction system was stirred at 0°C to react for 2 hours. The reaction mixture was sequentially washed with water (10 mL) and saturated brine (10 mL), dried with anhydrous sodium sulfate, and concentrated under reduced pressure to obtain a crude product, and the crude product was purified by chromatographic columns (PE:EA = 10:1) to obtain 3-cyclopropoxybenzyl4-methylbenzene sulfonate I018 (481 mg, colorless liquid, with the yield of 29%.

Step 6

Methyl 3-((3-cyclopropoxybenzyl)oxy)-5-(methylsulfonyl)isothiazole-4-carboxylate

Methyl 3-hydroxyl-5-(methylsulfonyl)isothiazol-4-carboxylate I009 (235 mg, 1.0 mmol) and potassium carbonate (410 mg, 3.0 mmol) were dissolved in DMSO (5 mL), followed by the addition of 3-cyclopropoxybenzyl4-methylbenzene sulfonate I018 (537 mg, 1.7 mmol), and the reaction system was stirred at 25 °C to react overnight. Water (20 mL) was added to the reaction mixture, which was then subjected to liquid separation and extraction with ethyl acetate (25 mL x 2), the organic phases were sequentially washed with saturated brine (30 mL), dried with anhydrous sodium sulfate, and concentrated under reduced pressure to obtain a crude product, and the crude product was purified by chromatographic columns (PE:EA = 5:1) to obtain methyl 3-((3-cyclopropoxybenzyl)oxy)-5-(methylsulfonyl)isothiazol-4-carboxylate I019 (210 mg, yellow solid), with the yield of 55%.

For MS-ESI, the calculated value was $[M+H]^+$ 384.1, and the measurement was 383.8.

$^1H$ NMR(400 MHz, $CDCl_3$) $\delta$ = 7.31-7.27 (m, 1H), 7.13 (d, J=2.0 Hz, 1H), 7.05-7.01 (m, 2H), 5.46 (s,2H), 3.95 (s,3H), 3.75-3.72 (m, 1H), 3.47 (s, 3H), 0.80-0.76 (m, 4H).

Step 7

Methyl 3-((3-cyclopropoxybenzyl)oxy)-5-((2,4-dimethoxybenzyl)amino)isothiazole-4-carboxylate

Methyl 3-((3-cyclopropoxybenzyl)oxy)-5-(methylsulfonyl)isothiazol-4-carboxylate I019 (220 mg, 0.6 mmol) was dissolved in THF(5 mL), followed by the addition of 2,4-dimethoxybenzylamine (478 mg, 2.9 mmol), and the reaction system was stirred at 60 °C to react overnight. The reaction mixture was directly concentrated under reduced pressure to obtain a crude product, and the crude product was purified by chromatographic columns (PE:EA = 5:1) to obtain methyl 3-((3-cyclopropoxybenzyl)oxy)-5-((2,4-dimethoxybenzyl)amino)isothiazol-4-carboxylate I020 (182 mg, yellow solid), with the yield of 68%.

For MS-ESI, the calculated value was $[M+H]^+$ 471.1, and the measurement was 470.9.

Step 8

Methyl 5-amino-3-((3-cyclopropoxybenzyl)oxy)isothiazole-4-carboxylate

Methyl 5-((2,4-dimethoxybenzyl)amino)-3-((1-methyl-1H-indazol-4-yl)methoxy)isothiazol-4-carboxylate I020 (170 mg, 0.4 mmol) was dissolved in DCM/$H_2O$ (5/1 mL), followed by the addition of DDQ (327 mg, 1.4 mmol), and the reaction system was stirred at 0 °C to react for 30 minutes. DCM (15 mL) was added to the reaction mixture, which was then sequentially washed with saturated sodium bicarbonate aqueous solution (15 mL) and saturated brine (15 mL), dried with anhydrous sodium sulfate, and concentrated under reduced pressure to obtain a crude product, and the crude product was purified by chromatographic columns (PE:EA = 4:1) to obtain a product methyl 5-amino-3-((3-cyclopropoxybenzyl) oxy)isothiazol-4-carboxylate I021 (110 mg, yellow solid), with the yield of 94%.

For MS-ESI, the calculated value was $[M+H]^+$ 321.0, and the measurement was 320.9.

Step 9

Methyl 3-((3-cyclopropoxybenzyl)oxy)-5-(3-(4-(pyrrolidin-1-yl)butyl)ureido)isothiazole-4-carboxylate

4-(Pyrrolidin-1-yl)butyl-1-amine (66 mg, 0.5 mmol) was dissolved in anhydrous THF (5 mL) and then cooled to 0°C under the protection of $N_2$, followed by the addition of CDI(76 mg, 0.5 mmol), the mixture was stirred at 0°C to react for 40 minutes and then stirred at 25°C to react for 30 minutes, followed by the addition of DMSO (5 mL), the pressure was then reduced to remove THF, followed by the addition of methyl 5-amino-3-((3-cyclopropoxybenzyl)oxy)isothiazol-4-carboxylate I021 (100 mg, 0.3 mmol) and potassium carbonate (86 mg, 0.6 mmol), and the reaction system was stirred at 25 °C to react overnight. Water (20 mL) was added to the reaction mixture, which was then subjected to liquid separation and extraction with ethyl acetate (20 mL x 2), the organic phases were sequentially washed with saturated brine (30 mL), dried with anhydrous sodium sulfate, and concentrated under reduced pressure to obtain a crude product, and the crude product was purified by chromatographic columns (DCM:MeOH = 10:1) to obtain methyl 3-((3-cyclopropoxybenzyl) oxy)-5-(3-(4-(pyrrolidin-1-yl)butyl)ureido)isothiazol-4-carboxylate I022 (120 mg, white solid), with the yield of 79%.

For MS-ESI, the calculated value was $[M+H]^+$ 489.2, and the measurement was 489.0.

$^1H$ NMR(400 MHz, DMSO-$d_6$) $\delta$ = 10.41 (brs, 1H), 8.43 (t, J=4.2 Hz, 1H), 7.30 (t, J=8.0 Hz, 1H), 7.15-7.12 (m, 1H), 7.04-6.98 (m, 2H), 5.33 (s, 2H), 3.86-3.77 (m, 4H), 3.19-3.08 (m, 6H), 2.54 (overlap,2H), 1.98-1.86 (m, 4H), 1.71-1.65 (m, 2H), 1.54-1.49 (m, 2H), 0.87-0.80 (m, 2H), 0.66-0.62 (m, 2H).

Step 10

3-((3-cyclopropoxybenzyl)oxy)-5-(3-(4-(pyrrolidin-1-yl)butyl)ureido)isothiazole-4-carboxamide In a microwave tube, methyl 3-((3-cyclopropoxybenzyl)oxy)-5-(3-(4-(pyrrolidin-1-yl)butyl)ureido)isothiazol-4-carboxylate I022 (120 mg, 0.2 mmol) was dissolved in anhydrous THF (2 mL), followed by the addition of ammonia methanol (7N, 6 mL), and the mixture was stirred at 50 °C to react for 96 hours. The reaction mixture was concentrated under reduced pressure to obtain a crude product, and the crude product was purified by the high-performance preparative chromatography ($NaHCO_3$) to obtain 3-((3-cyclopropoxybenzyl)oxy)-5-(3-(4-(pyrrolidinyl-1-yl)butyl)ureido)isothiazol-4-formamide T003 (120 mg, white solid), with the yield of 59%.

For MS-ESI, the calculated value was $[M+H]^+$ 474.2, and the measurement was 474.2.

$^1H$ NMR(400 MHz, DMSO-$d_6$) δ = 8.16 (s, 1H), 7.62 (s, 1H), 7.31 (t, J=8.0 Hz, 1H), 8.17-8.14 (m, 1H), 7.10-7.00 (m, 3H), 5.38 (s, 2H), 3.83-3.79 (m, 1H), 3.13-3.09 (m, 2H), 2.40-2.32 (m, 6H), 1.67-1.63 (m, 4H), 1.46-1.44 (m, 4H), 0.79-0.75 (m, 2H), 0.65-0.61 (m, 2H).

Example 3: Synthesis of Compound T004

Synthesis Route:

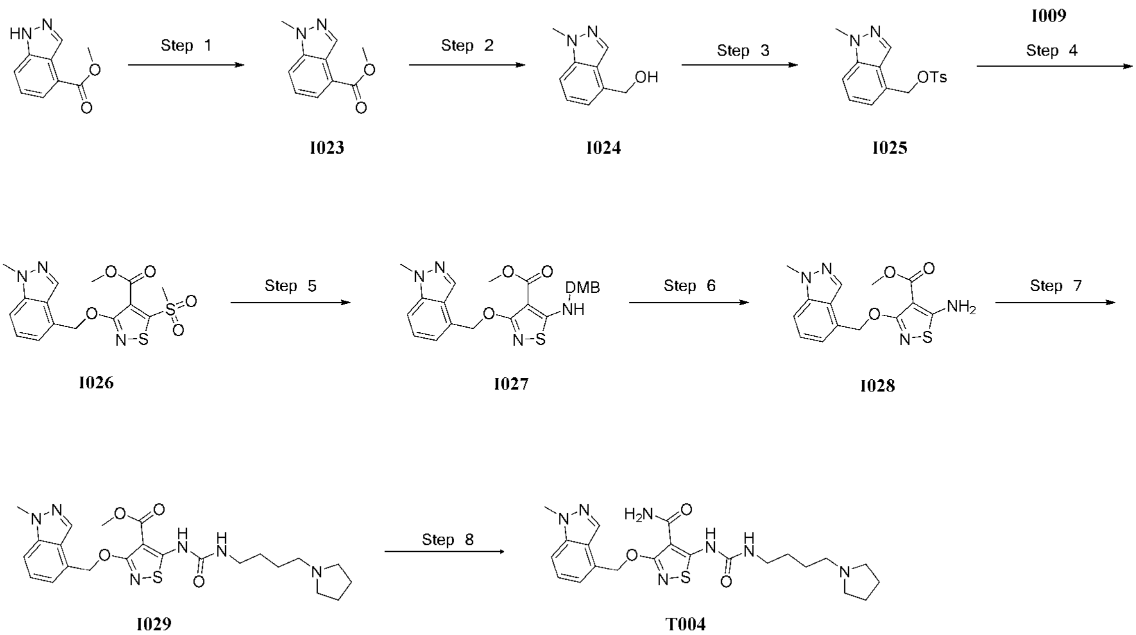


I023 I024 I025

I026 I027 I028

I029 T004

Step 1

Methyl 1-methyl-1H-indazole-4-carboxylate

Under the protection of $N_2$, methyl 1H-indazol-4-carboxylate (2.0 g, 11.4 mmol) was dissolved in DMF(15 mL), followed by the addition of 60% sodium hydride (684 mg, 17.1 mmol) in the ice water bath, the mixture was stirred in the ice water bath for 30 minutes, followed by the addition of iodomethane (2.4 g, 17.1 mmol), and the mixture was stirred at room temperature to react for 2 hours. The reaction mixture was quenched with ice water (50 mL), and then subjected to liquid separation and extraction with ethyl acetate (50 mL x 2), the organic phases were sequentially washed with saturated brine (60 mL), dried with anhydrous sodium sulfate, and concentrated under reduced pressure to obtain a crude product, and the crude product was purified by chromatographic columns (PE:EA = 1:1) to obtain methyl 1-methyl-1H-indazol-4-carboxylate I023 (1.2 g, white solid), with the yield of 55%.

For MS-ESI, the calculated value was $[M+H]^+$ 472.2, and the measurement was 472.2.

$^1H$ NMR(400 MHz, DMSO-$d_6$) δ = 8.47 (d, J=0.8 Hz, 1H), 7.92 (dd, *J=0.4,* 6.4 Hz, 1H), 7.60 (d, J=8.4 Hz, 1H), 7.44 (dd, J=7.2, 7.4 Hz, 1H), 4.11 (s, 3H), 4.01 (s, 3H).

Step 2

(1-methyl-1H-indazol-4-yl)methanol

Under the protection of $N_2$, methyl 1-methyl-1H-indazol-4-carboxylate I023 (1.3 g, 6.8 mmol) was dissolved in anhydrous THF (15 mL), followed by the addition of LAH(312 mg, 8.2 mmol) in the ice water bath, and the mixture was stirred in the ice water bath to react for 30 minutes. The reaction mixture was quenched with water (0.3 mL), followed by the sequential addition of 15% sodium hydroxide (0.3 mL) and water (0.9 mL), the mixture was dried with magnesium sulfate and filtered with diatomite to obtain solids, which were then washed with ethyl acetate (20 mL), and the filtrate was concentrated under reduced pressure to obtain (1-methyl-1H-indazol-4-yl)methanol I024 (900 mg, colorless liquid, yield: 82%).

For MS-ESI, the calculated value was $[M+H]^+$ 163.1, and the measurement was 163.0.

Step 3

(1-methyl-1H-indazol-4-yl)methyl 4-methylbenzenesulfonate

(1-Methyl-1H-indazol-4-yl)methanol I024 (900 mg, 5.6 mmol), triethylamine (1.1 g, 11.2 mmol) and DMAP(68 mg, 0.6 mmol) were dissolved in DCM (15 mL) and then cooled to 0°C under the protection of $N_2$, followed by the addition of TsCl(1.3 g, 6.7 mmol), and the reaction system was stirred at 0°C to react for 2 hours. The reaction mixture was sequentially washed with water (10 mL) and saturated brine (10 mL), dried with anhydrous sodium sulfate, and concentrated under reduced pressure to obtain a crude product, and the crude product was purified by chromatographic columns (PE:EA = 10:1) to obtain (1-methyl-1H-indazol-3-yl) methyl 4-methyl benzenesulfonate I025 (400 mg, white solid), with the yield of 32%.

Step 4

Methyl 3-((1-methyl-1H-indazol-4-yl)methoxy)-5-(methylsulfonyl)isothiazole-4-carboxylate

Methyl 3-hydroxyl-5-(methylsulfonyl)isothiazol-4-carboxylate I009 (400 mg, 1.7 mmol) and potassium carbonate (466 mg, 3.38 mmol) were dissolved in DMSO (10 mL), followed by the addition of methyl (1-methyl-1H-indazol-4-yl) 4-methyl benzenesulfonate I025 (537 mg, 1.7 mmol), and the reaction system was stirred at 25 °C to react overnight. Water (30 mL) was added to the reaction mixture, which was then subjected to liquid separation and extraction with ethyl acetate (40 mL x 2), the organic phases were sequentially washed with saturated brine (50 mL), dried with anhydrous sodium sulfate, and concentrated under reduced pressure to obtain a crude product, and the crude product was purified by chromatographic columns (PE:EA = 1:1) to obtain methyl 3-((1-methyl-1H-indazol-4-yl)methoxy)-5-(methylsulfonyl) isothiazol-4-carboxylate I026 (410 mg, white solid), with the yield of 63%.

For MS-ESI, the calculated value was $[M+H]^+$ 382.1, and the measurement was 381.9.

$^1H$ NMR(400 MHz, DMSO-$d_6$) $\delta$ = 8.21 (s, 1H), 7.44-7.42 (m, 2H), 7.26-7.24 (m, 1H), 4.14 (s, 3H), 3.99 (s,3H), 3.52 (s, 3H).

Step 5

Methyl methyl 5-((2,4-dimethoxybenzyl)amino)-3-((1-methyl-1H-indazol-4-yl)methoxy)isothiazole-4-carboxylate

Methyl 3-((1-methyl-1H-indazol-4-yl)methoxy)-5-(methylsulfonyl)isothiazol-4-carboxylate I026 (510 mg, 1.3 mmol) was dissolved in THF(10 mL), followed by the addition of 2,4-dimethoxybenzylamine (2.2 g, 13.4 mmol), and the reaction system was stirred at 65 °C to react overnight. The reaction mixture was directly concentrated under reduced pressure to obtain a crude product, and the crude product was purified by chromatographic columns (PE:EA = 1:1) to obtain methyl 5-((2,4-dimethoxybenzyl)amino)-3-((1-methyl-1H-indazol-4-yl)methoxy)isothiazol-4-carboxylate I027 (450 mg, yellow solid), with the yield of 72%.

For MS-ESI, the calculated value was $[M+H]^+$ 469.2, and the measurement was 469.0.

Step 6

Methyl 5-amino-3-((1-methyl-1H-indazol-4-yl)methoxy)isothiazole-4-carboxylate

Methyl 5-((2,4-dimethoxybenzyl)amino)-3-((1-methyl-1H-indazol-4-yl)methoxy)isothiazol-4-carboxylate I027

(430 mg, 0.9 mmol) was dissolved in DCM/$H_2O$ (10/2 mL), followed by the addition of DDQ (834 mg, 3.7 mmol), and the reaction system was stirred at 0 °C to react for 30 minutes. The reaction mixture was sequentially washed with saturated sodium bicarbonate aqueous solution (15 mL) and saturated brine (15 mL), dried with anhydrous sodium sulfate, and concentrated under reduced pressure to obtain a crude product, and the crude product was purified by chromatographic columns to obtain a product methyl 5-amino-3-(((1-methyl-1H-indazol-4-yl)methoxy)isothiazol-4-carboxylate I028 (240 mg, yellow solid), with the yield of 84%.

For MS-ESI, the calculated value was $[M+H]^+$ 319.1, and the measurement was 319.0.

Step 7

Methyl3-((1-methyl-1H-indazol-4-yl)methoxy)-5-(3-(4-(pyrrolidin-1-yl)butyl)ureido)isothiazole-4-carboxylate

4-(Pyrrolidin-1-yl)butyl-1-amine (170 mg, 1.2 mmol) was dissolved in anhydrous THF (5 mL) and then cooled to 0°C under the protection of $N_2$, followed by the addition of CDI(194 mg, 1.2 mmol), the mixture was stirred at 0°C to react for 40 minutes and then stirred at 25°C to react for 30 minutes, followed by the addition of DMSO (5 mL), the pressure was then reduced to remove THF, followed by the addition of methyl 5-amino-3-(((1-methyl-1H-indazol-4-yl)methoxy)isothiazol-4-carboxylate I028 (240 mg, 0.8 mmol) and potassium carbonate (221 mg, 1.6 mmol), and the reaction system was stirred at 25 °C to react overnight. Water (20 mL) was added to the reaction mixture, which was then subjected to liquid separation and extraction with ethyl acetate (20 mL x 2), the organic phases were sequentially washed with saturated brine (60 mL), dried with anhydrous sodium sulfate, and concentrated under reduced pressure to obtain a crude product, and the crude product was purified by chromatographic columns (DCM:MeOH = 10:1) to obtain methyl 3-((1-methyl-1H-indazol-4-yl)methoxy)-5-(3-(4-(pyrrolidin-1-yl)butyl)ureido)isothiazol-4-carboxylate I029 (210 mg, yellow solid), with the yield of 57%.

For MS-ESI, the calculated value was $[M+H]^+$ 487.2, and the measurement was 487.0.

$^1H$ NMR(400 MHz, DMSO-$d_6$) $\delta$ = 10.41 (brs, 1H), 8.36 (t, J=4.2 Hz, 1H), 8.19 (d, J=0.8 Hz, 1H), 7.59 (d, J=8.0 Hz, 1H), 7.40-7.36 (m, 1H), 7.20 (d, *J*=6.8 Hz, 1H), 5.67 (s, 2H), 4.05 (s, 3H), 3.81 (s,3H), 3.48-3.40 (m, 2H), 3.19-3.07 (m, 2H), 3.01-2.96 (m, 2H), 2.94-2.85 (m, 2H), 1.92-1.84 (m, 4H), 1.71-1.62 (m, 2H), 1.54-1.47 (m, 2H).

Step 8

3-((1-methyl-1H-indazol-4-yl)methoxy)-5-(3-(4-(pyrrolidin-1-yl)butyl)ureido)isothiazole-4-carboxamide

In a microwave tube, methyl 3-((1-methyl-1H-indazol-4-yl)methoxy)-5-(3-(4-(pyrrolidin-1-yl)butyl)ureido)isothiazol-4-carboxylate I029 (210 mg, 0.4 mmol) was dissolved in anhydrous THF (2 mL), followed by the addition of ammonia methanol (7N, 6 mL), and the mixture was stirred at 50 °C to react for 96 hours. The reaction mixture was concentrated under reduced pressure to obtain a crude product, and the crude product was purified by the high-performance preparative chromatography ($NaHCO_3$) to obtain 3-((1-methyl-1H-indazol-4-yl)methoxy)-5-(3-(4-(pyrrolidin-1-yl)butyl)ureido)isothiazol-4-formamide T004 (120 mg, white solid), with the yield of 59%.

For MS-ESI, the calculated value was $[M+H]^+$ 472.2, and the measurement was 472.2.

$^1H$ NMR(400 MHz, DMSO-$d_6$) $\delta$ = 11.01 (s, 1H), 8.19-8.16 m, 2H), 7.64 (d, J=8.4 Hz, 1H), 7.58 (s, 1H), 7.39 (t, *J*=7.2 Hz, 1H), 7.22 (d, *J=7.2* Hz, 1H), 7.01 (s, 1H), 5.73 (s, 2H), 4.06 (s, 3H), 3.13-3.09 (m, 2H), 2.39-2.35 (m, 6H), 1.67-1.60 (m, 4H), 1.46-1.42 (m, 4H).

Example 4: Synthesis of Compound T005

Synthesis Route:

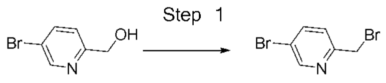


1030

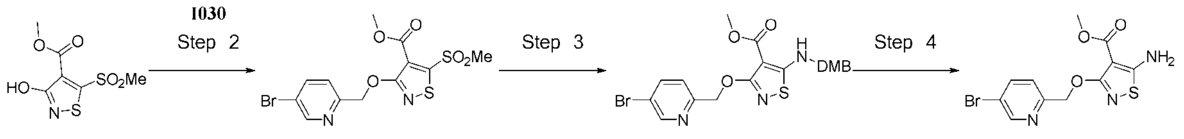


1009 1031 1032 1033

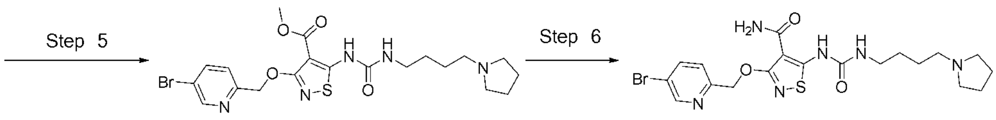


1034 T005

Step 1

5-bromo-2-(bromomethyl)pyridine

(5-Bromopyridin-2-yl)methanol (2.5 g, 13 mmol) was dissolved in THF (30 mL) and then cooled to 0°C, followed by the addition of $PBr_3$ (10.8 g, 39 mmol), and the reaction system was stirred at room temperature to react for about 16 hours. The reaction mixture was poured into water (60 mL), and extracted with ethyl acetate (60 mL x 3), and the organic phases were combined, sequentially washed with saturated sodium bicarbonate aqueous solution (50 mL) and saturated brine (50 mL), dried with anhydrous sodium sulfate, and concentrated under reduced pressure to obtain a crude product 5-bromo-2-(bromomethyl)pyridine I030 (4 g, crude product) as a red oily liquid product. The product was verified by LCMS.

Step 2

Methyl 3-((5-bromopyridin-2-yl)methoxy)-5-(methylsulfonyl)isothiazole-4-carboxylate

Methyl 3-hydroxyl-5-(methylsulfonyl)isothiazol-4-carboxylate I009 (2 g, 8.4 mmol) and potassium carbonate (2.3 g, 16.8 mmol) were dissolved in DMSO (30 mL), followed by the addition of 5-bromo-2-(bromomethyl)pyridine I030 (2.3 g, 9.2 mmol), and the reaction system was stirred at 25 °C to react for 5 hours. Water (50 mL) was added to the reaction mixture, which was then subjected to liquid separation and extraction with ethyl acetate (50 mL x 3), the organic phases were washed with saturated brine (50 mL), dried with anhydrous sodium sulfate, and concentrated under reduced pressure to obtain a crude product, and the crude product was purified by chromatographic columns (PE/EA = 5/1-2/1) to obtain a white solid product methyl 3-((5-bromopyridin-2-yl)methoxy)-5-(methylsulfonyl)isothiazol-4-carboxylate I031 (2 g, yield: 58.8%). The product was verified by LCMS.

Step 3

Methyl 3-((5-bromopyridin-2-yl)methoxy)-5-((2,4-dimethoxybenzyl)amino)isothiazole-4-carboxylate

Methyl 3-((5-bromopyridin-2-yl)methoxy)-5-(methylsulfonyl)isothiazol-4-carboxylate I031 (2 g, 4.9 mmol) was dissolved in THF(20 mL), followed by the addition of 2,4-dimethoxybenzylamine (8.2 g, 49 mmol), and the reaction system was stirred at 65 °C to react overnight. The reaction mixture was directly concentrated under reduced pressure to obtain a crude product, and the crude product was purified by chromatographic columns (PE/EA = 10/1-5/1) to obtain a yellow oily liquid methyl 3-((5-bromopyridin-2-yl)methoxy)-5-((2,4-dimethoxybenzyl)amino)isothiazol-4-carboxylate I032 (2.5 g, crude product).

The product was verified by LCMS.

Step 4

Methyl 5-amino-3-((5-bromopyridin-2-yl)methoxy)isothiazole-4-carboxylate

Methyl 3-((5-bromopyridin-2-yl)methoxy)-5-((2,4-dimethoxybenzyl)amino)isothiazol-4-carboxylate I032 (2.5 g, 5.1 mmol) was dissolved in DCM/$H_2O$ (10/2 mL), followed by the addition of DDQ (4.6 g, 20.4 mmol), and the reaction system was stirred at 0 °C to react for 30 minutes. The reaction mixture was sequentially washed with saturated sodium bicarbonate aqueous solution (50 mL) and saturated brine (50 mL), dried with anhydrous sodium sulfate, and concentrated under reduced pressure to obtain a crude product, and the crude product was purified by chromatographic columns (PE/EA = 5/1-3/1) to obtain a yellow solid product methyl 5-amino-3-((5-bromopyridin-2-yl)methoxy)isothiazol-4-carboxylate I033 (1.3 g, yield: 74.7%).

The product was verified by LCMS.

Step 5

Methyl 3-((5-bromopyridin-2-yl)methoxy)-5-(3-(4-(pyrrolidin-1-yl)butyl)ureido)isothiazole-4-carboxylate

4-(pyrrolidin-1-yl)butyl-1-amine (170 mg, 1.2 mmol) was dissolved in anhydrous THF (5 mL) and then cooled to 0°C under the protection of $N_2$, followed by the addition of CDI (194 mg, 1.2 mmol), the mixture was stirred at 0°C to react for 40 minutes and then stirred at 25°C to react for 30 minutes, followed by the addition of DMSO (5 mL), the pressure was then reduced to remove THF, followed by the addition of methyl 5-amino-3-((5-bromopyridin-2-yl)methoxy)isothiazol-4-carboxylate I033 (1.3 g, 3.8 mmol) and potassium carbonate (1.1 g, 7.6 mmol), and the reaction system was stirred at 25 °C to react overnight. Water (50 mL) was added to the reaction mixture, which was then subjected to liquid separation and extraction with ethyl acetate (50 mL x 3), the organic phases were washed with saturated brine (50 mL), dried with anhydrous sodium sulfate, and concentrated under reduced pressure to obtain a crude product, and the crude product was purified by chromatographic columns (PE/EA = 1/1-1/2) to obtain a colorless liquid product methyl 3-((5-bromopyridin-2-yl) methoxy)-5-(3-(4-(pyrrolidin-1-yl)butyl)ureido)isothiazol-4-carboxylate I034 (700 mg, yield: 36.6%).

The product was verified by LCMS.

Step 6

3-((5-bromopyridin-2-yl)methoxy)-5-(3-(4-(pyrrolidin-1-yl)butyl)ureido)isothiazole-4-carboxamide

In a microwave tube, methyl 3-((5-bromopyridin-2-yl)methoxy)-5-(3-(4-(pyrrolidin-1-yl)butyl)ureido)isothiazol-4-carboxylate I034 (300 mg, 0.59 mmol) was dissolved in $NH_3$/MeOH (4 mL, 13 mol/L), and the mixture was stirred at 60 °C to react for 16 hours. The reaction mixture was concentrated under reduced pressure to obtain a crude product, and the crude product was purified by the high-performance preparative chromatography to obtain a white solid product 3-((5-bromopyridin-2-yl)methoxy)-5-(3-(4-(pyrrolidin-1-yl)butyl)ureido)isothiazol-4-formamide

T005 (29 mg, yield: 10%).

The product was verified by LCMS, H-NMR and C-NMR.

$^1$H NMR (400 MHz, $CD_3OD$) δ: 8.68 (d, 1H, J=2.0Hz), 8.04 (dd, 1H, J=8.0, 2.0Hz), 7.48 (d, 1H, J=8.0Hz), 5.55 (s, 2H), 3.28 (t, 2H, J=6.0Hz), 2.57-2.65 (m, 6H), 1.84-1.87 (m, 4H), 1.61-1.63 (m, 4H).

$^{13}$C NMR (100 MHz, $CD_3$;OD) δ: 169.02, 165.79, 154.81, 154.75, 149.92, 139.82, 123.61, 119.67, 69.14, 55.76, 53.57, 39.53, 27.47, 25.49, 22.74.

Example 5: Synthesis of Compound T006

Synthesis Route:

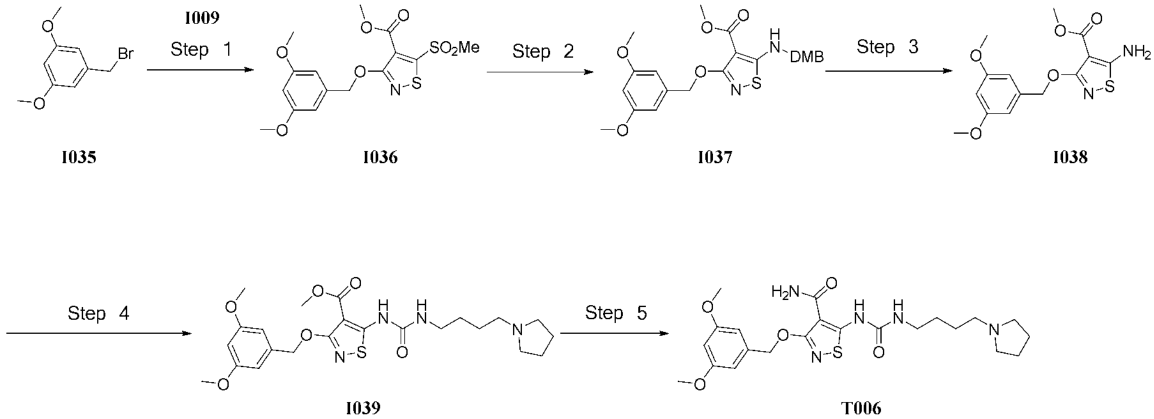


I035 I036 I037 I038 I039 T006

Step 1

Methyl 3-((3,5-dimethoxybenzyl)oxy)-5-(methylsulfonyl)isothiazole-4-carboxylate

Methyl 3-hydroxyl-5-(methylsulfonyl)isothiazol-4-carboxylate I009 (1.0 g, 4.21 mmol) and potassium carbonate (1.17 g, 8.43 mmol) was dissolved in DMSO (10 mL), followed by the addition of 1-(bromomethyl)-3,5-dimetoxybenzene I035 (1.06 g, 4.64 mmol), and the reaction system was stirred at 25 °C to react for 16 hours. Water (30 mL) was added to the reaction mixture, which was then subjected to liquid separation and extraction with ethyl acetate (50 mL x 3), the organic phases were sequentially washed with water (50 mL) and saturated brine (50 mL), dried with anhydrous sodium sulfate, and concentrated under reduced pressure to obtain a crude product, and the crude product was purified by chromatographic columns (PE/EA = 10/1~2/1) to obtain a yellow solid product methyl 3-((3,5-dimethoxybenzyl)oxy)-5-(methylsulfonyl)isothiazol-4-carboxylate I036 (0.5 g, yield: 30.6%).
The product was verified by LCMS and H-NMR.
$^1$H NMR (400 MHz, $CDCl_3$) δ: 6.63 (d, 2H, J=2.0Hz), 6.45 (t, 1H, J=2.4Hz), 5.45 (s, 2H), 3.99 (s, 3H), 3.89 (s, 6H), 3.50 (s, 3H).

Step 2

Methyl 5-((2,4-dimethoxybenzyl)amino)-3-((3,5-dimethoxybenzyl)oxy)isothiazole-4-carboxylate

Methyl 3-((3,5-dimethoxybenzyl)oxy)-5-(methylsulfonyl)isothiazol-4-carboxylate I036 (0.5 g, 1.29 mmol) was dissolved in THF(5 mL), followed by the addition of 2,4-dimethoxybenzylamine (2.16 g, 12.9 mmol), and the reaction system was stirred at 60 °C to react for 1 hour. 1N HCL solution (10 mL) was added to the reaction mixture, which was then extracted with ethyl acetate (10 mL x 3), and the organic phases were combined, washed with saturated brine (10 mL), then dried with anhydrous sodium sulfate, and concentrated under reduced pressure to obtain a white solid crude product methyl 5-((2,4-dimethoxybenzyl)amino)-3-((3,5-dimethoxybenzyl)oxy)isothiazol-4-carboxylate I037 (0.6 g, yield: 98.0%).
The product was verified by LCMS and H-NMR.
$^1$H NMR (400 MHz, $CDCl_3$) δ: 8.16 (t, 1H, J=6.0Hz), 7.18 (d, 1H, J=8.0Hz), 6.65 (d, 2H, J=2.0Hz), 6.45-6.50 (m, 2H), 6.41 (t, 1H, J=2.0Hz), 5.39 (s, 2H), 4.29 (d, 1H, J=6.0Hz), 3.87 (s, 3H), 3.85 (s, 3H), 3.82-3.83 (m, 9H).

Step 3

Methyl 5-amino-3-((3,5-dimethoxybenzyl)oxy)isothiazole-4-carboxylate

Methyl 5-((2,4-dimethoxybenzyl)amino)-3-((3,5-dimethoxybenzyl)oxy)isothiazol-4-carboxylate I037 (0.6 g, 1.26 mmol) was dissolved in DCM/$H_2O$ (6/0.6 mL), followed by the portionwise addition of DDQ (0.57 g, 2.53 mmol), and the reaction system was stirred at room temperature to react for 1 hour. Water (10 mL) was added to the reaction mixture, which was then extracted with DCM (10 mL x 3), the organic phases were combined, sequentially washed with water (10 mL x 2) and saturated brine (20 mL), dried with anhydrous sodium sulfate, and concentrated under reduced pressure to obtain a crude product, and the crude product was purified by chromatographic columns (PE/EA = 10/1~2/1) to obtain a yellow solid product methyl 5-amino-3-((3,5-dimethoxybenzyl)oxy)isothiazol-4-carboxylate I038 (0.35 g, yield: 85.3%).

The product was verified by LCMS and H-NMR.
$^1$H NMR (400 MHz, $CDCl_3$) δ: 6.66 (d, 2H, J=2.0Hz), 6.46 (s, 2H), 6.43 (t, 1H, J=2.4Hz), 5.41 (s, 2H), 3.89 (s, 3H), 3.83 (s, 6H).

Step 4

Methyl 3-((3,5-dimethoxybenzyl)oxy)-5-(3-(4-(pyrrolidin-1-yl)butyl)ureido)isothiazole-4-carboxylate

4-(Pyrrolidin-1-yl)butyl-1-amine (230 mg, 1.62 mmol) was dissolved in anhydrous THF (5 mL) and then cooled to 0°C under the protection of $N_2$, followed by the addition of CDI (262 mg, 1.62 mmol), the mixture was stirred at room temperature to react for 30 minutes, followed by the addition of DMSO (5 mL), the pressure was then reduced to remove THF, followed by the addition of methyl 5-amino-3-((3,5-dimethoxybenzyl)oxy)isothiazol-4-carboxylate I038 (350 mg, 1.08 mmol) and potassium carbonate (298 mg, 2.16 mmol), and the reaction system was stirred at 25 °C to react for 16 hours. Water (20 mL) was added to the reaction mixture, which was then subjected to liquid separation and extraction with ethyl acetate (10 mL x 3), the organic phases were sequentially washed with water (10 mL) and saturated brine (20 mL), dried with anhydrous sodium sulfate, and concentrated under reduced pressure to obtain a crude product, and the crude product was purified by chromatographic columns (DCM/MeOH = 50/1~10/1) to obtain a colorless liquid product methyl 3-((3,5-dimethoxybenzyl)oxy)-5-(3-(4-(pyrrolidin-1-yl)butyl)ureido)isothiazol-4-carboxylate I039 (300 mg, yield: 56.4%).
The product was verified by LCMS and H-NMR.
$^1$H NMR (400 MHz, $CDCl_3$) δ: 10.43 (s, 1H), 6.67 (d, 2H, J=2.0Hz), 6.41 (t, 1H, J=2.0Hz), 5.41 (s, 2H), 3.91 (s, 3H), 3.82 (s, 6H), 3.39 (t, 2H, J=2.0Hz), 3.08 (s, 3H), 2.94 (t, 2H, J=7.2Hz), 2.06-2.11 (m, 5H), 1.89-1.93 (m, 2H), 1.69-1.72 (m, 2H).

Step 5

3-((3,5-dimethoxybenzyl)oxy)-5-(3-(4-(pyrrolidin-1-yl)butyl)ureido)isothiazole-4-carboxamide

In a microwave tube, methyl 3-((3,5-dimethoxybenzyl)oxy)-5-(3-(4-(pyrrolidin-1-yl)butyl)ureido)isothiazol-4-carboxylate I039 (300 mg, 0.61 mmol) was dissolved in THF (3 mL) and $NH_3$/MeOH (3 mL, 13 mol/L), and the mixture was stirred at 60 °C to react for 48 hours. The reaction mixture was concentrated under reduced pressure to obtain a crude product, and the crude product was purified by the high-performance preparative chromatography (DCM/MeOH = 10/1, 0.1% NH3.H2O) to obtain a white solid product 3-((3,5-dimethoxybenzyl)oxy)-5-(3-(4-(pyrrolidin-1-yl)butyl)ureido)isothiazol-4-formamide T006 (50 mg, yield: 17.2%).
The product was verified by LCMS, H-NMR and C-NMR.
$^1$H NMR (400 MHz, $CDCl_3$) δ: 10.97 (br s, 1H), 7.80 (br s, 1H), 7.20 (s, 1H), 6.59 (d, 2H, J=2.0Hz), 6.46 (t, 1H, J=2.4Hz), 5.87 (br s, 1H), 5.40 (s, 2H), 3.81 (s, 6H), 3.32-3.34 (m, 2H), 2.63-2.73 (m, 6H), 1.91 (s, 4H), 1.68-1.74 (m, 4H).
$^{13}$C NMR (100 MHz, $CDCl_3$) δ: 169.53, 165.84, 161.86, 161.05, 154.18, 138.19, 106.18, 100.26, 70.37, 55.65, 55.41, 53.88, 27.74, 25.83, 23.39.

Example 6: Synthesis of Compound T007

Synthesis Route:

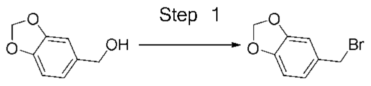


I040

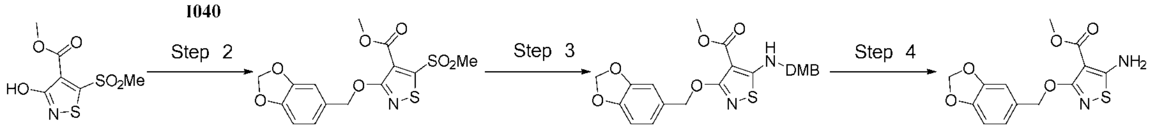


I009 I041 I042 I043

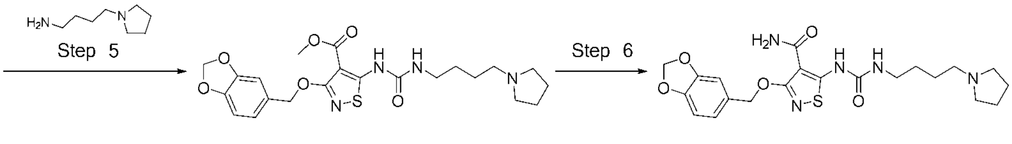


I044 T007

Step 1

5-(bromomethyl)benzo[d][1,3]dioxole

(2H-1,3-benzodioxol-5-yl)methanol (2.0 g, 13.2 mmol) was dissolved in DCM (20 mL) and then cooled to -40°C, followed by the addition of $PBr_3$ (3.56 g, 13.2 mmol), and the reaction system was stirred at -40°C to react for about 20 minutes. The reaction mixture was poured into water (30 mL), and extracted with ethyl acetate (20 mL x 3), and the organic phases were combined, washed with saturated brine (50 mL), dried with anhydrous sodium sulfate, and concentrated under reduced pressure to obtain a crude product 5-(bromomethyl)benzo[d][1,3]dioxole I040 (2 g, yield: 70.8%) as a white solid product.

The product was verified by LCMS and H-NMR.

$^1$H NMR (400 MHz, $CDCl_3$) δ: 6.88-6.90 (m, 2H), 6.78 (d, 1H, J=7.6Hz), 5.99 (s, 2H), 4.48 (s, 2H).

Step 2

Methyl 3-(benzo[d][1,3]dioxol-5-ylmethoxy)-5-(methylsulfonyl)isothiazole-4-carboxylate

Methyl 3-hydroxyl-5-(methylsulfonyl)isothiazol-4-carboxylate I009 (1.1 g, 4.64 mmol) and potassium carbonate (1.28 g, 9.27 mmol) were dissolved in DMSO (10 mL), followed by the addition of 5-(bromomethyl)benzo[d][1,3]dioxole I040 (1.0 g, 4.64 mmol), and the reaction system was stirred at room temperature to react for 2 hours. Water (30 mL) was added to the reaction mixture, which was then subjected to liquid separation and extraction with ethyl acetate (30 mL x 3), the organic phases were sequentially washed with water (50 mL) and saturated brine (50 mL), dried with anhydrous sodium sulfate, and concentrated under reduced pressure to obtain a crude product, and the crude product was stirred for 20 minutes in MTBE (20 mL) and filtered, and the filter cake was dried to obtain a yellow solid product methyl 3-(benzo[d][1,3]dioxol-5-ylmethoxy)-5-(methylsulfonyl)isothiazol-4-carboxylate I041 (0.85 g, yield: 49.4%).

The product was verified by LCMS and H-NMR.

$^1$H NMR (400 MHz, $CDCl_3$) δ: 6.92-6.95 (m, 2H), 6.80 (d, 1H, J=8.0Hz), 5.97 (s, 2H), 5.38 (s, 2H), 3.94 (s, 3H), 3.47 (s, 3H).

Step 3

Methyl 3-(benzo[d][1,3]dioxol-5-ylmethoxy)-5-((2,4-dimethoxybenzyl)amino)isothiazole-4-carboxylate

Methyl 3-(benzo[d][1,3]dioxol-5-ylmethoxy)-5-(methylsulfonyl)isothiazol-4-carboxylate I041 (0.85 g, 2.29 mmol) was dissolved in THF(10 mL), followed by the addition of 2,4-dimethoxybenzylamine (3.83 g, 22.9 mmol), and the reaction system was stirred at 60 °C to react for 1 hour. Water (20 mL) was added to the reaction mixture, which was then extracted with ethyl acetate (10 mL x 3), and the organic phases were combined, sequentially washed with 1N HCl (10 mL x 2) and saturated brine (10 mL), dried with anhydrous sodium sulfate, and concentrated under reduced pressure to obtain a yellow solid crude product methyl 3-(benzo[d][1,3]dioxol-5-ylmethoxy)-5-((2,4-dimethoxybenzyl)amino)isothiazol-4-carboxylate I042 (1 g, yield: 95.3%).

The product was verified by LCMS and H-NMR.

$^1$H NMR (400 MHz, $CDCl_3$) δ: 8.14 (t, 1H, J=5.6Hz), 7.18 (d, 1H, J=8.0Hz), 7.00 (s, 1H), 6.92-6.94 (m, 1H), 6.81 (d, 1H, J=8.0Hz), 6.45-6.50 (m, 2H), 5.97 (s, 2H), 5.34 (s, 2H), 4.29 (d, 1H, J=6.0Hz), 3.87 (s, 3H), 3.82-3.83 (m, 6H).

Step 4

Methyl 5-amino-3-(benzo[d][1,3]dioxol-5-ylmethoxy)isothiazole-4-carboxylate

Methyl 3-(benzo[d][1,3]dioxol-5-ylmethoxy)-5-((2,4-dimethoxybenzyl)amino)isothiazol-4-carboxylate I042 (1.0 g, 2.18 mmol) was dissolved in DCM/$H_2O$ (10/1mL), followed by the portionwise addition of DDQ (0.99 g, 4.36 mmol) at 0°C, and the reaction system was stirred at room temperature to react for 1 hour. Water (10 mL) was added to the reaction mixture, which was then extracted with DCM (10 mL x 3), the organic phases were combined, washed with saturated brine (20 mL), dried with anhydrous sodium sulfate, and concentrated under reduced pressure to obtain a crude product, and the crude product was purified by chromatographic columns (PE/EA = 10/1~2/1) to obtain a yellow solid product methyl 5-amino-3-(benzo[d][1,3]dioxol-5-ylmethoxy)isothiazol-4-carboxylate I043 (0.4 g, yield: 59.5%).

The product was verified by LCMS and H-NMR.

$^1$H NMR (400 MHz, DMSO-$d_6$) δ: 7.89 (s, 1H), 7.00 (s, 1H), 6.92-6.93 (m, 2H), 6.02 (s, 2H), 5.22 (s, 2H), 3.70 (s, 3H).

Step 5

Methyl 3-(benzo[d][1,3]dioxol-5-ylmethoxy)-5-(3-(4-(pyrrolidin-1-yl)butyl)ureido) isothiazole-4-carboxylate

4-(Pyrrolidin-1-yl)butyl-1-amine (277 mg, 1.95 mmol) was dissolved in anhydrous THF (5 mL) and then cooled to 0°C under the protection of $N_2$, followed by the addition of CDI (316 mg, 1.95 mmol), the mixture was stirred at room temperature to react for 30 minutes, followed by the addition of DMSO (5 mL), the pressure was then reduced to remove THF, followed by the addition of methyl 5-amino-3-(benzo[d][1,3]dioxol-5-ylmethoxy)isothiazol-4-carboxylate I043 (400 mg, 1.30 mmol) and potassium carbonate (359 mg, 2.59 mmol), and the reaction system was stirred at 25 °C to react for 16 hours. Water (15 mL) was added to the reaction mixture, which was then subjected to liquid separation and extraction with ethyl acetate (10 mL x 3), the organic phases were sequentially washed with water (10 mL) and saturated brine (20 mL), dried with anhydrous sodium sulfate, and concentrated under reduced pressure to obtain a crude product, and the crude product was purified by chromatographic columns (DCM/MeOH = 10/1) to obtain a yellow liquid product methyl 3-(benzo [d][1,3]dioxol-5-ylmethoxy)-5-(3-(4-(pyrrolidin-1-yl)butyl)ureido)isothiazol-4-carboxylate I044 (500 mg, yield: 80.9%).

The product was verified by LCMS and H-NMR.

$^1$H NMR (400 MHz, $CDCl_3$) δ: 10.27 (brs, 1H), 8.10 (br s, 1H), 7.00 (s, 1H), 6.94 (d, 2H, J=7.6Hz), 6.81 (d, 1H, J=8.0Hz), 5.98 (s, 2H), 5.35 (s, 2H), 3.88 (s, 3H), 3.34(s, 2H), 2.75 (s, 4H), 2.65 (t, 2H, J=6.0Hz), 1.95 (s, 4H), 1.70-1.75 (m, 4H).

Step 6

3-(benzo[d][1,3]dioxol-5-ylmethoxy)-5-(3-(4-(pyrrolidin-1-yl)butyl)ureido)isothiazole-4-carboxamide

In a microwave tube, methyl 3-(benzo[d][1,3]dioxol-5-ylmethoxy)-5-(3-(4-(pyrrolidin-1-yl)butyl)ureido)isothiazol-4-carboxylate I044 (200 mg, 0.42 mmol) was dissolved in $NH_3$/MeOH (4 mL, 13 mol/L), and the mixture was stirred at 60 °C to react for 16 hours. The reaction mixture was concentrated under reduced pressure to obtain a crude product, and the crude product was purified by the high-performance preparative chromatography to obtain a white solid product 3-(benzo[d][1,3]dioxol-5-ylmethoxy)-5-(3-(4-(pyrrolidin-1-yl)butyl)ureido)isothiazol-4-formamide T007 (40 mg, yield: 20.6%).

The product was verified by LCMS, H-NMR and C-NMR.

$^1$H NMR (400 MHz, DMSO-$d_6$) δ: 11.01 (s, 1H), 8.20 (s, 1H), 7.61 (s, 1H), 7.11 (d, 1H, J=1.2Hz), 6.98-7.02 (m, 2H), 6.92 (d, 1H, J=8.0Hz), 6.03 (s, 2H), 5.29 (s, 2H), 3.11-3.13 (m, 2H), 2.38-2.41 (m, 6H), 1.66-1.67 (m, 4H), 1.46 (s, 4H).

$^{13}$C NMR (100 MHz, DMSO-$d_6$) δ: 168.44, 164.93, 162.04, 154.49, 147.81, 147.75, 130.47, 122.97, 109.55, 108.60, 101.57, 97.94, 69.92, 55.66, 54.03, 27.66, 26.11, 23.54.

Example 7: Synthesis of Compound T009

Synthesis Route:

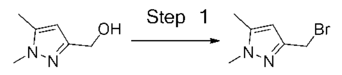


1049

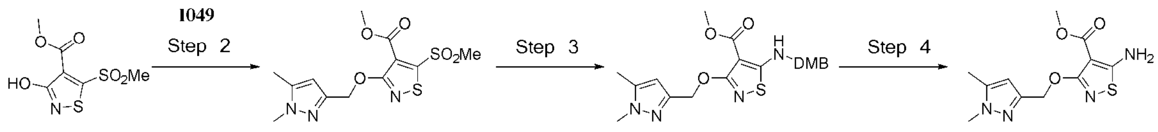


1009 1050 1051 1052

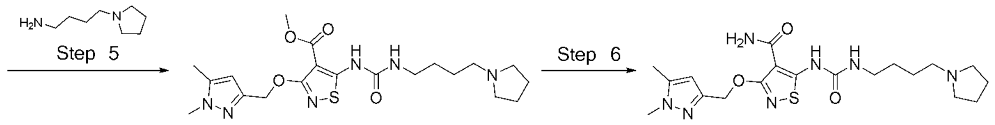


1053 T009

Step 1

3-(bromomethyl)-1,5-dimethyl-1H-pyrazole

(1,5-Dimethyl-1H-pyrazol-3-yl)methanol (2.0 g, 15.9 mmol) was dissolved in DCM (40 mL) and then cooled to 10°C, followed by the addition of $PBr_3$ (6.4 g, 23.8 mmol), and the reaction system was stirred at room temperature to react for about 16 hours. The reaction mixture was poured into water (50 mL), regulated to the pH of 8~9 by the addition of solid potassium carbonate, and extracted with ethyl acetate (20 mL x 3), and the organic phases were combined, washed with saturated brine (50 mL), dried with anhydrous sodium sulfate, and concentrated under reduced pressure to obtain a crude product 3-(bromomethyl)-1,5-dimethyl-1H-pyrazole I049 (2.7 g, yield: 90.1%) as a pale white solid product.

The product was verified by LCMS and H-NMR.

$^1$H NMR (400 MHz, $CDCl_3$) δ: 6.09 (s, 1H), 4.46 (s, 2H), 3.77 (s, 3H), 2.26 (s, 3H).

Step 2

Methyl 3-((1,5-dimethyl-1H-pyrazol-3-yl)methoxy)-5-(methylsulfonyl)isothiazole-4-carboxylate

Methyl 3-hydroxyl-5-(methylsulfonyl)isothiazol-4-carboxylate I009 (1.5 g, 6.32 mmol) and potassium carbonate (1.75 g, 12.6 mmol) were dissolved in DMF (15 mL), followed by the addition of 3-(bromomethyl)-1,5-dimethyl-1H-pyrazole I049 (1.26 g, 6.64 mmol), and the reaction system was stirred at room temperature to react for 2 hours. Water (50 mL) was added to the reaction mixture, which was then subjected to liquid separation and extraction with ethyl acetate (20 mL x 3), the organic phases were sequentially washed with water (50 mL) and saturated brine (50 mL), dried with anhydrous sodium sulfate, and concentrated under reduced pressure to obtain a crude product, and the crude product was purified by the column chromatography (DCM/MeOH = 2011-1011) to obtain a yellow solid product methyl 3-((1,5-dimethyl-1H-pyrazol-3-yl)methoxy)-5-(methylsulfonyl)isothiazol-4-carboxylate I050 (0.6 g, yield: 67.4%).

The product was verified by LCMS and H-NMR.

$^1$H NMR (400 MHz, $CDCl_3$) δ: 6.16 (s, 1H), 5.44 (s, 2H), 3.94 (s, 3H), 3.79 (s, 3H), 3.48 (s, 3H), 2.29 (s, 3H).

Step 3

Methyl 5-((2,4-dimethoxybenzyl)amino)-3-((1,5-dimethyl-1H-pyrazol-3-yl)methoxy)isothiazole-4-carboxylate

Methyl 3-((1,5-dimethyl-1H-pyrazol-3-yl)methoxy)-5-(methylsulfonyl)isothiazol-4-carboxylate I050 (1.9 g, 5.4 mmol) was dissolved in THF(20 mL), followed by the addition of 2,4-dimethoxybenzylamine (8.2 g, 49 mmol), and the reaction system was stirred at 65 °C to react for 3 hours. After the reaction was finished, the reaction mixture was added water to (100 mL), regulated with 4 N HCl solution to the pH of 4, and extracted with ethyl acetate (30 mL x 3), and the

organic phases were combined, sequentially washed with water (50 mL) and saturated brine (50 mL), dried with anhydrous sodium sulfate, and concentrated under elevated pressure to obtain a yellow solid product methyl 5-((2,4-dimethoxybenzyl)amino)-3-((1,5-dimethyl-1H-pyrazol-3-yl)methoxy)isothiazol-4-carboxylate I051 (2.5 g, yield: 99.6%).

The product was verified by LCMS and H-NMR.

$^1$H NMR (400 MHz, $CDCl_3$) δ: 8.12 (t, 1H, J=5.6Hz), 7.18 (d, 1H, J=8.0Hz), 6.44-6.49 (m, 2H), 6.15 (s, 1H), 5.37 (s, 2H), 4.28 (d, 1H, J=6.0Hz), 3.86 (s, 3H), 3.82 (s, 3H), 3.79 (s, 3H), 3.77 (s, 3H), 2.27 (s, 3H).

Step 4

Methyl 5-amino-3-((1,5-dimethyl-1H-pyrazol-3-yl)methoxy)isothiazole-4-carboxylate

Methyl 5-((2,4-dimethoxybenzyl)amino)-3-((1,5-dimethyl-1H-pyrazol-3-yl)methoxy)isothiazol-4-carboxylate I051 (1.5 g, 3.45 mmol) was dissolved in DCM/$H_2O$ (15/3mL), followed by the portionwise addition of DDQ (0.94 g, 4.14 mmol) at 0°C, and the reaction system was stirred at room temperature to react for 10 minutes. Water (20 mL) was added to the reaction mixture, which was then extracted with DCM (10 mL x2), the organic phases were combined, washed with saturated brine (20 mL), dried with anhydrous sodium sulfate, and concentrated under reduced pressure to obtain a crude product, and the crude product was purified by chromatographic columns (neutral alumina, PE/EA = 5/1-3/1-1/1-1/2)) to obtain a yellow solid product methyl 5-amino-3-((1,5-dimethyl-1H-pyrazol-3-yl)methoxy)isothiazol-4-carboxylate I052 (0.78 g, yield: 80%).

The product was verified by LCMS and H-NMR.

$^1$H NMR (400 MHz, $CDCl_3$) δ: 6.54 (s, 2H), 6.16 (s, 1H), 5.38 (s, 2H), 3.83 (s, 3H), 3.78 (s, 3H), 2.27 (s, 3H).

Step 5

Methyl 3-((1,5-dimethyl-1H-pyrazol-3-yl)methoxy)-5-(3-(4-(pyrrolidin-1-yl)butyl)ureido)isothiazole-4-carboxylate

4-(Pyrrolidin-1-yl)butyl-1-amine (281 mg, 1.97 mmol) was dissolved in anhydrous THF (25 mL) and then cooled to 0°C under the protection of $N_2$, followed by the addition of CDI (448 mg, 2.76 mmol), the mixture was stirred at room temperature to react for 60 minutes, followed by the addition of DMSO (25 mL), the pressure was then reduced to remove THF, followed by the addition of methyl 5-amino-3-((1,5-dimethyl-1H-pyrazol-3-yl)methoxy)isothiazol-4-carboxylate I052 (780 mg, 2.76 mmol and potassium carbonate (546 mg, 3.95 mmol), and the reaction system was stirred at 25 °C to react for 16 hours. Water (100 mL) was added to the reaction mixture, which was then subjected to liquid separation and extraction with ethyl acetate (50 mL x 3), the organic phases were sequentially washed with water (100 mL) and saturated brine (100 mL), dried with anhydrous sodium sulfate, and concentrated under reduced pressure to obtain a crude product, and the crude product was purified by the preparative thin-layer chromatography (DCM/MeOH = 10/1, 1% NH3.H2O) to obtain a yellow solid product methyl 3-((1,5-dimethyl-1H-pyrazol-3-yl)methoxy)-5-(3-(4-(pyrrolidin-1-yl)butyl)ureido)isothiazol-4-carboxylate I053 (700 mg, yield: 56.2%).

The product was verified by LCMS and H-NMR.

$^1$H NMR (400 MHz, $CDCl_3$) δ: 10.27 (br s, 1H), 8.15 (br s, 1H), 6.15 (s, 1H), 5.37 (s, 2H), 3.81 (s, 3H), 3.76 (s, 3H), 3.32 (t, 2H, J=5.6Hz), 2.66 (s, 4H), 2.58 (t, 2H, J=6.0Hz), 2.26 (s, 3H), 1.89 (s, 4H), 1.68-1.69 (m, 4H).

Step 6

3-((1,5-dimethyl-1H-pyrazol-3-yl)methoxy)-5-(3-(4-(pyrrolidin-1-yl)butyl)ureido)isothiazole-4-carboxamide

In a microwave tube, methyl 3-((1,5-dimethyl-1H-pyrazol-3-yl)methoxy)-5-(3-(4-(pyrrolidin-1-yl)butyl)ureido) isothiazol-4-carboxylate I053 (250 mg, 0.55 mmol) was dissolved in $NH_3$/MeOH (10 mL, 9.5 mol/L), and the mixture was stirred at 60 °C to react for 16 hours. The reaction mixture was concentrated under reduced pressure to obtain a crude product, and the crude product was purified by the preparative thin-layer chromatography (DCM/MeOH = 10/1, 1% NH3.H2O) to obtain a white solid product 3-((1,5-dimethyl-1H-pyrazol-3-yl)methoxy)-5-(3-(4-(pyrrolidin-1-yl)butyl)ureido) isothiazol-4-formamide T009 (75 mg, yield: 31%).

The product was verified by LCMS, H-NMR and C-NMR.

$^1$H NMR (400 MHz, $CDCl_3$) δ: 10.91 (br s, 1H), 7.74 (br s, 1H), 6.13 (s, 1H), 5.67 (s, 1H), 5.41 (s, 2H), 3.78 (s, 3H), 3.32 (d, 2H, J=3.6Hz), 2.50-2.57 (m, 6H), 2.28 (s, 3H), 1.85 (s, 4H), 1.66 (d, 4H, J=2.0Hz).

$^{13}$C NMR (100 MHz, $CDCl_3$) δ: 169.42, 165.96, 161.88, 154.05, 146.03, 139.62, 105.43, 97.44, 64.10, 55.87, 53.93, 40.55, 40.53, 36.10, 28.15, 26.66, 23.41, 11.21.

Example 8: Synthesis of Compound T013

Synthesis Route:

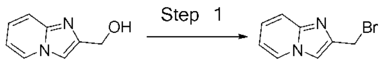


1071

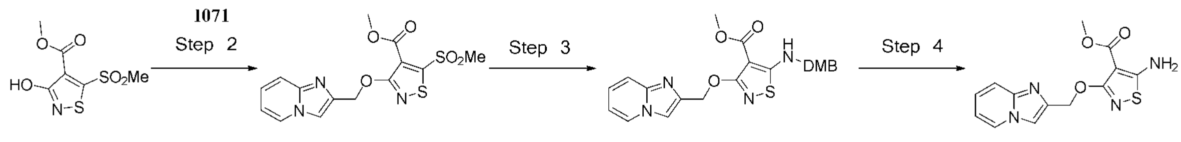


1009 1072 1073 1074

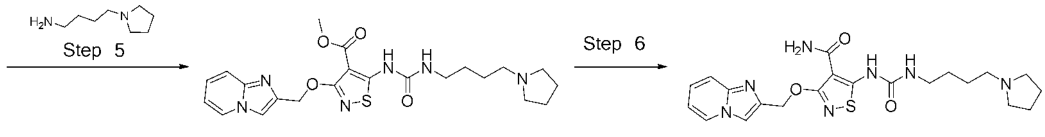


1075 T013

Step 1

2-(bromomethyl)imidazo[1,2-a]pyridine

{Imidazol[1,2-a]pyridin-2-yl}methanol (1.0 g, 6.7 mmol) was dissolved in DCM (40 mL) and then cooled to 0°C, followed by the addition of $PBr_3$ (3.6 g, 13.4 mmol), and the reaction system was stirred at room temperature to react for about 12 hours. After complete reaction, the reaction mixture was directly concentrated under reduced pressure to obtain a crude product 2-(bromomethyl)imidazol[1,2-a]pyridine I071 (1.0 g, crude product) as a white product.

The product was verified by LCMS.

Step 2

Methyl 3-(imidazo[1,2-a]pyridin-2-ylmethoxy)-5-(methylsulfonyl)isothiazole-4-carboxylate

Methyl 3-hydroxyl-5-(methylsulfonyl)isothiazol-4-carboxylate I009 (0.9 g, 3.8 mmol) and potassium carbonate (1.0 g, 7.6 mmol) were dissolved in DMF (20 mL), followed by the addition of 2-(bromomethyl)imidazol[1,2-a]pyridine I071 (1.0 g, crude product), and the reaction system was stirred at room temperature to react for 4 hours. Water (50 mL) was added to the reaction mixture, which was then extracted with ethyl acetate (30 mL x 3), and the organic phases were sequentially washed with water (30 mL) and saturated brine (30 mL), dried with anhydrous sodium sulfate, and concentrated under reduced pressure to obtain a crude product methyl 3-(imidazol[1,2-a]pyridin-2-methoxy)-5-(methylsulfonyl)isothiazol-4-carboxylate I072 (0.9 g, yield: 66.7%) as a yellow solid product.

The product was verified by LCMS and H-NMR.

$^1$H NMR (400 MHz, $CDCl_3$) $\delta$: 8.12 (d, 1H, J=6.8Hz), 7.71 (s, 1H), 7.62 (d, 1H, J=9.2Hz), 7.21-7.23 (m, 1H), 6.82-6.85 (m, 1H), 5.70 (s, 2H), 3.96 (s, 3H), 3.49 (s, 3H).

Step 3

Methyl 5-((2,4-dimethoxybenzyl)amino)-3-(imidazo[1,2-a]pyridin-2-ylmethoxy)isothiazole-4-carboxylate

Methyl 3-(imidazol[1,2-a]pyridin-2-methoxy)-5-(methylsulfonyl)isothiazol-4-carboxylate I072 (0.9 g, 2.4 mmol) was dissolved in THF(20 mL), followed by the addition of 2,4-dimethoxybenzylamine (4.0 g, 24 mmol), and the reaction system was stirred at 65°C to react for 1 hour. After the reaction was finished, the reaction mixture was cooled to room temperature, added to cold water (40 mL), and filtered to obtain solids, which were then washed with water (10 mL x 3) and dried to obtain a white solid product methyl 5-((2,4-dimethoxybenzyl)amino)-3-(imidazol[1,2-a]pyridin-2-ylmethoxy) isothiazol-4-carboxylate I073 (0.9 g, yield: 81.9%).

The product was verified by LCMS and H-NMR.

$^1$H NMR (400 MHz, $CDCl_3$) δ: 8.09-8.10 (m, 2H), 7.68 (s, 1H), 7.60 (d, 1H, J=9.2Hz), 7.17-7.19 (m, 2H), 6.78-6.79 (m, 1H), 6.44-6.49 (m, 2H), 5.63 (s, 2H), 4.29 (d, 2H, J=5.6Hz), 3.86 (s, 3H), 3.83 (s, 3H), 3.82 (s, 3H).

Step 4

Methyl 5-amino-3-(imidazo[1,2-a]pyridin-2-ylmethoxy)isothiazole-4-carboxylate

Methyl 5-((2,4-dimethoxybenzyl)amino)-3-(imidazol[1,2-a]pyridin-2-ylmethoxy)isothiazol-4-carboxylate I073 (0.9 g, 2.0 mmol) was dissolved in DCM/$H_2O$ (25/5mL), followed by the portionwise addition of DDQ (1.8 g, 8.0 mmol) at 0°C, and the reaction system was stirred at room temperature to react for 1 hour. After complete reaction, water (10 mL) was added to the reaction mixture, which was then washed with saturated sodium bicarbonate aqueous solution (10 mL), the organic phases were washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 5-amino-3-(imidazol[1,2-a]pyridin-2-methoxy)isothiazol-4-carboxylate I074 (250 mg, yield: 37.3%).

The product was verified by LCMS and H-NMR.

$^1$H NMR (400 MHz, $CDCl_3$) δ: 8.11 (d, 1H, J=6.8Hz), 7.70 (s, 1H), 7.61 (d, 1H, J=8.8Hz), 7.17-7.21 (m, 1H), 6.80 (t, 1H, J=6.8Hz), 6.59 (s, 1H), 5.64 (s, 2H), 3.87 (s, 3H).

Step 5

Methyl 3-(imidazo[1,2-a]pyridin-2-ylmethoxy)-5-(3-(4-(pyrrolidin-1-yl)butyl)ureido)isothiazole-4-carboxylate

4-(Pyrrolidin-1-yl)butyl-1-amine (125 mg, 0.88 mmol) was dissolved in anhydrous THF (6 mL) and then cooled to 0°C under the protection of $N_2$, followed by the addition of CDI (143 mg, 0.88 mmol), the mixture was then stirred at room temperature to react for 1 hour, followed by the addition of DMSO (6 mL), the pressure was then reduced to remove most of the THF, followed by the addition of methyl 5-amino-3-(imidazol[1,2-a]pyridin-2-methoxy)isothiazol-4-carboxylate I074 (200 mg, 0.64mmol) and potassium carbonate (177 mg, 1.28 mmol), and the reaction system was stirred at room temperature to react for 12 hours. After complete reaction, water (40 mL) was added to the reaction mixture, which was then extracted with dichloromethane (20 mL x 3), the organic phases were sequentially washed with water (30 mL) and saturated brine (30 mL), dried with anhydrous sodium sulfate, and concentrated under reduced pressure to obtain a crude product, and the crude product was purified by the preparative thin-layer chromatography (DCM/MeOH = 10/1, 1% NH3.H2O) to obtain a white solid product methyl 3-(imidazol[1,2-a]pyridin-2-ylmethoxy)-5-(3-(4-(pyrrolidin-1-yl)butyl) ureido)isothiazol-4-carboxylate I075 (130 mg, yield: 43%).

The product was verified by LCMS and H-NMR.

$^1$H NMR (400 MHz, $CDCl_3$) δ: 10.10 (br s, 1H), 8.20 (br s, 1H), 8.11 (d, 1H, J=6.8Hz), 7.71 (s, 1H), 7.58-7.60 (m, 1H), 7.18 (t, 1H, J=7.2Hz), 6.79 (t, 1H, J=6.8Hz), 5.64 (s, 2H), 3.82 (s, 3H), 3.34 (s, 2H), 2.63-2.73 (m, 6H), 1.94 (s, 4H), 1.71-1.75 (m, 4H).

Step 6

3-(imidazo[1,2-a]pyridin-2-ylmethoxy)-5-(3-(4-(pyrrolidin-1-yl)butyl)ureido)isothiazole-4-carboxamide

In a microwave tube, methyl 3-(imidazol[1,2-a]pyridin-2-ylmethoxy)-5-(3-(4-(pyrrolidin-1-yl)butyl)ureido)isothiazol-4-carboxylate I075 (140 mg, 0.28 mmol) was dissolved in anhydrous THF (2.5 mL) and $NH_3$/MeOH (10 mL, 8 mol/L), and the mixture was sealed to react at 60°C for 60 hours. The reaction mixture was concentrated under reduced pressure to obtain a crude product, and the crude product was purified by the preparative thin-layer chromatography (DCM/MeOH/$NH_3$.$H_2O$ = 10/1/0.1) to obtain a white solid product 3-(imidazol[1,2-a]pyridin-2-ylmethoxy)-5-(3-(4-(pyrrolidin-1-yl)butyl)ureido)isothiazol-4-formamide T013 (25 mg, yield: 17.1%).

The product was verified by LCMS, H-NMR and C-NMR.

$^1$H NMR (400 MHz, $CD_3OD$) δ: 8.43 (d, 1H, J=6.8Hz), 7.98 (s, 1H), 7.54-7.57 (m, 1H), 7.34-7.38 (m, 1H), 6.95 (t, 1H, J=6.8Hz), 5.60 (s, 2H), 3.27 (t, 2H, J=6.4Hz), 2.78 (s, 4H), 2.68-2.72 (m, 2H), 1.88-1.90 (m, 4H), 1.60-1.67 (m, 4H).

$^{13}$C NMR (100 MHz, $CD_3OD$) δ: 168. 81, 165.81, 161.82, 154.80, 145.26, 140.40, 126.74, 126.12, 115.97, 112.79, 112.53, 63.44, 55.11, 53.62, 27.02, 24.21, 22.67.

Example 9: Synthesis of Compound T015

Synthesis Route:

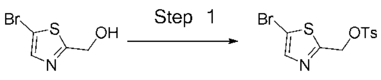


I081

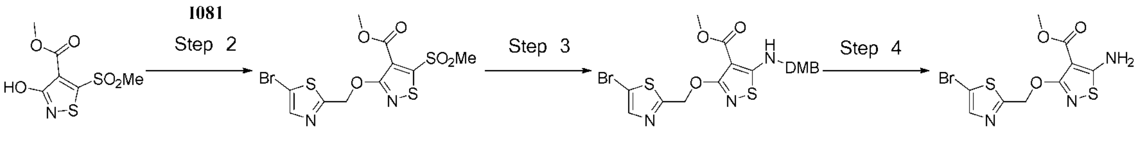


I009 I082 I083 I084

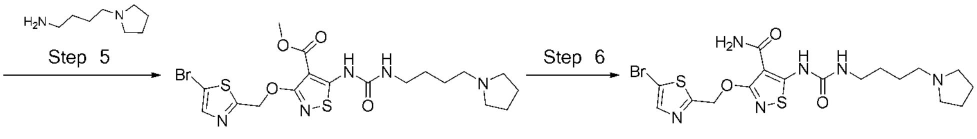


I085 T015

Step 1

(5-bromothiazol-2-yl)methyl 4-methylbenzenesulfonate

(5-Bromo-1,3-thiazol-2-yl)methanol (1.0 g, 5.15 mmol) and $Ts_2O$ (1.68 g, 5.15 mmol) were dissolved in DCM (10 mL) and then cooled to 0°C under the protection of $N_2$, followed by the dropwise addition of triethylamine (1.04 g, 10.3 mmol), and the reaction system was stirred at room temperature to react for 1 hour. After complete reaction, the reaction mixture was poured into water (10 mL) and extracted with dichloromethane (20 mLx2), the organic phases were combined, sequentially washed with water (50 mL) and saturated brine (50 mL), dried with anhydrous sodium sulfate, and concentrated under reduced pressure to obtain a crude product, and the crude product was purified by flash chromatographic columns (PE/EA = 20/1~2/1) to obtain a yellow solid product methyl (5-bromothiazol-2-yl)-4-methyl benzenesulfonate I081 (1.1 g, yield: 61.5%).

The product was verified by LCMS and H-NMR.

$^1$H NMR (400 MHz, $CDCl_3$) δ: 7.84 (d, 2H, J=8.4Hz), 7.64 (s, 1H), 7.38 (d, 1H, J=8.0Hz), 5.27 (s, 2H), 2.48 (s, 3H).

Step 2

Methyl 3-((5-bromothiazol-2-yl)methoxy)-5-(methylsulfonyl)isothiazole-4-carboxylate

Methyl 3-hydroxyl-5-(methylsulfonyl)isothiazol-4-carboxylate I009 (0.8 g, 3.37 mmol) and potassium carbonate (0.93 g, 6.74 mmol) were dissolved in DMSO (10 mL), followed by the addition of (5-bromothiazol-2-yl)-4-methylmethyl benzenesulfonate I081 (1.1 g, 3.37 mmol), and the reaction system was stirred at room temperature to react for 2 hours. After complete reaction, water (30 mL) was added to the reaction mixture, which was then extracted with ethyl acetate (10 mL x 3), the organic phases were combined, sequentially washed with water (20 mL) and saturated brine (20 mL), dried with anhydrous sodium sulfate, and concentrated under reduced pressure to obtain a crude product methyl 3-((5-bromothiazol-2-yl)methoxy)-5-(methylsulfonyl)isothiazol-4-carboxylate I082 (1.3 g, yield: 93.3%) as a yellow solid product.

The product was verified by LCMS and H-NMR.

$^1$H NMR (400 MHz, $CDCl_3$) δ: 7.71 (s, 1H), 5.70 (s, 2H), 4.00 (s, 3H), 3.52 (s, 3H).

Step 3

Methyl 3-((5-bromothiazol-2-yl)methoxy)-5-((2,4-dimethoxybenzyl)amino)isothiazole-4-carboxylate

Methyl 3-((5-bromothiazol-2-yl)methoxy)-5-(methylsulfonyl)isothiazol-4-carboxylate I082 (1.3 g, 3.15 mmol) was dissolved in THF(13 mL), followed by the addition of 2,4-dimethoxybenzylamine (5.26 g, 31.5 mmol), and the reaction system was stirred at 60°C to react for 2 hours. After the reaction was finished, the reaction mixture was cooled to room temperature, added to water (20 mL), and extracted with ethyl acetate (20 mL x 3), and the organic phases were combined, sequentially washed with 1N HCl solution (50 mL) and saturated brine (50 mL), dried with anhydrous sodium sulfate, and then concentrated under reduced pressure to obtain methyl 3-((5-bromothiazol-2-yl)methoxy)-5-((2,4-dimethoxybenzyl)amino)isothiazol-4-carboxylate I083 (1.5 g, yield: 95.3%) as a yellow solid product.
The product was verified by LCMS and H-NMR.
$^1H$ NMR (400 MHz, $CDCl_3$) δ: 8.17 (s, 1H), 7.67 (s, 1H), 7.18 (d, 1H, J = 8.0Hz), 6.45-6.50 (m, 2H), 5.62 (s, 2H), 4.30 (d, 1H, J = 6.0Hz), 3.83-3.87 (m, 9H).

Step 4

Methyl 5-amino-3-((5-bromothiazol-2-yl)methoxy)isothiazole-4-carboxylate

Methyl 3-((5-bromothiazol-2-yl)methoxy)-5-((2,4-dimethoxybenzyl)amino)isothiazol-4-carboxylate I083 (1.5 g, 3.00 mmol) was dissolved in DCM/$H_2O$ (15/3 mL), followed by the portionwise addition of DDQ (2.72 g, 12.0 mmol) at 0°C, and the reaction system was stirred at room temperature to react for 40 minutes. After complete reaction, water (20 mL) was added to the reaction mixture and extracted with dichloromethane (10 mL x 3), the organic phases were washed with saturated brine (20 mL), dried with anhydrous sodium sulfate, and concentrated under reduced pressure to obtain a crude product, and the crude product was purified by the column chromatography (PE/EA = 10/1~5/1)) to obtain a yellow solid product methyl 5-amino-3-((5-bromothiazol-2-yl)methoxy)isothiazol-4-carboxylate I084 (0.53 g, yield: 47.6%).
The product was verified by LCMS and H-NMR.
$^1H$ NMR (400 MHz, DMSO-$d_6$) δ: 7.99 (s, 2H), 7.89 (s, 1H), 5.56 (s, 2H), 3.73 (s, 3H).

Step 5

Methyl 3-((5-bromothiazol-2-yl)methoxy)-5-(3-(4-(pyrrolidin-1-yl)butyl)ureido)isothiazole-4-carboxylate

4-(Pyrrolidin-1-yl)butyl-1-amine (245 mg, 1.72 mmol) was dissolved in anhydrous THF (20 mL) and then cooled to 0°C under the protection of $N_2$, followed by the addition of CDI (279 mg, 1.72 mmol), the mixture was then stirred at room temperature to react for 30 minutes, followed by the addition of DMSO (20 mL), the pressure was then reduced to remove most of the THF, followed by the addition of methyl 5-amino-3-((5-bromothiazol-2-yl)methoxy)isothiazol-4-carboxylate I084 (430 mg, 1.23 mmol) and potassium carbonate (339 mg, 2.46 mmol), and the reaction system was stirred at room temperature to react for 16 hours. After complete reaction, water (60 mL) was added to the reaction mixture, which was then extracted with ethyl acetate (60 mL x 3), the organic phases were sequentially washed with water (40 mL) and saturated brine (50 mL), dried with anhydrous sodium sulfate, and concentrated under reduced pressure to obtain a crude product, and the crude product was purified by the column chromatography (DCM/MeOH = 30/1~20/1) to obtain a yellow solid product methyl 3-((5-bromothiazol-2-yl)methoxy)-5-(3-(4-(pyrrolidin-1-yl)butyl)ureido)isothiazol-4-carboxylate I085 (650 mg, yield: 82.8%).
The product was verified by LCMS and H-NMR.
$^1H$ NMR (400 MHz, $CDCl_3$) δ: 10.25 (br s, 1H), 8.34 (br s, 1H), 7.68 (s, 1H), 5.64 (s, 2H), 3.91 (s, 3H), 3.35(t, 2H, J=5.2Hz), 2.75 (s, 4H), 2.66 (t, 2H, J=5.2Hz), 1.96 (s, 4H), 1.73-1.77 (m, 4H).

Step 6

3-((5-bromothiazol-2-yl)methoxy)-5-(3-(4-(pyrrolidin-1-yl)butyl)ureido)isothiazole-4-carboxamide

In a microwave tube, methyl 3-((5-bromothiazol-2-yl)methoxy)-5-(3-(4-(pyrrolidin-1-yl)butyl)ureido)isothiazol-4-carboxylate I085 (400 mg, 0.77 mmol) was dissolved in $NH_3$/MeOH (3 mL, 10 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1) to obtain a white solid product methyl 3-((5-bromothiazol-2-yl)methoxy)-5-(3-(4-(pyrrolidin-1-yl)butyl)ureido)isothiazol-4-carboxylate T015(50 mg, yield: 12.9%).
The product was verified by LCMS, H-NMR and C-NMR.
$^1H$ NMR (400 MHz, DMSO-$d_6$) δ: 11.05 (s, 1H), 8.23 (t, 1H, J=5.6Hz), 7.92 (s, 1H), 7.68 (s, 1H), 6.14 (s, 1H), 5.66

(s, 2H), 3.13 (d, 2H, J=5.6Hz), 2.47 (s, 6H), 1.68 (s, 4H), 1.47 (s, 4H).

$^{13}C$ NMR (100 MHz, DMSO-$d_6$) δ: 168.89, 167.19, 164.65, 161.07, 154.54, 144.23, 110.58, 97.80, 66.46, 55.57, 54.00, 27.61, 25.92, 23.52.

Example 10: Synthesis of Compound T017

Synthesis Route:

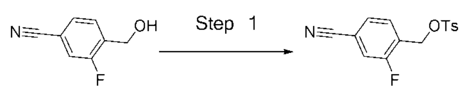


I088 I089

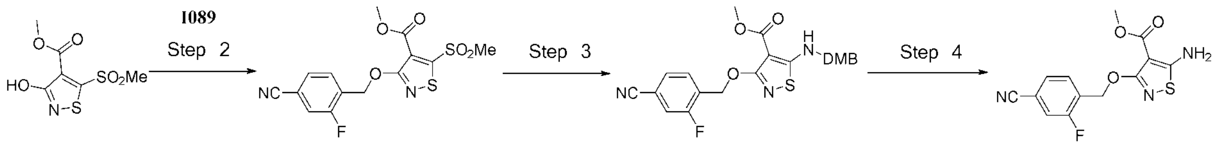


I009 I090 I091 I092

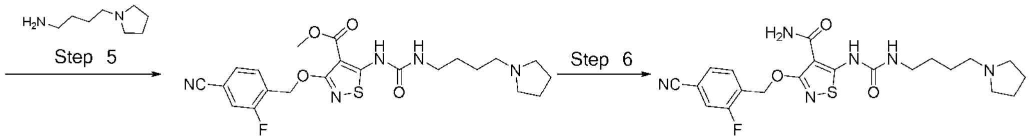


I093 T017

Step 1

4-cyano-2-fluorobenzyl 4-methylbenzenesulfonate

3-Fluoro-4-(hydroxymethyl)benzonitrile I088(2.0 g, 13.2 mmol) and $Ts_2O$ (4.32 g, 8.43 mmol) were dissolved in DCM (20 mL) and then cooled to 0°C under the protection of $N_2$, followed by the dropwise addition of triethylamine (2.68 g, 26.5 mmol), and the reaction system was stirred at room temperature to react for 4 hours. After complete reaction, the reaction mixture was sequentially extracted with water (40 mL) and dichloromethane, the organic phases were combined, washed with saturated brine (20 mL), dried with anhydrous sodium sulfate, and concentrated under reduced pressure to obtain a crude product, and the crude product was purified by the flash column chromatography (PE/EA = 10/1~2/1) to obtain a white solid product 4-cyano-2-fluorobenzyl-4-methylbenzenesulfonic acid I089(2.1 g, yield: 52.0%).

The product was verified by LCMS and H-NMR.

$^1H$ NMR (400 MHz, $CDCl_3$) δ: 7.83 (d, 2H, J=8.4Hz), 7.46-7.54 (m, 2H), 7.34-7.39 (m, 3H), 5.17 (s, 2H), 2.49 (s, 3H).

Step 2

Methyl 3-((4-cyano-2-fluorobenzyl)oxy)-5-(methylsulfonyl)isothiazole-4-carboxylate

Methyl 3-hydroxyl-5-(methylsulfonyl)isothiazol-4-carboxylate I009 (1.0 g, 4.21 mmol) and potassium carbonate (1.17 g, 8.43 mmol) were dissolved in DMSO (10 mL), followed by the addition of 4-cyano-2-fluorobenzyl-4-methylbenzenesulfonic acid I089 (1.42 g, 4.64 mmol), and the reaction system was stirred at room temperature to react for 2 hours. After complete reaction, water (30 mL) was added to the reaction mixture, which was then extracted with ethyl acetate (10 mL x 3), the organic phases were combined, sequentially washed with water (20 mL) and saturated brine (20 mL), dried with anhydrous sodium sulfate, and concentrated under reduced pressure to obtain a crude product, and the crude product was purified by the flash column chromatography (PE/EA = 10/1~2/1) to obtain a yellow solid product methyl 3-((4-cyano-2-fluorobenzyl)oxy)-5-(methylsulfonyl)isothiazol-4-carboxylate I090 (1.4 g, yield: 89.7%).

The product was verified by LCMS and H-NMR.

$^1$H NMR (400 MHz, $CDCl_3$) δ: 7.70 (t, 1H, J=8.0Hz), 7.53 (d, 1H, J=7.6Hz), 7.43 (dd, 1H, J=9.6, 1.6Hz), 5.62 (s, 2H), 4.00 (s, 3H), 3.51 (s, 3H).

Step 3

Methyl 3-((4-cyano-2-fluorobenzyl)oxy)-5-((2,4-dimethoxybenzyl)amino)isothiazole-4-carboxylate

Methyl 3-((4-cyano-2-fluorobenzyl)oxy)-5-(methylsulfonyl)isothiazol-4-carboxylate I090 (1.4 g, 3.78 mmol) was dissolved in THF(15 mL), followed by the addition of 2,4-dimethoxybenzylamine (6.32 g, 37.8 mmol), and the reaction system was stirred at 60°C to react for 2 hours. After the reaction was finished, the reaction mixture was cooled to room temperature, added to water (20 mL), and extracted with ethyl acetate (20 mL x 3), and the organic phases were combined, sequentially washed with water (50 mL) and saturated brine (50 mL), dried with anhydrous sodium sulfate, and then concentrated under reduced pressure to obtain a yellow solid product methyl 3-((4-cyano-2-fluorobenzyl)oxy)-5-((2,4-dimethoxybenzyl)amino)isothiazol-4-carboxylate I091 (1.7 g, yield: 98.3%).

The product was verified by LCMS and H-NMR.

$^1$H NMR (400 MHz, DMSO-$d_6$) δ: 8.45 (t, 1H, J=6.0Hz), 7.90 (d, 1H, J=10.0Hz), 7.70-7.77 (m, 2H), 7.17 (d, 1H, J=8.4Hz), 6.60 (d, 1H, J=4.4Hz), 6.51 (dd, 1H, J=8.4, 2.4Hz), 5.46 (s, 2H), 4.29 (d, 1H, J=6.0Hz), 3.83 (s, 3H), 3.76 (s, 6H).

Step 4

Methyl 5-amino-3-((4-cyano-2-fluorobenzyl)oxy)isothiazole-4-carboxylate

Methyl 3-((4-cyano-2-fluorobenzyl)oxy)-5-((2,4-dimethoxybenzyl)amino)isothiazol-4-carboxylate I091 (1.3 g, 2.84 mmol) was dissolved in DCM/$H_2O$ (10/2 mL), followed by the portionwise addition of DDQ (2.58 g, 11.4 mmol) at 0°C, and the reaction system was stirred at room temperature to react for 2 hours. After complete reaction, water (10 mL) was added to the reaction mixture, which was then extracted with dichloromethane (10 mL x 3), the organic phases were washed with saturated sodium bicarbonate aqueous solution (20 mL) and saturated brine (20 mL), dried with anhydrous sodium sulfate, and concentrated under reduced pressure to obtain a crude product, and the crude product was stirred and dissolved in PE/EA (v/v = 3/1, 5 mL) and filtered to obtain a filter cake, which was washed with dichloromethane (2 mL) and dried to obtain a yellow solid product methyl 5-amino-3-((4-cyano-2-fluorobenzyl)oxy)isothiazol-4-carboxylate I092 (0.6 g, yield: 68.7%).

The product was verified by LCMS and H-NMR.

$^1$H NMR (400 MHz, DMSO-$d_6$) δ: 7.89-7.95 (m, 3H), 7.72-7.79 (m, 2H), 5.46 (s, 2H), 3.73 (s, 3H).

Step 5

Methyl 3-((4-cyano-2-fluorobenzyl)oxy)-5-(3-(4-(pyrrolidin-1-yl)butyl)ureido)isothiazole-4-carboxylate

4-(Pyrrolidin-1-yl)butyl-1-amine (417 mg, 2.93 mmol) was dissolved in anhydrous THF (5 mL) and then cooled to 0°C under the protection of $N_2$, followed by the addition of CDI (475 mg, 2.93 mmol), the mixture was then stirred at room temperature to react for 30 minutes, followed by the addition of DMSO (5 mL), the pressure was then reduced to remove most of the THF, followed by the addition of methyl 5-amino-3-((4-cyano-2-fluorobenzyl)oxy)isothiazol-4-carboxylate I092 (600 mg, 1.95 mmol) and potassium carbonate (538 mg, 3.90 mmol), and the reaction system was stirred at room temperature to react for 16 hours. After complete reaction, water (20 mL) was added to the reaction mixture, which was then extracted with ethyl acetate (15 mL x 3), the organic phases were sequentially washed with water (20 mL) and saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a colorless oily liquid product methyl 3-((4-cyano-2-fluorobenzyl)oxy)-5-(3-(4-(pyrrolidin-1-yl)butyl)ureido)isothiazol-4-carboxylate I093 (350 mg, yield: 37.7%).

The product was verified by LCMS and H-NMR.

$^1$H NMR (400 MHz, $CDCl_3$) δ: 10.20 (br s, 1H), 8.32 (br s, 1H), 7.74 (t, 1H, J=7.6Hz), 7.50 (d, 1H, J=8.0Hz), 7.37-7.40 (m, 1H), 5.56 (s, 2H), 3.90 (s, 3H), 3.32-3.33 (m, 2H), 2.53-2.59 (m, 4H), 1.90 (s, 4H), 1.78-1.81 (m, 2H), 1.69 (s, 4H).

Step 6

3-((4-cyano-2-fluorobenzyl)oxy)-5-(3-(4-(pyrrolidin-1-yl)butyl)ureido)isothiazole-4-carboxamide

In a microwave tube, methyl 3-((4-cyano-2-fluorobenzyl)oxy)-5-(3-(4-(pyrrolidin-1-yl)butyl)ureido)isothiazol-4-carboxylate I093(350 mg, 0.74 mmol) was dissolved in $NH_3$/MeOH (4 mL, 10 mol/L), and the mixture was stirred at 60 °C to react for 16 hours. The reaction mixture was concentrated under reduced pressure to obtain a crude product, and the crude product was purified by the preparative thin-layer chromatography (DCM/MeOH = 10/1) to obtain a white solid product, which was dissolved in acetonitrile (5 mL) and filtered to obtain a solid product, and the solid product was dried to obtain 3-((4-cyano-2-fluorobenzyl)oxy)-5-(3-(4-(pyrrolidin-1-yl)butyl)ureido)isothiazol-4-formamide T017 (26 mg, yield: 7.7%).

The product was verified by LCMS, H-NMR and C-NMR.

$^1$H NMR (400 MHz, $CD_3OD$) δ: 7.71 (d, 1H, J=7.6Hz), 7.59-7.65 (m, 2H), 5.64 (s, 2H), 3.26 (t, 2H, J=6.4Hz), 2.66 (s, 4H), 2.59 (t, 2H, J=7.6Hz), 1.84-1.87 (m, 4H), 1.58-1.64 (m, 4H).

$^{13}$C NMR (100 MHz, $CD_3OD$) δ: 169.12, 165.68, 161.56, 161.24, 159.08, 154.71, 131.18, 131.13, 129.66, 129.52, 128.30, 128.26, 119.07, 118.82, 116.93, 116.90, 113.49, 113.40, 97.28, 63.11, 63.07, 55.65, 53.59, 39.47, 27.40, 25.29, 22.74.

Example 11: Synthesis of Compound T022

Synthesis Route:

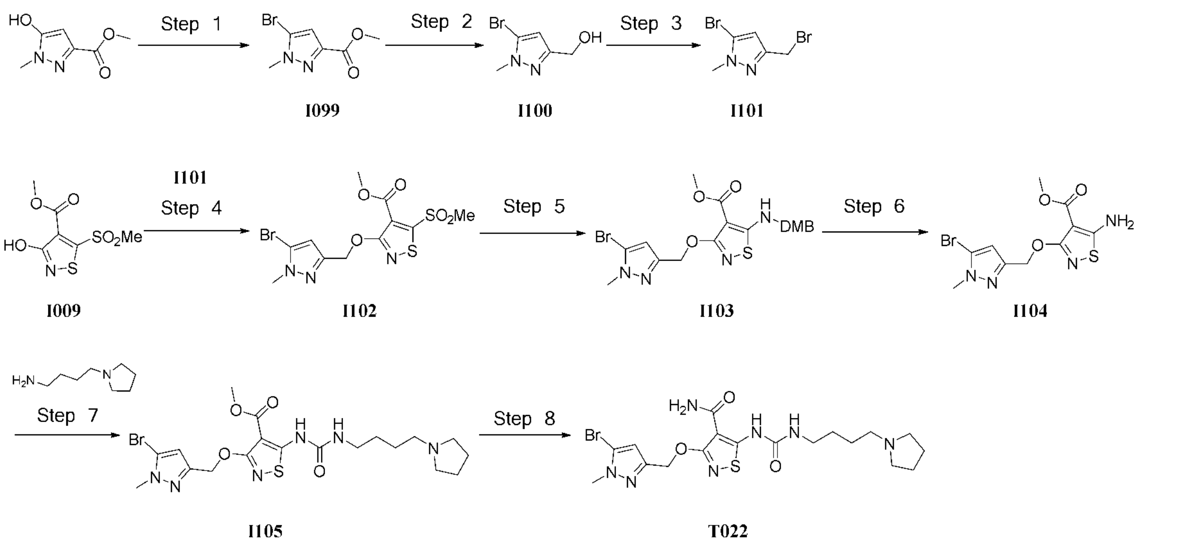


I099 I100 I101 I009 I102 I103 I104 I105 T022

Step 1

Methyl 5-bromo-1-methyl-1H-pyrazole-3-carboxylate

Methyl 5-hydroxyl-1-methyl-1H-pyrazol-3-carboxylate (10 g, 64 mmol) was dissolved in acetonitrile (50 mL), followed by the addition of $POBr_3$ (55 g, 192 mmol), and the mixture was warmed to 80°C and stirred to react for 24 hours. After complete reaction, the mixture was poured into water (200 mL), regulated with sodium bicarbonate to pH = 8, and extracted with ethyl acetate, the organic phases were combined, washed with saturated brine (100 mL x 3), dried with anhydrous sodium sulfate, and concentrated under reduced pressure to obtain a crude product, and the crude product was purified by the flash column chromatography (PE/EA = 10/1) to obtain a white solid product methyl 5-bromo-1-methyl-1H-pyrazol-3-carboxylate I099 (5 g, yield: 35.7%). The product was verified by LCMS.

Step 2

(5-bromo-1-methyl-1H-pyrazol-3-yl)methanol

Under the protection of nitrogen, methyl 5-bromo-1-methyl-1H-pyrazol-3-carboxylate I099 (2.5 g, 11.4 mmol) was dissolved in dry THF (30 mL), followed by the addition of $LiBH_4$ (14 mL, 28.5 mmol, 2 mol/L) at 0°C, and the mixture was then warmed to room temperature to react for about 12 hours. After complete reaction, the reaction mixture was poured

into saturated ammonium chloride aqueous solution (50 mL), and extracted with ethyl acetate (50 mL x 3), the organic phases were combined, sequentially washed with water and saturated brine, dried with anhydrous sodium sulfate, and concentrated under reduced pressure to obtain a crude product, and the crude product was purified by the flash column chromatography (PE/EA = 2/1) to obtain a colorless oily liquid product (5-bromo-1-methyl-1H-pyrazol-3-yl)methanol I100 (1.5 g, yield: 68.2%).

The product was verified by LCMS.

Step 3

5-bromo-3-(bromomethyl)-1-methyl-1H-pyrazole

(5-Bromo-1-methyl-1H-pyrazol-3-yl)methanol I100 (1.5 g, 7.89 mmol) was dissolved in dichloromethane (30 mL) and then cooled to 0°C, followed by the addition of $PBr_3$ (3.2 g, 11.9 mmol), and the mixture was warmed to room temperature to react for 16 hours. After complete reaction, the reaction mixture was poured into water (50 mL) and extracted with dichloromethane (50 mL x 3), and the organic phases were combined, sequentially washed with saturated sodium bicarbonate (50 mL) and saturated brine, dried with anhydrous sodium sulfate, and concentrated under reduced pressure to obtain a crude product 5-bromo-3-(bromomethyl)-1-methyl-1H-pyrazole 1101 (1.65 g,crude product) as a colorless oily liquid product.

The product was verified by LCMS.

Step 4

Methyl 3-((5-bromo-1-methyl-1H-pyrazol-3-yl)methoxy)-5-(methylsulfonyl)isothiazole-4-carboxylate

Methyl 3-hydroxyl-5-(methylsulfonyl)isothiazol-4-carboxylate I009 (1.5 g, 6.33 mmol) was dissolved in anhydrous DMF (20 mL), followed by the sequential addition of $K_2CO_3$ (1.75 g, 12.7 mmol) and 5-bromo-3-(bromomethyl)-1-methyl-1H-pyrazole I101 (1.61 g, 6.33 mmol) at room temperature, and the mixture was stirred to react for about 3 hours. After complete reaction, the reaction mixture was added to water (80 mL) and extracted with ethyl acetate, the organic phases were combined, sequentially washed with water and saturated brine, dried with anhydrous sodium sulfate, and concentrated under reduced pressure to obtain a crude product, and the crude product was purified by the flash column chromatography to obtain a white solid product methyl 3-((5-bromo-1-methyl-1H-pyrazol-3-yl)methoxy)-5-(methylsulfonyl)isothiazol-4-carboxylate I102 (1.35 g, yield: 51.9%).

The product was verified by LCMS and H-NMR.

$^1H$ NMR (400 MHz, DMSO-$d_6$) $\delta$: 6.56 (s, 1H), 5.37 (s, 2H), 3.87 (s, 3H), 3.82 (s, 3H), 3.61 (s, 3H).

Step 5

Methyl 3-((5-bromo-1-methyl-1H-pyrazol-3-yl)methoxy)-5-((2,4-dimethoxybenzyl)amino)isothiazole-4-carboxylate

Methyl 3-((5-bromo-1-methyl-1H-pyrazol-3-yl)methoxy)-5-(methylsulfonyl)isothiazol-4-carboxylate I102 (1.5 g, 3.66 mmol) was dissolved in dry THF (20 mL), followed by the addition of 2,4-dimethoxybenzylamine (3.05 g, 18.3 mmol), and the mixture was stirred at 60°C to react for about 5 hours. After complete reaction, the reaction mixture was directly concentrated under reduced pressure to obtain a crude product, and the crude product was purified by the column chromatography (PE/EA = 3/1) to obtain a white solid product methyl 3-((5-bromo-1-methyl-1H-pyrazol-3-yl)methoxy)-5-((2,4-dimethoxybenzyl)amino)isothiazol-4-carboxylate I103 (1.7 g, 94.4%).

The product was verified by LCMS.

Step 6

Methyl 5-amino-3-((5-bromo-1-methyl-1H-pyrazol-3-yl)methoxy)isothiazole-4-carboxylate

Methyl 3-((5-bromo-1-methyl-1H-pyrazol-3-yl)methoxy)-5-((2,4-dimethoxybenzyl)amino)isothiazol-4-carboxylate I103 (1.2 g, 2.41 mmol) was dissolved in DCM/$H_2O$ (15/3mL), followed by the portionwise addition of DDQ (1.0 g, 3.61 mmol) at 0°C, and the reaction system the mixture was stirred to react for 30 minutes. After complete reaction, water (100 mL) was added to the reaction mixture, which was then extracted with DCM (30 mL x2), the organic phases were combined, washed with saturated sodium bicarbonate (30 mL), water (20 mL) and saturated brine (20 mL), dried with anhydrous sodium sulfate, and concentrated under reduced pressure to obtain a crude product, and the crude product was purified by the flash column chromatography (PE/EA = 2/1) to obtain a white solid product methyl 5-amino-3-((5-bromo-1-methyl-1H-pyrazol-3-yl)methoxy)isothiazol-4-carboxylate I104 (0.45 g, yield: 53.7%).

The product was verified by LCMS.

Step 7

Methyl 3-((5-bromo-1-methyl-1H-pyrazol-3-yl)methoxy)-5-(3-(4-(pyrrolidin-1-yl)butyl)ureido)isothiazole-4-carboxylate

4-(Pyrrolidin-1-yl)butyl-1-amine (344 mg, 2.42 mmol) was dissolved in anhydrous THF (5 mL) and then cooled to 0°C under the protection of $N_2$, followed by the addition of CDI (392 mg, 2.42 mmol), the mixture was stirred to react for 30 minutes and then stirred at room temperature to react for 1 hour, followed by the addition of DMSO (10 mL), the pressure was then reduced to remove most of the THF, followed by the addition of methyl 5-amino-3-((5-bromo-1-methyl-1H-pyrazol-3-yl)methoxy)isothiazol-4-carboxylate I104 (600 mg, 1.73 mmol) and potassium carbonate (477 mg, 3.46 mmol), and the reaction system was stirred at room temperature to react for 16 hours. After complete reaction, water (50 mL) was added to the reaction mixture, which was then extracted with ethyl acetate (20 mL x 3), the organic phases were sequentially washed with water (30 mL) and saturated brine (30 mL), dried with anhydrous sodium sulfate, and concentrated under reduced pressure to obtain a crude product, and the crude product was purified by the column chromatography (DCM/MeOH = 10/1) to obtain a white solid product methyl 3-((5-bromo-1-methyl-1H-pyrazol-3-yl) methoxy)-5-(3-(4-(pyrrolidin-1-yl)butyl)ureido)isothiazol-4-carboxylate I105 (600 mg, yield: 67.4%).

The product was verified by LCMS.

Step 8

3-((5-bromo-1-methyl-1H-pyrazol-3-yl)methoxy)-5-(3-(4-(pyrrolidin-1-yl)butyl)ureido)isothiazole-4-carboxamide

In a microwave tube, methyl 3-((5-bromo-1-methyl-1H-pyrazol-3-yl)methoxy)-5-(3-(4-(pyrrolidin-1-yl)butyl)ureido)isothiazol-4-carboxylate 1105 (600 mg, 1.17 mmol) was dissolved in $NH_3$/MeOH (10 mL,13 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1) to obtain a white solid product 3-((5-bromo-1-methyl-1H-pyrazol-3-yl)methoxy)-5-(3-(4-(pyrrolidin-1-yl)butyl)ureido)isothiazol-4-formamide T022 (50 mg, yield: 8.6%).

The product was verified by LCMS, H-NMR and C-NMR.

$^1$H NMR (400 MHz, $CDCl_3$) δ: 10.90 (s, 1H), 7.73 (s, 1H), 7.21 (s, 1H), 6.42 (s, 1H), 5.72 (s, 1H), 5.42 (s, 2H), 3.89 (s, 3H), 3.31-3.32 (m, 2H), 2.47-2.53 (m, 6H), 2.10 (s, 2H), 1.83-1.87(m, 4H), 1.62-1.65 (m, 2H).

$^{13}$C NMR (100 MHz, $CDCl_3$) δ: 169.60, 165.86, 161.55, 154.02, 147.95, 113.99, 108.59, 63.72, 55.89, 53.93, 40.78, 40.57, 37.63, 28.35, 28.27, 28.13, 28.02, 26.77, 23.60, 23.41.

Example 12: Synthesis of Compound T023

Synthesis Route:

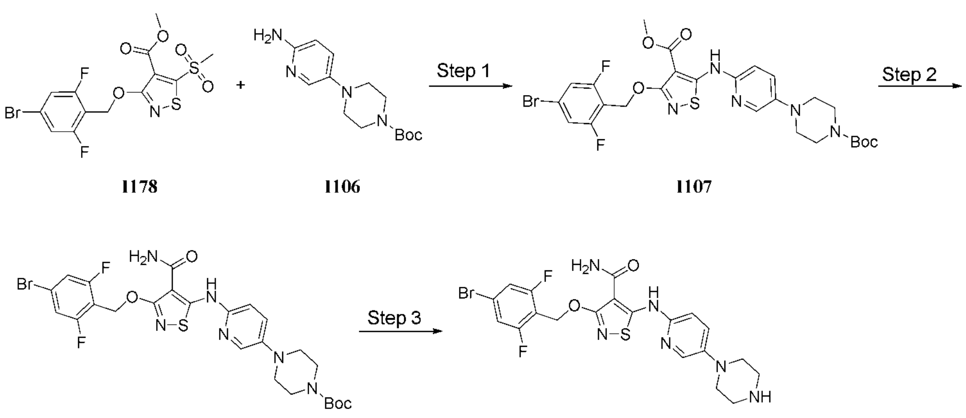


1178 1106 1107

1108 T023

Step 1

Methyl3-((4-bromo-2,6-difluorobenzyl)oxy)-5-((5-(4-(tert-butoxycarbonyl)piperazin-1-yl) pyridin-2-yl)amino)isothiazole-4-carboxylate

At 0°C, LiHMDS (1 M, 1.4 mL, 1.4 mmol) was added to the solution of methyl 3-(4-bromo-2,6-difluorobenzeneoxy)-5-methylsulfonyl-1,2-thiazol-4-carboxylate I178 (300 mg, 0.68 mmol) and 4-(6-aminopyridin-3-yl)piperazin-1-tert-butyl formate I106 (208 mg, 0.75 mmol) in THF (30 mL), stirred at 0°C to react for 0.5 hours, and then stirred at room temperature react for 0.5 hours. The reaction mixture was poured into water (30 mL) and then subjected to liquid separation and extraction with ethyl acetate (20 mL x 3), the organic phases were combined, washed with saturated brine (50 mL), dried with anhydrous sodium sulfate, and concentrated under reduced pressure to obtain a crude product, and the crude product was purified by the preparative thin-layer chromatography (PE/EA = 3/1) to obtain a yellow solid product methyl 3-((4-bromo-2,6-difluorobenzyloxy)oxy)-5-((5-(4-tert-butoxycarbonyl)piperazin-1-yl)pyridin-2-yl)amino)isothiazol-4-carboxylate I107 (22.5 mg, yield: 5%).

The product was verified by LCMS and HNMR.

$^1$H NMR (400 MHz, $CDCl_3$) δ: 10.80 (s, 1H), 8.11 (d, 1H, J=2.4Hz), 7.36-7.39 (m, 1H), 7.14-7.18 (m, 2H), 6.92 (d, 1H, J=8.8Hz), 5.48 (s, 2H), 3.84 (s, 3H), 3.62-3.64 (m, 4H), 3.11-3.13 (m, 4H), 1.52 (s, 9H).

Step 2

Tert-butyl4-(6-((3-((4-bromo-2,6-difluorobenzyl)oxy)-4-carbamoylisothiazol-5-yl)amino) pyridine -3-yl)piperazine-1-carboxylate

Methyl 3-((4-bromo-2,6-difluorobenzyloxy)oxy)-5-((5-(4-tert-butoxycarbonyl)piperazin-1-yl)pyridin-2-yl)amino) isothiazol-4-carboxylate I107 (110 mg, 0.17 mmol) was dissolved in methanol (1 mL) and $NH_3$/MeOH (9 mL, 10 mol/L). The mixture was sealed to react at 60°C for 72 hours. The reaction mixture was concentrated under reduced pressure to obtain a crude product, and the crude product was purified by the preparative thin-layer chromatography (PE/EA = 3/1) to obtain a yellow solid product tert-butyl 4-(6-((3-((4-bromo-2,6-difluorobenzyl)oxy)-4-carbamylisothiazol-5-yl)amino)pyridin-3-yl) piperazin-1-carboxylate I108 (50.0 mg, yield: 46%). The product was verified by LCMS and H-NMR.

$^1$H NMR (400 MHz, $CDCl_3$) δ: 11.52 (s, 1H), 8.10 (d, 1H, J=2.4Hz), 7.36-7.39 (m, 1H), 7.18-7.19 (m, 2H), 7.02 (s, 1H), 6.94 (d, 1H, J=8.4Hz), 5.56 (s, 2H), 5.29 (s, 1H), 3.61-3.64 (m, 4H), 3.10-3.12 (m, 4H), 1.58 (s, 9H).

Step 3

3-((4-bromo-2,6-difluorobenzyl)oxy)-5-((5-(piperazin-1-yl)pyridin-2-yl)amino)isothiazole-4-carboxamide

Tert-butyl 4-(6-((3-((4-bromo-2,6-difluorobenzyl)oxy)-4-carbamylisothiazol-5-yl)amino)pyridin-3-yl)piperazin-1-carboxylate I108 (80.0 mg, 0.13 mmol) was dissolved in HCl/MeOH (5 mL, 8 mol/L), and the mixture was stirred at 25 °C to react for 1.0 hour. The reaction mixture was concentrated under reduced pressure, and the residues were added to water (10 mL) and regulated with a sodium carbonate aqueous solution to the pH of 9.0. The mixture was subjected to liquid separation and extraction with ethyl acetate (10 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 20/1) to obtain a white solid product 3-((4-bromo-2,6-difluorobenzyl)oxy)-5-((5-(piperazin-1-yl)pyridin-2-yl)amino)isothiazol-4-formamide T023 (27.0 mg, yield: 40%).

The product was verified by LCMS, H-NMR and C-NMR.

$^1$H NMR (400 MHz, $CDCl_3$) δ: 11.48 (s, 1H), 8.09 (d, 1H, J=2.8Hz), 7.34-7.37 (m, 1H), 7.18-7.19 (m, 2H), 7.02 (s, 1H), 6.93 (d, 1H, J=8.8Hz), 5.56 (s, 2H), 5.31(s, 1H), 3.13-3.14 (m, 4H), 3.08-3.09 (m, 4H).

$^{13}$C NMR (100 MHz, $CDCl_3$) δ: 168.67, 166.37, 161.41, 144.65, 142.87, 134.04, 128.04, 115.87, 115.78, 115.58, 111.71, 111.34, 95.37, 57.51, 57.47, 50.98, 46.01.

Example 13: Synthesis of Compound T024

Synthesis Route:

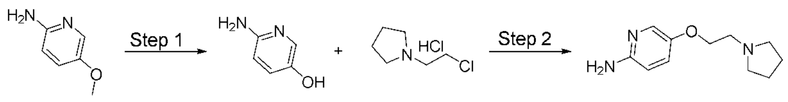


I109 I110

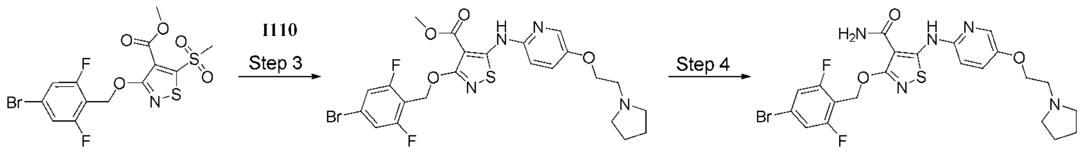


I178 I111 T024

Step 1

6-aminopyridin-3-ol

5-Methoxypyridin-2-amine (2.8 g, 22.6 mmol) was dissolved in 48% aq. HBr (15 mL), and the reaction system was stirred under reflux to react for 12 hours. The reaction mixture was cooled to room temperature, poured into cold water (20 mL), regulated with sodium carbonate aqueous solution to the PH of 7-8, and extracted wth ethyl acetate (40 mL x 10), the organic phases were combined, dried with anhydrous sodium sulfate, and concentrated under reduced pressure to obtain a black oily crude product 6-aminopyridin-3-ol I109 (1.3 g, yield: 52.3%).

The product was verified by LCMS and HNMR.

$^1$H NMR (400 MHz, $CDCl_3$) $\delta$: 8.62 (s, 1H), 7.50 (dd, 1H, J=3.2, 0.8Hz), 6.91 (dd, 1H, J=8.8, 3.2Hz), 6.34 (dd, 1H, J=8.8, 0.8Hz), 5.19 (s, 2H).

Step 2

5-(2-(pyrrolidin-1-yl)ethoxy)pyridin-2-amine

6-Aminopyridin-3-ol I109 (1.3 g, 11.8 mmol) was dissolved in DMF (15 mL), followed by the addition of 1-(2-chloroethyl)pyrrolidine hydrochloride (2.0 g, 11.8 mmol) and sodium hydroxide (1.9 g, 47.2 mmol), and the reaction system was stirred at 65°C to react for 2.0 hours. The reaction mixture was cooled to room temperature, poured into brine (40 mL), and extracted with DCM/MeOH = 10/1 (v/v, 30 mL x 10), the organic phases were combined, dried with anhydrous sodium sulfate, and concentrated under reduced pressure to obtain a crude product, and the crude product was purified by the silica-gel column chromatography (DCM/MeOH = 50/1~10/1) to obtain a black solid product 5-(2-pyrrolidin-1-ylethoxy) pyridin-2-amine I110 (1.3 g, yield: 53.1%). The product was verified by LCMS and HNMR.

$^1$H NMR (400 MHz, $CDCl_3$) $\delta$: 7.81 (d, 1H, J=2.8Hz), 7.13 (dd, 1H, J=8.8, 3.2Hz), 6.48 (d, 1H, J=8.8Hz), 4.19 (s, 2H), 4.06 (t, 2H, J=6.0Hz), 2.87 (t, 2H, J=6.0Hz),2.60-2.63 (m, 4H), 1.80-1.83 (m, 4H).

Step 3

Methyl 3-((4-bromo-2,6-difluorobenzyl)oxy)-5-((5-(2-(pyrrolidin-1-yl)ethoxy)pyridin-2-yl)amino)isothiazole-4-carboxylate

At 0°C, LiHMDS (1.4 mL, 1.4 mmol, 1mol/L) was dropwise added to the solution of methyl 3-(4-bromo-2,6-difluorobenzeneoxy)-5-methylsulfonyl-1,2-thiazol-4-carboxylate I178 (300 mg, 0.68 mmol) and 5-(2-pyrrolidin-1-ylethoxy)pyridin-2-amine I110 (155 mg, 0.75 mmol) in THF (30 mL). The mixture was stirred at 0-10°C to react for 1.0 hour, the reaction mixture was poured into $NH_4Cl$ aqueous solution (40 mL) and extracted with ethyl acetate (20 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 20/1) to obtain a white solid product methyl 3-(4-bromo-2,6-difluorobenzyloxy)-5-(5-(2-pyrrolidin-1-ylethoxy)pyridin-2-aminopyridin-4-carboxylate I111 (18 mg, yield: 4.8%).

The product was verified by LCMS and H-NMR.

$^1$H NMR (400 MHz, $CDCl_3$) $\delta$: 10.81 (s, 1H), 8.14 (d, 1H, J=2.8Hz), 7.36 (dd, 1H, J=8.8, 2.8Hz), 7.16 (d, 1H, J=6.8Hz), 6.92 (d, 1H, J=8.8Hz), 5.48 (s, 2H), 4.19 (t, 2H, J=6.0Hz), 2.96 (t, 2H, J=5.6Hz), 2.68 (m, 4H), 1.85-1.87 (m, 4H).

Step 4

3-((4-bromo-2,6-difluorobenzyl)oxy)-5-((5-(2-(pyrrolidin-1-yl)ethoxy)pyridin-2-yl)amino)isothiazole-4-carboxamide

Methyl 3-(4-bromo-2,6-difluorobenzyloxy)-5-(5-(2-pyrrolidin-1-ylethoxy)pyridin-2-aminopyridin-4-carboxylate I111 (110 mg, 0.19 mmol) was dissolved in $NH_3$/MeOH (5 mL, 10 mol/L) and THF(1 mL). The mixture was sealed to react at 60 °C for 72 hours. The reaction mixture was concentrated under reduced pressure to obtain a crude product, and the crude product was purified by the preparative thin-layer chromatography (DCM/MeOH = 20/1) to obtain a white solid product 3-(4-bromo-2,6-difluorobenzyloxy)-5-(5-(2-pyrrolidin-1-ylethoxy)pyridin-2-aminopyridin-4-formamide T024 (21 mg, yield: 19.6%).

The product was verified by LCMS, H-NMR and C-NMR.

$^1$H NMR (400 MHz, $CDCl_3$) δ: 11.53 (s, 1H), 8.13 (d, 1H, J =2.8Hz), 7.35 (dd, 1H, J =8.8, 2.8Hz), 7.18-7.20 (m, 2H), 7.02 (s, 1H), 6.93 (d, 1H, J =8.8Hz), 5.56 (s, 2H), 5.32 (s, 1H), 4.19 (t, 2H, J =5.6 Hz), 2.96 (t, 2H, J =5.6 Hz), 2.69 (m, 4H), 1.86 (m, 4H).

$^{13}$C NMR (100 MHz, $CDCl_3$) δ: 168.80, 166.37, 162.93, 161.40, 150.34, 145.27, 132.01, 126.74, 123.22, 115.86, 115.84, 115.59, 115.57, 111.70, 95.55, 68.10, 57.56, 57.53, 57.49, 55.03, 54.71, 23.50.

Example 14: Synthesis of Compound T026

Synthesis Route:

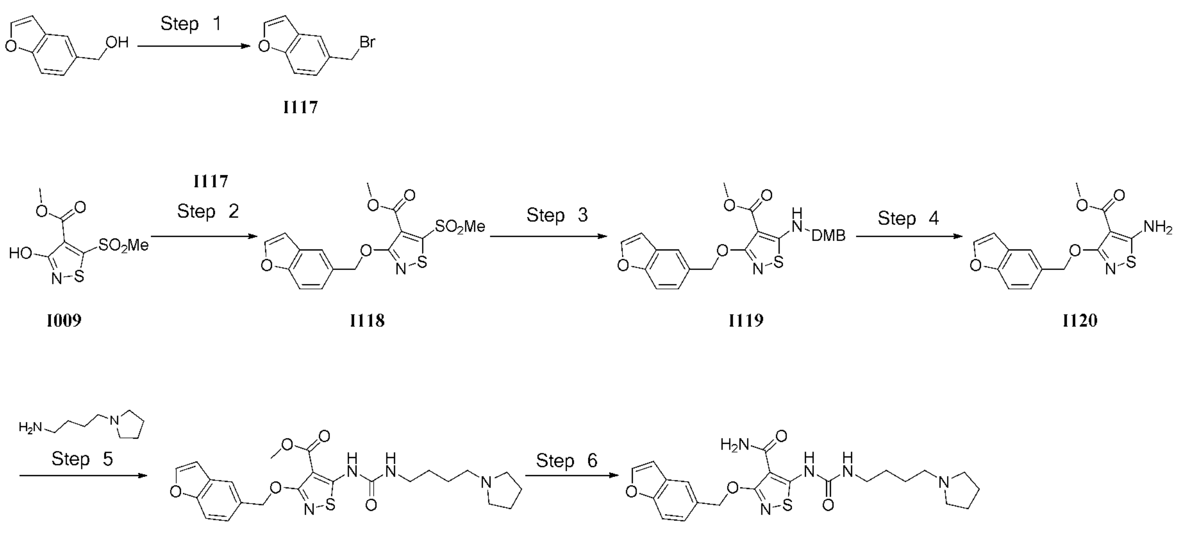


I117

I009 I118 I119 I120

I121 T026

Step 1

5-(bromomethyl)benzofuran

(1-Benzofuran-5-yl)methanol (3.0 g, 20.3 mmol) was dissolved in DCM (50 mL) and then cooled to 0°C, followed by the addition of $PBr_3$ (8.21 g, 30.4 mmol), and the reaction system was stirred at room temperature to react for about 2 hours. After complete reaction, the reaction mixture was poured into water (50 mL) and extracted with dichloromethane (50 mL x 2), and the organic phases were combined, washed with saturated sodium bicarbonate aqueous solution (50 mL) and saturated brine (50 mL), dried with anhydrous sodium sulfate, and concentrated under reduced pressure to obtain a yellow oily liquid product 5-(bromomethyl)benzofuran I117 (2.5 g, crude product).

The product was verified by LCMS.

Step 2

Methyl 3-(benzofuran-5-ylmethoxy)-5-(methylsulfonyl)isothiazole-4-carboxylate

Methyl 3-hydroxyl-5-(methylsulfonyl)isothiazol-4-carboxylate I009 (1.5 g, 6.33 mmol) and potassium carbonate (1.75 g, 12.7 mmol) were dissolved in DMF (10 mL), followed by the addition of 5-(bromomethyl)benzofuran I117 (1.33 g,

6.33 mmol), and the reaction system was stirred at room temperature to react for 3 hours. Water (20 mL) was added to the reaction mixture, which was then extracted with ethyl acetate (20 mL x 2), the organic phases were combined, sequentially washed with water and saturated brine, dried with anhydrous sodium sulfate, and concentrated under reduced pressure to obtain a crude product, and the crude product was purified by the flash column chromatography (PE/EA = 3/1) to obtain a white solid product methyl 3-(benzofuran-5-methoxy)-5-(methylsulfonyl)isothiazol-4-carboxylate I118 (1.5 g, yield: 65.2%).

The product was verified by LCMS and H-NMR.

$^1$H NMR (400 MHz, DMSO-$d_6$) δ: 8.03 (d, 1H, J=1.6Hz), 7.79 (s, 1H), 7.64 (d, 1H, J=8.4Hz), 7.44 (dd, 1H, J=8.8, 1.2Hz), 7.00 (d, 1H, J=1.2Hz), 5.58 (s, 2H), 3.88 (s, 3H), 3.61 (s, 3H).

Step 3

Methyl 3-(benzofuran-5-ylmethoxy)-5-((2,4-dimethoxybenzyl)amino)isothiazole-4-carboxylate

Methyl 3-(benzofuran-5-methoxy)-5-(methylsulfonyl)isothiazol-4-carboxylate I118 (2 g, 5.44 mmol) was dissolved in THF(50 mL), followed by the addition of 2,4-dimethoxybenzylamine (4.0 g, 24 mmol), and the reaction system was stirred at 65 °C to react for 6 hours. After the reaction was finished, the reaction mixture was directly concentrated under reduced pressure to obtain a crude product. The crude product was purified by flash chromatographic columns (PE/EA = *5/1)* to obtain a yellow solid product methyl 3-(benzofuran-5-methoxy)-5-((2,4-dimethoxybenzyl)amino)isothiazol-4-carboxylate I119 (2.0 g, yield: 81.0%).

The product was verified by LCMS.

Step 4

Methyl 5-amino-3-(benzofuran-5-ylmethoxy)isothiazole-4-carboxylate

Methyl 3-(benzofuran-5-methoxy)-5-((2,4-dimethoxybenzyl)amino)isothiazol-4-carboxylate I119 (1.5 g, 3.30 mmol) was dissolved in DCM/$H_2O$ (20/4mL), followed by the portionwise addition of DDQ (1.37 g, 4.95 mmol) at 0°C, and the reaction system was stirred at room temperature to react for 20 minutes. After complete reaction, water (10 mL) was added to the reaction mixture, which was then washed with saturated sodium bicarbonate aqueous solution (25 mL), the organic phase was washed with saturated brine (25 mL), dried with anhydrous sodium sulfate, and concentrated under reduced pressure to obtain a crude product, and the crude product was purified by the flash column chromatography (PE/EA = 5/1) to obtain a yellow solid product methyl 5-amino-3-(benzofuran-5-methoxy)isothiazol-4-carboxylate I120 (0.45 g, yield: 45%).

The product was verified by LCMS.

Step 5

Methyl 3-(benzofuran-5-ylmethoxy)-5-(3-(4-(pyrrolidin-1-yl)butyl)ureido)isothiazole-4-carboxylate

4-(Pyrrolidin-1-yl)butyl-1-amine (392 mg, 2.76 mmol) was dissolved in anhydrous THF (10 mL) and then cooled to 0°C under the protection of $N_2$, followed by the addition of CDI (448 mg, 2.76 mmol), the mixture was then stirred at room temperature to react for 1 hour, followed by the addition of DMSO (10 mL), the pressure was then reduced to remove most of the THF, followed by the addition of methyl 5-amino-3-(benzofuran-5-methoxy)isothiazol-4-carboxylate I120 (600 mg, 1.97 mmol) and potassium carbonate (545 mg, 3.95 mmol), and the reaction system was stirred at room temperature to react for 16 hours. After complete reaction, water (50 mL) was added to the reaction mixture, which was then extracted with ethyl acetate (20 mL x 3), the organic phases were sequentially washed with water (30 mL) and saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a yellow oily liquid product methyl 3-(benzofuran-5-methoxy)-5-(3-(4-(pyrrolidin-1-yl)butyl)ureido)isothiazol-4-carboxylate I121 (600 mg, yield: 64.4%).

The product was verified by LCMS.

Step 6

3-(benzofuran-5-ylmethoxy)-5-(3-(4-(pyrrolidin-1-yl)butyl)ureido)isothiazole-4-carboxamide

In a microwave tube, methyl 3-(benzofuran-5-methoxy)-5-(3-(4-(pyrrolidin-1-yl)butyl)ureido)isothiazol-4-carboxylate I121 (600 mg, 1.27 mmol) was dissolved in $NH_3$/MeOH (10 mL, 13 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1) to obtain a white solid product 3-(benzofuran-5-methoxy)-5-(3-(4-(pyrrolidin-1-yl)butyl)ureido)isothiazol-4-formamide T026 (60 mg, yield: 10.5%).

The product was verified by LCMS, H-NMR and C-NMR.

$^1$H NMR (400 MHz, $CD_3OD$) δ: 7.78-7.79 (m, 2H), 7.53 (d, 1H, J=8.8Hz), 7.44 (d, 1H, J=8.4Hz), 6.87 (d, 1H, J=1.2Hz), 5.54 (s, 2H), 3.25 (t, 2H, J=6.0Hz), 2.49-2.57 (m, 6H), 1.82 (s, 4H), 1.58-1.60 (m, 4H).

$^{13}$C NMR (100 MHz, $CD_3OD$) δ: 171.20, 168.32, 164.57, 157.42, 157.17, 148.27, 133.23, 130.22, 127.22, 123.83, 113.36, 108.68, 99.83, 72.81, 58.17, 56.00, 41.95, 29.90, 27.88, 25.18.

Example 15: Synthesis of Compound T027

Synthesis Route:

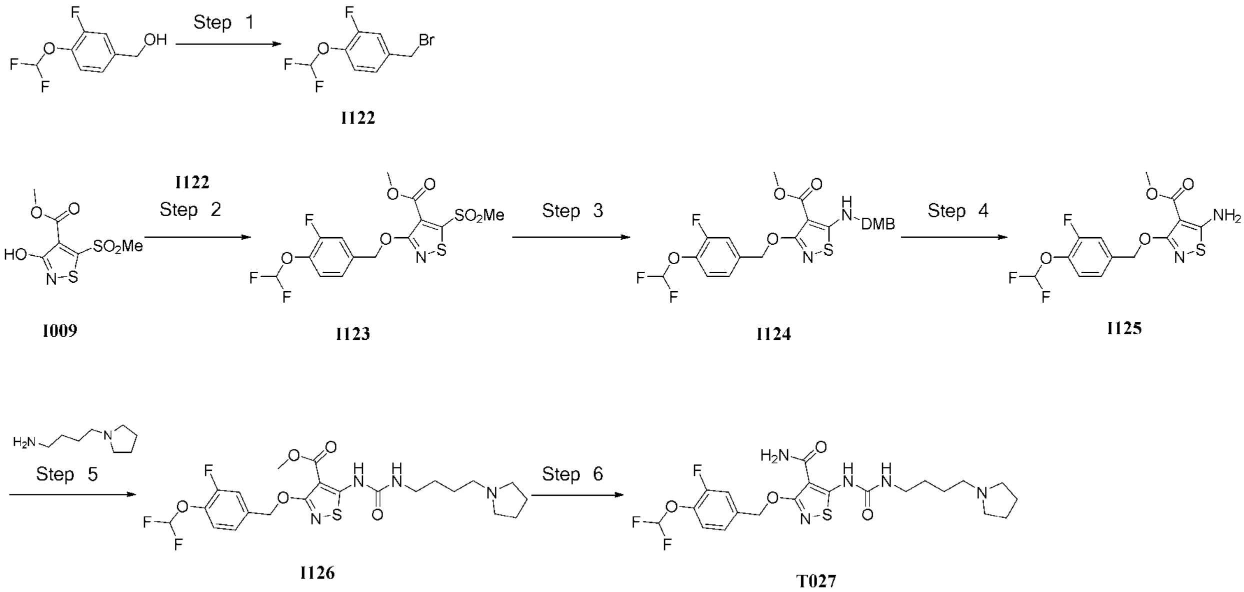


I122, I009, I123, I124, I125, I126, T027

Step 1

4-(bromomethyl)-1-(difluoromethoxy)-2-fluorobenzene

[4-(Difluoromethoxy)-3-fluorophenyl]methanol (3.0 g, 15.6 mmol) was dissolved in DCM (50 mL) and then cooled to 0°C, followed by the addition of $PBr_3$ (6.33 g, 23.4 mmol), and the reaction system was stirred at room temperature to react for about 3 hours. After complete reaction, the reaction mixture was added to water (50 mL) and extracted with dichloromethane (50 mL x 2), and the organic phases were combined, sequentially washed with saturated sodium bicarbonate aqueous solution and saturated brine, dried with anhydrous sodium sulfate, and concentrated under reduced pressure to obtain a crude product 4-(bromomethyl)-1-(difluoromethoxy)-2-fluorobenzene I122 (1.8 g crude product) as a colorless oily liquid product.

The product was verified by LCMS.

Step 2

Methyl 3-((4-(difluoromethoxy)-3-fluorobenzyl)oxy)-5-(methylsulfonyl)isothiazole-4-carboxylate

Methyl 3-hydroxyl-5-(methylsulfonyl)isothiazol-4-carboxylate I009 (1.5 g, 6.33 mmol) and potassium carbonate (1.75 g, 12.7 mmol) were dissolved in DMF (20 mL), followed by the addition of 4-(bromomethyl)-1-(difluoromethoxy)-2-fluorobenzene I122 (2.1 g, 8.24 mmol), and the reaction system was stirred at room temperature to react for 5 hours. Water (20 mL) was added to the reaction mixture, which was then subjected to liquid separation and extraction with, ethyl acetate

(20 mL x 3) the organic phases were sequentially washed with water (20 mL) and saturated brine (20 mL), dried with anhydrous sodium sulfate, and concentrated under reduced pressure to obtain a crude product, and the crude product was purified by the flash column chromatography (PE/EA = 3/1) to obtain a white solid product methyl 3-((4-(difluoromethoxy)-3-fluorobenzyl)oxy)-5-(methylsulfonyl)isothiazol-4-carboxylate I123 (1.5 g, yield: 57.7%).

The product was verified by LCMS and H-NMR.

$^1$H NMR (400 MHz, DMSO-$d_6$) δ: 7.55 (d, 1H, J=1.6Hz), 7.41-7.45 (m, 1H), 7.36-7.38 (m, 1H), 7.27 (t, 1H, J=73.2), 5.50 (s, 2H), 3.90 (s, 3H), 3.62 (s, 3H)

Step 3

Methyl 3-((4-(difluoromethoxy)-3-fluorobenzyl)oxy)-5-((2,4-dimethoxybenzyl)amino)isothiazole-4-carboxylate

Methyl 3-((4-(difluoromethoxy)-3-fluorobenzyl)oxy)-5-(methylsulfonyl)isothiazol-4-carboxylate I123 (2 g, 4.86 mmol) was dissolved in THF(20 mL), followed by the addition of 2,4-dimethoxybenzylamine (4.1 g, 24.6 mmol), and the reaction system was stirred at 60 °C to react for 6 hours. After the reaction was finished, the reaction mixture was directly concentrated under reduced pressure to obtain a crude product, and the crude product was purified by the flash column chromatography (PE/EA = 5/1) to obtain a white solid product methyl 3-((4-(difluoromethoxy)-3-fluorobenzyl)oxy)-5-((2,4-dimethoxybenzyl)amino)isothiazol-4-carboxylate I124 (2 g, yield: 82.6%).

The product was verified by LCMS.

Step 4

Methyl 5-amino-3-((4-(difluoromethoxy)-3-fluorobenzyl)oxy)isothiazole-4-carboxylate

Methyl 3-((4-(difluoromethoxy)-3-fluorobenzyl)oxy)-5-((2,4-dimethoxybenzyl)amino)isothiazol-4-carboxylate I124 (2.3 g, 4.62 mmol) was dissolved in DCM/$H_2O$ (20/4 mL), followed by the portionwise addition of DDQ (1.92 g, 6.93 mmol) at 0°C, and the reaction system was stirred at room temperature to react for 20 minutes. After complete reaction, water (10 mL) was added to the reaction mixture, which was then washed with saturated sodium bicarbonate aqueous solution (25 mL), the organic phase was washed with saturated brine (25 mL), dried with anhydrous sodium sulfate, and concentrated under reduced pressure to obtain a crude product, and the crude product was purified by the flash column chromatography (PE/EA = 5/) to obtain a yellow solid product methyl 5-amino-3-((4-(difluoromethoxy)-3-fluorobenzyl)oxy)isothiazol-4-carboxylate I125 (0.6 g, yield: 37.5%).

The product was verified by LCMS and H-NMR.

$^1$H NMR (400 MHz, $CDCl_3$) δ: 7.35 (dd, 1H, J=11.6, 1.6Hz), 7.23-7.26 (m, 2H), 6.57 (t, 1H, J=73.6Hz), 6.47 (s, 2H), 5.42 (s, 2H), 3.90 (s, 3H)

Step 5

Methyl 3-((4-(difluoromethoxy)-3-fluorobenzyl)oxy)-5-(3-(4-(pyrrolidin-1-yl)butyl)ureido)isothiazole-4-carboxylate

4-(Pyrrolidin-1-yl)butyl-1-amine (343 mg, 2.42 mmol) was dissolved in anhydrous THF (10 mL) and then cooled to 0°C under the protection of $N_2$, followed by the addition of CDI (491 mg, 2.42 mmol), the mixture was then stirred at room temperature to react for 1 hour, followed by the addition of DMSO (10 mL), the pressure was then reduced to remove most of the THF, followed by the addition of methyl 5-amino-3-((4-(difluoromethoxy)-3-fluorobenzyl)oxy)isothiazol-4-carboxylate I125 (600 mg, 1.72 mmol) and potassium carbonate (476 mg, 3.45 mmol), and the reaction system was stirred at room temperature to react for 16 hours. After complete reaction, water (40 mL) was added to the reaction mixture, which was then extracted with ethyl acetate (15 mL x 3), the organic phases were sequentially washed with water (30 mL) and saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a yellow oily liquid product methyl 3-((4-(difluoromethoxy)-3-fluorobenzyl)oxy)-5-(3-(4-(pyrrolidin-1-yl)butyl)ureido)isothiazol-4-carboxylate I126 (600 mg, yield: 69.4%).

The product was verified by LCMS.

Step 6

3-((4-(difluoromethoxy)-3-fluorobenzyl)oxy)-5-(3-(4-(pyrrolidin-1-yl)butyl)ureido)isothiazole-4-carboxamide

In a microwave tube, methyl 3-((4-(difluoromethoxy)-3-fluorobenzyl)oxy)-5-(3-(4-(pyrrolidin-1-yl)butyl)ureido)isothiazol-4-carboxylate I126 (600 mg, 1.20 mmol) was dissolved in $NH_3$/MeOH (10 mL, 13 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1) to obtain a white solid product 3-((4-(difluoromethoxy)-3-fluorobenzyl)oxy)-5-(3-(4-(pyrrolidin-1-yl)butyl)ureido)isothiazol-4-formamide T027 (60 mg, yield: 10.3%).

The product was verified by LCMS, H-NMR and C-NMR.

$^1$H NMR (400 MHz, $CD_3OD$) δ: 7.32-7.43 (m, 3H), 6.86 (t, 1H, J=73.6Hz), 5.48 (s, 2H), 3.26 (t, 2H, J=6.0Hz), 2.51-2.60 (m, 6H), 1.81-1.84 (m, 4H), 1.59-1.63 (m, 4H).

$^{13}$C NMR (100 MHz, $CD_3OD$) δ: 168.97, 165.82, 161.67, 154.99, 154.75, 152.53, 135.80, 135.74, 124.41, 124.37, 122.18, 118.87, 116.66, 116.46, 116.28, 113.67, 97.33, 68.51, 55.73, 53.57, 39.53, 27.45, 25.43, 22.74.

Example 16: Synthesis of Compound T028

Synthesis Route:

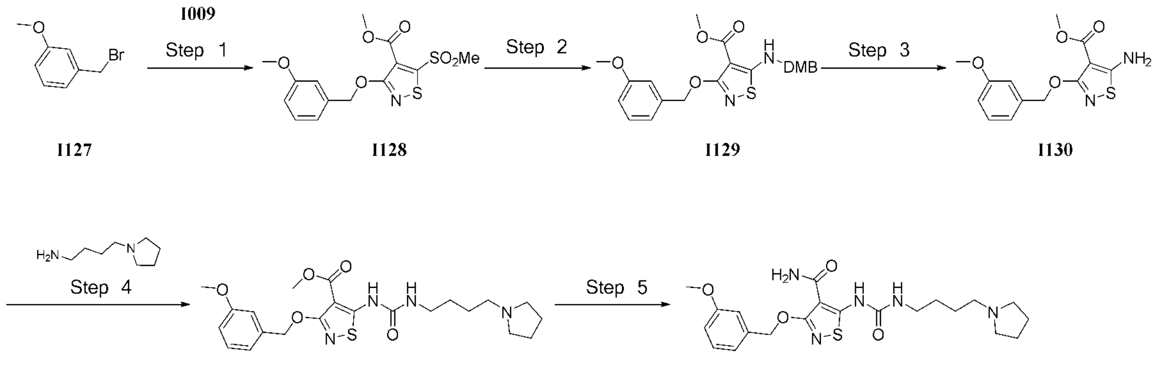


I127 I128 I129 I130

I131 T028

Step 1

Methyl 3-((3-methoxybenzyl)oxy)-5-(methylsulfonyl)isothiazole-4-carboxylate

Methyl 3-hydroxyl-5-(methylsulfonyl)isothiazol-4-carboxylate I009 (500 mg, 2.1 mmol) and potassium carbonate (580 mg, 4.2 mmol) were dissolved in DMF (5 mL), followed by the addition of 1-(bromomethyl)-3-metoxybenzene I127 (420 mg, 2.1mol), and the reaction system was stirred at room temperature to react for 1 hour. Water (30 mL) was added to the reaction mixture, which was filtered to obtain a filter cake, and the filter cake was dried to obtain a white solid product methyl 3-((3-methoxybenzyl)oxy)-5-(methylsulfonyl)isothiazol-4-carboxylate I128 (438 mg, yield: 58.4%).

The product was verified by LCMS and H-NMR.

$^1$H NMR (400 MHz, $CDCl_3$) δ: 7.32 (t, 1H, J=8.0Hz), 7.03-7.06 (m, 2H), 6.90 (dd, 1H, J=8.4, 2.0Hz), 5.49 (s, 2H), 3.98 (s, 3H), 3.85 (s, 3H), 3.50 (s, 3H).

Step 2

Methyl 5-((2,4-dimethoxybenzyl)amino)-3-((3-methoxybenzyl)oxy)isothiazole-4-carboxylate

Methyl 3-((3-methoxybenzyl)oxy)-5-(methylsulfonyl)isothiazol-4-carboxylate I128 (0.5 g, 1.4 mmol) was dissolved in THF(10 mL), followed by the addition of 2,4-dimethoxybenzylamine (2.3 g, 14 mmol), and the reaction system was stirred at 60 °C to react for 2 hours. After the reaction was finished, the reaction mixture was allowed to return to room temperature, added to cold water (40 mL), and filtered to obtain solids, which were then dried to obtain a yellow solid product methyl 5-((2,4-dimethoxybenzyl)amino)-3-((3-methoxyphenyl)oxy)isothiazol-4-carboxylate I129 (500 mg,, crude product).

The product was verified by LCMS.

Step 3

Methyl 5-amino-3-((3-methoxybenzyl)oxy)isothiazole-4-carboxylate

Methyl 5-((2,4-dimethoxybenzyl)amino)-3-((3-methoxyphenyl)oxy)isothiazol-4-carboxylate I129 (450 mg, 1. mmol) was dissolved in DCM/$H_2O$ (5/1 mL), followed by the portionwise addition of DDQ (920 mg, 4.04 mmol) at 0°C, and the reaction system was stirred at room temperature to react for 1 hour. After complete reaction, the reaction mixture was directly concentrated under reduced pressure to obtain a crude product, and the crude product was purified by the flash column chromatography (PE/EA = 5/1~3/1) to obtain a white solid product methyl 5-amino-3-((3-methoxybenzyl) oxy)isothiazol-4-carboxylate 1130 (200 mg, yield: 67.1%).

The product was verified by LCMS.

Step 4

Methyl 3-((3-methoxybenzyl)oxy)-5-(3-(4-(pyrrolidin-1-yl)butyl)ureido)isothiazole-4-carboxylate

4-(Pyrrolidin-1-yl)butyl-1-amine (102 mg, 0.72 mmol) was dissolved in anhydrous THF (5 mL) and then cooled to 0°C under the protection of $N_2$, followed by the addition of CDI (117 mg, 0.72 mmol), the mixture was then stirred at room temperature to react for 1 hour, followed by the addition of DMSO (6 mL), the pressure was then reduced to remove most of the THF, followed by the addition of methyl 5-amino-3-((3-methoxybenzyl)oxy)isothiazol-4-carboxylate I130 (150 mg, 0.51 mmol) and potassium carbonate (141 mg, 1.02 mmol), and the reaction system was stirred at room temperature to react for 1 hour. After complete reaction, water (5 mL) was added to the reaction mixture, which was filtered to obtain solids, i.e., the filter cake, which was then dried to obtain a crude product, and the crude product was purified by the preparative thin-layer chromatography (DCM/MeOH = 10/1) to obtain a yellow solid product methyl 3-((3-methoxybenzyl)oxy)-5-(3-(4-(pyrrolidin-1-yl)butyl)ureido)isothiazol-4-carboxylate I131 (75 mg, yield: 31.8%).

The product was verified by LCMS and H-NMR.

$^1$H NMR (400 MHz, $CDCl_3$) δ: 10.22 (s, 1H), 8.24 (s, 1H), 7.28-7.32 (m, 1H), 7.04-7.07 (m, 2H), 6.87 (d, 1H, J=7.6Hz), 5.44 (s, 2H), 3.89 (s, 3H), 3.84 (s, 3H), 3.33 (s, 2H), 2.61-2.64 (m, 4H), 2.54-2.56 (m, 2H),1.90 (s, 4H), 1.26-1.30 (m, 4H).

Step 5

3-((3-methoxybenzyl)oxy)-5-(3-(4-(pyrrolidin-1-yl)butyl)ureido)isothiazole-4-carboxamide

In a microwave tube, methyl 3-((3-methoxybenzyl)oxy)-5-(3-(4-(pyrrolidin-1-yl)butyl)ureido)isothiazol-4-carboxylate I131 (100 mg, 0.22 mmol) was dissolved in $NH_3$/MeOH (3 mL, 9 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1) to obtain a white solid product 3-((3-methoxybenzyl)oxy)-5-(3-(4-(pyrrolidin-1-yl)butyl)ureido)isothiazol-4-formamide T028 (15 mg, yield: 15.4%).

The product was verified by LCMS, H-NMR and C-NMR.

$^1$H NMR (400 MHz, $CDCl_3$) δ: 10.94 (br s, 1H), 7.82 (br s, 1H), 7.33 (t, 1H, J=8.0Hz), 7.19 (s, 1H), 7.03 (d, 1H, J=7.6Hz), 7.00 (d, 1H, J=2.4Hz), 6.92 (dd, 1H, J=8.0, 2.4Hz), 5.48 (s, 1H), 5.45 (s, 2H), 3.84 (s, 3H), 3.34 (s, 2H), 2.61-2.69 (m, 6H), 1.92 (s, 4H), 1.71 (s, 4H).

$^{13}$C NMR (100 MHz, $CDCl_3$) δ: 169.58, 165.95, 161.89, 159.84, 154.08, 137.46, 129.83, 120.52, 113.93, 113.91, 70.34, 55.85, 55.29, 53.91, 28.11, 26.61, 23.40.

Example 17: Synthesis of Compound T029

Synthesis Route:

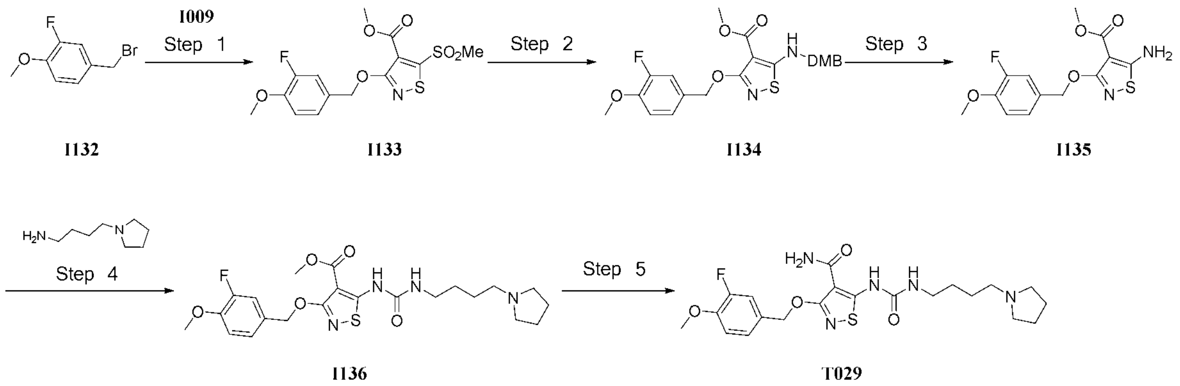


I132 I133 I134 I135

I136 T029

Step 1

Methyl 3-((3-fluoro-4-methoxybenzyl)oxy)-5-(methylsulfonyl)isothiazole-4-carboxylate

Methyl 3-hydroxyl-5-(methylsulfonyl)isothiazol-4-carboxylate I009 (500 mg, 2.1 mmol) and potassium carbonate (580 mg, 4.2 mmol) were dissolved in DMF (5 mL), followed by the addition of 4-(bromomethyl)-2-fluoro-1-metoxybenzene I132 (462 mg, 2.1 mmol), and the reaction system was stirred at room temperature to react for 1 hour. Water (30 mL) was added to the reaction mixture, which was filtered to obtain solids, namely, the filter cake, which was then dried to obtain a crude product methyl 3-((3-fluoro-4-methoxybenzyl)oxy)-5-(methylsulfonyl)isothiazol-4-carboxylate I133 (500 mg, yield: 63.2%) as a yellow solid product.

The product was verified by LCMS and H-NMR.

$^1$H NMR (400 MHz, $CDCl_3$) δ: 7.18-7.25 (m, 2H), 6.98 (t, 1H, J=8.4Hz), 5.42 (s, 2H), 3.98 (s, 3H), 3.92 (s, 3H), 3.49 (s, 3H).

Step 2

Methyl 5-((2,4-dimethoxybenzyl)amino)-3-((3-fluoro-4-methoxybenzyl)oxy)isothiazole-4-carboxylate

Methyl 3-((3-fluoro-4-methoxybenzyl)oxy)-5-(methylsulfonyl)isothiazol-4-carboxylate I133 (0.58 g, 1.5 mmol) was dissolved in THF(20 mL), followed by the addition of 2,4-dimethoxybenzylamine (2.5 g, 15 mmol), and the reaction system was stirred at 60 °C to react for 2 hours. After the reaction was finished, the reaction mixture was allowed to return to room temperature, added to water (40 mL), and filtered to obtain solids, which were then dried to obtain a yellow solid product methyl 5-((2,4-dimethoxybenzyl)amino)-3-((3-fluoro-4-methoxyphenyl)oxy)isothiazol-4-carboxylate I134 (550 mg, yield: 77.0%).

The product was verified by LCMS.

Step 3

Methyl 5-amino-3-((3-fluoro-4-methoxybenzyl)oxy)isothiazole-4-carboxylate

Methyl 5-((2,4-dimethoxybenzyl)amino)-3-((3-fluoro-4-methoxyphenyl)oxy)isothiazol-4-carboxylate I134 (500 mg, 1.08 mmol) was dissolved in DCM/$H_2O$ (5/1 mL), followed by the portionwise addition of DDQ (983mg, 4.32 mmol) at 0°C, and the reaction system was stirred at 0°C to react for 1 hour. After complete reaction, the reaction mixture was concentrated under reduced pressure to obtain a crude product, and the crude product was purified by the flash column chromatography (PE/EA = 5/1~3/1) to obtain a yellow solid product methyl 5-amino-3-((3-fluoro-4-methoxybenzyl)oxy) isothiazol-4-carboxylate I135 (220 mg, yield: 65.1%).

The product was verified by LCMS.

Step 4

Methyl 3-((3-fluoro-4-methoxybenzyl)oxy)-5-(3-(4-(pyrrolidin-1-yl)butyl)ureido)isothiazole -4-carboxylate

4-(Pyrrolidin-1-yl)butyl-1-amine (108 mg, 0.76 mmol) was dissolved in anhydrous THF (6 mL) and then cooled to 0°C under the protection of $N_2$, followed by the addition of CDI (123 mg, 0.76 mmol), the mixture was then stirred at room

temperature to react for 1 hour, followed by the addition of DMSO (6 mL), the pressure was then reduced to remove most of the THF, followed by the addition of methyl 5-amino-3-((3-fluoro-4-methoxybenzyl)oxy)isothiazol-4-carboxylate I135 (170 mg, 0.54 mmol) and potassium carbonate (149 mg, 1.08 mmol), and the reaction system was stirred at room temperature to react for 1 hour. After complete reaction, water (5 mL) was added to the reaction mixture, which was filtered to obtain solids, the solids were dried to obtain a crude product, and the crude product was purified by the preparative thin-layer chromatography (DCM/MeOH = 10/1) to obtain a yellow solid product methyl 3-((3-fluoro-4-methoxybenzyl) oxy)-5-(3-(4-(pyrrolidin-1-yl)butyl)ureido)isothiazol-4-carboxylate 1136 (100 mg, yield: 38.4%).

The product was verified by LCMS and H-NMR.

$^1$H NMR (400 MHz, $CDCl_3$) δ: 10.25 (s, 1H), 8.24 (s, 1H), 7.25-7.28 (m, 1H,), 7.17 (d, 1H, J=8.4Hz), 6.97 (t, 1H, J=8.4Hz), 5.37 (s, 2H), 3.92 (s, 3H), 3.89 (s, 3H), 3.32-3.33 (m, 2H), 2.60 (s, 4H), 2.52-2.55 (m, 2H),1.89-1.91 (m, 4H), 1.72 (s, 4H).

Step 5

3-((3-fluoro-4-methoxybenzyl)oxy)-5-(3-(4-(pyrrolidin-1-yl)butyl)ureido)isothiazole-4-carboxamide

In a microwave tube, methyl 3-((3-fluoro-4-methoxybenzyl)oxy)-5-(3-(4-(pyrrolidin-1-yl)butyl)ureido)isothiazol-4-carboxylate I136 (140 mg, 0.28 mmol) was dissolved in $NH_3$/MeOH (3 mL, 9 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1) to obtain a white solid product 3-((3-fluoro-4-methoxybenzyl)oxy)-5-(3-(4-(pyrrolidin-1-yl)butyl)ureido)isothiazol-4-formamide T029 (15 mg, yield: 11.9%).

The product was verified by LCMS, H-NMR and C-NMR.

$^1$H NMR (400 MHz, $CD_3OD$) δ: 7.25-7.29 (m, 2H), 7.13 (d, 1H, J=8.4Hz), 5.40 (s, 2H), 3.90 (s, 3H), 3.25-3.32 (m, 3H), 2.56-2.65 (m, 5H), 1.83-1.87(m, 4H), 1.61-1.62 (m, 4H).

$^{13}$C NMR (100 MHz, $CD_3OD$) δ: 168.81, 165.86, 161.94,154.76, 150.90, 147.96, 129.14, 124.71, 124.67, 115.97, 115.79, 113.26, 69.17, 55.62, 55.32, 53.59, 39.41, 29.33, 27.38, 25.21, 22.72.

Example 18: Synthesis of Compound T030

Synthesis Route:

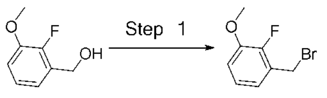


I137

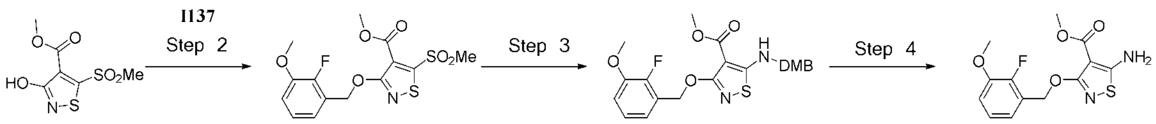


I009 I138 I139 I140

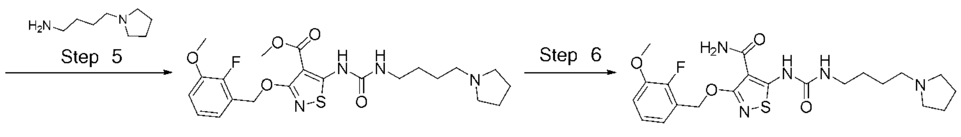


I141 T030

Step 1

1-(bromomethyl)-2-fluoro-3-methoxybenzene

(2-Fluoro-3-methoxyphenyl)methanol (0.5 g, 3.2 mmol) was dissolved in DCM (15 mL) and then cooled to 0°C, followed by the addition of $PBr_3$ (1.0 g, 3.8 mmol) under the protection of nitrogen, and the reaction system was stirred at

room temperature to react for 1 hour. After complete reaction, the reaction mixture was directly concentrated under reduced pressure to obtain a crude product 1-(bromomethyl)-2-fluoro-3-metoxybenzene 1137 (1 g, crude product) as a white product.

The product was verified by LCMS.

Step 2

Methyl 3-((2-fluoro-3-methoxybenzyl)oxy)-5-(methylsulfonyl)isothiazole-4-carboxylate

Methyl 3-hydroxyl-5-(methylsulfonyl)isothiazol-4-carboxylate I009 (0.8 g, 3.2 mmol) and potassium carbonate (1.8 g, 12.8 mmol) were dissolved in DMF (15 mL), followed by the addition of 1-(bromomethyl)-2-fluoro-3-metoxybenzene I137 (0.9 g, crude product), and the reaction system was stirred at room temperature to react for 2 hours. Water (50 mL) was added to the reaction mixture, which was then extracted with ethyl acetate (50 mL x 3), and the organic phases were sequentially washed with water (50 mL) and saturated brine (50 mL), dried with anhydrous sodium sulfate, and concentrated under reduced pressure to obtain a crude product methyl 3-((2-fluoro-3-methoxybenzyl)oxy)-5-(methylsulfonyl)isothiazol-4-carboxylate I138 (0.7 g, yield: 58.3%) as a white solid product.

The product was verified by LCMS and H-NMR.

$^1$H NMR (400 MHz, $CDCl_3$) $\delta$: 7.08-7.77 (m, 2H), 6.95-7.05 (m, 1H), 5.57 (s, 2H), 3.96 (s, 3H), 3.90 (s, 3H), 3.49 (s, 3H).

Step 3

Methyl 5-((2,4-dimethoxybenzyl)amino)-3-((2-fluoro-3-methoxybenzyl)oxy)isothiazole-4-carboxylate

Methyl 3-((2-fluoro-3-methoxybenzyl)oxy)-5-(methylsulfonyl)isothiazol-4-carboxylate I138 (0.7 g, 1.9 mmol) was dissolved in THF(10 mL), followed by the addition of 2,4-dimethoxybenzylamine (2.2 g, 13.3 mmol), and the reaction system was stirred at 60 °C to react for 1 hours. After the reaction was finished, the reaction mixture was allowed to return to room temperature, added to water (40 mL), and filtered to obtain solids, which were then washed with water (10 mL x 3) and dried to obtain a white solid product methyl 5-((2,4-dimethoxybenzyl)amino)-3-((2-fluoro-3-methoxyphenyl)oxy)isothiazol-4-carboxylate I139 (0.6 g, yield: 70.6%).

The product was verified by LCMS and H-NMR.

$^1$H NMR (400 MHz, $CDCl_3$) $\delta$: 8.13 (br s, 1H), 7.15-7.19 (m, 2H), 7.09 (t, 1H, J=8.0Hz), 6.94 (t, 1H, J=8.0Hz), 6.45-6.49 (m, 2H), 5.52 (s, 2H), 4.29 (d, 2H, J=5.6Hz), 3.92 (s, 3H), 3.87 (s, 3H), 3.83 (s, 3H), 3.82 (s, 3H).

Step 4

Methyl 5-amino-3-((2-fluoro-3-methoxybenzyl)oxy)isothiazole-4-carboxylate

Methyl 5-((2,4-dimethoxybenzyl)amino)-3-((2-fluoro-3-methoxyphenyl)oxy)isothiazol-4-carboxylate I139 (0.4 g, 0.86 mmol) was dissolved in DCM/$H_2O$ (12/2.4 mL), followed by the portionwise addition of DDQ (0.78 g, 3.4 mmol) at 0°C, and the reaction system was stirred at room temperature to react for 2 hours. After complete reaction, water (25 mL) was added to the reaction mixture, which was then extracted with dichloromethane (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (PE/EA = 2/1) to obtain a yellow solid product methyl 5-amino-3-((2-fluoro-3-methoxybenzyl)oxy)isothiazol-4-carboxylate I140 (180 mg, yield: 67.2%).

The product was verified by LCMS and H-NMR.

$^1$H NMR (400 MHz, $CDCl_3$) $\delta$: 7.15-7.17 (m, 1H), 7.10-7.12 (m, 1H), 6.95 (d, 1H, J=1.2Hz), 6.45 (br s, 2H), 5.53 (s, 2H), 3.92 (s, 3H), 3.87 (s, 3H).

Step 5

Methyl 3-((2-fluoro-3-methoxybenzyl)oxy)-5-(3-(4-(pyrrolidin-1-yl)butyl)ureido)isothiazole-4-carboxylate

4-(Pyrrolidin-1-yl)butyl-1-amine (116 mg, 0.82 mmol) was dissolved in anhydrous THF (6 mL) and then cooled to 0°C under the protection of $N_2$, followed by the addition of CDI (132 mg, 0.82 mmol), the mixture was then stirred at room temperature to react for 1 hour, followed by the addition of DMSO (6 mL), the pressure was then reduced to remove most of the THF, followed by the addition of methyl 5-amino-3-((2-fluoro-3-methoxybenzyl)oxy)isothiazol-4-carboxylate I140 (180

mg, 0.58) and potassium carbonate (160 mg, 1.16 mmol), and the reaction system was stirred at room temperature to react for 8 hours. After complete reaction, water (20 mL) was added to the reaction mixture, which was then extracted with dichloromethane (10 mL x 3), the organic phases were sequentially washed with water (30 mL) and saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-((2-fluoro-3-methoxybenzyl)oxy)-5-(3-(4-(pyrrolidin-1-yl)butyl)ureido)isothiazol-4-carboxylate I141 (130 mg, yield: 46.8%).

The product was verified by LCMS and H-NMR.

$^1$H NMR (400 MHz, $CDCl_3$) δ: 10.10 (br, 1H), 7.90 (br, 1H), 7.08-7.15 (m, 2H), 6.94 (d, 1H, J=1.6Hz), 5.53 (s, 2H), 3.91 (s, 3H), 3.88 (s, 3H), 3.33 (s, 2H), 2.68 (s, 4H), 2.60 (t, 2H, J=6.4Hz), 1.91-2.00 (m, 4H), 1.68-1.72 (m, 4H).

Step 6

3-((2-fluoro-3-methoxybenzyl)oxy)-5-(3-(4-(pyrrolidin-1-yl)butyl)ureido)isothiazole-4-carboxamide

In a microwave tube, methyl 3-((2-fluoro-3-methoxybenzyl)oxy)-5-(3-(4-(pyrrolidin-1-yl)butyl)ureido)isothiazol-4-carboxylate I141 (180 mg, 0.37 mmol) was dissolved in $NH_3$/MeOH (6 mL, 9 mol/L), and the mixture was sealed to react at 60 °C for 72 hours. The reaction mixture was concentrated under reduced pressure to obtain a crude product, and the crude product was purified by the preparative thin-layer chromatography (DCM/MeOH/$NH_3$.$H_2O$ = 10/1/0.1) to obtain a white solid product 3-((2-fluoro-3-methoxybenzyl)oxy)-5-(3-(4-(pyrrolidin-1-yl)butyl)ureido)isothiazol-4-formamide T030 (25 mg, yield: 12.9%).

The product was verified by LCMS, H-NMR and C-NMR.

$^1$H NMR (400 MHz, DMSO-$d_6$) δ: 11.00 (s, 1H), 8.20 (s, 1H), 7.64 (s, 1H), 7.14-7.19 (m, 2H), 7.08-7.10 (m, 1H), 6.95 (s, 1H), 5.47 (s, 2H), 3.85 (s, 3H), 3.12 (d, 2H, J=5.6Hz), 2.37-2.40 (m, 6H), 1.64-1.68 (m, 4H), 1.46 (s, 4H).

$^{13}$C NMR (100 MHz, DMSO-$d_6$) δ: 168. 61, 164.82, 161.68, 154.51, 124.88, 124.38, 114.53, 97.81, 64.07, 56.52, 55.68, 54.04, 27.65, 26.11, 23.54.

Example 19: Synthesis of Compound T032

Synthesis Route:

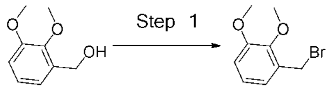


I147

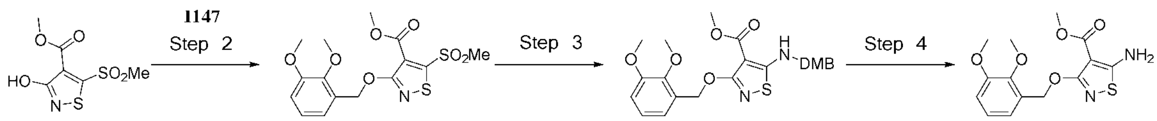


I009 I148 I149 I150

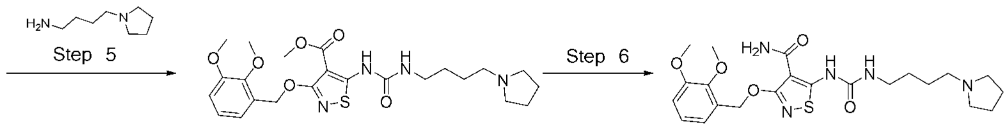


I151 T032

Step 1

1-(bromomethyl)-2,3-dimethoxybenzene

(2,3-Dimethoxyphenyl)methanol (0.5 g, 2.97 mmol) was dissolved in DCM (15 mL) and then cooled to 0°C, followed by the addition of $PBr_3$ (0.27 g, 0.99 mmol), and the reaction system was stirred below 10°C to react for about 1

hour. After complete reaction, the reaction mixture was poured into water (40 mL) and extracted with dichloromethane, and the organic phases were combined, sequentially washed with water saturated brine, dried with anhydrous sodium sulfate, and concentrated under reduced pressure to obtain a crude product 1-(bromomethyl)-2,3-dimetoxybenzene I147 (0.6 g, yield: 87.3%) as a colorless oily liquid.

The product was verified by LCMS and H-NMR.

$^1$H NMR (400 MHz, $CDCl_3$) δ: 7.05 (t, J = 8.0 Hz, 1H), 6.98 (dd, J = 7.6, 1.6 Hz, 1H), 6.90 (dd, J = 8.0, 1.6 Hz, 1H), 4.59 (s, 2H), 3.98 (s, 3H), 3.89 (s, 3H).

Step 2

Methyl 3-((2,3-dimethoxybenzyl)oxy)-5-(methylsulfonyl)isothiazole-4-carboxylate

Methyl 3-hydroxyl-5-(methylsulfonyl)isothiazol-4-carboxylate I009 (0.51 g, 2.16mmol) and potassium carbonate (0.90 g, 6.49 mmol) was dissolved in DMF (5 mL), followed by the addition of 1-(bromomethyl)-2,3-dimetoxybenzene I147 (0.6 g, 2.60 mmol), and the reaction system was stirred at room temperature to react for 1 hour. Water (30 mL) was added to the reaction mixture, which was then filtered to obtain solids, namely, the filter cake, which was then washed with water and dried to obtain a crude product methyl 3-((2,3-dimethoxybenzyl)oxy)-5-(methylsulfonyl)isothiazol-4-carboxylate I148 (0.6 g, yield: 72.0%) as a white solid product.

The product was verified by LCMS and H-NMR.

$^1$H NMR (400 MHz, $CDCl_3$) δ: 7.05-7.14 (m, 2H), 6.93-6.99 (m, 1H), 5.57 (s, 2H), 3.95 (s, 3H), 3.92 (s, 3H), 3.91 (s, 3H), 3.50 (s, 3H).

Step 3

Methyl 5-((2,4-dimethoxybenzyl)amino)-3-((2,3-dimethoxybenzyl)oxy)isothiazole-4-carboxylate

Methyl 3-((2,3-dimethoxybenzyl)oxy)-5-(methylsulfonyl)isothiazol-4-carboxylate I148 (0.5 g, 1.29 mmol) was dissolved in THF(5 mL), followed by the addition of 2,4-dimethoxybenzylamine (2.16 g, 12.9 mmol), and the reaction system was stirred at 65 °C to react for 1 hour. After the reaction was finished, the reaction mixture was added to water (30 mL), and filtered to obtain solids, namely, the filter cake, which was then washed with water and dried to obtain a yellow solid product methyl 5-((2,4-dimethoxybenzyl)amino)-3-((2,3-dimethoxybenzyl)oxy)isothiazol-4-carboxylate I149 (0.54 g, yield: 88.4%).

The product was verified by LCMS and H-NMR.

$^1$H NMR (400 MHz, $CDCl_3$) δ: 8.14 (t, *J* = 6.0 Hz, 1H), 7.15-7.21 (m, 2H), 7.09 (t, *J* = 7.9 Hz, 1H), 6.91 (dd, *J* = 8.1, 1.6 Hz, 1H), 6.44-6.51 (m, 2H), 5.49 (s, 2H), 3.90 (s, 3H), 3.90 (s, 3H), 3.87 (s, 3H), 3.81-3.83 (m, 6H).

Step 4

Methyl 5-amino-3-((2,3-dimethoxybenzyl)oxy)isothiazole-4-carboxylate

Methyl 5-((2,4-dimethoxybenzyl)amino)-3-((2,3-dimethoxybenzyl)oxy)isothiazol-4-carboxylate I149 (0.6 g, 1.26 mmol) was dissolved in DCM/$H_2O$ (6/1.2 mL), followed by the portionwise addition of DDQ (0.34 g, 1.52 mmol) at 0°C, and the reaction system was stirred at room temperature to react for half an hour. After complete reaction, neutral alumina was added to the reaction mixture, which was then concentrated under reduced pressure to remove most of the solution, and the crude product was purified by the column chromatography (neutral alumina, PE/EA = 10/1~1/1) to obtain a white solid product methyl 5-amino-3-((2,3-dimethoxybenzyl)oxy)isothiazol-4-carboxylate I150 (0.35 g, yield: 85.3%).

The product was verified by LCMS and H-NMR.

$^1$H NMR (400 MHz, $CDCl_3$) δ: 7.18 (dd, *J* = 7.8, 1.6 Hz, 1H), 7.10 (t, *J* = 7.9 Hz, 1H), 6.93 (dd, *J* = 8.1, 1.6 Hz, 1H), 6.38-6.49 (m, 2H), 5.51 (s, 2H), 3.90-3.91 (m, 6H), 3.85 (s, 3H).

Step 5

Methyl 3-((2,3-dimethoxybenzyl)oxy)-5-(3-(4-(pyrrolidin-1-yl)butyl)ureido)isothiazole-4-carboxylate

4-(Pyrrolidin-1-yl)butyl-1-amine (123 mg, 0.86 mmol) was dissolved in anhydrous THF (5 mL) and then cooled to 0°C under the protection of $N_2$, followed by the addition of CDI (140 mg, 0.86 mmol), the mixture was stirred at room temperature to react for 1 hour, followed by the addition of DMSO (5 mL), the pressure was then reduced to remove most of the THF, followed by the addition of methyl 5-amino-3-((2,3-dimethoxybenzyl)oxy)isothiazol-4-carboxylate I150 (200 mg,

0.62 mmol) and potassium carbonate (170 mg, 1.23 mmol), and the reaction system was stirred at room temperature to react for 1 hour. After complete reaction, wate (10 mL)r was added to the reaction mixture, which was filtered to obtain solids, i.e., the filter cake, which was then washed with water and dried to obtain a crude product methyl 3-((2,3-dimethoxybenzyl)oxy)-5-(3-(4-(pyrrolidin-1-yl)butyl)ureido)isothiazol-4-carboxylate I151 (200 mg, yield: 65.8%) as a white slid product.

The product was verified by LCMS and H-NMR.

$^1$H NMR (400 MHz, $CDCl_3$) δ: 10.22 (br s, 1H), 8.20 (br s, 1H), 7.18 (dd, *J* = 7.7, 1.6 Hz, 1H), 7.09 (t, *J* = 7.9 Hz, 1H), 6.92 (dd, *J* = 8.1, 1.5 Hz, 1H), 5.52 (s, 2H), 3.90-3.91 (m, 6H), 3.86 (s, 3H), 3.33 (d, *J* = 5.4 Hz, 2H), 2.47-2.63 (m, 6H), 1.84-1.96 (m, 4H), 1.68-1.74 (m, 4H).

Step 6

3-((2,3-dimethoxybenzyl)oxy)-5-(3-(4-(pyrrolidin-1-yl)butyl)ureido)isothiazole-4-carboxamide

In a microwave tube, methyl 3-((2,3-dimethoxybenzyl)oxy)-5-(3-(4-(pyrrolidin-1-yl)butyl)ureido)isothiazol-4-carboxylate I151 (200 mg, 0.42 mmol) was dissolved in $NH_3$/MeOH (14 mL,10 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((2,3-dimethoxybenzyl)oxy)-5-(3-(4-(pyrrolidin-1-yl)butyl)ureido)isothiazol-4-formamide T032 (27 mg, yield: 13.9%).

The product was verified by LCMS, H-NMR and C-NMR.

$^1$H NMR (400 MHz, $CDCl_3$) δ: 10.91 (br s, 1H), 7.76 (br s, 1H), 7.25-7.29 (m, 1H), 7.09 (t, J= 7.8 Hz, 1H), 7.03 (dd, *J* = 7.8, 1.7 Hz, 1H), 6.96 (dd, *J* = 8.0, 1.7 Hz, 1H), 5.65 (s, 1H), 5.52 (s, 2H), 3.89-3.90 (m, 6H), 3.26-3.37 (m, 2H), 2.52-2.63 (m, 4H), 2.51 (t, 2H, J=6.4Hz), 1.74-1.92 (m, 4H), 1.58-1.73 (m, 4H).

$^{13}$C NMR (100 MHz, $CDCl_3$) δ: 169.46, 165.95, 162.04, 154.02, 152.80, 147.81, 129.68, 124.16, 121.94, 112.97, 97.39, 65.79, 61.08, 55.87, 55.81, 53.91, 40.56, 28.13, 26.73, 23.40.

Example 20: Synthesis of Compound T033

Synthesis Route:

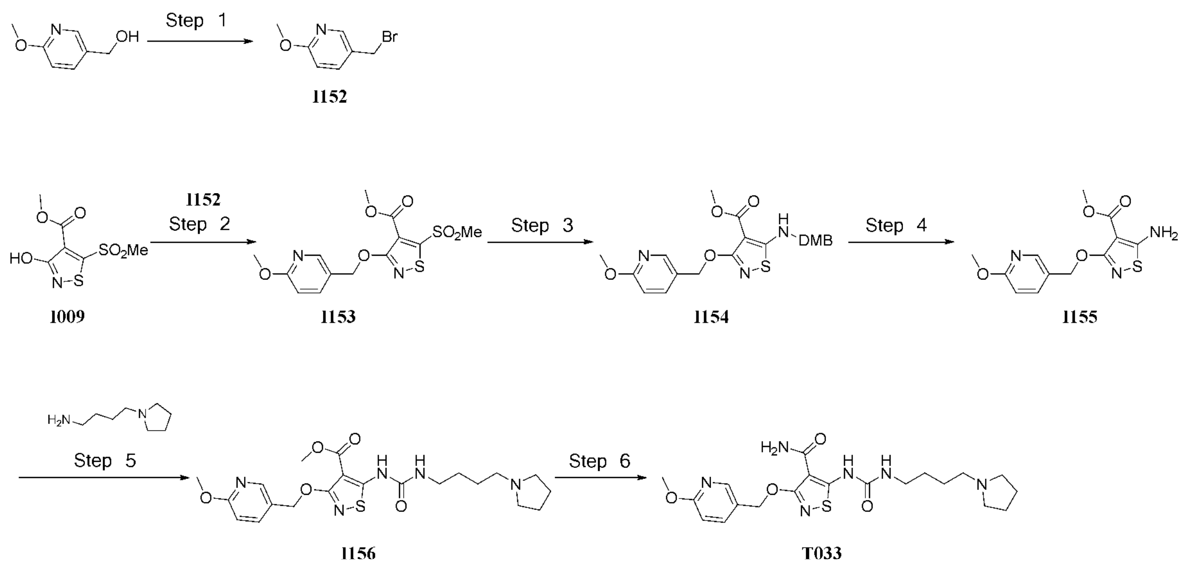


I152, I009, I153, I154, I155, I156, T033

Step 1

5-(bromomethyl)-2-methoxypyridine

(6-Methoxypyridin-3-yl)methanol (0.5 g, 3.6 mmol) was dissolved in DCM (15 mL) and then cooled to 0°C, followed by the addition of $PBr_3$ (1.1 g, 4.0 mmol), and the reaction system was stirred at room temperature to react for

about 4 hours. After complete reaction, the reaction mixture was directly concentrated under reduced pressure to obtain a crude product 5-(bromomethyl)-2-methoxypyridine I152 (1.0 g, crude product) as a yellow solid product.

The product was verified by LCMS and then directly used in the next step of reaction.

Step 2

Methyl 3-((6-methoxypyridin-3-yl)methoxy)-5-(methylsulfonyl)isothiazole-4-carboxylate

Methyl 3-hydroxyl-5-(methylsulfonyl)isothiazol-4-carboxylate I009 (0.85 g, 3.6 mmol) and potassium carbonate (2.0 g, 14.4 mmol) were dissolved in DMF (15 mL), followed by the addition of 5-(bromomethyl)-2-methoxypyridine I152 (1.0 g, crude product), and the reaction system was stirred at room temperature to react for 2 hours. Water (50 mL) was added to the reaction mixture, which was then filtered to obtain solids, namely, the filter cake, which was then washed with water and dried to obtain a crude product methyl 3-((6-methoxypyridin-3-yl)methoxy)-5-(methylsulfonyl)isothiazol-4-carboxylate I153 (0.8 g, yield: 61.5%) as a white solid product.

The product was verified by LCMS and H-NMR.

$^1$H NMR (400 MHz, $CDCl_3$) δ: 8.29 (s, 1H), 7.72 (d, 2H, J=8.4Hz),6.79 (d, 2H, J=8.4Hz), 5.44 (s, 2H), 3.97 (s, 3H), 3.95 (s, 3H), 3.49 (s, 3H).

Step 3

Methyl 5-((2,4-dimethoxyphenyl)amino)-3-((6-methoxypyridin-3-yl)methoxy)isothiazole-4-carboxylate

Methyl 3-((6-methoxypyridin-3-yl)methoxy)-5-(methylsulfonyl)isothiazol-4-carboxylate I153 (0.79 g, 2.2 mmol) was dissolved in THF(15 mL), followed by the addition of 2,4-dimethoxybenzylamine (.6 g, 15.4 mmol), and the reaction system was stirred at 60 °C to react for 1 hour. After the reaction was finished, the reaction mixture was added to cold water (50 mL), and filtered to obtain solids, which were then washed with water and dried to obtain a white solid product methyl 5-((2,4-dimethoxyphenyl)amino)-3-((6-methoxypyridin-3-yl)methoxy)isothiazol-4-carboxylate I154 (0.7 g, yield: 71.4%).

The product was verified by LCMS and H-NMR.

$^1$H NMR (400 MHz, $CDCl_3$) δ: 8.27 (d, 1H, J=2.4Hz), 8.13 (t, 1H, J=6.0Hz), 7.71 (dd, 1H, J=8.4, 2.4Hz), 7.18 (d, 1H, J=8.4Hz), 6.76 (d, 1H, J=8.4Hz), 6.44-6.49 (m, 2H), 5.36 (s, 2H), 4.28 (d, 1H, J=6.4Hz), 3.96 (s, 3H), 3.86 (s, 3H), 3.82 (s, 3H), 3.80 (s, 3H).

Step 4

Methyl 5-amino-3-((6-methoxypyridin-3-yl)methoxy)isothiazole-4-carboxylate

Methyl 5-((2,4-dimethoxyphenyl)amino)-3-((6-methoxypyridin-3-yl)methoxy)isothiazol-4-carboxylate I154 (0.67 g, 1.50 mmol) was dissolved in DCM/$H_2O$ (25/5 mL), followed by the portionwise addition of DDQ (1.37 g, 6.02 mmol) at 0°C, and the reaction system was stirred at room temperature to react for 1 hour. After complete reaction, saturated sodium bicarbonate (25 mL) was added to the reaction mixture, which was then extracted with DCM (25 mL x 2), the organic phases were combined, washed with saturated brine (20 mL), dried with anhydrous sodium sulfate, and concentrated under reduced pressure to obtain a crude product, and the crude product was purified by the preparative thin-layer chromatography (PE/EA = 1/1) to obtain a yellow solid product methyl 5-amino-3-((6-methoxypyridin-3-yl)methoxy) isothiazol-4-carboxylate I155 (290 mg, yield: 65.3%).

The product was verified by LCMS and H-NMR.

$^1$H NMR (400 MHz, $CDCl_3$) δ: 8.28 (d, 1H, J=2.4Hz), 7.72(dd, 1H, J=8.4, 2.4Hz), 6.77 (d, 1H, J=8.4Hz), 6.45 (s, 2H), 5.37 (s, 2H), 3.96 (s, 3H), 3.84 (s, 3H).

Step 5

Methyl 3-((6-methoxypyridin-3-yl)methoxy)-5-(3-(4-(pyrrolidin-1-yl)butyl)ureido) isothiazole-4-carboxylate

4-(Pyrrolidin-1-yl)butyl-1-amine (189 mg, 1.33 mmol) was dissolved in anhydrous THF (5 mL) and then cooled to 0°C under the protection of $N_2$, followed by the addition of CDI (191 mg, 1.33 mmol), the mixture was stirred at room temperature to react for 1 hour, followed by the addition of DMSO (5 mL), the pressure was then reduced to remove most of the THF, followed by the addition of methyl 5-amino-3-((6-methoxypyridin-3-yl)methoxy)isothiazol-4-carboxylate I155 (280 mg, 0.95 mmol) and potassium carbonate (262 mg, 1.90 mmol), and the reaction system was stirred at room temperature to react for 8 hours. After complete reaction, water (20 mL) was added to the reaction mixture, which was then

extracted with dichloromethane (10 mL x 3), the organic phases were washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-((6-methoxypyridin-3-yl)methoxy)-5-(3-(4-(pyrrolidin-1-yl)butyl)ureido)isothiazol-4-carboxylate I156 (230 mg, yield: 52.3%).

The product was verified by LCMS and H-NMR.

$^1$H NMR (400 MHz, $CDCl_3$) δ: 10.30 (br s, 1H), 8.28 (d, 1H, J=2.0Hz), 8.01 (br s, 1H), 7.71 (dd, 1H, J=8.4, 2.4Hz), 6.76 (d, 1H, J=8.8Hz), 5.37 (s, 2H), 3.95 (s, 3H), 3.85 (s, 3H), 3.32 (s, 2H), 2.51-2.60 (m, 6H), 1.87-1.89 (m, 4H), 1.66-1.68 (m, 4H).

Step 6

3-[(6-methoxypyridin-3-yl)methoxy]-5-({[4-(pyrrolidin-1-yl)butyl]carbamoyl}amino)-4,5-dihydro-1,2-thiazole-4-carboxamide

In a microwave tube, methyl 3-((6-methoxypyridin-3-yl)methoxy)-5-(3-(4-(pyrrolidin-1-yl)butyl)ureido)isothiazol-4-carboxylate I156 (220 mg, 0.47 mmol) was dissolved in $NH_3$/MeOH (10 mL,10 mol/L), and the mixture was sealed to react at 60 °C for 72 hours. The reaction mixture was concentrated under reduced pressure to obtain a crude product, and the crude product was purified by the preparative thin-layer chromatography (DCM/MeOH = 10/1) to obtain a white solid product 3-[(6-methoxypyridin-3-yl)methoxy]-5-({[4-(pyrrolidin-1-yl)butyl]carbamoyl}amino)-4,5-dihydro-1,2-thiazol-4-formamide T033 (23 mg, yield: 10.8%). The product was verified by LCMS, H-NMR and C-NMR.

$^1$H NMR (400 MHz, $CD_3OD$) δ: 8.29 (d, 1H, J=2.4Hz), 7.86 (dd, 1H, J=8.4, 2.0Hz), 6.84 (d, 1H, J=8.8Hz), 5.44 (s, 2H), 3.93 (s, 3H), 3.26 (t, 2H, J=6.8Hz), 2.69 (s, 4H), 2.62 (t, 2H, J=7.2Hz), 1.84-1.88 (m, 4H), 1.59-1.62 (m, 4H).

$^{13}$C NMR (100 MHz, $CD_3OD$) δ: 168.85, 165.81, 164.44, 161.84, 154.76, 147.24, 139.88, 125.06, 110.34, 67.10, 55.43, 53.61, 52.77, 39.23, 27.26, 24.84, 22.71.

Example 21: Synthesis of Compound T035

Synthesis Route:

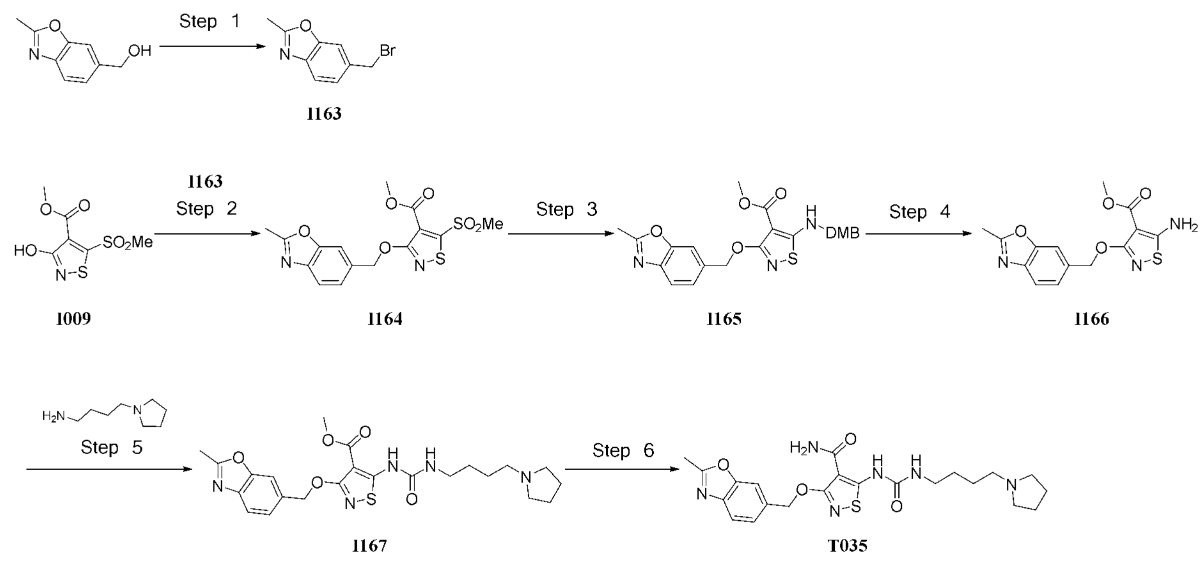


Step 1

6-(bromomethyl)-2-methylbenzo[d]oxazole

(2-Methyl-1,3-benzooxazol-6-yl)methanol (1 g, 6.1 mmol) was dissolved in DCM (10 mL) and then cooled to 0°C, followed by the addition of $PBr_3$ (1.66 g, 6.1 mmol), and the reaction system was stirred at room temperature to react for about half an hour. After complete reaction, the reaction mixture was directly concentrated under reduced pressure to obtain a crude product 6-(bromomethyl)-2-methylbenzo[d]oxazole I163 (1.1 g, crude product) as a yellow solid product.

The product was verified by LCMS.

Step 2

Methyl 3-((2-methylbenzo[d]oxazol-6-yl)methoxy)-5-(methylsulfonyl)isothiazole-4-carboxylate

Methyl 3-hydroxyl-5-(methylsulfonyl)isothiazol-4-carboxylate I009 (1 g, 4.42 mmol) and potassium carbonate (0.61 g, 4.42 mmol) were dissolved in DMF (20 mL), followed by the addition of 6-(bromomethyl)-2-methylbenzo[d]oxazole I163 (1.05 g, 4.42 mmol), and the reaction system was stirred at room temperature to react for 2 hours. Water (20 mL) was added to the reaction mixture, which was then filtered to obtain solids, and the solids were dried to obtain a crude product methyl 3-((2-methylbenzo[d]oxazol-6-yl)methoxy)-5-(methylsulfonyl)isothiazol-4-carboxylate I164 (1 g, yield: 59.2%) as a yellow solid.

The product was verified by LCMS and H-NMR.

$^1$H NMR (400 MHz, DMSO-$d_6$) δ: 7.79 (s, 1H), 7.69 (s, 1H, J=8.0Hz), 7.47 (s, 1H, J=0.8Hz), 5.60 (s, 2H), 3.88 (s, 3H), 3.62 (s, 3H), 2.62 (s, 3H).

Step 3

Methyl 5-((2,4-dimethoxybenzyl)amino)-3-((2-methylbenzo[d]oxazol-6-yl)methoxy)isothiazole-4-carboxylate

Methyl 3-((2-methylbenzo[d]oxazol-6-yl)methoxy)-5-(methylsulfonyl)isothiazol-4-carboxylate I164 (1 g, 2.61 mmol) was dissolved in THF(10 mL), followed by the addition of 2,4-dimethoxybenzylamine (4.37 g, 26.15 mmol), and the reaction system was stirred at 65 °C to react for 1 hour. After the reaction was finished, the reaction mixture was added to water (30 mL) and then filtered to obtain solids, which were then dried to obtain a yellow solid product methyl 5-((2,4-dimethoxybenzyl)amino)-3-((2-methylbenzo[d]oxazol-6-yl)methoxy)isothiazol-4-carboxylate I165 (550 mg, yield: 77%).

The product was verified by LCMS and H-NMR.

$^1$H NMR (400 MHz, DMSO-$d_6$) δ: 8.41 (t, 1H, J=6.0Hz), 7.70 (s, 1H), 7.63 (d, 1H, J=8.0Hz), 7.38-7.40 (m, 1H), 7.16 (d, 1H, J=8.0Hz), 6.59 (d, 1H, J=2.4Hz), 6.50 (dd, 1H, J=8.0, 2.0Hz), 5.43 (s, 2H), 4.27 (d, 1H, J=6.0Hz), 3.82 (s, 3H), 3.73-3.74 (m, 6H), 2.60 (s, 3H).

Step 4

Methyl 5-amino-3-((2-methylbenzo[d]oxazol-6-yl)methoxy)isothiazole-4-carboxylate Methyl 5-((2,4-dimethoxybenzyl)amino)-3-((2-methylbenzo[d]oxazol-6-yl)methoxy)isothiazol-4-carboxylate I165 (1 g, 2.13 mmol) was dissolved in DCM/$H_2O$ (10/2 mL), followed by the portionwise addition of DDQ (1.93 g, 8.52 mmol) at 0°C, and the mixture was stirred to react for 1 hour. After complete reaction, water (100 mL) was added to the reaction mixture, which was then extracted with DCM (30 mL x2), the organic phases were combined, washed with saturated brine (20 mL), dried with anhydrous sodium sulfate, and concentrated under reduced pressure to obtain a crude product, and the crude product was purified by the column chromatography (PE/EA = 5/1~2/1) to obtain a yellow solid product methyl 5-amino-3-((2-methylbenzo[d]oxazol-6-yl)methoxy)isothiazol-4-carboxylate I166 (300 mg, yield: 44.1%).

The product was verified by LCMS and H-NMR.

$^1$H NMR (400 MHz, DMSO-$d_6$) δ: 7.92 (s, 2H), 7.73 (s, 1H), 7.65 (d, 1H, J=7.6Hz), 7.40-7.43 (m, 1H), 6.64-6.70 (m, 1H), 5.44 (s, 2H), 3.72 (s, 3H), 2.62 (s, 3H).

Step 5

Methyl 3-((2-methylbenzo[d]oxazol-6-yl)methoxy)-5-(3-(4-(pyrrolidin-1-yl)butyl)ureido)isothiazole-4-carboxylate

4-(Pyrrolidin-1-yl)butyl-1-amine (134 mg, 0.94 mmol) was dissolved in anhydrous THF (5 mL) and then cooled to 0°C under the protection of $N_2$, followed by the addition of CDI (300 mg, 0.94 mmol), the mixture was stirred at room temperature to react for 1 hour, followed by the addition of DMSO (5 mL), the pressure was then reduced to remove most of the THF, followed by the addition of methyl 5-amino-3-((2-methylbenzo[d]oxazol-6-yl)methoxy)isothiazol-4-carboxylate I166 (300 mg, 0.94 mmol) and potassium carbonate (130 mg, 0.94 mmol), and the reaction system was stirred at room temperature to react for 2 hours. After complete reaction, water (20 mL) was added to the reaction mixture, which was then extracted with ethyl acetate (20 mL x 3), and the organic phases were sequentially washed with water (30 mL) and saturated brine (30 mL), dried with anhydrous sodium sulfate, and concentrated under reduced pressure to obtain a crude product methyl 3-((2-methylbenzo[d]oxazol-6-yl)methoxy)-5-(3-(4-(pyrrolidin-1-yl)butyl)ureido)isothiazol-4-carboxylate

I167 (200 mg, yield: 43.7%) as a yellow solid product.

The product was verified by LCMS.

Step 6

3-((2-methylbenzo[d]oxazol-6-yl)methoxy)-5-(3-(4-(pyrrolidin-1-yl)butyl)ureido) isothiazole-4-carboxamide

In a microwave tube, methyl 3-((2-methylbenzo[d]oxazol-6-yl)methoxy)-5-(3-(4-(pyrrolidin-1-yl)butyl)ureido) isothiazol-4-carboxylate I167 (200 mg, 0.41 mmol) was dissolved in $NH_3$/MeOH (5 mL, 9 mol/L), and the mixture was sealed to react at 60 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((2-methylbenzo[d]oxazol-6-yl)methoxy)-5-(3-(4-(pyrrolidin-1-yl)butyl)ureido) isothiazol-4-formamide T035 (15 mg, yield: 11.9%).

The product was verified by LCMS, H-NMR and C-NMR.

$^1H$ NMR (400 MHz, $CDCl_3$) δ: 10.96 (br s, 1H), 7.75 (br s, 1H), 7.68 (d, 1H, J=8Hz), 7.56 (s, 1H), 7.40-7.42 (m, 2H), 7.13 (s, 1H), 5.57 (s, 2H), 5.53 (s, 1H), 3.34 (s, 2H), 2.67-2.71 (s, 3H), 2.57 (s, 3H), 1.92 (s, 4H), 1.71 (s, 4H).

$^{13}C$ NMR (100 MHz, $CDCl_3$) δ: 169.62, 165.97, 164.74, 161.73, 154.15, 151.07, 141.83, 132.67, 124.74, 119.51, 110.55, 70.40, 55.81, 53.88, 40.91 28.00, 26.43, 23.36, 14.59, 1.02.

Example 22: Synthesis of Compound T036

Synthesis Route:

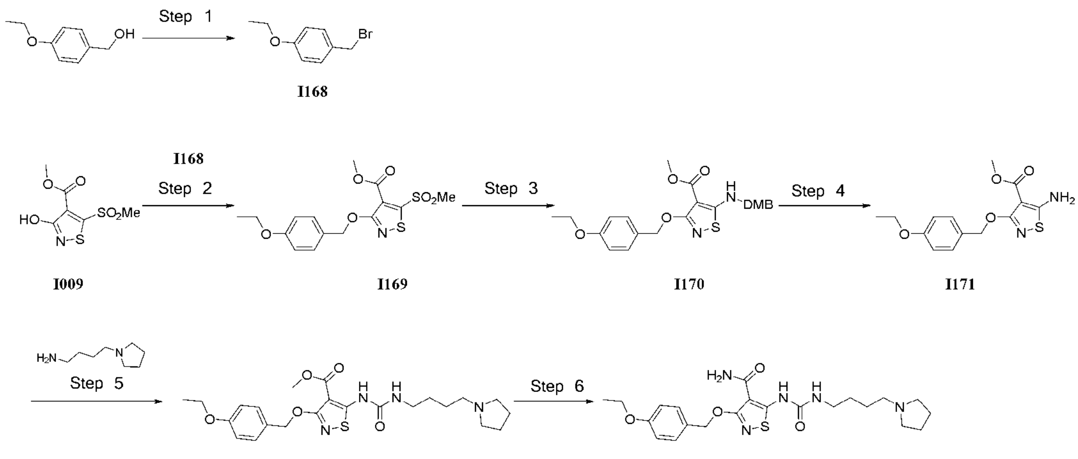


I168

I009 I169 I170 I171

I172 T036

Step 1

1-(bromomethyl)-4-ethoxybenzene

(4-Ethoxyphenyl)methanol (500 mg, 3.29 mmol) was dissolved in DCM (15 mL) and then cooled to 0°C, followed by the addition of $PBr_3$ (296 mg, 1.10 mmol), and the reaction system was stirred at 10°C to react for about 1 hour. After complete reaction, the mixture was washed with water and saturated brine, and the organic phases were dried with anhydrous sodium sulfate, and then concentrated under reduced pressure to obtain a crude product 1-(bromomethyl)-4-ethoxybenzene I168 (600 mg, yield: 84.9%) as a yellow oily liquid product.

The product was verified by LCMS and H-NMR.

$^1H$ NMR (400 MHz, $CDCl_3$) δ: 7.32-7.35 (m, 2H), 6.86-6.89 (m, 2H), 4.53 (s, 2H), 4.06 (q, 2H, J=7.2Hz), 1.44 (t, 3H, J=7.2Hz).

Step 2

Methyl 3-((4-ethoxybenzyl)oxy)-5-(methylsulfonyl)isothiazole-4-carboxylate

Methyl 3-hydroxyl-5-(methylsulfonyl)isothiazol-4-carboxylate I009 (500 mg, 2.11 mmol) and potassium carbonate (74 mg, 6.32 mmol) were dissolved in DMF (5 mL), followed by the addition of 1-(bromomethyl)-4-ethoxybenzene I168 (544 mg, 2.53 mmol), and the reaction system was stirred at room temperature to react for 1 hour. Water (20 mL) was added to the reaction mixture, which was then filtered to obtain solids, namely, the filter cake, which was then washed with water and dried to obtain a crude product methyl 3-((4-ethoxybenzyl)oxy)-5-(methylsulfonyl)isothiazol-4-carboxylate I169 (610 mg, yield: 77.9%) as a light white solid product.
The product was verified by LCMS and H-NMR.
$^1$H NMR (400 MHz, $CDCl_3$) δ: 7.40 (d, 2H, J=8.8Hz), 6.92 (d, 2H, J=8.8Hz), 5.44 (s, 2H), 4.07 (q, 2H, J=7.2Hz), 3.96 (s, 3H), 3.48 (s, 3H), 1.44 (t, 3H, J=7.2Hz).

Step 3

Methyl 5-((2,4-dimethoxybenzyl)amino)-3-((4-ethoxybenzyl)oxy)isothiazole-4-carboxylate

Methyl 3-((4-ethoxybenzyl)oxy)-5-(methylsulfonyl)isothiazol-4-carboxylate I169 (500 mg, 1.35 mmol) was dissolved in THF(5 mL), followed by the addition of 2,4-dimethoxybenzylamine (2.25 g, 13.5 mmol), and the reaction system was stirred at 65 °C to react for 1 hour. After the reaction was finished, water(20 mL) was added to the reaction mixture, which was then extracted with ethyl acetate (50 mL x 3), the organic phases were combined, sequentially washed with water (20 mL) and saturated brine (20 mL), dried with anhydrous sodium sulfate, and concentrated under reduced pressure to obtain a crude product, and the crude product was purified by the flash column chromatography (PE/EA = 10/1-5/1-3/1) to obtain a light white solid product methyl 5-((2,4-dimethoxybenzyl)amino)-3-((4-ethoxybenzyl)oxy)isothiazol-4-carboxylate I170 (550 mg, yield: 89.1%).
The product was verified by LCMS and H-NMR.
$^1$H NMR (400 MHz, $CDCl_3$) δ: 8.13 (t, 1H, J=6.0Hz), 7.41 (d, 2H, J=2.8Hz), 7.18 (d, 1H, J=8.4Hz), 6.89-6.92 (m, 2H), 6.45-6.49 (m, 2H), 5.37 (s, 2H), 4.29 (d, 2H, J=6.0Hz), 4.06 (q, 2H, J=7.2Hz), 3.86(s, 3H), 3.83 (s, 3H), 3.81 (s, 3H), 1.43 (t, 3H, J=6.8Hz).

Step 4

Methyl 5-amino-3-((4-ethoxybenzyl)oxy)isothiazole-4-carboxylate

Methyl 5-((2,4-dimethoxybenzyl)amino)-3-((4-ethoxybenzyl)oxy)isothiazol-4-carboxylate I170 (550 mg, 1.20 mmol) was dissolved in DCM/$H_2O$ (11/2.2 mL), followed by the portionwise addition of DDQ (817 mg, 3.60 mmol) at 0°C, and the reaction system was stirred at room temperature to react for 1 hour. After complete reaction, water (40 mL) was added to the reaction mixture, which was then filtered to remove solids, the filtrate was extracted with dichloromethane, the organic phases were combined, washed with saturated brine, dried with anhydrous sodium sulfate, and concentrated under reduced pressure to obtain a crude product, and the crude product was purified by the column chromatography (PE/EA = 20/1-10/1-5/1-1/1) to obtain a yellow solid product methyl 5-amino-3-((4-ethoxybenzyl)oxy)isothiazol-4-carboxylate I171 (290 mg, yield: 78.4%).
The product was verified by LCMS and H-NMR.
$^1$H NMR (400 MHz, $CDCl_3$) δ: 7.40 (d, 2H, J=8.8Hz), 6.90-6.92 (m, 2H), 6.44 (s, 2H), 5.38 (s, 2H), 4.06 (q, 2H, J=7.2Hz), 3.85 (s, 3H), 1.44 (t, 3H, J=7.2Hz).

Step 5

Methyl 3-((4-ethoxybenzyl)oxy)-5-(3-(4-(pyrrolidin-1-yl)butyl)ureido)isothiazole-4-carboxylate

4-(Pyrrolidin-1-yl)butyl-1-amine (129 mg, 0.91 mmol) was dissolved in anhydrous THF (5 mL) and then cooled to 0°C under the protection of $N_2$, followed by the addition of CDI (147 mg, 0.91 mmol), the mixture was stirred at room temperature to react for 1 hour, followed by the addition of DMSO (5 mL), the pressure was then reduced to remove most of the THF, followed by the addition of methyl 5-amino-3-((4-ethoxybenzyl)oxy)isothiazol-4-carboxylate I171 (200 mg, 0.65 mmol) and potassium carbonate (179 mg, 1.30 mmol), and the reaction system was stirred at room temperature to react for 1 hour. After complete reaction, water (10 mL) was added to the reaction mixture, which was filtered to obtain solids, i.e., the filter cake, which was then washed with water and dried to obtain a crude product methyl 3-((4-ethoxybenzyl)oxy)-5-(3-(4-(pyrrolidin-1-yl)butyl)ureido)isothiazol-4-carboxylate I172 (180 mg, yield: 58.2%) as a pale white solid product. The product was verified by LCMS and H-NMR.

$^{1}H$ NMR (400 MHz, $CDCl_3$) δ: 10.22 (br s, 1H), 8.18 (br s, 1H), 7.41 (d, 2H, J=8.8Hz), 6.91 (d, 1H, J=8.8Hz), 5.39 (s, 2H), 4.06 (q, 2H, J=7.2Hz), 3.96 (s, 3H), 3.33 (d, 2H, J=5.2Hz), 2.52-2.64 (m, 6H), 1.89-1.91 (m, 4H), 1.68 (s, 4H), 1.44 (t, 3H, J=7.2Hz).

Step 6

3-((4-ethoxybenzyl)oxy)-5-(3-(4-(pyrrolidin-1-yl)butyl)ureido)isothiazole-4-carboxamide

In a microwave tube, methyl 3-((4-ethoxybenzyl)oxy)-5-(3-(4-(pyrrolidin-1-yl)butyl)ureido)isothiazol-4-carboxylate I172 (100 mg, 0.21 mmol) was dissolved in $NH_3$/MeOH (15 mL, 9 mol/L), and the mixture was sealed to react at 60 °C for 16 hours. The reaction mixture was concentrated under reduced pressure to obtain a crude product, and the crude product was purified by the preparative thin-layer chromatography (DCM/MeOH/$NH_3.H_2O$(25%) = 100/10/1) to obtain a white solid product 3-((4-ethoxybenzyl)oxy)-5-(3-(4-(pyrrolidin-1-yl)butyl)ureido)isothiazol-4-formamide T036 (30 mg, yield: 31.0%).

The product was verified by LCMS, H-NMR and C-NMR.

$^{1}H$ NMR (400 MHz, $CDCl_3$) δ: 10.91 (s, 1H), 7.82 (s, 1H), 7.38 (d, 2H, J=8.8Hz), 7.18 (s, 1H), 6.92 (d, 2H, J=8.4Hz), 5.60 (s, 1H), 5.39 (s, 2H), 4.06 (q, 2H, J=7.2Hz), 3.33 (s, 2H), 2.51-2.58 (m, 6H), 1.86 (s, 4H), 1.67-1.68 (m, 4H), 1.44 (t, 3H, J=7.2Hz).

$^{13}C$ NMR (100 MHz, $CDCl_3$) δ: 169.48, 165.91, 162.09, 159.27, 153.99, 130.23, 127.84, 114.63, 70.33, 63.52, 55.56, 53.90, 40.58, 28.15, 26.72, 23.41, 14.82.

Example 23: Synthesis of Compound T042

Synthesis Route:

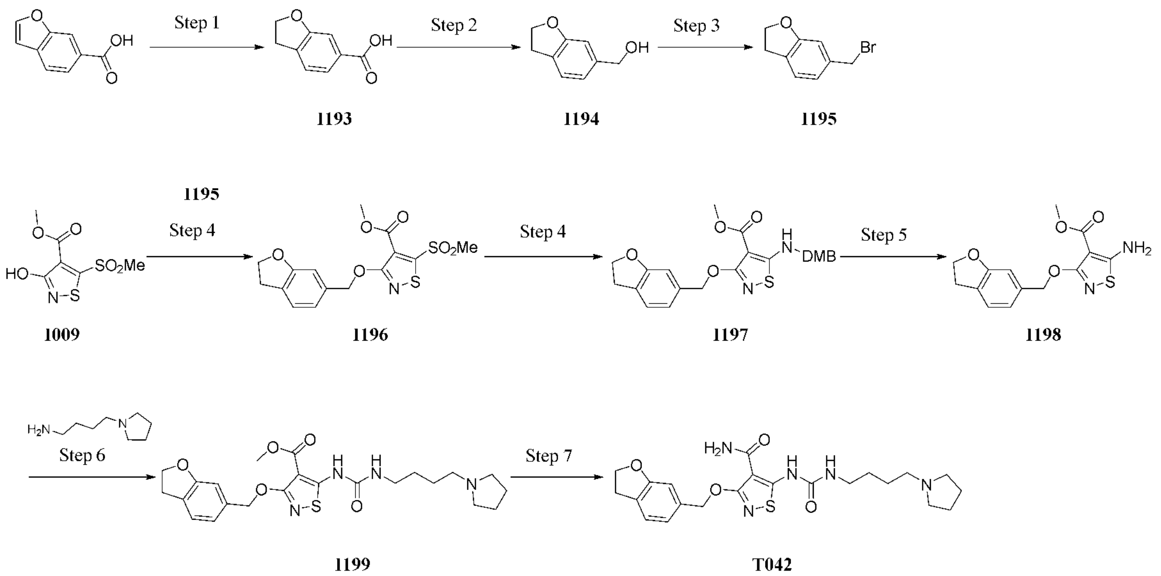


1193 1194 1195

1009 1196 1197 1198

1199 T042

Step 1

2,3-dihydrobenzofuran-6-carboxylic acid

Benzofuran-6-carboxylic acid(4 g, 24.67 mmol) and Pd/C (899 mg) were dissolved in methanol (50 mL), and in the atmosphere of hydrogen (20 psi), the mixture was stirred at room temperature to react for 16 hours. After complete reaction, the mixture was filtered with diatomite, and the filtrate was concentrated under reduced pressure to obtain a crude product 2,3-dihydrobenzofuran-6-carboxylic acid I193 (3.7 g, yield: 91.4%) as a white solid product.

The product was verified by LCMS and H-NMR.

$^{1}H$ NMR (400 MHz, DMSO-$d_6$) δ 7.46 (dd, 1H, J = 7.6, 1.6 Hz), 7.32 (d, 1H, J = 7.6 Hz), 7.23 (d, 1H, J = 1.2 Hz), 4.57 (t, 2H, J = 8.8 Hz), 3.23 (t, 2H, J = 8.8 Hz).

Step 2

(2,3-dihydrobenzofuran-6-yl)methanol

Under the protection of $N_2$, 2,3-dihydrobenzofuran-6-carboxylic acid I193 (3.7 g, 22.54 mmol) was dissolved in anhydrous THF (50 mL), followed by the addition of in the ice water bath $BH_3.Me_2S$ (10 M, 5.63 mL, 56.3 mmol), and the mixture was warmed to 60°C and stirred to react for 16 hours. The reaction mixture was poured into methanol (100 mL), and concentrated under reduced pressure to obtain a crude product (2,3-dihydrobenzofuran-6-yl)methanol I194 (3.3 g, yield: 97.49%) as a colorless liquid product.

The product was verified by LCMS and then directly used in the next step of reaction.

Step 3

6-(bromomethyl)-2,3-dihydrobenzofuran

(2,3-Dihydrobenzofuran-6-yl)methanol I194 (3.3 g, 21.97 mmol) was dissolved in DCM (50 mL) and then cooled to 0°C, followed by the addition of $PBr_3$ (8.92 g, 32.96 mmol), and the reaction system was stirred at room temperature to react for 1 hour. After complete reaction, the mixture was added to a cold saturated sodium bicarbonate aqueous solution and extracted with dichloromethane, and the organic phases were combined, washed with saturated brine, dried with anhydrous sodium sulfate, and concentrated under reduced pressure to obtain a crude product 6-(bromomethyl)-2,3-dihydrobenzofuran I195 (4 g, yield: 85.3%) as a white product.

The product was verified by LCMS.

Step 4

Methyl 3-((2,3-dihydrobenzofuran-6-yl)methoxy)-5-(methylsulfonyl)isothiazole-4-carboxylate

Methyl 3-hydroxyl-5-(methylsulfonyl)isothiazol-4-carboxylate I009 (1.0 g, 4.21 mmol) and potassium carbonate (1.17 g, 8.43 mmol) were dissolved in DMF (10 mL), followed by the addition of 6-(bromomethyl)-2,3-dihydrobenzofuran I195 (0.99 g, 4.64 mmol), and the reaction system was stirred at room temperature to react for 4 hours. Water (50 mL) was added to the reaction mixture, which was then extracted with ethyl acetate (20 mL x 3), the organic phases were sequentially washed with water (20 mL) and saturated brine (20 mL), dried with anhydrous sodium sulfate, and concentrated under reduced pressure to obtain a crude product, and the crude product was purified by the flash column chromatography (PE/EA = 10/1) to obtain a white solid product methyl 3-((2,3-dihydrobenzofuran-6-yl)methoxy)-5-(methylsulfonyl)isothiazol-4-carboxylate I196 (1.2 g, yield: 77.1%).

The product was verified by LCMS and H-NMR.

$^1H$ NMR (400 MHz, DMSO-$d_6$) $\delta$: 7.24 (d, 1H, J=7.6Hz), 6.93 (d, 1H, J=7.6Hz), 6.86 (s, 1H), 5.41 (s, 2H), 4.53 (t, 2H, J=8.8Hz), 3.88 (s, 3H), 3.61 (s, 3H), 3.17 (t, 2H, J=8.8Hz).

Step 5

Methyl 3-((2,3-dihydrobenzofuran-6-yl)methoxy)-5-((2,4-dimethoxybenzyl)amino) isothiazole-4-carboxylate

Methyl 3-((2,3-dihydrobenzofuran-6-yl)methoxy)-5-(methylsulfonyl)isothiazol-4-carboxylate I196 (1.4 g, 3.79 mmol) was dissolved in THF(25 mL), followed by the addition of 2,4-dimethoxybenzylamine (3.17 g, 18.95 mmol), and the reaction system was stirred at 60 °C to react for 4 hours. After the reaction was finished, the reaction mixture was added to ice water (30 mL), regulated to the pH of 5, and extracted with ethyl acetate (10 mL x 3), and the organic phases were combined, sequentially washed with water (20 mL) and saturated brine (20 mL), dried with anhydrous sodium sulfate, and concentrated under reduced pressure to obtain a white solid product methyl 3-((2,3-dihydrobenzofuran-6-yl)methoxy)-5-((2,4-dimethoxybenzyl)amino)isothiazol-4-carboxylate I197 (1.3 g, yield: 75.1%).

The product was verified by LCMS and H-NMR.

1H NMR (400MHz, $CDCl_3$): $\delta$ (ppm) 8.15 (t, 1H, J=5.6Hz), 7.18 (d, 2H, J=8.4Hz), 6.94 (t, 2H, J=3.2Hz), 6.45-6.49 (m, 2H), 5.38 (s, 2H), 4.59 (t, 2H, J=8.8Hz), 4.29 (d, 2H, J=6.0Hz), 3.86 (s, 3H), 3.84 (s, 3H), 3.82 (s, 3H), 3.22 (t, 2H, J=8.8Hz).

Step 6

Methyl 5-amino-3-((2,3-dihydrobenzofuran-6-yl)methoxy)isothiazole-4-carboxylate

Methyl 3-((2,3-dihydrobenzofuran-6-yl)methoxy)-5-((2,4-dimethoxybenzyl)amino)isothiazol-4-carboxylate I197 (1.64 g, 3.59 mmol) was dissolved in DCM/$H_2O$ (15/3mL), followed by the portionwise addition of DDQ (1.22 g, 5.39 mmol) at 0°C, and the reaction system was stirred at room temperature to react for 20 minutes. After complete reaction, water (30 mL) was added to the reaction mixture, which was then extracted with DCM (30 mL x2), the organic phases were combined, washed with saturated brine (20 mL), dried with anhydrous sodium sulfate, and concentrated under reduced pressure to obtain a crude product, and the crude product was purified by chromatographic columns (PE/EA = 10/1-3/1) to obtain a yellow solid product methyl 5-amino-3-((2,3-dihydrobenzofuran-6-yl)methoxy)isothiazol-4-carboxylate I198 (0.4 g, yield: 36.4%).
The product was verified by LCMS.

Step 7

Methyl 3-((2,3-dihydrobenzofuran-6-yl)methoxy)-5-(3-(4-(pyrrolidin-1-yl)butyl)ureido)isothiazole-4-carboxylate

4-(Pyrrolidin-1-yl)butyl-1-amine (390 mg, 2.74 mmol) was dissolved in anhydrous THF (10 mL) and then cooled to 0° under the protection of $N_2$, followed by the addition of CDI (445 mg, 2.74 mmol), the mixture was stirred at room temperature to react for 1 hour, followed by the addition of DMSO (10 mL), the pressure was then reduced to remove most of the THF, followed by the addition of methyl 5-amino-3-((2,3-dihydrobenzofuran-6-yl)methoxy)isothiazol-4-carboxylate I198 (0.6 g, 1.96 mmol) and potassium carbonate (541 mg, 3.92 mmol), and the reaction system was stirred at room temperature to react for 16 hours. After complete reaction, water (40 mL) was added to the reaction mixture, which was then extracted with ethyl acetate (20 mL x 3), the organic phases were sequentially washed with water (30 mL) and saturated brine (30 mL), dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((2,3-dihydrobenzofuran-6-yl)methoxy)-5-(3-(4-(pyrrolidin-1-yl)butyl) ureido)isothiazol-4-carboxylate I199 (0.6 g, yield: 64.6%).
The product was verified by LCMS.

Step 8

3-((2,3-dihydrobenzofuran-6-yl)methoxy)-5-(3-(4-(pyrrolidin-1-yl)butyl)ureido)isothiazole-4-carboxamide

In a microwave tube, methyl 3-((2,3-dihydrobenzofuran-6-yl)methoxy)-5-(3-(4-(pyrrolidin-1-yl)butyl)ureido)isothiazol-4-carboxylate I199 (400 mg, 0.84 mmol) was dissolved in $NH_3$/MeOH (20 mL, 9 mol/L), and the mixture was sealed and stirred at 60 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((2,3-dihydrobenzofuran-6-yl)methoxy)-5-(3-(4-(pyrrolidin-1-yl)butyl)ureido) isothiazol-4-formamide T042 (15 mg, yield: 3.87%).
The product was verified by LCMS, H-NMR and C-NMR.
$^1$H NMR (400 MHz, $CDCl_3$) $\delta$: 11.67 (br s, 1H), 11.10 (s, 1H), 7.37 (s, 1H), 7.21 (d, 1H, J=6.4Hz), 6.92 (d, 1H, J=7.6Hz), 6.87 (s, 1H), 6.14 (br s, 1H), 5.38 (s, 2H), 4.61 (t, 2H, J=8.8Hz), 3.79 (s, 2H), 3.37-3.38 (m, 2H), 3.23 (t, 2H, J=8.8Hz), 3.12-3.14 (m, 2H), 2.86 (s, 2H), 2.08-2.18 (m, 4H), 1.98-2.01 (m, 2H), 1.70-1.72 (m, 2H).
$^{13}$C NMR (100 MHz, DMSO-$d_6$) $\delta$: 180.13, 168.46, 164.94, 162.01, 160.36, 154.50, 136.71, 127.94, 125.39, 120.86, 109.30, 97.92, 71.53, 69.98, 55.61, 54.01, 29.33, 27.64, 25.98, 23.52.

Example 24: Synthesis of Compound T044

Synthesis Route:

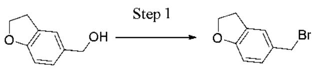


I203

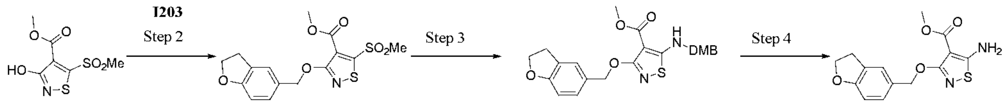


I009 I204 I205 I206

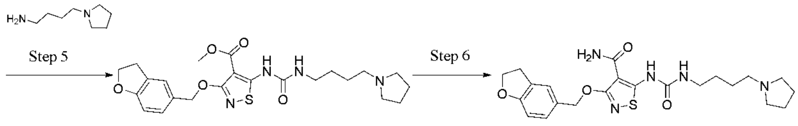


I207 T044

Step 1

5-(bromomethyl)-2,3-dihydrobenzofuran

5-(Hydroxymethyl)-2,3-dihydrobenzofuran (1 g, 6.66 mmol) was dissolved in DCM (10 mL) and then cooled to 0°C, followed by the addition of $PBr_3$ (2.7 g, 9.99 mmol), and the reaction system was stirred at room temperature to react for 1 hour. After complete reaction, the mixture was added to a cold saturated sodium bicarbonate aqueous solution (20 mL x 2) for liquid separation, and the organic phases were then washed with saturated brine, dried with anhydrous sodium sulfate, and concentrated under reduced pressure to obtain a crude product 5-(bromomethyl)-2,3-dihydrobenzofuran I203 (1.2 g, yield: 84.6%) as a white solid product.
The product was verified by LCMS.

Step 2

Methyl 3-((2,3-dihydrobenzofuran-5-yl)methoxy)-5-(methylsulfonyl)isothiazole-4-carboxylate

Methyl 3-hydroxyl-5-(methylsulfonyl)isothiazol-4-carboxylate I009 (0.5 g, 2.11 mmol) and potassium carbonate (0.58 g, 4.21 mmol) were dissolved in DMF (5 mL), followed by the addition of 5-(bromomethyl)-2,3-dihydrobenzofuran I203 (494 mg, 2.32 mmol), and the reaction system was stirred at room temperature to react for 16 hours. Water (20 mL) was added to the reaction mixture, which was then subjected to liquid separation and extraction with ethyl acetate (20 mL x 3), the organic phases were sequentially washed with water (20 mL) and saturated brine (20 mL), dried with anhydrous sodium sulfate, concentrated under reduced pressure to obtain a crude product, and the crude product was purified by the flash column chromatography (PE/EA = 10/1) to obtain a white solid product methyl 3-((2,3-dihydrobenzofuran-5-yl) methoxy)-5-(methylsulfonyl)isothiazol-4-carboxylate I204 (0.5 g, yield: 64.2%).
The product was verified by LCMS and H-NMR.
$^1$H NMR (400MHz, $CDCl_3$): $\delta$ (ppm) 7.32 (s,1H), 7.23 (d,1H, J=7.6Hz), 6.79 (d,1H, J=8.0Hz), 5.41 (s, 2H), 4.58-4.63 (m, 2H), 3.95 (s, 3H), 3.47 (s, 3H), 3.22-3.26 (m,2H).

Step 3

Methyl 3-((2,3-dihydrobenzofuran-5-yl)methoxy)-5-((2,4-dimethoxybenzyl)amino)isothiazole-4-carboxylate

Methyl 3-((2,3-dihydrobenzofuran-5-yl)methoxy)-5-(methylsulfonyl)isothiazol-4-carboxylate I204 (0.2 g, 0.54 mmol) was dissolved in THF(5 mL), followed by the addition of 2,4-dimethoxybenzylamine (453 mg, 2.71 mmol), and the reaction system was stirred at 60 °C to react for 4 hours. After the reaction was finished, the reaction mixture was added to ice water (10 mL), regulated to the pH of 5, and extracted with ethyl acetate (10 mL x 3), and the organic phases were combined, sequentially washed with water and saturated brine, dried with anhydrous sodium sulfate, and concentrated under reduced pressure to obtain a white solid product methyl 3-((2,3-dihydrobenzofuran-5-yl)methoxy)-5-((2,4-dimethoxybenzyl)amino)isothiazol-4-carboxylate
I205 (0.19 g, yield: 76.9%).
The product was verified by LCMS.

Step 4

Methyl 5-amino-3-((2,3-dihydrobenzofuran-5-yl)methoxy)isothiazole-4-carboxylate

Methyl 3-((2,3-dihydrobenzofuran-5-yl)methoxy)-5-((2,4-dimethoxybenzyl)amino)isothiazol-4-carboxylate I205 (683 mg, 1.50 mmol) was dissolved in DCM/$H_2O$ (5/1 mL), followed by the portionwise addition of DDQ (509 mg, 2.24 mmol) at 0°C, and the reaction system was stirred at room temperature to react for half an hour. After complete reaction, the mixture was filtered, the filtrate was added to water (10 mL) and extracted with DCM (20 mL x2), the organic phases were combined, washed with saturated brine, dried with anhydrous sodium sulfate, and concentrated under reduced pressure to obtain a crude product, and the crude product was purified by chromatographic columns (PE/EA = 10/1~3/1) to obtain a yellow solid product methyl 5-amino-3-((2,3-dihydrobenzofuran-5-yl)methoxy)isothiazol-4-carboxylate I206 (0.1 g, yield: 21.8%).

The product was verified by LCMS.

Step 5

Methyl 3-((2,3-dihydrobenzofuran-5-yl)methoxy)-5-(3-(4-(pyrrolidin-1-yl)butyl)ureido)isothiazole-4-carboxylate

4-(Pyrrolidin-1-yl)butyl-1-amine (65. mg, 0.46 mmol) was dissolved in anhydrous THF (2 mL) and then cooled to 0°C under the protection of $N_2$, followed by the addition of CDI (74 mg, 0.46 mmol), the mixture was stirred to react for 30 minutes and then stirred at room temperature to react for 1 hour, followed by the addition of DMSO (2 mL), the pressure was then reduced to remove most of the THF, followed by the addition of methyl 5-amino-3-((2,3-dihydrobenzofuran-5-yl)methoxy)isothiazol-4-carboxylate I206 (0.1 g, 0.33 mmol) and potassium carbonate (90 mg, 0.65 mmol), and the reaction system was stirred at room temperature to react for 16 hours. After complete reaction, water (20 mL) was added to the reaction mixture, which was then extracted with ethyl acetate (20 mL x 3), and the organic phases were sequentially washed with water and saturated brine, dried with anhydrous sodium sulfate, and concentrated under reduced pressure to obtain a crude product methyl 3-((2,3-dihydrobenzofuran-5-yl)methoxy)-5-(3-(4-(pyrrolidin-1-yl)butyl)ureido)isothiazol-4-carboxylate I207 (0.1 g, yield: 64.6%) as a yellow oily liquid product. The product was verified by LCMS.

Step 6

3-((2,3-dihydrobenzofuran-5-yl)methoxy)-5-(3-(4-(pyrrolidin-1-yl)butyl)ureido)isothiazole-4-carboxamide

In a microwave tube, methyl 3-((2,3-dihydrobenzofuran-5-yl)methoxy)-5-(3-(4-(pyrrolidin-1-yl)butyl)ureido)isothiazol-4-carboxylate I207 (0.25 g, 0.53 mmol) was dissolved in $NH_3$/MeOH (6 mL, 9 mol/L), and the mixture was stirred at 65 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((2,3-dihydrobenzofuran-5-yl)methoxy)-5-(3-(4-(pyrrolidin-1-yl)butyl)ureido)isothiazol-4-formamide T044 (18 mg, yield: 7.44%).

The product was verified by LCMS, H-NMR and C-NMR.

$^1$H NMR (400MHz, $CDCl_3$): δ (ppm) 11.07 (s,1 H), 7.44-7.47 (m, 1H), 7.28-7.30 (m, 1H), 7.16-7.22 (m, 2H), 6.80 (d,1H, J=8.4Hz), 3.59 (s, 1H), 3.37 (s, 2H), 4.59-4.63 (m, 2H), 3.26-3.37 (m, 2H), 3.06-3.26 (m, 2H), 2.93-2.99 (m, 4H), 2.86-2.92 (m, 2H), 2.03-2.35 (m, 4H), 1.86-2.00 (m, 2H), 1.65-1.86 (m, 2H).

$^{13}$C NMR (100 MHz, $CDCl_3$) δ: 169.34, 165.76, 162.15, 160.53, 154.25, 129.15, 127.89, 127.60, 125.84, 109.34, 97.49, 71.50, 71.42, 70.63, 55.46, 53.85, 39.80, 39.74, 29.58, 29.49, 27.40, 25.02, 23.37

Example 25: Synthesis of Compound T045

Synthesis Route:

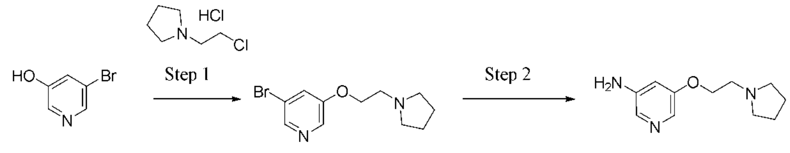


I208 I209

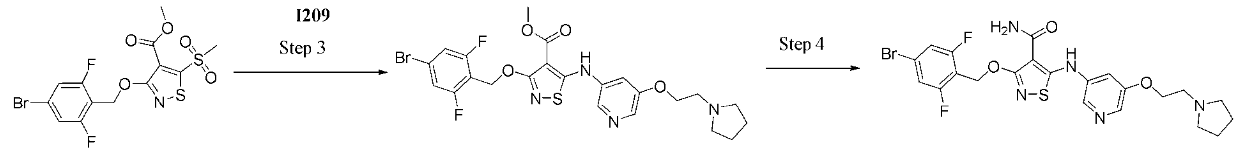


I178 I210 T045

Step 1

3-bromo-5-(2-(pyrrolidin-1-yl)ethoxy)pyridine

5-Bromopyridin-3-ol (1.5 g, 8.62 mmol) was dissolved in DMF (15.0 mL), followed by the addition of 1-(2-chloroethyl)pyrrolidine · hydrochloride (1.76 g, 10.35 mmol) and sodium hydroxide (1.38 g, 34.48 mmol), and the reaction system was warmed to 70 °C and stirred to react for 2 hours. The reaction mixture was poured into water (60 mL), and extracted with ethyl acetate (20 mL x 3), the organic phases were combined, washed with saturated brine, dried with anhydrous sodium sulfate, and concentrated under reduced pressure to obtain a crude product, and the crude product was purified by the flash column chromatography to obtain a yellow oily liquid product 3-bromo-5-(2-(pyrrolidin-1-yl)ethoxy) pyridine I208 (1.6 g, yield: 68.5%).

The product was verified by LCMS.

Step 2

5-(2-(pyrrolidin-1-yl)ethoxy)pyridin-3-amine

3-Bromo-5-(2-(pyrrolidin-1-yl)ethoxy)pyridine I208 (1 g, 3.69 mmol) was dissolved in NMP (10 mL), followed by the addition of CuO (594 mg, 7.38 mmol) and $NH_3 \cdot H_2O$ (10 mL) at room temperature. The mixture was stirred at 120°C to react for 16 hours. After complete reaction, undissolved substances were filtered and removed with diatomite, the filtrate was extracted with dichloromethane, the organic phases were combined, dried with anhydrous sodium sulfate, and concentrated under reduced pressure to obtain a crude product, and the crude product was purified by the flash column chromatography (DCM/MeOH = 120/1-40/1, 0.1% $NH_3.H_2O$) to obtain a yellow solid product 5-(2-(pyrrolidin-1-yl)ethoxy) pyridin-3-amine I209 (0.6 g, yield: 78.5%).

The product was verified by LCMS.

Step 3

Methyl 3-((4-bromo-2,6-difluorobenzyl)oxy)-5-((5-(2-(pyrrolidin-1-yl)ethoxy)pyridin-3-yl)amino)isothiazole-4-carboxylate

Methyl 3-[(4-bromo-2,6-difluorophenyl)methoxy]-5-methylsulfonyl-1,2-thiazol-4-carboxylate I178 (0.9 g, 2.04 mmol) was dissolved in THF (30 mL), followed by the addition of LiHMDS (1 M, 4.50 mL) and 5-(2-(pyrrolidin-1-yl)ethoxy) pyridin-3-amine I209 (464 mg, 2.24 mmol) at room temperature, and the mixture was stirred at 30 °C to react for 5 hours. After complete reaction, the mixture was poured into water (50 mL), and extracted with ethyl acetate, the organic phases were combined, washed with saturated brine, dried with anhydrous sodium sulfate, and concentrated under reduced pressure to obtain a crude product, and the crude product was purified by the flash column chromatography (DCM/MeOH = 150/1-120/1) to obtain a yellow oily liquid product methyl 3-((4-bromo-2,6-difluorophenyl)oxy)-5-((5-(2-(pyrrolidin-1-yl) ethoxy)pyridin-3-yl)amino)isothiazol-4-carboxylate I210 (0.27 g, yield: 23.3%).

The product was verified by LCMS.

Step 4

3-((4-bromo-2,6-difluorobenzyl)oxy)-5-((5-(2-(pyrrolidin-1-yl)ethoxy)pyridin-3-yl)amino)isothiazole-4-carboxamide

In a microwave tube, methyl 3-((4-bromo-2,6-difluorophenyl)oxy)-5-((5-(2-(pyrrolidin-1-yl)ethoxy)pyridin-3-yl)amino)isothiazol-4-carboxylate I210 (0.36 g, 0.63 mmol) was dissolved in $NH_3$/MeOH (15 mL, 9 mol/L), and the mixture was sealed and stirred at 65 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((4-bromo-2,6-difluorophenyl)oxy)-5-((5-(2-(pyrrolidin-1-yl)ethoxy)pyridin-3-yl)amino)isothiazol-4-formamide T045 (30 mg, yield: 8.56%). The product was verified by LCMS, H-NMR and C-NMR.

$^1$H NMR (400 MHz, DMSO-$d_6$) δ: 11.17 (s, 1H), 8.08-8.13 (m, 2H), 7.69 (s, 1H), 7.57-7.59 (d, 2H, J=8.0 Hz), 7.28-7.29 (m, 1H), 6.85 (s, 1H), 5.51 (s, 2 H). 4.19 (t, 2H, J=5.6Hz), 2.85 (s, 2H), 2.57 (m, 4H), 1.70 (s, 4H).

$^{13}$C NMR (100 MHz, $CDCl_3$+$CD_3OD$) δ: 172.32, 166.01, 162.87, 162.80, 160.35, 160.26, 155.27, 137.15, 132.64, 132.04, 123.56, 115.87, 115.65, 115.58, 111.02, 110.91, 110.84, 96.47, 66.41, 58.01, 54.62, 54.35, 29.62, 23.24.

Example 26: Synthesis of Compound T046

Synthesis Route:

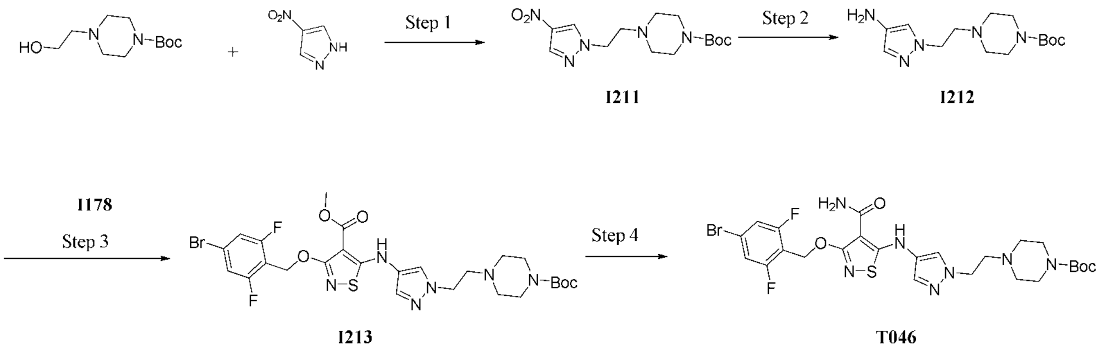


I211 I212

I213 T046

Step 1

Tert-butyl 4-(2-(4-nitro-1H-pyrazol-1-yl) ethyl) piperazine-1-carboxylate

Tert-butyl 4-(2-hydroxyethyl)piperazin-1-carboxylate (3 g, 13.0 mmol) was dissolved in THF (30 mL), followed by the addition of 4-nitro-1H-pyrazole (1.6 g, 14.3 mmol), DIAD (4 g, 19.5 mmol) and $PPh_3$ (5.1 g, 19.5 mmol) at room temperature, and the mixture was then stirred to react for 2 hours. After complete reaction, the mixture was added to water (100 mL) and extracted with ethyl acetate (50 mL x 2), and the organic phases were combined, washed with saturated brine, dried with anhydrous sodium sulfate, and concentrated under reduced pressure to obtain a crude product tert-butyl 4-(2-(4-nitro-1H-pyrazol-1-yl)ethyl)piperazin-1-carboxylate I211 (5 g, crude product) as a yellow solid product, which was then directly used in the next step of reaction.

The product was veriifed by LCMS.

Step 2

Tert-butyl 4-(2-(4-amino-1H-pyrazol-1-yl) ethyl) piperazine-1-carboxylate

Tert-butyl 4-(2-(4-nitro-1H-pyrazol-1-yl)ethyl)piperazin-1-carboxylate I211 (5 g, crude) and Pd/C (0.5 g) was dissolved in anhydrous methanol MeOH (100 mL), and in the atmosphere of $H_2$ at 50 psi at room temperature, the mixture was stirred to react for 2 hours. After complete reaction, the mixture was and filtered, and the filtrate was concentrated under reduced pressure to obtain a crude product tert-butyl 4-(2-(4-amino-1H-pyrazol-1-yl)ethyl)piperazin-1-carboxylate I212 (2.5 g, yield: 55%) as a red solid product.

The product was verified by LCMS and H-NMR.

$^1$H NMR (400 MHz, $CDCl_3$) δ: 7.17 (s, 1H), 7.10 (s, 1H), 4.14 (t, 2H, J=6.4Hz), 3.43 (t, 4H, J=4.8Hz), 2.79 (t, 1H, J=6.4Hz), 2.42 (s, 4H), 1.47 (s, 9H).

Step 3

Methyl 3-((4-bromo-2,6-difluorobenzyl) oxy)-5-((1-(2-(4-(tert-butoxycarbonyl) piperazin-1-yl) ethyl)-1H-pyrazol-4-yl) amino) isothiazole-4-carboxylate

Methyl 3-[(4-bromo-2,6-difluorophenyl)methoxy]-5-methylsulfonyl-1,2-thiazol-4-carboxylate I178 (300 mg, 0.68 mmol) and tert-butyl 4-(2-(4-amino-1H-pyrazol-1-yl)ethyl)piperazin-1-carboxylate I212 (240 mg, 0.30 mmol) were dissolved in anhydrous THF (30 mL), followed by the addition of LiHMDS (1 M, 1.4 mL, 1.36 mmol) at room temperature, and the mixture was stirred to react for about 1 hour. After complete reaction, water (30 mL) was added to the mixture, which was then extracted with ethyl acetate (30 mL x 3), the organic phases were combined, washed with saturated brine, dried with anhydrous sodium sulfate, and concentrated under reduced pressure to obtain a crude product, and the crude product was purified by the high-performance preparative chromatography to obtain a white solid product methyl 3-((4-bromo-2,6-difluorophenyl)oxy)-5-((1-(2-(4-(tert-butoxycarbonyl)piperazin-1-yl)ethyl)-1H-pyrazol-4-yl)amino)isothiazol-4-carboxylate I213 (10 mg, yield: 2.2%).

The product was verified by LCMS and H-NMR.

$^1$H NMR (400 MHz, $CDCl_3$) δ: 9.32 (s, 1H), 7.60 (s, 1H), 7.50 (s, 1H), 7.16 (d, 2H, J=6.8Hz), 5.45 (s, 2H), 4.25 (t, 2H, J=6.4Hz), 3.81 (s, 3H), 3.44-3.45 (m, 4H), 2.83 (t, 2H, J=6.0Hz), 2.45 (s, 4H), 1.48 (s, 9H).

Step 4

Tert-butyl 4-(2-(4-((3-((4-bromo-2,6-difluorobenzyl)oxy)-4-carbamoylisothiazol-5-yl)amino) - 1H-pyrazol-1-yl)ethyl)piperazine-1-carboxylate

In a microwave tube, methyl 3-((4-bromo-2,6-difluorophenyl)oxy)-5-((1-(2-(4-(tert-butoxycarbonyl)piperazin-1-yl)ethyl)-1H-pyrazol-4-yl)amino)isothiazol-4-carboxylate I213 (160 mg, 0.24 mmol) was dissolved in $NH_3$/MeOH (5 mL, 9 mol/L), and the mixture was sealed and stirred at 70 °C to react for 48 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product tert-butyl 4-(2-(4-((3-((4-bromo-2,6-difluorophenyl)oxy)-4-carbamoylisothiazol-5-yl)amino)-1H-pyrazol-1-yl)ethyl)piperazin-1-carboxylate T046 (7 mg, yield: 4.4%).

The product was verified by LCMS and H-NMR.

$^1$H NMR (400 MHz, $CDCl_3$) δ: 10.09 (s, 1H), 7.68 (s, 1H), 7.53 (s, 1H), 7.19 (d, 2H, J=6.8Hz), 6.83 (s, 1H), 5.54 (s, 2H), 5.34 (s, 1H), 4.53 (s, 2H), 3.62 (s, 4H), 2.54-2.66 (m, 4H), 1.63-1.68 (m, 2H), 1.47 (s, 9H).

Example 27: Synthesis of Compound T049

Synthesis Route:

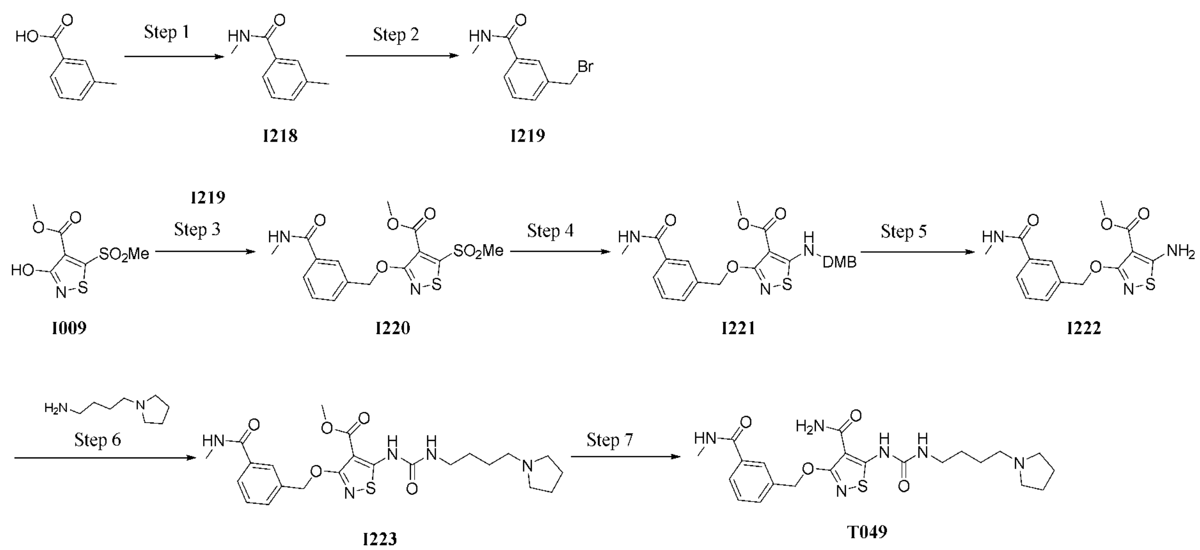

Step 1

N,3-dimethylbenzamide

N,3-dimethylbenzoic acid (5 g, 36.72 mmol) was dissolved in anhydrous DMF (50 mL), followed by the addition of methylamine hydrochloric acid (2.73 g, 40.40 mmol), DIEA (23.73 g, 183.62 mmol), EDCI (8.45 g, 44.07 mmol) and HOBt (5.95 g, 44.07 mmol) at room temperature, and the mixture was stirred to react for 3 hours. After complete reaction, the mixture was poured into water (200 mL), and extracted with ethyl acetate (100 mL x 3), the organic phases were combined, sequentially washed with water and saturated brine, dried with anhydrous sodium sulfate, and concentrated under reduced pressure to obtain a crude product, and the crude product was purified by the flash column chromatography (PE/EA = 3/1-1/1) to obtain a yellow oily liquid product N, 3-dimethylbenzamide I218 (4.8 g, yield: 87.6%).

The product was verified by LCMS and H-NMR.

$^1$H NMR (400 MHz, $CDCl_3$) $\delta$: 7.61 (s, 1H), 7.49-7.57 (m, 1H), 7.31-7.34 (m, 2H), 6.23 (s, 1H), 3.03 (d, 3H, J=4.4Hz), 2.41 (s, 3H).

Step 2

3-(bromomethyl)-N-methylbenzamide

N,3-dimethylbenzamide I218 (4 g, 26.81 mmol) was dissolved in $CCl_4$ (80 mL), followed by the addition of AIBN (440 mg, 2.68 mmol) and NBS (5.73 g, 32.17 mmol) at room temperature, and the mixture was heated to 80°C in an oil bath and then stirred under reflux to react for 16 hours. After complete reaction, the mixture was washed with water and saturated brine, dried with anhydrous sodium sulfate, and concentrated under reduced pressure to obtain a crude product, and the crude product was purified by the flash column chromatography (PE/EA = 10/1-5/1-1/1) to obtain a tan oily liquid product 3-(bromomethyl)-N-methylbenzamideI219 (4.6 g, crude product).

Step 3

Methyl 3-((3-(methylcarbamoyl)benzyl)oxy)-5-(methylsulfonyl)isothiazole-4-carboxylate

Methyl 3-hydroxyl-5-(methylsulfonyl)isothiazol-4-carboxylate I009 (2 g, 8.43 mmol) and potassium carbonate (3.5 g, 25.29 mmol) were dissolved in DMF (40 mL), followed by the addition of 3-(bromomethyl)-N-methylbenzamide I219 (4.61 g, 10.12 mmol), and the reaction system was stirred at room temperature to react for 1 hour. Water (200 mL) was added to the reaction mixture, which was then subjected to liquid separation and extraction with ethyl acetate (50 mL x 3), the organic phases were sequentially washed with water and saturated brine, dried with anhydrous sodium sulfate, and concentrated under reduced pressure to obtain a crude product, and the crude product was purified by the flash column chromatography (PE/EA = 5/1-3/1-1/1-1/2) to obtain a white solid product methyl 3-((3-(methylcarbamoyl)benzyl) oxy)-5-(methylsulfonyl)isothiazol-4-carboxylate I220 (2.4 g, yield: 74.06%).

The product was verified by LCMS and H-NMR.

$^1$H NMR (400 MHz, $CDCl_3$) $\delta$: 7.93 (s, 1H), 7.72 (d, 1H, J=7.6Hz), 7.60 (d, 1H, J=8.0Hz), 7.47 (t, 1H, J=8.0Hz), 6.23 (s, 1H), 5.54 (s, 2H), 4.00 (s, 3H), 3.50 (s, 3H), 3.05 (d, 3H, J = 5.2Hz).

Step 4

Methyl 5-((2,4-dimethylbenzyl)amino)-3-((3-(methylcarbamoyl)benzyl)oxy)isothiazole-4-carboxylate

Methyl 3-((3-(methylcarbamoyl)benzyl)oxy)-5-(methylsulfonyl)isothiazol-4-carboxylate I220 (2 g, 5.2 mmol) was dissolved in THF(20 mL), followed by the addition of 2,4-dimethoxybenzylamine (8.7 g, 52.03 mmol), and the reaction system was stirred at 65 °C to react for 1 hour. After the reaction was finished, the reaction mixture was added to water (100 mL), and filtered to obtain the filter cake, which was washed with water and dried to obtain a white solid product methyl 5-((2,4-dimethylbenzyl)amino)-3-((3-(methylcarbamoyl)benzyl)oxy)isothiazol-4-carboxylate I221 (2.2 g, yield: 89.7%).

The product was verified by LCMS and H-NMR.

$^1$H NMR (400 MHz, $CDCl_3$) $\delta$: 8.13 (t, 1H, J=5.6Hz), 7.91 (s, 1H), 7.71 (d, 1H, J=7.6Hz), 7.59 (d, 1H, J=8.0Hz), 7.44 (t, 1H, J=7.6Hz), 7.18 (d, 1H, J=8.4Hz), 6.39-6.50 (m, 2H), 6.21 (s, 1H), 5.46 (s, 2H), 4.29 (d, 2H, J=6.0Hz), 3.87 (s, 6H), 3.82 (s, 3H), 3.04 (d, 2H, J=4.8Hz).

Step 5

Methyl 5-amino-3-((3-(methylcarbamoyl)benzyl)oxy)isothiazole-4-carboxylate

Methyl 5-((2,4-dimethylbenzyl)amino)-3-((3-(methylcarbamoyl)benzyl)oxy)isothiazol-4-carboxylate I221 (2 g, 4.24 mmol) was dissolved in DCM/$H_2O$ (150/30mL), followed by the portionwise addition of DDQ (3.85 g, 16.97 mmol) at 0°C, and the reaction system was stirred at room temperature to react for 5 minutes. After complete reaction, neutral alumina was added to the mixture, and the liquid was then concentrated under reduced pressure, and then purified by chromatographic columns (neutral alumina, PE/EA = 100/1-50-1-20/1) to obtain a faint yellow solid product methyl 5-amino-3-((3-(methylcarbamoyl)benzyl)oxy)isothiazol-4-carboxylate I222 (1 g, yield: 73.37%).
The product was verified by LCMS.

Step 6

Methyl 3-((3-(methylcarbamoyl)benzyl)oxy)-5-(3-(4-(pyrrolidin-1-yl)butyl)ureido)isothiazole-4-carboxylate

4-(Pyrrolidin-1-yl)butyl-1-amine (177 mg, 1.24 mmol) was dissolved in anhydrous THF (4 mL) and then cooled to 0°C under the protection of $N_2$, followed by the addition of CDI (179 mg, 1.24 mmol), the mixture was stirred to react for 30 minutes and then stirred at room temperature to react for 1 hour, followed by the addition of DMSO (4 mL), the pressure was then reduced to remove most of the THF, followed by the addition of methyl 5-amino-3-((3-(methylcarbamoyl)benzyl)oxy) isothiazol-4-carboxylate I222 (400 mg, 1.24 mmol) and potassium carbonate (344 mg, 2.49 mmol), and the reaction system was stirred at room temperature to react for 1 hour. After complete reaction, water (20 mL) was added to the reaction mixture, which was then extracted with ethyl acetate (10 mLx3), and the organic phases were sequentially washed with water (30 mL) and saturated brine (30 mL), dried with anhydrous sodium sulfate, and concentrated under reduced pressure to obtain a crude product methyl 3-((3-(methylcarbamoyl)benzyl)oxy)-5-(3-(4-(pyrrolidin-1-yl)butyl) ureido)isothiazol-4-carboxylate I223, which was then directly used in the next step of reaction.
The product was verified by LCMS and H-NMR.
$^1$H NMR (400 MHz, $CDCl_3$) $\delta$: 10.23 (br s, 1H), 8.10 (br s, 1H), 7.94 (s, 1H), 7.70 (d, 1H, J=5.6Hz), 7.59 (d, 1H, J=7.6Hz), 7.44 (t, 1H, J=7.6Hz), 6.30 (s, 1H), 5.47 (s, 2H), 3.90 (s, 3H), 3.31 (s, 2H), 3.04 (d, 3H, J=4.8Hz), 2.59 (s, 4H), 2.52 (t, 2H, J=6.0Hz), 1.86-1.88 (m, 4H), 1.66-1.68 (m, 4H).

Step 7

3-((3-(methylcarbamoyl)benzyl)oxy)-5-(3-(4-(pyrrolidin-1-yl)butyl)ureido)isothiazole-4-carboxamide

In a microwave tube, methyl 3-((3-(methylcarbamoyl)benzyl)oxy)-5-(3-(4-(pyrrolidin-1-yl)butyl)ureido)isothiazol-4-carboxylate I223 (150 mg, 0.31 mmol) was dissolved in $NH_3$/MeOH (15 mL, 9 mol/L), and the mixture was sealed and stirred at 65 °C to react for 24 hours. The reaction mixture was concentrated under reduced pressure to obtain a crude product, and the crude product was purified by the high-performance preparative chromatography to obtain a white solid product 3-((3-(methylcarbamoyl)benzyl)oxy)-5-(3-(4-(pyrrolidin-1-yl)butyl)ureido)isothiazol-4-formamide T049 (25.7 mg, yield: 17.7%).
The product was verified by LCMS, H-NMR and C-NMR.
$^1$H NMR (400 MHz, $CDCl_3$) $\delta$: 10.92 (br s, 1H), 7.88 (s, 2H), 7.76 (d, 1H, J=7.6Hz), 7.59 (d, 1H, J=7.2Hz), 7.48 (t, 1H, J=8.0Hz), 7.12 (s, 2H), 6.22 (s, 1H), 5.52 (s, 2H), 5.48 (s, 1H), 3.33 (s, 3H), 3.05 (d, 3H, J=4.8Hz), 2.59 (s, 4H), 2.48-2.56 (m, 2H), 1.88 (s, 4H), 1.71-1.69 (m, 4H).
$^{13}$C NMR (100 MHz, $CDCl_3$ + $CD_3OD$) $\delta$: 173.17, 172.60, 169.79, 165.76, 158.43, 140.24, 138.82, 135.08, 132.85, 131.04, 130.83, 73.83, 59.91, 57.88, 43.82, 31.52, 30.45, 29.70, 27.07.

Example 28: Synthesis of Compound T050

Synthesis Route:

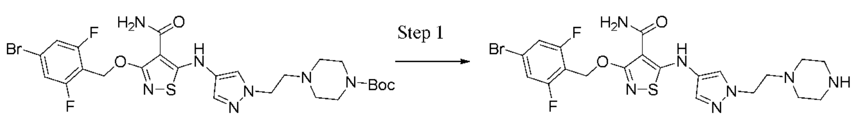


T046 T050

Step 1

3-((4-bromo-2,6-difluorobenzyl)oxy)-5-((1-(2-(piperazin-1-yl)ethyl)-1H-pyrazol-4-yl)amino) isothiazole-4-carboxamide

At room temperature, tert-butyl 4-(2-(4-((3-((4-bromo-2,6-difluorophenyl)oxy)-4-carbamoylisothiazol-5-yl)amino)-1H-pyrazol-1-yl)ethyl)piperazin-1-carboxylate T046 (50 mg, 0.078 mmol) was dissolved in HCl/MeOH (5 mL, 8 mol/L), and then stirred to react for 2 hours. After complete reaction, the mixture was directly concentrated under reduced pressure, water was added to the residues, the resulting mixture was regulated with saturated sodium carbonate to pH = 9 and extracted with ethyl acetate (10 mL x 3), the organic phases were combined, washed with saturated brine, dried with anhydrous sodium sulfate, and concentrated under reduced pressure to obtain a crude product, and the crude product was purified by the preparative thin-layer chromatography (DCM/MeOH = 10/1) to obtain a white solid product 3-((4-bromo-2,6-difluorophenyl)oxy)-5-((1-(2-(piperazin-1-yl)ethyl)-1H-pyrazol-4-yl)amino)isothiazol-4-formamide T050 (18.8 mg, yield: 44.5%).

The product was verified by LCMS, H-NMR and C-NMR.

$^1$H NMR (400 MHz, $CDCl_3$) δ: 10.04 (s, 1H), 7.60 (s, 1H), 7.49 (s, 1H), 7.18-7.25 (m, 2H), 6.82 (s, 1H), 5.54 (s, 2H), 5.32 (s, 1H), 4.24 (t, 2H, J=6.0Hz), 2.92-2.95 (m, 4H), 2.80-2.83 (m, 2H), 2.37-2.50 (m, 2H).

$^{13}$C NMR (100 MHz, $CDCl_3$) δ: 176.72, 166.01, 162.96, 162.91, 162.89, 160.43, 160.35, 133.93, 132.55, 123.55, 123.42, 123.30, 123.05, 122.46, 115.91, 115.88, 115.82, 115.69, 115.64, 115.61, 115.54, 111.36, 111.16, 94.28, 58.18, 57.68, 57.64, 57.61, 54.27, 54.20, 54.17, 50.26, 46.00,45.93.

Example 29: Synthesis of Compound T051

Synthesis Route:

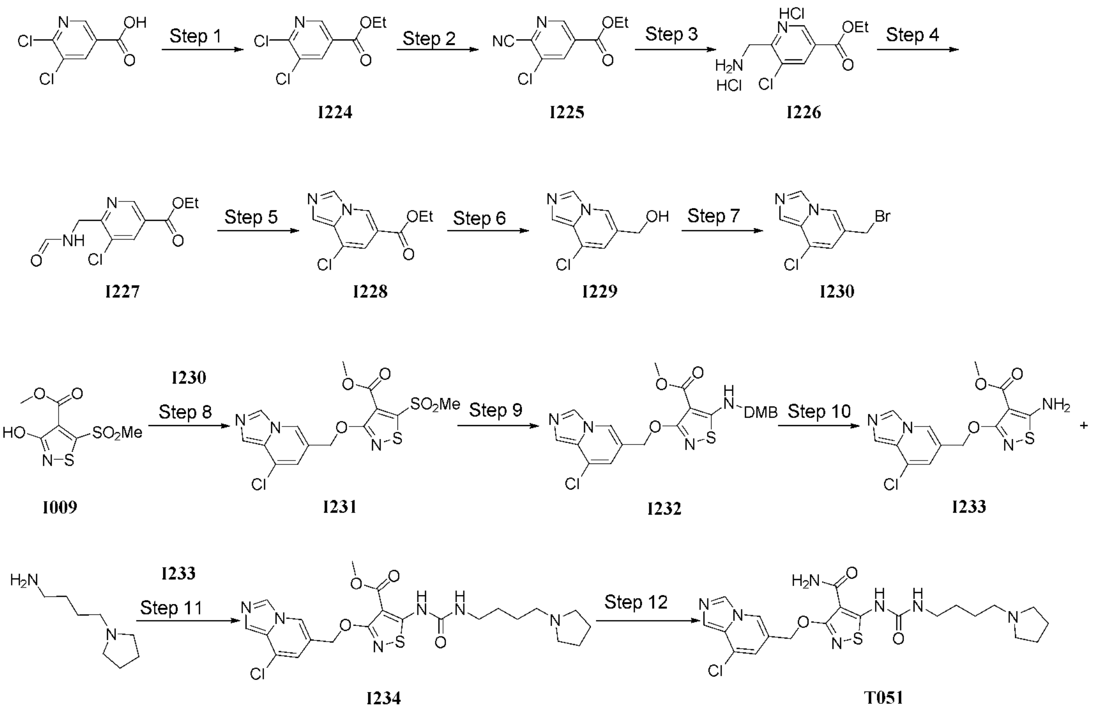


Step 1

Ethyl 5,6-dichloronicotinate

5,6-dichloronicotinic acid (15 g, 78 mmol) and dichlorosulfoxide (27.6 g, 234 mmol) were dissolved in ethanol(150 mL), and the reaction system was stirred at 60 °C to react for 1.0 hour. The reaction mixture was concentrated under reduced pressure, the residues were poured into water (100 mL), and extracted with ethyl acetate (100 mL x 3), and the organic phases were combined, washed with saturated brine (200 mL), dried with anhydrous sodium sulfate, and

concentrated under reduced pressure to obtain a white solid crude product was ethyl 5,6-dichloronicotinate I224 (15 g, yield: 87 %).

The product was verified by LCMS and H-NMR.

$^1$H NMR (400 MHz, $CDCl_3$) δ 8.89 (d, J = 2.0 Hz, 1H), 8.37 (d, J = 1.6 Hz, 1H), 4.44 (dd, J = 21.6, 7.0 Hz, 2H), 1.43 (t, J = 7.2 Hz, 3H).

Step 2

Ethyl 5-chloro-6-cyanonicotinate:

Ethyl 5,6-dichloronicotinate I224 (8 g, 36 mmol), zinc cyanide (3.16 mg, 27 mmol) and tetrakis(triphenylphosphine)palladium (4.15 g, 3.6 mmol) were dissolved in DMF(80 mL), and the reaction system was stirred at 100 °C to react for 16 hours. The reaction mixture was poured into water (300 mL), and extracted with ethyl acetate (100 mL x 3), and the organic phases were combined, washed with saturated brine (200 mL), dried with anhydrous sodium sulfate, and concentrated under reduced pressure to obtain a crude product. The crude product was purified by the silica-gel column chromatography (PE/EA = 50:1~30:1) to obtain a white solid product ethyl 5-chloro-6-cyanonicotinate I225 (3 g, yield: 38.7 %).

The product was verified by LCMS and H-NMR.

$^1$H NMR (400 MHz, $CDCl_3$) δ 9.17 (d, J = 1.6 Hz, 1H), 8.46 (d, J = 1.6 Hz, 1H), 4.49 (q, J = 7.1 Hz, 2H), 1.46 (t, J = 7.2 Hz, 3H).

Step 3

Ethyl 6-(aminomethyl)-5-chloronicotinate:

Ethyl 5-chloro-6-cyanonicotinate I225 (8.8 g, 41.78 mmol) was dissolved in acetic acid (100 mL), followed by the addition of raney nickel (2.45 g, 41.78 mmol), and in the atmosphere of hydrogen, the reaction system was stirred at room temperature to react for 6 hours. The reaction mixture was filtered, and the filtrate was concentrated under reduced pressure to obtain a yellow solid crude product ethyl 6-(aminomethyl)-5-chloronicotinate I226 (4.40 g, yield: 58.4% ).

The product was verified by LCMS and H-NMR.

$^1$H NMR (400 MHz, $D_2O$) δ 8.74 (s, 1H), 8.13 (s, 1H), 4.27 (s, 3H), 4.13 (s, 2H), 1.10 (s, 3H).

Step 4

Ethyl 5-chloro-6-(formamidomethyl)nicotinate :

Ethyl 6-(aminomethyl)-5-chloronicotinate I226 (8.8 g, 41.00 mmol) was dissolved in acetic anhydride (20 mL) and formic acid (100 mL), and the reaction system was stirred at 100 °C to react for 16 hours. The reaction mixture was poured into water (100 mL), and extracted with ethyl acetate (100 mL x 3), and the organic phases were combined, washed with saturated brine (200 mL), dried with anhydrous sodium sulfate, and concentrated under reduced pressure to obtain a white solid crude product ethyl 5-chloro-6-(formamidomethyl)nicotinate I227 (6 g, yield: 60.3%). The product was verified by LCMS.

Step 5

Ethyl 8-chloroimidazo[1,5-a]pyridine-6-carboxylate

5-Chloro-6-(formamidomethyl)ethyl nicotinate I227 (6 g, 24.73 mmol) was dissolved in toluene (50 mL), followed by the addition of phosphorus oxychloride (15.17 g, 98.90 mmol), and the reaction system was stirred at 100°C to react for 16 hours. The reaction mixture was poured into water (100 mL), and extracted with ethyl acetate (100 mL x 3), and the organic phases were combined, washed with saturated brine (200 mL), dried with anhydrous sodium sulfate, and concentrated under reduced pressure to obtain a crude product. The crude product was purified by the silica-gel column chromatography (PE/EA = 1:1) to obtain a yellow solid product ethyl 8-chloroimidazo[1,5-a]pyridin-6-carboxylate I228 (2.5 g, yield: 45.0%).

The product was verified by LCMS and H-NMR.

$^1$H NMR (400 MHz, DMSO-$d_6$) δ 9.15 (s, 1H), 8.69 (s, 1H), 7.54 (s, 1H), 7.22 (d, J = 0.4 Hz, 1H), 4.34 (d, J = 7.2 Hz, 2H), 1.34 (t, J = 7.2 Hz, 3H).

Step 6

(8-chloroimidazo[1,5-a]pyridin-6-yl)methanol

Ethyl 8-chloroimidazo[1,5-a]pyridin-6-ethyl carboxylate I228 (2 g, 8.90 mmol) was dissolved in THF(20 mL), followed by the dropwise addition of DIBAL-H (7.2 mL, 35.61 mmol) at 0°C, and the reaction system was stirred at room temperature to react for 2.0 hours. The reaction mixture was quenched with water (15 mL), filtered with diatomite, and washed with ethyl acetate, and the filtrate was concentrated under reduced pressure to obtain a crude product. The crude product was purified by the silica-gel column chromatography (PE/EA = 10:1~ 2:1) to obtain a yellow solid product (8-chloroimidazo[1,5-a]pyridin-6-yl)methanol I229 (360 mg, yield: 22.14%).
The product was verified by LCMS and H-NMR.
$^1$H NMR (400 MHz, DMSO-$d_6$) δ 8.49 (s, 1H), 8.27 (s, 1H), 7.42 (s, 1H), 6.93 (s, 1H), 5.42 (t, J = 5.6 Hz, 1H), 4.42-4.44 (m, 2H).

Step 7

6-(bromomethyl)-8-chloroimidazo[1,5-a]pyridine

(8-Chloroimidazo[1,5-a]pyridin-6-yl)methanol I229 (270 mg, 1.48 mmol) was dissolved in DCM (20 mL), followed by the addition of $PBr_3$ (400 mg, 1.48 mmol), and the reaction system was stirred at room temperature to react for 30 minutes. The reaction mixture was concentrated under reduced pressure to obtain a yellow solid crude product6-(bromomethyl)-8-chloroimidazo[1,5-a]pyridine I230 (330 mg, yield: 90.9% ).
The product was verified by LCMS.

Step 8

Methyl 3-((8-chloroimidazo[1,5-a]pyridin-6-yl)methoxy)-5-(methylsulfonyl)isothiazole-4-carboxylate

Methyl 3-hydroxyl-5-(methylsulfonyl)isothiazol-4-carboxylate I009 (319 mg, 1.34 mmol) was dissolved in DMF(5 mL), followed by the addition of 6-(bromomethyl)-8-chloroimidazo[1,5-a]pyridine I230 (330 mg, 1.34 mmol) and potassium carbonate (557 mg, 4.03 mmol), and the reaction system was stirred at room temperature to react for 2 hours. The reaction mixture was poured into water (20 mL), and extracted with ethyl acetate (10 mL x 3), and the organic phases were combined, washed with saturated brine (20 mL), dried with anhydrous sodium sulfate, and concentrated under reduced pressure to obtain a crude product. The crude product was purified by the preparative thin-layer chromatography (PE/EA = 1:1) to obtain a yellow solid product methyl 3-((8-chloroimidazo[1,5-a]pyridin-6-yl)methoxy)-5-(methylsulfonyl)isothiazol-4-formate I231 (120 mg, yield: 22.2%).
The product was verified by LCMS and H-NMR.
$^1$H NMR (400MHz, DMSO-$d_6$): δ (ppm) 8.57 (s, 1H), 8.53 (s, 1H), 7.49 (s, 1H), 7.11 (s, 1H), 5.44 (s, 1H), 3.89 (s, 1H), 3.62 (s, 1H).

Step 9

Methyl 3-((8-chloroimidazo[1,5-a]pyridin-6-yl)methoxy)-5-((2,4-dimethoxybenzyl)amino) isothiazole-4-carboxylate

3-((8-Chloroimidazo[1,5-a]pyridin-6-yl)methoxy)-5-(methylsulfonyl)isothiazol-4-methyl formate I231 (120 mg, 0.30 mmol) was dissolved in THF(10 mL), followed by the addition of 2,4-dimethoxybenzylamine (50 mg, 0.30 mmol), and the reaction system was stirred at 60 °C to react for 2 hours. The mixture was poured into water (10 mL) and extracted with ethyl acetate (10 mL x 3), and the organic phases were combined, washed with saturated brine (20 mL), dried with anhydrous sodium sulfate, and concentrated under reduced pressure to obtain a crude product. The crude product was purified by the high-performance liquid chromatography to obtain a yellow solid product methyl 3-((8-chloroimidazo[1,5-a]pyridin-6-yl)methoxy)-5-((2,4-dimethoxybenzyl)amino)isothiazol-4-carboxylate I232 (60 mg, yield: 41.1%).
The product was verified by LCMS and H-NMR.
$^1$H NMR (400 MHz, DMSO-$d_6$) δ 8.55 (s, 1H), 8.42 (s, 1H), 7.46 (s, 1H), 7.17 (d, J = 7.7 Hz, 1H), 7.05 (s, 1H), 6.60 (s, 1H), 6.51 (d, J = 7.7 Hz, 1H), 5.27 (s, 2H), 4.29 (d, J = 5.2 Hz, 2H), 3.84 (s, 3H), 3.76 (s, 6H).

Step 10

Methyl 5-amino-3-((8-chloroimidazo[1,5-a]pyridin-6-yl)methoxy)isothiazole-4-carboxylate

Methyl 3-((8-chloroimidazo[1,5-a]pyridin-6-yl)methoxy)-5-((2,4-dimethoxybenzyl)amino)isothiazol-4-carboxylate I232 (50 mg, 0.10 mmol) was dissolved in DCM (5 mL) and water (1 mL), followed by the portionwise addition of DDQ (93 mg, 0.41 mmol) at 0°C, and the reaction system was stirred at 0 °C to react for 0.5 hours. The reaction mixture was poured into water (10 mL) and extracted with DCM (10 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (PE/EA = 1/1) to obtain a yellow solid product methyl 5-amino-3-((8-chloroimidazo[1,5-a]pyridin-6-yl)methoxy)isothiazol-4-carboxylate I233 (13.5 mg, yield: 38.9%).

The product was verified by LCMS and HNMR.

$^1H$ NMR (400MHz, DMSO-$d_6$): δ (ppm) 8.56 (s, 1H), 8.43 (s, 1H), 7.47 (s, 1H), 7.06 (s, 1H), 6.88 (s, 1H), 5.27 (s, 2H), 3.73 (s, 3H).

Step 11

Methyl 3-((8-chloroimidazo[1,5-a]pyridin-6-yl)methoxy)-5-(3-(4-(pyrrolidin-1-yl)butyl)ureido)isothiazole-4-carboxylate

4-(Pyrrolidin-1-yl)butan-1-amine (12 mg, 83 μmol) was dissolved in anhydrous THF (2 mL), followed by the addition of CDI (13 mg, 83 μmol) under the protection of $N_2$, the mixture was stirred at room temperature to react for 2.0 hours, followed by the addition of DMSO (2 mL), the pressure was then reduced to remove THF, followed by the addition of methyl 5-amino-3-((8-chloroimidazo[1,5-a]pyridin-6-yl)methoxy)isothiazol-4-carboxylate I233 (20 mg, 59 μmol) and potassium carbonate (16 mg, 118 μmol), and the reaction system was stirred at room temperature to react for 2.0 hours. Water (8 mL) was poured into the reaction mixture, which was then subjected to liquid separation and extraction with ethyl acetate (5 mL x 3), and the organic phases were washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a yellow solid product methyl 3-((8-chloroimidazo[1,5-a]pyridin-6-yl)methoxy)-5-(3-(4-(pyrrolidin-1-yl)butyl)ureido)isothiazol-4-formate I234 (15 mg, yield: 50.11%).

The product was verified by LCMS and H-NMR.

$^1H$ NMR (400MHz, $CD_3OD$): δ (ppm) 8.44 (s, 1H), 8.36 (s, 1H), 7.48 (s, 1H), 7.06 (s, 1H), 5.33 (s, 2H), 3.91 (s, 3H), 3.32-3.34 (m, 2H), 2.73 (m, 4H), 2.66-2.68 (m, 2H), 1.87-1.88 (m, 4H), 1.60-1.62 (m, 4H).

Step 12

3-((8-chloroimidazo[1,5-a]pyridin-6-yl)methoxy)-5-(3-(4-(pyrrolidin-1-yl)butyl)ureido)isothiazole-4-carboxamide

3-((8-Chloroimidazo[1,5-a]pyridin-6-yl)methoxy)-5-(3-(4-(pyrrolidin-1-yl)butyl)ureido)isothiazol-4-methyl formate I234 (14 mg, 28 μmol) was dissolved in $NH_3$/MeOH (1 mL, 10 mol/L). The mixture was stirred at 65 °C to react for 16 hours. The reaction mixture was concentrated under reduced pressure to obtain a crude product, and the crude product was purified by the preparative thin-layer chromatography (DCM/MeOH/$NH_3.H_2O$ = 10 /1/0.1%) to obtain a yellow solid product 3-((8-chloroimidazo[1,5-a]pyridin-6-yl)methoxy)-5-(3-(4-(pyrrolidin-1-yl)butyl)ureido)isothiazol-4-formamide T051 (1.2 mg, yield: 8.83%).

The product was verified by LCMS and HNMR.

$^1H$ NMR (400MHz, $CD_3OD$): δ (ppm) 8.46 (s, 1H), 8.43 (s, 1H), 7.50 (s, 1H), 7.14 (s, 1H), 7.08 (s, 1H), 5.45 (s, 2H), 3.24 (s, 2H), 2.05-2.07 (s, 2H), 1.96 (s, 4H), 1.76 (s, 4H) 1.62-1.66 (m, 4H).

Example 30: Synthesis of Compound T052

Synthesis Route:

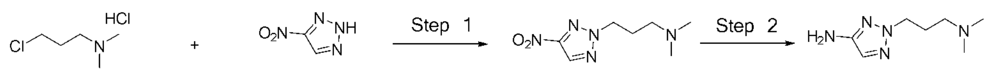


I235 I236

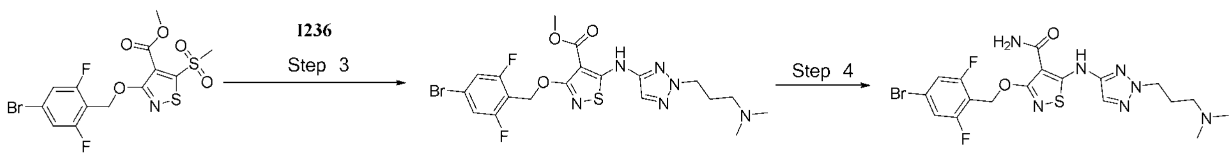


I178 I237 T052

Step 1

N,N-dimethyl-3-(4-nitro-2H-1,2,3-triazol-2-yl)propan-1-amine

(3-Chloropropyl)dimethylamine·hydrochloride (3.05 g, 19.29 mmol) was dissolved in acetonitrile (20.0 mL), followed by the addition of 4-nitro-2H-1,2,3-triazole (2 g, 17.53 mmol) and potassium carbonate (2.67 g, 19.29 mmol), and the reaction system was warmed to 80 °C and stirred to react for 16 hours. After complete reaction, the reaction mixture was poured into water (50 mL), and extracted with ethyl acetate (20 mL x 3), the organic phases were combined, washed with saturated brine, dried with anhydrous sodium sulfate, and concentrated under reduced pressure to obtain a crude product, and the crude product was purified by the flash column chromatography (DCM/MeOH = 120/1-50/1) to obtain a yellow oily liquid product N, N-dimethyl-3-(4-nitro-2H-1,2,3-triazol-2-yl)propan-1-amine I235 (1.6 g, yield: 45.8%).

The product was verified by LCMS and HNMR.

$^1$H NMR (400MHz, DMSO-$d_6$): δ (ppm) 8.69 (s, 1H), 4.55-4.59 (m, 2H), 2.20-2.23 (m, 2H), 2.11 (s, 6H), 2.02-2.07 (m, 2H).

Step 2

2-(3-(dimethylamino)propyl)-2H-1,2,3-triazol-4-amine

N,N-dimethyl-3-(4-nitro-2H-1,2,3-triazol-2-yl)propan-1-amine I235 (1.6 g, 8.03 mmol) and Pd/C (146 mg) were dissolved in anhydrous methanol MeOH (20 mL), and with a hydrogen balloon inserted, the mixture was stirred at 30°C to react for 16 hours. After complete reaction, the mixture was filtered with diatomite and washed with methanol, and the filtrate was concentrated under reduced pressure to obtain a crude product 2-(3-(dimethylamino)propyl)-2H-1,2,3-triazol-4-amine I236 (1.3 g, yield: 95.6%) as a yellow oily liquid product.

The product was verified by LCMS.

Step 3

Methyl 3-((4-bromo-2,6-difluorobenzyl)oxy)-5-((2-(3-(dimethylamino)propyl)-2H-1,2,3-triazol-4-yl)amino)isothiazole-4-carboxylate

Methyl 3-[(4-bromo-2,6-difluorophenyl)methoxy]-5-methylsulfonyl-1,2-thiazol-4-carboxylate I178 (1 g, 2.26 mmol) and 2-(3-(dimethylamino)propyl)-2H-1,2,3-triazol-4-amine I236 (421 mg, 2.49 mmol) were dissolved in anhydrous THF (20 mL), followed by the addition of LiHMDS (1 M, 5.00 mL) at room temperature, and the mixture was then stirred at 30 °C to react for about 2 hours. After complete reaction, water (30 mL) was added to the mixture, which was then extracted with ethyl acetate (20 mL x 3), the organic phases were combined, washed with saturated brine, dried with anhydrous sodium sulfate, and concentrated under reduced pressure to obtain a crude product, and the crude product was purified by the high-performance preparative chromatography to obtain a yellow oily liquid product methyl 3-((4-bromo-2,6-difluorophenyl)oxy)-5-((2-(3-(dimethylamino)propyl)-2H-1,2,3-triazol-4-yl)amino)isothiazol-4-carboxylate I237 (0.2 g, yield: 16.7%).

The product was verified by LCMS.

Step 4

3-((4-bromo-2,6-difluorobenzyl)oxy)-5-((2-(3-(dimethylamino)propyl)-2H-1,2,3-triazol-4-yl)amino)isothiazole-4-carboxamide

In a microwave tube, methyl 3-((4-bromo-2,6-difluorophenyl)oxy)-5-((2-(3-(dimethylamino)propyl)-2H-1,2,3-triazol-4-yl)amino)isothiazol-4-carboxylate I237 (0.3 g, 0.56 mmol) was dissolved in $NH_3$/MeOH (10 mL, 9 mol/L), and the mixture was sealed and stirred at 65 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((4-bromo-2,6-difluorophenyl)oxy)-5-((2-(3-(dimethylamino)propyl)-2H-1,2,3-triazol-4-yl)amino)isothiazol-4-formamide T052 (25.6 mg, yield: 8.78%).
The product was verified by LCMS, H-NMR and C-NMR.
$^1$H NMR (400 MHz, $CD_3OD$) δ: 7.48 (s, 1 H), 7.33-7.35 (d, 2 H, J=8.0Hz), 5.56(s, 2 H), 4.43-4.46 (m, 2 H), 2.42-2.46 (m, 2 H), 2.30 (s, 6 H), 2.16-2.20 (m, 2 H).
$^{13}$C NMR (100 MHz, $CDCl_3$) δ: 171.30, 166.09, 162.99, 162.91, 161.82, 160.45, 160.37, 145.69, 123.39, 120.71, 115.90, 115.87, 115.82, 115.68, 115.63, 115.61, 111.43, 95.48, 57.75, 57.72, 57.68, 56.38, 53.15, 45.40, 27.35.

Example 31: Synthesis of Compound T053

Synthesis Route:

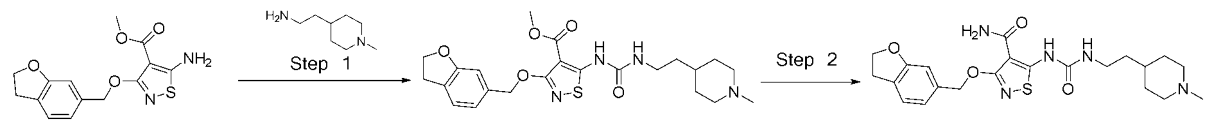


I198 I238 T053

Step 1

methyl 3-((2,3-dihydrobenzofuran-6-yl)methoxy)-5-(3-(2-(1-methylpiperidin-4-yl)ethyl)ureido)isothiazole-4-carboxylate

2-(1-Methylpiperidin-4-yl)ethyl-1-amine (150 mg, 1.05 mmol) was dissolved in anhydrous THF (10 mL) and then cooled to 0°C under the protection of $N_2$, followed by the addition of CDI (170 mg, 1.05 mmol), the mixture was stirred at room temperature to react for 1 hour, followed by the addition of DMSO (10 mL), the pressure was then reduced to remove most of the THF, followed by the addition of methyl 5-amino-3-[(2,3-dihydro-1-benzofuran-6-yl)methoxy]-1,2-thiazol-4-carboxylate I198 (230 mg, 0.75 mmol) and potassium carbonate (208 mg, 1.50 mmol), and the reaction system was stirred at room temperature to react for 16 hours. After complete reaction, water (40 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((2,3-dihydrobenzofuran-6-yl)methoxy)-5-(3-(2-(1-methylpiperidin-4-yl)ethyl)ureido)isothiazol-4-carboxylate I238 (220 mg, yield: 61.74%).
The product was verified by LCMS and H-NMR.
$^1$H NMR (400MHz, DMSO-$d_6$): δ (ppm) 10.37 (s, 1H), 8.12 (t, 1H, J=1.2Hz), 7.22 (d, 1H, J=7.6Hz), 6.90 (d, 1H, J=7.6Hz), 6.84 (s, 1H), 5.28 (s, 2H), 4.53 (t, 2H, J=8.8Hz), 3.81 (s, 3H), 3.14-3.19 (m, 4H), 2.73 (d, 2H, J=11.2Hz), 2.13 (s, 3H), 1.78-1.84 (m, 2H), 1.62 (d, 2H, J=12.0Hz), 1.38 (q, 2H, J=6.8Hz), 1.09-1.25 (m, 3H).

Step 2

3-((2,3-dihydrobenzofuran-6-yl)methoxy)-5-(3-(2-(1-methylpiperidin-4-yl)ethyl)ureido)isothiazole-4-carboxamide

In a microwave tube, methyl 3-((2,3-dihydrobenzofuran-6-yl)methoxy)-5-(3-(2-(1-methylpiperidin-4-yl)ethyl)ureido)isothiazol-4-carboxylate I238 (140 mg, 0.30 mmol) was dissolved in $NH_3$/MeOH (5 mL, 9 mol/L), and the mixture was sealed and stirred at 60 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((2,3-dihydrobenzofuran-6-yl)methoxy)-5-(3-(2-(1-methylpiperidin-4-yl)ethyl)ureido)isothiazol-4-formamide T053 (17 mg, yield: 12.54%).
The product was verified by LCMS, H-NMR and C-NMR.

$^1$H NMR (400 MHz, DMSO-$d_6$) δ: 11.00 (s, 1H), 8.16 (d, 1H, J=5.2Hz), 7.61 (s, 1H), 7.22 (d, 1H, J=7.2Hz), 7.03 (s, 1H), 6.93 (d, 1H, J=7.6Hz), 6.89 (s, 1H), 5.32 (s, 2H), 4.53 (t, 2H, J=8.8Hz), 3.12-3.19 (m, 4H), 2.76 (d, 2H, J=11.6Hz), 2.15 (s, 3H), 1.86 (t, 2H, J=11.2Hz), 1.62 (d, 2H, J=12.0Hz), 1.37 (q, 2H, J=6.8Hz), 1.24-1.26 (m, 1H), 1.12-1.18 (m, 2H).

$^{13}$C NMR (100 MHz, DMSO-$d_6$) δ: 168.47, 164.97, 162.01, 160.36, 154.46, 136.70, 127.93, 125.38, 120.82, 109.26, 97.92, 71.53, 69.98, 55.72, 46.44, 37.50, 36.23, 32.38, 31.95, 29.33.

Example 32: Synthesis of Compound T054

Synthesis Route:

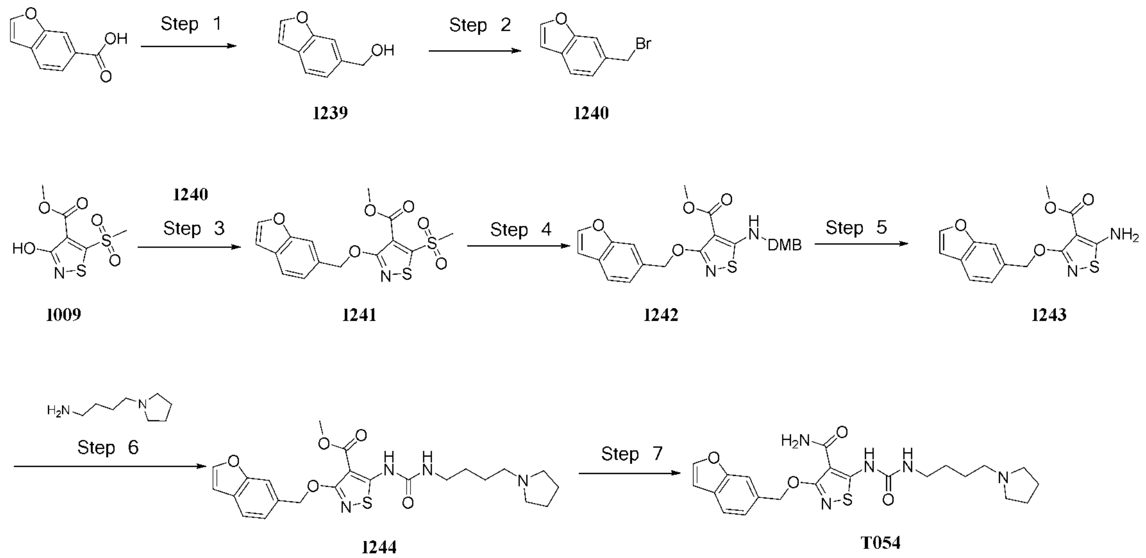


1239 1240

1009 1241 1242 1243

1244 T054

Step 1

Benzofuran-6-ylmethanol

Under the protection of $N_2$, benzofuran-6-carboxylic acid (0.5 g, 3.08 mmol) was dissolved in anhydrous THF (20 mL), followed by the addition of $BH_3.SMe_2$ (10 M, 770.93μL) in the ice water bath, and the mixture was warmed to 60°C and stirred to react for 16 hours. The reaction mixture was poured into methanol (20 mL), then stirred at 60°C to react for 0.5 hours, concentrated under reduced pressure to remove most of the solvent, subsequently poured into water, and extracted with ethyl acetate (10 mL x 3), the organic phases were combined, washed with saturated brine, dried with anhydrous sodium sulfate, and concentrated under reduced pressure to obtain a crude product, and the crude product was purified by the preparative thin-layer chromatography to obtain a yellow oily liquid product benzofuran-6-ylmethanol I239 (0.15 g, 1.01 mmol, yield: 32.83%). The product was verified by LCMS and H-NMR.

$^1$H NMR (400MHz, DMSO-$d_6$): δ (ppm) 7.94 (d, 1H, J=2.4Hz), 7.52-7.80 (m, 2H), 7.20-7.22 (d, 1H, J=8.0Hz), 8.92 (s, 1H), 5.25-5.28 (m, 1H), 4.60-4.61 (m, 2H).

Step 2

6-(bromomethyl)benzofuran

Benzofuran-6-ylmethanol I239 (2.1 g, 14.17 mmol) was dissolved in DCM (30 mL) and then cooled to 0°C, followed by the addition of $PBr_3$ (3.84 g, 14.17 mmol), and the reaction system was stirred at room temperature to react for 1 hour. After complete reaction, the mixture was washed with cold saturated sodium bicarbonate (10 mL x 2), and after liquid separation, the organic phases were washed with saturated brine, dried with anhydrous sodium sulfate, and concentrated under reduced pressure to obtain a crude product 6-(bromomethyl)benzofuran I240 (1.9 g, 9.00 mmol, yield: 63.51%) as a yellow oily liquid product.

The product was verified by LCMS.

Step 3

Methyl 3-(benzofuran-6-ylmethoxy)-5-(methylsulfonyl)isothiazole-4-carboxylate

Methyl 3-hydroxyl-5-(methylsulfonyl)isothiazol-4-carboxylate I009 (1.3 g, 5.48 mmol) and potassium carbonate (1.51 g, 10.96 mmol) were dissolved in DMF (15 mL), followed by the addition of 6-(bromomethyl)benzofuran I240 (1.39 g, 6.58 mmol), and the reaction system was stirred at room temperature to react for 4 hours. Water (40 mL) was added to the reaction mixture, which was then subjected to liquid separation and extraction with ethyl acetate (20 mL x 3), the organic phases were sequentially washed with water and saturated brine, dried with anhydrous sodium sulfate, and concentrated under reduced pressure to obtain a crude product, and the crude product was purified by the flash column chromatography (PE/EA = 10/1-5/1) to obtain a white solid product methyl 3-(benzofuran-6-methoxy)-5-(methylsulfonyl)isothiazol-4-carboxylate I241 (0.27 g, 734.90μmol, yield: 13.41%).

The product was verified by LCMS and H-NMR.

$^1H$ NMR (400MHz, DMSO-$d_6$): δ (ppm) 8.03 (d, 1H, J=2.0Hz), 7.68-7.73 (m, 2H), 7.37-7.39 (m, 1H), 6.98-6.99 (m, 1H), 5.60 (s, 2H), 3.88 (s, 3H), 3.61 (s, 3H).

Step 4

Methyl 3-(benzofuran-6-ylmethoxy)-5-((2,4-dimethylbenzyl)amino)isothiazole-4-carboxylate

Methyl 3-(benzofuran-6-methoxy)-5-(methylsulfonyl)isothiazol-4-carboxylate I241 (0.27 g, 734.90 μmol) was dissolved in THF(10 mL), followed by the addition of 2,4-dimethoxybenzylamine (614.39 mg, 3.67 mmol), and the reaction system was stirred at 65 °C to react for 4 hours. After the reaction was finished, the reaction mixture was added to ice water (10 mL), and regulated to pH of 5, the aqueous phase was extracted with ethyl acetate (10 mL x 3), the organic phases were combined, sequentially washed with water and saturated brine, dried with anhydrous sodium sulfate, and concentrated under reduced pressure to obtain a crude product, and the crude product was purified by the preparative thin-layer chromatography (PE/EA = 3/1) to obtain a white solid product methyl 3-(benzofuran-6-methoxy)-5-((2,4-dimethylbenzyl)amino)isothiazol-4-carboxylate I242 (0.19 g, 418.05μmol, yield: 56.88%).

The product was verified by LCMS.

Step 5

Methyl 5-amino-3-(benzofuran-6-ylmethoxy)isothiazole-4-carboxylate

Methyl 3-(benzofuran-6-methoxy)-5-((2,4-dimethylbenzyl)amino)isothiazol-4-carboxylate I242 (0.22 g, 484.05 μmol) was dissolved in DCM/$H_2O$ (15/3 mL), followed by the portionwise addition of DDQ (164.82 mg, 726.08 μmol) at 0°C, and the reaction system was stirred at room temperature to react for 0.3 hours. After complete reaction, water (10 mL) was added to the reaction mixture, which was then extracted with DCM (10 mL x 2), the organic phases were combined, washed with saturated brine, dried with anhydrous sodium sulfate, and concentrated under reduced pressure to obtain a crude product, and the crude product was purified by the preparative thin-layer chromatography (PE/EA = 3/1) to obtain a white solid product methyl 5-amino-3-(benzofuran-6-methoxy)isothiazol-4-carboxylate I243 (0.1 g, 328.60 μmol, yield: 67.89%).

The product was verified by LCMS.

Step 6

Methyl 3-(benzofuran-6-ylmethoxy)-5-(3-(4-(pyrrolidin-1-yl)butyl)ureido)isothiazole-4-carboxylate

4-(Pyrrolidin-1-yl)butyl-1-amine (65.44 mg, 460.04 μmol) was dissolved in anhydrous THF (10 mL) and then cooled to 0°C under the protection of $N_2$, followed by the addition of CDI (74.14 mg, 460.04 μmol), the mixture was stirred at room temperature to react for 1 hour, followed by the addition of DMSO (10 mL), the pressure was then reduced to remove most of the THF, followed by the addition of methyl 5-amino-3-(benzofuran-6-methoxy)isothiazol-4-carboxylate I243 (0.1 g, 328.60 μmol) and potassium carbonate (90.83 mg, 657.20 μmol), and the reaction system was stirred at room temperature to react for 1 hour. After complete reaction, water (30 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-(benzofuran-6-ylmethoxy)-5-(3-(4-(pyrrolidin-1-yl)butyl)ureido)isothiazol-4-carboxylate I244 (0.1

g, 211.62 μmol, yield: 64.40%).

The product was verified by LCMS and H-NMR.

$^1$H NMR (400MHz, $CD_3OD$): δ (ppm) 7.76 (d, 1H, J=2.4Hz), 7.60-7.69 (m, 2H), 7.33-7.35 (m, 1H), 6.84 (d,1H, J=1.2Hz), 5.49 (s, 2H), 3.88-3.89 (m, 3H), 3.32-3.33 (m, 4H), 2.54-2.63 (m, 4H), 1.82-1.86 (m, 4H), 1.60-1.64 (m, 4H).

Step 7

3-(benzofuran-6-ylmethoxy)-5-(3-(4-(pyrrolidin-1-yl)butyl)ureido)isothiazole-4-carboxamide

In a microwave tube, methyl 3-(benzofuran-6-ylmethoxy)-5-(3-(4-(pyrrolidin-1-yl)butyl)ureido)isothiazol-4-carboxylate I244 (0.1 g, 211.62 μmol) was dissolved in $NH_3$/MeOH (10 mL, 9 mol/L), and the mixture was stirred at 60 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-(benzofuran-6-methoxy)-5-(3-(4-(pyrrolidin-1-yl)butyl)ureido)isothiazol-4-formamide T054 (24.5 mg, 53.55 μmol, yield: 25.30%).

The product was verified by LCMS, H-NMR and C-NMR.

$^1$H NMR (400MHz, $CDCl_3$): δ (ppm) 10.90 (s,1 H), 7.89 (d, 1H, J=1.2Hz), 7.68 (d, 1H, J=2Hz), 7.62-7.64 (m, 2H), 7.33-7.35 (m, 1H), 7.18 (s, 1H), 6.81 (d, 1H, J=1.2Hz), 5.71 (s, 1H), 5.57-5.63 (m, 2H), 3.32-3.33 (m, 2H), 2.48-2.54 (m, 6H), 1.84-1.87 (m, 4H), 1.64-1.66 (m,4H).

$^{13}$C NMR (100MHz, $CDCl_3$): δ (ppm) 169.58, 165.97, 161.94, 154.93, 154.06, 145.85, 132.27, 127.77, 123.43, 121.40, 111.69, 106.54, 97.29, 70.74, 55.86, 53.91, 40.56, 28.10, 26.72, 23.38.

Example 33: Synthesis of Compound T055

Synthesis Route:

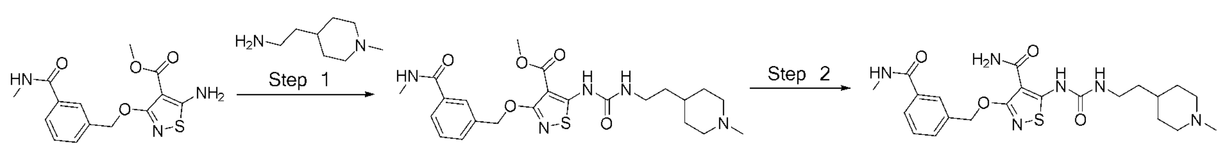


I222 I245 T055

Step 1

methyl 3-((3-(methylcarbamoyl)benzyl)oxy)-5-(3-(2-(1-methylpiperidin-4-yl)ethyl)ureido)isothiazole-4-carboxylate

2-(1-Methyl-4-piperidyl)ethylamine (92.95 mg, 653.49 mmol) was dissolved in anhydrous THF (10 mL) and then cooled to 0°C under the protection of $N_2$, followed by the addition of CDI (94.07 mg, 653.49 mmol), the mixture was stirred at room temperature to react for 1 hour, followed by the addition of DMSO (10 mL), the pressure was then reduced to remove most of the THF, followed by the addition of methyl 5-amino-3-((4-(methylcarbamoyl)benzyl)oxy)isothiazol-4-carboxylate I222 (150.00 mg, 466.78 mmol) and potassium carbonate (64.51 mg, 466.78 mmol), and the reaction system was stirred at room temperature to react for 16 hours. After complete reaction, water (40 mL) was added to the reaction mixture, which was then extracted with ethyl acetate (10 mL x 3), the organic phases were combined, sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% NH3.H2O) to obtain a white solid product methyl 3-((3-(methylcarbamoyl)benzyl)oxy)-5-(3-(2-(1-methylpiperidin-4-yl) ethyl)ureido)isothiazol-4-carboxylate I245 (120 mg, 245.10 μmol, yield: 52.51%).

The product was verified by LCMS and H-NMR.

1H NMR (400MHz, DMSO-$d_6$ ): δ (ppm) 10.39 (s, 1H), 8.46 (d, 1H, J=4.4Hz), 8.13 (t, 1H, J=5.6Hz), 7.96 (s, 1H), 7.77 (d, 1H, J=8.0Hz), 7.59 (d, 1H, J=7.6Hz), 7.47 (t, 1H, J=8.0Hz), 5.41 (s, 2H), 3.84 (s, 3H), 3.18 (dd, 2H, J=12.8, 6.8Hz), 2.79 (d, 3H, J=4.4Hz), 2.74 (d, 2H, J=11.2Hz), 2.13 (s, 3H), 1.81 (t, 2H, J=11.2Hz), 1.62 (d, 2H, J=11.6Hz), 1.39 (q, 2H, J=6.8Hz), 1.24-1.25 (m, 1H), 1.09-1.19 (m, 2H).

Step 2

3-((3-(methylcarbamoyl)benzyl)oxy)-5-(3-(2-(1-methylpiperidin-4-yl)ethyl)ureido)isothia zole-4-carboxamide

In a microwave tube, methyl 3-((3-(methylcarbamoyl)benzyl)oxy)-5-(3-(2-(1-methylpiperidin-4-yl)ethyl)ureido) isothiazol-4-carboxylate I245 (120.00 mg, 245.10 μmol) was dissolved in $NH_3$/MeOH (20 mL, 9 mol/L), and the mixture was stirred at 60 °C to react for 16 hours. The reaction mixture was concentrated under reduced pressure to obtain a crude product, and the crude product was purified by the preparative thin-layer chromatography (DCM/MeOH = 10/1, 1% NH3.H2O) to obtain a white solid product 3-((3-(methylcarbamoyl)benzyl)oxy)-5-(3-(2-(1-methylpiperidin-4-yl)ethyl) ureido)isothiazol-4-formamide T055 (28.5 mg, 60.05 μmol, yield: 24.50%).
The product was verified by LCMS, H-NMR and C-NMR.
1H NMR (400MHz, $CD_3OD$): δ (ppm) 7.94 (s, 1H), 7.81 (d, 1H, J=7.6Hz), 7.67 (d, 1H, J=7.6Hz), 7.51 (t, 1H, J=7.6Hz), 5.54 (s, 1H), 3.28 (t, 1H, J=7.2Hz), 2.94 (s, 5H), 2.31 (d, 1H, J=8.0Hz), 2.05-2.10 (m, 2H), 1.79 (d, 2H, J=12.4Hz), 1.51 (dd, 2H, J=13.6, 7.2Hz), 1.40 (s, 1H), 1.29-1.35 (m, 2H).
$^{13}$C NMR (100 MHz, DMSO-d6) δ 168.52, 166.84, 166.76, 164.89, 161.93, 154.46, 137.16, 135.25, 131.21, 128.97, 127.42, 127.18, 97.92, 69.58, 55.85, 46.67, 37.51, 36.29, 32.51, 32.13, 26.73, 26.60.

Example 34: Synthesis of Compound T056

Synthesis Route:

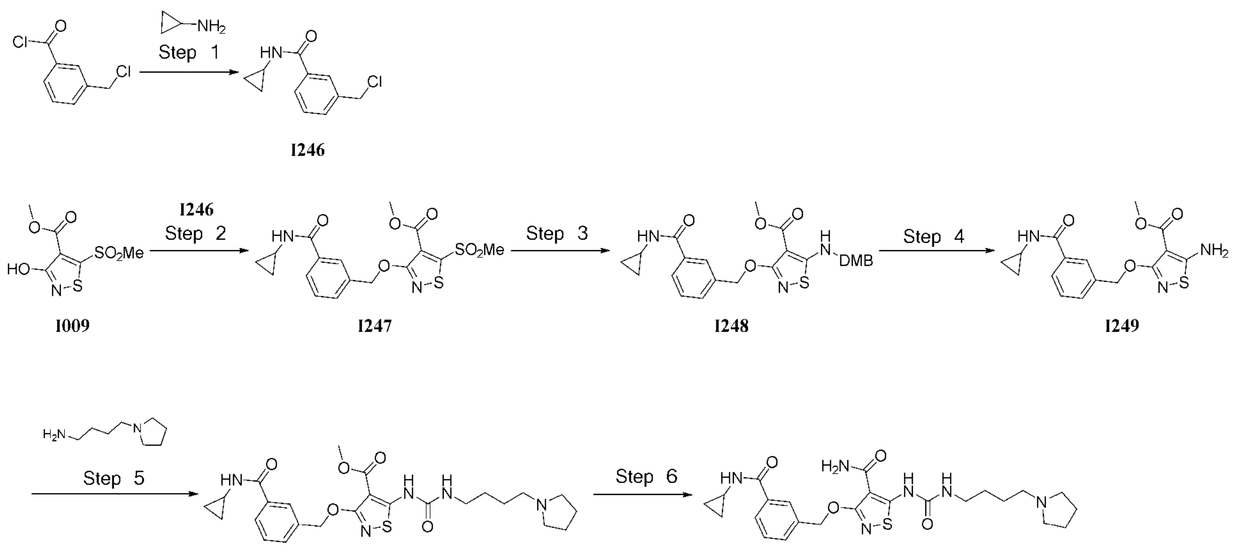


I246 I009 I247 I248 I249 I250 T056

Step 1

3-(chloromethyl)-N-cyclopropylbenzamide

3-(Chloromethyl)benzoyl chloride (0.2 g, 1.06 mmol) was dissolved in DCM (10 mL), followed by the addition of cyclopropyl amine (66.45 mg, 1.16 mmol) and triethylamine (321.17 mg, 3.17 mmol) at 0 °C, and the mixture was stirred to react for about 1 hour. After complete reaction, the mixture was added to water (20 mL) and extracted with dichloromethane (10 mL x 3), and the organic phases were combined, washed with saturated brine, dried with anhydrous sodium sulfate, and concentrated under reduced pressure to obtain a white solid crude product 3-(chloromethyl)-N-isopropylbenzamide I246 (0.18 g, 858.49 μmol, yield: 81.14%).
The product was verified by LCMS and H-NMR.
$^1$H NMR (400MHz, $CDCl_3$): δ (ppm) 7.78 (s, 1H), 7.69 (d, 1H, J=7.6Hz), 7.54 (d, 1H, J=7.6Hz), 7.41-7.45 (m, 1H), 6.31 (s, 1H), 2.90-2.95 (m, 1H), 0.88-0.92 (m, 2H), 0.63-0.67 (m, 2H).

Step 2

Methyl 3-((3-(cyclopropylcarbamoyl)benzyl)oxy)-5-(methylsulfonyl)isothiazole-4-carboxylate

Methyl 3-hydroxyl-5-(methylsulfonyl)isothiazol-4-carboxylate I009 (0.3 g, 1.26 mmol) and potassium carbonate (349.51 mg, 2.53 mmol) were dissolved in DMF (5 mL), followed by the addition of 3-(chloromethyl)-N-isopropylbenzamide I246 (318.15 mg, 1.52 mmol), and the reaction system was stirred at room temperature to react for 4 hours. Water (20 mL) was added to the reaction mixture, which was then subjected to liquid separation and extraction with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (PE/EA = 1/1) to obtain a white solid product methyl 3-((3-(cyclopropylcarbamoyl) benzyl)oxy)-5-(methylsulfonyl)isothiazol-4-carboxylate I247 (0.1 g, 243.63 μmol, yield: 19.27%).

The product was verified by LCMS and H-NMR.

$^1$H NMR (400MHz, $CDCl_3$): δ (ppm) 7.92 (s, 1H), 7.70 (d, 1H, J=7.6Hz), 7.58 (d, 1H, J=7.6Hz), 7.43-7.47 (m, 1H), 6.34 (s, 1H), 5.53 (s, 1H), 4.00 (s, 3H), 3.49 (s, 3H), 2.91-2.95 (m, 1H), 0.88-0.93 (m, 2H), 0.63-0.67 (m, 2H).

Step 3

Methyl 3-((3-(cyclopropylcarbamoyl)benzyl)oxy)-5-((2,4-dimethoxybenzyl)amino)isothiazole-4-carboxylate

Methyl 3-((3-(cyclopropylcarbamoyl)benzyl)oxy)-5-(methylsulfonyl)isothiazol-4-carboxylate I247 (0.14 g, 341.08 μmol) was dissolved in THF(10 mL), followed by the addition of 2,4-dimethoxybenzylamine (570.30 mg, 3.41 mmol), and the reaction system was stirred at 65 °C to react for 4 hours. After the reaction was finished, water (10 mL) was added to the reaction mixture, which was then extracted with ethyl acetate (10 mL x 3), the organic phases were combined, sequentially washed with water and saturated brine, dried with anhydrous sodium sulfate, and concentrated under reduced pressure to obtain a crude product, and the crude product was purified by the preparative thin-layer chromatography (PE/EA = 1/1) to obtain a white solid product methyl 3-((3-(cyclopropylcarbamoyl)benzyl)oxy)-5-((2,4-dimethoxybenzyl)amino)isothiazol-4-carboxylate I248 (0.15 g, 301.47 μmol, yield: 88.39%).

The product was verified by LCMS and H-NMR.

$^1$H NMR (400 MHz, $CDCl_3$): δ (ppm) 8.09-8.12 (m, 1H), 7.89 (s, 1H), 7.68 (d, 1H, J=8Hz), 7.57(d, 1H, J=7.6Hz), 7.41-7.45 (m, 1H), 7.18 (d, 1H, J=8Hz), 6.45-6.49 (m, 2H), 6.29 (s, 1H), 5.45 (s, 2H), 4.28 (d, 2H, J=6Hz), 3.82-3.86 (m, 9H), 2.91-2.94 (m, 1H), 0.87-0.92 (m, 2H), 0.62-0.66 (m, 2H).

Step 4

Methyl 5-amino-3-((3-(cyclopropylcarbamoyl)benzyl)oxy)isothiazole-4-carboxylate

Methyl 3-((3-(cyclopropylcarbamoyl)benzyl)oxy)-5-((2,4-dimethoxybenzyl)amino)isothiazol-4-carboxylate I248 (0.15 g, 301.47 μmol) was dissolved in DCM/$H_2O$ (8/2 mL), followed by the portionwise addition of DDQ (68.43 mg, 301.47 μmol) at 0°C, and the reaction system was stirred at room temperature to react for 0.5 hours. After complete reaction, water (10 mL) was added to the reaction mixture, which was then extracted with DCM (10 mL x2), the organic phases were combined, washed with saturated brine, dried with anhydrous sodium sulfate, and concentrated under reduced pressure to obtain a crude product, and the crude product was purified by the preparative thin-layer chromatography to obtain a yellow solid product methyl 5-amino-3-((3-(cyclopropylcarbamoyl)benzyl)oxy)isothiazol-4-carboxylate I249 (30 mg, 86.36 μmol, yield: 28.65%).

The product was verified by LCMS and H-NMR.

$^1$H NMR (400 MHz, $CDCl_3$): δ (ppm) 7.92 (s, 1H), 7.70 (d, 1H, J=8Hz), 7.60 (d, 1H, J=8Hz), 7.42-7.46 (m, 1H), 6.47 (s, 1H), 6.30 (s, 1H), 5.47 (s, 2H), 3.90-3.92 (m, 3H), 2.91-2.96 (m, 1H), 0.88-0.93 (m, 2H), 0.63-0.67 (m, 2H).

Step 5

Methyl 3-((3-(cyclopropylcarbamoyl)benzyl)oxy)-5-(3-(4-(pyrrolidin-1-yl)butyl)ureido)isothiazole-4-carboxylate

4-(Pyrrolidin-1-yl)butyl-1-amine (28.66 mg, 201.50 μmol) was dissolved in anhydrous THF (5 mL) and then cooled to 0°C under the protection of $N_2$, followed by the addition of CDI (32.47 mg, 201.50 μmol), the mixture was stirred at room temperature to react for 1 hour, followed by the addition of DMSO (5 mL), the pressure was then reduced to remove most of the THF, followed by the addition of methyl 5-amino-3-((3-(cyclopropylcarbamoyl)benzyl)oxy)isothiazol-4-carboxylate I249 (50 mg, 143.93 μmol) and potassium carbonate (39.79 mg, 287.86 μmol), and the reaction system was stirred at room temperature to react for 2 hours. After complete reaction, water (20 mL) was added to the reaction mixture, which was then extracted with ethyl acetate (10 mL x 3), the organic phases were sequentially washed with water

and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((3-(cyclopropylcarbamoyl)benzyl)oxy)-5-(3-(4-(pyrrolidin-1-yl)butyl)ureido)isothiazol-4-carboxylate I250 (40 mg, 77.58 μmol, yield: 53.90%).

The product was verified by LCMS and H-NMR.

$^1H$ NMR (400MHz, $CDCl_3$) δ (ppm) 10.19-10.23 (m, 1H), 8.21-8.26 (m, 1H), 7.92 (s, 1H), 7.67-7.70 (m, 1H), 7.58 (d, 1H, J=7.6Hz), 7.38-7.45 (m, 1H), 6.36 (s, 1H), 5.48 (s, 2H), 3.82-3.90 (m, 3H), 3.32 (s, 2H), 2.90-2.96 (m, 1H), 2.42-2.64 (m, 6H), 1.91-1.94 (m, 4H), 1.58-1.69 (m, 4H), 0.83-0.92 (m, 2H), 0.60-0.67 (m, 2H).

Step 6

3-((3-(cyclopropylcarbamoyl)benzyl)oxy)-5-(3-(4-(pyrrolidin-1-yl)butyl)ureido)isothiazole-4-carboxamide

In a microwave tube, methyl 3-((3-(cyclopropylcarbamoyl)benzyl)oxy)-5-(3-(4-(pyrrolidin-1-yl)butyl)ureido)isothiazol-4-carboxylate I250 (0.1 g, 193.94 μmol) was dissolved in $NH_3$/MeOH (5 mL, 9 mol/L), and the mixture was sealed in the tube and stirred at 40 °C to react for 16 hours. After complete reaction, the reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(cyclopropylcarbamoyl)benzyl)oxy)-5-(3-(4-(pyrrolidin-1-yl)butyl)ureido)isothiazol-4-formamide T056 (22.8 mg, 45.54 μmol, yield: 23.48%).

The product was verified by LCMS, H-NMR and C-NMR.

$^1H$ NMR (400MHz, $CD_3OD$) δ (ppm) 7.93 (s, 1H), 7.79 (d, 1H, J=7.6Hz), 7.66 (d, 1H, J=7.6Hz), 7.49 (t, 1H, J=15.2Hz), 5.53 (s, 2H), 3.26 (t, 2H, J=12.8Hz), 2.84-2.89 (m, 1H), 2.70 (s, 4H), 2.61-2.65 (m, 2H), 1.85-1.88 (m, 4H), 1.59-1.66 (m, 4H), 0.80-0.85 (m, 2H), 0.64-0.67 (m, 2H).

$^{13}C$ NMR (100MHz, $CD_3OD$): δ (ppm) 169.99, 168.88, 165.82, 161.87, 154.73, 136.81, 134.59, 131.09, 128.51, 126.85, 91.16, 69.50, 55.68, 53.58, 53.42, 39.51, 33.70, 27.43, 25.34, 22.75, 22.67, 5.17.

Example 35: Synthesis of Compound T057

Synthesis Route:

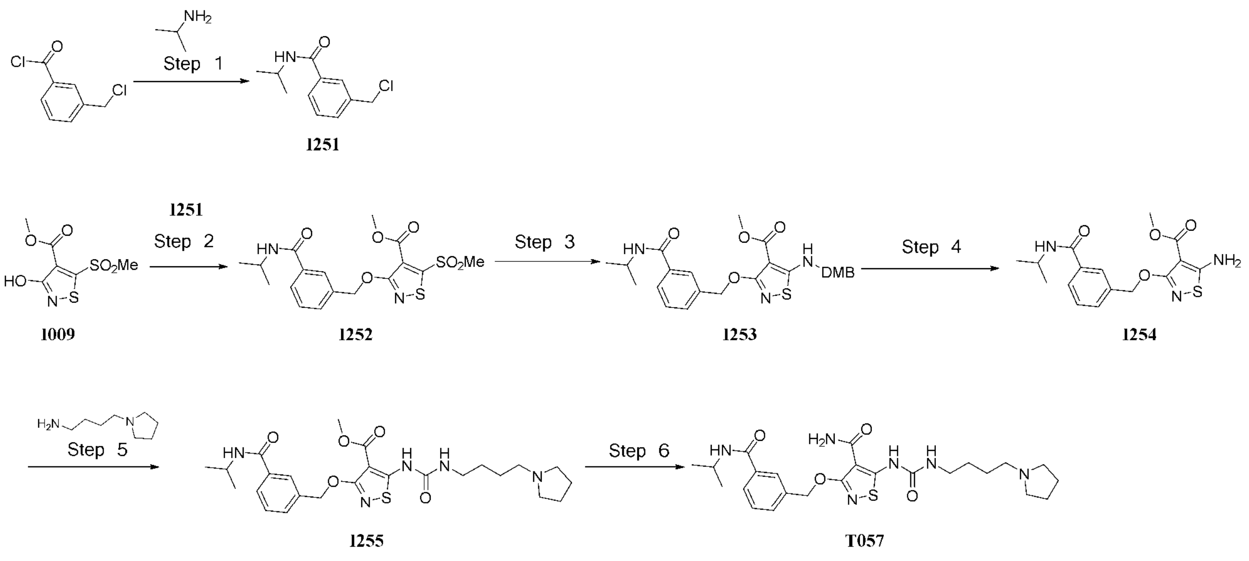


Step 1

3-(chloromethyl)-N-isopropylbenzamide

3-(Chloromethyl)benzoyl chloride (1.08 g, 5.71 mmol) was dissolved in DCM (10 mL), followed by the addition of isopropyl amine (371.52 mg, 6.29 mmol, 537.66 μL) and triethylamine (578.19 mg, 5.71 mmol) at 0 °C, and the mixture was stirred to react for about 1 hour. After complete reaction, the mixture was added to water (20 mL) and extracted with

dichloromethane (10 mL x 3), and the organic phases were combined, washed with saturated brine, dried with anhydrous sodium sulfate, and concentrated under reduced pressure to obtain a white solid crude product 3-(chloromethyl)-N-isopropylbenzamide I251 (590 mg, 2.79 mmol, yield: 48.78%).

The product was verified by LCMS and H-NMR.

$^1$H NMR (400MHz, DMSO-$d_6$): δ (ppm) 8.28 (d, J = 7.6 Hz, 1H), 7.91 (s, 1H), 7.81 (d, J = 8.0 Hz, 1H), 7.58 (d, J = 7.6 Hz, 1H), 7.47 (t, J = 7.6 Hz, 1H), 4.81 (s, 2H), 4.17-4.03 (m, 1H), 1.18 (s, 3H), 1.17 (s, 3H).

Step 2

Methyl 3-((3-(isopropylcarbamoyl)benzyl)oxy)-5-(methylsulfonyl)isothiazole-4-carboxylate

Methyl 3-hydroxyl-5-(methylsulfonyl)isothiazol-4-carboxylate I009 (727.38 mg, 3.07 mmol) and potassium carbonate (770.42 mg, 5.57 mmol) were dissolved in DMF (15 mL), followed by the addition of 3-(chloromethyl)-N-isopropylbenzamide I251 (590 mg, 2.79 mmol), and the reaction system was stirred at room temperature to react for 16 hours. Water (30 mL) was added to the reaction mixture, which was then subjected to liquid separation and extraction with ethyl acetate (20 mL x 3), the organic phases were sequentially washed with water and saturated brine, dried with anhydrous sodium sulfate, and concentrated under reduced pressure to obtain a crude product, and the crude product was purified by the flash column chromatography (PE/EA = 1/1) to obtain a white solid product methyl 3-((3-(isopropylcarbamoyl)benzyl)oxy)-5-(methylsulfonyl)isothiazol-4-carboxylate I252 (319 mg, 773.37 μmol, yield: 27.75%).

The product was verified by LCMS and H-NMR.

$^1$H NMR (400MHz, DMSO-$d_6$): δ (ppm) 8.26 (d, J = 7.6 Hz, 1H), 7.96 (s, 1H), 7.83 (d, J = 7.6 Hz, 1H), 7.62 (d, J = 7.2 Hz, 1H), 7.49 (t, J = 7.6 Hz, 1H), 5.54 (s, 2H), 4.16-4.05 (m, 1H), 3.90 (s, 3H), 3.62 (s, 4H), 1.18 (s, 3H), 1.17 (s, 3H).

Step 3

Methyl 5-((2,4-dimethoxybenzyl)amino)-3-((3-(isopropylcarbamoyl)benzyl)oxy) isothiazole-4-carboxylate

Methyl 3-((3-(isopropylcarbamoyl)benzyl)oxy)-5-(methylsulfonyl)isothiazol-4-carboxylate I252 (612 mg, 1.48 mmol) was dissolved in THF(10 mL), followed by the addition of 2,4-dimethoxybenzylamine (248.08 mg, 1.48 mmol), and the reaction system was stirred at 65 °C to react for 1 hour. After the reaction was finished, water (20 mL) was added to the reaction mixture, which was then extracted with ethyl acetate (20 mL x 3), the organic phases were combined, sequentially washed with water and saturated brine, dried with anhydrous sodium sulfate, and concentrated under reduced pressure to obtain a crude product, and the crude product was purified by the preparative thin-layer chromatography (PE/EA = 1/1) to obtain a white solid product methyl 5-((2,4-dimethoxybenzyl)amino)-3-((3-(isopropylcarbamoyl)benzyl)oxy)isothiazol-4-carboxylate I253 (615 mg, 1.23 mmol, yield: 82.97%).

The product was verified by LCMS and H-NMR.

$^1$H NMR (400 MHz, DMSO-$d_6$): δ (ppm) 8.43 (t, J = 2.4 Hz, 1H), 8.23 (d, J = 8.4 Hz, 1H), 7.92 (s, 1H), 7.77 (d, J = 6.8 Hz, 1H), 7.56 (d, J = 8.0 Hz, 1H), 7.45 (t, J = 8.0 Hz, 1H), 7.26 (d, J = 8.4 Hz, 1H), 7.17 (d, J = 8.4 Hz, 1H), 6.60 (d, J = 2.4 Hz, 2H), 6.55 - 6.48 (m, 2H), 4.29 (d, J = 6.0 Hz, 1H), 4.14 - 4.07 (m, 1H), 3.81 (s, 3H), 3.77 (s, 3H), 3.75 (s, 3H), 1.17 (s, 3H), 1.16 (s, 3H).

Step 4

Methyl 5-amino-3-((3-(isopropylcarbamoyl)benzyl)oxy)isothiazole-4-carboxylate

Methyl 5-((2,4-dimethoxybenzyl)amino)-3-((3-(isopropylcarbamoyl)benzyl)oxy)isothiazol-4-carboxylate I253 (615.00 mg, 1.23 mmol) was dissolved in DCM/$H_2O$ (5/1 mL), followed by the portionwise addition of DDQ (838.35 mg, 3.69 mmol) at 0°C, and the reaction system was stirred at room temperature to react for half an hour. After complete reaction, water (20 mL) was added to the reaction mixture, which was then extracted with DCM (10 mL x2), the organic phases were combined, washed with saturated brine, dried with anhydrous sodium sulfate, and concentrated under reduced pressure to obtain a crude product, and the crude product was purified by the preparative thin-layer chromatography (PE/EA = 1/1) to obtain a yellow solid product methyl 5-amino-3-((3-(isopropylcarbamoyl)benzyl)oxy)isothiazol-4-carboxylate I254 (192 mg, 549.51 μmol, yield: 44.64%).

The product was verified by LCMS and H-NMR.

$^1$H NMR (400 MHz, $CDCl_3$): δ (ppm) 7.92 (s, 1H), 7.71 (d, J = 7.6 Hz, 1H), 7.59 (d, J = 7.6 Hz, 1H), 7.45 (t, J = 7.6 Hz, 1H), 6.56 (s, 2H), 6.00 (s, 1H), 5.48 (s, 2H), 4.38 - 4.26 (m, 1H), 3.90 (s, 3H), 1.30 (s, 3H), 1.29 (s, 3H).

Step 5

Methyl 3-((3-(isopropylcarbamoyl)benzyl)oxy)-5-(3-(4-(pyrrolidin-1-yl)butyl)ureido)isothiazole-4-carboxylate

4-(Pyrrolidin-1-yl)butyl-1-amine (109.43 mg, 769.31 μmol) was dissolved in anhydrous THF (5 mL) and then cooled to 0°C under the protection of $N_2$, followed by the addition of CDI (123.98 mg, 769.31 μmol), the mixture was stirred at room temperature to react for 1 hour, followed by the addition of DMSO (5 mL), the pressure was then reduced to remove most of the THF, followed by the addition of methyl 5-amino-3-((3-(isopropylcarbamoyl)benzyl)oxy)isothiazol-4-carboxylate I254 (192 mg, 549.51 μmol) and potassium carbonate (151.89 mg, 1.10 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((3-(isopropylcarbamoyl)benzyl)oxy)-5-(3-(4-(pyrrolidin-1-yl)butyl)ureido)isothiazol-4-carboxylate I255 (193 mg, 372.85 μmol, yield: 67.85%).

The product was verified by LCMS and H-NMR.

$^1$H NMR (400MHz, $CDCl_3$) δ (ppm) 10.19 (s, 1H), 8.26 (s, 1H), 7.91 (s, 1H), 7.68 (d, J = 7.6 Hz, 1H), 7.56 (d, J = 7.6 Hz, 1H), 7.43 (t, J = 7.6 Hz, 1H), 7.11 (s, 1H), 5.47 (s, 2H), 4.35- 4.24 (m, 1H), 3.90 (s, 3H), 3.30 (s, 2H), 2.59 (s, 4H), 2.52 (t, J = 5.8 Hz, 2H), 1.88 (s, 4H), 1.67 (s, 4H),1.28 (s, 4H), 1.26 (s, 4H).

Step 6

3-((3-(isopropylcarbamoyl)benzyl)oxy)-5-(3-(4-(pyrrolidin-1-yl)butyl)ureido)isothiazole-4-carboxamide

In a microwave tube, methyl 3-((3-(isopropylcarbamoyl)benzyl)oxy)-5-(3-(4-(pyrrolidin-1-yl)butyl)ureido)isothiazol-4-carboxylate I255 (80 mg, 154.55 μmol) was dissolved in $NH_3$/MeOH (5 mL, 9 mol/L), and the mixture was stirred at 40 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(isopropylcarbamoyl)benzyl)oxy)-5-(3-(4-(pyrrolidin-1-yl)butyl)ureido) isothiazol-4-formamide T057 (22.7 mg, 45.16 μmol, yield: 29.22%).

The product was verified by LCMS, H-NMR and C-NMR.

$^1$H NMR (400MHz, $CDCl_3$) : δ (ppm) 10.93 (s, 1H), 7.93-7.77 (m, 2H), 7.72 (d, J = 7.6 Hz, 1H), 7.56 (d, J = 7.6 Hz, 1H), 7.45 (t, J = 7.6 Hz, 1H), 7.09 (s, 1H), 5.94 (d, J = 1.2 Hz, 1H), 5.49 (s, 2H), 5.45 (s, 1H), 4.43-4.24 (m, 1H), 3.31 (s, 2H), 2.67 (s, 3H), 2.58 (s, 2H), 1.90 (s, 4H), 1.68 (s, 4H), 1.28 (s, 3H), 1.26 (s, 3H).

$^{13}$C NMR (100MHz, $CD_3OD$): δ (ppm) 168.85, 167.65, 165.80, 161.86, 154.70, 136.73, 135.14, 130.89, 128.45, 126.89, 97.36, 69.52, 55.70, 53.56, 41.80, 39.51, 27.44, 25.40, 22.74, 21.15.

Example 36: Synthesis of Compound T058

Synthesis Route:

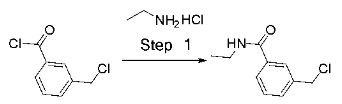


I256

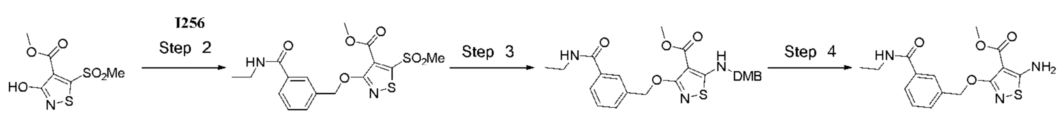


I009 I257 I258 I259

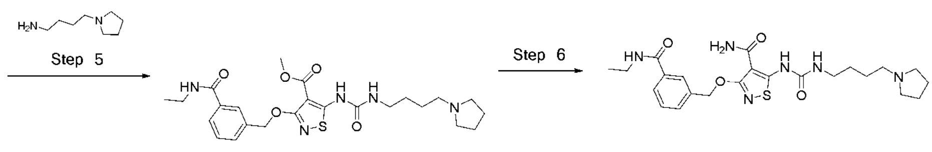


I260 T058

Step 1

3-(chloromethyl)-N-ethylbenzamide

3-(Chloromethyl)benzoyl chloride (1.08 g, 5.71 mmol) was dissolved in DCM (30 mL), followed by the addition of ethylamine· hydrochloride (647.05 mg, 7.93 mmol) and triethylamine (2.41 g, 23.80 mmol) at 0 °C, and the mixture was stirred to react for about 1 hour. After complete reaction, the mixture was added to water (50 mL) and extracted with dichloromethane (20 mL x 3), and the organic phases were combined, washed with saturated brine, dried with anhydrous sodium sulfate, and concentrated under reduced pressure to obtain a white solid crude product 3-(chloromethyl)-N-ethylbenzamide I256 (940 mg, 4.76 mmol, yield: 59.93%).
The product was verified by LCMS and H-NMR.
$^1$H NMR (400MHz, $CDCl_3$): δ (ppm) 7.81 (s, 1H), 7.72 (d, 1H, J=7.6Hz), 7.53 (d, 1H, J=8.0Hz), 7.44 (t, 1H, J=7.6Hz), 6.26 (s, 1H), 4.63 (s, 2H), 3.48-3.55 (m, 2H), 1.28 (t, 3H, J=7.2Hz).

Step 2

Methyl 3-((3-(ethylcarbamoyl)benzyl)oxy)-5-(methylsulfonyl)isothiazole-4-carboxylate

Methyl 3-hydroxyl-5-(methylsulfonyl)isothiazol-4-carboxylate I009 (888.23 mg, 3.74 mmol) and potassium carbonate (1.03 g, 7.49 mmol) were dissolved in DMF (30 mL), followed by the addition of 3-(chloromethyl)-N-ethylbenzamide I256 (740 mg, 3.74 mmol), and the reaction system was stirred at room temperature to react for 16 hours. Water (100 mL) was added to the reaction mixture, which was then subjected to liquid separation and extraction with ethyl acetate (30 mL x 3), the organic phases were sequentially washed with water and saturated brine, dried with anhydrous sodium sulfate, and concentrated under reduced pressure to obtain a crude product, and the crude product was purified by the flash column chromatography to obtain a yellow solid product methyl 3-((3-(ethylcarbamoyl)benzyl)oxy)-5-(methylsulfonyl)isothiazol-4-carboxylate I257 (680 mg, 1.71 mmol, yield: 45.58%).
The product was verified by LCMS and H-NMR.
$^1$H NMR (400MHz, $CDCl_3$): δ (ppm) 7.93 (s, 1H), 7.73 (d, 1H, J=8.0Hz), 7.58 (d, 1H, J=7.6Hz), 7.46 (t, 1H, J=7.6Hz), 6.29 (s, 1H), 5.53 (s, 2H), 3.99 (s, 3H), 3.49-3.55 (m, 5H), 1.28 (t, 3H, J=7.2Hz).

Step 3

Methyl 5-((2,4-dimethoxybenzyl)amino)-3-((3-(ethylcarbamoyl)benzyl)oxy)isothiazole-4-carboxylate

Methyl 3-((3-(ethylcarbamoyl)benzyl)oxy)-5-(methylsulfonyl)isothiazol-4-carboxylate I257 (300 mg, 752.91 μmol) was dissolved in THF(20 mL), followed by the addition of 2,4-dimethoxybenzylamine (629.45 mg, 3.76 mmol), and the reaction system was stirred at 65 °C to react for 1 hour. After the reaction was finished, the reaction mixture was added to ice water (50 mL), and then extracted with ethyl acetate (10 mL x 3), the organic phases were combined, sequentially washed with water and saturated brine, dried with anhydrous sodium sulfate, and concentrated under reduced pressure to obtain a crude product, and the crude product was purified by the preparative thin-layer chromatography (PE/EA = 1/1) to obtain a white solid product methyl 5-((2,4-dimethoxybenzyl)amino)-3-((3-(ethylcarbamoyl) benzyl)oxy)isothiazol-4-carboxylate
I258 (300 mg, 617.85 μmol, yield: 82.06%).
The product was verified by LCMS and H-NMR.
$^1$H NMR (400 MHz, DMSO-$d_6$) δ 8.42-8.49 (m, 2H), 7.92 (s, 1H), 7.76 (d, 1H, J = 7.6 Hz), 7.56 (d, 1H, J = 7.6 Hz), 7.47 (d, 1H, J = 7.6 Hz), 7.17 (d, 1H, J = 8.4 Hz), 6.60 (d, 1H, J = 2.4 Hz), 6.51 (dd, 1H, J = 8.4, 2.4 Hz), 5.38 (s, 2H), 4.29 (d, 2H, J = 6.2 Hz), 3.83 (s, 3H), 3.75 (d, 6H, J = 0.8Hz), 3.25-3.32 (m 2H),1.12 (t, 3H, J = 7.2 Hz).

Step 4

Methyl 5-amino-3-((3-(ethylcarbamoyl)benzyl)oxy)isothiazole-4-carboxylate

Methyl 5-((2,4-dimethoxybenzyl)amino)-3-((3-(ethylcarbamoyl)benzyl)oxy)isothiazol-4-carboxylate I258 (500.00 mg, 1.03 mmol) was dissolved in DCM/$H_2O$ (30/10 mL), followed by the portionwise addition of DDQ (467.52 mg, 2.06 mmol)at 0°C, and the reaction system was stirred at room temperature to react for 1 hour. After complete reaction, water (20 mL) was added to the reaction mixture, which was then extracted with DCM (10 mL x2), the organic phases were combined, washed with saturated brine, dried with anhydrous sodium sulfate, and concentrated under reduced pressure to obtain a crude product, and the crude product was purified by the preparative thin-layer chromatography (DCM/MeOH =

20/1) to obtain a yellow solid product methyl 5-amino-3-((3-(ethylcarbamoyl)benzyl)oxy)isothiazol-4-carboxylate I259 (170 mg, 506.89 μmol, yield: 49.22%).

The product was verified by LCMS and H-NMR.

$^1$H NMR (400 MHz, DMSO-$d_6$) δ 8.48 (s, 1H), 7.93 (s, 2H), 7.77 (d, 1H, J = 7.6 Hz), 7.57 (d, 2H, J = 7.6 Hz), 7.47 (t, 1H, J = 7.76Hz), 5.38 (s, 2H), 3.74 (s, 3H), 3.63-3.17 (m, 2H), 1.13 (t, 3H, J = 8.0 Hz).

Step 5

Methyl 3-((3-(ethylcarbamoyl)benzyl)oxy)-5-(3-(4-(pyrrolidin-1-yl)butyl)ureido)isothiazole-4-carboxylate

4-(Pyrrolidin-1-yl)butyl-1-amine (100.94 mg, 709.65 μmol) was dissolved in anhydrous THF (15 mL) and then cooled to 0°C under the protection of $N_2$, followed by the addition of CDI (115.07 mg, 709.65 μmol), the mixture was stirred at room temperature to react for 1 hour, followed by the addition of DMSO (15 mL), the pressure was then reduced to remove most of the THF, followed by the addition of methyl 5-amino-3-((3-(ethylcarbamoyl)benzyl)oxy)isothiazol-4-carboxylate I259 (170 mg, 506.89 μmol) and potassium carbonate (70.06 mg, 506.89 μmol), and the reaction system was stirred at room temperature to react for 16 hours. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((3-(ethylcarbamoyl)benzyl)oxy)-5-(3-(4-(pyrrolidin-1-yl)butyl)ureido)isothiazol-4-carboxylate I260 (45 mg, 89.35 μmol, yield: 17.63%). The product was verified by LCMS and H-NMR.

$^1$H NMR (400MHz, $CDCl_3$) δ 10.21 (s, 1H), 8.18 (s, 1H), 7.94 (s, 1H), 7.71 (d, 1H, J = 7.6 Hz), 7.58 (d, 1H, J = 7.6 Hz), 7.44 (t, 1H, J = 7.6 Hz), 6.21 (s, 1H), 5.47 (s, 1H), 3.90 (s, 3H), 3.50-3.56 (m, 2H), 3.32 (s, 2H), 2.61 (s, 4H), 2.54 (d, 2H, J = 6.2 Hz), 2.11 (s, 2H), 1.90 (d, 2H, J = 8.4 Hz), 1.68 (d, 2H, J = 8.4 Hz), 1.28 (t, 3H, J = 7.4 Hz).

Step 6

3-((3-(ethylcarbamoyl)benzyl)oxy)-5-(3-(4-(pyrrolidin-1-yl)butyl)ureido)isothiazole-4-carboxamide

In a microwave tube, methyl 3-((3-(ethylcarbamoyl)benzyl)oxy)-5-(3-(4-(pyrrolidin-1-yl)butyl)ureido)isothiazol-4-carboxylate I260 (29 mg, 57.58 μmol) was dissolved in $NH_3$/MeOH (5 mL, 9 mol/L), and the mixture was sealed and stirred at 60 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(ethylcarbamoyl)benzyl)oxy)-5-(3-(4-(pyrrolidin-1-yl)butyl)ureido)isothiazol-4-formamide T058 (5.7 mg, 11.67 μmol, yield: 20.26%).

The product was verified by LCMS, H-NMR and C-NMR.

$^1$H NMR (400MHz, $CD_3OD$) δ 7.95 (s, 1H), 7.81 (d, 1H, J = 7.6 Hz), 7.67 (d, 1H, J = 7.6 Hz), 7.51 (t, 1H, J = 7.6 Hz), 5.53 (s, 2H), 3.40-3.46 (m, 2H), 3.26 (t, 2H, J = 6.2 Hz), 2.67-2.68 (m, 4H), 2.61 (t, 2H, J = 8.4 Hz),1.84-1.88 (m, 4H), 1.59-1.65 (m, 4H), 1.24 (t, 3H, J = 7.4 Hz).

$^{13}$C NMR (100MHz, $CD_3OD$): δ (ppm) 168.88, 168.24, 165.81, 161.88, 154.74, 136.85, 134.94, 130.97, 129.45, 128.52, 126.80, 69.51, 55.56, 53.61, 39.42, 34.49, 27.35, 25.12, 25.12, 22.73, 13.49.

Example 37: Synthesis of Compound T061

Synthesis Route:

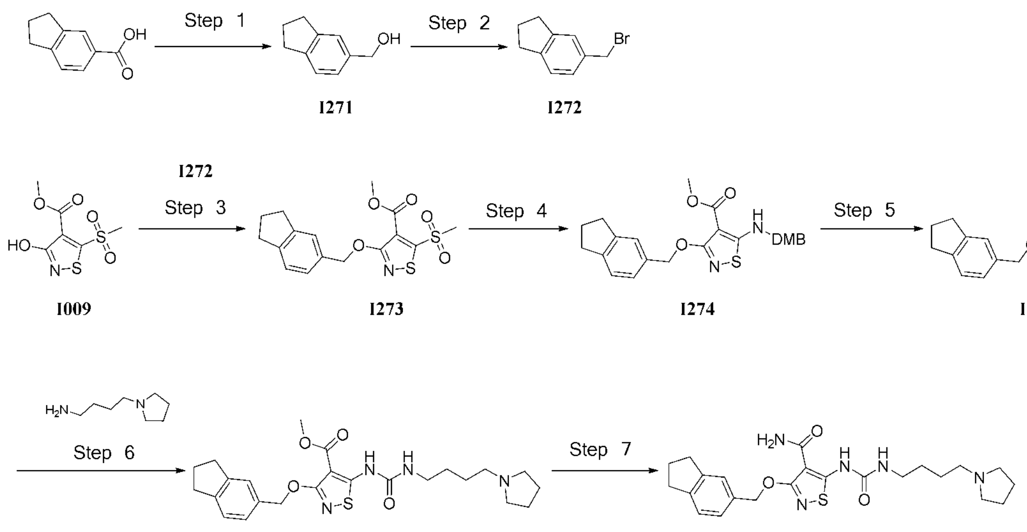


I271 I272

I009 I273 I274 I275

I276 T061

Step 1

(2,3-dihydro-1H-inden-5-yl)methanol

Under the protection of $N_2$, 2,3-dihydro-1H-inden-5-carboxylic acid(2 g, 12.33 mmol) was dissolved in anhydrous THF (20 mL), followed by the addition of in the ice water bath $BH_3.SMe_2$ (10 M, 3.08 mL), and the mixture was warmed to 60°C and stirred to react for 16 hours. The reaction mixture was poured into methanol (10 mL), stirred at 60°C for half an hour, and concentrated under reduced pressure to obtain a crude product, and the crude product was purified by the flash column chromatography (PE/EA = 5/1) to obtain a yellow solid product (2,3-dihydro-1H-inden-5-yl)methanol I271 (1.7 g, 11.47 mmol, yield: 93.02%).

The product was verified by LCMS and H-NMR.

$^1H$ NMR (400MHz, DMSO-$d_6$): δ (ppm) 7.16 (d, J = 8.8 Hz, 2H), 7.06 (d, J = 7.6 Hz, 1H), 5.06 (t, J = 5.6 Hz, 1H), 4.45 (d, J = 5.6 Hz, 2H), 2.89 - 2.77 (m, 4H), 2.04 -1.97 (m, 2H)..

Step 2

5-(bromomethyl)-2,3-dihydro-1H-indene

(2,3-Dihydro-1H-inden-5-yl)methanol I271 (1.0 g, 6.75 mmol) was dissolved in DCM (10 mL) and then cooled to 0°C, followed by the addition of $PBr_3$ (2.74 g, 10.12 mmol), and the reaction system was stirred at room temperature to react for 1 hour. After complete reaction, the mixture was poured into water (30 mL) and extracted with dichloromethane, and the organic phases were combined, washed with saturated brine, dried with anhydrous sodium sulfate, and concentrated under reduced pressure to obtain a crude product 5-(bromomethyl)-2,3-dihydro-1H-indene I272 (1.4 g, 6.63 mmol, yield: 98.29%) as a yellow oily liquid product.

The product was verified by LCMS and H-NMR.

$^1H$ NMR (400MHz, DMSO-$d_6$): δ (ppm) 7.29 (s, 1H), 7.19 (s, 2H), 4.68 (s, 2H), 2.93 - 2.67 (m, 4H), 2.08 - 1.87 (m, 2H).

Step 3

Methyl 3-((2,3-dihydro-1H-inden-5-yl)methoxy)-5-(methylsulfonyl)isothiazole-4-carboxylate

Methyl 3-hydroxyl-5-(methylsulfonyl)isothiazol-4-carboxylate I009 (1.4 g, 5.90 mmol) and potassium carbonate (1.63 g, 11.80 mmol) were dissolved in DMF (20 mL), followed by the addition of 5-(bromomethyl)-2,3-dihydro-1H-indene I272 (1.25 g, 5.90 mmol), and the reaction system was stirred at room temperature to react for 4 hours. Water (100 mL) was added to the reaction mixture, which was then subjected to liquid separation and extraction with ethyl acetate (30 mL x 3), the organic phases were sequentially washed with water and saturated brine, dried with anhydrous sodium sulfate, and concentrated under reduced pressure to obtain a crude product, and the crude product was purified by the flash column chromatography to obtain a white solid product methyl 3-((2,3-dihydro-1H-inden-5-yl)methoxy)-5-(methylsulfonyl)isothiazol-4-carboxylate I273 (1.7 g, 4.63 mmol, yield: 78.41%).

$^1H$ NMR (400MHz, DMSO-$d_6$): δ (ppm) 6.98 (s, 1H), 6.93 - 6.85 (m, 2H), 5.09 (s, 2H), 3.53 (s, 3H), 3.26 (s, 3H), 12.19 - 2.11 (m, 4H), 1.76 - 1.58 (m, 2H).

Step 4

Methyl 3-((2,3-dihydro-1H-inden-5-yl)methoxy)-5-((2,4-dimethoxybenzyl)amino)isothiazole-4-carboxylate

Methyl 3-((2,3-dihydro-1H-inden-5-yl)methoxy)-5-(methylsulfonyl)isothiazol-4-carboxylate I273 (1.7 g, 4.63 mmol) was dissolved in THF(10 mL), followed by the addition of 2,4-dimethoxybenzylamine (7.74 g, 46.27 mmol), and the reaction system was stirred at 60 °C to react for 1 hour. After the reaction was finished, water (50 mL) was added to the reaction mixture, which was then extracted with ethyl acetate (50 mL x 3), the organic phases were combined, sequentially washed with water and saturated brine, dried with anhydrous sodium sulfate, and concentrated under reduced pressure to obtain a crude product, and the crude product was purified by the preparative thin-layer chromatography (PE/EA = 1/1) to obtain a white solid product methyl 3-((2,3-dihydro-1H-inden-5-yl)methoxy)-5-((2,4-dimethoxybenzyl)amino)isothiazol-4-carboxylate I274 (1.8 g, 3.96 mmol, yield: 85.59%).

The product was verified by LCMS and H-NMR.

$^1H$ NMR (400MHz, DMSO-$d_6$): δ (ppm) 8.41 (t, J = 6.0 Hz, 1H), 7.27 (s, 1H), 7.23 - 7.14 (m,, 3H), 6.60 (d, J = 2.4 Hz, 1H), 6.51 (dd, J = 8.4, 2.4 Hz, 1H), 5.28 (s, 2H), 4.28 (d, J = 6.4 Hz, 2H), 3.83 (s, 3H), 3.76 (s, 3H), 3.72 (s, 3H), 2.89 - 2.81 (m, 4H), 2.07 - 1.93 (m, 2H).

Step 5

Methyl 5-amino-3-((2,3-dihydro-1H-inden-5-yl)methoxy)isothiazole-4-carboxylate

Methyl 3-((2,3-dihydro-1H-inden-5-yl)methoxy)-5-((2,4-dimethoxybenzyl)amino)isothiazol-4-carboxylate I274 (670 mg, 1.47 mmol) was dissolved in DCM/$H_2O$ (5/1 mL), followed by the portionwise addition of DDQ (669.21 mg, 2.95 mmol) at 0°C, and the reaction system was stirred at room temperature to react for half an hour. After complete reaction, wate (20 mL)r was added to the reaction mixture, which was then extracted with DCM (30 mL x3), the organic phases were combined, washed with saturated brine, dried with anhydrous sodium sulfate, and concentrated under reduced pressure to obtain a crude product, and the crude product was purified by the preparative thin-layer chromatography (PE/EA = 1/1) to obtain a yellow solid product methyl 5-amino-3-((2,3-dihydro-1H-inden-5-yl)methoxy)isothiazol-4-carboxylate I275 (430 mg, 1.41 mmol, yield: 95.85%).

The product was verified by LCMS and H-NMR.

$^1H$ NMR (400MHz, $CDCl_3$): δ (ppm) 7.33 (s, 1H), 7.22 (s, 2H), 5.40 (s, 2H), 3.84 (s, 3H), 2.95 - 2.87 (m, 4H), 2.15 - 2.01 (m, 4H).

Step 6

Methyl 3-((2,3-dihydro-1H-inden-5-yl)methoxy)-5-(3-(4-(pyrrolidin-1-yl)butyl)ureido)isothiazole-4-carboxylate

4-(Pyrrolidin-1-yl)butyl-1-amine (215.91 mg, 1.52 mmol) was dissolved in anhydrous THF (5 mL) and then cooled to 0°C under the protection of $N_2$, followed by the addition of CDI (244.63 mg, 1.52 mmol), the mixture was stirred at room temperature to react for 1 hour, followed by the addition of DMSO (5 mL), the pressure was then reduced to remove most of the THF, followed by the addition of methyl 5-amino-3-((2,3-dihydro-1H-inden-5-yl)methoxy)isothiazol-4-carboxylate I275 (330 mg, 1.08 mmol) and potassium carbonate (299.69 mg, 2.17 mmol), and the reaction system was stirred at room temperature to react for 2 hours. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((2,3-dihydro-1H-inden-5-yl)methoxy)-5-(3-(4-(pyrrolidin-1-yl)butyl)ureido)isothiazol-4-carboxylate I276 (100 mg, 211.60 μmol, yield: 19.52%).

The product was verified by LCMS and H-NMR.

$^1H$ NMR (400MHz, $CDCl_3$): δ (ppm) 10.36 (s, 1H), 7.35 (s, 1H), 7.25 (s, 2H), 5.43 (s, 2H), 5.37 (s, 1H), 3.89 (s, 3H), 3.51 (s, 2H), 3.42- 3.33 (m, 2H), 2.98 - 2.84 (m, 6H), 2.16 - 2.05 (m, 2H), 1.95 (t, J = 11.2 Hz, 2H), 1.60 - 1.48 (m, 4H), 1.35 (s, 4H).

Step 7

3-((2,3-dihydro-1H-inden-5-yl)methoxy)-5-(3-(4-(pyrrolidin-1-yl)butyl)ureido)isothiazole-4-carboxamide

In a microwave tube, methyl 3-((2,3-dihydro-1H-inden-5-yl)methoxy)-5-(3-(4-(pyrrolidin-1-yl)butyl)ureido)isothiazol-4-carboxylate I276 (100 mg, 211.60 μmol) was dissolved in $NH_3$/MeOH (5 mL, 9 mol/L), and the mixture was sealed and stirred at 40 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((2,3-dihydro-1H-inden-5-yl)methoxy)-5-(3-(4-(pyrrolidin-1-yl)butyl)ureido) isothiazol-4-formamide T061 (17.8 mg, 38.90 μmol, yield: 18.38%). The product was verified by LCMS, H-NMR and C-NMR.

$^1$H NMR (400MHz, $CD_3OD$): δ (ppm) 7.30 (s, 1H), 7.21 (s, 2H), 5.39 (s, 2H), 3.29 - 3.21 (m, 2H), 3.01 (d, J = 12.0 Hz, 2H), 2.93 - 2.85 (m, 4H), 2.40 (s, 3H), 2.25 (t, J = 12.0 Hz, 2H), 2.13 - 1.98 (m, 2H), 1.82 (d, J = 13.2 Hz, 2H), 1.54 - 1.41 (m, 3H), 1.34 - 1.27 (m, 3H).

$^{13}$C NMR (100MHz, $CD_3OD$): δ (ppm) 168.71, 162.20, 154.70, 144.45, 133.93, 126.29, 124.15, 124.01, 70.39, 54.97, 44.35, 37.15, 35.65, 32.11, 30.82, 25.19.

Example 38: Synthesis of Compound T062

Synthesis Route:

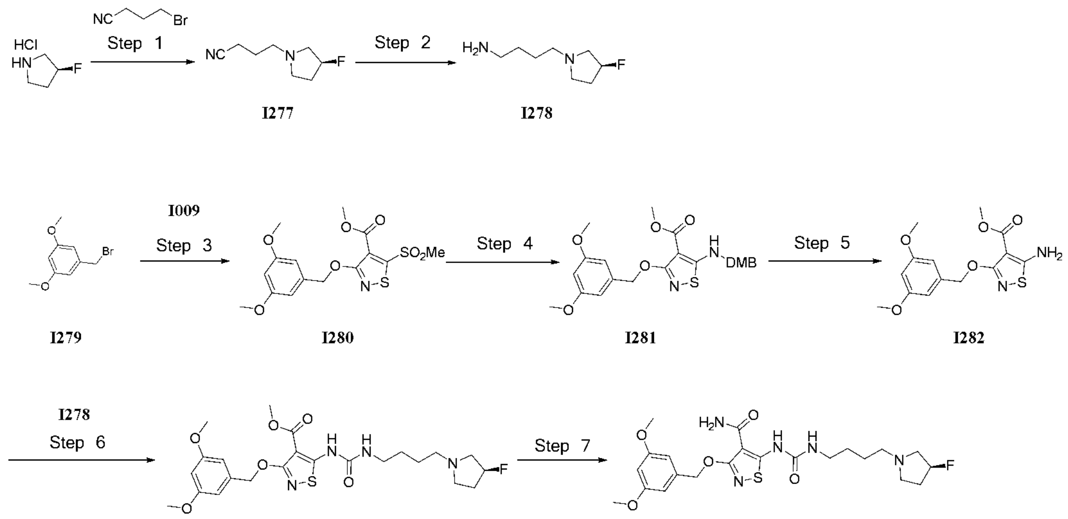


I277 I278 I279 I280 I281 I282 I283 T062

Step 1

(S)-4-(3-fluoropyrrolidin-1-yl)butanenitrile

4-Bromobutyronitrile (5.83 g, 39.42 mmol) was dissolved in acetonitrile (30.0 mL), followed by the addition of (3S)-3-fluoropyrrolidine (788 mg, 9.26 mmol) and potassium carbonate (9.91 g, 71.67 mmol), and the reaction system was stirred at room temperature to react for 16 hours. The reaction mixture was poured into water (50 mL), and extracted with ethyl acetate (30 mL x 3), and the organic phases were combined, washed with saturated brine, dried with anhydrous sodium sulfate, and concentrated under reduced pressure to obtain a crude product, which was then purified by the flash column chromatography to obtain a yellow oily liquid product (S)-4-(3-fluoropyrrolidin-1-yl)butyronitrile I277 (3.3 g, 21.13 mmol, yield: 58.95%).

The product was verified by LCMS and HNMR.

$^1$H NMR (400MHz, $CDCl_3$): δ (ppm) 5.27 - 5.07 (m, 1H), 2.93 - 2.80 (m, 2H), 2.77 - 2.64 (m, 1H), 2.61 (t, J = 6.8 Hz, 2H), 2.50 - 2.43 (m, 2H), 2.44 - 2.38 (m, 1H), 2.26 - 1.96 (m, 2H), 1.85 (p, J = 7.0 Hz, 2H).

Step 2

(S)-4-(3-fluoropyrrolidin-1-yl)butan-1-amine

(S)-4-(3-fluoropyrrolidin-1-yl)butyronitrile I277 (400 mg, 2.56 mmol) was dissolved in methanol (10 mL), followed by the addition of raney nickel (150 mg), with a hydrogen balloon inserted, the reaction system was stirred at 25°C to react for 16 hours. After complete reaction, the reaction mixture was filtered, and the filtrate was concentrated under reduced pressure to obtain a yellow oily liquid crude product (S) -4-(3-fluoropyrrolidin-1-yl)butyl-1-amine I278 (400 mg, 2.50 mmol, yield: 97.48%).

The product was verified by LCMS and H-NMR.

$^1$H NMR (400MHz, $CDCl_3$): δ (ppm) 5.25 - 5.01 (m, 1H), 2.95 - 2.78 (m, 2H), 2.74 - 2.66 (m, 2H), 2.65 - 2.52 (m, 1H), 2.50 - 2.43 (m, 2H), 2.44 - 2.32 (m, 1H), 2.21 - 1.94 (m, 2H), 1.59 - 1.41 (m, 4H).

Step 3

Methyl 3-((3,5-dimethoxybenzyl)oxy)-5-(methylsulfonyl)isothiazole-4-carboxylate

Methyl 3-hydroxyl-5-(methylsulfonyl)isothiazol-4-carboxylate I009 (10 g, 42.15 mmol) and potassium carbonate (11.65 g, 84.30 mmol) were dissolved in DMF (100 mL), followed by the addition of 2,4-dimethoxybenzyl bromide I279 (10.71 g, 46.36 mmol), and the reaction system was stirred at room temperature to react for 16 hours. Water (300 mL) was added to the reaction mixture, which was then subjected to liquid separation and extraction with ethyl acetate (80 mL x 3), the organic phases were sequentially washed with water and saturated brine, dried with anhydrous sodium sulfate, and concentrated under reduced pressure to obtain a crude product, and the crude product was purified by the flash column chromatography to obtain a yellow solid product methyl 3-((3,5-dimethoxybenzyl)oxy)-5-(methylsulfonyl)isothiazol-4-carboxylate I280 (10 g, 25.81 mmol, yield: 61.24%).

The product was verified by LCMS and H-NMR.

$^1$H NMR (400MHz, $CDCl_3$): δ (ppm) 6.62 (d, J=2.4Hz, 2H), 6.44-6.45 (m, 1H), 5.45 (s, 2H), 3.98 (s, 3H), 3.82 (s, 6H), 3.49 (s, 3H).

Step 4

Methyl 5-((2,4-dimethoxybenzyl)amino)-3-((3,5-dimethoxybenzyl)oxy)isothiazole-4-carboxylate

Methyl 3-((3,5-dimethoxybenzyl)oxy)-5-(methylsulfonyl)isothiazol-4-carboxylate I280 (9.2 g, 23.75 mmol) was dissolved in THF(100 mL), followed by the addition of 2,4-dimethoxybenzylamine (23.82 g, 142.48 mmol), and the reaction system was stirred at 60 °C to react for 2 hour. After the reaction was finished, water (200 mL) was added to the reaction mixture, which was then extracted with ethyl acetate (80 mL x 3), the organic phases were combined, sequentially washed with water and saturated brine, dried with anhydrous sodium sulfate, and concentrated under reduced pressure to obtain a crude product, and the crude product was purified by the flash column chromatography (PE/EA = 10/1-3/1) to obtain a white solid product methyl 5-((2,4-dimethoxybenzyl)amino)-3-((3,5-dimethoxybenzyl)oxy)isothiazol-4-carboxylate I281 (9.2 g, 19.39 mmol, yield: 81.65%).

The product was verified by LCMS and H-NMR.

$^1$H NMR (400MHz, $CDCl_3$): δ (ppm) 8.14-8.15 (m, 1H), 7.18 (d, J=8.4Hz, 1H), 6.64 (d, J=2Hz, 2H), 6.40-6.49 (m, 3H), 5.38 (s, 2H), 4.29 (d, J=5.6Hz, 2H), 3.77-3.86 (m, 15H).

Step 5

Methyl 5-amino-3-((3,5-dimethoxybenzyl)oxy)isothiazole-4-carboxylate

Methyl 5-((2,4-dimethoxybenzyl)amino)-3-((3,5-dimethoxybenzyl)oxy)isothiazol-4-carboxylate I281 (2 g, 4.21 mmol) was dissolved in DCM/$H_2O$ (16/3 mL), followed by the portionwise addition of DDQ (1.91 g, 8.43 mmol) at 0°C, and the reaction system was stirred at room temperature to react for half an hour. After complete reaction, water (50 mL) was added to the reaction mixture, which was then extracted with DCM (30 mL x2), the organic phases were combined, washed with saturated brine, dried with anhydrous sodium sulfate, and concentrated under reduced pressure to obtain a crude product, and the crude product was purified by the flash column chromatography (PE/EA = 10/1-3/1) to obtain a white solid product methyl 5-amino-3-((3,5-dimethoxybenzyl)oxy)isothiazol-4-carboxylate I282 (1 g, 3.08 mmol, yield: 73.15%).

The product was verified by LCMS and H-NMR.

$^1$H NMR (400MHz, DMSO-$d_6$): δ (ppm) 7.91 (s, 1H), 6.61 (d, J=1.2Hz, 2H), 6.42 (s, 1H), 5.27 (s, 2H), 3.72-3.74 (m, 6H), 3.36 (s, 3H).

Step 6

Methyl (S)-3-((3,5-dimethoxybenzyl)oxy)-5-(3-(4-(3-fluoropyrrolidin-1-yl)butyl)ureido)isothiazole-4-carboxylate

(S)-4-(3-fluoropyrrolidin-1-yl)butyl-1-amine I278 (331.97 mg, 2.07 mmol) was dissolved in anhydrous THF (5 mL) and then cooled to 0°C under the protection of $N_2$, followed by the addition of CDI (335.94 mg, 2.07 mmol), the mixture was stirred at room temperature to react for 1 hour, followed by the addition of DMSO (5 mL), the pressure was then reduced to remove most of the THF, followed by the addition of methyl 5-amino-3-((3,5-dimethoxybenzyl)oxy)isothiazol-4-carboxylate I282 (480 mg, 1.48 mmol) and potassium carbonate (409.05 mg, 2.96 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow oily liquid product methyl (S)-3-((3,5-dimethoxybenzyl)oxy)-5-(3-(4-(3-fluoropyrrolidin-1-yl)butyl)ureido) isothiazol-4-carboxylate I283 (270 mg, 528.81 μmol, yield: 35.73%).

The product was verified by LCMS and H-NMR.

$^1$H NMR (400MHz, $CDCl_3$): δ (ppm) 10.19 (s, 1H), 7.39 (s, 1H), 6.63 (d, J = 2.0 Hz, 2H), 6.40 (t, J = 2.0 Hz, 1H), 5.40 (s, 2H), 5.34 - 5.12 (m, 1H), 3.87 (s, 2H), 3.80 (s, 5H), 3.43 - 3.22 (m, 2H), 3.05 - 2.86 (m, 2H), 2.62 (s, 5H), 2.59 - 2.43 (m, 3H), 2.28 - 2.12 (m, 2H).

Step 7

(S)-3-((3,5-dimethoxybenzyl)oxy)-5-(3-(4-(3-fluoropyrrolidin-1-yl)butyl)ureido)isothiazole-4-carboxamide

In a microwave tube, methyl (S)-3-((3,5-dimethoxybenzyl)oxy)-5-(3-(4-(3-fluoropyrrolidin-1-yl)butyl)ureido)isothiazol-4-carboxylate I283 (270 mg, 528.81 μmol) was dissolved in $NH_3$/MeOH (5 mL, 9 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product, which was washed with acetonitrile/petroleum ether(v/v = 1/10)), and dried to obtain (S) -3-((3,5-dimethoxybenzyl)oxy)-5-(3-(4-(3-fluoropyrrolidin-1-yl)butyl)ureido)isothiazol-4-formamide T062 (53.2 mg, 107.35 μmol, yield: 20.30%).

The product was verified by LCMS, H-NMR and C-NMR.

$^1$H NMR (400MHz, $CD_3OD$): δ (ppm) 6.62 (d, J = 2.4 Hz, 2H), 5.27 - 5.07 (m, 1H), 5.38 (s, 2H), 5.27 - 5.06 (m, 1H), 3.77 (s, 6H), 3.28 - 3.22 (m, 2H), 3.03 - 2.85 (m, 2H), 2.77 - 2.58 (m, 1H), 2.56- 2.48 (m, 2H), 2.46 - 2.37 (m, 1H), 2.26 - 2.10 (m, 1H), 2.09 - 1.89 (m, 1H), 1.63 - 1.53 (m, 4H).

$^{13}$C NMR (100MHz, $CD_3OD$): δ (ppm) 168.79, 165.87, 161.99, 161.16, 154.71, 138.41, 105.53, 99.77, 93.72, 91.98, 69.94, 60.31, 60.08, 54.39, 51.97, 39.48, 32.10, 31.87, 27.34, 25.24.

Example 39: Synthesis of Compound T063

Synthesis Route:

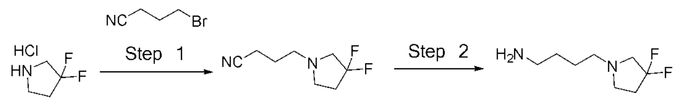


I284 I285

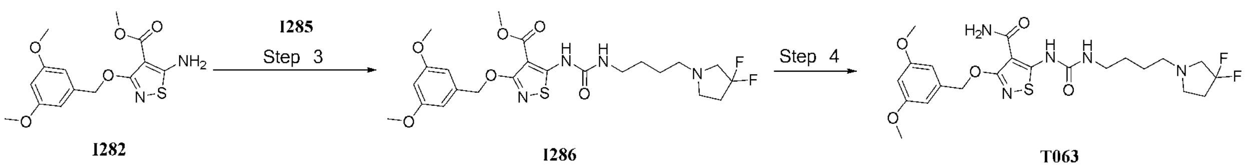


I282 I286 T063

Step 1

4-(3,3-difluoropyrrolidin-1-yl)butanenitrile

Tetrabromobutyronitrile (1 g, 6.17 mmol) was dissolved in acetonitrile (10.0 mL), followed by the addition of 3,3-difluoropyrrolidine · hydrochloride (1 g, 6.97 mmol) and potassium carbonate (2.41 g, 17.41 mmol), and the reaction system was stirred at room temperature to react for 16 hours. The reaction mixture was poured into water (20 mL), and extracted with ethyl acetate (20 mL x 3), the organic phases were combined, washed with saturated brine, dried with anhydrous sodium sulfate, and concentrated under reduced pressure to obtain a crude product, and the crude product was purified by the flash column chromatography (PE/EA = 1011-511) to obtain a yellow oily liquid product 4-(3,3-difluoropyrrolidin-1-yl)butyronitrile I284 (450 mg, 2.58 mmol, yield: 37.09%).

The product was verified by LCMS and HNMR.

$^1$H NMR (400MHz, $CDCl_3$): δ (ppm) 2.90 (t, J = 13.2 Hz, 2H), 2.76 (t, J = 6.8Hz, 2H), 2.61 (t, J = 6.8Hz, 2H), 2.47 (t, J = 7.2 Hz, 2H), 2.37 - 2.18 (m, 2H), 1.80-1.87 (m, 2H).

Step 2

4-(3,3-difluoropyrrolidin-1-yl)butan-1-amine

4-(3,3-Difluoropyrrolidin-1-yl)butyronitrile I284 (500 mg, 2.87 mmol) was dissolved in methanol (15 mL), followed by the addition of raney nickel (100 mg), and in the atmosphere of hydrogen at 25psi, the reaction system was stirred at 25°C to react for 5 hours. After complete reaction, the reaction mixture was filtered, and the filtrate was concentrated under reduced pressure to obtain a yellow oily liquid crude product 4-(3,3-difluoropyrrolidin-1-yl)butyl-1-amine I285 (420 mg, 2.36 mmol, yield: 82.10%).

The product was verified by LCMS and H-NMR.

$^1$H NMR (400MHz, $CDCl_3$): δ (ppm) 3.03 (s, 2H), 2.87-2.94 (m, 2H), 2.70-2.77 (m, 4H), 2.42-2.54 (m, 2H), 2.33-2.34 (m, 2H), 1.45-1.64 (m, 4H).

Step 3

Methyl 5-(3-(4-(3,3-difluoropyrrolidin-1-yl)butyl)ureido)-3-((3,5-dimethoxybenzyl)oxy)isothiazole-4-carboxylate

4-(3,3-Difluoropyrrolidin-1-yl)butyl-1-amine I285 (153.85 mg, 863.26 μmol) was dissolved in anhydrous THF (15 mL) and then cooled to 0°C under the protection of $N_2$, followed by the addition of CDI (139.98 mg, 863.26 μmol), the mixture was stirred at room temperature to react for 1 hour, followed by the addition of DMSO (15 mL), the pressure was then reduced to remove most of the THF, followed by the addition of methyl 5-amino-3-((3,5-dimethoxybenzyl)oxy) isothiazol-4-carboxylate I282 (200 mg, 616.61 μmol) and potassium carbonate (85.22 mg, 616.61 μmol), and the reaction system was stirred at room temperature to react for 1 hour. After complete reaction, water (50 mL) was added to the reaction mixture, which was then extracted with ethyl acetate (30 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 15/1) to obtain a white solid product methyl 5-(3-(4-(3,3-difluoropyrrolidin-1-yl)butyl)ureido)-3-((3,5-dimethoxybenzyl)oxy)isothiazol-4-carboxylate I286 (135 mg, 255.41 μmol, yield: 41.42%).

The product was verified by LCMS and H-NMR.

$^1$H NMR (400MHz, $CDCl_3$): δ (ppm) 10.32 (s, 1H), 6.65 (d, J = 2.4 Hz, 2H), 6.42 (t, J = 2.4 Hz, 1H), 5.31 (s, 1H),5.40 (s, 2H), 3.90 (s, 2H), 3.82 (s, 6H), 3.33-3.38 (m, 2H), 2.92 (t, J = 13.2 Hz, 2H), 2.78 (t, J = 6.8 Hz, 2H), 2.53 (t, J = 6.4 Hz, 2H), 2.22 - 2.45 (m, 2H), 1.58-1.69 (m, 2H).

Step 4

5-(3-(4-(3,3-difluoropyrrolidin-1-yl)butyl)ureido)-3-((3,5-dimethoxybenzyl)oxy)isothiazole-4-carboxamide

In a microwave tube, methyl 5-(3-(4-(3,3-difluoropyrrolidin-1-yl)butyl)ureido)-3-((3,5-dimethoxybenzyl)oxy)isothiazol-4-carboxylate I286 (100 mg, 189.19 μmol) was dissolved in $NH_3$/MeOH (10 mL, 9 mol/L), and the mixture was sealed to react at 40 °C for 16 hours. The reaction mixture was concentrated under reduced pressure to obtain a crude product, and the crude product was purified by the high-performance preparative chromatography to obtain a white solid product 5-(3-(4-(3,3-difluoropyrrolidin-1-yl)butyl)ureido)-3-((3,5-dimethoxybenzyl)oxy)isothiazol-4-formamide T063 (25.2 mg, 49.07 μmol, yield: 25.94%).

The product was verified by LCMS, H-NMR and C-NMR.

$^1$H NMR (400MHz, $CD_3OD$): δ (ppm) 6.61 (d, J = 2.4 Hz, 2H), 6.45 (t, J = 2.4 Hz, 1H), 5.37 (s, 2H), 3.77 (s, 6H), 3.25 (t, J = 6.4 Hz, 2H), 2.90 (t, J = 13.2 Hz, 2H), 2.75 (t, J = 6.8 Hz, 2H), 2.49 (t, J = 6.8 Hz, 2H), 2.22-2.30 (m, 2H), 1.56 - 1.58 (m, 4H).

$^{13}$C NMR (100MHz, $CD_3OD$): δ (ppm) 168.76, 165.88, 161.99, 161.14, 154.69, 138.39, 132.16, 129.70, 127.25, 105.53, 99.75, 97.39, 69.95, 61.74, 61.45, 61.15, 55.22, 54.42, 51.91, 51.87, 51.83, 39.50, 35.38, 35.14, 34.90, 27.18, 24.78.

Example 40: Synthesis of Compound T064

Synthesis Route:

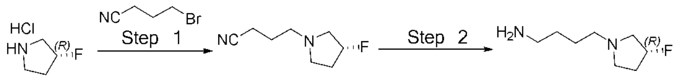


I287 I288

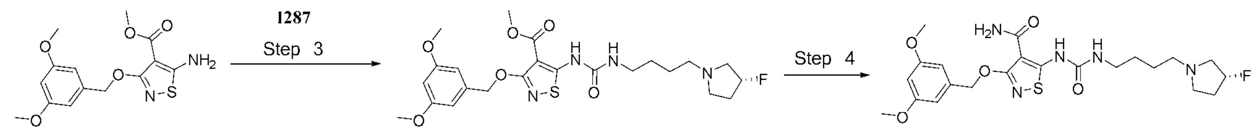


I282 I289 T064

Step 1

(R)-4-(3-fluoropyrrolidin-1-yl)butanenitrile

4-bromobutyronitrile (3.54 g, 23.89 mmol) was dissolved in acetonitrile (30.0 mL), followed by the addition of (3R)-3-fluoropyrrolidine · hydrochloride (3 g, 23.89 mmol) and potassium carbonate (9.91 g, 71.67 mmol), and the reaction system was stirred at room temperature to react for 16 hours. The reaction mixture was poured into water (50 mL), and extracted with ethyl acetate (30 mL x 3), the organic phases were combined, washed with saturated brine, dried with anhydrous sodium sulfate, and concentrated under reduced pressure to obtain a crude product, and the crude product was purified by the flash column chromatography (DCM/MeOH = 200/1-100/1) to obtain a yellow oily liquid product (R) -4-(3-fluoropyrrolidin-1-yl)butyronitrile I287 (2.25 g, 14.40 mmol, yield: 60.29%).

The product was verified by LCMS and HNMR.

$^1$H NMR (400MHz, $CDCl_3$): δ (ppm) 5.08-5.22 (m, 1H), 2.82-2.91 (m, 2H), 2.39-2.76 (m, 6H), 2.03-2.21 (m, 2H), 1.81-2.01 (m, 2H).

Step 2

(R)-4-(3-fluoropyrrolidin-1-yl)butan-1-amine

(R)-4-(3-fluoropyrrolidin-1-yl)butyronitrile I287 (0.5 g, 3.20 mmol) was dissolved in methanol (10 mL), followed by the addition of raney nickel (180 mg), and in the atmosphere of hydrogen, the reaction system was stirred at 25°C to react for 4 hours. After complete reaction, the reaction mixture was filtered, and the filtrate was concentrated under reduced pressure to obtain a yellow oily crude product (R) -4-(3-fluoropyrrolidin-1-yl)butyl-1-amine I288 (0.32 g, 2.00 mmol, yield: 62.39%).

The product was verified by LCMS and H-NMR.

$^1$H NMR (400MHz, $CDCl_3$): δ (ppm) 5.07-5.24 (m, 1H), 2.80-2.91 (m, 2H), 2.69-2.73 (m, 2H), 2.59-2.65 (m, 1H), 2.40-2.49 (m, 2H), 2.36-2.40 (m, 1H), 1.97-2.20 (m, 2H), 1.40-1.58 (m, 6H).

Step 3

Methyl (R)-3-((3,5-dimethoxybenzyl)oxy)-5-(3-(4-(3-fluoropyrrolidin-1-yl)butyl)ureido)isothiazole-4-carboxylate

(R)-4-(3-fluoropyrrolidin-1-yl)butyl-1-amine I288 (165.99 mg, 1.04 mmol) was dissolved in anhydrous THF (5 mL) and then cooled to 0°C under the protection of $N_2$, followed by the addition of CDI (167.97 mg, 1.04 mmol), the mixture was stirred at room temperature to react for 1 hour, followed by the addition of DMSO (5 mL), the pressure was then reduced to remove most of the THF, followed by the addition of methyl 5-amino-3-((3,5-dimethoxybenzyl)oxy)isothiazol-4-carboxylate I282 (0.24 g, 739.94 μmol) and potassium carbonate (204.53 mg, 1.48 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 15/1) to obtain a white solid product methyl (R)-3-((3,5-dimethoxybenzyl)oxy)-5-(3-(4-(3-fluoropyrrolidin-1-yl)butyl)ureido)isothiazol-4-carboxylate I289 (0.2 g, 391.71 μmol, yield: 52.94%).

The product was verified by LCMS and H-NMR.

$^1$H NMR (400MHz, $CDCl_3$): δ (ppm) 10.24 (s, 1H), 7.45 (s, 1H), 6.64 (d, J=2Hz, 2H), 6.40-6.41 (t, J=4.8Hz, 1H), 5.39 (s, 2H), 3.88 (s, 3H), 3.81 (s, 6H), 3.27-3.36 (m, 2H), 2.89-3.01 (m, 2H), 2.65-2.70 (m, 4H), 2.51-2.62 (m, 2H), 2.42-2.48 (m, 1H), 2.04-2.24 (m, 2H), 1.63-1.72 (m, 2H).

Step 4

(R)-3-((3,5-dimethoxybenzyl)oxy)-5-(3-(4-(3-fluoropyrrolidin-1-yl)butyl)ureido)isothiazole-4-carboxamide

In a microwave tube, methyl (R)-3-((3,5-dimethoxybenzyl)oxy)-5-(3-(4-(3-fluoropyrrolidin-1-yl)butyl)ureido)isothiazol-4-carboxylate I289 (0.2 g, 391.71 μmol) was dissolved in $NH_3$/MeOH (6 mL, 9 mol/L), and the mixture was sealed and stirred at 40 °C to react for 16 hours. The reaction mixture was concentrated under reduced pressure to obtain a crude product, and the crude product was purified by the preparative thin-layer chromatography (DCM/MeOH = 15/1 to obtain a white solid product (R) -3-((3,5-dimethoxybenzyl)oxy)-5-(3-(4-(3-fluoropyrrolidin-1-yl)butyl)ureido)isothiazol-4-formamide T064 (47 mg, 94.84 μmol, yield: 24.21%).

The product was verified by LCMS, H-NMR and C-NMR.

$^1$H NMR (400MHz, $CD_3OD$): δ (ppm) 6.63 (d, J=2.4Hz, 2H), 6.46-6.47 (t, J=4.4Hz, 1H), 5.39 (s, 2H), 5.09-5.26 (m, 1H), 3.79 (s, 6H), 3.24-3.28 (t, J=12.8Hz, 2H), 2.89-3.01 (m, 2H), 2.40-2.72 (m, 4H), 1.97-2.24 (m, 2H), 1.59-1.60 (m, 4H).

$^{13}$C NMR (100MHz, $CD_3OD$): δ (ppm) 168.78, 165.88, 161.99, 161.15, 154.68, 138.40, 105.54, 99.75, 97.38, 93.77, 92.03, 69.96, 60.33, 60.09, 55.63, 54.43, 51.98, 39.53, 32.12, 31.91, 27.36, 25.29.

Example 41: Synthesis of Compound T065

Synthesis Route:

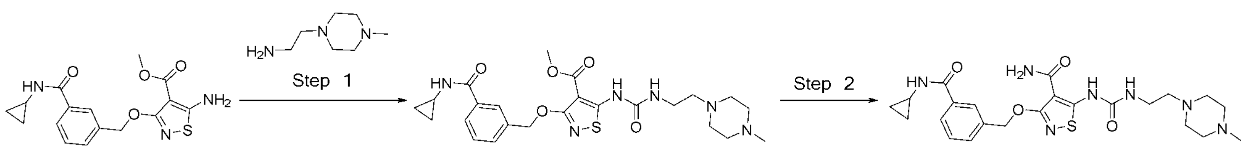


I249 I290 T065

Step 1

Methyl 3-((3-(cyclopropylcarbamoyl)benzyl)oxy)-5-(3-(2-(4-methylpiperazin-1-yl)ethyl) ureido) isothia zole-4-carboxylate

2-(4-Methylpiperazin-1-yl)ethylamine (115.4 mg, 806.01 μmol) was dissolved in anhydrous THF (5 mL) and then cooled to 0°C under the protection of $N_2$, followed by the addition of CDI (130.7 mg, 806.01 μmol), the mixture was stirred at room temperature to react for 1 hour, followed by the addition of DMSO (5 mL), the pressure was then reduced to remove most of the THF, followed by the addition of methyl 5-amino-3-((3-(cyclopropylcarbamoyl)benzyl)oxy)isothiazol-4-carboxylate I249 (200 mg, 575.72 μmol) and potassium carbonate (159.1 mg, 1.15 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((3-(cyclopropylcarbamoyl)benzyl)oxy)-5-(3-(2-(4-methylpiperazin-1-yl)ethyl)ureido)isothiazol-4-carboxylate I290 (163 mg, 315.52 μmol, yield: 54.80%).

The product was verified by LCMS and H-NMR.

$^1H$ NMR (400MHz, $CDCl_3$): δ (ppm) 10.35 (s, 1H), 7.94 (s, 1H), 7.67 (d, J=8.0 Hz, 1H), 7.59 (d, J=7.6 Hz, 1H), 7.45 (t, J=7.6 Hz, 1H), 6.28(s, 1H), 5.85 (s, 1H), 5.49 (s, 2H), 3.96 (s, 3H), 3.46-3.42 (m, 2H), 2.96-2.91 (m, 1H), 2.59-2.51 (m, 8H), 2.34 (s, 3H), 0.94-0.86 (m, 2H), 0.67-0.63 (m, 2H).

Step 2

3-((3-(cyclopropylcarbamoyl)benzyl)oxy)-5-(3-(2-(4-methylpiperazin-1-yl)ethyl)ureido) isothiazole-4-carboxamide

In a microwave tube, methyl 3-((3-(cyclopropylcarbamoyl)benzyl)oxy)-5-(3-(2-(4-methylpiperazin-1-yl)ethyl) ureido)isothiazol-4-carboxylate I290 (160 mg, 309.71 μmol) was dissolved in $NH_3$/MeOH (9 mL, 9 mol/L), and the mixture was sealed and stirred at 40 °C to react for 10 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(cyclopropylcarbamoyl)benzyl)oxy)-5-(3-(2-(4-methylpiperazin-1-yl)ethyl)ureido)isothiazol-4-formamide T065 (25.4 mg, 50.64 μmol, yield: 16.35%).

The product was verified by LCMS, H-NMR and C-NMR.

$^1H$ NMR (400MHz, $CDCl_3$): δ (ppm) 7.93 (s, 1H), 7.79 (d, J=7.6 Hz, 1H), 7.67 (d, J=7.6 Hz, 1H), 7.50 (t, J=7.6 Hz, 1H), 5.53 (s, 2H), 3.39 (t, J=6.4 Hz, 2H), 3.34-3.21 (m, 3H), 2.87 (t, J=4.0 Hz, 1H), 2.57-2.54 (m, 7H), 2.34 (s, 3H), 0.84-0.80 (m, 2H), 0.68-0.65 (m, 2H).

$^{13}C$ NMR (100 MHz, $CDCl_3$) δ: 170.02, 168.86, 161.87, 154.74, 13684, 134.62, 131.08, 128.50, 126.85, 97.44, 69.48, 56.90, 54.23, 52.05, 46.97, 36.83, 22.65, 5.15.

Example 42: Synthesis of Compound T066

Synthesis Route:

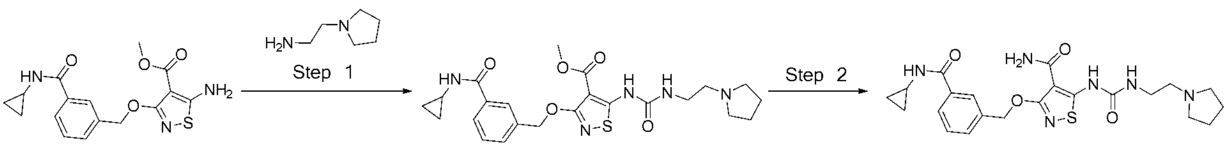


I249 I291 T066

Step 1

Methyl 3-((3-(cyclopropylcarbamoyl)benzyl)oxy)-5-(3-(2-(pyrrolidin-1-yl)ethyl)ureido) iso thiazole-4-carboxylate

2-Pyrrolidin-1-ethylamine (92. mg, 806.01 μmmol) was dissolved in anhydrous THF (5 mL) and then cooled to 0°C under the protection of $N_2$, followed by the addition of CDI (130.7 mg, 806.01 μmol), the mixture was stirred at room temperature to react for 1 hour, followed by the addition of DMSO (5 mL), the pressure was then reduced to remove most of the THF, followed by the addition of methyl 5-amino-3-((3-(cyclopropylcarbamoyl)benzyl)oxy)isothiazol-4-carboxylate I249 (200 mg, 575.72 μmol) and potassium carbonate (159.1 mg, 1.15 mmol), and the reaction system was stirred at room

temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((3-(cyclopropylcarbamoyl)benzyl)oxy)-5-(3-(2-(pyrrolidin-1-yl)ethyl)ureido)isothiazol-4-carboxylate I291 (160 mg, 328.16 μmol, yield: 57.0%). The product was verified by LCMS and H-NMR.

$^1H$ NMR (400MHz, $CDCl_3$): δ (ppm) 10.42 (s, 1H), 7.93 (s, 1H), 7.68 (d, J=8.0 Hz, 1H), 7.58 (d, J=7.6 Hz, 1H), 7.44 (t, J=7.6 Hz, 1H), 6.29 (s, 1H), 6.08 (s, 1H), 5.48 (s, 2H), 3.95 (s, 3H), 3.47-3.43 (m, 2H), 2.95-2.93 (m, 1H), 2.70 (t, J=5.6 Hz, 2H), 2.58 (s, 4H), 1.84 (s, 4H), 0.92-0.89 (m, 2H), 0.67-0.63 (m, 2H).

Step 2

3-((3-(cyclopropylcarbamoyl)benzyl)oxy)-5-(3-(2-(pyrrolidin-1-yl)ethyl)ureido)isothiazole-4-carboxamide

In a microwave tube, methyl 3-((3-(cyclopropylcarbamoyl)benzyl)oxy)-5-(3-(2-(pyrrolidin-1-yl)ethyl)ureido)isothiazol-4-carboxylate I291 (160 mg, 328.16 μmol) was dissolved in $NH_3$/MeOH (9 mL, 9 mol/L), and the mixture was sealed and stirred at 40 °C to react for 10 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(cyclopropylcarbamoyl)benzyl)oxy)-5-(3-(2-(pyrrolidin-1-yl)ethyl)ureido) isothiazol-4-formamide T066 (24 mg, 50.79 μmol, yield: 15.48%). The product was verified by LCMS, H-NMR and C-NMR.

$^1H$ NMR (400MHz, $CDCl_3$): δ (ppm) 11.15 (s, 1H), 8.02-7.89 (m, 3H), 7.58 (d, J=7.6 Hz, 1H), 7.49 (t, J=7.6 Hz, 1H), 7.10 (s, 1H), 5.45-5.40 (m, 3H), 3.51-3.40 (m, 2H), 2.97-2.92 (m, 1H), 2.57-2.51 (m, 2H), 8.02-7.89 (m, 3H), 2.37-2.26 (m, 4H), 1.56 (s, 4H), 0.85 (s, 2H), 0.67 (s, 2H). $^{13}C$ NMR (100 MHz, $CDCl_3$) δ: 168.84, 168.26, 166.33, 161.62, 154.97, 135.91, 135.08, 131.75, 129.98, 126.91, 97.17, 70.43, 55.16, 53.49, 38.81, 23.26, 23.10.

Example 43: Synthesis of Compound T067

Synthesis Route:

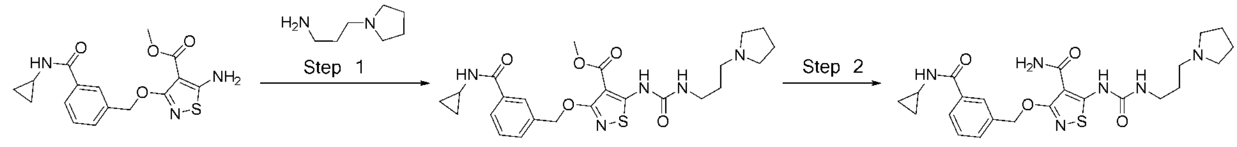


I249 I292 T067

Step 1

Methyl 3-((3-(cyclopropyl carbamoyl) benzyl) oxy)-5-(3-(3-(pyrrolidin-1-yl) propyl) ureido) isothia zole-4-carboxylate

3-(Pyrrolidin-1-yl)propyl-1-amine (129.2 mg, 1.01 mmol) was dissolved in anhydrous THF (10 mL) and then cooled to 0°C under the protection of $N_2$, followed by the addition of CDI (163.4 mg, 1.01 mmol), the mixture was stirred at room temperature to react for 1 hour, followed by the addition of DMSO (10 mL), the pressure was then reduced to remove most of the THF, followed by the addition of methyl 5-amino-3-((3-(cyclopropylcarbamoyl)benzyl)oxy)isothiazol-4-carboxylate I249 (250 mg, 0.72 mmol) and potassium carbonate (198.9 mg, 1.44 mmol), and the reaction system was stirred at room temperature to react for 1 hour. After complete reaction, water (50 mL) was added to the reaction mixture, which was then extracted with ethyl acetate (30 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow oily product methyl 3-((3-(cyclopropylcarbamoyl)benzyl)oxy)-5-(3-(3-(pyrrolidin-1-yl)propyl)ureido)isothiazol-4-carboxylate I292 (180 mg, 0.35 mmol, yield: 49.86%).

The product was verified by LCMS and H-NMR.

$^1H$ NMR (400MHz, $CDCl_3$): δ (ppm) 7.92 (s, 1H), 7.72-7.68 (m, 2H), 7.59-7.58 (m, 1H), 7.46-7.42 (m, 1H), 7.14 (s, 1H), 5.48 (s, 2H), 3.94 (s, 3H), 3.52-3.47 (m, 2H), 2.93-2.94 (m, 1H), 2.86-2.79 (m, 4H), 1.97-1.84 (m, 4H), 1.80-1.72 (m, 4H), 0.91-0.83 (m, 2H), 0.67-0.65 (m, 2H).

Step 2

3-((3-(cyclopropylcarbamoyl)benzyl)oxy)-5-(3-(3-(pyrrolidin-1-yl)propyl)ureido) isothiazo le-4-carboxamide

In a microwave tube, methyl 3-((3-(cyclopropylcarbamoyl)benzyl)oxy)-5-(3-(3-(pyrrolidin-1-yl)propyl)ureido) isothiazol-4-carboxylate I292 (180 mg, 358.85 μmol) was dissolved in $NH_3$/MeOH (5 mL, 9 mol/L), and the mixture was sealed and stirred at 40 °C to react for 10 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(cyclopropylcarbamoyl)benzyl)oxy)-5-(3-(3-(pyrrolidin-1-yl) propyl)ureido)isothiazol- 4-formamide T067 (21 mg, 43.16 μmol, yield: 12.03%). The product was verified by LCMS, H-NMR and C-NMR.
$^1$H NMR (400MHz, $CDCl_3$): δ (ppm) 10.94 (s, 1H), 8.03 (s, 1H), 7.86 (s, 1H), 7.75 (d, J=7.6 Hz, 1H), 7.59 (d, J=7.6 Hz, 1H), 7.47 (t, J=7.6 Hz, 1H), 7.21 (s, 1H), 7.09 (s, 1H), 6.37 (s, 1H), 5.49 (s, 2H), 3.41-3.39 (m, 2H), 2.95-2.91 (m, 1H), 2.61 (t, J=6.0 Hz, 2H), 2.50 (s, 4H), 1.79 (s, 6H), 0.93-0.88 (m, 2H), 0.67-0.63 (m, 2H).
$^{13}$C NMR (100 MHz, $CDCl_3$) δ: 169.60, 168.34, 166.53, 161.63, 154.15, 136.51, 135.05, 131.45, 129.04, 127.06, 127.00, 69.93, 53.72, 26.98, 23.28, 23.21, 6.82.

Example 44: Synthesis of Compound T068

Synthesis Route:

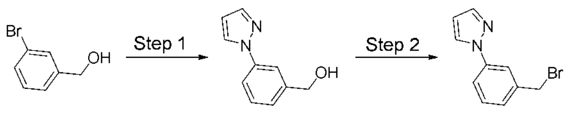


I293 I294

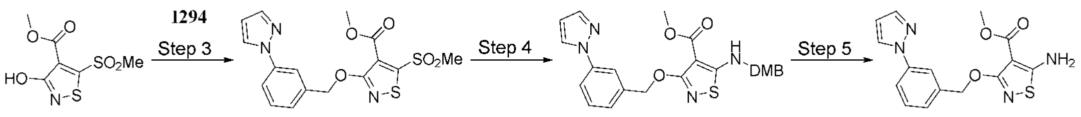


I009 I295 I296 I297

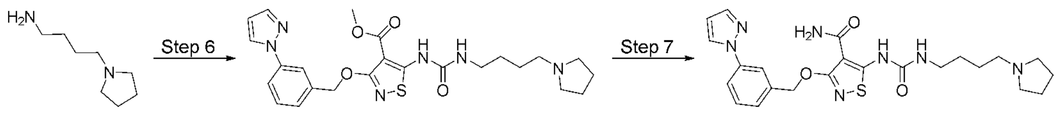


I298 T068

Step 1

(3-(1H-pyrazol-1-yl) phenyl) methanol

(3-Bromophenyl)methanol (2 g, 10.69 mmol) was dissolved in NMP (20 mL), followed by the addition of 1*H*-pyrazole (800.76 mg, 11.76 mmol), potassium carbonate (4.43 g, 32.08 mmol) and cuprous iodide (407.31 mg, 2.14 mmol). In the atmosphere of nitrogen, the reaction system was stirred at 100 °C to react for 16 hours. The reaction mixture was poured into water (100 mL), and extracted with ethyl acetate (50 mL x 3), and the organic phases were combined, washed with saturated brine (100 mL), dried with anhydrous sodium sulfate, and concentrated under reduced pressure to obtain a crude product. The crude product was purified by the silica-gel column chromatography (PE/EA = 30/1-5/1) to obtain a yellow solid product 3-(1H-pyrazolyl)phenyl)methanol I293 (1 g, 5.74 mmol, yield: 53.68%).
The product was verified by LCMS and H-NMR.
$^1$H NMR (400MHz, $CDCl_3$): δ (ppm) 7.92 (d, J=2.0Hz, 1H), 7.69-7.70 (m, 2H), 7.55-7.58 (m, 2H), 7.35-7.41 (m, 1H), 7.25-7.26 (t, J=7.6Hz, 1H), 6.44-6.45 (t, J= 4Hz, 1H), 4.73 (s, 2H).

Step 2

1-(3-(bromomethyl) phenyl)-1H-pyrazole

3-(1H-pyrazolyl)phenyl)methanol I293 (1 g, 5.74 mmol) was dissolved in DCM (20 mL), followed by the addition of $PBr_3$(2.33 g, 8.61 mmol), and the reaction system was stirred at 25 °C to react for 1.0 hour. The reaction mixture was poured into water (25 mL), and extracted with DCM (20 mL x 3), and the organic phases were combined, washed with saturated brine (20 mL), dried with anhydrous sodium sulfate, and concentrated under reduced pressure to obtain a yellow oily product 1-(3-(bromomethyl)phenyl)-1H-pyrazole I294 (1.2 g, 5.06 mmol, yield: 88.17%).
The product was verified by LCMS.

Step 3

Methyl 3-((3-(1H-pyrazol-1-yl) benzyl) oxy)-5-(methylsulfonyl) isothiazole-4-carboxylate

Methyl 3-hydroxyl-5-(methylsulfonyl)isothiazol-4-carboxylate I009 (1.1 g, 4.64 mmol) was dissolved in DMF(15 mL), followed by the addition of 1-(3-(bromomethyl)phenyl)-1H-pyrazole I294 (1.21 g, 5.10 mmol) and potassium carbonate (1.28 g, 9.27 mmol), and the reaction system was stirred at 25 °C to react for 4.0 hours. The reaction mixture was poured into water (80 mL), and extracted with ethyl acetate (30 mL x 3), and the organic phases were combined, washed with saturated brine (80 mL), dried with anhydrous sodium sulfate, and concentrated under reduced pressure to obtain a crude product. The crude product was purified by the silica-gel column chromatography (PE/EA = 30/1-3/1) to obtain a white solid product 3-((3-(1H-pyrazol-1-yl)benzyl)oxy)-5-(methylsulfonyl)isothiazol-4-methyl formate I295 (0.6 g, 1.53 mmol, yield: 32.89%).
The product was verified by LCMS and H-NMR.
$^1$H NMR (400MHz, $CDCl_3$): δ (ppm) 7.97-7.98 (m, 1H), 7.90 (s, 1H), 7.75 (d, J =1.6Hz,1H), 7.65-7.68 (m, 1H), 7.46-7.51 (m, 1H), 7.39 (d, J=8Hz, 1H), 6.50-6.52 (m, 1H), 5.57 (s, 2H), 4.00 (s, 3H), 3.50 (s, 3H).

Step 4

Methyl 3-((3-(1H-pyrazol-1-yl) benzyl) oxy)-5-((2,4-dimethylbenzyl) amino) isothiazole-4-carboxylate

3-((3-(1H-pyrazol-1-yl)benzyl)oxy)-5-(methylsulfonyl)isothiazol-4-methyl formate I295 (0.27 g, 734.90 μmol) was dissolved in THF(10 mL), followed by the addition of 2,4-dimethoxybenzylamine (614.39 mg, 3.67 mmol), and the reaction system was stirred at 60 °C to react for 4.0 hours. The mixture was poured into cold water (10 mL), and extracted with ethyl acetate (10 mL x 3), and the organic phases were combined, washed with saturated brine, dried with anhydrous sodium sulfate, and concentrated under reduced pressure to obtain a crude product. The crude product was purified by the preparative thin-layer chromatography (PE/EA = 3/1) to obtain a white solid product methyl 3-((3-(1H-pyrazol-1-yl)benzyl) oxy)-5-((2,4-dimethylbenzyl)amino)isothiazol-4-carboxylate I296 (0.19 g, 418.05μmol, yield: 56.88%).
The product was verified by LCMS and H-NMR.
$^1$H NMR (400MHz, DMSO-$d_6$): δ (ppm) 8.49 (d, J=2.4Hz, 1H), 8.44 (s, 1H), 7.97 (s, 1H), 7.76-7.77 (m, 2H), 7.50 (t, J=16Hz, 1H), 7.35 (d, J=8.0Hz, 1H), 7.17 (d, J=8.4Hz, 1H), 6.59 (d, J=2.0Hz, 1H), 6.56 (t, J=2.4Hz, 1H), 6.49-6.52 (m, 1H), 5.49 (s, 2H), 4.29 (d, J=2.0Hz, 2H), 3.83 (s, 3H), 3.76 (d, J=8.4Hz, 6H).

Step 5

Methyl 3-((3-(1H-pyrazol-1-yl) benzyl) oxy)-5-aminoisothiazole-4-carboxylate

Methyl 3-((3-(1H-pyrazol-1-yl)benzyl)oxy)-5-((2,4-dimethylbenzyl)amino)isothiazol-4-carboxylate I296 (500.00 mg, 1.04 mmol) was dissolved in DCM (12 mL) and water (4 mL), followed by the addition of DDQ (472.39 mg, 2.08 mmol), and the reaction system was stirred at 25 °C to react for 1.0 hour. The reaction mixture was poured into water (100 mL), and extracted with DCM (100 mL x 3), the organic phases were combined, washed with saturated brine (200 mL), dried with anhydrous sodium sulfate, and concentrated under reduced pressure to obtain a crude product, and the crude product was purified by the preparative thin-layer chromatography (PE/EA = 3/1) to obtain a white solid product methyl 3-((3-(1H-pyrazol-1-yl)benzyl)oxy)-5-aminoisothiazol-4-carboxylate I297 (210 mg, 635.67 μmol, yield: 61.09%).
The product was verified by LCMS.

Step 6

Methyl 3-((3-(1H-pyrazol-1-yl) benzyl) oxy)-5-(3-(4-(pyrrolidin-1-yl) butyl) ureido) isothiazole-4-carboxylate

4-(Pyrrolidin-1-yl)butan-1-amine (126.59 mg, 889.94 μmol) was dissolved in anhydrous THF (5 mL), followed by the addition of CDI (144.30 mg, 889.94 μmol) under the protection of $N_2$, the mixture was stirred at 25°C to react for 1.0 hour, followed by the addition of DMSO (5 mL), the pressure was then reduced to remove THF, followed by the addition of methyl 3-((3-(1H-pyrazol-1-yl)benzyl)oxy)-5-aminoisothiazol-4-carboxylate I297 (210 mg, 635.67 μmol) and potassium carbonate (175.71 mg, 1.27 mmol), and the reaction system was stirred at 25 °C to react for 1.0 hour. Water (10 mL) was poured into the reaction mixture, which was then subjected to liquid separation and extraction with DCM (10 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-((3-(1H-pyrazol-1-yl)benzyl) oxy)-5-(3-(4-(pyrrolidin-1-yl)butyl)ureido)isothiazol-4-carboxylate I298 (130 mg, 260.73 μmol, yield: 41.02%).

The product was verified by LCMS and H-NMR.

$^1$H NMR (400MHz, $CDCl_3$): δ (ppm) 10.27 (s, 1H), 8.11 (s, 1H), 7.97 (d, J = 2.5 Hz, 1H), 7.93 (s, 1H), 7.64-7.75 (m, 3H), 7.45 (t, J = 7.9 Hz, 1H), 7.38 (d, J = 7.7 Hz, 1H), 7.13 (s, 1H), 6.51 - 6.48 (m, 1H), 5.52 (s, 2H), 3.93 (s, 3H), 3.35 (s, 2H), 2.75 (s, 4H), 2.65 (s, 1H), 1.96 (s, 4H), 1.28 (s, 4H).

Step 7

3-((3-(1H-pyrazol-1-yl)benzyl)oxy)-5-(3-(4-(pyrrolidin-1-yl)butyl)ureido) isothiazole-4-carboxamide

Methyl 3-((3-(1H-pyrazol-1-yl)benzyl)oxy)-5-(3-(4-(pyrrolidin-1-yl)butyl)ureido)isothiazol-4-carboxylate I298 (130 mg, 260.73 μmol) was dissolved in $NH_3$/MeOH (10 mL, 10 mol/L). The mixture was sealed to react at 40 °C for 16 hours. The reaction mixture was concentrated under reduced pressure to obtain a crude product, and the crude product was purified by the high-performance liquid chromatography to obtain a white solid product 3-((3-(1H-pyrazol-1-yl)benzyl) oxy)-5-(3-(4-(pyrrolidin-1-yl)butyl)ureido)isothiazol-4-formamide T068 (13.1 mg, 27.09 μmol, yield: 10.39%).

The product was verified by LCMS, H-NMR and C-NMR.

$^1$H NMR (400MHz, $CDCl_3$): δ (ppm) 10.92 (s, 1H), 7.94 (d, J = 2.4 Hz, 1H), 7.82 (s, 1H), 7.73 (s, 1H), 7.71 (d, J = 8.4 Hz, 1H), 7.51 (t, J = 8.0 Hz, 1H), 7.39 (d, J = 7.6 Hz, 1H), 7.13 (s, 1H), 6.48 (s, 1H), 5.52 (s, 2H), 5.39 (s, 1H), 3.31 (s, 2H), 2.52-2.58 (m, 6H), 1.87 (s, 4H), 1.65 (s, 4H).

$^{13}$C NMR (100 MHz, $CDCl_3$) δ 169.67, 165.86, 161.70, 154.08, 141.32, 140.51, 137.62, 129.88, 126.82, 126.26, 119.12, 107.90, 69.90, 55.82, 53.88, 28.00, 26.55, 23.37.

Example 45: Synthesis of Compound T069

Synthesis Route:

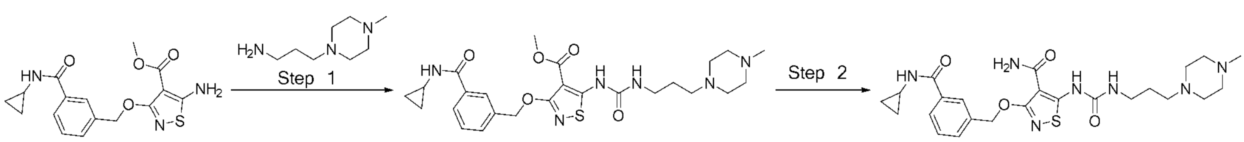


I249 I299 T069

Step 1

Methyl3-((3-(cyclopropylcarbamoyl) benzyl) oxy)-5-(3-(3-(4-methylpiperazin-1-yl) propyl) ureido) isothiazole-4-carboxylate

3-(4-Methylpiperazin-1-yl)propyl-1-amine (126.8 mg, 0.8 mmol) was dissolved in anhydrous THF (5 mL) and then cooled to 0°C under the protection of $N_2$, followed by the addition of CDI (130.7 mg, 0.8 mmol), the mixture was stirred at room temperature to react for 1 hour, followed by the addition of DMSO (5 mL), the pressure was then reduced to remove most of the THF, followed by the addition of methyl 5-amino-3-((3-(cyclopropylcarbamoyl)benzyl)oxy)isothiazol-4-carboxylate I249 (200 mg, 0.6 mmol) and potassium carbonate (159.1 mg, 1.15 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and

saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((3-(cyclopropylcarbamoyl)benzyl)oxy)-5-(3-(3-(4-methylpiperazin-1-yl)propyl)ureido)isothiazol-4-carboxylate I299 (160 mg, 301.52 μmol, yield: 52.37%).

The product was verified by LCMS and H-NMR.

$^1H$ NMR (400MHz, DMSO-d6 ): δ (ppm) 10.38 (s, 1H), 8.46 (d, *J* = 4.0 Hz, 1H), 8.13 (t, *J* = 5.3 Hz, 1H), 7.92 (s, 1H), 7.74 (d, *J* = 7.7 Hz, 1H), 7.57 (d, *J* = 7.8 Hz, 1H), 7.45 (t, *J* = 7.7 Hz, 1H), 5.39 (s, 2H), 3.83 (s, 3H), 3.15 (dd, *J* = 12.2, 6.3 Hz, 2H), 2.84 (d, *J* = 7.3, 3.7 Hz, 1H), 2.49 - 2.48 (m, 2H), 2.45 - 2.20 (m, 8H), 2.13 (s, 3H), 1.66 - 1.52 (m, 2H), 0.73 - 0.63 (m, 2H), 0.60 - 0.53 (m, 2H).

Step 2

3-((3-(cyclopropylcarbamoyl) benzyl) oxy)-5-(3-(3-(4-methylpiperazin-1-yl) propyl) ureido) isothiazole-4-carboxamide

In a microwave tube, methyl 3-((3-(cyclopropylcarbamoyl)benzyl)oxy)-5-(3-(3-(4-methylpiperazin-1-yl)propyl) ureido)isothiazol-4-carboxylate I299 (220 mg, 0.4 mmol) was dissolved in $NH_3$/MeOH (9 mL, 9 mol/L), and the mixture was sealed and stirred at 40 °C to react for 10 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(cyclopropylcarbamoyl)benzyl)oxy)-5-(3-(3-(4-methylpiperazin-1-yl)propyl)ureido)isothiazol-4-formamide T069 (26.3 mg, 51.01 μmol, yield: 12.30%).

The product was verified by LCMS, H-NMR and C-NMR.

$^1H$ NMR (400MHz, $CDCl_3$): δ (ppm) 11.08 (s, 1H), 7.84 (s, 1H), 7.71-6.86 (m, *J* = 7.8 Hz, 1H), 7.58-7.56 (m, *J* = 7.7 Hz, 2H), 7.47-7.43 (m, *J* = 7.7 Hz, 1H), 7.08 (s, 1H), 6.25 (s, 1H), 5.49 (s, 3H), 3.42 (m, *J*= 10.8, 5.3 Hz, 2H), 2.97 - 2.85 (m,1H), 2.81 - 2.34 (m, 3H), 2.33 (s, 9H), 1.78 - 1.69 (m, 3H), 0.88 (t, *J* = 6.2 Hz, 2H), 0.63 (d, *J* = 2.4 Hz, 2H).

$^{13}C$ NMR (100MHz, $CDCl_3$) δ (ppm) 169.52, 168.53, 165.83, 161.67, 153.90, 136.52, 135.06, 131.39, 128.98, 127.09, 126.93, 69.88, 56.72, 55.04, 53.00, 45.88, 25.04, 23.22, 6.77

Example 46: Synthesis of Compound T070

Synthesis Route:

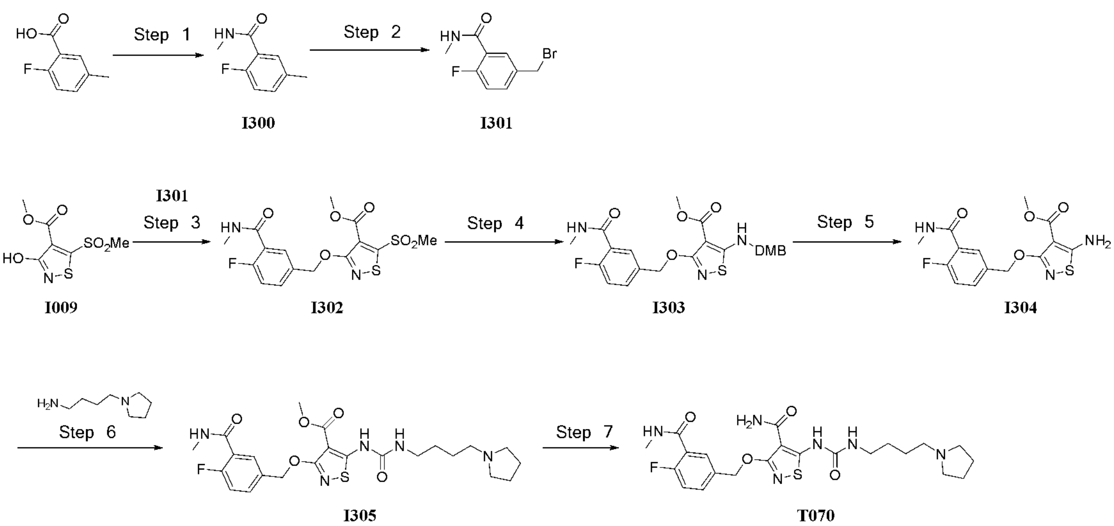


I300 I301 I009 I301 I302 I303 I304 I305 T070

Step 1

2-fluoro-N,5-dimethylbenzamide

2-Fluoro-5-methylbenzoic acid (200 mg, 1.06 mmol) was dissolved in DCM (5 mL) and then cooled to 0°C, followed by the addition of methylamine hydrochloride (2.19 g, 32.44 mmol, CL), EDCI (9.33 g, 48.66 mmol), HOBT (6.58 g, 48.66 mmol) and triethylamine (9.85 g, 97.32mmol), and the reaction system was stirred at room temperature to react for 3

hours. After complete reaction, the reaction mixture was poured into water (300 mL), and extracted with ethyl acetate (50 mL x 3), the organic phases were combined, washed with water and saturated brine, dried with anhydrous sodium sulfate, and concentrated under reduced pressure to obtain a crude product, and the crude product was purified by the flash column chromatography (PE/EA = 10/1-5/1) to obtain a white solid 2-fluoro-N, 5-dimethylbenzamide I300 (2.6 g, 15.55 mmol, yield: 47.94%).

The product was verified by LCMS and H-NMR.

$^1$H NMR (400 MHz, $CDCl_3$) δ 7.87 (dd, J = 7.6, 2.0 Hz, 1H), 7.20-7.24 (m, 1H), 6.95-7.00 (m, 1H), 6.77 (s, 1H) 3.02 (dd, J = 3.6, 1.2 Hz, 3H), 2.35 (s, 3H).

Step 2

5-(bromomethyl)-2-fluoro-N-methylbenzamide

2-Fluoro-N, 5-dimethylbenzamide I300 (2 g, 11.96 mmol) was dissolved in acetonitrile (30 mL), followed by the addition of AIBN (392.89 mg, 2.39 mmol) and NBS (2.77 g, 15.55 mmol) at room temperature, and the mixture was stirred at 70°C to react for 16 hours. After complete reaction, the mixture was poured into water (10 mL), and extracted with ethyl acetate, the organic phases were combined, washed with water and saturated brine, dried with anhydrous sodium sulfate, and concentrated under reduced pressure to obtain a crude product, and the crude product was purified by the flash column chromatography (PE/EA = 10/1-3/1) to obtain a yellow solid product 5-(bromomethyl)-2-fluoro-N-methylbenzamide I301 (1.3 g, 5.28 mmol, yield: 44.16%).

The product was verified by LCMS and H-NMR.

$^1$H NMR (400 MHz, $CDCl_3$) δ 8.14 (dd, J = 7.2, 2.4 Hz, 1H), 7.50 - 7.54 (m, 1H), 7.10-7.15 (m, 1H), 6.75 (s, 1H), 4.51 (s, 2H), 3.06 (dd, J = 4.8, 1.2 Hz, 3H).

Step 3

Methyl 3-((4-fluoro-3-(methylcarbamoyl)benzyl)oxy)-5-(methylsulfonyl)isothiazole-4-carboxylate

Methyl 3-hydroxyl-5-(methylsulfonyl)isothiazol-4-carboxylate I009 (1.50 g, 6.34 mmol) and potassium carbonate (1.46 g, 10.57 mmol) were dissolved in DMF (20 mL), followed by the addition of 5-(bromomethyl)-2-fluoro-N-methylbenzamide I301 (1.3 g, 5.28 mmol), and the reaction system was stirred at room temperature to react for 1 hour. After complete reaction, water (10 mL) was added to the reaction mixture, which was then subjected to liquid separation and extraction with ethyl acetate (10 mLx3), and the organic phases were sequentially washed with water and saturated brine, dried with anhydrous sodium sulfate, and concentrated under reduced pressure to obtain a crude product methyl 3-((4-fluoro-3-(methylcarbamoyl)benzyl)oxy)-5-(methylsulfonyl)isothiazol-4-carboxylate I302 as a yellow solid product.

The product was verified by LCMS and H-NMR.

$^1$H NMR (400MHz, $CDCl_3$): δ (ppm) 8.25 (dd, J = 7.6, 2.4 Hz, 1H), 7.57-7.59 (m, 1H), 7.14-7.19 (m, 1H), 6.75 (s, 1H), 5.50 (s, 2H), 4.00 (s, 3H), 3.50 (s, 3H), 3.06-3.07 (m, 3H).

Step 4

Methyl 5-((2,4-dimethoxybenzyl)amino)-3-((4-fluoro-3-(methylcarbamoyl)benzyl)oxy)isothiazole-4-carboxylate

Methyl 3-((4-fluoro-3-(methylcarbamoyl)benzyl)oxy)-5-(methylsulfonyl)isothiazol-4-carboxylate I302 (1.5 g, 3.73 mmol) was dissolved in THF(30 mL), followed by the addition of 2,4-dimethoxybenzylamine (2.49 g, 14.91 mmol), and the reaction system was stirred at 65 °C to react for 1 hour. After the reaction was finished, the reaction mixture was added to water (30 mL), and extracted with ethyl acetate (20 mL x 3), and the organic phases were combined, sequentially washed with water and saturated brine, dried with anhydrous sodium sulfate, and concentrated under reduced pressure to obtain a white solid product methyl 5-((2,4-dimethoxybenzyl)amino)-3-((4-fluoro-3-(methylcarbamoyl)benzyl)oxy)isothiazol-4-carboxylate I303 (1.5 g, 3.06 mmol, yield: 82.21%).

The product was verified by LCMS and H-NMR.

$^1$HNMR (400 MHz, $CDCl_3$) δ 8.27-8.31 (m, 1H), 8.27 - 8.03 (m, 1H), 7.55-7.59 (m, 1H), 7.01-7.19 (m, 2H), 6.76 (s, 1H), 6.45-6.49 (m, 2H), 5.42 (s, 2H), 4.28 (d, J = 6.0 Hz, 2H), 3.79-3.91 (m, 9H), 3.06 (d, J = 4.0 Hz, 3H).

Step 5

Methyl 5-amino-3-((4-fluoro-3-(methylcarbamoyl)benzyl)oxy)isothiazole-4-carboxylate

Methyl 5-((2,4-dimethoxybenzyl)amino)-3-((4-fluoro-3-(methylcarbamoyl)benzyl)oxy)isothiazol-4-carboxylate I303 (1 g, 2.04 mmol) was dissolved in DCM/$H_2O$ (20/4 mL), followed by the portionwise addition of DDQ (463.73 mg, 2.04 mmol) at 0°C, and the reaction system was stirred at room temperature to react for 0.5 hours. After complete reaction, water (10 mL) was added to the reaction mixture, which was then extracted with DCM (10 mL x2), the organic phases were combined, washed with saturated brine, dried with anhydrous sodium sulfate, and concentrated under reduced pressure to obtain a crude product, and the crude product was purified by the flash column chromatography (PE/EA = 5/1-EA) to obtain a yellow solid product methyl 5-amino-3-((4-fluoro-3-(methylcarbamoyl)benzyl)oxy)isothiazol-4-carboxylate I304 (260 mg, 766.19 μmol, yield: 37.51%).
The product was verified by LCMS and H-NMR.
$^1H$ NMR (400 MHz, $CDCl_3$) δ 8.27 (dd, J = 7.6, 3.6 Hz, 1H), 7.51-7.65 (m, 1H), 7.12-7.17 (m, 1H), 6.76 (s, 1H), 6.47 (s, 2H), 5.45 (s, 2H), 3.92 (s, 3H), 3.07 (d, J = 4.0 Hz, 3H).

Step 6

Methyl 3-((4-fluoro-3-(methylcarbamoyl)benzyl)oxy)-5-(3-(4-(pyrrolidin-1-yl)butyl)ureido)isothiazole-4-carboxylate

4-(Pyrrolidin-1-yl)butyl-1-amine (176.05 mg, 1.24 mmol) was dissolved in anhydrous THF (5 mL) and then cooled to 0°C under the protection of $N_2$, followed by the addition of CDI (200.69 mg, 1.24 mmol), the mixture was stirred at room temperature to react for 1 hour, followed by the addition of DMSO (5 mL), the pressure was then reduced to remove most of the THF, followed by the addition of methyl 5-amino-3-((4-fluoro-3-(methylcarbamoyl)benzyl)oxy)isothiazol-4-carboxylate I304 (300 mg, 884.07 μmol) and potassium carbonate (244.37 mg, 1.77 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((4-fluoro-3-(methylcarbamoyl)benzyl)oxy)-5-(3-(4-(pyrrolidin-1-yl)butyl)ureido) isothiazol-4-carboxylate
I305 (130 mg, 256.12 μmol, yield: 28.97%).
The product was verified by LCMS and H-NMR.
$^1H$ NMR (400 MHz, $CDCl_3$) δ 10.23 (s, 1H), 8.29 (dd, J = 7.2, 2.0 Hz, 1H), 7.55-7.59 (m, 1H), 7.14 (dd, J = 12, 4.8 Hz, 1H), 6.76 (s, 1H), 5.45 (s, 2H), 3.95 (s, 3H), 3.34 (s, 2H), 3.07 (dd, J = 4.8, 1.2 Hz, 3H), 2.56-2.65 (m, 6H), 1.92 (s, 4H), 1.69 (d, J = 7.6 Hz, 4H).

Step 7

3-((4-fluoro-3-(methylcarbamoyl)benzyl)oxy)-5-(3-(4-(pyrrolidin-1-yl)butyl)ureido)isothiazole-4-carboxamide

In a microwave tube, methyl 3-((4-fluoro-3-(methylcarbamoyl)benzyl)oxy)-5-(3-(4-(pyrrolidin-1-yl)butyl)ureido) isothiazol-4-carboxylate I305 (70 mg, 137.91 μmol) was dissolved in $NH_3$/MeOH (3 mL, 9 mol/L), and the mixture was sealed to react at 40 °C for 16 hours. The reaction mixture was concentrated under reduced pressure to obtain a crude product, and the crude product was purified by the high-performance preparative chromatography to obtain a white solid product 3-((4-fluoro-3-(methylcarbamoyl)benzyl)oxy)-5-(3-(4-(pyrrolidin-1-yl)butyl)ureido)isothiazol-4-formamide T070 (15 mg, 30.45 μmol, yield: 22.08%).
The product was verified by LCMS, H-NMR and C-NMR.
$^1H$ NMR (400 MHz, $CD_3OD$) δ 7.87 (d, J = 5.2 Hz, 1H), 7.66 (t, J = 4.8 Hz, 1H), 7.23 (t, J = 10.0 Hz, 1H), 5.48 (s, 2H), 3.24 (s, 2H), 2.94 (s, 3H), 2.57 (s, 4H), 2.51 (t, J = 7.6 Hz, 2H), 1.81 (s, 4H), 1.58 (s, 4H).
$^{13}C$ NMR (100 MHz, $CD_3OD$) δ 168.91, 165.79, 165.48, 165.47, 161.72, 160.99, 158.50, 154.71,133.08, 133.05, 132.83, 132.74, 130.27, 130.24, 122.90, 122.76, 116.24, 116.01, 97.35, 68.66, 55.73, 53.58, 39.53, 27.46, 25.55, 25.43, 22.75.

Example 47: Synthesis of Compound T071

Synthesis Route:

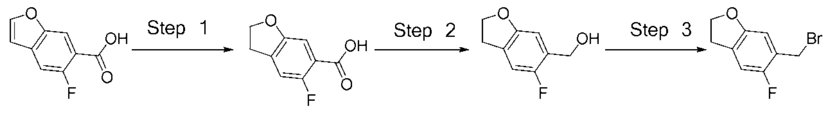


I306 I307 I308

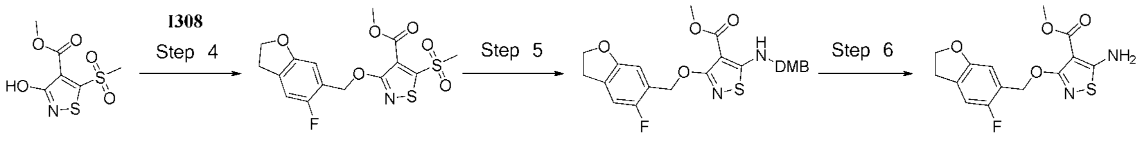


I009 I309 I310 I311

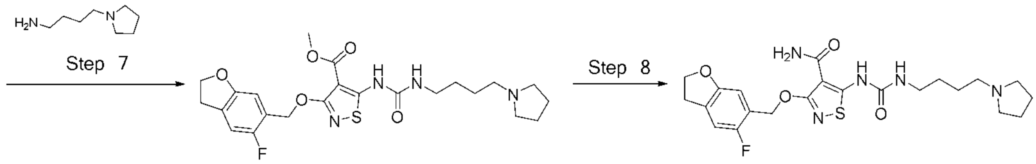


I312 T071

Step 1

5-fluoro-2,3-dihydrobenzofuran-6-carboxylic acid

5-Fluorobenzofuran-6-carboxylic acid (2 g, 11.10 mmol) in MeOH (50 mL) was added to Pd/C (899 mg) at room temperature, and then stirred at room temperature in the atmosphere of hydrogen (20 psi) to react for 48 hours. After complete reaction, the mixture was filtered with diatomite, and the filtrate was concentrated under reduced pressure to obtain a crude product 5-fluoro-2,3-dihydrobenzofuran-6-carboxylic acid I306 (1.8 g, 9.88 mmol, yield: 89.00%) as a white solid product.

The product was verified by LCMS and H-NMR.

$^1$H NMR (400MHz, DMSO-$d_6$): δ (ppm) 7.19 (d, J=10Hz, 1H), 7.08 (d, J=5.6Hz, 1H), 4.55-4.59 (m, 2H), 3.17-3.26 (m, 2H).

Step 2

(5-fluoro-2,3-dihydrobenzofuran-6-yl) methanol

Under the protection of $N_2$, 5-fluoro-2,3-dihydrobenzofuran-6-carboxylic acid I306 (1.8 g, 9.88 mmol) was dissolved in anhydrous THF (20 mL), followed by the addition of $BH_3.SMe_2$ (10 M, 1.5 mL) in the ice water bath, and the mixture was warmed to 60°C and stirred to react for 6 hours. After complete reaction, methanol (50 mL) was poured into the reaction mixture, which was then stirred at 60°C for half an hour and concentrated under reduced pressure to obtain a crude product (5-fluoro-2,3-dihydrobenzofuran-6-yl)methanol I307 (1.5 g, 8.92 mmol, yield: 90.26%) as a yellow solid product.

The product was verified by LCMS and H-NMR.

$^1$H NMR (400MHz, $CDCl_3$): δ (ppm) 6.92 (d, J=9.2Hz, 1H), 6.82 (d, J=5.6Hz, 1H), 4.70 (s, 2H), 4.60 (t, J=8.4Hz, 2H), 3.22 (t, J=8.8Hz, 2H).

Step 3

6-(bromomethyl)-5-fluoro-2,3-dihydrobenzofuran

(5-Fluoro-2,3-dihydrobenzofuran-6-yl)methanol I307 (1 g, 5.95 mmol) was dissolved in DCM (20 mL) and then cooled to 0°C, followed by the addition of $PBr_3$ (3.2 g, 11.89 mmol), and the reaction system was stirred at room temperature to react for 1 hour. After complete reaction, the mixture was poured into water (30 mL) and extracted with dichloromethane, and the organic phases were combined, washed with saturated brine, dried with anhydrous sodium sulfate, and concentrated under reduced pressure to obtain a yellow oily liquid crude product 6-(bromomethyl)-5-fluoro-2,3-dihydrobenzofuran I308 (1.2 g, 5.19 mmol, yield: 87.34%).

The product was verified by LCMS and H-NMR.

$^1$H NMR (400MHz, $CDCl_3$): δ (ppm) 6.92 (d, J=9.2Hz, 1H), 6.77 (d, J=6.0Hz, 1H), 4.60 (t, J=8.8Hz, 2H), 4.48 (s, 2H), 3.22 (t, J=8.4Hz, 2H).

Step 4

Methyl 3-((5-fluoro-2,3-dihydrobenzofuran-6-yl) methoxy)-5-(methylsulfonyl) isothiazole-4-carboxylate

Methyl 3-hydroxyl-5-(methylsulfonyl)isothiazol-4-carboxylate I009 (1 g, 4.21 mmol) and potassium carbonate (1.8 g, 12.64 mmol) were dissolved in DMF (10 mL), followed by the addition of 6-(bromomethyl)-5-fluoro-2,3-dihydrobenzofuran I308 (1.2 g, 5.06 mmol), and the reaction system was stirred at room temperature to react for 1 hour. After complete reaction, water (30 mL) was added to the reaction mixture, which was filtered to obtain a filter cake, and the filter cake was dissolved in ethyl acetate (50 mL), dried with anhydrous sodium sulfate, and concentrated under reduced pressure to obtain a yellow solid product methyl 3-((5-fluoro-2,3-dihydrobenzofuran-6-yl)methoxy)-5-(methylsulfonyl) isothiazol-4-carboxylate I309 (1.5 g, 3.87 mmol, yield: 91.86%).

The product was verified by LCMS and H-NMR.

$^1$H NMR (400MHz, $CDCl_3$): δ (ppm) 6.95 (d, J=9.2Hz, 1H), 6.90 (d, J=5.6Hz, 1H), 5.50 (s, 2H), 4.63-4.58 (m, 2H), 3.97 (s, 3H), 3.50 (s, 3H), 3.24 (t, J=8.8Hz, 2H).

Step 5

Methyl 5-((2,4-dimethylbenzyl) amino)-3-((5-fluoro-2,3-dihydrobenzofuran-6-yl) methoxy) isothiazole-4-carboxylate

Methyl 3-((5-fluoro-2,3-dihydrobenzofuran-6-yl)methoxy)-5-(methylsulfonyl)isothiazol-4-carboxylate I309 (1.5 g, 3.87 mmol) was dissolved in THF(20 mL), followed by the addition of 2,4-dimethoxybenzylamine (6.5 g, 38.72 mmol), and the reaction system was stirred at 60 °C to react for 2 hours. After the reaction was finished, the reaction mixture was added to water (50 mL) and then filtered, the filter cake was dissolved in ethyl acetate, and the mixture was dried with anhydrous sodium sulfate, and concentrated under reduced pressure to obtain a white solid product methyl 5-((2,4-dimethylbenzyl)amino)-3-((5-fluoro-2,3-dihydrobenzofuran-6-yl)methoxy)isothiazol-4-carboxylate I310 (1.7 g, 3.58 mmol, yield: 92.53%).

The product was verified by LCMS and H-NMR.

$^1$H NMR (400MHz, $CDCl_3$): δ (ppm) 8.15 (s, 1H), 7.18 (d, J=8.4Hz, 1H), 6.98 (d, J=5.6Hz, 1H), 6.91 (d, J=9.2Hz, 1H), 6.50-6.45 (m, 1H), 5.43 (s, 2H), 4.58 (t, J=8.8Hz, 2H), 4.29 (d, J=5.6Hz, 1H), 3.86 (s, 3H), 3.84 (s, 3H), 3.82 (s, 3H), 3.21 (t, J=8.8Hz, 2H).

Step 6

Methyl 5-amino-3-((5-fluoro-2,3-dihydrobenzofuran-6-yl) methoxy) isothiazole-4-carboxylate

Methyl 5-((2,4-dimethylbenzyl)amino)-3-((5-fluoro-2,3-dihydrobenzofuran-6-yl)methoxy)isothiazol-4-carboxylate 1310 (1 g, 2.11 mmol) was dissolved in DCM/$H_2O$ (20/4 mL), followed by the portionwise addition of DDQ (956.8 mg, 4.21 mmol) at 0°C, and the reaction system was stirred at room temperature to react for 2 hours. After complete reaction, water (100 mL) was added to the reaction mixture, which was then extracted with DCM (100 mL x2), the organic phases were combined, washed with saturated brine, dried with anhydrous sodium sulfate, and concentrated under reduced pressure to obtain a crude product, and the crude product was purified by chromatographic columns (neutral alumina, PE/EA = 10/1-5/1) to obtain a white solid product methyl 5-amino-3-((5-fluoro-2,3-dihydrobenzofuran-6-yl) methoxy)isothiazol-4-carboxylate I311 (400 mg, 1.23 mmol, yield: 58.52%).

The product was verified by LCMS and H-NMR.

$^1$H NMR (400 MHz, $CDCl_3$) δ 6.95 (dd, J = 22.4, 7.4 Hz, 2H), 6.47 (s, 2H), 5.43 (d, J = 12.0 Hz, 2H), 4.60 (t, J = 8.7 Hz, 2H), 3.88 (s, 3H), 3.22 (t, J = 8.6 Hz, 2H).

Step 7

Methyl 3-((5-fluoro-2,3-dihydrobenzofuran-6-yl) methoxy)-5-(3-(4-(pyrrolidin-1-yl) butyl) ureido) isothiazole-4-carboxylate

4-(Pyrrolidin-1-yl)butyl-1-amine (184.2 mg, 1.29 mmol) was dissolved in anhydrous THF (5 mL) and then cooled to 0°C under the protection of $N_2$, followed by the addition of CDI (186.4 mg, 1.29 mmol), the mixture was stirred at room

temperature to react for 1 hour, followed by the addition of DMSO (5 mL), the pressure was then reduced to remove most of the THF, followed by the addition of methyl 5-amino-3-((5-fluoro-2,3-dihydrobenzofuran-6-yl)methoxy)isothiazol-4-carboxylate I311 (300 mg, 0.9 mmol) and potassium carbonate (383.5 mg, 2.77 mmol), and the reaction system was stirred at room temperature to react for 1 hour. After complete reaction, water (30 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((5-fluoro-2,3-dihydrobenzofuran-6-yl)methoxy)-5-(3-(4-(pyrrolidin-1-yl)butyl)ureido)isothiazol-4-carboxylate I312 (200 mg, 406.04 μmol, yield: 43.90%).

The product was verified by LCMS and H-NMR.

$^1$H NMR (400 MHz, $CDCl_3$) δ 10.27 (s, 1H), 8.20 (s, 1H), 7.14 (s, 2H), 6.95 (dd, J = 22.3, 7.1 Hz, 2H), 5.45 (s, 2H), 4.59 (t, J = 8.6 Hz, 2H), 3.90 (s, 3H), 3.34 (s, 2H), 3.22 (t, J = 8.6 Hz, 2H), 2.96 - 2.46 (m, 6H), 1.97 (s, 2H), 1.82 - 1.53 (m, 4H), 1.27 (s, 2H).

Step 8

3-((5-fluoro-2,3-dihydrobenzofuran-6-yl) methoxy)-5-(3-(4-(pyrrolidin-1-yl) butyl) ureido) isothiazole-4-carboxamide

In a microwave tube, methyl 3-((5-fluoro-2,3-dihydrobenzofuran-6-yl)methoxy)-5-(3-(4-(pyrrolidin-1-yl)butyl) ureido)isothiazol-4-carboxylate I312 (200 mg, 0.4 mmol) was dissolved in $NH_3$/MeOH (10 mL, 9 mol/L), and the mixture was sealed to react at 40 °C for 16 hours. The reaction mixture was concentrated under reduced pressure to obtain a crude product, and the crude product was purified by the high-performance preparative chromatography to obtain a white solid product 3-((5-fluoro-2,3-dihydrobenzofuran-6-yl)methoxy)-5-(3-(4-(pyrrolidin-1-yl)butyl)ureido)isothiazol-4-formamide T071 (30 mg, 62.82 μmol, yield: 15.47%).

The product was verified by LCMS, H-NMR and C-NMR.

$^1$H NMR (400MHz, $CDCl_3$): δ (ppm) 10.91 (s, 1H), 7.90 (s, 2H), 7.16 (s, 1H), 6.97 (d, J = 9.0 Hz, 1H), 6.84 (d, J = 5.6 Hz, 1H), 5.47 (s, 2H), 4.61 (t, J = 8.7 Hz, 2H), 3.33 (s, 2H), 3.24 (t, J = 8.7 Hz, 2H), 2.66 - 2.51 (m, 6H), 1.90 (s, 2H), 1.69 (s, 6H).

$^{13}$C NMR (100MHz, $CDCl_3$): δ (ppm) 169.61, 165.83, 161.66, 156.88, 156.07, 154.50, 153.97, 129.22, 129.14, 121.98, 121.82, 112.46, 112.21, 110.29, 110.25, 71.70, 64.59, 64.55, 55.91, 53.91, 40.60, 30.02, 58.20, 26.83, 23.42

Example 48: Synthesis of Compound T072

Synthesis Route:

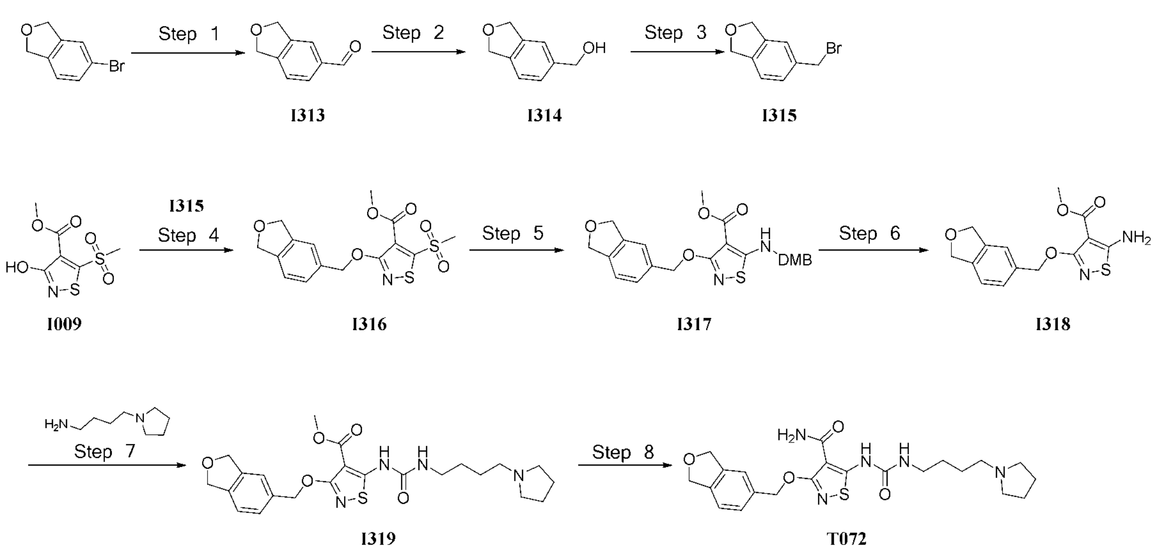


Step 1

1,3-dihydroisobenzofuran-5-carbaldehyde

5-Bromo-1,3-dihydro-2-benzofuran (3 g, 15.07 mmol) was dissolved in THF (50 mL) and cooled to -70 °C under the protection of nitrogen, followed by the addition of n-BuLi (2.5 M, 7.2 mL), the mixture was stirred to react for about 1 hour, followed by the addition of DMF (3.3 g, 45.22 mmol), and the mixture was then stirred to react for about 2 hours. After complete reaction, the mixture was poured into water (30 mL) and extracted with ethyl acetate, and the organic phases were combined, washed with saturated brine, dried with anhydrous sodium sulfate, and concentrated under reduced pressure to obtain a yellow oily liquid product 1,3-dihydroisobenzofuran-5-formaldehyde I313 (2 g, 13.50 mmol, yield: 89.56% ).
The product was verified by LCMS and H-NMR.
$^1$H NMR (400MHz, $CDCl_3$): δ (ppm) 10.05 (s, 1H), 7.83 (d, J = 8.4 Hz, 1H), 7.79 (s, 1H), 7.23 (d, J = 7.6 Hz, 1H) 5.19 (s, 4H)

Step 2

(1,3-dihydroisobenzofuran-5-yl) methanol

Under the protection of $N_2$, 1,3-dihydroisobenzofuran-5-formaldehyde I313 (3 g, 20.25 mmol) was dissolved in anhydrous methanol (30 mL), followed by the addition of $NaBH_4$ (766.1 mg, 20.25 mmol) in the ice water bath, and the mixture was then stirred at room temperature to react for 1 hour. The reaction mixture was quenched with water (30 mL) and then extracted with ethyl acetate (30 mL x2), the organic phases were combined, washed with saturated brine, dried with anhydrous sodium sulfate, and concentrated under reduced pressure to obtain a crude product, and the crude product was purified by the column chromatography (PE/EA = 3/1) to obtain a yellow oily liquid product (1,3-dihydroisobenzofuran-5-yl)methanol I314 (1.56 g, 10.39 mmol, yield: 51.30%).
The product was verified by LCMS and H-NMR
$^1$H NMR (400MHz, $CDCl_3$): δ (ppm) 7.24-7.30 (m, 3H), 5.13 (s, 4H), 4.74 (t, J = 4.8 Hz, 2H), 1.68 (t, J = 5.2 Hz, 1H).

Step 3

6-(bromomethyl)-5-fluoro-2,3-dihydrobenzofuran

(1,3-Dihydroisobenzofuran-5-yl)methanol I314 (1 g, 6.66 mmol) was dissolved in DCM (15 mL) and then cooled to 0°C, followed by the addition of $PBr_3$ (3.6 g, 13.32 mmol), and the reaction system was stirred at room temperature to react for 1 hour. After complete reaction, the mixture was added to water (30 mL) andextracted with dichloromethane, and the organic phases were combined, washed with saturated brine, dried with anhydrous sodium sulfate, and concentrated under reduced pressure to obtain a yellow oily liquid product 6-(bromomethyl)-5-fluoro-2,3-dihydrobenzofuran I315 (1.2 g, 5.63 mmol, yield: 84.58%).
The product was verified by LCMS and H-NMR.
$^1$H NMR (400 MHz, $CDCl_3$) δ (ppm) 7.31 (t, J = 5.6 Hz, 2H), 7.23 (d, J = 7.6 Hz, 1H), 5.12 (s, 4H), 4.54 (s, 2H).

Step 4

Methyl 3-((1,3-dihydroisobenzofuran-5-yl) methoxy)-5-(methylsulfonyl) isothiazole-4-carboxylate

Methyl 3-hydroxyl-5-(methylsulfonyl)isothiazol-4-carboxylate I009 (1.2 g, 5.63 mmol) and potassium carbonate (1.17 g, 8.45 mmol) were dissolved in DMF (20 mL), followed by the addition of 6-(bromomethyl)-5-fluoro-2,3-dihydrobenzofuran I315 (1.3 g, 5.63 mmol), and the reaction system was stirred at room temperature to react for 2 hour. Water (30 mL) was added to the reaction mixture which was then filtered, the filter cake was dissolved in ethyl acetate (50), and the organic phase was washed with saturated brine, dried with anhydrous sodium sulfate, and concentrated under reduced pressure to obtain a yellow solid product methyl 3-((1,3-dihydroisobenzofuran-5-yl)methoxy)-5-(methylsulfonyl)isothiazol-4-carboxylate I316 (2 g, 5.41 mmol, yield: 96.13%).
The product was verified by LCMS and H-NMR.
$^1$H NMR (400MHz, $CDCl_3$): δ (ppm) 7.36-7.40 (m, 2H), 7.27 (s, 1H), 5.52 (s, 2H), 6.90 (d, J=5.6Hz, 1H), 5.15 (s, 4H), 3.98 (s, 3H), 3.49(s, 3H).

Step 5

Methyl 3-((1,3-dihydroisobenzofuran-5-yl) methoxy)-5-((2,4-dimethylbenzyl) amino) isothiazole-4-carboxylate

Methyl 3-((1,3-dihydroisobenzofuran-5-yl)methoxy)-5-(methylsulfonyl)isothiazol-4-carboxylate I316 (1.9 g, 5.14 mmol) was dissolved in THF(15 mL), followed by the addition of 2,4-dimethoxybenzylamine (860 mg, 5.14 mmol), and the reaction system was stirred at 60 °C to react for 2 hours. After the reaction was finished, water (50 mL) was added to the reaction mixture, which was then extracted with ethyl acetate (50 mL x 3), the organic phases were combined, sequentially washed with water and saturated brine, dried with anhydrous sodium sulfate, and concentrated under reduced pressure to obtain a crude product, and the crude product was purified by the flash column chromatography (PE/EA = 15/1-5/1) to obtain a white solid product methyl 3-((1,3-dihydroisobenzofuran-5-yl)methoxy)-5-((2,4-dimethylbenzyl)amino)isothiazol-4-carboxylate I317 (1.9 g, 4.16 mmol, yield: 80.92%).
The product was verified by LCMS and H-NMR.
$^1H$ NMR (400 MHz, $CDCl_3$) δ (ppm) 8.13 (s, 1H), 7.37 (d, J = 9.2Hz, 2H), 7.21 (dd, J = 7.6Hz, 8Hz, 2H), 6.45-6.53 (m, 2H), 5.45 (s, 2H), 5.13 (s, 4H), 4.29 (d, J = 6.4Hz, 2H), 3.76-3.88 (m, 9H).

Step 6

Methyl 5-amino-3-((1,3-dihydroisobenzofuran-5-yl) methoxy) isothiazole-4-carboxylate

Methyl 3-((1,3-dihydroisobenzofuran-5-yl)methoxy)-5-((2,4-dimethylbenzyl)amino)isothiazol-4-carboxylate I317 (1.5 g, 3.29 mmol) was dissolved in DCM/$H_2O$ (20/4 mL), followed by the portionwise addition of DDQ (1.49 g, 6.57 mmol) at 0°C, and the reaction system was stirred at room temperature to react for 0.5 hours. After complete reaction, water (30 mL) was added to the reaction mixture, which was then extracted with DCM (30 mL x3), the organic phases were combined, washed with saturated brine, dried with anhydrous sodium sulfate, and concentrated under reduced pressure to obtain a crude product, and the crude product was purified by the column chromatography (PE/EA = 5/1-EA) to obtain a yellow solid product methyl 5-amino-3-((1,3-dihydroisobenzofuran-5-yl)methoxy)isothiazol-4-carboxylate I318 (1 g, 3.26 mmol, yield: 99.35%).
The product was verified by LCMS and H-NMR.
$^1H$ NMR (400 MHz, $CDCl_3$) δ (ppm) 7.38 (d, J = 10.4 Hz, 2H), 7.25 (d, J = 7.6 Hz, 1H), 6.50 (s, 2H), 5.46 (s, 2H), 5.14 (s, 4H), 3.87 (s, 3H).

Step 7

Methyl 3-((1,3-dihydroisobenzofuran-5-yl) methoxy)-5-(3-(4-(pyrrolidin-1-yl) butyl) ureido) isothiazole-4-carboxylate

4-(Pyrrolidin-1-yl)butyl-1-amine (195.02 mg, 1.37 mmol) was dissolved in anhydrous THF (5 mL) and then cooled to 0°C under the protection of $N_2$, followed by the addition of CDI (222.31 mg, 1.37 mmol), the mixture was stirred at room temperature to react for 1 hour, followed by the addition of DMSO (5 mL), the pressure was then reduced to remove most of the THF, followed by the addition of methyl 5-amino-3-((1,3-dihydroisobenzofuran-5-yl)methoxy)isothiazol-4-carboxylate I318 (300 mg, 979.32 μmol) and potassium carbonate (270.69 mg, 1.96 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((1,3-dihydroisobenzofuran-5-yl)methoxy)-5-(3-(4-(pyrrolidin-1-yl)butyl)ureido) isothiazol-4-carboxylate I319 (180 mg, 379.29 μmol, yield: 38.73%).
The product was verified by LCMS and H-NMR.
$^1H$ NMR (400MHz, $CDCl_3$): δ (ppm) 10.23 (s, 1H), 8.17 (s, 1H), 7.37 - 7.42 (m, 2H), 9.25 (d, J = 7.6 Hz, 1H), 5.47 (s, 2H), 5.13 (s, 4H), 3.84 (s, 3H), 3.34 (s, 2H), 2.73 (s, 4H), 2.58-2.65 (m, 2H), 2.05 (s, 4H), 1.76 (s, 4H).

Step 8

3-((1,3-dihydroisobenzofuran-5-yl) methoxy)-5-(3-(4-(pyrrolidin-1-yl) butyl) ureido) isothiazole-4-carboxamide

In a microwave tube, methyl 3-((1,3-dihydroisobenzofuran-5-yl)methoxy)-5-(3-(4-(pyrrolidin-1-yl)butyl)ureido) isothiazol-4-carboxylate I319 (120 mg, 252.86 μmol) was dissolved in $NH_3$/MeOH (3 mL, 9 mol/L), and the mixture was sealed to react at 40 °C for 16 hours. The reaction mixture was concentrated under reduced pressure to obtain a crude product, and the crude product was purified by the high-performance preparative liquid chromatography (DCM/MeOH = 10/1, 1% $NH_3.H_2O$) to obtain a white solid product 3-((1,3-dihydroisobenzofuran-5-yl)methoxy)-5-(3-(4-(pyrrolidin-1-yl)butyl)ureido)isothiazol-4-formamide T072 (13 mg, 28.29 μmol, yield: 11.19%).

The product was verified by LCMS, H-NMR and C-NMR.

$^1H$ NMR (400 MHz, $CD_3OD+CDCl_3$) δ (ppm) 7.42 (d, J = 4.0 Hz, 2H), 7.32 (s, 1H), 5.50 (s, 2H), 5.10 (s, 4H), 3.27 (t, J = 6.4 Hz, 2H), 2.66-2.75 (m, 6H), 1.89 (s, 4H), 1.60-1.62 (m, 4H).

$^{13}C$ NMR (100 MHz, $CD_3OD+CDCl_3$) δ (ppm) 168.96, 165.95, 162.01, 154.71, 139.37, 139.17, 135.44, 134.89, 127.68, 121.09, 120.95, 97.45, 73.12, 70.07, 55.84, 53.81, 39.71, 27.54, 25.48, 22.96.

Example 49: Synthesis of Compound T073

Synthesis Route:

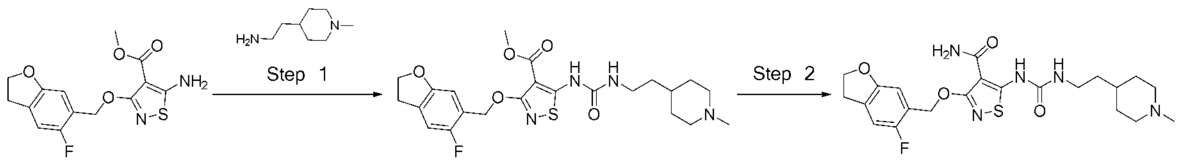


I311 I320 T073

Step 1

Methyl 3-((5-fluoro-2,3-dihydrobenzofuran-6-yl)methoxy)-5-(3-(2-(1-methylpiperidin-4-yl)ethyl)ureido)isothiazole-4-carboxylate

2-(1-Methyl-4-piperidyl)ethylamine (73.7 mg, 518.00 μmol) was dissolved in anhydrous THF (5 mL) and then cooled to 0°C Under the protection of $N_2$, followed by the addition of CDI (74.6 mg, 518.00 μmol), the mixture was stirred at room temperature to react for 1 hour, followed by the addition of DMSO (5 mL), the pressure was then reduced to remove most of the THF, followed by the addition of methyl 5-amino-3-((5-fluoro-2,3-dihydrobenzofuran-6-yl)methoxy)isothiazol-4-carboxylate I311 (120 mg, 370.00 μmol) and potassium carbonate (153.4 mg, 1.11 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((5-fluoro-2,3-dihydrobenzofuran-6-yl)methoxy)-5-(3-(2-(1-methylpiperidin-4-yl)ethyl)ureido)isothiazol-4-carboxylate I320 (35 mg, 71.06 μmol, yield: 19.20%).

The product was verified by LCMS and H-NMR.

$^1H$ NMR (400 MHz, $CDCl_3$) δ 10.37 (s, 1H), 6.94 (dd, J = 13.4, 7.4 Hz, 2H), 5.45 (s, 2H), 5.27 (s, 1H), 4.59 (t, J = 8.7 Hz, 2H), 3.92 (d, J = 9.7 Hz, 3H), 3.39 (dd, J = 13.4, 6.8 Hz, 2H), 3.22 (t, J = 8.6 Hz, 2H), 2.93 (d, J = 10.9 Hz, 2H), 2.33 (s, 3H), 2.00 (t, J = 10.6 Hz, 2H), 1.74 (d, J = 9.5 Hz, 2H), 1.56 (d, J = 5.6 Hz, 2H), 1.33 (d, J = 42.9 Hz, 3H).

Step 2

(3-((5-fluoro-2,3-dihydrobenzofuran-6-yl)methoxy)-5-(3-(2-(1-methylpiperidin-4-yl)ethyl)ureido)isothiazole-4-carboxamide

In a microwave tube, methyl 3-((5-fluoro-2,3-dihydrobenzofuran-6-yl)methoxy)-5-(3-(2-(1-methylpiperidin-4-yl)ethyl)ureido)isothiazol-4-carboxylate I320 (35 mg, 71.06 μmol) was dissolved in $NH_3$/MeOH (4 mL, 9 mol/L), and the mixture was sealed to react at 40 °C for 16 hours. The reaction mixture was concentrated under reduced pressure to obtain a crude product, and the crude product was purified by the high-performance preparative liquid chromatography to obtain a white solid product (3-((5-fluoro-2,3-dihydrobenzofuran-6-yl)methoxy)-5-(3-(2-(1-methylpiperidin-4-yl)ethyl)ureido)isothiazol-4-formamide T073 (16.5 mg, 34.55 μmol, yield: 48.62%).

The product was verified by LCMS, H-NMR and C-NMR.

$^1H$ NMR (400 MHz, $CDCl_3$) δ 11.07 (s, 1H), 7.19 (s, 1H), 6.97 (d, J = 9.0 Hz, 1H), 6.84 (d, J = 5.6 Hz, 1H), 5.47 (s, 2H), 5.39 (s, 1H), 5.16 (s, 1H), 4.61 (t, J = 8.7 Hz, 2H), 3.37 (dd, J = 13.4, 6.8 Hz, 2H), 3.24 (t, J = 8.7 Hz, 2H), 2.87 (d, J = 10.7 Hz, 2H), 2.29 (s, 3H), 1.92 (d, J = 10.2 Hz, 2H), 1.53 (s, 3H), 1.30 (d, J = 23.1 Hz, 4H).

$^{13}C$ NMR (100MHz, $CDCl_3$): δ (ppm) 169.44, 165.85, 161.65, 156.89, 156.06, 154.45, 153.91, 129.31, 129.22,

121.87, 121.70, 112.47, 112.23, 110.29, 110.25, 71.70, 64.69, 55.53, 46.04, 36.26, 32.52, 31.75, 29.99.

Example 50: Synthesis of Compound T074

Synthesis Route:

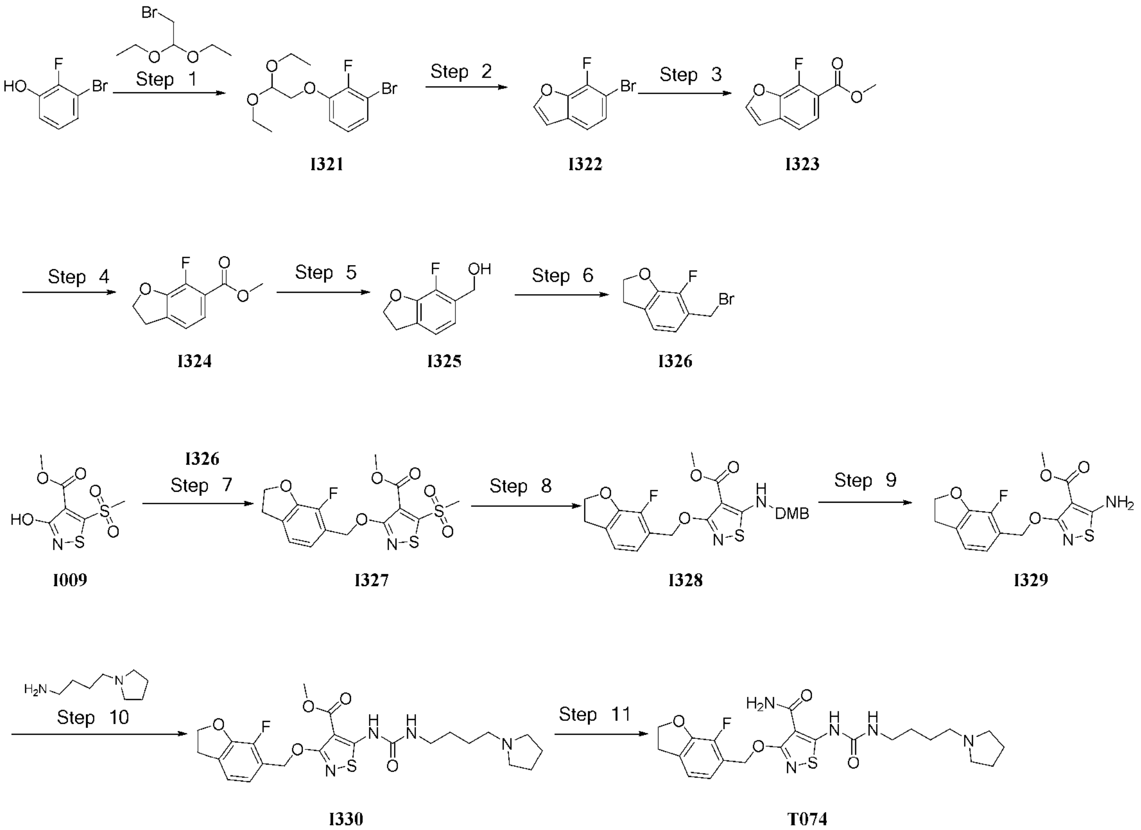


I321 I322 I323 I324 I325 I326 I009 I327 I328 I329 I330 T074

Step 1

Bromo-3-(2,2-diethoxyethoxy)-2-fluorobenzene

3-Bromo-2-fluorophenol (1 g, 5.24 mmol) was dissolved in DMSO (15 mL), followed by the addition of 2-bromo-1,1-diethoxyethane (1.03 g, 5.24 mmol) and potassium carbonate (1.09 g, 7.86 mmol) at room temperature, and the mixture was warmed to 85 °C and stirred to react for about 16 hours. After complete reaction, the mixture was poured into water (60 mL), and extracted with ethyl acetate (20 mL x 3), the organic phases were combined, washed with saturated brine, dried with anhydrous sodium sulfate, and concentrated under reduced pressure to obtain a crude product, and the crude product was purified by the preparative thin-layer chromatography (PE/EA = 10/1) to obtain an oily liquid product 1-bromo-3-(2,2-diethoxyethoxy)-2-fluorobenzene I321 (1.3 g, 4.23 mmol, yield: 80.77%).

The product was verified by LCMS and H-NMR.

$^1$H NMR (400 MHz, $CDCl_3$) δ 7.19 - 7.00 (m, 1H), 7.00 - 6.86 (m, 2H), 4.86 (t, J = 5.6 Hz, 1H), 4.08 (d, J = 5.2 Hz, 2H), 3.77 - 3.82 (m, 2H), 3.65 - 3.69 (m, 2H), 1.26 (t, J = 7.2 Hz, 6H).

Step 2

6-bromo-7-fluorobenzofuran

1-Bromo-3-(2,2-diethoxyethoxy)-2-fluorobenzene I321 (18 g, 58.60 mmol) was dissolved in toluene (200 mL), followed by the addition of polyphosphoric acid (200 mL) at room temperature, and the mixture was warmed to 100 °C and stirred to react for about 16 hours. After complete reaction, the mixture was poured into water (100 mL), and extracted with

ethyl acetate (80 mL x 3), the organic phases were combined, washed with saturated brine, dried with anhydrous sodium sulfate, and concentrated under reduced pressure to obtain a crude product, and the crude product was purified by the flash column chromatography (PE/EA = 10/1) to obtain a white solid product 6-bromo-7-fluorobenzofuran I322 (4.5 g, 20.93 mmol, yield: 35.71%).

The product was verified by LCMS and H-NMR.

$^1$H NMR (400MHz, $CDCl_3$): δ (ppm) 7.65 (d, J=2.1Hz, 1H), 7.39-7.37 (m, 1H), 7.16-7.13 (m, 1H), 6.81-6.80 (m, 1H).

Step 3

Methyl 7-fluorobenzofuran-6-carboxylate

6-Bromo-7-fluorobenzofuran I322 (4.5 g, 20.93 mmol) was dissolved in methanol (10 mL), followed by the addition of Pd(dppf)$Cl_2$ (3.06 g, 4.19 mmol) and NaOAc (5.15 g, 62.79 mmol) at room temperature, and in the presence of CO (gas, 2 MPa), the mixture was warmed to 140 °C to react for about 16 hours. After complete reaction, the mixture was directly concentrated under reduced pressure to obtain a crude product, and the crude product was purified by the flash column chromatography (PE/EA = 10/1) to obtain a white solid product methyl 7-fluorobenzofuran-6-carboxylate I323 (3.8 g, 19.57 mmol, yield: 93.52%)

The product was verified by LCMS and H-NMR.

$^1$H NMR (400MHz, $CDCl_3$): δ (ppm) 7.83-7.78 (m, 2H), 7.38 (d, J=8.3Hz, 1H), 6.85-6.84 (m, 1H), 3.97 (s, 3H).

Step 4

Methyl 7-fluoro-2,3-dihydrobenzofuran-6-carboxylate

Methyl 7-fluorobenzofuran-6-carboxylate I323 (3.1 g, 15.97 mmol) was dissolved in methanol (30 mL), followed by the addition of Pd/C (1.94 g, 15.97 mmol) under the protection of nitrogen, and then, in the presence of hydrogen (20 psi), the mixture was allowed to react at room temperature for about 16 hours. After complete reaction, the mixture was filtered with diatomite, the filtrate was concentrated under reduced pressure to obtain a crude product, and the crude product was purified by the flash column chromatography (PE/EA = 10/1) to obtain a white solid product methyl 7-fluoro-2,3-dihydrobenzofuran-6-carboxylate I324 (2.4 g, 12.23 mmol, yield: 76.62%).

The product was verified by LCMS and H-NMR.

$^1$H NMR (400MHz, $CDCl_3$): δ (ppm) 7.44-7.40 (m, 1H), 7.01 (d, J = 7.8Hz, 1H), 4.73-4.69 (t, J = 8.8Hz, 2H), 3.91 (s, 3H), 3.30 (t, J = 8.8Hz, 2H).

Step 5

(7-fluoro-2,3-dihydrobenzofuran-6-yl)methanol

Methyl 7-fluoro-2,3-dihydrobenzofuran-6-carboxylate I324 (2.4 g, 12.23 mmol) was dissolved in anhydrous THF (30 mL), followed by the addition of LAH (414.98 mg, 12.23 mmol) at room temperature, and the mixture was then stirred at 25 °C to react for about 1 hour. After complete reaction, the mixture was quenched with 10% aq NaOH (15 mL) and then filtered, the liquid phase was extracted with ethyl acetate, the organic phases were combined, washed with water and saturated brine, dried with anhydrous sodium sulfate, and concentrated under reduced pressure to obtain a crude product, and the crude product was purified by the flash column chromatography (PE/EA = 10/1) to obtain a white solid product (7-fluoro-2,3-dihydrobenzofuran-6-yl)methanol I325 (1.5 g, 8.92 mmol, yield: 72.91%).

$^1$H NMR (400 MHz, $CDCl_3$) δ (ppm) 6.95 (d, J = 7.6 Hz, 1H), 6.88 - 6.82 (m, 1H), 4.72 (s, 2H), 4.68 (t, J = 8.7 Hz, 2H), 3.25 (t, J = 8.7 Hz, 2H).

Step 6

6-(bromomethyl)-7-fluoro-2,3-dihydrobenzofuran

(7-Fluoro-2,3-dihydrobenzofuran-6-yl)methanol I325 (1.5 g, 8.92 mmol) was dissolved in DCM (20 mL) and then cooled to 0°C, followed by the addition of $PBr_3$ (2.90 g, 10.70 mmol), and the reaction system was stirred at room temperature to react for 0.5 hour. After complete reaction, the mixture was added to water (50 mL) and then extracted with dichloromethane, the organic phases were combined, washed with saturated brine, dried with anhydrous sodium sulfate, and concentrated under reduced pressure to obtain a crude product, and the crude product was purified by the flash

column chromatography (PE/EA = 10/1) to obtain a yellow oily liquid product 6-(bromomethyl)-7-fluoro-2,3-dihydrobenzofuran I326 (2 g, 8.66 mmol, yield: 97.04%).

The product was verified by LCMS and H-NMR.

$^1$H NMR (400 MHz, $CDCl_3$) δ 6.93 (d, *J* = 7.6 Hz, 1H), 6.84 (dd, *J* = 7.5, 6.1 Hz, 1H), 4.68 (t, *J*= 8.8 Hz, 2H), 4.51 (s, 2H), 3.25 (t, *J* = 8.7 Hz, 2H).

Step 7

Methyl 3-((7-fluoro-2,3-dihydrobenzofuran-6-yl)methoxy)-5-(methylsulfonyl)isothiazole-4-carboxylate

Methyl 3-hydroxyl-5-(methylsulfonyl)isothiazol-4-carboxylate I009 (2.05 g, 8.66 mmol) and potassium carbonate (1.79 g, 12.98 mmol) were dissolved in DMF (20 mL), followed by the addition of 6-(bromomethyl)-7-fluoro-2,3-dihydrobenzofuran I326 (2 g, 8.66 mmol), and the reaction system was stirred at room temperature to react for 1 hour. Water (100 mL) was added to the reaction mixture, which was then subjected to liquid separation and extraction with ethyl acetate (50 mL x 3), the organic phases were sequentially washed with water and saturated brine, dried with anhydrous sodium sulfate, and concentrated under reduced pressure to obtain a crude product, and the crude product was purified by the flash column chromatography (PE/EA = 10/1-1/1) to obtain a white solid product methyl 3-((7-fluoro-2,3-dihydrobenzofuran-6-yl)methoxy)-5-(methylsulfonyl)isothiazol-4-carboxylate I327 (3 g, 7.74 mmol, yield: 89.47%).

The product was verified by LCMS and H-NMR.

$^1$H NMR (400 MHz, $CDCl_3$) δ 6.97-6.92 (m, 2H), 5.51 (s, 2H), 4.69 (m, 2H), 3.93 (s, 3H), 3.47 (s, 3H), 3.27 (t, J = 8.7 Hz, 2H).

Step 8

Methyl 5-((2,4-dimethylbenzyl)amino)-3-((7-fluoro-2,3-dihydrobenzofuran-6-yl)methoxy)isothiazole-4-carboxylate

Methyl 3-((7-fluoro-2,3-dihydrobenzofuran-6-yl)methoxy)-5-(methylsulfonyl)isothiazol-4-carboxylate I327 (3 g, 7.74mmol) was dissolved in THF(30 mL), followed by the addition of 2,4-dimethoxybenzylamine (3.88 g, 23.23 mmol), and the reaction system was stirred at 60°C to react for 4 hours. After the reaction was finished, the reaction mixture was added to water (50 mL), and extracted with ethyl acetate (30 mL x 3), the organic phases were combined, sequentially washed with water and saturated brine, dried with anhydrous sodium sulfate, and concentrated under reduced pressure to obtain a crude product, and the crude product was purified by the flash column chromatography to obtain a white solid product methyl 5-((2,4-dimethylbenzyl)amino)-3-((7-fluoro-2,3-dihydrobenzofuran-6-yl)methoxy)isothiazol-4-carboxylate I328 (2 g, 4.21 mmol, yield: 54.43%).

The product was verified by LCMS.

Step 9

Methyl 5-amino-3-((7-fluoro-2,3-dihydrobenzofuran-6-yl)methoxy)isothiazole-4-carboxylate

Methyl 5-((2,4-dimethylbenzyl)amino)-3-((7-fluoro-2,3-dihydrobenzofuran-6-yl)methoxy)isothiazol-4-carboxylate I328 (965.08 mg, 2.03 mmol) was dissolved in DCM/$H_2O$ (6/2mL), followed by the portionwise addition of DDQ (461.69 mg, 2.03 mmol) at 0°C, and the reaction system was stirred at room temperature to react for 0.5 hours. After complete reaction, water (30 mL) was added to the reaction mixture, which was then extracted with DCM (10 mL x2), the organic phases were combined, washed with saturated brine, dried with anhydrous sodium sulfate, and concentrated under reduced pressure to obtain a crude product, and the crude product was purified by the preparative thin-layer chromatography (PE/EA = 3/1) to obtain a white solid product methyl 5-amino-3-((7-fluoro-2,3-dihydrobenzofuran-6-yl)methoxy)isothiazol-4-carboxylate I329 (350 mg, 1.08 mmol, yield: 53.06%).

The product was verified by LCMS and H-NMR.

$^1$H NMR (400MHz, $CDCl_3$): δ (ppm) 7.00 - 6.95 (m, 2H), 5.46 (s, 2H), 4.68 (t, J = 8.7 Hz, 2H), 3.83 (s, 3H), 3.26 (t, J = 8.7 Hz, 2H).

Step 10

Methyl 3-((7-fluoro-2,3-dihydrobenzofuran-6-yl)methoxy)-5-(3-(4-(pyrrolidin-1-yl)butyl)ureido)isothiazole-4-carboxylate

4-(Pyrrolidin-1-yl)butyl-1-amine (131.57 mg, 924.99 mmol) was dissolved in anhydrous THF (3 mL) and then cooled to 0°C under the protection of $N_2$, followed by the addition of CDI (209.98 mg, 1.29 mmol), the mixture was stirred at

room temperature to react for 1 hour, followed by the addition of DMSO (3 mL), the pressure was then reduced to remove most of the THF, followed by the addition of methyl 5-amino-3-((7-fluoro-2,3-dihydrobenzofuran-6-yl)methoxy)isothiazol-4-carboxylate I329 (300 mg, 924.99 μmol) and potassium carbonate (255.68 mg, 1.85 mmol), and the reaction system was stirred at room temperature to react for 1 hour. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((7-fluoro-2,3-dihydrobenzofuran-6-yl)methoxy)-5-(3-(4-(pyrrolidin-1-yl)butyl)ureido)isothiazol-4-carboxylate I330 (120 mg, 243.62 μmol, yield: 26.34%).

The product was verified by LCMS and H-NMR.

$^1H$ NMR (400MHz, $CDCl_3$): δ (ppm) 10.23 (s, 1H),7.01-6.94 (m, 2H), 5.47 (s, 2H), 4.68 (t, J = 8.7 Hz, 2H), 3.49 (s, 3H), 3.31-3.24 (m, 4H), 2.65 (d, J = 29.5 Hz, 6H), 1.90 (s, 4H), 1.69 (s, 4H).

Step 11

3-((7-fluoro-2,3-dihydrobenzofuran-6-yl)methoxy)-5-(3-(4-(pyrrolidin-1-yl)butyl)ureido)isothiazole-4-carboxamide

In a microwave tube, methyl 3-((7-fluoro-2,3-dihydrobenzofuran-6-yl)methoxy)-5-(3-(4-(pyrrolidin-1-yl)butyl) ureido)isothiazol-4-carboxylate I330 (150 mg, 304.53 μmol) was dissolved in $NH_3$/MeOH (4 mL, 9 mol/L), and the mixture was sealed to react at 40 °C for 16 hours. The reaction mixture was concentrated under reduced pressure to obtain a crude product, and the crude product was purified by the high-performance preparative liquid chromatography to obtain a white solid product 3-((7-fluoro-2,3-dihydrobenzofuran-6-yl)methoxy)-5-(3-(4-(pyrrolidin-1-yl)butyl)ureido)isothiazol-4-formamide T074 (23.5 mg, 49.21 μmol, yield: 16.16%).

The product was verified by LCMS, H-NMR and C-NMR.

$^1H$ NMR (400 MHz, $CDCl_3$): δ (ppm) 10.89 (s, 1H), 7.85 (s, 1H), 6.97 - 6.88 (m, 2H), 5.49 (s, 2H), 4.70 (t, J = 8.8 Hz, 2H), 3.29 - 3.25 (m, 4H), 2.55 (d, J = 26.1 Hz, 6H), 1.87 (m, 4H), 1.66 (s, 4H).

$^{13}C$ NMR (100 MHz, MeOD&$CDCl_3$) δ 170.26, 154.34, 148.36, 137.18, 136.77, 135.57, 133.59, 131.07, 130.42, 129.19, 127.72, 126.38, 125.52, 122.34, 122.17, 121.34, 65.96, 56.71, 54.30, 52.78, 45.05, 25.90.

Example 51: Synthesis of Compound T075

Synthesis Route:

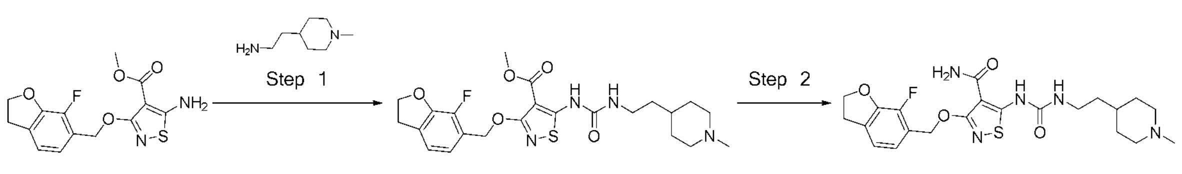


I329 I331 T075

Step 1

Methyl 3-((7-fluoro-2,3-dihydrobenzofuran-6-yl)methoxy)-5-(3-(2-(1-methylpiperidin-4-yl)ethyl)ureido)isothiazole-4-carboxylate

2-(1-Methyl-4-piperidyl)ethylamine (184.20 mg, 1.29 mmol) was dissolved in anhydrous THF (3 mL) and then cooled to 0°C under the protection of $N_2$, followed by the addition of CDI (209.98 mg, 1.29 mmol), the mixture was stirred at room temperature to react for 1 hour, followed by the addition of DMSO (5 mL), the pressure was then reduced to remove most of the THF, followed by the addition of methyl 5-amino-3-((7-fluoro-2,3-dihydrobenzofuran-6-yl)methoxy)isothiazol-4-carboxylate I329 (300 mg, 924.99 μmol) and potassium carbonate (255.68 mg, 1.85 mmol), and the reaction system was stirred at room temperature to react for 1 hour. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((7-fluoro-2,3-dihydrobenzofuran-6-yl)methoxy)-5-(3-(2-(1-methylpiperidin-4-yl)ethyl)ureido)isothiazol-4-carboxylate I331 (260 mg, 527.85 μmol, yield: 57.07%).

The product was verified by LCMS and H-NMR.

$^1$H NMR (400 MHz, $CDCl_3$) δ 10.31 (s, 1H), 6.97-6.96 (m, 2H), 5.47 (s, 2H), 4.68 (t, J = 8.7 Hz, 3H), 3.86-3.85 (m, 3H), 3.49 (s, 2H), 3.39-3.34 (m, 2H), 3.28-3.24 (m, 2H), 2.87 (d, J = 12.5 Hz, 2H), 2.28 (s, 2H), 1.54 (s, 8H).

Step 2

3-((7-fluoro-2,3-dihydrobenzofuran-6-yl)methoxy)-5-(3-(2-(1-methylpiperidin-4-yl)ethyl)ureido)isothiazole-4-carboxamide

In a microwave tube, methyl 3-((7-fluoro-2,3-dihydrobenzofuran-6-yl)methoxy)-5-(3-(2-(1-methylpiperidin-4-yl)ethyl)ureido)isothiazol-4-carboxylate I331 (260 mg, 527.85 μmol) was dissolved in $NH_3$/MeOH (5 mL, 9 mol/L), and the mixture was sealed to react at 40°C for 16 hours. The reaction mixture was concentrated under reduced pressure to obtain a crude product, and the crude product was purified by the high-performance preparative liquid chromatography to obtain a white solid product 3-((7-fluoro-2,3-dihydrobenzofuran-6-yl)methoxy)-5-(3-(2-(1-methylpiperidin-4-yl)ethyl)ureido)isothiazol-4-formamide T075 (22.3 mg, 46.70 μmol, yield: 8.85%).

The product was verified by LCMS, H-NMR and C-NMR.

$^1$H NMR (400MHz, $CDCl_3$): δ (ppm) 11.00 (s, 1H), 7.19 (s, 1H), 6.97 - 6.95 (m, 1H), 6.91-6.87 (m, 1H), 5.94 (s, 1H), 5.57 (s, 1H), 5.49 (s, 2H), 4.69 (t, J = 8.7 Hz, 2H), 3.35-3.24 (m, 6H), 2.89 (d, J = 11.0 Hz, 1H), 2.28 (s, 3H), 1.98 - 1.92 (m, 2H), 1.69-1.67 (m, 2H), 1.51-1.50 (m, 2H), 1.35-1.32 (m, 2H).

$^{13}$C NMR (100 MHz, $CDCl_3$) δ 169.40, 165.88, 161.65, 153.81, 146.94, 146.84, 145.28, 131.86, 131.82, 122.99, 122.88, 122.56, 120.03, 19.99, 97.44, 72.92, 64.75, 64.72, 55.60, 46.17, 39.82, 38.23, 35.95, 32.60, 31.90, 30.09.

Example 52: Synthesis of Compound T076

Synthesis Route:

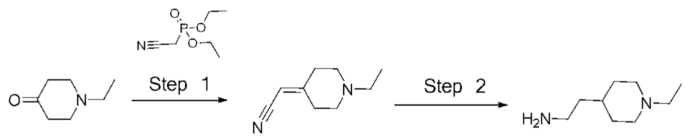


I332 I333

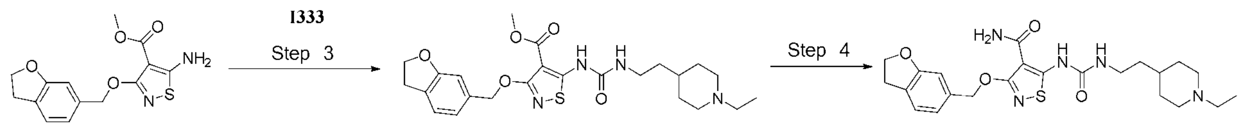


I198 I334 T076

Step 1

2-(1-ethylpiperidin-4-ylidene) acetonitrile

2-Diethoxyacetonitrile phosphate (8.4 g, 47.18 mmol) was dissolved in DMF (50 mL), followed by the addition of NaH (2.4 g, 58.97 mmol, 60% purity) at 0 °C, the mixture was stirred to react for about 1 hour, followed by the addition of 1-ethylpiperidin-4-one(5 g, 39.31 mmol), and the mixture was warmed to 25 °C to react for about 16 hours. After complete reaction, the mixture was poured into water (200 mL), and extracted with ethyl acetate (100 mL x 3), the organic phases were combined, washed with saturated brine, dried with anhydrous sodium sulfate, and concentrated under reduced pressure to obtain a crude product, and the crude product was purified by the column chromatography (neutral alumina, DCM/MeOH = 1/0~10/1) to obtain a yellow oily liquid product 2-(1-ethylpiperidin-4-ylidene)acetonitrile I332 (4 g, 26.63 mmol, yield: 67.73%). The product was verified by LCMS and H-NMR.

$^1$H NMR (400 MHz, $CDCl_3$) δ 5.09 (s, 1H), 2.61 (d, J = 5.6 Hz, 2H), 2.56 - 2.48 (m, 4H), 2.47 - 2.36 (m, 4H), 1.08 (t, J = 7.2 Hz, 3H).

Step 2

2-(1-ethyl-4-piperidyl) ethanamine

2-(1-Ethylpiperidin-4-ylidene)acetonitrile I332 (500 mg, 3.33 mmol) was dissolved in methanol (10 mL), followed by the addition of raney nickel (196 mg, 3.33 mmol) and $NH_3/H_2O$ (2 mL), and in the atmosphere of hydrogen (20 psi), the reaction system was stirred at room temperature to react for 4 hours. The reaction mixture was filtered, and the filtrate was concentrated under reduced pressure to obtain a yellow solid crude product 2-(1-ethyl-4-piperidyl)ethylamine I333 (250 mg, 1.60 mmol, yield: 48.07%).

The product was verified by LCMS and H-NMR.

$^1H$ NMR (400 MHz, $CDCl_3$) δ 2.82 (d, J = 83.2 Hz, 4H), 2.37 (s, 2H), 1.76 (d, J = 70.4 Hz, 4H), 1.27 (s, 6H), 1.07 (s, 4H).

Step 3

Methyl 3-(2,3-dihydrobenzofuran-6-ylmethoxy)-5-[2-(1-ethyl-4-piperidyl)ethylcarbamoylamino]isothiazole-4-carboxylate

2-(1-Ethyl-4-piperidyl)ethylamine I333 (205 mg, 1.31 mmol) was dissolved in anhydrous THF (4 mL) and then cooled to 0°C under the protection of $N_2$, followed by the addition of CDI (211 mg, 1.30 mmol), the mixture was stirred at room temperature to react for 1 hour, followed by the addition of DMSO (4 mL), the pressure was then reduced to remove most of the THF, followed by the addition of methyl 5-amino-3-(2,3-dihydrobenzofuran-6-ylmethoxy)isothiazol-4-carboxylate I198 (200 mg, 652.88 μmmol) and potassium carbonate (226 mg, 1.64 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-(2,3-dihydrobenzofuran-6-ylmethoxy)-5-[2-(1-ethyl-4-piperidyl)ethylcarbamoylamino]isothiazol-4-carboxylate I334 (120 mg, 245.60 μmol, yield: 37.62%).

The product was verified by LCMS and H-NMR.

$^1H$ NMR (400 MHz, $CDCl_3$) δ 10.37 (s, 1H), 7.19 (d, J = 8.0 Hz, 1H), 6.94 (d, J = 4.8 Hz, 2H), 5.41 (s, 2H), 4.60 (t, J = 8.8 Hz, 2H), 3.91 (s, 3H), 3.39 (dd, J = 13.6, 6.8 Hz, 2H), 3.22 (t, J = 8.8 Hz, 2H), 3.02 (d, J = 11.2 Hz, 2H), 2.51 - 2.43 (m, 2H), 1.96 (s, 2H), 1.75 (d, J = 9.2 Hz, 2H), 1.56 (s, 2H), 1.38 (d, J = 13.2 Hz, 3H), 1.13 (t, J = 7.2 Hz, 3H).

Step 4

3-(2,3-dihydrobenzofuran-6-ylmethoxy)-5-[2-(1-ethyl-4-piperidyl)ethylcarbamoylamino]isothiazole-4-carboxamide

In a microwave tube, methyl 3-(2,3-dihydrobenzofuran-6-ylmethoxy)-5-[2-(1-ethyl-4-piperidyl)ethylcarbamoylamino]isothiazol-4-carboxylate I334 (120 mg, 245.60 μmol) was dissolved in $NH_3$/MeOH (6 mL, 9 mol/L), and the mixture was stirred at 60 °C to react for 20 hours. The reaction mixture was concentrated under reduced pressure to obtain a crude product, and the crude product was purified by the high-performance preparative liquid chromatography to obtain a white solid product 3-(2,3-dihydrobenzofuran-6-methoxy)-5-[2-(1-ethyl-4-piperidyl)ethylcarbamoylamino]isothiazol-4-formamide T076 (20.3 mg, 42.86 μmol, yield: 17.45%).

The product was verified by LCMS, H-NMR and C-NMR.

$^1H$ NMR (400 MHz, $CDCl_3$) δ 11.09 (s, 1H), 7.22 (d, J = 7.2 Hz, 2H), 6.92 (d, J = 7.6 Hz, 1H), 6.88 (s, 1H), 5.45 (s, 1H), 5.41 (s, 2H), 4.62 (t, J = 8.8 Hz, 2H), 3.37 (dd, J = 13.2, 6.4 Hz, 2H), 3.24 (t, J = 8.8 Hz, 2H), 3.01 (d, J = 10.4 Hz, 2H), 2.47 (dd, J = 14.4, 7.2 Hz, 2H), 1.96 (s, 2H), 1.75 (d, J = 8.8 Hz, 2H), 1.54 (s, 2H), 1.39 (s, 2H), 1.27 (s, 3H), 1.13 (t, J = 7.2 Hz, 3H).

$^{13}C$ NMR (100 MHz, $CDCl_3$) 169.36, 165.84, 161.93, 160.50, 135.90, 127.59, 125.02, 120.59, 116.41, 109.17, 71.48, 70.51, 53.46, 53.26, 52.55, 36.26, 33.24, 31.77, 29.70, 29.52, 11.87.

Example 53: Synthesis of Compound T077

Synthesis Route:

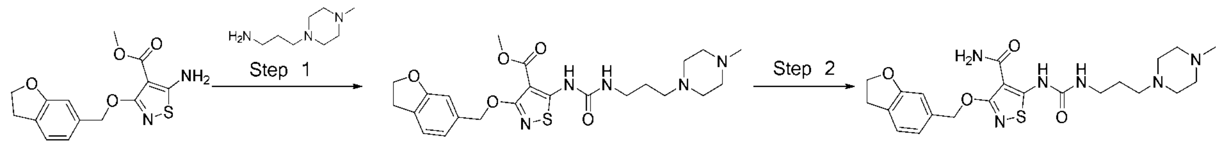


1198 1335 T077

Step 1

Methyl 3-(2,3-dihydrobenzofuran-6-ylmethoxy)-5-[3-(4-methylpiperazin-1-yl)propylcarbamoylamino]isothiazole-4-carboxylate

3-(4-Methylpiperazin-1-yl)propyl-1-amine (71.9 mg, 457.01 μmol) was dissolved in anhydrous THF (2 mL) and then cooled to 0°C under the protection of $N_2$, followed by the addition of CDI (74.1 mg, 457.01 μmol), the mixture was stirred at room temperature to react for 1 hour, followed by the addition of DMSO (2 mL), the pressure was then reduced to remove most of the THF, followed by the addition of methyl 5-amino-3-((2,3-dihydrobenzofuran-6-yl)methoxy)isothiazol-4-carboxylate I198 (100 mg, 326.44 μmol) and potassium carbonate (90.23 mg, 652.88 μmol), and the reaction system was stirred at room temperature to react for 1 hour. After complete reaction, water (30 mL) was added to the reaction mixture, which was then extracted with ethyl acetate (15 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-(2,3-dihydrobenzofuran-6-ylmethoxy)-5-[3-(4-methylpiperazin-1-yl)propylcarbamoylamino]isothiazol-4-carboxylate I335 (140 mg, 285.96 μmol, yield: 87.60%).

The product was verified by LCMS and H-NMR.

$^1H$ NMR (400MHz, $CDCl_3$): δ (ppm)7.17 (d, J = 7.8 Hz, 1H), 7.12 (s, 1H), 6.93 (s, 1H), 5.39 (s, 2H), 4.58 (t, J = 8.7 Hz, 2H), 3.87 (s, 3H), 3.72 (dd, J = 14.1, 7.1 Hz, 2H), 3.20 (t, J = 8.7 Hz, 3H), 2.61 (m, 10H), 2.39 (s, 3H), 1.75 (s, 2H).

Step 2

3-(2,3-dihydrobenzofuran-6-ylmethoxy)-5-[3-(4-methylpiperazin-1-yl)propylcarbamoylamino]isothiazole-4-carboxamide

In a microwave tube, methyl 3-(2,3-dihydrobenzofuran-6-ylmethoxy)-5-[3-(4-methylpiperazin-1-yl)propylcarbamoylamino]isothiazol-4-carboxylate I335 (200 mg, 408.51 μmol) was dissolved in $NH_3$/MeOH (4 mL, 9 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-(2,3-dihydrobenzofuran-6-methoxy)-5-[3-(4-methylpiperazin-1-yl)propylcarbamoylamino]isothiazol-4-formamide T077 (34.5 mg, 72.70 μmol, yield: 17.80%).

The product was verified by LCMS, H-NMR and C-NMR.

$^1H$ NMR (400MHz, $CDCl_3$): δ (ppm) 11.05 (s, 1H), 7.59 (s, 1H), 7.19 (d, J = 7.5 Hz, 2H), 6.91 (d, J = 7.6 Hz, 1H), 6.86 (s, 1H), 5.77 (s, 1H), 5.39 (s, 2H), 4.59 (t, J = 8.7 Hz, 2H), 3.42 (dd, J = 11.1, 5.5 Hz, 2H), 3.22 (t, J = 8.7 Hz, 2H), 2.87 - 2.31 (m, 10H), 2.31 (s, 3H), 1.76-1.73(m, 3H)

$^{13}C$ NMR (101 MHz, $CDCl_3$) δ 171.17, 169.44, 165.80, 161.99, 160.53, 136.07, 127.56, 125.01, 120.59, 109.20, 71.47, 70.49, 67.99, 60.41, 57.21, 55.10, 52.98, 45.82, 29.54, 24.57, 21.06, 14.20.

Example 54: Synthesis of Compound T078

Synthesis Route:

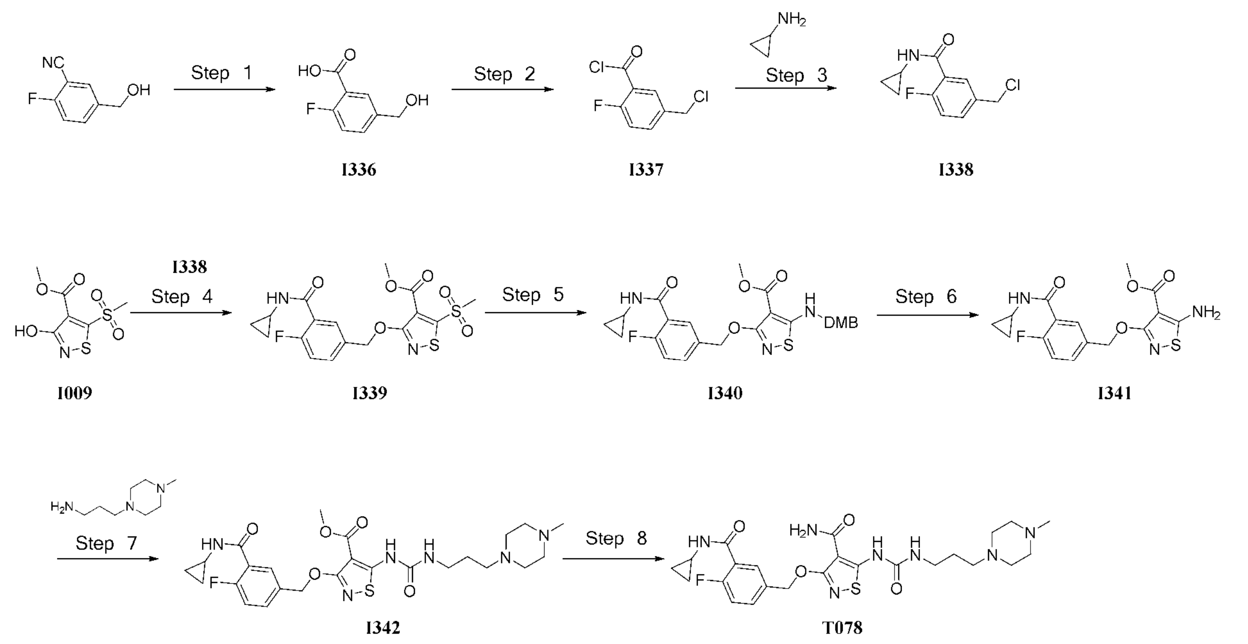


I336 I337 I338

I338

I009 I339 I340 I341

I342 T078

Step 1

2-fluoro-5-(hydroxymethyl)benzoic acid

2-Fluoro-5-(hydroxymethyl)cyanophenyl (500 mg, 3.31 mmol) was dissolved in methanol (1.5 mL) and water (1.5 mL), followed by the addition of NaOH (290 mg, 7.25 mmol) at room temperature, and the mixture was warmed to 60 °C and stirred to react for about 8 hours. After complete reaction, the reaction mixture was regulated with 5M hydrochloric acid solution the pH of 3, and concentrated under reduced pressure to obtain a white solid product 2-fluoro-5-(hydroxymethyl) benzoic acid I336 (350 mg, 2.06 mmol, yield: 62.18%).

The product was verified by LCMS and H-NMR.

$^1$H NMR (400 MHz, MeOD) δ 7.80 (d, J = 6.4 Hz, 1H), 7.48 (s, 1H), 7.15 - 7.09 (m, 1H), 4.61 (s, 2H).

Step 2

5-(chloromethyl)-2-fluoro-benzoyl chloride

2-Fluoro-5-(hydroxymethyl)benzoic acid I336 (2 g, 12.27 mmol) was dissolved in toluene (40 mL) and DMF(0.2 mL), followed by the addition of $SOCl_2$ (7.50 g, 63.04 mmol) at room temperature, and the reaction system was warmed to 60°C and stirred to react for about 2 hours. After complete reaction, the reaction mixture was directly concentrated under reduced pressure to obtain a crude product 5-(chloromethyl)-2-fluoro-benzoyl chloride I337 (2 g, 9.66 mmol, yield: 65.74%) as a yellow oily liquid product.

The product was verified by LCMS.

Step 3

5-(chloromethyl)-N-cyclopropyl-2-fluoro-benzamide

5-(Chloromethyl)-2-fluoro-benzoyl chloride I337 (150 mg, 724.54 μmol) was dissolved in DCM (4 mL), followed by the addition of cyclopropyl amine (62 mg, 1.09 mmol) and triethylamine (220 mg, 2.17 mmol) at 0 °C, and the mixture was stirred to react for about 2 hours. After complete reaction, the mixture was directly concentrated under reduced pressure to obtain a crude product, and the crude product was purified by the preparative thin-layer chromatography (PE/EA = 1/1) to obtain a white solid product 5-(chloromethyl)-N-cyclopropyl-2-fluoro-benzamide I338 (50 mg, 219.62 μmol, yield: 30.31%).

The product was verified by LCMS and H-NMR.

$^1$H NMR (400 MHz, $CDCl_3$) δ 8.13 (dd, J = 7.2, 2.4 Hz, 1H), 7.55 - 7.49 (m, 1H), 7.12 (dd, J = 11.6, 8.4 Hz, 1H), 6.82 (d, J = 10.4 Hz, 1H), 4.61 (s, 2H), 2.96 (td, J = 6.4, 3.6 Hz, 1H), 0.90 (t, J = 6.4 Hz, 2H), 0.68 - 0.60 (m, 2H).

Step 4

Methyl 3-[[3-(cyclopropylcarbamoyl)-4-fluoro-phenyl]methoxy]-5-methylsulfonyl-isothiazole-4-carboxylate

Methyl 3-hydroxyl-5-(methylsulfonyl)isothiazol-4-carboxylate I009 (1.1 g, 4.64 mmol) and potassium carbonate (2.0 g, 14.33 mmol) was dissolved in DMF (20 mL), followed by the addition of 5-(chloromethyl)-N-cyclopropyl-2-fluorobenzamide I338 (1.4 g, 6.15 mmol), and the reaction system was stirred at room temperature to react for 2 hours. After complete reaction, water (100 mL) was added to the reaction mixture, which was then subjected to liquid separation and extraction with ethyl acetate (50 mL x 3), the organic phases were sequentially washed with water and saturated brine, dried with anhydrous sodium sulfate, and concentrated under reduced pressure to obtain a crude product, the crude product was stirred in MTBE (30 mL) and filtered, and the filter cake was dried to obtain a yellow solid product methyl 3-[[3-(cyclopropylcarbamoyl)-4-fluoro-phenyl]methoxy]-5-methylsulfonylisothiazol-4-carboxylate I339 (1 g, 2.33 mmol, yield: 50.34%).
The product was verified by LCMS and H-NMR.
$^1$H NMR (400 MHz, $CDCl_3$) δ 8.25 (dd, J = 7.6, 2.4 Hz, 1H), 7.60 - 7.56 (m, 1H), 7.15 (dd, J = 11.6, 8.4 Hz, 1H), 6.85 (d, J = 13.2 Hz, 1H), 5.50 (s, 2H), 4.01 (s, 3H), 3.51 (s, 3H), 2.99 - 2.94 (m, 1H), 0.93 - 0.90 (m, 2H), 0.67 - 0.64 (m, 2H).

Step 5

Methyl 3-[[3-(cyclopropylcarbamoyl)-4-fluoro-phenyl]methoxy]-5-[(2,4-dimethoxyphenyl)methylamino]isothiazole-4-carboxylate

Methyl 3-[[3-(cyclopropylcarbamoyl)-4-fluoro-phenyl]methoxy]-5-methylsulfonylisothiazol-4-carboxylate I339 (700 mg, 1.63 mmol) was dissolved in THF(15 mL), followed by the addition of 2,4-dimethoxybenzylamine (2.7 g, 16.34 mmol), and the reaction system was stirred at 65 °C to react for 1 hour. After the reaction was finished, the reaction mixture was added to water (30 mL), sitrred for about 20 minutes and then filtered, and the filter cake was dried to obtain a yellow solid product methyl 3-[[3-(cyclopropylcarbamoyl)-4-fluoro-phenyl]methoxy]-5-[(2,4-dimethoxyphenyl)methylamino]isothiazol-4-carboxylate I340 (500 mg, 969.83 μmol, yield: 59.36%).
The product was verified by LCMS and H-NMR.
$^1$H NMR (400 MHz, $CDCl_3$) δ 8.21 (dd, J = 7.6, 2.4 Hz, 1H), 8.13 (t, J = 5.6 Hz, 1H), 7.59 - 7.52 (m, 1H), 7.17 (d, J = 8.4 Hz, 1H), 7.10 (dd, J = 11.6, 8.4 Hz, 1H), 6.86 (d, J = 12.4 Hz, 1H), 6.49 - 6.44 (m, 2H), 5.41 (s, 2H), 4.28 (d, J = 6.0 Hz, 2H), 3.87 (s, 3H), 3.86 (s, 3H), 3.81 (s, 3H), 2.95 (dt, J = 10.4, 3.6 Hz, 1H), 0.89 (q, J = 6.8 Hz, 2H), 0.66 - 0.60 (m, 2H).

Step 6

Methyl 5-amino-3-[[3-(cyclopropylcarbamoyl)-4-fluoro-phenyl]methoxy]isothiazole-4-carboxylate

Methyl 3-[[3-(cyclopropylcarbamoyl)-4-fluoro-phenyl]methoxy]-5-[(2,4-dimethoxyphenyl)methylamino]isothiazol-4-carboxylate I340 (600 mg, 1.16 mmol) was dissolved in DCM/$H_2O$ (10/2 mL), followed by the portionwise addition of DDQ (528 mg, 2.33 mmol) at 0°C, and the reaction system was stirred at room temperature to react for 2 hours. After complete reaction, water (30 mL) was added to the reaction mixture, which was then extracted with ethyl acetate (20 mL x3), the organic phases were combined, washed with saturated brine, dried with anhydrous sodium sulfate, and concentrated under reduced pressure to obtain a crude product, and the crude product was purified by chromatographic columns (neutral alumina, PE/EA = 10/1-5/1) to obtain a white solid product methyl 5-amino-3-[[3-(cyclopropylcarbamoyl)-4-fluorophenyl]methoxy]isothiazol-4-carboxylate I341 (340 mg, 930.54 μmol, yield: 79.96%).
The product was verified by LCMS and H-NMR.
$^1$H NMR (400 MHz, $CDCl_3$) δ 8.25 (dd, J = 7.6, 2.0 Hz, 1H), 7.60 - 7.55 (m, 1H), 7.12 (dd, J = 11.8, 8.4 Hz, 1H), 6.84 (d, J = 12.0 Hz, 1H), 6.50 (s, 2H), 5.44 (s, 2H), 3.92 (s, 3H), 2.96 (dt, J = 10.4, 3.6 Hz, 1H), 0.91 (q, J = 6.8 Hz, 2H), 0.67 - 0.62 (m, 2H).

Step 7

Methyl 3-[[3-(cyclopropylcarbamoyl)-4-fluoro-phenyl]methoxy]-5-[3-(4-methylpiperazin-1-yl)propylcarbamoylamino]isothiazole-4-carboxylate

3-(4-Methylpiperazin-1-yl)propyl-1-amine (116 mg, 738.96 μmol) was dissolved in anhydrous THF (5 mL) and then cooled to 0°C under the protection of $N_2$, followed by the addition of CDI (120 mg, 738.96 μmol), the mixture was stirred at room temperature to react for 1 hour, followed by the addition of DMSO (5 mL), the pressure was then reduced to

remove most of the THF, followed by the addition of methyl 5-amino-3-[[3-(cyclopropylcarbamoyl)-4-fluorophenyl] methoxy]isothiazol-4-carboxylate I341 (180 mg, 492.64 μmol) and potassium carbonate (136 mg, 985.28 μmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[[3-(cyclopropylcarbamoyl)-4-fluoro-phenyl]methoxy]-5-[3-(4-methylpiperazin-1-yl)propylcarbamoylamino]isothiazol-4-carboxylate I342 (90 mg, 164.05 μmol, yield: 33.30%). The product was verified by LCMS and H-NMR.

$^1$H NMR (400 MHz, $CDCl_3$) δ 10.39 (s, 1H), 8.29 (dd, J = 7.6, 2.0 Hz, 1H), 8.11 (s, 1H), 7.59 - 7.53 (m, 1H), 7.12 (dd, J = 11.6, 8.4 Hz, 1H), 6.84 (d, J = 12.8 Hz, 1H), 5.45 (s, 2H), 3.96 (d, J = 5.2 Hz, 3H), 3.47 (d, J = 5.2 Hz, 2H), 2.97 (dd, J = 7.2, 3.6 Hz, 1H), 2.61 (dd, J = 12.8, 7.2 Hz, 10H), 2.40 (s, 3H), 1.78 - 1.73 (m, 2H), 0.90 (t, J = 6.4 Hz, 2H), 0.65 (q, J = 6.8 Hz, 2H).

Step 8

3-[[3-(cyclopropylcarbamoyl)-4-fluoro-phenyl]methoxy]-5-[3-(4-methylpiperazin-1-yl)propylcarbamoylamino]isothiazole-4-carboxamide

In a microwave tube, methyl 3-[[3-(cyclopropylcarbamoyl)-4-fluoro-phenyl]methoxy]-5-[3-(4-methylpiperazin-1-yl)propylcarbamoylamino]isothiazol-4-carboxylate I342 (100 mg, 182.27 μmol) was dissolved in $NH_3$/MeOH (6 mL, 9 mol/L), and the mixture was sealed and stirred at 60 °C to react for 16 hours. The reaction mixture was concentrated under reduced pressure to obtain a crude product, and the crude product was purified by the high-performance preparative chromatography to obtain a white solid product 3-[[3-(cyclopropylcarbamoyl)-4-fluorophenyl]methoxy]-5-[3-(4-methylpiperazin-1-yl)propylcarbamoylamino]isothiazol-4-formamide

T078 (17.8 mg, 33.36 μmol, yield: 18.30%).

The product was verified by LCMS, H-NMR and C-NMR.

$^1$H NMR (400 MHz, $CD_3OD$) δ 7.80 (dd, J = 6.8, 2.0 Hz, 1H), 7.67 (d, J = 3.2 Hz, 1H), 7.23 (m, 1H), 5.50 (s, 2H), 3.28 (d, J = 6.8 Hz, 2H), 2.88 (dd, J = 7.2, 3.6 Hz, 1H), 2.51 (dd, J = 31.6, 24.4 Hz, 10H), 2.36 (s, 3H), 1.79 - 1.73 (m, 2H), 0.86 - 0.81 (m, 2H), 0.66 - 0.62 (m, 2H).

$^{13}$C NMR (100 MHz, $CDCl_3$) 169.56, 165.85, 164.26, 164.23, 161.67, 161.52, 159.20, 154.04, 133.48, 133.38, 132.90, 132.87, 131.98, 131.96, 121.29, 121.16, 116.81, 116.36, 69.08, 56.32, 54.77, 52.69, 45.66, 29.69, 25.26, 23.19, 6.88.

Example 55: Synthesis of Compound T079

Synthesis Route:

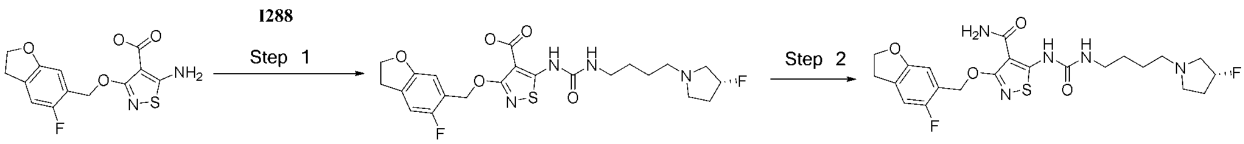


I311 I343 T079

Step 1

Methyl 3-[(5-fluoro-2,3-dihydrobenzofuran-6-yl)methoxy]-5-[4-[(3R)-3-fluoropyrrolidin-1-yl]butylcarbamoylamino]isothiazole-4-carboxylate

4-[(3R)-3-fluoropyrrolidin-1-yl]butyl-1-amine I288 (198 mg, 1.23 mmol) was dissolved in anhydrous THF (5 mL) and then cooled to 0°C under the protection of $N_2$, followed by the addition of CDI (200 mg, 1.23 mmol), the mixture was stirred at room temperature to react for 1 hour, followed by the addition of DMSO (5 mL), the pressure was then reduced to remove most of the THF, followed by the addition of methyl 5-amino-3-((5-fluoro-2,3-dihydrobenzofuran-6-yl)methoxy) isothiazol-4-carboxylate I311 (200 mg, 617 μmmol) and potassium carbonate (170 mg, 1.23 mmol), and the reaction system was stirred at room temperature to react for 1 hour. After complete reaction, water (20 mL) was added to the reaction mixture, which was then extracted with ethyl acetate (10 mL x 3), the organic phases were sequentially washed

with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(5-fluoro-2,3-dihydrobenzofuran-6-yl)methoxy]-5-[4-[(3R)-3-fluoropyrrolidin-1-yl]butylaminoacylamino]isothiazol-4-carboxylate I343 (140 mg, 274.21 μmol, yield: 44.47%). The product was verified by LCMS and H-NMR.

$^1$H NMR (400 MHz, $CDCl_3$) δ 10.22 (s, 1H), 6.97 (d, J = 5.6 Hz, 1H), 6.92 (d, J = 9.2 Hz, 1H), 5.45 (s, 2H), 5.37 - 5.16 (m, 1H), 4.59 (t, J = 8.8 Hz, 2H), 3.89 (s, 3H), 3.34 (dd, J = 11.2, 5.6 Hz, 2H), 3.22 (t, J = 8.8 Hz, 2H), 3.04 - 2.89 (m, 2H), 2.82 - 2.67 (m, 1H), 2.61 - 2.46 (m, 3H), 2.29 - 2.14 (m, 2H), 1.72 - 1.60 (m, 4H).

Step 2

3-[(5-fluoro-2,3-dihydrobenzofuran-6-yl)methoxy]-5-[4-[(3R)-3-fluoropyrrolidin-1-yl]butylcarbamoylamino]isothiazole-4-carboxamide

In a microwave tube, methyl 3-[(5-fluoro-2,3-dihydrobenzofuran-6-yl)methoxy]-5-[4-[(3R)-3-fluoropyrrolidin-1-yl]butylaminoacylamino]isothiazol-4-carboxylate I343 (120 mg, 235.04 μmol) was dissolved in $NH_3$/MeOH (6 mL, 9 mol/L), and the mixture was sealed to react at 60 °C for 20 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(5-fluoro-2,3-dihydrobenzofuran-6-yl)methoxy]-5-[4-[(3R)-3-fluoropyrrolidin-1-yl]butyrylamino]isothiazol-4-formamide T079 (15.0 mg, 30.27 μmol, yield: 12.88%).

The product was verified by LCMS, H-NMR and C-NMR.

$^1$H NMR (400 MHz, MeOD) δ 7.01 (d, J = 9.2 Hz, 1H), 6.82 (d, J = 5.6 Hz, 1H), 5.44 (s, 2H), 5.19 (m, 1H), 4.57 (t, J = 8.7 Hz, 2H), 3.28 - 3.19 (m, 4H), 2.97 (s, 2H), 2.70 (m, 1H), 2.57 (d, J = 6.8 Hz, 2H), 2.48 (dd, J = 15.6, 8.4 Hz, 1H), 2.21 (m, 1H), 2.05 (s, 1H), 1.60 (s, 4H).

Example 56: Synthesis of Compound T080

Synthesis Route:

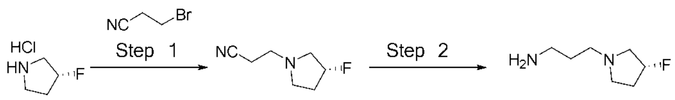


I344 I345

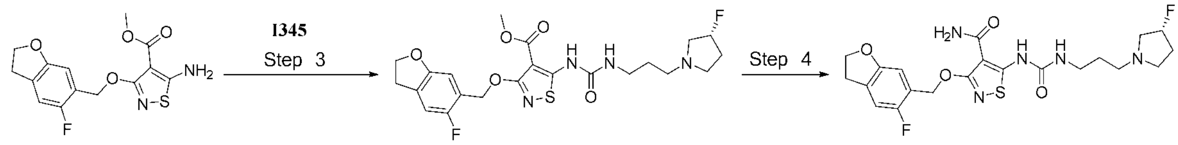


I311 I346 T080

Step 1

3-[(3R)-3-fluoropyrrolidin-1-yl] ropanenitrile

(3R)-3-fluoropyrrolidine (1 g, 11.22 mmol) was dissolved in acetonitrile (10.0 mL), followed by the addition of bromopropionitrile (1.7g, 12.34 mmol) and potassium carbonate (3.1 g, 22.44 mmol), and the reaction system was stirred at room temperature to react for 16 hours. The reaction mixture was poured into water (30 mL), and extracted with ethyl acetate (20 mL x 3), and the organic phases were combined, washed with saturated brine, dried with anhydrous sodium sulfate, and concentrated under reduced pressure to obtain a yellow oily crude product 3-[(3R)-3-fluoropyrrolidin-1-yl] propanenitrile I344 (600 mg, 4.22 mmol, yield: 37.61%).

The product was verified by LCMS and HNMR.

$^1$H NMR (400 MHz, $CDCl_3$) δ 5.17 (d, J = 54.8 Hz, 1H), 2.79 - 2.95 (m, 5H), 2.52 - 2.58 (m, 3H), 2.02-2.22 (m, 2H).

Step 2

3-[(3R)-3-fluoropyrrolidin-1-yl]propan-1-amine

3-[(3R)-3-fluoropyrrolidin-1-yl]propanenitrile I344 (600 mg, 4.22 mmol) was dissolved in methanol (2 mL), followed by the addition of raney nickel (247.7 mg, 4.22 mmol) and $NH_3.H_2O$ (0.2 mL), and in the atmosphere of hydrogen (20 psi), the reaction system was stirred at 25°C to react for 16 hours. After complete reaction, the reaction mixture was filtered, and the filtrate was concentrated under reduced pressure to obtain a yellow oily liquid crude product 3-[(3R)-3-fluoropyrrolidin-1-yl]propyl-1-amine I345 (450 mg, 3.08 mmol, yield: 72.93%).
The product was verified by LCMS and H-NMR.
$^1H$ NMR (400 MHz, $CDCl_3$) δ 5.25 - 5.05 (m, 1H), 2.84-2.91 (m, 2H), 2.59 - 2.76 (m, 3H), 2.49-2.54 (m, 2H), 2.36-2.42 (m, 1H), 1.95 - 2.20 (m, 2H), 1.62-1.72 (m, 2H), 1.38 (s, 2H).

Step 3

Methyl 3-[(5-fluoro-2,3-dihydrobenzofuran-6-yl)methoxy]-5-[3-[(3R)-3-fluoropyrrolidin-1-yl]propylcarbamoylamino]isothiazole-4-carboxylate

3-[(3R)-3-fluoropyrrolidin-1-yl]propyl-1-amine I345 (126.2 mg, 863.33 μmol) was dissolved in anhydrous THF (3 mL) and then cooled to 0°C under the protection of $N_2$, followed by the addition of CDI (140.0 mg, 863.33 μmol), the mixture was stirred at room temperature to react for 1 hour, followed by the addition of DMSO (3 mL), the pressure was then reduced to remove most of the THF, followed by the addition of methyl 5-amino-3-((5-fluoro-2,3-dihydrobenzofuran-6-yl)methoxy)isothiazol-4-carboxylate I311 (200 mg, 616.66 μmol) and potassium carbonate (255.7 mg, 1.85 mmol), and the reaction system was stirred at room temperature to react for 2 hours. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(5-fluoro-2,3-dihydrobenzofuran-6-yl)methoxy]-5-[3-[(3R)-3-fluoropyrrolidin-1-yl]propylcarbamoylamino]isothiazol-4-carboxylate I346 (109 mg, 219.53 μ mol, yield: 35.60%). The product was verified by LCMS R.

Step 4

3-[(5-fluoro-2,3-dihydrobenzofuran-6-yl)methoxy]-5-[3-[(3R)-3-fluoropyrrolidin-1-yl]propylcarbamoylamino]isothiazole-4-carboxamide

In a microwave tube, methyl 3-[(5-fluoro-2,3-dihydrobenzofuran-6-yl)methoxy]-5-[3-[(3R)-3-fluoropyrrolidin-1-yl]propylcarbamoylamino]isothiazol-4-carboxylate I346 (110 mg, 221.54 μmol) was dissolved in $NH_3$/MeOH (3 mL, 9 mol/L), and the mixture was sealed and stirred at 40 °C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(5-fluoro-2,3-dihydrobenzofuran-6-yl)methoxy]-5-[3-[(3R)-3-fluoropyrrolidin-1-yl]propylcarbamoylamino]isothiazol-4-formamide T080 (18.4 mg, 38.21 μmol, yield: 17.25%). The product was verified by LCMS, H-NMR and C-NMR.
$^1H$ NMR (400MHz, $CDCl_3$): δ (ppm)δ 10.90 (s, 1H), 7.58 (s, 1H), 7.17 (s, 1H), 6.94 (d, J = 9.2 Hz, 1H), 6.81 (d, J = 5.6 Hz, 1H), 6.07 (s, 1H), 5.44 (s, 2H), 5.19 (d, J = 54.0 Hz, 1H), 4.58 (t, J = 8.8 Hz, 2H), 3.48 - 3.51 (m, 1H), 3.37 - 3.42 (m, 1H), 3.21 (t, J = 8.8 Hz, 2H), 3.08 - 3.13 (m, 2H), 2.78 (s, 1H), 2.54-2.57 (m, 1H), 2.32 (s, 2H), 2.05-2.19 (m, 3H), 1.76 - 1.79 (m, 2H).
$^{13}C$ NMR (100 MHz, $CDCl_3$) δ 169.51, 166.72, 161.67, 156.86, 156.04, 154.47, 154.27, 129.11, 129.02, 121.94, 121.77, 112.34, 112.09, 110.34, 110.30, 97.23, 94.15, 92.41, 71.66, 64.50, 59.57, 52.51, 38.45, 32.20, 31.98, 30.02, 26.84, 1.91.

Example 57: Synthesis of Compound T081

Synthesis Route:

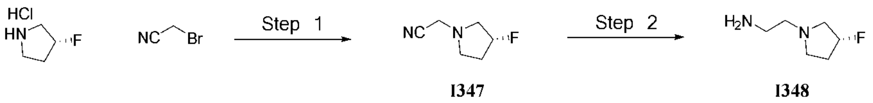


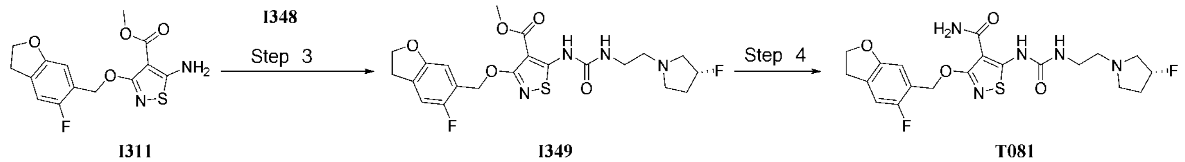


Step 1

2-[(3R)-3-fluoropyrrolidin-1-yl]acetonitrile

(3R)-3-fluoropyrrolidine (1 g, 7.96 mmol, CL) was dissolved in acetonitrile (3 mL), followed by the addition of 2-bromoacetonitrile (1.1 g, 8.76 mmol) and potassium carbonate (2.2 g, 15.93 mmol), and the reaction system was stirred at room temperature to react for 16 hours. The reaction mixture was poured into water (50 mL), and extracted with ethyl acetate (30 mL x 3), and the organic phases were combined, washed with saturated brine, dried with anhydrous sodium sulfate, and concentrated under reduced pressure to obtain a yellow oily crude product 2-[(3R)-3-fluoropyrrolidin-1-yl] acetonitrile I347 (800 mg, 6.24 mmol, yield: 78.39%).
The product was verified by LCMS and HNMR.
$^1H$ NMR (400 MHz, $CDCl_3$) δ 5.27 - 5.12 (m, 1H), 3.71 (s, 1H), 3.04 - 2.95 (m, 2H), 2.91-2.79 (m, 1H), 2.65 - 2.59 (m, 1H), 2.35 - 2.00 (m, 2H).

Step 2

2-[(3R)-3-fluoropyrrolidin-1-yl] ethanamine

2-[(3R)-3-fluoropyrrolidin-1-yl]acetonitrile I347 (2 g, 12.03 mmol) was dissolved in methanol (30 mL), followed by the addition of raney nickel (2 g, 12.03 mmol) and $NH_2.H_2O$ (1 mL), and in the atmosphere of hydrogen (20pis), the reaction system was stirred at 25°C to react for 16 hours. The reaction mixture was filtered, and the filtrate was concentrated under reduced pressure to obtain a green oily liquid product 2-[(3R)-3-fluoropyrrolidin-1-yl]ethylamine I348 (800 mg, 6.05 mmol, yield: 99.44%).
The product was verified by LCMS and H-NMR.
$^1H$ NMR (400MHz, $CDCl_3$): δ (ppm) 5.15 (d, J = 55.4 Hz, 1H), 3.03 - 2.70 (m, 4H), 2.66 - 2.23 (m, 4H), 3.23 - 1.99 (m, 2H), 1.77 (s, 2H).

Step 3

Methyl 3-[(5-fluoro-2,3-dihydrobenzofuran-6-yl)methoxy]-5-[2-[(3R)-3-fluoropyrrolidin-1-yl]ethylcarbamoylamino]isothiazole-4-carboxylate

2-[(3R)-3-fluoropyrrolidin-1-yl]ethylamine I348 (116.2 mg, 878.74 μmol) was dissolved in anhydrous THF (2 mL) and then cooled to 0°C under the protection of $N_2$, followed by the addition of CDI (142.4 mg, 877.94 μmol), the mixture was stirred at room temperature to react for 1 hour, followed by the addition of DMSO (2 mL), the pressure was then reduced to remove most of the THF, followed by the addition of methyl 5-amino-3-[(5-fluoro-2,3-dihydrobenzofuran-6-yl)methoxy] isothiazol-4-carboxylate I311 (190 mg, 585.83 μmol) and potassium carbonate (161.9 mg, 1.17 mmol), and the reaction system was stirred at room temperature to react for 1 hour.After complete reaction, water (30 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(5-fluoro-2,3-dihydrobenzofuran-6-yl)methoxy]-5-[2-[(3R)-3-fluoropyrrolidin-1-yl]ethylcarbamoylamino]isothiazol-4-carboxylate I349 (80 mg, 165.80 μmol, yield: 28.30%). The product was verified by LCMS and H-NMR.
$^1H$ NMR (400 MHz, $CDCl_3$) δ 10.34 (s, 1H), 6.95 (d, J = 5.7 Hz, 1H), 6.91 (d, J = 9.3 Hz, 1H), 5.72 (s, 1H), 5.44 (s, 2H), 5.27 (s, 1H), 5.13 (s, 1H), 4.58 (t, J = 8.6 Hz, 2H), 3.49 (d, J = 4.0 Hz 1H), 3.42 (d, J = 6.3 Hz, 2H), 3.20 (t, J = 8.7 Hz, 2H), 2.95 - 2.81 (m, 4H), 2.71 (d, J = 5.3 Hz, 2H), 2.52 - 2.45 (m, 2H).

Step 4

3-[(5-fluoro-2,3-dihydrobenzofuran-6-yl)methoxy]-5-[2-[(3R)-3-fluoropyrrolidin-1-yl]ethylcarbamoylamino]isothiazole-4-carboxamide

In a microwave tube, methyl 3-[(5-fluoro-2,3-dihydrobenzofuran-6-yl)methoxy]-5-[2-[(3R)-3-fluoropyrrolidin-1-yl]ethylcarbamoylamino]isothiazol-4-carboxylate I349 (87 mg, 180.31 μmol) was dissolved in $NH_3$/MeOH (3 mL, 9 mol/L), and the mixture was stirred at 40°C to react for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(5-fluoro-2,3-dihydrobenzofuran-6-yl)methoxy]-5-[2-[(3R)-3-fluoropyrrolidin-1-yl]ethylcarbamoylamino]isothiazol-4-formamide

T081 (15.5 mg, 33.16 μmol, yield: 18.39%).

The product was verified by LCMS, H-NMR and C-NMR.

$^1$H NMR (400MHz, $CDCl_3$ ): δ (ppm)11.03 (s, 1H), 7.21 (s, 1H), 7.04 (s, 1H), 6.93 (d, J = 9.1 Hz, 1H), 6.82 (d, J = 5.6 Hz, 1H), 5.44 (s, 2H), 5.14 -5.00 (m, 1H), 4.58 (t, J = 8.7 Hz, 2H), 3.43 (s, 2H), 3.21 (t, J = 8.6 Hz, 2H), 3.00-2.90(m, 2H), 2.78 (s, 1H), 2.68 (s, 2H), 2.40 (s, 1H), 2.09-1.93(m, 2H).

$^{13}$C NMR (100 MHz, $CDCl_3$) 169.29, 166.49, 161.68, 156.85, 156.03, 154.79, 154.46, 129.10, 129.01, 122.00, 121.83, 112.30, 112.05, 110.37, 110.33, 97.62, 93.93, 92.18, 71.67, 64.44, 61.11, 56.29, 52.25, 39.09, 32.43, 32.20, 30.01.

Example 58: Synthesis of Compound T082

Synthesis Route:

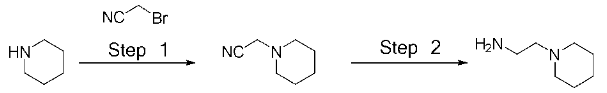


I350 I351

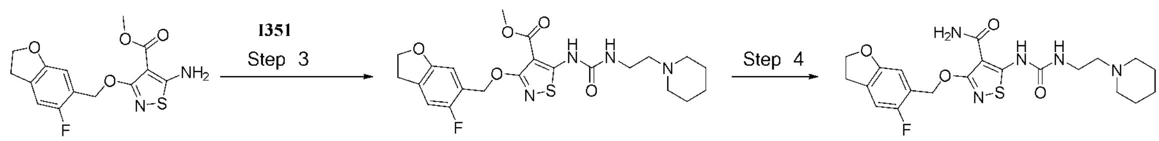


I311 I352 T082

Step 1

2-(1-piperidyl)acetonitrile

Piperidine (3 g, 35.23 mmol) was dissolved in acetonitrile (30 mL), followed by the addition of 2-bromoacetonitrile (4.7 g, 38.76 mmol) and potassium carbonate (9.7 g, 70.47 mmol), and the reaction system was stirred at room temperature to react for 16 hours. The reaction mixture was poured into water (100 mL), and extracted with ethyl acetate (50 mL x 3), the organic phases were combined, washed with saturated brine, dried with anhydrous sodium sulfate, and concentrated under reduced pressure to obtain a crude product, and the crude product was purified by the flash column chromatography (PE/EA = 20/1) to obtain a yellow oily product 2-(1-piperidyl)acetonitrile I350 (3 g, 24.16 mmol, yield: 68.57%).

The product was verified by LCMS and HNMR.

$^1$H NMR (400 MHz, $CDCl_3$): δ (ppm) 3.52 (s, 2H), 2.53-2.52 (m, 4H), 1.70-1.62 (m, 4H), 1.50-1.44 (m, 1H)

Step 2

2-(1-piperidyl)ethanamine

2-(1-Piperidyl)acetonitrile I350 (3 g, 24.16 mmol) was dissolved in methanol (30 mL), followed by the addition of raney nickel (1.4 g, 24.16 mmol) and $NH_2.H_2O$ (1 mL), and in the atmosphere of hydrogen (20pis), the reaction system was stirred at 25°C to react for 16 hours. The reaction mixture was filtered, and the filtrate was concentrated under reduced

pressure to obtain a yellow oily liquid product 2-(1-piperidyl)ethylamine I351 (2.5 g, 19.50 mmol, yield: 80.71%).

The product was verified by LCMS and H-NMR.

$^1$ H NMR (400MHz, $CDCl_3$): δ (ppm) 4.83 (s, 2H), 2.81-2.70 (m, 2H), 2.48-2.36 (m, 6H), 1.59-1.56 (m, 4H), 1.45-1.44 (m, 2H).

Step 3

Methyl3-[(5-fluoro-2,3-dihydrobenzofuran-6-yl)methoxy]-5-[2-(1-piperidyl)ethylcarbam oylamino]isothiazole-4-carboxylate

2-(1-Piperidyl)ethylamine I351 (110.7 mg, 863.33 μmol) was dissolved in anhydrous THF (5 mL) and then cooled to 0°C under the protection of $N_2$, followed by the addition of CDI (140 mg, 863.33 μmol), the mixture was stirred to react for 30 minutes and then stirred at room temperature to react for 1 hour, followed by the addition of DMSO (5 mL), the pressure was then reduced to remove most of the THF, followed by the addition of methyl 5-amino-3-[(5-fluoro-2,3-dihydrobenzofuran-6-yl)methoxy]isothiazol-4-carboxylate I311 (200 mg, 616.66 μmol) and potassium carbonate (255.7 mg, 1.85 mmol), and the reaction system was stirred at room temperature to react for 1 hour. After complete reaction, water (30 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(5-fluoro-2,3-dihydrobenzofuran-6-yl)methoxy]-5-[2-(1-piperidyl)ethylformamido]isothiazol-4-carboxylate I352 (150 mg, 313.46 μmol, yield: 50.83%). The product was verified by LCMS and H-NMR.

$^1$H NMR (400MHz, $CDCl_3$): δ (ppm) 6.98 (d, J = 6.0 Hz, 1H), 6.93 (d, J = 8.8 Hz, 1H), 5.46 (s, 2H), 4.60 (t, J = 8.8Hz, 2H), 3.93 (s, 3H), 3.44 (s, 2H), 3.23 (t, J = 8.4 Hz, 1H), 2.56-2.48 (m, 6H), 1.51 (s, 4H), 1.29-1.25 (m, 2H).

Step 4

3-[(5-fluoro-2,3-dihydrobenzofuran-6-yl)methoxy]-5-[2-(1-piperidyl)ethylcarbamoy lamino]isothiazole-4-carboxamide

In a microwave tube, methyl 3-[(5-fluoro-2,3-dihydrobenzofuran-6-yl)methoxy]-5-[2-(1-piperidyl)ethylformamido]isothiazol-4-carboxylate I352 (150 mg, 313.46 μmol) was dissolved in $NH_3$/MeOH (5 mL, 9 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(5-fluoro-2,3-dihydrobenzofuran-6-yl)methoxy]-5-[2-(1-piperidyl)ethylcarbamoylamino]isothiazol-4-formamide T082 (21.7 mg, 46.82 μmol, yield: 14.94%).

The product was verified by LCMS, H-NMR and C-NMR.

1H NMR (400MHz, $CDCl_3$): δ (ppm) 11.02 (s, 1H), 7.45-7.30 (m, 2H), 7.24 (s, 1H), 6.96 (d, J = 9.2 Hz, 1H), 6.84 (d, J = 5.6 Hz, 1H), 5.46 (s, 2H), 4.60 (t, J = 8.8 Hz, 2H), 3.43-3.42 (m, 2H), 3.23 (t, J = 8.8 Hz, 2H), 2.51-2.49 (m, 2H), 2.39 (s, 4H), 1.46 (s, 4H), 1.39 (s, 2H).

$^{13}$C NMR (100MHz, $CDCl_3$&$CD_3OD$): δ (ppm) 168.91, 165.91, 162.98, 161.30, 160.03, 156.85, 155.96, 154.56, 154.46, 129.49,129.40, 128.17, 121.49, 112.34, 109.51, 99.98, 97.52, 71.48, 63.34, 54.27, 53.38, 36.74, 35.51, 29.78, 25.10, 23.68, 23.58, 22.18.

Example 59: Synthesis of Compound T083

Synthesis Route:

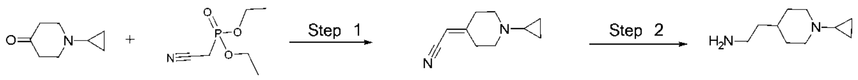


I353

I354

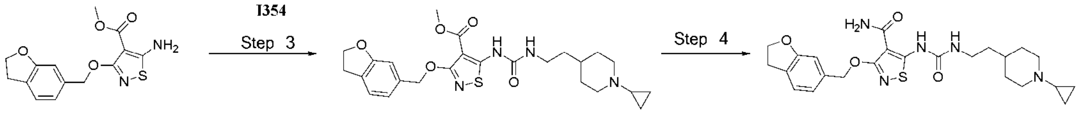


I198

I355

T083

Step 1

2-(1-cyclopropylpiperidin-4-ylidene) acetonitrile

2-Diethoxyacetonitrile phosphate (6.1 g, 34.48 mmol) was dissolved in DMF (50 mL), followed by the addition of NaH (21.38 g, 34.48 mmol, 60%) at 0 °C, the mixture was stirred to react for about 1 hour, followed by the addition of 1-ethylpiperidin-4-one(4.0 g, 28.74 mmol), and the mixture was warmed to 25 °C to react for about 1 hour. After complete reaction, the mixture was poured into water (50 mL), and extracted with ethyl acetate (50 mL x 3), the organic phases were combined, washed with saturated brine, dried with anhydrous sodium sulfate, and concentrated under reduced pressure to obtain a crude product, and the crude product was purified by the flash column chromatography (PE/EA = 10/1-1) to obtain a yellow oily liquid product 2-(1-ethylpiperidin-4-ylidene)acetonitrile I353 (2.5 g, 15.41 mmol, yield: 53.63%).

The product was verified by LCMS and H-NMR.

$^1$H NMR (400 MHz, $CDCl_3$) δ 5.09 (s, 1H), 2.66 - 2.72 (m, 4H), 2.56 (t, J = 6.4 Hz, 2H), 2.33 (t, J = 5.2 Hz, 2H), 2.03 (s, 1H), 1.60-1.66 (m, 1H), 0.45 - 0.50 (m, 2H), 0.40 - 0.44 (m, 2H).

Step 2

2-(1-cyclopropyl-4-piperidyl) ethanamine

2-(1-Cyclopropylpiperidin-4-ylidene)acetonitrile I353 (2.4 g, 14.79 mmol) was dissolved in methanol (10 mL), followed by the addition of raney nickel (868.3 mg, 14.79 mmol) and $NH_3.H_2O$ (0.5 mL), and in the atmosphere of hydrogen (20 psi), the reaction system was stirred at room temperature to react for 4 hours. The reaction mixture was filtered, and the filtrate was concentrated under reduced pressure to obtain a white solid crude product 2-(1-cyclopropyl-4-piperidyl) ethylamine I354 (2 g, 11.89 mmol, yield: 80.34%).

The product was verified by LCMS and H-NMR.

$^1$H NMR (400 MHz, $CDCl_3$) δ 3.00 (d, J = 10.0 Hz, 2H), 2.58-2.72(m, 2H), 2.12 (t, J = 11.2 Hz, 2H), 1.64 (d, J = 12.8 Hz, 2H), 1.53 (s, 2H), 1.28 - 1.45 (m, 4H), 1.17 - 1.22 (m, 2H), 0.41 (d, J = 12.4 Hz, 4H).

Step 3

Methyl 5-[2-(1-cyclopropyl-4-piperidyl) ethylcarbamoylamino]-3-(2,3-dihydrobenzofuran-6-ylmethoxy) isothiazole-4-carboxylate

2-(1-Cyclopropyl-4-piperidyl)ethylamine I354 (153.8 mg, 914.03 μmol) was dissolved in anhydrous THF (3 mL) and then cooled to 0°C under the protection of $N_2$, followed by the addition of CDI (148.2 mg, 914.03 μmol), the mixture was stirred at room temperature to react for 1 hour, followed by the addition of DMSO (3 mL), the pressure was then reduced to remove most of the THF, followed by the addition of methyl 5-amino-3-(2,3-dihydrobenzofuran-6-ylmethoxy)isothiazol-4-carboxylate I198 (200 mg, 652.88 μmmol) and potassium carbonate (270.7 mg, 1.96 mmol), and the reaction system was stirred at room temperature to react for 1 hour. After complete reaction, water (20 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 5-[2-(1-cyclopropyl-4-piperidyl)ethylcarbamoylamino]-3-(2,3-dihydrobenzofuran-6-ylmethoxy)isothiazol-4-carboxylate I355 (20 mg, 39.95 μmol, yield: 6.12%).

The product was verified by LCMS.

Step 4

5-[2-(1-cyclopropyl-4-piperidyl) ethylcarbamoylamino]-3-(2,3-dihydrobenzofuran-6-ylmethoxy) isothiazole-4-carboxamide

In a microwave tube, methyl 5-[2-(1-cyclopropyl-4-piperidyl)ethylcarbamoylamino]-3-(2,3-dihydrobenzofuran-6-ylmethoxy)isothiazol-4-carboxylate I355 (30 mg, 59.93 μmol) was dissolved in $NH_3$/MeOH (4 mL, 9 mol/L), and the mixture was sealed to react at 40 °C for 16 hours. The reaction mixture was concentrated under reduced pressure to obtain a crude product, and the crude product was purified by the high-performance preparative liquid chromatography to obtain a white solid product 5-[2-(1-cyclopropyl-4-piperidyl)ethylcarbamoylamino]-3-(2,3-dihydrobenzofuran-6-ylmethoxy)isothiazol-4-formamide T083 (5 mg, 10.30 μmol, yield: 17.18%). The product was verified by LCMS and H-NMR.

$^1$H NMR (400MHz, $CDCl_3$): δ (ppm) δ 11.07 (d, J = 63.2 Hz, 2H), 7.26 (s, 1H), 7.21 (d, J = 7.6 Hz, 1H), 6.91 (d, J = 7.6 Hz, 1H), 6.85 (s, 1H), 6.54 (s, 1H), 6.15 (s, 1H), 5.38 (s, 2H), 4.60 (t, J = 8.8 Hz, 2H), 3.70 (d, J = 11.2 Hz, 2H), 3.33 (s, 3H), 3.23 (t, J = 8.4 Hz, 2H), 2.82 (s, 2H), 2.35 (s, 1H), 1.93 (d, J = 12.4 Hz, 2H), 1.79 - 1.49 (m, 6H).

Example 60: Synthesis of Compound T084

Synthesis Route:

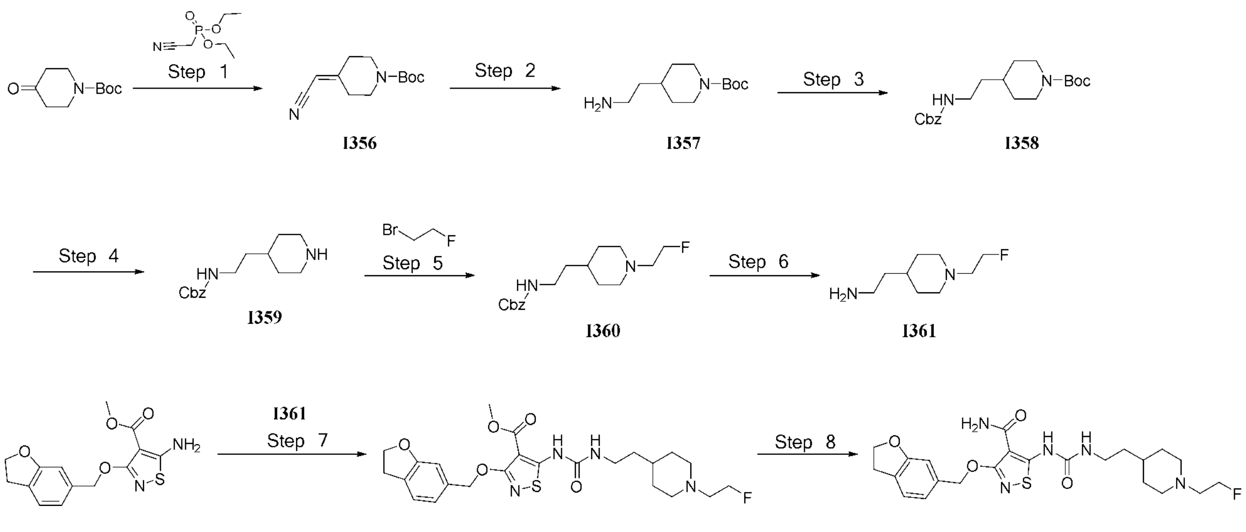


I356 I357 I358

I359 I360 I361

I198 I362 T084

Step 1

Tert-butyl 4-(cyanomethylene) piperidine-1-carboxylate

2-Diethoxyacetonitrile phosphate (889 mg, 5.02 mmol) was dissolved in anhydrous THF (10 mL), followed by the addition of NaH (120.5 mg, 5.02 mmol, 60%) at 0 °C, the mixture was stirred to react for about 1 hour, followed by the addition of tert-butyl 4-oxypiperidin-1-carboxylate (1 g, 5.02 mmol), and the mixture was warmed to 25 °C to react for about 2 hours. After complete reaction, the mixture was poured into water (30 mL), and extracted with ethyl acetate (30 mL x 3), the organic phases were combined, washed with saturated brine, dried with anhydrous sodium sulfate, and concentrated under reduced pressure to obtain a crude product, and the crude product was purified by the column chromatography (PE/EA = 20/1) to obtain a white solid product tert-butyl 4-(cyanomethylene)piperidin-1-carboxylate I356 (0.8 g, 3.60 mmol, yield: 71.71%).

The product was verified by LCMS and H-NMR.

$^1$H NMR (400MHz, $CDCl_3$): δ (ppm) 5.22 (s, H), 3.56-3.50 (m, 4H), 2.58 (t, J = 5.6 Hz, 2H), 2.35 (t, J = 5.6 Hz, 2H), 1.49 (s, 9H).

Step 2

Tert-butyl 4-(2-aminoethyl) piperidine-1-carboxylate

Tert-butyl 4-(cyanomethylene)piperidin-1-carboxylate I356 (0.2 g, 899.75 μmol) was dissolved in methanol (5 mL), followed by the addition of raney nickel (52.8 mg, 899.75 μmol) and $NH_3.H_2O$ (31.5 mg, 899.75 μmol), and in the atmosphere of hydrogen (20 psi), the reaction system was stirred at room temperature to react for 4 hours. The reaction mixture was filtered, and the filtrate was concentrated under reduced pressure to obtain a yellow oily liquid product tert-butyl 4-(2-aminoethyl)piperidin-1-carboxylate I357 (150 mg, 656.94 μmol, yield: 73.01%).
The product was verified by LCMS and H-NMR.
$^1$H NMR (400 MHz, $CDCl_3$) δ (ppm) 4.09 (s, 2H), 2.82-2.66 (m, 4H), 1.66 (d, J = 12.8 Hz, 2H), 1.48 (s, 9H), 1.44-1.39 (m, 2H), 1.31-1.21 (m, 1H), 1.18-1.08 (s, 2H).

Step 3

Tert-butyl 4-[2-(benzyloxycarbonylamino) ethyl] piperidine-1-carboxylate

Tert-butyl 4-(2-aminoethyl)piperidin-1-carboxylate I357 (1.7 g, 7.23 mmol) was dissolved in THF (15 mL) and $H_2O$ (1.5 mL), followed by the addition of $Na_2CO_3$ (2.3 g, 21.68 mmol) at room temperature and then the dropwise addition of Cbz-Cl (1.85 g, 10.84 mmol), to allow for reaction for about 2 hours. After complete reaction, the mixture was added to ice water (45 mL) and extracted with dichloromethane, the organic phases were combined, washed with saturated brine, dried with anhydrous sodium sulfate, and concentrated under reduced pressure to obtain a crude product, and the crude product was purified by the flash column chromatography (PE/EA = 6:1) to obtain a yellow oily liquid product tert-butyl 4-[2-(benzyloxycarbonylamino)ethyl]piperidin-1-carboxylate I358 (1.8 g, 4.97 mmol, yield: 68.7%).
The product was verified by LCMS and H-NMR.
$^1$H NMR (400 MHz, $CDCl_3$) δ (ppm) 7.37-7.26 (m, 5H), 5.11 (s, 2H), 4.70 (s, 1H), 4.08 (t, J = 8.0 Hz, 2H), 3.25 (t, J = 8.0 Hz, 2H), 2.68 (t, J = 8.0 Hz, 2H), 1.58-1.56 (m, 2H), 1.49 (s,1H), 1.45-1.46 (m, 4H), 1.27 (s, 9H).

Step 4

Benzyl N-[2-(4-piperidyl) ethyl] carbamate

Tert-butyl 4-[2-(benzyloxycarbonylamino)ethyl]piperidin-1-carboxylate I358 (1.8 g, 4.82 mmol) was dissolved in DCM (15 mL), followed by the addition of TFA (1.8 g, 15.32 mmol) at room temperature, and the mixture was stirred to react for 16 hours. After complete reaction, the mixture was added to 5% $NaHCO_3$ solution (35 mL) at 0~5°C, regulated with $Na_2CO_3$ to the pH of 8~9, and extracted with dichloromethane (20 mL x 3), and the organic phases were combined, washed with saturated brine, dried with anhydrous sodium sulfate, and concentrated under reduced pressure to obtain a yellow oily liquid product N-[2-(4-piperidyl)ethyl]benzyl carbamate I359 (1.1 g, 4.03 mmol, yield: 83.67%).
The product was verified by LCMS.

Step 5

Benzyl N-[2-[1-(2-fluoroethyl)-4-piperidyl]ethyl]carbamate

2-Fluoroethyl bromide (1.5 g, 11.63 mmol) was dissolved in acetonitrile (30 mL), followed by the addition of NaI (2.1 g, 14.19 mmol) at room temperature, the mixture was warmed to 50°C to react for about 1 hour and then cooled to room temperature, followed by the addition of $Cs_2CO_3$ (10.3 g, 31.71 mmol) and N-[2-(4-piperidyl)ethyl]benzyl carbamate I359 (2.8 g, 10.57 mmol), and the mixture was warmed to 75 °C to react for about 2.5 hours. After complete reaction, the mixture was poured into water (100 mL) and extracted with dichloromethane, the organic phases were combined, washed with saturated brine, dried with anhydrous sodium sulfate, and concentrated under reduced pressure to obtain a crude product, and the crude product was purified by the column chromatography (DCM/MeOH = 20:1) to obtain a yellow oily liquid product N-[2-[1-(2-fluoroethyl)-4-piperidyl]ethyl]benzyl carbamate I360 (2.1 g, 6.77 mmol, yield: 64.06%).
The product was verified by LCMS and H-NMR.
$^1$H NMR (400 MHz, $CDCl_3$) δ (ppm) 7.38-7.28 (m, 5H), 5.11 (s, 2H),4.64 (d, J = 8.0 Hz, 1H), 4.51 (d, J = 8.0 Hz, 1H), 3.23 (t, J = 8.0 Hz, 2H), 2.67-2.65 (m, 2H), 2.04 (m, 2H), 2.04 (m, 3H), 1.32-1.28 (m, 2H),1.26-1.24 (m, 3H).

Step 6

2-[1-(2-fluoroethyl)-4-piperidyl] ethanamine

N-[2-[1-(2-fluoroethyl)-4-piperidyl]ethyl]benzyl carbamate I360 (1.2 g, 3.84 mmol) was dissolved in anhydrous methanol (80 mL), followed by the addition of Pd/C (1.2 g, 11.09 mmol) at room temperature, and in the atmosphere of hydrogen (20 psi), the mixture was stirred to react for about 1 hour. After complete reaction, the mixture was and filtered, and the filtrate was concentrated to obtain a colorless oily liquid product 2-[1-(2-fluoroethyl)-4-piperidyl]ethylamine I361 (632.9 mg, 3.63 mmol, yield: 94.60%).

The product was verified by LCMS and H-NMR.

$^1$H NMR (400 MHz, $CDCl_3$) δ (ppm) 4.50 (s, 1H), 3.49 (d, J = 8.0 Hz, 2H), 2.71 (d, J = 8.0 Hz, 3H), 2.67 (s, 1H), 2.02 (d, J = 8.0 Hz, 2H), 1.53 (t, J = 8.0 Hz, 2H), 1.40-1.23 (m, 8H).

Step 7

Methyl 3-(2,3-dihydrobenzofuran-6-ylmethoxy)-5-[2-[1-(2-fluoroethyl)-4-piperidyl]ethyl carbamoylamino]isothiazole-4-carboxylate

2-[1-(2-fluoroethyl)-4-piperidyl]ethylamine I361 (159.3 mg, 914.16 μmol) was dissolved in anhydrous THF (3 mL) and then cooled to 0°C under the protection of $N_2$, followed by the addition of CDI (148.7 mg, 916.86 μmol), the mixture was stirred at room temperature to react for 1 hour, followed by the addition of DMSO (3 mL), the pressure was then reduced to remove most of the THF, followed by the addition of methyl 5-amino-3-(2,3-dihydrobenzofuran-6-ylmethoxy) isothiazol-4-carboxylate I198 (200 mg, 652.88 μmmol) and potassium carbonate (180.5 mg, 1.31 mmol), and the reaction system was stirred at room temperature to react for 1 hour. After complete reaction, water (10 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-(2,3-dihydrobenzofuran-6-methoxy)-5-[2-[1-(2-fluoroethyl)-4-piperidyl] ethylcarbamoylamino]isothiazol-4-carboxylate I362 (267.2 mg, 527.45 μmol, yield: 80.79%).

The product was verified by LCMS and H-NMR.

$^1$H NMR (400 MHz, $CDCl_3$) δ (ppm) 7.28 (d, J = 8.0 Hz, 1H), 7.19-7.17 (m, 1H), 6.94-6.92 (m, 1H), 5.32 (s ,2H), 4.59-4.52 (m, 3H), 3.94 (s, 2H), 3.35 (m, 2H), 3.24-3.20 (m, 2H), 2.98 (t, J = 16.0 Hz, 2H), 2.64-2.47 (m, 6H), 2.09-2.06 (m, 2H), 1.52 (m, 2H), 1.27 (m, 1H),1.26-1.24 (m, 2H).

Step 8

3-(2,3-dihydrobenzofuran-6-ylmethoxy)-5-[2-[1-(2-fluoroethyl)-4-piperidyl]ethylcarbam oylamino]isothiazole-4-carboxamide

In a microwave tube, methyl 3-(2,3-dihydrobenzofuran-6-methoxy)-5-[2-[1-(2-fluoroethyl)-4-piperidyl]ethylcarbamoylamino]isothiazol-4-carboxylate I362 (125.2 mg, 247.14 μmol) was dissolved in $NH_3$/MeOH (4 mL, 9 mol/L), and the mixture was stirred at 40 °C to react for 16 hours. The reaction mixture was concentrated under reduced pressure to obtain a crude product, and the crude product was purified by the preparative thin-layer chromatography (DCM/MeOH = 10/1) to obtain a white solid product 3-(2,3-dihydrobenzofuran-6-methoxy)-5-[2-[1-(2-fluoroethyl)-4-piperidyl]ethylformamido] isothiazol-4-formamide T084 (37.7 mg, 76.69 μmol, yield: 31.03%).

The product was verified by LCMS, H-NMR and C-NMR.

$^1$H NMR (400 MHz, $CD_3OD$) δ 7.75 (s, 2H), 7.21-7.19 (d, J = 8.0 Hz, 1H), 6.93-6.91 (d, J = 8.0 Hz, 1H), 6.83 (s, 1H), 5.36 (s, 2H), 4.82 (t, J = 28.0 Hz, 2H),4.56-4.52 (m, J = 16.0 Hz, 2H), 3.21-3.19 (t, J = 8.0 Hz, 2H), 3.00 (t, J = 8.0 Hz, 2H), 2.76-2.69 (m, J = 28.0 Hz, 2H), 2.14 (m, J = 12.0 Hz, 2H), 1.17(m, J = 8.0 Hz, 2H), 1.27 (m, J = 8.0 Hz, 1H), 1.50 (m, J = 8.0 Hz, 2H), 1.36 (m, J = 8.0 Hz, 2H), 1.33-1.30 (m, J = 12.0 Hz, 3H).

$^{13}$C NMR (100MHz, $CDCl_3$): δ (ppm) 169.36, 165.86, 161.95, 160.53, 135.98, 127.62, 125.03, 109.21, 71.49, 70.56, 58.50, 58.31, 54.04, 36.31, 32.87, 31.75, 29.54.

Example 61: Synthesis of Compound T085

Synthesis Route:

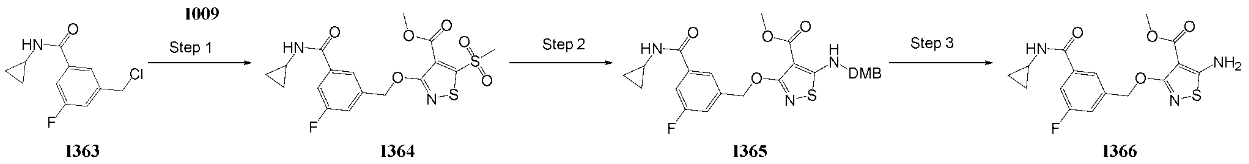

I363 I364 I365 I366

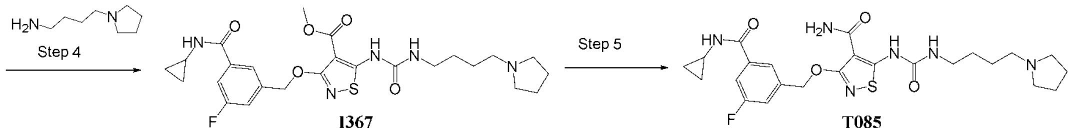

I367 T085

Step 1

Methyl 3-[[3-(cyclopropylcarbamoyl)-5-fluoro-phenyl]methoxy]-5-methylsulfonyl-isothiazole-4-carboxylate

Methyl 3-hydroxyl-5-(methylsulfonyl)isothiazol-4-carboxylate I009 (1 g, 4.21 mmol) and potassium carbonate (1.8 g, 12.64 mmol) were dissolved in DMF (20 mL), followed by the addition of 3-(chloromethyl)-N-cyclopropyl-5-fluorobenzamide I363 (960 mg, 4.21 mmol), and the reaction system was stirred at room temperature to react for 2 hours. Water (50 mL) was added to the reaction mixture, which was then subjected to liquid separation and extraction with ethyl acetate (30 mL x 3), the organic phases were sequentially washed with water and saturated brine, dried with anhydrous sodium sulfate, and concentrated under reduced pressure to obtain a crude product, the crude product was stirred in MTBE (50 mL) for 10 minutes and then filtered, and the filter cake was dried to obtain a yellow solid product methyl 3-[[3-(cyclopropylcarbamoyl)-5-fluoro-phenyl]methoxy]-5-methylsulfonylisothiazol-4-carboxylate I364 (1.1 g, 2.57 mmol, yield: 60.91%).

The product was verified by LCMS and H-NMR.

$^1$H NMR (400 MHz, $CDCl_3$) $\delta$ 7.67 (s, 1H), 7.43 (d, J = 8.8 Hz, 1H), 7.31 (d, J = 8.8 Hz, 1H), 6.33 (s, 1H), 5.52 (s, 2H), 4.02 (s, 3H), 3.50 (s, 3H), 2.95-2.90 (m, 1H), 0.92 (t, J = 6.4 Hz, 2H), 0.66 (s, 2H).

Step 2

Methyl 3-[[3-(cyclopropylcarbamoyl)-5-fluoro-phenyl]methoxy]-5-[(2,4-dimethoxyphenyl)methylamino]isothiazole-4-carboxylate

Methyl 3-[[3-(cyclopropylcarbamoyl)-5-fluoro-phenyl]methoxy]-5-methylsulfonylisothiazol-4-carboxylate I364 (700 mg, 1.63 mmol) was dissolved in THF(10 mL), followed by the addition of 2,4-dimethoxybenzylamine (2.7 g, 16.15 mmol), and the reaction system was stirred at 65 °C to react for 1 hours. After the reaction was finished, the reaction mixture was added to water (50 mL) and then filtered, and the filter cake was dried to obtain a yellow solid product methyl 3-[[3-(cyclopropylcarbamoyl)-5-fluoro-phenyl]methoxy]-5-[(2,4-dimethoxyphenyl)methylamino]isothiazol-4-carboxylate I365 (600 mg, 1.16 mmol, yield: 71.23%).

The product was verified by LCMS and H-NMR.

$^1$H NMR (400 MHz, $CDCl_3$) $\delta$ 8.12 (t, J = 5.6 Hz, 1H), 7.62 (s, 1H), 7.40 (d, J = 8.8 Hz, 1H), 7.33 (d, J = 9.2 Hz, 1H), 7.18 (d, J = 8.4 Hz, 1H), 6.51-6.45 (m, 2H), 6.29 (s, 1H), 5.44 (s, 2H), 4.29 (d, J = 6.0 Hz, 2H), 3.88 (s, 3H), 3.87 (s, 3H), 3.83 (s, 3H), 2.92 (dd, J = 7.2, 3.6 Hz, 1H), 0.91 (q, J = 6.8 Hz, 2H), 0.65 (q, J = 6.8 Hz, 2H).

Step 3

Methyl 5-amino-3-[[3-(cyclopropylcarbamoyl)-5-fluoro-phenyl]methoxy]isothiazole-4-carboxylate

Methyl 3-[[3-(cyclopropylcarbamoyl)-5-fluoro-phenyl]methoxy]-5-[(2,4-dimethoxyphenyl)methylamino]isothiazol-4-carboxylate I365 (650 mg, 1.26 mmol) was dissolved in DCM/$H_2O$ (10/2 mL), followed by the portionwise addition of DDQ (570 mg, 2.51 mmol) at 0°C, and the reaction system was stirred at room temperature to react for 2 hours. After complete reaction, water (50 mL) was added to the reaction mixture, which was then extracted with ethyl acetate (30 mL x3), the organic phases were combined, washed with saturated brine, dried with anhydrous sodium sulfate, and concentrated under reduced pressure to obtain a crude product, and the crude product was purified by chromatographic columns (neutral alumina, PE/EA = 10/1~0/1) to obtain a white solid product methyl 5-amino-3-[[3-(cyclopropylcarbamoyl)-5-fluorophenyl]methoxy]isothiazol-4-carboxylate I366 (250 mg, 684.22 μmol, yield: 54.27%).

The product was verified by LCMS and H-NMR.

$^1H$ NMR (400 MHz, $CDCl_3$) δ 7.65 (s, 1H), 7.41 (d, J = 8.8 Hz, 1H), 7.34 (d, J = 9.6 Hz, 1H), 6.47 (s, 2H), 6.26 (s, 1H), 5.46 (s, 2H), 3.93 (d, J = 3.2 Hz, 3H), 2.93 (d, J = 3.2 Hz, 1H), 0.92 (d, J = 6.0 Hz, 2H), 0.66 (s, 2H).

Step 4

Methyl 3-[[3-(cyclopropylcarbamoyl)-5-fluoro-phenyl]methoxy]-5-(4-pyrrolidin-1-ylbutylcarbamoylamino)isothiazole-4-carboxylate

4-(Pyrrolidin-1-yl)butyl-1-amine (150 mg, 1.05 mmol) was dissolved in anhydrous THF (4 mL) and then cooled to 0°C under the protection of $N_2$, followed by the addition of CDI (150 mg, 925.08 μmol), the mixture was stirred at room temperature to react for 1 hour, followed by the addition of DMSO (4 mL), the pressure was then reduced to remove most of the THF, followed by the addition of methyl 5-amino-3-[[3-(cyclopropylcarbamoyl)-5-fluorophenyl]methoxy]isothiazol-4-carboxylate I366 (200 mg, 547.38 μmol) and potassium carbonate (150 mg, 1.09 mmol), and the reaction system was stirred at room temperature to react for 1 hour. After complete reaction, water (30 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[[3-(cyclopropylcarbamoyl)-5-fluoro-phenyl]methoxy]-5-(4-pyrrolidin-1-ylbutylcarbamoylamino)isothiazol-4-carboxylate I367 (110 mg, 206.14 μmol, yield: 37.66%). The product was verified by LCMS and H-NMR.

$^1H$ NMR (400 MHz, $CDCl_3$) δ 10.21 (s, 1H), 8.30 (s, 1H), 7.64 (s, 1H), 7.41 (d, J = 8.8 Hz, 1H), 7.33 (d, J = 9.2 Hz, 1H), 6.32 (s, 1H), 5.45 (s, 2H), 3.94 (s, 3H), 3.34 (s, 2H), 2.96-2.90 (m, 1H), 2.73 (d, J = 40.4 Hz, 6H), 1.97 (s, 4H), 1.81-1.67 (m, 4H), 0.90 (t, J = 6.4 Hz, 2H), 0.66 (t, J = 8.0 Hz, 2H).

Step 5

3-[[3-(cyclopropylcarbamoyl)-5-fluoro-phenyl]methoxy]-5-(4-pyrrolidin-1-ylbutylcarbamoylamino)isothiazole-4-carboxamide

In a microwave tube, methyl 3-[[3-(cyclopropylcarbamoyl)-5-fluoro-phenyl]methoxy]-5-(4-pyrrolidin-1-ylbutylcarbamoylamino)isothiazol-4-carboxylate I367 (100 mg, 187.40 μmol) was dissolved in $NH_3$/MeOH (14 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[[3-(cyclopropylcarbamoyl)-5-fluorophenyl]methoxy]-5-(4-pyrrolidin-1-ylbutylcarbamoylamino)isothiazol-4-formamide T085 (13.4 mg, 25.84 μmol, yield: 13.79%).

The product was verified by LCMS, H-NMR and C-NMR.

$^1H$ NMR (400 MHz, $CDCl_3$) δ 11.05 (s, 1H), 7.92 (s, 1H), 7.66 (s, 1H), 7.51 (d, J = 8.8 Hz, 1H), 7.32 (s, 1H), 7.00 (s, 1H), 6.75 (s, 1H), 5.96 (s, 1H), 5.45 (s, 2H), 3.29 (s, 2H), 2.93 (dd, J = 7.2, 3.6 Hz, 5H), 2.77 (s, 2H), 2.01 (s, 4H), 1.80-1.74 (m, 2H), 1.63 (s, 2H), 0.94-0.87 (m, 2H), 0.69 (s, 2H).

$^{13}C$ NMR (100 MHz, $CDCl_3$) δ 169.57, 167.36, 165.68, 163.97, 161.34, 154.14, 138.80, 137.20, 122.56, 118.41, 114.60, 114.37, 69.15, 55.75, 53.93, 53.44, 27.80, 26.11, 23.36, 23.31, 6.68.

Example 62: Synthesis of Compound T086

Synthesis Route:

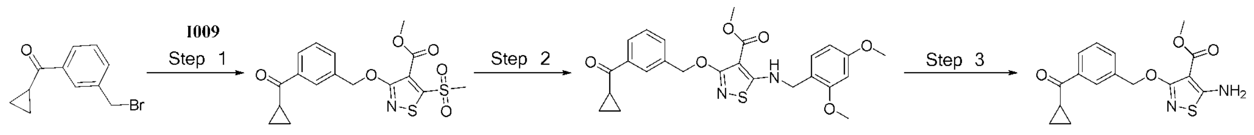


I368 I369 I370 I371

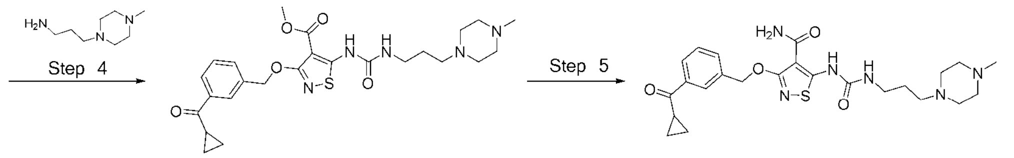


I372 T086

Step 1

Methyl 3-((3-(cyclopropanecarbonyl) benzyl) oxy)-5-(methylsulfonyl) isothiazole-4-carboxylate

Methyl 3-hydroxyl-5-(methylsulfonyl)isothiazol-4-carboxylate I009 (1 g, 4.21 mmol) and potassium carbonate (1.8 g, 12.64 mmol) were dissolved in DMF (10 mL), followed by the addition of [3-(bromomethyl)phenyl]-cyclopropyl ketone I368 (1.2 g, 5.06 mmol), and the reaction system was stirred at room temperature to react for 1 hour. Water (30 mL) was added to the reaction mixture, which was then filtered, and the filter cake was washed with ethyl acetate and saturated brine, dried with anhydrous sodium sulfate, and concentrated under reduced pressure to obtain a yellow solid product methyl 3-((3-(cyclopropylcarbonyl)benzyl)oxy)-5-(methylsulfonyl)isothiazol-4-carboxylate I369 (1.6 g, 4.05 mmol, yield: 95.99%).

The product was verified by LCMS and H-NMR.

$^1$H NMR (400 MHz, $CDCl_3$) δ (ppm) 8.12 (s, 1H), 8.02-7.94 (m, 1H), 7.66 (d, J = 7.6 Hz, 1H), 7.53-7.45 (m, 2H), 5.55 (s, 2H), 3.97 (s, 3H), 3.48 (s, 3H), 2.72-2.65 (m, 1H), 1.28-1.22 (m, 2H), 1.09-1.05 (m, 2H).

Step 2

Methyl 3-((3-(cyclopropanecarbonyl) benzyl) oxy)-5-((2,4-dimethylbenzyl) amino) isothiazole-4-carboxylate

Methyl 3-((3-(cyclopropylcarbonyl)benzyl)oxy)-5-(methylsulfonyl)isothiazol-4-carboxylate I369 (1.6 g, 4.05 mmol) was dissolved in THF(2 mL), followed by the addition of 2,4-dimethoxybenzylamine (6.77 g, 40.46 mmol), and the reaction system was stirred at 65 °C to react for 5 hours. After the reaction was finished, water (20 mL) was added to the reaction mixture, which was then extracted with ethyl acetate (10 mL x 3), the organic phases were combined, sequentially washed with water and saturated brine, dried with anhydrous sodium sulfate, and concentrated under reduced pressure to obtain a crude product, and the crude product was purified by the flash column chromatography (PE/EA = 10/1-0/1) to obtain a yellow solid product methyl 3-((3-(cyclopropylcarbonyl)benzyl)oxy)-5-((2,4-dimethylbenzyl)amino)isothiazol-4-carboxylate I370 (1.2 g, 2.49 mmol, yield: 61.46%).

The product was verified by LCMS and H-NMR.

$^1$H NMR (400 MHz, $CDCl_3$) δ (ppm) 8.14 (d, J = 7.2 Hz, 1H), 7.95 (d, J = 7.6 Hz, 1H), 7.64 (d, J = 7.6 Hz, 1H), 7.47 (t, J = 7.6 Hz, 1H), 7.16 (d, J = 8.4 Hz, 1H), 6.47-6.43 (m, 2H), 5.47 (s, 2H), 4.27 (d, J = 6.0 Hz, 2H), 3.84 (s, 6H), 3.80 (s, 3H), 2.72-2.66 (m, 1H), 1.27-1.22 (m, 2H), 1.07-1.02 (m, 2H).

Step 3

Methyl 5-amino-3-((3-(cyclopropanecarbonyl) benzyl) oxy) isothiazole-4-carboxylate

Methyl 3-((3-(cyclopropylcarbonyl)benzyl)oxy)-5-((2,4-dimethylbenzyl)amino)isothiazol-4-carboxylate I370 (1.3 g, 2.66 mmol) was dissolved in DCM/$H_2O$ (15/3 mL), followed by the portionwise addition of DDQ (906.9 mg, 4.00 mmol) at 0°C, and the reaction system was stirred at room temperature to react for 0.5 hours. After complete reaction, water (30 mL) was added to the reaction mixture, which was then extracted with DCM (20 mL x3), the organic phases were combined, washed with saturated brine, dried with anhydrous sodium sulfate, and concentrated under reduced pressure to obtain a crude product, and the crude product was purified by chromatographic columns (PE/EA = 10/1-3/1) to obtain a yellow solid product methyl 5-amino-3-((3-(cyclopropylcarbonyl)benzyl)oxy)isothiazol-4-carboxylate I371 (600 mg, 1.81 mmol, yield: 67.78%).

The product was verified by LCMS and H-NMR.

$^1$H NMR (400 MHz, $CDCl_3$) δ 8.16 (s, 1H), 7.96 (d, J = 8.0 Hz, 1H), 7.65 (d, J = 7.6 Hz, 1H), 7.49 (t, J = 8.0 Hz, 1H), 6.46 (s, 2H), 5.49 (s, 2H), 3.88 (s, 3H), 2.72-2.67 (m, 1H), 1.28-1.24 (m, 2H), 1.08-1.03 (m, 2H).

Step 4

Methyl 3-((3-(cyclopropanecarbonyl)benzyl)oxy)-5-(3-(3-(4-methylpiperazin-1-yl)propyl)ureido)isothiazole-4-carboxylate

3-(4-Methylpiperazin-1-yl)propyl-1-amine (198.7 mg, 1.26 mmol) was dissolved in anhydrous THF (5 mL) and then cooled to 0°C under the protection of $N_2$, followed by the addition of CDI (181.9 mg, 1.26 mmol), the mixture was stirred at room temperature to react for 1 hour, followed by the addition of DMSO (5 mL), the pressure was then reduced to remove most of the THF, followed by the addition of methyl 5-amino-3-((3-(cyclopropylcarbonyl)benzyl)oxy)isothiazol-4-carboxylate I371 (300 mg, 902.60 μmol) and potassium carbonate (374.2 mg, 2.71 mmol), and the reaction system was stirred at room temperature to react for 1 hour. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, the crude product was stirred in MTBE(30 mL) for 20 minutes and then filtered, and the filter cake was dried to obtain a yellow solid product methyl 3-((3-(cyclopropylcarbonyl)benzyl)oxy)-5-(3-(3-(4-methylpiperazin-1-yl)propyl)ureido)isothiazol-4-carboxylate I372 (1 g, 2.33 mmol, yield: 50.34%).

The product was verified by LCMS and H-NMR.

$^1$H NMR (400 MHz, $CDCl_3$) δ 10.37 (s, 1H), 8.17 (s, 1H), 7.96 (d, J = 7.6 Hz, 1H), 7.64 (d, J = 7.6 Hz, 1H), 7.48 (t, J = 7.6 Hz, 1H), 5.50 (s, 2H), 3.90 (s, 3H), 3.46-3.42 (m, 2H), 2.73-2.68 (m, 1H), 2.62-2.55 (m, 6H), 2.36 (s, 3H), 1.72-1.66 (m, 6H), 1.27-1.23 (m, 2H), 1.08-1.03 (m, 2H).

Step 5

3-((3-(cyclopropanecarbonyl) benzyl) oxy)-5-(3-(3-(4-methylpiperazin-1-yl) propyl) ureido) isothiazole-4-carboxamide

In a microwave tube, methyl 3-((3-(cyclopropylcarbonyl)benzyl)oxy)-5-(3-(3-(4-methylpiperazin-1-yl)propyl)ureido)isothiazol-4-carboxylate I372 (300 mg, 581.82 μmol) was dissolved in $NH_3$/MeOH (4 mL, 9 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((3-(cyclopropylcarbonyl)benzyl)oxy)-5-(3-(3-(4-methylpiperazin-1-yl)propyl)ureido)isothiazol-4-formamide T086 (46 mg, 91.89 μ mol, yield: 15.79%).

The product was verified by LCMS, H-NMR and C-NMR.

$^1$H NMR (400MHz, $CDCl_3$ ): δ (ppm) 11.10 (s, 1H), 8.11 (s, 1H), 8.04 (d, J = 7.6 Hz, 1H), 7.68 (d, J = 7.6 Hz, 1H), 7.64-7.46 (m, 2H), 7.12 (s, 1H), 5.56 (s, 3H), 3.47-3.43 (m, 2H), 2.73-2.67 (m, 1H), 2.62-2.41 (m, 6H), 2.35 (s, 3H), 1.81-1.76 (m, 2H), 1.64 (s, 4H), 1.27-1.30 (m, 2H), 1.12-1.07 (m, 1H).

$^{13}$C NMR (100 MHz, $CDCl_3$) δ 200.22, 169.65, 166.03, 161.67, 153.94, 138.51, 136.46, 132.64, 129.03, 128.25, 127.96, 97.18, 77.37, 77.05, 76.73, 69.98, 56.34, 54.96, 52.99, 45.90, 39.71, 29.70, 24.92, 17.29, 11.93.

Example 63: Synthesis of Compound T087

Synthesis Route:

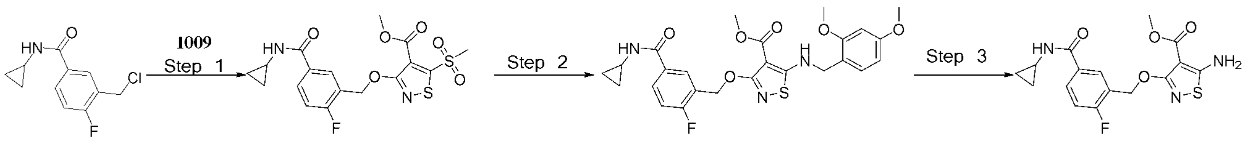


I373 I374 I375 I376

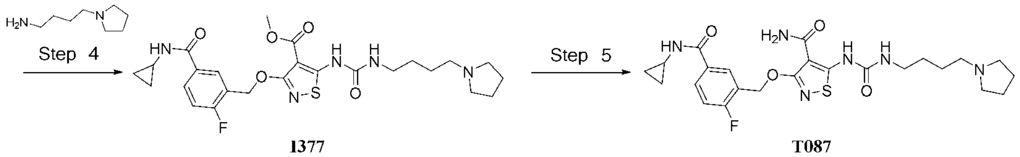


I377 T087

Step 1

Methyl3-[[5-(cyclopropylcarbamoyl)-2-fluoro-phenyl]methoxy]-5-methylsulfonyl-isothiazole-4-carboxylate

Methyl 3-hydroxyl-5-(methylsulfonyl)isothiazol-4-carboxylate I009 (2.1 g, 8.85 mmol) and potassium carbonate (3.7 g, 26.55 mmol) was dissolved in DMF (20 mL), followed by the addition of 3-(chloromethyl)-N-cyclopropyl-4-fluorobenzamide I373 (2 g, 8.85 mmol), and the reaction system was stirred at room temperature to react for 16 hours. Water (100 mL) was added to the reaction mixture, which was then filtered, and the filter cake was dissolved in ethyl acetate, sequentially washed with water and saturated brine, dried with anhydrous sodium sulfate, and concentrated under reduced pressure to obtain a yellow solid product methyl 3-[[5-(cyclopropylcarbamoyl)-2-fluoro-phenyl]methoxy]-5-methylsulfonylisothiazol-4-carboxylate

I374 (2.5 g, 5.83 mmol, yield: 65.92%).

The product was verified by LCMS and H-NMR.

$^1$H NMR (400 MHz, $CDCl_3$): δ (ppm) 8.03 (dd, J = 6.8 Hz, 2.4 Hz, 1H), 7.76-7.73 (m, 1H), 7.16-7.13 (m, 1H), 6.29 (s, 1H), 5.57 (s, 2H), 4.00 (s, 3H), 3.49 (s, 3H), 2.94-2.89 (m, 1H), 0.92-0.86 (m, 2H), 0.66-0.62 (m, 2H).

Step 2

Methyl3-[[5-(cyclopropylcarbamoyl)-2-fluoro-phenyl]methoxy]-5-[(2,4-dimethoxyphenyl) methylamino]isothiazole-4-carboxylate

Methyl 3-[[5-(cyclopropylcarbamoyl)-2-fluoro-phenyl]methoxy]-5-methylsulfonylisothiazol-4-carboxylate I374 (2.5 g, 5.74 mmol) was dissolved in THF(25 mL), followed by the addition of 2,4-dimethoxybenzylamine (9.6 g, 57.42 mmol), and the reaction system was stirred at 65 °C to react for 2 hours. After the reaction was finished, the reaction mixture was added to water (100 mL) and then filtered, and the filter cake was dissolved in ethyl acetate, sequentially washed with water and saturated brine, dried with anhydrous sodium sulfate, and concentrated under reduced pressure to obtain a yellow solid product methyl 3-[[5-(cyclopropylcarbamoyl)-2-fluorophenyl]methoxy]-5-[(2,4-dimethoxyphenyl)methylamino]isothiazol-4-carboxylate I375 (2 g, 3.88 mmol, yield: 67.57%).

The product was verified by LCMS and H-NMR.

$^1$H NMR (400MHz, $CDCl_3$): δ (ppm) 8.09-8.06 (m, 2H), 7.73-7.69 (m, 1H), 7.16 (d, J = 8.4 Hz, 1H), 7.09 (d, J = 9.2 Hz, 1H), 6.48-6.43 (m, 2H), 6.26 (s, 1H), 5.48 (s, 2H), 4.27 (d, J = 6.0 Hz, 2H), 3.87 (s, 3H), 3.85 (s, 3H), 3.81 (s, 3H), 2.92-2.88 (m, 1H), 0.90-0.86 (m, 2H), 0.64-0.60 (m, 2H).

Step 3

Methyl5-amino-3-[[5-(cyclopropylcarbamoyl)-2-fluoro-phenyl]methoxy]isothiazole-4-carboxylate

Methyl 3-[[5-(cyclopropylcarbamoyl)-2-fluoro-phenyl]methoxy]-5-[(2,4-dimethoxyphenyl)methylamino]isothiazol-4-carboxylate I375 (2 g, 3.88 mmol) was dissolved in DCM/$H_2O$ (60/10 mL), followed by the portionwise addition of DDQ (880.6 mg, 3.88 mmol) at 0°C, and the reaction system was stirred at room temperature to react for 1 hour. After complete reaction, water (50 mL) was added to the reaction mixture, which was then extracted with DCM (30 mL x2), the organic phases were combined, washed with saturated brine, dried with anhydrous sodium sulfate, and concentrated under reduced pressure to obtain a crude product, and the crude product was purified by chromatographic columns (neutral alumina, PE/EA = 1/0-1/1) to obtain a yellow solid product methyl 5-amino-3-[[5-(cyclopropylcarbamoyl)-2-fluorophenyl]methoxy]isothiazol-4-carboxylate I376 (1.16 g, 3.17 mmol, yield: 81.84%).

The product was verified by LCMS and H-NMR.

$^1H$ NMR (400MHz, $CD_3OD$): δ (ppm) 8.15 (d, J = 6.4 Hz, 1H), 7.80 (s, 1H), 7.18 (t, J = 8.8 Hz, 1H), 5.46 (s, 2H), 3.87 (d, J = 0.8 Hz, 3H), 2.88-2.85 (m, 1H), 0.86-0.81 (m, 2H), 0.67 (s, 2H).

Step 4

Methyl3-[[5-(cyclopropylcarbamoyl)-2-fluoro-phenyl]methoxy]-5-(4-pyrrolidin-1-ylbutyl carbamoylamino)isothiazole-4-carboxylate

4-(Pyrrolidin-1-yl)butyl-1-amine (292 mg, 2.05 mmol) was dissolved in anhydrous THF (10 mL) and then cooled to 0°C under the protection of $N_2$, followed by the addition of CDI (332.8 mg, 2.05 mmol), the mixture was stirred at room temperature to react for 1 hour, followed by the addition of DMSO (10 mL), the pressure was then reduced to remove most of the THF, followed by the addition of methyl 5-amino-3-[[5-(cyclopropylcarbamoyl)-2-fluorophenyl]methoxy]isothiazol-4-carboxylate I376 (500 mg, 1.37 mmol) and potassium carbonate (567.4 mg, 4.11 mmol), and the reaction system was stirred at room temperature to react for 1 hour. After complete reaction, water (50 mL) was added to the reaction mixture, which was then extracted with ethyl acetate (30 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[[5-(cyclopropylcarbamoyl)-2-fluoro-phenyl]methoxy]-5-(4-pyrrolidin-1-ylbutylcarbamoylamino)isothiazol-4-carboxylate I377 (500 mg, 937.01 μmol, yield: 68.47%). The product was verified by LCMS and H-NMR.

$^1H$NMR (400MHz, $CDCl_3$): δ (ppm) 8.11 (s, 1H), 7.72 (s, 1H), 7.13 (s, 1H), 6.37 (s, 1H), 5.52 (s, 2H), 3.95 (s, 3H), 3.33 (s, 2H), 2.94 (s, 1H), 2.65 (s, 4H), 2.58 (s, 2H), 1.93 (s, 4H), 1.71 (s, 4H), 1.29 (s, 2H), 0.92 (s, 2H), 0.67 (s, 2H).

Step 5

3-[[5-(cyclopropylcarbamoyl)-2-fluoro-phenyl]methoxy]-5-(4-pyrrolidin-1-ylbutylcarbamo ylamino)isothiazole-4-carboxamide

In a microwave tube, methyl 3-[[5-(cyclopropylcarbamoyl)-2-fluoro-phenyl]methoxy]-5-(4-pyrrolidin-1-ylbutylcarbamoylamino)isothiazol-4-carboxylate I377 (500 mg, 937.01 μmol) was dissolved in $NH_3$/MeOH (10 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a yellow solid product 3-[[5-(cyclopropylcarbamoyl)-2-fluorophenyl]methoxy]-5-(4-pyrrolidin-1-ylbutylcarbamoylamino)isothiazol-4-formamide T087 (166 mg, 320.09 μmol, yield: 34.16%).

The product was verified by LCMS, H-NMR, C-NMR and F-NMR.

$^1H$ NMR (400MHz, $CD_3OD$): δ (ppm) δ 8.02 (dd, J = 5.6, 1.6 Hz, 1H), 7.89-7.86 (m, 1H), 7.26 (t, J = 7.6 Hz, 1H), 5.59 (s, 2H), 3.27 (t, J = 5.6 Hz, 2H), 2.87-2.83 (m, 1H), 2.74 (s, 4H), 2.68-2.65 (m, 2H), 1.89-1.87 (m, 4H), 1.67-1.58 (m, 4H), 0.84-0.80 (m, 2H), 0.66-0.63 (m, 2H).

$^{13}C$ NMR (100MHz, $CD_3OD$) δ (ppm) 170.37, 170.20, 167.14, 165.69, 163.17, 162.97, 156.13, 131.58, 131.53, 131.17, 131.08, 124.92, 116.85, 116.62, 98.73, 65.17, 65.13, 57.09, 54.99, 40.93, 28.84, 26.76, 24.16, 24.10,6.57.

$^{19}F$ NMR (400MHz, $CD_3OD$): δ (ppm) -115.29

Example 64: Synthesis of Compound T088

Synthesis Route:

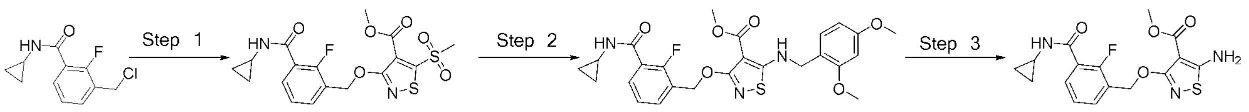


I378 I379 I380 I381

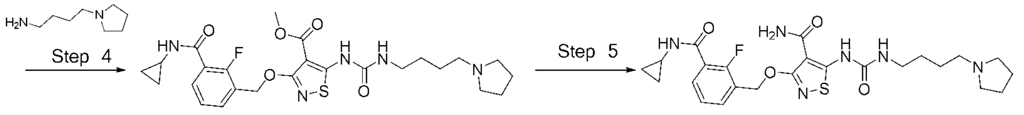


I382 T088

Step 1

Methyl 3-[[3-(cyclopropylcarbamoyl)-2-fluoro-phenyl] methoxy]-5-methylsulfonyl-isothiazole-4-carboxylate

Methyl 3-hydroxyl-5-(methylsulfonyl)isothiazol-4-carboxylate I009 (1 g, 4.21 mmol) and potassium carbonate (1.8 g, 12.64 mmol) was dissolved in DMF (10 mL), followed by the addition of 3-(chloromethyl)-N-cyclopropyl-2-fluoro-benzamide I378 (1.2 g, 5.06 mmol), and the reaction system was stirred at room temperature to react for 16 hours. Water (50 mL) was added to the reaction mixture, which was then filtered, and the filter cake was dissolved in ethyl acetate, sequentially washed with water and saturated brine, dried with anhydrous sodium sulfate, and concentrated under reduced pressure to obtain a yellow solid product methyl 3-[[3-(cyclopropylcarbamoyl)-2-fluoro-phenyl]methoxy]-5-methylsulfonylisothiazol-4-carboxylate

I379 (1.1 g, 2.57 mmol, yield: 60.91%).

The product was verified by LCMS and H-NMR.

$^1$H NMR (400MHz, $CDCl_3$): δ (ppm) 8.09 (t, J = 6.8 Hz, 1H), 7.65 (t, J = 6.8 Hz, 1H), 7.30 (d, J = 7.6 Hz, 1H), 6.78 (d, J = 11.6 Hz, 1H), 5.56 (s, 2H), 3.95 (s, 3H), 3.48 (s, 3H), 2.97- 2.92 (m, 1H), 0.87-0.93 (m, 2H), 0.61-0.66 (m, 1H).

Step 2

Methyl 3-[[3-(cyclopropylcarbamoyl)-2-fluoro-phenyl] methoxy]-5-[(2,4-dimethoxyphenyl) methylamino] isothiazole-4-carboxylate

Methyl 3-[[3-(cyclopropylcarbamoyl)-2-fluoro-phenyl]methoxy]-5-methylsulfonylisothiazol-4-carboxylate I379 (3 g, 7.85 mmol) was dissolved in THF(50 mL), followed by the addition of 2,4-dimethoxybenzylamine (13.1 g, 78.5 mmol), and the reaction system was stirred at 65 °C to react for 2.5 hours. After the reaction was finished, the reaction mixture was added to water (50 mL) and then filtered, and the filter cake was dried to obtain a yellow solid product methyl 3-[[3-(cyclopropylcarbamoyl)-2-fluoro-phenyl]methoxy]-5-[(2,4-dimethoxyphenyl)methylamino]isothiazol-4-carboxylate I380 (1 g, 1.94 mmol, yield: 75.55%). The product was verified by LCMS and H-NMR.

$^1$H NMR (400MHz, DMSO_$d_6$): δ (ppm) 8.44 (d, J = 4.4 Hz, 2H), 7.61 (s, 1H), 7.48 (s, 1H), 7.27 (t, J = 7.6 Hz, 1H), 7.16 (d, J = 8.4 Hz, 1H), 6.59 (d, J = 2.4 Hz, 1H), 6.53-6.46 (m, 1H), 5.39 (s, 2H), 4.28 (d, J = 6.0 Hz, 2H), 3.83 (s, 3H), 3.74 (d, J = 7.2 Hz, 3H), 2.83 (d, J = 4.0 Hz, 1H), 0.75-0.65 (m, 2H), 0.57-0.48 (m, 2H).

Step 3

Methyl 5-amino-3-[[3-(cyclopropylcarbamoyl)-2-fluoro-phenyl] methoxy] isothiazole-4-carboxylate

Methyl 3-[[3-(cyclopropylcarbamoyl)-2-fluoro-phenyl]methoxy]-5-[(2,4-dimethoxyphenyl)methylamino]isothiazol-4-carboxylate I380 (1 g, 1.94 mmol) was dissolved in DCM/$H_2O$ (5/1 mL), followed by the portionwise addition of DDQ (660.5 mg, 2.91 mmol) at 0°C, and the reaction system was stirred at room temperature to react for 1 hour. After complete reaction, water (20 mL) was added to the reaction mixture, which was then extracted with DCM (30 mL x3), the organic phases were combined, washed with saturated brine, dried with anhydrous sodium sulfate, and concentrated under reduced pressure to obtain a crude product, and the crude product was purified by chromatographic columns (PE/EA = 20/1-0/1) to obtain a white solid product methyl 5-amino-3-[[3-(cyclopropylcarbamoyl)-2-fluoro-phenyl]methoxy] isothiazol-4-carboxylate I381 (350 mg, 957.91 μ mol, yield:49.39%).

The product was verified by LCMS and H-NMR.

$^1$H NMR (400 MHz, $CDCl_3$) δ (ppm) 8.16 (s, 1H), 7.96 (d, J = 8.0 Hz, 1H), 7.65 (d, J = 7.6 Hz, 1H), 7.49 (t, J = 8.0 Hz, 1H), 6.46 (s, 2H), 5.49 (s, 2H), 3.88 (s, 3H), 2.72-2.67 (m, 1H), 1.28-1.24 (m, 2H), 1.08-1.03 (m, 2H).

Step 4

Methyl 3-[[3-(cyclopropylcarbamoyl)-2-fluoro-phenyl] methoxy]-5-(4-pyrrolidin-1-ylbutylcarbamoylamino) isothiazole-4-carboxylate

4-(Pyrrolidin-1-yl)butyl-1-amine (81.8 mg, 574.75 μmol) was dissolved in anhydrous THF (3 mL) and then cooled to 0°C under the protection of $N_2$, followed by the addition of CDI (93.2 mg, 574.75 μmol), the mixture was stirred at room temperature to react for 1 hour, followed by the addition of DMSO (3 mL), the pressure was then reduced to remove most of the THF, followed by the addition of methyl 5-amino-3-[[3-(cyclopropylcarbamoyl)-2-fluorophenyl]methoxy]isothiazol-4-carboxylate I381 (150 mg, 410.53 μmol) and potassium carbonate (170.2 mg, 1.23 mmol), and the reaction system was stirred at room temperature to react for 1 hour. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[[3-(cyclopropylcarbamoyl)-2-fluoro-phenyl]methoxy]-5-(4-pyrrolidin-1-ylbutylcarbamoylamino)isothiazol-4-carboxylate I382 (80 mg, 149.92 μ mol, yield: 36.52%).

The product was verified by LCMS and H-NMR.

$^1H$ NMR (400MHz, $CDCl_3$ ): δ (ppm) 8.10-8.03 (m, 1H), 7.72 (t, J = 6.4 Hz, 1H), 6.85 (d, J = 11.8 Hz, 1H), 5.52 (s, 2H), 3.88 (s, 3H), 3.33 (s, 2H), 2.99-2.93 (m, 1H), 2.59 (s, 3H), 2.53 (t, J = 6.0 Hz, 1H), 1.89 (s, 4H), 1.80-1.68 (m, 6H), 0.92-0.87 (m, 2H), 0.67-0.62 (m, 2H).

Step 5

3-[[3-(cyclopropylcarbamoyl)-2-fluoro-phenyl] methoxy]-5-(4-pyrrolidin-1-ylbutylcarbamoylamino) isothiazole-4-carboxamide

In a microwave tube, methyl 3-[[3-(cyclopropylcarbamoyl)-2-fluoro-phenyl]methoxy]-5-(4-pyrrolidin-1-ylbutylcarbamoylamino)isothiazol-4-carboxylate I382 (200 mg, 374.80 μmol) was dissolved in $NH_3$/MeOH (5 mL,10 mol/L), and the mixture was sealed to react at 40°C for 20 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[[3-(cyclopropylcarbamoyl)-2-fluorophenyl]methoxy]-5-(4-pyrrolidin-1-ylbutylcarbamoylamino)isothiazol-4-formamide T088 (4 mg, 7.71 μ mol, yield: 2.06%).

The product was verified by LCMS, H-NMR and C-NMR.

$^1H$ NMR (400MHz, $CDCl_3$ ): δ (ppm) 11.10 (s, 1H), 8.11 (s, 1H), 8.04 (d, J = 7.6 Hz, 1H), 7.68 (d, J = 7.6 Hz, 1H), 7.46-7.64 (m, 2H), 7.12 (s, 1H), 5.56 (s, 3H), 3.43-3.47 (m, 2H), 2.67-2.73 (m, 1H), 2.41-2.62 (m, 6H), 2.35 (s, 3H), 1.76-1.81 (m, 2H), 1.64 (s, 4H), 1.27-1.30 (m, 2H), 1.07-1.12 (m, 1H).

$^{13}C$ NMR (100 MHz, $CDCl_3$) δ 200.22, 169.65, 166.03, 161.67 (s, 3H), 153.94, 138.51, 136.46, 132.64, 129.03, 128.25, 127.96 (s, 5H), 97.18, 77.37, 77.05, 76.73, 69.98, 56.34, 54.96, 52.99, 45.90, 39.71, 29.70, 24.92, 17.29, 11.93, 1.02.

Example 65: Synthesis of Compound T089

Synthesis Route:

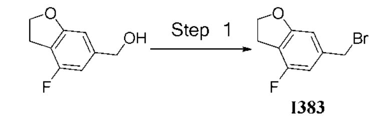


I383

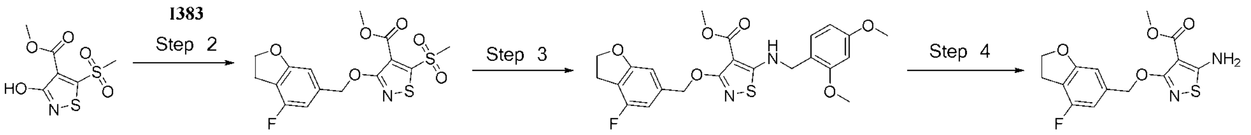


I009 I384 I385 I386

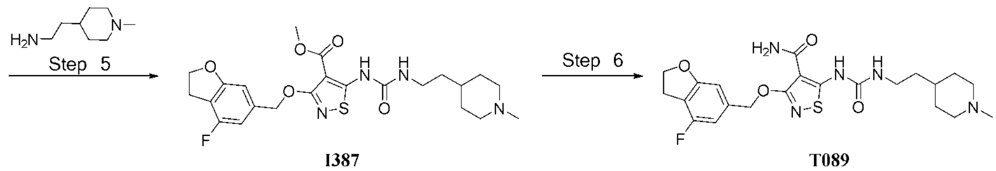


I387 T089

Step 1

6-(bromomethyl)-4-fluoro-2,3-dihydrobenzofuran

(4-Fluoro-2,3-dihydrobenzofuran-6-yl)methanol (1 g, 5.95 mmol) was dissolved in DCM (10 mL) and then cooled to 0°C, followed by the addition of $PBr_3$ (1.6 g, 5.95 mmol), and the reaction system was stirred at room temperature to react for 1 hour. After complete reaction, the reaction mixture was added to water (20 mL) and extracted with dichloromethane (10 mL x 3), and the organic phases were combined, sequentially washed with water and saturated brine, dried with anhydrous sodium sulfate, and directly concentrated under reduced pressure to obtain a yellow oily liquid product 6-(bromomethyl)-4-fluoro-2,3-dihydrobenzofuran I383 (1.1 g, 4.76 mmol, yield: 80.06%).
The product was verified by LCMS and H-NMR.
$^1H$ NMR (400MHz, $CDCl_3$): δ (ppm) 6.62-6.60 (m, 2H), 4.63 (t, J = 8.7 Hz, 2H), 4.39 (s, 2H), 3.23 (t, J = 8.7 Hz, 2H).

Step 2

Methyl 3-((4-fluoro-2,3-dihydrobenzofuran-6-yl) methoxy)-5-(methylsulfonyl) isothiazole-4-carboxylate

Methyl 3-hydroxyl-5-(methylsulfonyl)isothiazol-4-carboxylate I009 (940 mg, 3.96 mmol) and potassium carbonate (2.3 g, 16.80 mmol) were dissolved in DMF (3 mL), followed by the addition of 6-(bromomethyl)-4-fluoro-2,3-dihydrobenzofuran I383 (1.6 g, 11.89 mmol), and the reaction system was stirred at room temperature to react for 1 hour. Water (50 mL) was added to the reaction mixture, which was filtered, and the filter cake was dried to obtain a yellow solid product methyl 3-((4-fluoro-2,3-dihydrobenzofuran-6-yl)methoxy)-5-(methylsulfonyl)isothiazol-4-carboxylate I384 (1 g, 2.58 mmol, yield: 65.15%).
The product was verified by LCMS and H-NMR.
$^1H$ NMR (400MHz, $CDCl_3$): δ (ppm) 6.67-6.62 (m, 2H), 5.40 (s, 2H), 4.64 (t, J = 8.7 Hz, 2H), 3.97 (s, 3H), 3.48 (s, 3H), 3.24 (t, J = 8.7 Hz, 2H).

Step 3

Methyl 5-((2,4-dimethoxybenzyl) amino)-3-((4-fluoro-2,3-dihydrobenzofuran-6-yl) methoxy) isothiazole-4-carboxylate

Methyl 3-((4-fluoro-2,3-dihydrobenzofuran-6-yl)methoxy)-5-(methylsulfonyl)isothiazol-4-carboxylate I384 (1.2 g, 3.10 mmol) was dissolved in THF(20 mL), followed by the addition of 2,4-dimethoxybenzylamine (5.2 g, 30.98 mmol), and the reaction system was stirred at 65 °C to react for 1 hours. After the reaction was finished, the reaction mixture was added to water (40 mL) and then filtered, and the filter cake was dried to obtain a white solid product methyl 5-((2,4-dimethoxybenzyl)amino)-3-((4-fluoro-2,3-dihydrobenzofuran-6-yl)methoxy)isothiazol-4-carboxylate I385 (1.2 g, 2.53 mmol, yield: 81.64%).
The product was verified by LCMS and H-NMR.
$^1H$ NMR (400MHz, $CDCl_3$ ): δ (ppm) 7.16 (d, J = 8.1 Hz, 1H), 6.68 (s, 1H), 6.47-6.42 (m, 3H), 5.30 (s, 2H), 4.62 (t, J = 8.7 Hz, 2H), 4.26 (d, J = 6.0 Hz, 2H), 3.84 (s, 3H), 3.83 (s, 3H), 3.80 (s, 3H), 3.22 (t, J = 8.7 Hz, 2H).

Step 4

Methyl 5-amino-3-((4-fluoro-2,3-dihydrobenzofuran-6-yl) methoxy) isothiazole-4-carboxylate

Methyl 5-((2,4-dimethoxybenzyl)amino)-3-((4-fluoro-2,3-dihydrobenzofuran-6-yl)methoxy)isothiazol-4-carboxylate I385 (1 g, 2.11 mmol) was dissolved in DCM/$H_2O$ (10/2 mL), followed by the portionwise addition of DDQ (478.4 mg, 2.11 mmol) at 0°C, and the reaction system was stirred at room temperature to react for 0.5 hours, followed by the addition of DDQ (47.8 mg, 0.21 mmol), to allow for reaction for 1 hour. After complete reaction, water (50 mL) was added to the reaction mixture, which was then extracted with DCM (20 mL x3), the organic phases were combined, washed with saturated brine, dried with anhydrous sodium sulfate, and concentrated under reduced pressure to obtain a crude product, and the crude product was purified by chromatographic columns (neutral alumina, PE/EA = 10/1-5/1) to obtain a red solid product methyl 5-amino-3-((4-fluoro-2,3-dihydrobenzofuran-6-yl)methoxy)isothiazol-4-carboxylate I386 (180 mg, 555.00 $\mu$mol, yield: 26.33%).

The product was verified by LCMS and H-NMR.

$^1$H NMR (400MHz, $CDCl_3$): $\delta$ (ppm) 6.70 (d, J = 7.6 Hz, 1H), 6.43 (s, 1H), 5.35 (s, 2H), 4.63 (t, J = 8.7 Hz, 1H), 3.87 (s, 3H), 3.23 (t, J = 8.7 Hz, 2H).

Step 5

Methyl 3-[(4-fluoro-2,3-dihydrobenzofuran-6-yl) methoxy]-5-[2-(1-methyl-4-piperidyl) ethylcarbamoylamino] isothiazole-4-carboxylate

2-(1-Methyl-4-piperidyl)ethylamine (675.4 mg, 4.75 mmol) was dissolved in anhydrous THF (10 mL) and then cooled to 0°C under the protection of $N_2$, followed by the addition of CDI (683.5 mg, 4.22 mmol), the mixture was stirred at room temperature to react for 1 hour, followed by the addition of DMSO (10 mL), the pressure was then reduced to remove most of the THF, followed by the addition of methyl 5-amino-3-((4-fluoro-2,3-dihydrobenzofuran-6-yl)methoxy)isothiazol-4-carboxylate I386 (1.1 g, 3.39 mmol) and potassium carbonate (1.4 g, 10.17 mmol), and the reaction system was stirred at room temperature to react for 1 hour. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(4-fluoro-2,3-dihydrobenzofuran-6-yl)methoxy]-5-[2-(1-methyl-4-piperidyl)ethylcarbamoylamino]isothiazol-4-carboxylate I387 (180 mg, 365.44 $\mu$mol, yield: 10.77%).

The product was verified by LCMS and H-NMR.

$^1$H NMR (400MHz, $CDCl_3$ ): $\delta$ (ppm)10.32 (s, 1H), 7.69 (s, 1H), 7.11 (s, 2H), 6.69 (d, J = 6.0 Hz, 2H), 5.35 (s, 2H), 4.63 (t, J = 8.7 Hz, 2H), 3.89 (s, 3H), 3.35 (dd, J = 13.5 Hz, 6.7 Hz, 2H), 3.23 (t, J = 8.7 Hz, 2H), 2.86 (d, J = 11.3 Hz, 2H), 2.27 (s, 3H), 1.93 (s, 2H), 1.29 (d, J = 28.1 Hz, 9H).

Step 6

3-((4-fluoro-2,3-dihydrobenzofuran-6-yl) methoxy)-5-(3-(2-(1-methylpiperidin-4-yl) ethyl) ureido) isothiazole-4-carboxamide

In a microwave tube, methyl 3-[(4-fluoro-2,3-dihydrobenzofuran-6-yl)methoxy]-5-[2-(1-methyl-4-piperidyl)ethylcarbamoylamino]isothiazol-4-carboxylate I387 (200 mg, 406.04 $\mu$mol) was dissolved in $NH_3$/MeOH (4 mL,10 mol/L), and the mixture was sealed to react at 40 °C for 16 hours. The reaction mixture was concentrated under reduced pressure to obtain a crude product, and the crude product was purified by the column chromatography (DCM/MeOH = 10/1) to obtain a white solid product 3-((4-fluoro-2,3-dihydrobenzofuran-6-yl)methoxy)-5-(3-(2-(1-methylpiperidin-4-yl)ethyl)ureido)isothiazol-4-formamide T089 (10 mg, 20.94 $\mu$mol, yield: 5.16%).

The product was verified by LCMS, H-NMR, C-NMR and F-NMR.

$^1$H NMR (400MHz, $CDCl_3$ ): $\delta$ (ppm) 11.05 (s, 1H), 7.16 (s, 1H), 6.65-6.24 (m, 2H), 5.43 (s, 1H), 5.37 (s, 2H), 5.32 (s, 1H), 4.65 (t, J = 8.7 Hz, 2H), 3.37-3.33 (m, 2H), 3.25 (t, J = 8.8 Hz, 2H), 2.85 (d, J = 11.0 Hz, 2H), 2.26 (s, 3H), 1.91-1.85 (m, 2H), 1.73-1.66 (m, 2H), 1.56-1.47 (m, 2H), 1.37-1.27 (m, 3H).

$^{13}$C NMR (100 MHz, $CDCl_3$) $\delta$ (ppm) 169.46, 165.73, 161.64, 160.45, 153.87, 138.28, 113.80, 113.57, 107.36, 105.06, 72.23, 69.85, 55.72, 46.33, 36.35, 32.63, 32.05, 26.53

$^{19}$F NMR (376 MHz, $CDCl_3$) $\delta$ (ppm) -116.31

Example 66: Synthesis of Compound T090

Synthesis Route:

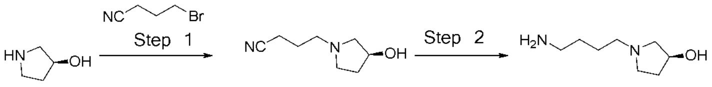


1388 1389

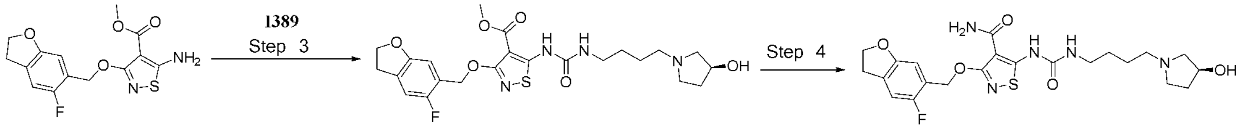


1311 1390 T090

Step 1

4-[(3S)-3-hydroxypyrrolidin-1-yl] butanenitrile

(3S)-pyrrolidin-3-ol (3 g, 34.44 mmol) was dissolved in acetonitrile (40 mL), followed by the addition of 4-bromobutyronitrile (4.6 g, 31.30 mmol) and potassium carbonate (13 g, 93.91 mmol), and the reaction system was stirred at 30 °C to react for 3 hours. The reaction mixture was poured into water (100 mL), and extracted with ethyl acetate (60 mL x 3), and the organic phases were combined, washed with saturated brine, dried with anhydrous sodium sulfate, and concentrated under reduced pressure to obtain a red oily liquid product 4-[(3S)-3-hydroxylpyrrolidin-1-yl]butyronitrile I388 (3 g, 19.45 mmol, yield: 62.14%).

The product was verified by LCMS and HNMR.

$^1$H NMR (400 MHz, $CD_3OD$): δ (ppm) 4.38-4.26 (m, 1H), 2.83-2.68 (m, 2H), 2.62-2.45 (m, 6H), 2.18-2.05 (m, 1H), 1.87-1.76 m, 2H), 1.76-1.64 (m, 1H).

Step 2

(3S)-1-(4-aminobutyl) pyrrolidin-3-ol

4-[(3S)-3-hydroxylpyrrolidin-1-yl]butyronitrile I388 (1 g, 6.48 mmol) was dissolved in methanol (15 mL), followed by the addition of raney nickel (418.7 mg, 7.13 mmol), and in the atmosphere of hydrogen (20 psi), the reaction system was stirred at 25°C to react for 16 hours. The reaction mixture was filtered, and the filtrate was concentrated under reduced pressure to obtain a yellow oily liquid product (3S)-1-(4-aminobutyl)pyrrolidin-3-ol I389 (1 g, 6.32 mmol, yield: 97.45%). The product was verified by LCMS and H-NMR.

$^1$H NMR (400MHz, $CDCl_3$): δ (ppm) δ 4.35 (s, 1H), 2.80-2.66 (m, 4H), 2.60-2.45 (m, 4H), 2.17-2.06 (m, 1H), 1.77-1.67 (m, 1H), 1.64-1.48 (s, 4H).

Step 3

Methyl 3-[(5-fluoro-2,3-dihydrobenzofuran-6-yl) methoxy]-5-[4-[(3S)-3-hydroxypyrrolidin-1-yl] butylcarbamoylamino] isothiazole-4-carboxylate

(3S)-1-(4-aminobutyl)pyrrolidin-3-ol I389 (205 mg, 1.29 mmol) was dissolved in anhydrous THF (5 mL) and then cooled to 0°C under the protection of $N_2$, followed by the addition of CDI (210 mg, 1.29 mmol), the mixture was stirred at room temperature to react for 1 hour, followed by the addition of DMSO (5 mL), the pressure was then reduced to remove most of the THF, followed by the addition of methyl 5-amino-3-[(5-fluoro-2,3-dihydrobenzofuran-6-yl)methoxy]isothiazol-4-carboxylate I311 (300 mg, 924.99 μmol) and potassium carbonate (383.5 mg, 2.77 mmol), and the reaction system was stirred at room temperature to react for 1 hour. After complete reaction, water (30 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(5-fluoro-2,3-dihydrobenzofuran-6-yl)methoxy]-5-[4-[(3S)-3-hydroxylpyrrolidin-1-yl]butylaminoacylamino]isothiazol-4-carboxylate I390 (180 mg, 353.94 μmol, ield: 38.26%y).

The product was verified by LCMS and H-NMR.

$^1H$ NMR (400MHz, $CDCl_3$): δ (ppm) δ 10.03 (s, 1H), 8.58 (s, 1H), 6.95 (d, J = 5.6 Hz, 1H), 6.90 (d, J = 9.2 Hz, 1H), 5.42 (s, 3H), 4.57 (t, J = 9.2 Hz, 2H), 3.87 (s, 3H), 3.49 (s, 3H), 3.20 (t, J = 8.8 Hz, 4H), 3.04 (d, J = 10.4 Hz, 1H), 2.70-2.15 (m, 8H), 1.95 (s, 1H).

Step 4

3-[(5-fluoro-2,3-dihydrobenzofuran-6-yl) methoxy]-5-[4-[(3S)-3-hydroxypyrrolidin-1-yl] butylcarbamoylamino] isothiazole-4-carboxamide

In a microwave tube, methyl 3-[(5-fluoro-2,3-dihydrobenzofuran-6-yl)methoxy]-5-[4-[(3S)-3-hydroxylpyrrolidin-1-yl]butylaminoacylamino]isothiazol-4-carboxylate I390 (180 mg, 353.94 μmol) was dissolved in $NH_3$/MeOH (5 mL, 10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(5-fluoro-2,3-dihydrobenzofuran-6-yl) methoxy]-5-[4-[(3S)-3-hydroxylpyrrolidin-1-yl]butyrylamino]isothiazol-4-formamide T090 (27 mg, 54.71 μmol, yield: 15.46%).

The product was verified by LCMS, H-NMR, C-NMR and F-NMR.

$^1H$ NMR (400MHz, $CDCl_3$&$CD_3OD$): δ (ppm) 7.01 (d, J = 9.6 Hz, 1H), 6.82 (d, J = 5.6 Hz, 1H), 5.43 (s, 2H), 4.55 (t, J = 8.8 Hz, 2H), 4.39 (s, 1H), 3.27-3.18 (m, 4H), 2.98-2.88 (m, 2H), 2.79-2.61 (m, 4H), 2.19-2.10 (m, 1H), 1.79 (s, 1H), 1.60 (s, 4H).

$^{13}C$ NMR (100MHz, $CDCl_3$&$CD_3OD$): δ (ppm) 165.79, 158.67, 116.23, 113.87, 75.54, 73.83, 68.40, 66.10, 59.89, 56.48, 43.53, 37.65, 33.67, 31.30, 28.90.

$^{19}F$ NMR (100MHz, $CDCl_3$&$CD_3OD$): δ (ppm) 125.65.

Example 67: Synthesis of Compound T091

Synthesis Route:

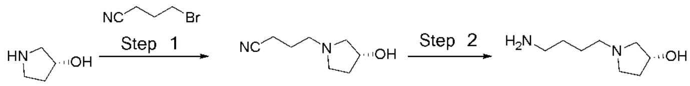


I391 I392

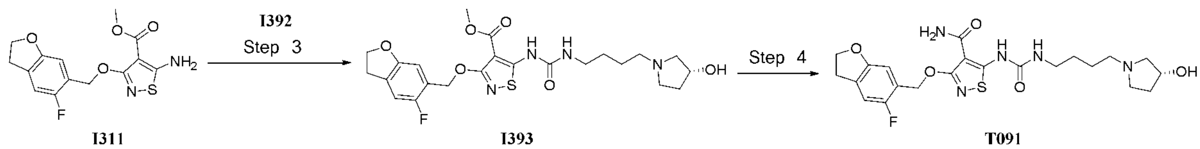


I311 I393 T091

Step 1

4-[(3R)-3-hydroxypyrrolidin-1-yl] butanenitrile

(3R)-pyrrolidin-3-ol (3 g, 34.44 mmol) was dissolved in acetonitrile (40 mL), followed by the addition of 4-bromobutyronitrile (4.6 g, 31.30 mmol) and potassium carbonate (13 g, 93.91 mmol), and the reaction system was stirred at 30 °C to react for 3 hours. The reaction mixture was poured into water (100 mL), and extracted with ethyl acetate (60 mL x 3), and the organic phases were combined, washed with saturated brine, dried with anhydrous sodium sulfate, and concentrated under reduced pressure to obtain a red oily liquid product 4-[(3R)-3-hydroxylpyrrolidin-1-yl]butyronitrile I391 (2.5 g, 16.21 mmol, yield: 51.79%).

The product was verified by LCMS and HNMR.

$^1H$ NMR (400MHz, $CD_3OD$): δ (ppm) 4.39-4.26 (m, 1H), 2.83-2.70 (m, 2H), 2.61-2.46 (m, 6H), 2.20-2.05 (m, 1H), 1.88-1.77 (m, 2H), 1.76-1.65 (m, 1H).

Step 2

(3R)-1-(4-aminobutyl) pyrrolidin-3-ol

4-[(3R)-3-hydroxylpyrrolidin-1-yl]butyronitrile I391 (500 mg, 3.24 mmol) was dissolved in methanol (3 mL), followed by the addition of raney nickel (190.3 mg, 3.24 mmol) and $NH_3.H_2O$ (0.2 mL), and in the atmosphere of hydrogen (20 psi), the reaction system was stirred at 25°C to react for 16 hours. The reaction mixture was filtered, and the filtrate was concentrated under reduced pressure to obtain a yellow oily liquid product (3R)-1-(4-aminobutyl)pyrrolidin-3-ol I392 (400 mg, 2.53 mmol, yield: 77.96%).

The product was verified by LCMS and H-NMR.

$^1$H NMR (400MHz, $CD_3OD$): δ (ppm) 4.34 (s, 1H), 2.87-2.62 (m, 4H), 2.59-2.36 (m, 4H), 2.18-2.04 (m, 1H), 1.78-1.63 (m, 1H), 1.62-1.42 (m, 4H).

Step 3

Methyl 3-[(5-fluoro-2,3-dihydrobenzofuran-6-yl) methoxy]-5-[4-[(3R)-3-hydroxypyrrolidin-1-yl] butylcarbamoylamino] isothiazole-4-carboxylate

(3R)-1-(4-aminobutyl)pyrrolidin-3-ol I392 (273.2 mg, 1.73 mmol) was dissolved in anhydrous THF (10 mL) and then cooled to 0°C under the protection of $N_2$, followed by the addition of CDI (248.6 mg, 1.53 mmol), the mixture was stirred at room temperature to react for 1 hour, followed by the addition of DMSO (10 mL), the pressure was then reduced to remove most of the THF, followed by the addition of methyl 5-amino-3-[(5-fluoro-2,3-dihydrobenzofuran-6-yl)methoxy] isothiazol-4-carboxylate I311 (400 mg, 1.23 m mol) and potassium carbonate (511.4 mg, 3.70 mmol), and the reaction system was stirred at room temperature to react for 1 hour. After complete reaction, water (30 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(5-fluoro-2,3-dihydrobenzofuran-6-yl)methoxy]-5-[4-[(3R)-3-hydroxylpyrrolidin-1-yl]butylaminoacylamino]isothiazol-4-carboxylate I393 (360 mg, 707.88 μ mol, yield: 57.40%). The product was verified by LCMS and H-NMR.

$^1$H NMR (400MHz, $CDCl_3$): δ (ppm) 6.97 (d, J = 9.2 Hz, 1H), 6.89 (d, J = 6.0 Hz, 1H), 5.33 (s, 2H), 4.55 (t, J = 8.4 Hz, 1H), 4.38 (s, 1H), 3.86 (s, 3H), 3.34 (s, 2H), 3.26 (t, J = 6.0 Hz, 2H), 3.20 (t, J = 8.4 Hz, 2H), 2.95-2.84 (m, 2H), 2.76-2.57 (m, 4H), 2.19-2.11 (m, 1H), 1.81 -1.73 (m, 1H), 1.59 (s, 4H).

Step 4

3-[(5-fluoro-2,3-dihydrobenzofuran-6-yl) methoxy]-5-[4-[(3R)-3-hydroxypyrrolidin-1-yl] butylcarbamoylamino] isothiazole-4-carboxamide

In a microwave tube, methyl 3-[(5-fluoro-2,3-dihydrobenzofuran-6-yl)methoxy]-5-[4-[(3R)-3-hydroxylpyrrolidin-1-yl]butylaminoacylamino]isothiazol-4-carboxylate I393 (200 mg, 353.94 μmol) was dissolved in $NH_3$/MeOH (5 mL, 10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(5-fluoro-2,3-dihydrobenzofuran-6-yl) methoxy]-5-[4-[(3R)-3-hydroxylpyrrolidin-1-yl]butyrylamino]isothiazol-4-formamide T091 (32 mg, 64.84 μ mol, yield: 16.49%).

The product was verified by LCMS, H-NMR, C-NMR and F-NMR.

$^1$H NMR (400 MHz, $CDCl_3$) δ (ppm) δ 10.56 (s, 1H), 8.59 (s, 2H), 6.94 (d, J = 8.8 Hz, 1H), 6.81 (d, J = 5.6 Hz, 1H), 5.44 (s, 2H), 5.32 (s, 1H), 4.58 (t, J = 8.8 Hz, 2H), 4.49 (s, 1H), 3.52 (s, 2H), 3.21 (t, J = 8.8 Hz, 4H), 3.06 (d, J = 10.4 Hz, 2H), 2.67 (s, 2H), 2.47 (d, J = 48.0 Hz, 3H), 2.21 (s, 2H), 1.97-1.92 (m, 1H).

$^{13}$C NMR (100 MHz, $CDCl_3$) δ (ppm) δ 169.65, 165.76, 161.59, 156.82, 156.02, 154.43, 154.24, 129.22, 129.13, 121.95, 121.78, 112.42, 112.17, 110.17, 110.14, 97.09, 70.77, 64.56, 64.52, 63.04, 55.42, 51.79, 40.34, 33.93, 29.96, 29.68, 28.21, 26.37.

$^{19}$F NMR (400 MHz, $CDCl_3$) δ (ppm) δ -128.65.

Example 68: Synthesis of Compound T092

Synthesis Route:

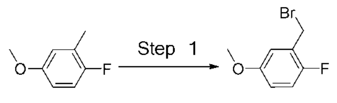


I394

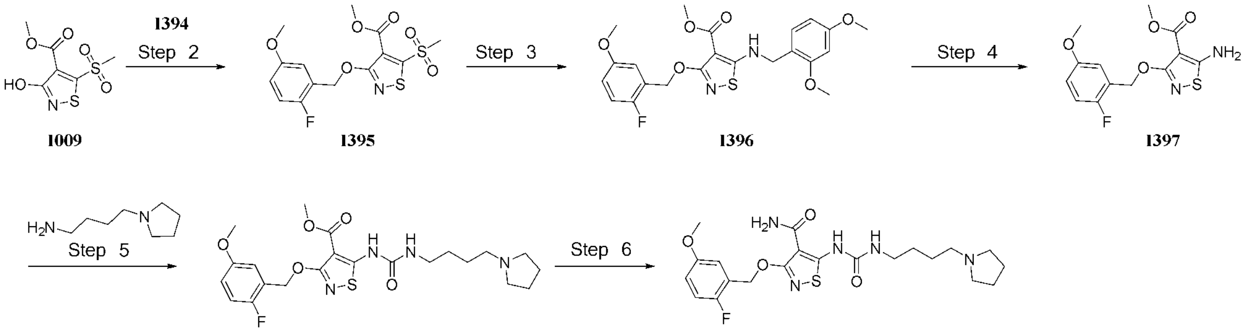


I009 I395 I396 I397

I398 T092

Step 1

2-(bromomethyl)-1-fluoro-4-methoxy-benzene

1-Fluoro-4-methoxy-2-methyl-benzene (8 g, 57.08 mmol) was dissolved in $CCl_4$ (5 mL), followed by the addition of AIBN (9.4 g, 57.08 mmol) and NBS (10.2 g, 57.08 mmol) at room temperature, and the mixture was stirred at 70°C to react for 3 hours. After complete reaction, the mixture was poured into water, and extracted with ethyl acetate, the organic phases were combined, washed with water and saturated brine, dried with anhydrous sodium sulfate, and concentrated under reduced pressure to obtain a crude product, and the crude product was purified by the flash column chromatography (PE/EA = 1/0-10/1) to obtain a yellow solid product 2-(bromomethyl)-1-fluoro-4-metoxybenzene I394 (10 g, 45.65 mmol, yield: 79.98%).
The product was verified by LCMS and H-NMR.
$^1$H NMR (400MHz, $CDCl_3$): δ (ppm) 6.98-6.94 (m, 1H), 6.90-6.83 (m, 1H), 6.81-6.79 (m, 1H), 4.48 (s, 2H), 3.79 (s, 3H).

Step 2

Methyl 3-[(2-fluoro-5-methoxy-phenyl)methoxy]-5-methylsulfonyl-isothiazole-4-carboxylate

Methyl 3-hydroxyl-5-(methylsulfonyl)isothiazol-4-carboxylate I009 (1.4 g, 5.90 mmol) and potassium carbonate (2.5 g, 17.70 mmol) were dissolved in DMF (20 mL), followed by the addition of 2-(bromomethyl)-1-fluoro-4-metoxybenzene I394 (1.6 g, 7.08 mmol), and the reaction system was stirred at room temperature to react for 2 hours. Water (150 mL) was added to the reaction mixture, which was then filtered to obtain a filter cake, and the filter cake was dried to obtain a white solid product methyl 3-[(2-fluoro-5-methoxy-phenyl)methoxy]-5-methylsulfonylisothiazol-4-carboxylate I395 (1.2 g, 3.20 mmol, yield: 54.17%).
The product was verified by LCMS and H-NMR.
$^1$H NMR (400MHz, $CDCl_3$): δ (ppm) 7.07-6.96 (m, 2H), 6.88-6.78 (m, 1H), 5.52 (s, 2H), 3.96 (s, 3H), 3.80 (s, 3H), 3.48 (s, 3H).

Step 3

Methyl 5-[(2,4-dimethoxyphenyl) methylamino]-3-[(2-fluoro-5-methoxy-phenyl) methoxy] isothiazole-4-carboxylate

Methyl 3-[(2-fluoro-5-methoxy-phenyl)methoxy]-5-methylsulfonylisothiazol-4-carboxylate I395 (1.2 g, 3.20 mmol) was dissolved in THF(5 mL), followed by the addition of 2,4-dimethoxybenzylamine (5.3 g, 31.97 mmol), and the reaction system was stirred at 65 °C to react for 1 hour. After the reaction was finished, the reaction mixture was added to water and filtered to obtain a filter cake, which was then dried to obtain a white solid product methyl 5-[(2,4-dimethoxyphenyl)methylamino]-3-[(2-fluoro-5-methoxy-phenyl)methoxy]isothiazol-4-carboxylate I396 (1.3 g, 2.81 mmol, yield: 87.93%).

The product was verified by LCMS and H-NMR.

$^1$H NMR (400MHz, $CDCl_3$): δ (ppm) 8.13 (s, 1H), 7.19-7.08 (m, 2H), 6.96 (t, J = 8.8 Hz, 1H), 6.90-6.74 (m, 1H), 6.52-6.41 (m, 2H), 5.50 (s, 2H), 4.29 (d, J = 6.0 Hz, 1H), 3.92-3.78 (m, 12H).

Step 4

Methyl 5-amino-3-[(2-fluoro-5-methoxy-phenyl)methoxy]isothiazole-4-carboxylate

Methyl 5-[(2,4-dimethoxyphenyl)methylamino]-3-[(2-fluoro-5-methoxyphenyl)methoxy]isothiazol-4-carboxylate I396 (1.3 g, 2.81 mmol) was dissolved in DCM/$H_2O$ (20/4 mL), followed by the portionwise addition of DDQ (1.3 g, 5.62 mmol) at 0°C, and the reaction system was stirred at room temperature to react for 1 hour. After complete reaction, water (30 mL) was added to the reaction mixture, which was then extracted with DCM (20 mL x2), the organic phases were combined, washed with saturated brine, dried with anhydrous sodium sulfate, and concentrated under reduced pressure to obtain a crude product, and the crude product was purified by chromatographic columns (PE/EA = 20/1-0/1) to obtain a yellow solid product methyl 5-amino-3-[(2-fluoro-5-methoxy-phenyl)methoxy]isothiazol-4-carboxylate I397 (800 mg, 2.56 mmol, yield: 91.13%).

The product was verified by LCMS and H-NMR.

$^1$H NMR (400MHz, $CDCl_3$): δ (ppm) 7.16-7.11 (m, 1H), 6.98 (t, J = 9.2 Hz, 1H), 6.82-6.75 (m, 1H), 6.44 (s, 2H), 5.47 (s, 2H), 3.85 (s, 3H), 3.80 (s, 3H).

Step 5

Methyl 3-[(2-fluoro-5-methoxy-phenyl)methoxy]-5-(4-pyrrolidin-1-ylbutylcarbamoylamino)isothiazole-4-carboxylate

4-(Pyrrolidin-1-yl)butyl-1-amine (251 mg, 1.79 mmol) was dissolved in anhydrous THF (4 mL) and then cooled to 0°C under the protection of $N_2$, followed by the addition of CDI (164 mg, 1.14 mmol), the mixture was stirred at room temperature to react for 1 hour, followed by the addition of DMSO (4 mL), the pressure was then reduced to remove most of the THF, followed by the addition of methyl 5-amino-3-[(2-fluoro-5-methoxy-phenyl)methoxy]isothiazol-4-carboxylate I397 (400 mg, 1.28 mmol) and potassium carbonate (531 mg, 3.84 mmol), and the reaction system was stirred at room temperature to react for 1 hour. After complete reaction, water (10 mL) was added to the reaction mixture, which was then extracted with ethyl acetate (10 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[(2-fluoro-5-methoxy-phenyl)methoxy]-5-(4-pyrrolidin-1-ylbutylcarbamate)isothiazol-4-carboxylate I398 (200 mg, 416.19 μ mol, yield: 32.50%).

The product was verified by LCMS and H-NMR.

$^1$H NMR (400MHz, $CD_3OD$): δ (ppm) 7.17-7.13 (m, 1H), 7.02 (t, J = 9.2 Hz, 1H), 6.89-6.83 (m, 1H), 5.39 (s, 2H), 3.87 (s, 3H), 3.78 (s, 3H), 3.26 (t, J = 6.8 Hz, 1H), 2.67-2.53 (m, 6H), 1.83 (s, 4H), 1.60 (s, 4H).

Step 6

3-[(2-fluoro-5-methoxy-phenyl) methoxy]-5-(4-pyrrolidin-1-ylbutylcarbamoylamino) isothiazole-4-carboxamide

In a microwave tube, methyl 3-[(2-fluoro-5-methoxy-phenyl)methoxy]-5-(4-pyrrolidin-1-ylbutylcarbamate)isothiazol-4-carboxylate I398 (200 mg, 416.19 μmol) was dissolved in $NH_3$/MeOH (8 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(2-fluoro-5-methoxy-phenyl)methoxy]-5-(4-pyrrolidin-1-ylbutylcarbamate) isothiazol-4-formamide T092 (20 mg, 42.96 μmol, yield: 10.32%). The product was verified by LCMS, H-NMR, C-NMR and F-NMR.

$^1$H NMR (400MHz, $CD_3OD$): δ (ppm) 7.10-7.03 (m, 2H), 6.95-6.89 (m, 1H), 5.49 (s, 2H), 3.77 (s, 3H), 3.25 (t, J = 6.8 Hz, 2H), 2.70 (s, 4H), 2.65-2.60 (m, 2H), 1.89-1.80 (m, 4H), 1.65-1.54 (m, 4H).

$^{13}$C NMR (100MHz, $CD_3OD$): δ (ppm) 170.30, 167.21, 163.13, 157.98, 157.38, 156.16, 155.60, 125.21, 125.05, 117.22, 116.99, 116.68, 116.64, 116.50, 116.42, 65.52, 56.98, 56.26, 55.01, 40.82, 28.76, 26.52, 24.12.

$^{19}$F NMR (400MHz, $CD_3OD$): δ (ppm) -131.45.

Example 69: Synthesis of Compound T093

Synthesis Route:

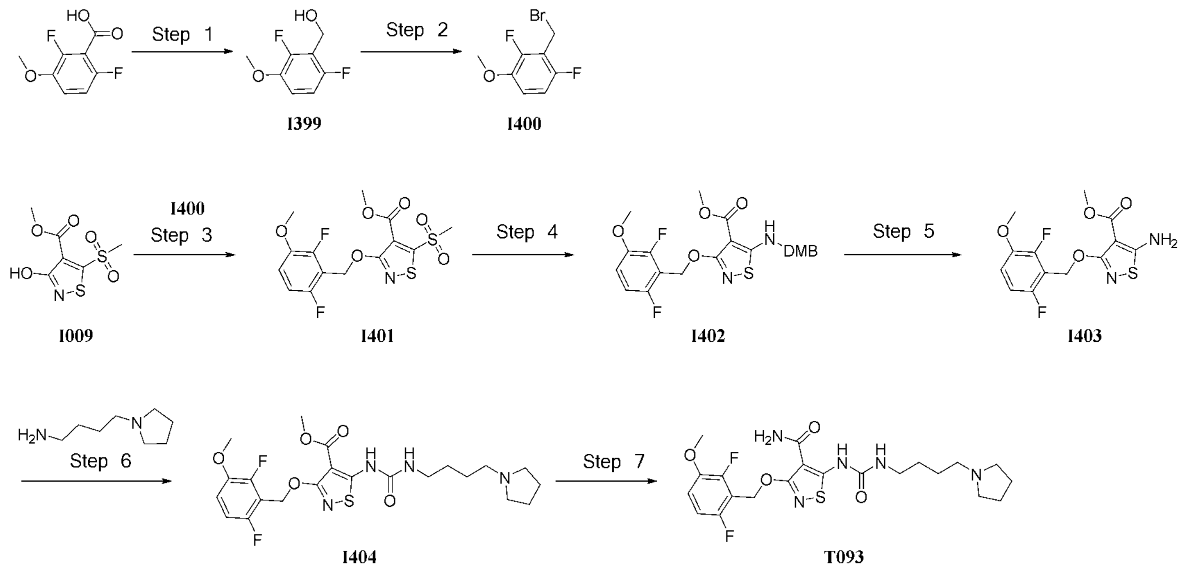


Step 1

(2,6-difluoro-3-methoxyphenyl) methanol

Under the protection of $N_2$, 2,6-difluoro-3-metoxybenzoic acid (3 g, 15.95 mmol) was dissolved in anhydrous THF (30 mL), followed by the addition of $BH_3.SMe_2$ (1.4 g, 19.14 mmol) in the ice water bath, and the mixture was warmed to 60°C and stirred to react for 16 hours. The reaction mixture was poured into methanol (20 mL), stirred at 60°C for half an hour, and concentrated under reduced pressure to obtain a colorless oily liquid product (2,6-difluoro-3-methoxyphenyl) methanol I399 (2.5 g, 14.36 mmol, yield: 90.02%).

The product was verified by LCMS and H-NMR.

$^1$H NMR (400MHz, $CDCl_3$): δ (ppm) 6.89-6.80 (m, 2H), 4.79 (s, 2H), 3.86 (s, 3H).

Step 2

2-(bromomethyl)-1,3-difluoro-4-methoxybenzene

(2,6-Difluoro-3-methoxyphenyl)methanol I399 (2.5 g, 14.36 mmol) was dissolved in DCM (30 mL) and then cooled to 0°C, followed by the addition of $PBr_3$ (4.6 g, 17.23 mmol), and the reaction system was stirred at room temperature to react for 1 hour. After complete reaction, the mixture was poured into water (50 mL) and extracted with dichloromethane (30 mL x 3), and the organic phases were combined, washed with saturated brine, dried with anhydrous sodium sulfate, and concentrated under reduced pressure to obtain a yellow oily liquid product 2-(bromomethyl)-1,3-difluoro-4-metoxybenzene I400 (2.2 g, yield: 78%).

The product was verified by LCMS and H-NMR.

$^1$H NMR (400MHz, $CDCl_3$): δ (ppm) 6.90-6.80 (m, 2H), 4.53 (s, 2H), 3.87 (s, 3H).

Step 3

Methyl 3-((2,6-difluoro-3-methoxybenzyl) oxy)-5-(methylsulfonyl) isothiazole-4-carboxylate

Methyl 3-hydroxyl-5-(methylsulfonyl)isothiazol-4-carboxylate I009 (1.5 g, 6.32 mmol) and potassium carbonate (2.6 g, 18.97 mmol) were dissolved in DMF (20 mL), followed by the addition of 2-(bromomethyl)-1,3-difluoro-4-metoxybenzene I400 (1.8 g, 7.59 mmol), and the reaction system was stirred at room temperature to react for 1 hour. Water (50 mL) was added to the reaction mixture, which was then filtered to obtain a filter cake, and the filter cake was dried to obtain a yellow solid product methyl 3-((2,6-difluoro-3-methoxybenzyl)oxy)-5-(methylsulfonyl)isothiazol-4-carboxylate I401 (1.8 g, 4.58 mmol, yield: 72.37%).

The product was verified by LCMS and H-NMR.
$^1H$ NMR (400MHz, $CDCl_3$): δ (ppm) 6.97-6.93 (m, 1H), 6.89-6.85 (m, 1H), 5.56 (s, 2H), 3.86 (s, 6H), 3.47 (s, 3H).

Step 4

Methyl 3-((2,6-difluoro-3-methoxybenzyl) oxy)-5-((2,4-dimethylbenzyl) amino) isothiazole-4-carboxylate

Methyl 3-((2,6-difluoro-3-methoxybenzyl)oxy)-5-(methylsulfonyl)isothiazol-4-carboxylate I401 (1.8 g, 4.58 mmol) was dissolved in THF(20 mL), followed by the addition of 2,4-dimethoxybenzylamine (765.1 mg, 4.58 mmol), and the reaction system was stirred at 65 °C to react for 2 hours. After the reaction was finished, the reaction mixture was added to water (30 mL) and filtered to obtain a filter cake, which was then dried to obtain a yellow solid product methyl 3-((2,6-difluoro-3-methoxybenzyl)oxy)-5-((2,4-dimethylbenzyl)amino)isothiazol-4-carboxylate
I402 (2.1 g, 4.37 mmol, yield: 95.52%).
The product was verified by LCMS and H-NMR.
$^1H$ NMR (400MHz, $CDCl_3$ ): δ (ppm) 8.10 (s, 1H), 7.16 (d, J = 8.2 Hz, 1H), 6.92 (td, J = 9.1, 5.1 Hz, 1H), 6.95 - 6.89 (m, 1H), 6.86 - 6.81 (m, 2H), 5.46 (s, 2H), 4.26 (d, J = 5.9 Hz, 2H), 3.87 (s, 3H), 3.84(s, 3H), 3.80 (s,3H), 3.71 (s, 3H).

Step 5

Methyl 5-amino-3-((2,6-difluoro-3-methoxybenzyl) oxy) isothiazole-4-carboxylate

Methyl 3-((2,6-difluoro-3-methoxybenzyl)oxy)-5-((2,4-dimethylbenzyl)amino)isothiazol-4-carboxylate I402 (2 g, 4.16 mmol) was dissolved in DCM/$H_2O$ (21/7 mL), followed by the portionwise addition of DDQ (1.9 g, 8.32 mmol) at 0°C, and the reaction system was stirred at room temperature to react for 2 hours. After complete reaction, water (50 mL) was added to the reaction mixture, which was then extracted with DCM (30 mL x2), the organic phases were combined, washed with saturated brine, dried with anhydrous sodium sulfate, and concentrated under reduced pressure to obtain a crude product, and the crude product was purified by chromatographic columns (PE/EA = 10-/1-3/1) to obtain a yellow solid product methyl 5-amino-3-((2,6-difluoro-3-methoxybenzyl)oxy)isothiazol-4-carboxylate I403 (1.4 g, yield: 70%).
The product was verified by LCMS and H-NMR.
$^1H$ NMR (400MHz, $CDCl_3$): δ (ppm) 6.96-6.90 (m, 1H), 6.87-6.82 (m, 1H), 6.41 (s, 2H), 5.48 (s, 2H), 3.88 (s, 3H), 3.76 (s, 3H).

Step 6

Methyl 3-((2,6-difluoro-3-methoxybenzyl) oxy)-5-(3-(4-(pyrrolidin-1-yl) butyl) ureido) isothiazole-4-carboxylate

4-(Pyrrolidin-1-yl)butyl-1-amine (241.1 mg, 1.70 mmol) was dissolved in anhydrous THF (5 mL) and then cooled to 0°C under the protection of $N_2$, followed by the addition of CDI (274.9 mg, 1.70 mmol), the mixture was stirred at room temperature to react for 1 hour, followed by the addition of DMSO (5mL), the pressure was then reduced to remove most of the THF, followed by the addition of methyl 5-amino-3-((2,6-difluoro-3-methoxybenzyl)oxy)isothiazol-4-carboxylate I403 (400 mg, 1.21 mmol) and potassium carbonate (502.1 mg, 3.63 mmol), and the reaction system was stirred at room temperature to react for 2 hours. After complete reaction, water (60 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((2,6-difluoro-3-methoxybenzyl)oxy)-5-(3-(4-(pyrrolidin-1-yl)butyl)ureido)isothiazol-4-carboxylate I404 (400 mg, 802.34 μmol, yield: 66.25%).
The product was verified by LCMS and H-NMR.
$^1H$ NMR (400MHz, $CDCl_3$): δ (ppm) 10.22 (s, 1H), 8.09 (s, 1H), 6.95-6.89 (m, 1H), 6.84 (t, J = 9.0 Hz, 1H), 5.48 (s, 2H), 3.87 (s, 3H), 3.73 (s, 3H), 3.30 (s, 2H), 2.60 (d, J = 11.6 Hz, 4H), 2.53 (d, J = 6.1 Hz, 2H), 1.86 (s, 4H), 1.66 (s, 4H).

Step 7

3-((2,6-difluoro-3-methoxybenzyl) oxy)-5-(3-(4-(pyrrolidin-1-yl) butyl) ureido) isothiazole-4-carboxamide

In a microwave tube, methyl 3-((2,6-difluoro-3-methoxybenzyl)oxy)-5-(3-(4-(pyrrolidin-1-yl)butyl)ureido)isothiazol-4-carboxylate I404 (200 mg, 401.17 μmol) was dissolved in $NH_3$/MeOH (3 mL,10 mol/L), and the mixture was sealed to react at 40 °C for 16 hours. The reaction mixture was concentrated under reduced pressure to obtain a crude product, and

the crude product was purified by the high-performance preparative chromatography (DCM/MeOH = 10/1, 1% $NH_3.H_2O$) to obtain a white solid product 3-((2,6-difluoro-3-methoxybenzyl)oxy)-5-(3-(4-(pyrrolidin-1-yl)butyl)ureido)isothiazol-4-formamide T093 (17 mg, 35.16 μmol, yield: 8.76%).

The product was verified by LCMS, H-NMR and C-NMR.

$^1H$ NMR (400MHz, $CDCl_3$): δ (ppm) 10.87 (s, 1H), 7.87 (s, 1H), 7.05 (s, 1H), 7.00 - 6.92 (m, 1H), 6.90 - 6.85 (m, 1H), 5.57 (s, 2H), 5.46 (s, 1H), 3.88 (s, 3H), 3.30 (s, 2H), 2.54 (s, 4H), 2.49 (t, J = 6.1 Hz, 2H), 1.84 (s, 4H), 1.76 (s, 4H).

$^{13}C$ NMR (101 MHz, $CDCl_3$) δ 169.61, 165.82,161.40, 156.33, 156.27, 154.03, 153.90, 153.84, 152.49, 152.42, 149.98, 149.91, 144.43, 144.40, 144.32, 144.29, 114.17, 114.15, 114.08, 114.05, 112.96, 112.80,112.75, 112.60, 110.43, 110.39, 110.20, 110.16, 97.23, 58.47, 58.43, 58.39, 56.82, 55.86, 53.90, 40.55, 29.70, 28.11, 26.72, 23.37

Example 70: Synthesis of Compound T094

Synthesis Route:

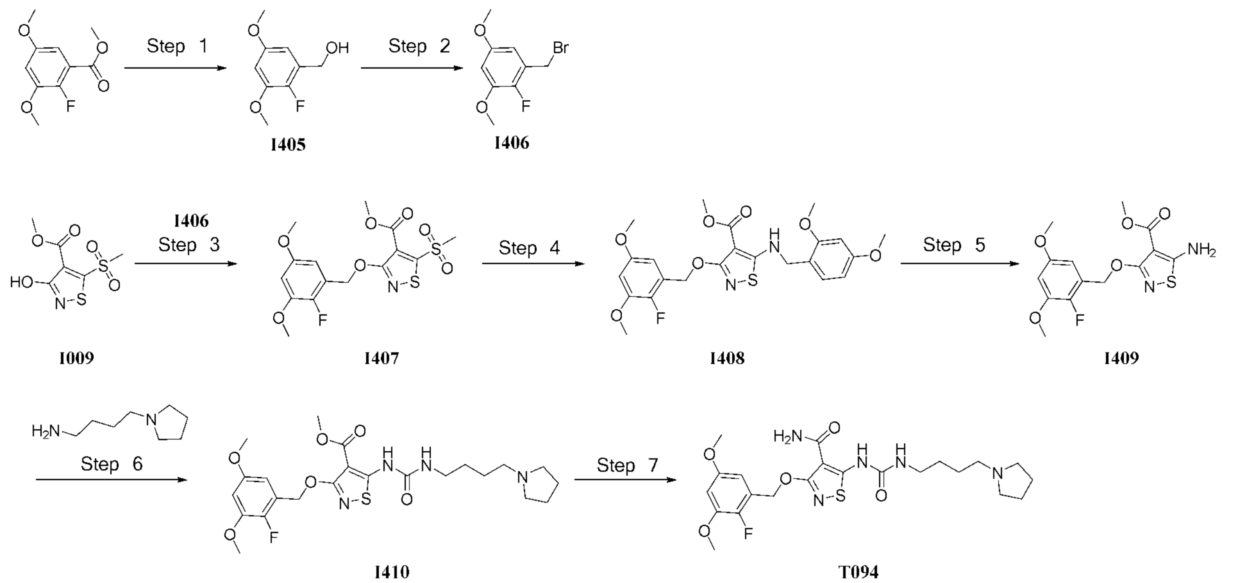


1405 1406 1009 1407 1408 1409 I410 T094

Step 1

(2-fluoro-3,5-dimethoxy-phenyl)methanol

2-Fluoro-3,5-dimetoxybenzenemethyl formate (1.5 g, 7.00 mmol) was dissolved in anhydrous THF (15 mL), followed by the addition of LAH (356.3 mg, 10.50 mmol) at 0 °C, and the mixture was stirred to react for 1 hour. After complete reaction, the mixture was added to ice water (50 mL) and extracted with ethyl acetate, and the organic phases were combined, washed with saturated brine, dried with anhydrous sodium sulfate, and concentrated under reduced pressure to obtain a yellow oily liquid product (2-fluoro-3,5-dimethoxyphenyl)methanol I405 (1.2 g, 6.45 mmol, yield: 92.04% ).

The product was verified by LCMS and H-NMR.

$^1H$ NMR (400MHz, $CDCl_3$): δ (ppm) 6.52-6.49 (m, 1H), 6.47 (dd, J = 6.8 Hz, 2.8 Hz, 1H), 4.74 (s, 2H), 3.86 (s, 3H), 3.79 (s, 3H).

Step 2

1-(bromomethyl)-2-fluoro-3,5-dimethoxy-benzene

(2-Fluoro-3,5-dimethoxyphenyl)methanol I405 (2 g, 10.74 mmol) was dissolved in DCM (20 mL) and then cooled to 0°C, followed by the addition of $PBr_3$ (3.49 g, 12.89 mmol), and the reaction system was stirred at 0 °C to react for about 1 hour. After complete reaction, the mixture was added to water and extracted with dichloromethane, and the organic phases were combined, washed with saturated brine, dried with anhydrous sodium sulfate, and concentrated under reduced

pressure to obtain a yellow solid product 1-(bromomethyl)-2-fluoro-3,5-dimetoxybenzene I406 (2.6 g, 10.44 mmol, yield: 97.17%).

The product was verified by LCMS and H-NMR.

$^1$H NMR (400MHz, $CDCl_3$): δ (ppm) 6.53-6.40 (m, 2H), 4.14 (s, 1H), 3.86 (d, J = 2.8 Hz, 1H), 3.78 (d, J = 1.2 Hz, 1H).

Step 3

Methyl 3-[(2-fluoro-3,5-dimethoxy-phenyl)methoxy]-5-methylsulfonyl-isothiazole-4-carboxylate

Methyl 3-hydroxyl-5-(methylsulfonyl)isothiazol-4-carboxylate I009 (2 g, 8.43 mmol) and potassium carbonate (3.5 g, 25.29 mmol) were dissolved in DMF (25 mL), followed by the addition of 1-(bromomethyl)-2-fluoro-3,5-dimetoxybenzene I406 (2.5 g, 10.12mmol), and the reaction system was stirred at room temperature to react for 2 hours. Water (100 mL) was added to the reaction mixture, which was then filtered, and the filter cake was dissolved in ethyl acetate, washed with saturated brine, dried with anhydrous sodium sulfate, and concentrated under reduced pressure to obtain a white solid product methyl 3-[(2-fluoro-3,5-dimethoxyphenyl)methoxy]-5-methylsulfonylisothiazol-4-carboxylate I407 (1.8 g, 4.46 mmol, yield: 52.96%).

The product was verified by LCMS and H-NMR.

$^1$H NMR (400MHz, $CDCl_3$): δ (ppm) 6.61-6.56 (m, 1H), 6.52 (dd, J = 6.8 Hz, 3.2 Hz, 1H), 5.53 (s, 2H), 3.95 (s, 3H), 3.88 (s, 3H), 3.79 (s, 3H), 3.48 (s, 3H).

Step 4

Methyl 5-[(2,4-dimethoxyphenyl)methylamino]-3-[(2-fluoro-3,5-dimethoxy-phenyl) metho xy]isothiazole-4-carboxylate

Methyl 3-[(2-fluoro-3,5-dimethoxyphenyl)methoxy]-5-methylsulfonylisothiazol-4-carboxylate I407 (1.8 g, 4.44 mmol) was dissolved in THF(40 mL), followed by the addition of 2,4-dimethoxybenzylamine (7.4 g, 44.40 mmol), and the reaction system was stirred at 65 °C to react for 1 hour. After the reaction was finished, the reaction mixture was added to water (100 mL), and then filtered, and the filtrate was dissolved in ethyl acetate, washed with saturated brine, dried with anhydrous sodium sulfate, and concentrated under reduced pressure to obtain a yellow solid product methyl 5-[(2,4-dimethoxyphenyl)methylamino]-3-[(2-fluoro-3,5-dimethoxyphenyl)methoxy]isothiazol-4-carboxylate I408 (1.8 g, 3.65 mmol, yield: 82.32%).

The product was verified by LCMS and H-NMR.

$^1$H NMR (400MHz, $CDCl_3$ ): δ (ppm) 8.13 (s, 1H), 7.16 (d, J = 8.4 Hz, 1H), 6.70-6.68 (m, 1H), 6.49 -6.41 (m, 3H), 5.46 (s, 2H), 4.27 (d, J = 6.0 Hz, 2H), 3.85 (d, J = 6.8 Hz, 6H), 3.83-3.78 (m, 9H).

Step 5

Methyl 5-amino-3-[(2-fluoro-3,5-dimethoxy-phenyl)methoxy]isothiazole-4-carboxylate

Methyl 5-[(2,4-dimethoxyphenyl)methylamino]-3-[(2-fluoro-3,5-dimethoxyphenyl)methoxy]isothiazol-4-carboxylate I408 (1.8 g, 3.65 mmol) was dissolved in DCM/$H_2O$ (80/16 mL), followed by the portionwise addition of DDQ (2.5 g, 10.96 mmol) at 0°C, and the reaction system was stirred at room temperature to react for 1 hour. After complete reaction, water (100 mL) was added to the reaction mixture, which was then extracted with DCM (80 mL x3), the organic phases were combined, washed with saturated brine, dried with anhydrous sodium sulfate, and concentrated under reduced pressure to obtain a crude product, and the crude product was purified by chromatographic columns (neutral alumina, PE/EA = 10/1-1/1) to obtain a white solid product methyl 5-amino-3-[(2-fluoro-3,5-dimethoxyphenyl)methoxy] isothiazol-4-carboxylate I409 (30 mg, 1.26 mmol, yield: 34.37%).

The product was verified by LCMS and H-NMR.

$^1$H NMR (400MHz, $CDCl_3$ ): δ (ppm) 6.71-6.67 (m, 1H), 6.49 (dd, J = 7.2 Hz, 3.2 Hz, 1H), 6.44 (s, 2H), 5.48 (s, 2H), 3.86 (d, J = 5.2 Hz, 6H), 3.80 (s, 3H).

Step 6

Methyl 3-[(2-fluoro-3,5-dimethoxy-phenyl)methoxy]-5-(4-pyrrolidin-1-ylbutylcarbamo yla mino)isothiazole-4-carboxylate

4-(Pyrrolidin-1-yl)butyl-1-amine (249.3 mg, 1.75 mmol) was dissolved in anhydrous THF (5 mL) and then cooled to 0°C under the protection of $N_2$, followed by the addition of CDI (284.2 mg, 1.75 mmol), the mixture was stirred at room

temperature to react for 1 hour, followed by the addition of DMSO (5 mL), the pressure was then reduced to remove most of the THF, followed by the addition of methyl 5-amino-3-[(2-fluoro-3,5-dimethoxyphenyl)methoxy]isothiazol-4-carboxylate I409 (400 mg, 1.17 mmol) and potassium carbonate (484.5 mg, 3.51 mmol), and the reaction system was stirred at room temperature to react for 1 hour. After complete reaction, water (20 mL) was added to the reaction mixture, which was then filtered, and the filtrate was dissolved in ethyl acetate, washed with saturated brine, dried with anhydrous sodium sulfate, and concentrated under reduced pressure to obtain a yellow solid product methyl 3-[(2-fluoro-3,5-dimethoxyphenyl) methoxy]-5-(4-pyrrolidin-1-ylbutylcarbonylamino)isothiazol-4-carboxylate I410 (400 mg, 783.43 μmol, yield: 67.05%).

The product was verified by LCMS and H-NMR.

$^1$H NMR (400MHz, $CDCl_3$ ): δ (ppm) 10.25 (s, 1H), 8.15 (s, 1H), 6.70-6.68 (m, 1H), 6.48 (dd, J = 6.8 Hz, 2.8 Hz, 1H), 5.49 (s, 2H), 3.87-3.79 (m, 9H), 3.33 (s, 2H), 2.76 (s, 2H), 2.66 (s, 2H), 2.62 (s, 2H), 1.95 (s, 4H), 1.75-1.72 (m, 4H).

Step 7

3-[(2-fluoro-3,5-dimethoxy-phenyl)methoxy]-5-(4-pyrrolidin-1-ylbutylcarbamoylamino) isothiazole-4-carboxamide

In a microwave tube, methyl 3-[(2-fluoro-3,5-dimethoxyphenyl)methoxy]-5-(4-pyrrolidin-1-ylbutylcarbonylamino)isothiazol-4-carboxylate I410 (400 mg, 783.43 μmol) was dissolved in $NH_3$/MeOH (8 mL,10 mol/L), and the mixture was sealed to react at 40 °C for 20 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(2-fluoro-3,5-dimethoxyphenyl)methoxy]-5-(4-pyrrolidin-1-ylbutylcarbamate) isothiazol-4-formamide T094 (41 mg, 82.73 μmol, yield: 10.56%). The product was verified by LCMS, H-NMR, C-NMR and F-NMR.

$^1$H NMR (400 MHz, $CD_3OD$) δ (ppm) 6.65 (dd, J = 7.2 Hz, 2.8 Hz, 1H), 6.58 (dd, J = 4.4 Hz, 3.2 Hz, 1H), 5.47 (s, 2H), 3.85 (s, 3H), 3.76 (s, 3H), 3.25 (t, J = 6.8 Hz, 2H), 2.71 (s, 4H), 2.66-2.60 (m, 2H), 1.89-1.82 (m, 4H), 1.67-1.55 (m, 4H).

$^{13}$C NMR (100 MHz, $CD_3OD$) δ (ppm) 170.26, 167.20, 163.15, 157.43, 157.41, 156.17, 149.85, 149.73, 148.01, 145.63, 125.21, 125.08, 105.79, 102.35, 98.78, 85.53, 85.48, 56.82, 56.23, 55.02, 49.66, 49.45, 49.23, 49.02, 48.81, 48.59, 48.38, 40.73, 28.65, 26.22, 24.11.

$^{19}$FNMR (400 MHz, $CDCl_3$): δ (ppm) -152.45.

Example 71: Synthesis of Compound T095

Synthesis Route:

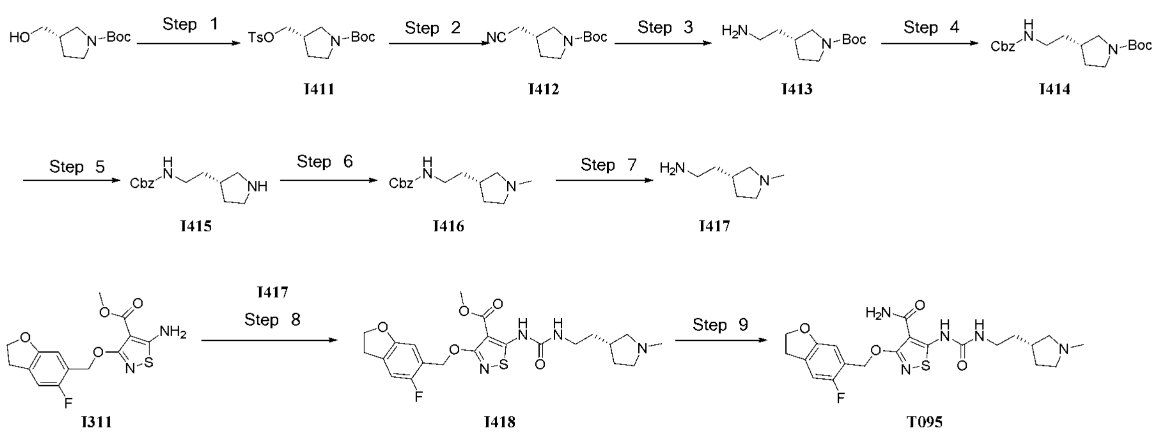


Step 1

Tert-butyl (3S)-3-(p-tolylsulfonyloxymethyl) pyrrolidine-1-carboxylate

Tert-butyl (3S)-3-(hydroxymethyl)pyrrolidin-1-carboxylate (2 g, 9.94 mmol) was dissolved in DCM (40 mL), followed by the addition of triethylamine (3 g, 29.65 mmol) and TsCl (2.8 g, 14.69 mmol) at room temperature, to allow for subsequent reaction for about 2 hours. After complete reaction, the mixture was directly concentrated under reduced pressure to obtain a crude product, and the crude product was purified by the flash column chromatography (PE/EA =

3/1-1/1) to obtain a white solid product tert-butyl (3S)-3-(p-toluenesulfonyloxymethyl)pyrrolidin-1-carboxylate I411 (2 g, 5.63 mmol, yield: 56.62%).

The product was verified by LCMS and H-NMR.

$^1$H NMR (400 MHz, $CDCl_3$) δ 7.80 (d, J = 8.0 Hz, 2H), 7.37 (d, J = 8.0 Hz, 2H), 3.99 (d, J = 6.8 Hz, 2H), 3.50 (s, 3H), 3.02 (dd, J = 11.2, 6.8 Hz, 1H), 2.47 (s, 3H), 1.97 (s, 1H), 1.47 (s, 2H), 1.45 (s, 9H).

Step 2

Tert-butyl (3R)-3-(cyanomethyl)pyrrolidine-1-carboxylate

Tert-butyl (3S)-3-(p-toluenesulfonyloxymethyl)pyrrolidin-1-carboxylate I411 (3 g, 8.44 mmol) was dissolved in DMSO (10 mL), followed by the addition of NaCN (447.7 mg, 8.44 mmol) at room temperature, and the mixture was warmed to 100 °C and stirred to react for about 16 hours. After complete reaction, the mixture was poured into water and extracted with ethyl acetate, and the organic phases were combined, washed with saturated brine, dried with anhydrous sodium sulfate, and concentrated under reduced pressure to obtain a yellow oily liquid product tert-butyl (3R)-3-(cyanomethyl)pyrrolidin-1-carboxylate I412 (1.5 g, 7.13 mmol, yield: 84.52%).

The product was verified by LCMS and H-NMR.

$^1$H NMR (400MHz, $CDCl_3$ ): δ (ppm) 3.66-3.57 (m, 1H), 3.54-3.41 (m, 1H), 3.41-3.30 (m, 1H), 2.58-2.39 (m, 1H), 2.12 (s, 1H), 1.85-1.55 (m, 2H), 1.45 (s, 9H).

Step 3

Tert-butyl(3S)-3-(2-aminoethyl)pyrrolidine-1-carboxylate

Tert-butyl (3R)-3-(cyanomethyl)pyrrolidin-1-carboxylate I412 (1.5 g, 7.13 mmol) was dissolved in methanol (20 mL), followed by the addition of raney nickel (1.5 g), and in the atmosphere of hydrogen (20 psi), the reaction system was stirred at room temperature to react for 2 hours. The reaction mixture was filtered, and the filtrate was concentrated under reduced pressure to obtain a white solid product tert-butyl (3S)-3-(2-aminoethyl)pyrrolidin-1-carboxylate I413 (1.5 g, 7.00 mmol, yield: 98.12%).

The product was verified by LCMS and H-NMR.

$^1$H NMR (400MHz, $CDCl_3$): δ (ppm) 3.45-3.15(m, 4H), 2.85-2.80 (m, 1H), 2.73-2.69 (m, 1H), 2.14-1.95 (m, 2H), 1.77-1.49 (m, 5H), 1.44 (s, 9H).

Step 4

Tert-butyl (3S)-3-[2-(benzyloxycarbonylamino)ethyl]pyrrolidine-1-carboxylate

Tert-butyl (3S)-3-(2-aminoethyl)pyrrolidin-1-carboxylate I413 (1.5 g, 7.00 mmol) was dissolved in the mixed solution of THF (15 mL) and $H_2O$ (5 mL), followed by the addition of $Na_2CO_3$ (2.2 g, 21.00 mmol) and benzyl chloroformate (1.8 g, 10.50 mmol) at room temperature, and the mixture was stirred to react for about 2 hours. The mixture was poured into water and extracted with ethyl acetate, the organic phases were combined, washed with saturated brine, dried with anhydrous sodium sulfate, and concentrated under reduced pressure to obtain a crude product, and the crude product was purified by the preparative thin-layer chromatography (DCM/MeOH = 10/ 1) to obtain a yellow oily liquid product benzyl N-[2-[(3R)-pyrrolidin-3-yl]ethyl]carbamate I414 (1.7 g, 4.88 mmol, yield: 69.71%).

The product was verified by LCMS and H-NMR.

$^1$H NMR (400MHz, $CDCl_3$): δ (ppm) 7.35-7.33 (m, 5H), 5.11 (d, J = 9.8 Hz, 1H), 4.75 (s, 1H), 3.52-3.10 (m, 10H),2.87 (s, 1H), 1.45 (s, 9H).

Step 5

Benzyl N-[2-[(3R)-pyrrolidin-3-yl]ethyl]carbamate

Benzyl N-[2-[(3R)-pyrrolidin-3-yl]ethyl]carbamate I414 (1.6 g, 4.59 mmol) was dissolved in MTBE/HCl (20 mL), and the mixture was stirred at 25 °C to react for about 2 hours. After complete reaction, the reaction mixture was directly concentrated under reduced pressure to obtain a yellow oily liquid product N-[2-[(3R)-pyrrolidin-3-yl]ethyl]benzyl carbamate I415 (1.1 g, 4.19 mmol, yield: 91.21%).

The product was verified by LCMS and H-NMR.

$^1$H NMR (400 MHz, $CDCl_3$) δ 9.58 (s, 2H), 7.34 (s, 3H), 5.08 (s, 2H), 3.74-3.21 (m, 8H), 2.88 (s, 1H), 2.45-2.04 (m,

5H).

Step 6

Benzyl N-[2-[(3S)-1-methylpyrrolidin-3-yl]ethyl]carbamate

N-[2-[(3R)-pyrrolidin-3-yl]ethyl]benzyl carbamate I415 (1 g, 4.03 mmol) was dissolved in anhydrous THF (10 mL), followed by the addition of formaldehyde (4 mL) at room temperature, and the mixture was stirred to react for about 1 hour, followed by the further addition of $NaBH(OAc)_3$ (3.1 g, 14.51 mmol), to allow for reaction at the room temperature for about 2 hours. After complete reaction, the mixture was directly concentrated to obtain a crude product, and the crude product was purified by the flash column chromatography to obtain a yellow solid product N-[2-[(3S)-1-methylpyrrolidin-3-yl]ethyl]benzyl carbamate I416 (600 mg, 2.29 mmol, yield: 56.79%).
The product was verified by LCMS and H-NMR.
$^1$H NMR (400MHz, $CDCl_3$): δ (ppm) 7.35 (s, 5H), 5.09 (d, J = 8.2 Hz, 2H), 3.18 (dd, J = 13.6, 7.0 Hz, 3H), 2.67-2.57 (m, 2H), 2.49-2.41 (m, 2H), 2.32 (s, 3H), 1.60-1.57 (m, 3H), 1.49-1.37 (m, 2H).

Step 7

2-[(3S)-1-methylpyrrolidin-3-yl]ethanamine

N-[2-[(3S)-1-methylpyrrolidin-3-yl]ethyl]benzyl carbamate I416 (190 mg, 724.23 μmol) was dissolved in methanol (10 mL), followed by the addition of $Pd(OH)_2/C$ (50 mg, 724.23 μmol) at room temperature, and in the atmosphere of hydrogen, the mixture was stirred to react for about 6 hours. After complete reaction, the mixture was filtered, the filter cake was washed with methanol, and the filtrates were combined and concentrated under reduced pressure to obtain a colorless oily liquid product 2-2-[(3S)-1-methylpyrrolidin-3-yl]ethylamine I417 (90 mg, 701.95 μmol, yield: 96.92%).
The product was verified by LCMS.

Step 8

Methyl 3-[(5-fluoro-2,3-dihydrobenzofuran-6-yl) methoxy]-5-[2-[(3S)-1-methylpyrrolidin-3-yl] ethylcarbamoylamino] isothiazole-4-carboxylate

2-[(3S)-1-methylpyrrolidin-3-yl]ethylamine I417 (118.6 mg, 924.99 μmol) was dissolved in anhydrous THF (4 mL) and then cooled to 0°C under the protection of $N_2$, followed by the addition of CDI (133.2 mg, 924.99 μmol), the mixture was stirred at room temperature to react for 1 hour, followed by the addition of DMSO (4 mL), the pressure was then reduced to remove most of the THF, followed by the addition of methyl 5-amino-3-[(5-fluoro-2,3-dihydrobenzofuran-6-yl) methoxy]isothiazol-4-carboxylate I311 (200 mg, 616.66 μmol) and potassium carbonate (255.3 mg, 1.85 mmol), and the reaction system was stirred at room temperature to react for 1 hour. After complete reaction, water (20 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[(5-fluoro-2,3-dihydrobenzofuran-6-yl)methoxy]-5-[2-[(3S)-1-methylpyrrolidin-3-yl]ethylcarbamoylamino]isothiazol-4-carboxylate I418 (180 mg, 376.15 μmol, yield: 61.00%). The product was verified by LCMS and H-NMR.
$^1$H NMR (400MHz, $CDCl_3$): δ (ppm) 6.97 (d, J = 9.2 Hz, 1H), 6.89 (d, J = 5.6 Hz, 1H), 5.33 (s, 2H), 4.55 (t, J = 8.6 Hz, 2H), 3.86 (s, 3H), 3.29-3.16 (m, 5H), 3.12-3.00 (m, 2H), 2.70 (s, 4H), 2.45-2.35 (m, 1H), 2.25-2.15 (m, 1H), 1.75-1.65 (m, 3H).

Step 9

3-[(5-fluoro-2,3-dihydrobenzofuran-6-yl) methoxy]-5-[2-[(3S)-1-methylpyrrolidin-3-yl] ethylcarbamoylamino] isothiazole-4-carboxamide

In a microwave tube, methyl 3-[(5-fluoro-2,3-dihydrobenzofuran-6-yl)methoxy]-5-[2-[(3S)-1-methylpyrrolidin-3-yl]ethylcarbamoylamino]isothiazol-4-carboxylate I418 (100 mg, 208.97 μmol) was dissolved in $NH_3$/MeOH (8 mL,10 mol/L), and the mixture was sealed to react at 60 °C for 20 hours. The reaction mixture was concentrated under reduced pressure to obtain a crude product, and the crude product was purified by the preparative thin-layer chromatography (DCM/MeOH = 7/1) to obtain a white solid product 3-[(5-fluoro-2,3-dihydrobenzofuran-6-yl)methoxy]-5-[2-[(3S)-1-methylpyrrolidin-3-yl]ethylcarbamoylamino]isothiazol-4-formamide T095 (26 mg, 56.09 μmol, yield: 26.84%).

The product was verified by LCMS, H-NMR and C-NMR.

$^{1}$H NMR (400 MHz, $CD_3OD$) δ (ppm) 7.02 (d, *J* = 9.4 Hz, 1H), 6.82 (d, *J* = 5.8 Hz, 1H), 5.43 (s, 2H), 4.55 (t, *J* = 8.8 Hz, 2H), 3.25-3.18 (m, 4H), 2.87 (dd, *J* = 9.0, 7.4 Hz, 1H), 2.71 (dd, *J* = 14.8, 8.6 Hz, 1H), 2.56-2.48 (m, 1H), 2.37 (s, 3H), 2.21 (s, 2H), 2.12-2.03 (m, 1H), 1.67-1.57 (m, 2H), 1.53-1.45 (m, 1H).

$^{13}$C NMR (100 MHz, $CD_3OD$) δ (ppm) 170.14, 163.24, 157.56, 156.19, 131.19, 131.10, 113.39, 113.14, 111.06, 111.03, 72.86, 61.53, 56.67, 41.71, 39.51, 36.40, 34.61, 30.92, 30.81

Example 72: Synthesis of Compound T096

Synthesis Route:

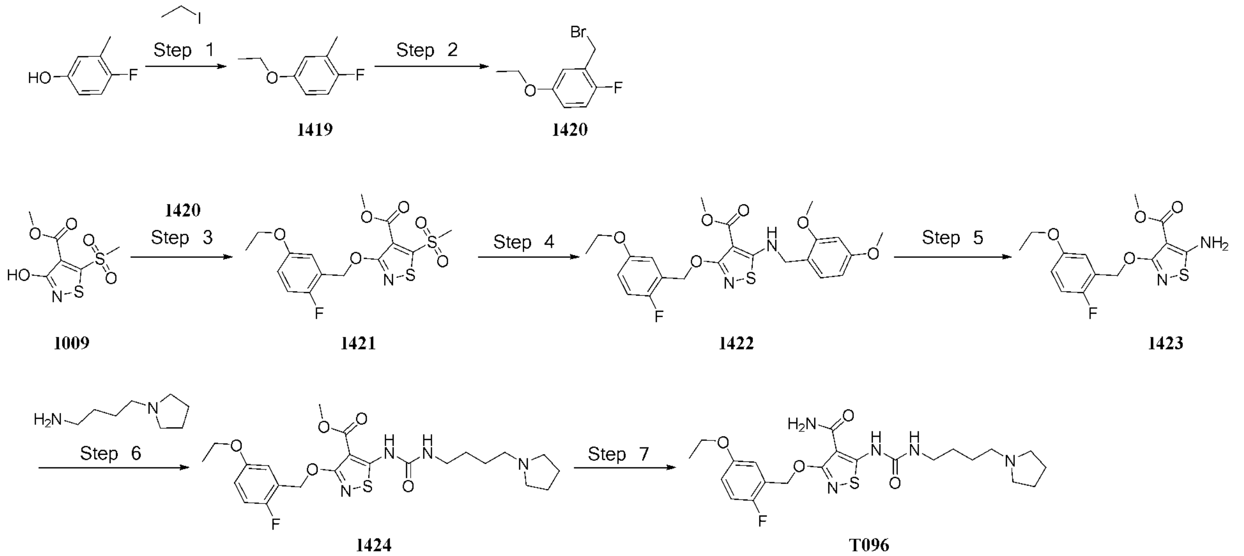


1419, 1420, 1009, 1421, 1422, 1423, 1424, T096

Step 1

4-ethoxy-1-fluoro-2-methyl-benzene

4-Fluoro-3-methylphenol (5 g, 39.64 mmol) was dissolved in dry DMF (80 mL), followed by the addition of NaH (3.17 g, 79.28 mmol, 60%) at 0°C, the mixture was warmed to room temperature to react for about half an hour, followed by the slow addition of iodoethane (18.55 g, 118.93 mmol), to allow for reaction at room temperature for 1 hour, and the mixture was then warmed to 70 °C to react for 3 hours. After complete reaction, the mixture was added to water and extracted with ethyl acetate, the organic phases were combined, washed with saturated brine, dried with anhydrous sodium sulfate, and concentrated under reduced pressure to obtain a crude product, and the crude product was purified by the flash column chromatography (PE/EA = 10/1) to obtain a colorless oily liquid product 4-ethoxy-1-fluoro-2-methyl benzene I419 (5 g, 32.43 mmol, yield: 81.81%).

1H NMR (400MHz, $CDCl_3$): δ (ppm) δ 6.88 (t, J = 9.0 Hz, 1H), 6.74-6.57 (m, 2H), 3.96 (q, J = 7.0 Hz, 2H), 2.23 (d, J = 1.6 Hz, 3H), 1.38 (t, J = 7.0 Hz, 3H).

Step 2

2-(bromomethyl)-4-ethoxy-1-fluoro-benzene

4-Ethoxy-1-fluoro-2-methyl benzene I419 (3 g, 19.46 mmol) was dissolved in $CCl_4$ (40 mL), followed by the addition of AIBN (3.19 g, 19.46 mmol) and NBS (3.46 g, 19.46 mmol) at room temperature, and the mixture was stirred at 70°C to react for 3 hours. After complete reaction, the mixture was directly concentrated under reduced pressure to obtain a crude product, and the crude product was purified by the flash column chromatography (PE/EA = 1/0-10/1) to obtain a colorless oily liquid product 2-(bromomethyl)-4-ethoxy-1-fluorobenzene I420 (2.5 g, 10.73 mmol, yield: 55.13%).

The product was verified by LCMS and H-NMR.

1H NMR (400MHz, $CDCl_3$): δ (ppm) 6.96 (t, J = 9.2 Hz, 1H), 6.91-6.86 (m, 1H), 6.83-6.75 (m, 1H), 4.47 (d, J = 0.8 Hz, 2H), 4.04-3.94 (m, 2H), 1.43-1.36 (m, 3H).

Step 3

Methyl 3-[(5-ethoxy-2-fluoro-phenyl)methoxy]-5-methylsulfonyl-isothiazole-4-carboxylate Methyl 3-hydroxyl-5-(methylsulfonyl)isothiazol-4-carboxylate I009 (1.5 g, 6.32 mmol) and potassium carbonate (1.5 g, 6.32 mmol) were dissolved in DMF (20 mL), followed by the addition of 2-(bromomethyl)-4-ethoxy-1-fluorobenzene I420 (1.77 g, 7.59 mmol), and the reaction system was stirred at room temperature to react for 2 hours. Water (100 mL) was added to the reaction mixture, which was then filtered, and the filter cake was dissolved in ethyl acetate, washed with water and saturated brine, dried with anhydrous sodium sulfate, and concentrated under reduced pressure to obtain a yellow solid product methyl 3-[(5-ethoxy-2-fluoro-phenyl)methoxy]-5-methylsulfonylisothiazol-4-carboxylate I421 (2.2 g, 5.65 mmol, yield: 89.36%).

The product was verified by LCMS and H-NMR.

1H NMR (400MHz, $CDCl_3$): δ(ppm) 7.08 - 6.93 (m, 2H), 6.84 - 6.80 (m, 1H), 5.51 (s, 2H), 4.04 - 3.97 (m, 2H), 3.94 (d, J = 12.2 Hz, 3H), 3.49 (s, 3H), 1.43 - 1.37 (m, 3H).

Step 4

Methyl 5-[(2,4-dimethoxyphenyl)methylamino]-3-[(5-ethoxy-2-fluoro-phenyl)metho xy]isothiazole-4-carboxylate

Methyl 3-[(5-ethoxy-2-fluoro-phenyl)methoxy]-5-methylsulfonylisothiazol-4-carboxylate I421 (2.15 g, 5.52 mmol) was dissolved in THF(20 mL), followed by the addition of 2,4-dimethoxybenzylamine (9.23 g, 55.21 mmol), and the reaction system was stirred at 65 °C to react for 1 hour. After the reaction was finished, the reaction mixture was added to water (50 mL), and then filtered, and the filter cake was dissolved in ethyl acetate, washed with saturated brine, dried with anhydrous sodium sulfate, and concentrated under reduced pressure to obtain a white solid product methyl 5-[(2,4-dimethoxyphenyl)methylamino]-3-[(5-ethoxy-2-fluorophenyl)methoxy]isothiazol-4-carboxylate I422 (1.8 g, 3.78 mmol, yield: 68.42%).

The product was verified by LCMS and H-NMR.

$^1H$ NMR (400MHz, $CDCl_3$): δ (ppm) 8.13 (t, J = 5.8 Hz, 1H), 7.21 - 7.07 (m, 2H), 6.95 (t, J = 9.2 Hz, 1H), 6.81 - 6.72 (m, 1H), 6.51 - 6.40 (m, 2H), 5.45 (s, 2H), 4.27 (d, J = 6.0 Hz, 2H), 4.00 (q, J = 7.0 Hz, 2H), 3.85 (s, 3H), 3.82 (s, 3H), 3.80 (s, 3H), 1.45 - 1.34 (m, 3H).

Step 5

Methyl 5-amino-3-((5-ethoxy-2-fluorobenzyl) oxy) isothiazole-4-carboxylate

Methyl 5-[(2,4-dimethoxyphenyl)methylamino]-3-[(5-ethoxy-2-fluorophenyl)methoxy]isothiazol-4-carboxylate I422 (1.7 g, 3.57 mmol) was dissolved in DCM/$H_2O$ (21/7 mL), followed by the portionwise addition of DDQ (1.6 g, 7.14 mmol) at 0°C, and the reaction system was stirred at room temperature to react for 1 hour. After complete reaction, water (30 mL) was added to the reaction mixture, which was then extracted with DCM (20 mL x3), the organic phases were combined, washed with saturated brine, dried with anhydrous sodium sulfate, and concentrated under reduced pressure to obtain a crude product, and the crude product was purified by chromatographic columns (PE/EA = 10/1-3/1) to obtain a white solid product methyl 5-amino-3-((5-ethoxy-2-fluorobenzyl)oxy)isothiazol-4-carboxylate I423 (500 mg, 1.53 mmol, yield: 42.95%).

The product was verified by LCMS.

Step 6

Methyl 3-((5-ethoxy-2-fluorobenzyl)oxy)-5-(3-(4-(pyrrolidin-1-yl)butyl)ureido)isothiazole-4-carboxylate

4-(Pyrrolidin-1-yl)butyl-1-amine (209.21 mg, 1.47 mmol) was dissolved in anhydrous THF (5 mL) and then cooled to 0°C under the protection of $N_2$, followed by the addition of CDI (238.50 mg, 1.47 mmol), the mixture was stirred at room temperature to react for 1 hour, followed by the addition of DMSO (5 mL), the pressure was then reduced to remove most of the THF, followed by the addition of methyl 5-amino-3-((5-ethoxy-2-fluorobenzyl)oxy)isothiazol-4-carboxylate I423 (400 mg, 1.23 mmol) and potassium carbonate (508.20 mg, 3.68 mmol), and the reaction system was stirred at room temperature to react for 1 hour. After complete reaction, water (60 mL) was added to the reaction mixture, which was then extracted with ethyl acetate (20 mL x 3), the organic phases were sequentially washed with water and saturated brine,

dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-((5-ethoxy-2-fluorobenzyl)oxy)-5-(3-(4-(pyrrolidin-1-yl)butyl)ureido)isothiazol-4-carboxylate I424 (400 mg, 808.77 μmol, yield: 65.98%).

The product was verified by LCMS and H-NMR.

$^1H$ NMR (400MHz, $CDCl_3$): δ (ppm) 10.22 (s, 1H), 8.22 (s, 1H), 7.12 (s, 1H), 6.98 (t, J = 9.1 Hz, 1H), 6.79 (s, 1H), 5.48(s, 2H), 4.01 (q, J = 7.0 Hz, 2H), 3.88 (s, 3H), 3.32 (s, 2H), 2.60(s, 4H), 2.53 (t, J = 6.0 Hz, 2H), 1.89 (s, 6H), 1.68 (s, 4H), 1.40 (t, J = 7.0 Hz, 3H).

Step 7

3-((5-ethoxy-2-fluorobenzyl) oxy)-5-(3-(4-(pyrrolidin-1-yl) butyl) ureido) isothiazole-4-carboxamide

In a microwave tube, methyl 3-((5-ethoxy-2-fluorobenzyl)oxy)-5-(3-(4-(pyrrolidin-1-yl)butyl)ureido)isothiazol-4-carboxylate I424 (100 mg, 202.19 μmol) was dissolved in $NH_3$/MeOH (6 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((5-ethoxy-2-fluorobenzyl)oxy)-5-(3-(4-(pyrrolidin-1-yl)butyl)ureido)isothiazol-4-formamide T096 (30 mg, 62.56 μmol, yield: 30.94%).

The product was verified by LCMS, H-NMR, C-NMR and F-NMR.

$^1H$ NMR (500MHz $CD_3OD$): δ (ppm) 7.09-7.04 (m, 2H), 6.94 - 6.90 (m, 1H), 5.51 (s, 2H), 4.04-4.00 (m, 2H), 3.28 (t, J = 5.0 Hz 2H), 2.89 (s, 4H), 2.82 - 2.79(m, 2H), 1.95-1.92 (m, 4H), 1.72 - 1.66 (m, 2H), 1.64-1.59 (m, 2H), 1.38 (t, J = 7.0 Hz, 3H).

$^{13}C$ NMR (100 MHz, $CD_3OD$) δ 170.30, 167.22,163.14, 157.90, 156.65,156.63,158.16, 155.25, 125.16, 125.00, 117.25, 117.21, 117.19, 117.16, 117.08, 116.98, 98.77, 65.53, 65.50, 65.18, 56.98, 55.00, 40.83, 28.76, 26.53, 24.13, 15.10

Example 73: Synthesis of Compound T097

Synthesis Route:

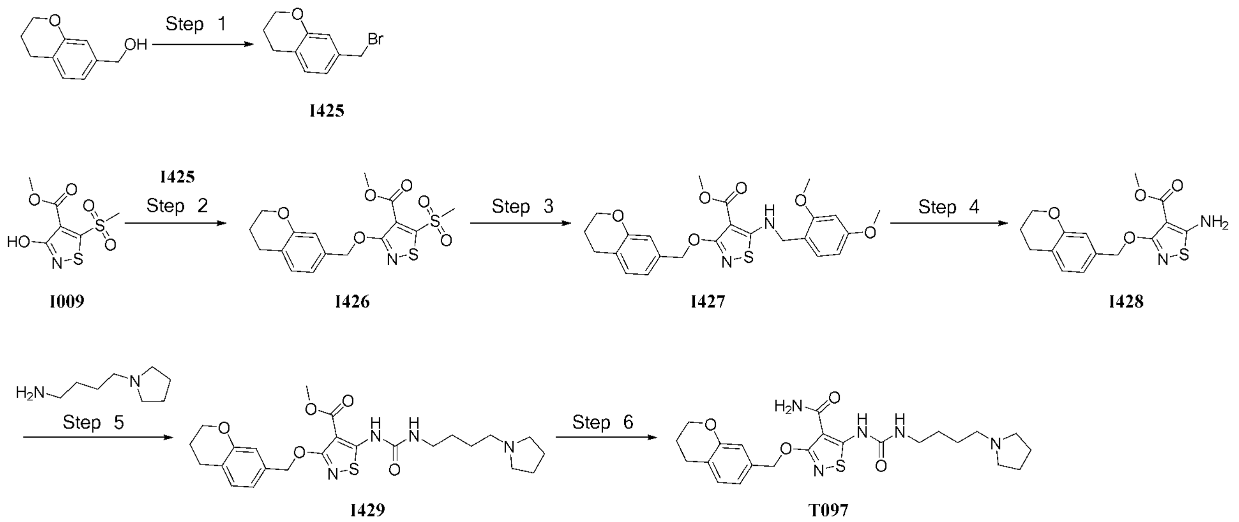


1425 1009 1426 1427 1428 1429 T097

Step 1

7-(bromomethyl)chromane

Chroman-7-yl ethanol (1.5 g, 9.14 mmol) was dissolved in DCM (20 mL) and cooled to 0°C, followed by the addition of $PBr_3$ (4.9 g, 18.27 mmol), and the reaction system was stirred at room temperature to react for 1 hour. After complete reaction, the mixture was added to water and extracted with dichloromethane, and the organic phases were

combined, washed with saturated brine, dried with anhydrous sodium sulfate, and concentrated under reduced pressure to obtain a yellow oily liquid product 7-(bromomethyl)chromane I425 (1.7 g, 7.49 mmol, yield: 81.94%).

The product was verified by LCMS and H-NMR.

$^1$H NMR (400 MHz, $CDCl_3$) δ (ppm) 7.00 (d, J = 7.8 Hz, 2H), 6.87-6.82 (m, 2H), 4.42 (s, 2H), 4.18 (t, J = 5.2 Hz, 2H), 2.77 (t, J = 6.5 Hz, 2H), 1.99-1.97 (m, 2H).

Step 2

Methyl 3-(chroman-7-ylmethoxy)-5-methylsulfonyl-isothiazole-4-carboxylate

Methyl 3-hydroxyl-5-(methylsulfonyl)isothiazol-4-carboxylate I009 (1.5 g, 6.32 mmol) and potassium carbonate (2.6 g, 18.97 mmol) was dissolved in DMF (30 mL), followed by the addition of 7-(bromomethyl)chromane I425 (1.7 g, 7.59 mmol), and the reaction system was stirred at room temperature to react for 1 hour. Water (100 mL) was added to the reaction mixture, which was then filtered, and the filter cake was dissolved in ethyl acetate, washed with saturated brine, dried with anhydrous sodium sulfate, and concentrated under reduced pressure to obtain a yellow solid product methyl 3-(chroman-7-ylmethoxy)-5-methylsulfonylisothiazol-4-carboxylate I426 (1.8 g, 4.69 mmol, yield: 74.25%).

The product was verified by LCMS and H-NMR.

$^1$H NMR (400MHz, $CDCl_3$ ): δ (ppm) 7.04 (d, J = 7.7 Hz, 1H), 6.91-6.88 (m, 2H), 5.40 (s, 2H), 4.20- 4.17 (m, 2H), 3.95 (s, 3H), 3.47 (s, 3H), 2.79 (t, J = 6.4 Hz, 2H), 2.04-1.98 (m, 2H).

Step 3

Methyl 3-(chroman-7-ylmethoxy)-5-[(2,4-dimethoxyphenyl) methylamino] isothiazole-4-carboxylate

Methyl 3-(chroman-7-ylmethoxy)-5-methylsulfonylisothiazol-4-carboxylate I426 (1.8 g, 4.69 mmol) was dissolved in THF(20 mL), followed by the addition of 2,4-dimethoxybenzylamine (7.8 g, 46.94 mmol), and the reaction system was stirred at 65 °C to react for 1 hour. After the reaction was finished, the reaction mixture was added to water (30 mL) and filtered, and the filter cake was dissolved in ethyl acetate, washed with saturated brine, dried with anhydrous sodium sulfate, and concentrated under reduced pressure to obtain a yellow solid product methyl 3-(chroman--7-ylmethoxy)-5-[(2,4-dimethoxyphenyl)methylamino]isothiazol-4-carboxylate I427 (2 g, 4.25 mmol, yield: 90.54%).

The product was verified by LCMS and H-NMR.

$^1$H NMR (400MHz, $CDCl_3$): δ (ppm) 8.11 (s, 1H), 7.15 (d, J = 8.2 Hz, 1H), 7.01 (d, J = 7.8 Hz, 1H), 6.90 (d, J = 4.0 Hz, 2H), 6.47-6.42 (m, 2H), 5.33 (s, 2H), 4.26 (d, J = 6.0 Hz, 2H), 4.19 -4.14 (m, 2H), 3.84 (s, 3H), 3.81 (s, 3H), 3.80 (s, 3H), 2.77 (t, J = 8.0 Hz, 2H), 2.02 -1.96 (m, 2H).

Step 4

Methyl 5-amino-3-(chroman-7-ylmethoxy)isothiazole-4-carboxylate

Methyl 3-(chroman--7-ylmethoxy)-5-[(2,4-dimethoxyphenyl)methylamino]isothiazol-4-carboxylate I427 (2 g, 4.25 mmol) was dissolved in DCM/$H_2O$ (21/5 mL), followed by the portionwise addition of DDQ (1.9 g, 8.50 mmol) at 0°C, and the reaction system was stirred at room temperature to react for 1 hour. After complete reaction, water (50 mL) was added to the reaction mixture, which was then extracted with DCM (20 mL x3), the organic phases were combined, washed with saturated brine, dried with anhydrous sodium sulfate, and concentrated under reduced pressure to obtain a crude product, and the crude product was purified by chromatographic columns (PE/EA = 10/1-3/1) to obtain a yellow solid product methyl 5-amino-3-(chroman-7-ylmethoxy)isothiazol-4-carboxylate I428 (770 mg, 2.40 mmol, yield: 56.55%).

The product was verified by LCMS and H-NMR.

$^1$H NMR (400 MHz, $CDCl_3$) δ (ppm) 7.03 (d, J = 7.5 Hz, 1H), 6.91 (d, J = 8.2 Hz, 2H), 6.42 (s, 2H), 5.35 (s, 2H), 4.22-4.16 (m, 2H), 3.85 (s, 3H), 2.78 (t, J = 6.4 Hz, 2H), 2.03-1.97 (m, 2H).

Step 5

Methyl 3-(chroman-7-ylmethoxy)-5-(4-pyrrolidin-1-ylbutylcarbamoylamino)isothiazole-4-carboxylate

4-(Pyrrolidin-1-yl)butyl-1-amine (372.9 mg, 2.62 mmol) was dissolved in anhydrous THF (7 mL) and then cooled to 0°C under the protection of $N_2$, followed by the addition of CDI (314.5 mg, 1.94 mmol), the mixture was stirred at room temperature to react for 1 hour, followed by the addition of DMSO(7 mL), the pressure was reduced to remove most of the THF, followed by the addition of methyl 5-amino-3-(chroman-7-ylmethoxy)isothiazol-4-carboxylate I428 (700 mg, 2.19

mmol) and potassium carbonate (301.9 mg, 2.19 mmol), and the reaction system was stirred at room temperature to react for 1 hour. After complete reaction, water (50 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-(chroman-7-ylmethoxy)-5-(4-pyrrolidin-1-ylbutylcarbamate)isothiazol-4-carboxylate I429 (250 mg, 511.67 μmol, yield: 23.42%).

The product was verified by LCMS and H-NMR.

$^1H$ NMR (400MHz, $CDCl_3$): δ (ppm) 10.23 (s, 1H), 7.83 (s, 1H), 7.02 (d, J = 8.1 Hz, 1H), 6.93-6.90 (m, 2H), 5.35 (s, 2H), 4.17 (t, J = 5.2 , 2H), 3.86 (s, 3H), 2.77 (t, J = 6.5 Hz, 2H), 2.62-2.49 (m, 8H), 2.04-1.98 (m, 4H), 1.87-1.84 (m, 6H).

Step 6

3-(chroman-7-ylmethoxy)-5-(4-pyrrolidin-1-ylbutylcarbamoylamino)isothiazole-4-carboxamide

In a microwave tube, methyl 3-(chroman-7-ylmethoxy)-5-(4-pyrrolidin-1-ylbutylcarbamate)isothiazol-4-carboxylate I429 (280 mg, 573.07 μmol) was dissolved in $NH_3$/MeOH (6 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-(chroman--7-ylmethoxy)-5-(4-pyrrolidin-1-ylbutylcarbamate)isothiazol-4-formamide T097 (33 mg, 69.68 μmol, yield: 12.16%).

The product was verified by LCMS, H-NMR and C-NMR.

$^1H$ NMR (400MHz, $CDCl_3$): δ (ppm) 10.91 (s, 1H), 7.68 (s, 1H), 7.18 (s, 1H), 7.05 (d, J = 7.7 Hz, 1H), 6.90-6.88 (m, 1H), 6.85 (s, 1H), 5.42 (s, 1H), 5.36 (s, 2H), 4.49 (t, J = 5.2 Hz, 2H), 3.31 (s, 2H), 2.79 (t, J = 6.5 Hz, 2H), 2.68 (s, 4H), 2.59 (d, J = 6.1 Hz, 2H), 2.13-1.98 (m, 6H), 1.68 (d, J = 3.2 Hz, 4H).

$^{13}C$ NMR (100MHz, $CDCl_3$) δ (ppm) 169.52, 165.90, 161.98, 155.05, 154.05, 135.14, 130.16, 122.59, 119.98, 116.49, 70.21, 66.51, 55.84, 53.91, 28.05, 26.59, 24.74, 23.40, 22.23.

Example 74: Synthesis of Compound T098

Synthesis Route:

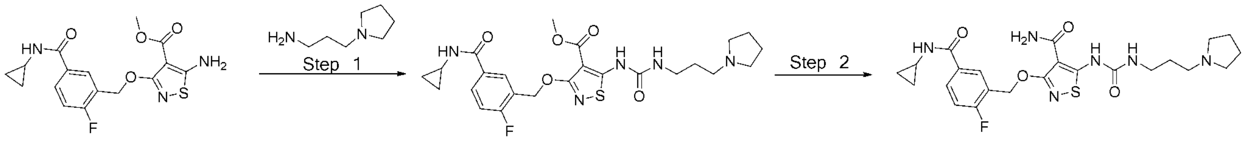


I376 1430 T098

Step 1

methyl 3-((5-(cyclopropylcarbamoyl)-2-fluorobenzyl)oxy)-5-(3-(3-(pyrrolidin-1-yl)propyl)ureido)isothiazole-4-carboxylate

3-(Pyrrolidin-1-yl)propyl-1-amine (100 mg, 780.01 μmol) was dissolved in anhydrous THF (4 mL) and then cooled to 0°C under the protection of $N_2$, followed by the addition of CDI (126 mg, 780.01 μmol), the mixture was stirred at room temperature to react for 1 hour, followed by the addition of DMSO(4 mL), the pressure was reduced to remove most of the THF, followed by the addition of methyl 5-amino-3-[[5-(cyclopropylcarbamoyl)-2-fluoro-phenyl]methoxy]isothiazol-4-carboxylate I376 (190 mg, 520.01 μmol) and potassium carbonate (215 mg, 1.56 mmol), and the reaction system was stirred at room temperature to react for 1 hour. After complete reaction, water (20 mL) was added to the reaction mixture, which was then extracted with ethyl acetate (10 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-((5-(cyclopropylcarbamoyl)-2-fluorobenzyl)oxy)-5-(3-(3-(pyrrolidin-1-yl)propyl)ureido)isothiazol-4-carboxylate I430 (150 mg, 288.69 μmol, yield: 55.52%).

The product was verified by LCMS and H-NMR.

$^1$H NMR (400 MHz, $CDCl_3$) δ 10.21 (s, 1H), 8.09 (d, J = 5.6 Hz, 1H), 7.86 (s, 1H), 7.70 (s, 1H), 7.14-7.05 (m, 1H), 6.29 (s, 1H), 5.49 (s, 2H), 3.93 (s, 3H), 3.47 (d, J = 15.6 Hz, 2H), 2.90 (s, 1H), 2.72 (d, J = 30.4 Hz, 6H), 1.86 (d, J = 34.8 Hz, 6H), 0.88 (d, J = 5.6 Hz, 2H), 0.63 (s, 2H).

Step 2

3-((5-(cyclopropylcarbamoyl)-2-fluorobenzyl)oxy)-5-(3-(3-(pyrrolidin-1-yl)propyl)ureido)isothiazole-4-carboxamide

In a microwave tube, methyl 3-((5-(cyclopropylcarbamoyl)-2-fluorobenzyl)oxy)-5-(3-(3-(pyrrolidin-1-yl)propyl) ureido)isothiazol-4-carboxylate I430 (100 mg, 192.46 μmol) was dissolved in $NH_3$/MeOH (14 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-((5-(cyclopropylcarbamoyl)-2-fluorobenzyl)oxy)-5-(3-(3-(pyrrolidin-1-yl)propyl)ureido)isothiazol-4-formamide T098 (25 mg, 49.55 μmol, yield: 25.74%).

The product was verified by LCMS, H-NMR, C-NMR and F-NMR.

$^1$H NMR (400 MHz, $CD_3OD$) δ 8.00 (dd, J = 7.2, 2.4 Hz, 1H), 7.85 (m, 1H), 7.34 (s, 1H), 7.24 (dd, J = 9.6, 8.8 Hz, 1H), 5.57 (s, 2H), 3.27 (d, J = 6.8 Hz, 2H), 2.86-2.79 (m, 1H), 2.67 (dd, J = 17.6, 9.6 Hz, 6H), 1.84 (m, 6H), 0.79 (dt, J = 7.2, 3.6 Hz, 2H), 0.66-0.59 (m, 2H).

$^{13}$C NMR (100 MHz, $CD_3OD$) δ 170.37, 170.25, 167.16, 165.70, 163.19, 162.99, 156.14, 132.12, 131.57, 131.07, 124.94, 116.85, 116.63, 65.17, 55.04, 54.80, 29.42, 29.71, 24.18, 24.08, 6.55. $^{19}$FNMR (400 MHz, $CD_3OD$) δ -115.43.

Example 75: Synthesis of Compound T099

Synthesis Route:

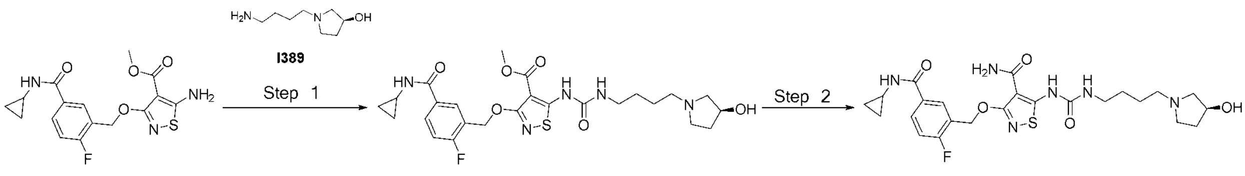


I376 I431 T099

Step 1

Methyl 3-[[5-(cyclopropylcarbamoyl)-2-fluoro-phenyl]methoxy]-5-[4-[(3S)-3-hydroxypyrrol idin-1-yl]butylcarbamoylamino]isothiazole-4-carboxylate

(3S)-1-(4-aminobutyl)pyrrolidin-3-ol I389 (110.4 mg, 697.91 μmol) was dissolved in anhydrous THF (6 mL) and then cooled to 0°C under the protection of $N_2$, followed by the addition of CDI (100.5 mg, 697.91 μmol), the mixture was stirred at room temperature to react for 1 hour, followed by the addition of DMSO(6 mL), the pressure was reduced to remove most of the THF, followed by the addition of methyl 5-amino-3-[[5-(cyclopropylcarbamoyl)-2-fluorophenyl] methoxy]isothiazol-4-carboxylate I376 (170 mg, 465.27 μmol) and potassium carbonate (192.6 mg, 1.40 mmol), and the reaction system was stirred at room temperature to react for 1 hour. After complete reaction, water (60 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[[5-(cyclopropylcarbamoyl)-2-fluoro-phenyl]methoxy]-5-[4-[(3S)-3-hydroxylpyrrol-1-yl]butylcarbamoylamino]isothiazol-4-carboxylate I431 (145 mg, 263.82 μmol, yield: 56.70%). The product was verified by LCMS and H-NMR.

$^1$H NMR (400MHz, $CDCl_3$): δ (ppm) 8.18 (d, J = 7.0 Hz, 1H), 7.82 (d, J = 2.8 Hz, 1H), 7.30-7.13 (m, 1H), 5.50 (d, J = 6.4 Hz, 2H), 3.95 (s, 3H), 3.31-3.03 (m, 7H), 2.87 (dd, J = 7.2, 3.4 Hz, 2H), 2.31-2.17 (m, 1H), 2.01 (s, 1H), 1.76 (s, 2H), 1.69-1.58 (m, 2H), 1.32 (s, 1H), 0.94-0.75 (m, 2H), 0.67 (s, 2H).

Step 2

3-[[5-(cyclopropylcarbamoyl)-2-fluoro-phenyl]methoxy]-5-[4-[(3S)-3-hydroxypyrrolidin-1-yl]butylcarbamoylamino]isothiazole-4-carboxamide

In a microwave tube, methyl 3-[[5-(cyclopropylcarbamoyl)-2-fluoro-phenyl]methoxy]-5-[4-[(3S)-3-hydroxylpyrrol-1-yl]butylcarbamoylamino]isothiazol-4-carboxylate I431 (145 mg, 263.82 μmol) was dissolved in $NH_3$/MeOH (6 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 5/1) to obtain a white solid product 3-[[5-(cyclopropylcarbamoyl)-2-fluoro-phenyl]methoxy]-5-[4-[(3S)-3-hydroxylpyrrolidin-1-yl]butylcarbamoylamino]isothiazol-4-formamide T099 (18 mg, 33.67 μmol, yield: 12.76%).

The product was verified by LCMS, H-NMR, C-NMR and F-NMR.

$^1$H NMR (400MHz, $CD_3OD$): δ (ppm) 8.02-7.99 (m, 1H), 7.87-7.83 (m, 1H), 7.24 (t, J = 9.6 Hz, 1H), 5.56 (s, 2H), 4.45-4.40 (m, 1H), 3.26 (t, J = 6.8 Hz, 2H), 3.11-3.02 (m, 2H), 2.92-2.75 (m, 5H), 2.22-2.13 (m, 1H), 1.86-1.83 (m, 1H), 1.68-1.57 (m, 4H), 0.82-0.75 (m, 2H), 0.65-0.61 (m, 2H).

$^{13}$C NMR (100 MHz, $CD_3OD$) δ 170.35, 170.28, 165.70, 163.68,163.21, 163.09, 156.21, 132.08, 131.58, 131.53, 131.10, 125.02, 116.85, 116.63, 70.94, 65.17, 63.21, 57.10, 53.77, 40.63, 34.63, 30.73, 28.47, 25.64, 24.08, 6.54.

$^{19}$F NMR (400MHz, $CD_3OD$): δ (ppm) -115.39.

Example 76: Synthesis of Compound T100

Synthesis Route:

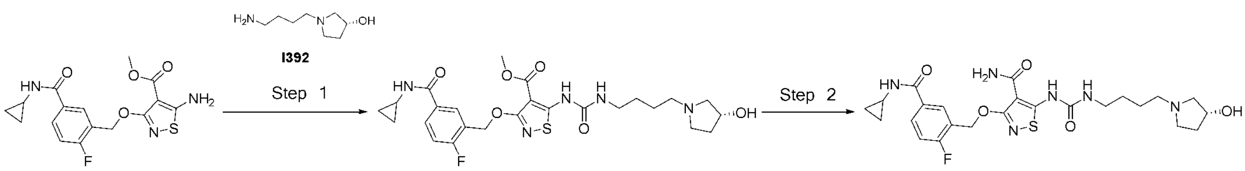


I376 1432 T100

Step 1

Methyl 3-[[5-(cyclopropylcarbamoyl)-2-fluoro-phenyl] methoxy]-5-[4-[(3R)-3-hydroxyp yrr olidin-1-yl]butylcarbamoylamino] isothiazole-4-carboxylate

(3R)-1-(4-aminobutyl)pyrrolidin-3-ol I392 (91.0 mg, 574.75 μmmol) was dissolved in anhydrous THF (5 mL) and then cooled to 0°C under the protection of $N_2$, followed by the addition of CDI (199.7 mg, 1.23 mmol), the mixture was stirred at room temperature to react for 1 hour, followed by the addition of DMSO(5 mL), the pressure was reduced to remove most of the THF, followed by the addition of methyl 5-amino-3-[[5-(cyclopropylcarbamoyl)-2-fluorophenyl] methoxy]isothiazol-4-carboxylate I376 (150 mg, 410.53 μmol) and potassium carbonate (170.2 mg, 1.23 mmol), and the reaction system was stirred at room temperature to react for 1 hour. After complete reaction, water (60 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[[5-(cyclopropylcarbamoyl)-2-fluoro-phenyl]methoxy]-5-[4-[(3R)-3-hydroxylpyrrolidin-1-yl]butylcarbamoylamino]isothiazol-4-carboxylate I432 (80 mg, 145.56 μmol, yield: 35.46%). The product was verified by LCMS and H-NMR.

$^1$H NMR (400MHz, $CDCl_3$): δ (ppm) 10.05 (s, 1H), 8.72 (s, 1H), 8.12 (s, 1H), 7.70 (s, 1H), 7.17-7.09 (m, 1H), 6.29-6.21 (m, 1H), 5.52 (s, 2H), 4.58 (s, 1H), 3.96 (s, 3H), 3.76 (s, 1H), 3.65-3.40 (m, 3H), 3.33-3.15 (m, 3H), 3.13-3.03 (m, 1H), 2.93 (s, 1H), 2.68 (s, 1H), 2.58 (s, 1H), 2.44 (s, 1H), 2.27 (s, 2H), 1.98 (s, 2H), 0.92 (s, 2H), 0.66 (s, 2H).

Step 2

3-[(5-fluoro-2,3-dihydrobenzofuran-6-yl)methoxy]-5-[3-(4-methylpiperazin-1-yl) propylcar bamoylamino] isothiazole-4-carboxamide

In a microwave tube, methyl 3-[[5-(cyclopropylcarbamoyl)-2-fluoro-phenyl]methoxy]-5-[4-[(3R)-3-hydroxylpyrrolidin-1-yl]butylcarbamoylamino]isothiazol-4-carboxylate I432 (80 mg, 145.56 μmol) was dissolved in $NH_3$/MeOH (6 mL,10 mol/L), and the mixture was sealed to react at 40°C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product methyl 3-[(5-fluoro-2,3-dihydrobenzofuran-6-yl) methoxy]-5-[3-(4-methylpiperazin-1-yl) propylcar bamoylamino] isothiazole-4-carboxamide T100 (20 mg, 37.41 μmol, yield: 25.70%).

The product was verified by LCMS, H-NMR, C-NMR and F-NMR.

$^1$HNMR (400MHz, $CD_3OD$): δ (ppm) 8.00 (dd, J = 7.4, 2.4 Hz, 1H), 7.88-7.81 (m, 1H), 7.28 -7.19 (m, 1H), 5.56 (s, 2H), 4.43-4.36 (m, 1H), 3.25 (t, J = 6.4 Hz, 2H), 3.00-2.91(m, 2H), 2.87-2.76 (m, 2H), 2.75-2.66 (m, 3H), 2.19-2.11 (m, 1H), 1.84-1.75 (m, 1H), 1.65-1.54 (m, 4H), 0.83-0.77 (m, 2H), 0.66-0.60 (m, 2H).

$^{13}$CNMR (100 MHz, $CD_3OD$): δ (ppm) 170.35, 170.23, 167.14, 165.69, 163.18, 162.98, 156.16, 132.10, 132.07, 131.57, 131.53, 131.15, 131.06, 125.09, 124.93, 116.84, 116.62, 98.76, 71.13, 65.16, 65.13, 63.34, 57.19, 53.77, 40.76, 34.83, 33.08, 30.76, 30.49, 28.60, 28.13, 26.07, 24.09, 23.75, 14.47, 6.56, -0.02

$^{19}$FNMR (400 MHz, $CDCl_3$): δ (ppm) -112.88.

Example 77: Synthesis of Compound T101

Synthesis Route:

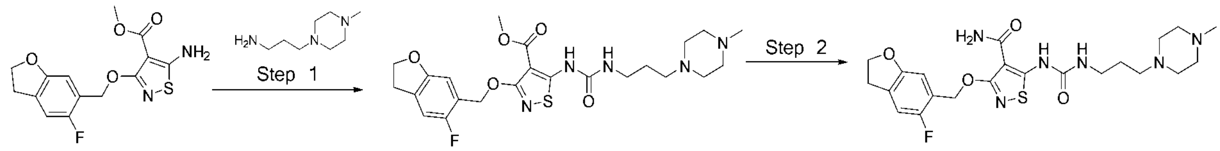


I311 I433 T101

Step 1

Methyl3-[(5-fluoro-2,3-dihydrobenzofuran-6-yl)methoxy]-5-[3-(4-methylpiperazin-1-yl)propylcarbamoylamino]isothiazole-4-carboxylate

3-(4-Methylpiperazin-1-yl)propyl-1-amine (237.6 mg, 1.51 mmol) was dissolved in anhydrous THF (3 mL) and then cooled to 0°C under the protection of $N_2$, followed by the addition of CDI (525 mg, 3.24 mmol), the mixture was stirred at room temperature to react for 1 hour, followed by the addition of DMSO(3 mL), the pressure was reduced to remove most of the THF, followed by the addition of methyl 5-amino-3-[(5-fluoro-2,3-dihydrobenzofuran-6-yl)methoxy]isothiazol-4-carboxylate I311 (350 mg, 1.08 mmol) and potassium carbonate (447.4 mg, 3.24 mmol), and the reaction system was stirred at room temperature to react for 1 hour. After complete reaction, water (60 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[(5-fluoro-2,3-dihydrobenzofuran-6-yl)methoxy]-5-[3-(4-methylpiperazin-1-yl)propylcarbamoylamino]isothiazol-4-carboxylate I433 (350 mg, 689.55 μmol, yield: 63.90%).

The product was verified by LCMS and H-NMR.

$^1$H NMR (400 MHz, $CDCl_3$) δ (ppm) 10.38 (s, 1H), 8.07 (s, 1H), 6.95 (d, J = 5.6 Hz, 1H), 6.91 (d, J = 9.2 Hz, 1H), 5.44 (s, 2H), 4.58 (t, J = 8.8 Hz, 2H), 3.88 (s, 3H), 3.73 (q, J = 7.2 Hz, 6H), 3.45 (d, J = 4.8 Hz, 2H), 3.20 (t, J = 9.2 Hz, 2H), 2.57 (d, J = 5.6 Hz, 3H), 2.37 (s, 2H), 1.74 (s, 2H).

Step 2

3-[(5-fluoro-2,3-dihydrobenzofuran-6-yl)methoxy]-5-[3-(4-methylpiperazin-1-yl) propylcar bamoylamino] isothiazole-4-carboxamide

In a microwave tube, methyl 3-[(5-fluoro-2,3-dihydrobenzofuran-6-yl)methoxy]-5-[3-(4-methylpiperazin-1-yl) propylcarbamoylamino]isothiazol-4-carboxylate I433 (200 mg, 394.03 μmol) was dissolved in $NH_3$/MeOH (10 mL,10 mol/L), and the mixture was stirred at 40 °C to react for 16 hours. The reaction mixture was concentrated under reduced pressure to obtain a crude product, and the crude product was purified by the preparative thin-layer chromatography

(DCM/MeOH = 10/1, 1% $NH_3.H_2O$) to obtain a white solid product methyl 3-[(5-fluoro-2,3-dihydrobenzofuran-6-yl) methoxy]-5-[3-(4-methylpiperazin-1-yl) propylcar bamoylamino] isothiazole-4-carboxamide T101 (56 mg, 113.69 μmol, yield: 28.85%).

The product was verified by LCMS, H-NMR, C-NMR and F-NMR.

$^1H$ NMR (400 MHz, $CD_3OD$) δ (ppm) 7.02 (d, J = 9.2 Hz, 1H), 6.82 (d, J = 5.6 Hz, 1H), 5.43 (s, 2H), 4.55 (t, J = 8.8 Hz, 2H), 3.30-3.18 (m, 6H), 2.64-2.41 (m, 8H), 2.30 (s, 3H), 1.77-1.70 (m, 2H).

$^{13}C$ NMR (100 MHz, $CD_3OD$&$CDCl_3$) δ (ppm) 170.27, 167.29, 163.22, 158.22, 157.53, 156.10, 155.85, 131.05, 130.96, 123.24, 123.07, 113.48, 113.23, 111.17, 111.13, 98.86, 79.51, 79.19, 78.86, 72.89, 65.71, 65.67, 56.90, 55.68, 54.78, 53.68, 46.14, 39.59, 30.93, 27.70.

$^{19}F$ NMR (400 MHz, $CD_3OD$) δ (ppm) -144.14.

Example 78: Synthesis of Compound T102

Synthesis Route:

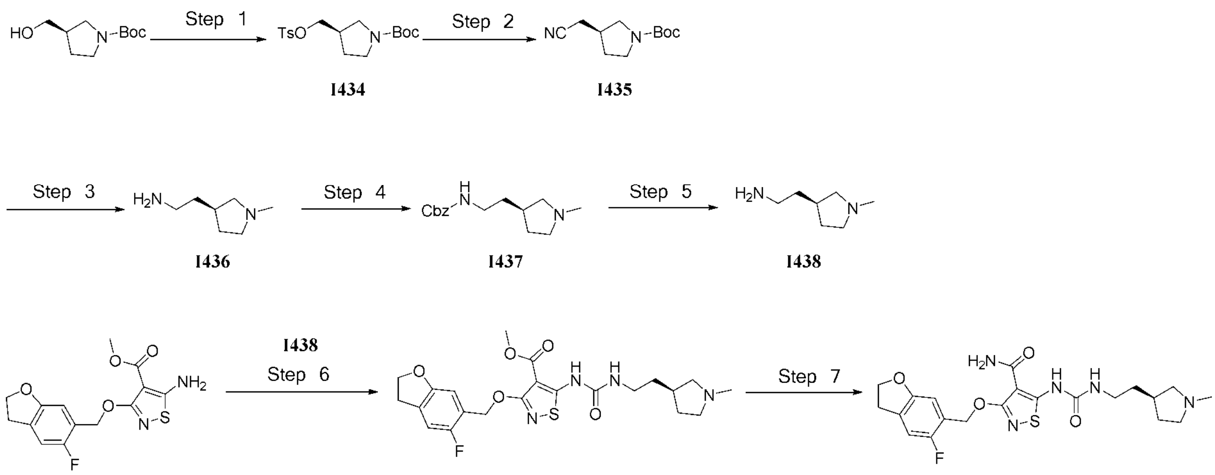


1434, 1435, 1436, 1437, 1438, 1311, 1439, T102

Step 1

Tert-butyl (3R)-3-(p-tolylsulfonyloxymethyl)pyrrolidine-1-carboxylate

Tert-butyl (3R)-3-(hydroxymethyl)pyrrolidin-1-carboxylate (7 g, 34.78 mmol) was dissolved in DCM (70 mL), followed by the addition of triethylamine (10.6 g, 104.34 mmol) and TsCl (9.9 g, 52.17 mmol) at room temperature, to allow for subsequent reaction for about 2 hours. After complete reaction, the mixture was poured into water and extracted with ethyl acetate, and the organic phases were combined, washed with saturated brine, dried with anhydrous sodium sulfate, and concentrated under reduced pressure to obtain a crude product, which was then purfied by the flash column chromatography (PE/EA = 10/1-3/1) to obtain a white solid product tert-butyl (3R)-3-(p-toluenesulfonyloxymethyl) pyrrolidin-1-carboxylate I434 (10 g, 28.13 mmol, yield: 80.89%). The product was verified by LCMS and H-NMR.

$^1H$ NMR (400MHz, $CDCl_3$ ): δ (ppm) 7.79 (d, J = 8.2 Hz, 2H), 7.36 (d, J = 8.2 Hz, 2H), 3.98-3.94 (m, 2H), 3.48-3.43 (m, 2H), 3.37-3.25 (m, 2H), 3.03-2.98 (m, 1H), 2.56-2.49 (m, 1H), 2.46 (s, 3H), 1.96 (d, J = 5.2 Hz, 1H), 1.62 (s, 1H), 1.44 (s, 9H).

Step 2

Tert-butyl (3S)-3-(cyanomethyl)pyrrolidine-1-carboxylate

Tert-butyl (3R)-3-(p-toluenesulfonyloxymethyl)pyrrolidin-1-carboxylate I434 (10 g, 28.13 mmol) was dissolved in DMSO (100 mL), followed by the addition of NaCN (1.5 g, 30.95 mmol) at room temperature, and the mixture was warmed to 100 °C and then stirred to react for about 16 hours. After complete reaction, the mixture was poured into water and extracted with ethyl acetate, and the organic phases were combined, washed with saturated brine, dried with anhydrous sodium sulfate, and concentrated under reduced pressure to obtain a yellow oily liquid product tert-butyl (3S)-3-(cyanomethyl)pyrrolidin-1-carboxylate I435 (5 g, 23.78 mmol, yield: 84.52%).

The product was verified by LCMS and H-NMR.

$^1$H NMR (400MHz, $CDCl_3$ ): δ (ppm) 7.79 (d, J = 8.2 Hz, 2H), 7.36 (d, J = 8.2 Hz, 2H), 3.98-3.94 (m, 2H), 3.48-3.43 (m, 2H), 3.37-3.25 (m, 2H), 3.03-2.98 (m, 1H), 2.56-2.49 (m, 1H), 2.46 (s, 3H), 1.96 (d, J = 5.2 Hz, 1H), 1.62 (s, 1H), 1.44 (s, 9H).

Step 3

2-[(3R)-1-methylpyrrolidin-3-yl]ethanamine

Tert-butyl (3S)-3-(cyanomethyl)pyrrolidin-1-carboxylate I435 (1 g, 4.76 mmol) was dissolved in dry THF (30 mL) and cooled to 0°C, followed by the addition of LAH (806.57 mg, 23.78 mmol), and the reaction system was stirred at room temperature to react for 30 minutes, and then warmed to 70 °C to react for 3 hours. The reaction was quenched with water (1 mL), followed by the slow addition of NaOH (15%, 1 mL) and water (3 mL), the reaction was stirred at room temperature for about 30 minutes and filtered to remove solids, and the filtrate was concentrated under reduced pressure to obtain a colorless oily liquid product 2-[(3R)-1-methylpyrrolidin-3-yl]ethylamine I436 (600 mg, 4.68 mmol, yield: 98.40%).

The product was verified by LCMS.

Step 4

Benzyl N-[2-[(3R)-1-methylpyrrolidin-3-yl]ethyl]carbamate

2-[(3R)-1-methylpyrrolidin-3-yl]ethylamine I436 (600 mg, 4.68 mmol) was dissolved in THF (20 mL), followed by the addition of $Na_2CO_3$ (1.5 g, 14.04 mmol) and benzyl chloroformate (1.6 g, 9.36 mmol) at room temperature, and then, the mixture was stirred to react for about 16 hours. The mixture was poured into water and extracted with ethyl acetate, the organic phases were combined, washed with saturated brine, dried with anhydrous sodium sulfate, and concentrated under reduced pressure to obtain a crude product, which was then purified by the medium-pressure preparative thin-layer chromatography (5%-40% $CH_3CN$ in $H_2O$) to obtain a colorless oily liquid product N-[2-[(3R)-1-methylpyrrolidin-3-yl]ethyl] benzyl carbamate I437 (200 mg, 762.35 μmol, yield: 16.29%).

The product was verified by LCMS and H-NMR.

$^1$H NMR (400MHz, $CD_3OD$): δ (ppm) 7.36 (d, J = 4.0 Hz, 5H), 5.16 (s, 1H), 5.09 (s, 1H), 3.21 (d, J = 5.2 Hz, 2H), 3.11-3.06 (m, 2H), 2.47-2.41 (m, 1H), 2.25-2.19 (m, 1H), 2.00 (s, 3H), 1.74-1.63 (m, 4H).

Step 5

2-[(3R)-1-methylpyrrolidin-3-yl]ethanamine

N-[2-[(3R)-1-methylpyrrolidin-3-yl]ethyl]benzyl carbamate I437 (150 mg, 571.76 μmol) was dissolved in methanol (10 mL), followed by the addition of $Pd(OH)_2/C$ (50 mg, 571.76 μmol) at room temperature, and in the atmosphere of hydrogen, the mixture was stirred to react for about 6 hours. After complete reaction, the mixture was filtered, the filter cake washed with methanol, the filtrates were combined and concentrated under reduced pressure to obtain a colorless oily liquid product 2-[(3R)-1-methylpyrrolidin-3-yl]ethylamine I438 (70 mg, 545.96 μmol, yield: 95.49%).

Step 6

Methyl 3-[(5-fluoro-2,3-dihydrobenzofuran-6-yl)methoxy]-5-[2-[(3R)-1-methylpyrrolidin-3-yl]ethylcarbamoylamino]isothiazole-4-carboxylate

2-[(3R)-1-methylpyrrolidin-3-yl]ethylamine I438 (79.1 mg, 616.66 μmol) was dissolved in anhydrous THF (4 mL) and then cooled to 0°C under the protection of $N_2$, followed by the addition of CDI (88.8 mg, 616.66 μmol), the mixture was stirred at room temperature to react for 1 hour, followed by the addition of DMSO (4 mL), the pressure was then reduced to remove most of the THF, followed by the addition of methyl 5-amino-3-[(5-fluoro-2,3-dihydrobenzofuran-6-yl)methoxy] isothiazol-4-carboxylate I311 (200 mg, 616.66 μmol) and potassium carbonate (255.3 mg, 1.85 mmol), and the reaction system was stirred at room temperature to react for 1 hour. After complete reaction, water (20 mL) was added to the reaction mixture, which was then extracted with ethyl acetate (20 mL x 3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[(5-fluoro-2,3-dihydrobenzofuran-6-yl)methoxy]-5-[2-[(3R)-1-methylpyrrolidin-3-yl]ethylcarbamoylamino]isothiazol-4-carboxylate I439 (80 mg, 167.18 μmol, yield: 27.11%). The product was verified by LCMS and H-NMR.

$^1H$ NMR (400 MHz, $CDCl_3$) δ (ppm) 10.30 (s, 1H), 7.42 (s, 1H), 6.85 (dd, J = 19.8, 7.4 Hz, 2H), 5.35 (s, 2H), 4.49 (s, 2H), 3.79 (s, 3H), 3.42 (s, 2H), 2.74 (s, 3H), 2.56 (s, 2H), 2.35-2.09 (m, 3H), 1.98 (s, 1H), 1.76-1.64 (m, 3H), 1.61-1.49 (m, 2H).

Step 7

3-[(5-fluoro-2,3-dihydrobenzofuran-6-yl)methoxy]-5-[2-[(3R)-1-methylpyrrolidin-3-yl]ethylcarbamoylamino]isothiazole-4-carboxamide

In a microwave tube, methyl 3-[(5-fluoro-2,3-dihydrobenzofuran-6-yl)methoxy]-5-[2-[(3R)-1-methylpyrrolidin-3-yl]ethylcarbamoylamino]isothiazol-4-carboxylate I439 (60 mg, 125.38 μmol) was dissolved in $NH_3$/MeOH (6 mL,10 mol/L), followed by the addition of 5 drops of DMSO at room temperature, and the mixture was sealed to react at 40 °C for 16 hours. The reaction mixture was concentrated under reduced pressure to obtain a crude product, and the crude product was purified by the preparative thin-layer chromatography (DCM/MeOH = 7/1) to obtain a white solid product 3-[(5-fluoro-2,3-dihydrobenzofuran-6-yl)methoxy]-5-[2-[(3S)-1-methylpyrrolidin-3-yl]ethylcarbamoylamino]isothiazol-4-formamide T102 (24 mg, 51.78 μmol, yield: 41.30%).

The product was verified by LCMS, H-NMR, C-NMR and F-NMR.

$^1H$ NMR (400MHz, $CD_3OD$): δ (ppm) 7.03 (d, J = 9.4 Hz, 1H), 6.83 (d, J = 5.8 Hz, 1H), 5.44 (s, 2H), 4.56 (t, J = 8.8 Hz, 2H), 3.23 (dd, J = 15.8, 6.6 Hz, 4H), 3.16-3.09 (m, 1H), 2.99-2.91 (m, 1H), 2.84 (s, 1H), 2.57 (s, 3H), 2.51-2.45 (m, 1H), 2.35 (dt, J = 15.2, 7.8 Hz, 1H), 2.17 (dt, J = 13.4, 6.7 Hz, 1H), 1.68-1.58 (m, 3H).

$^{13}C$ NMR (100 MHz, $CDCl_3$) δ (ppm) 169.38, 161.69, 156.83, 156.04, 154.45, 154.24, 129.25, 129.16, 121.74, 112.44, 112.19, 110.21, 110.17, 97.48, 71.69, 61.68, 53.44, 41.93, 30.68, 29.98, 29.69, 29.35, 22.68, 14.11.

$^{19}F$ NMR (100 MHz, $CDCl_3$) δ (ppm) -131.11

Example 79: Synthesis of Compound T103

Synthesis Route:

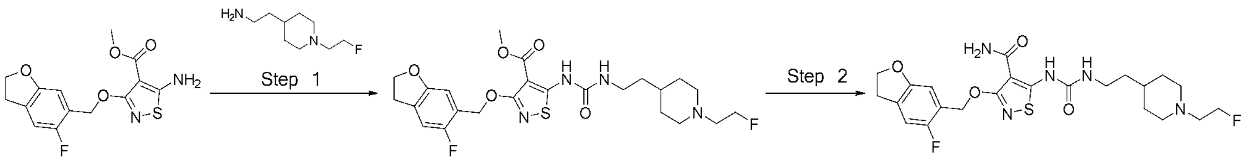


I311 I440 T103

Step 1

Methyl 3-[(5-fluoro-2,3-dihydrobenzofuran-6-yl)methoxy]-5-[2-[1-(2-fluoroethyl)-4-piperidyl]ethylcarbamoylamino]isothiazole-4-carboxylate

2-[1-(2-Fluoroethyl)-4-piperidyl]ethylamine (257.9 mg, 1.48 mmol) was dissolved in anhydrous THF (4 mL) and then cooled to 0°C under the protection of $N_2$, followed by the addition of CDI (240 mg, 1.48 mmol), the mixture was stirred at room temperature to react for 1 hour, followed by the addition of DMSO(4 mL), the pressure was reduced to remove most of the THF, followed by the addition of methyl 5-amino-3-[(5-fluoro-2,3-dihydrobenzofuran-6-yl)methoxy]isothiazol-4-carboxylate I311 (400 mg, 1.23 mmol) and potassium carbonate (511.4 mg, 3.70 mmol), and the reaction system was stirred at room temperature to react for 1 hour. After complete reaction, water (60 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[(5-fluoro-2,3-dihydrobenzofuran-6-yl)methoxy]-5-[2-[1-(2-fluoroethyl)-4-piperidyl] ethylcarbamoylamino]isothiazol-4-carboxylate I440 (300 mg, 571.89 μmol, yield: 46.37%).

The product was verified by LCMS and H-NMR.

$^1H$ NMR (400MHz, $CDCl_3$): δ (ppm) 10.33 (s,1H), 6.94-6.89 (m, 2H), 5.43 (s, 2H), 5.19 (s, 0H), 4.62 (t, J = 5.0 Hz, 1H), 4.57 (t, J = 8.7 Hz, 2H), 4.50 (t, J = 5.0 Hz, 1H), 3.88 (s, 3H), 3.36 (dd, J = 13.8, 6.6 Hz, 2H), 3.20 (t, J = 8.7 Hz, 2H), 2.96

(d, J = 10.8 Hz, 2H), 2.71 (t, J = 5.0 Hz, 1H), 2.64 (t, J = 5.0 Hz, 1H), 2.05 (t, J = 10.0 Hz, 2H), 1.71 (d, J = 9.8 Hz, 2H), 1.52 (s, 2H), 1.34 (s, 3H).

Step 2

3-[(5-fluoro-2,3-dihydrobenzofuran-6-yl)methoxy]-5-[2-[1-(2-fluoroethyl)-4-piperidyl] ethylcarbamoylamino]isothiazole-4-carboxamide

In a microwave tube, methyl 3-[(5-fluoro-2,3-dihydrobenzofuran-6-yl)methoxy]-5-[2-[1-(2-fluoroethyl)-4-piperidyl]ethylcarbamoylamino]isothiazol-4-carboxylate I440 (300 mg, 571.89 μmol) was dissolved in $NH_3$/MeOH (5 mL, 10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(5-fluoro-2,3-dihydrobenzofuran-6-yl)methoxy]-5-[2-[1-(2-fluoroethyl)-4-piperidyl]ethylcarbamoylamino]isothiazol-4-formamide T103 (54 mg, 105.97μmol, yield: 18.53%).

The product was verified by LCMS, H-NMR, C-NMR and F-NMR.

$^1$H NMR (400MHz, $CDCl_3$ ): δ (ppm) 11.05 (s, 1H), 7.18 (s, 1H), 6.94 (d, J = 9.0 Hz, 2H), 6.81 (d, J = 5.6 Hz, 2H), 5.48 (s, 1H), 5.45 (s, 2H), 4.69 (t, J = 4.0 Hz, 1H), 4.58 (t, J = 8.0 Hz, 2H), 3.34 (dd, J = 13.0, 6.6 Hz, 2H), 3.21 (t, J = 8.7 Hz, 2H), 3.06 (d, J = 10.9 Hz, 2H), 2.78 (d, J = 28.0 Hz, 2H), 2.21-2.10 (m, 1H), 1.75-1.73 (m, 2H), 1.57-1.48 (m, 1H), 1.45-1.33 (m, 2H).

$^{13}$C NMR (100MHz, $CDCl_3$) δ (ppm) 169.44 , 165.86, 161.65 , 156.88, 156.07 ,154.50, 153.99, 129.32, 129.23, 121.85, 121.69, 112.47 , 112.23 , 110.27 ,110.23, 82.17, 80.50 , 71.70 , 64.69, 64.65 , 58.32 , 58.12, 53.97, 36.15, 32.66 , 31.37 , 29.98, 29.69.

$^{19}$F NMR (400 MHz, $CDCl_3$) δ (ppm) -113.19, -128.64.

Example 80: Synthesis of Compound T104

Synthesis Route:

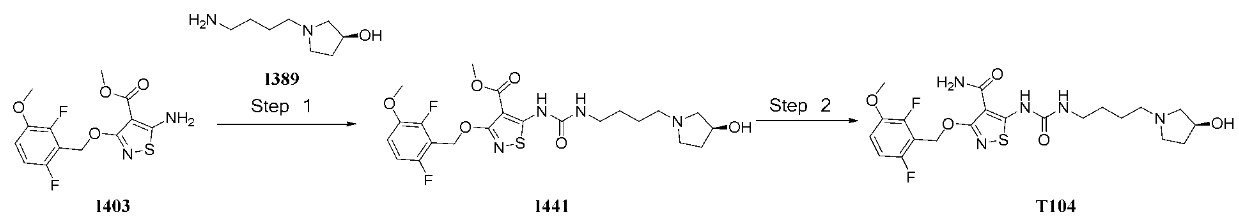


Step 1

Methyl 3-[(2,6-difluoro-3-methoxy-phenyl) methoxy]-5-[4-[(3S)-3-hydroxypyrrolidin-1-yl] butylcarbamoylamino] isothiazole-4-carboxylate

(3S)-1-(4-aminobutyl)pyrrolidin-3-ol I389 (161 mg, 1.0 mmol) was dissolved in anhydrous THF (5 mL) and then cooled to 0°C under the protection of $N_2$, followed by the addition of CDI (313.8 mg, 2.2 mmol), the mixture was stirred at room temperature to react for 1 hour, followed by the addition of DMSO(5 mL), the pressure was reduced to remove most of the THF, followed by the addition of methyl 5-amino-3-[(2,6-difluoro-3-methoxyphenyl)methoxy]isothiazol-4-carboxylate I403 (240 mg, 726.60 μmol) and potassium carbonate (301.3 mg, 2.18 mmol), and the reaction system was stirred at room temperature to react for 1 hour. After complete reaction, water (60 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[(2,6-difluoro-3-methoxyphenyl)methoxy]-5-[4-[(3S)-3-hydroxylpyrrolidin-1-yl]butylaminoacylamino] isothiazol-4-carboxylate I441 (210 mg, 408.1 μmol, yield: 56.17%).

The product was verified by LCMS and H-NMR.

$^1$H NMR (400 MHz, $CDCl_3$) δ 9.99 (s, 1H), 7.59 (s, 1H), 6.56-6.56 (m, 1H), 6.52-6.44 (m, 1H), 5.01 (s, 2H), 3.90 (s, 1H), 3.77 (s, 1H), 3.47 (d, J = 4.0 Hz, 3H), 3.35 (d, J = 4.0 Hz, 3H), 2.93 (s, 1H), 2.82 (s, 2H), 2.37-2.20 (m, 3H), 2.17 (d, J =

1.8 Hz, 3H), 2.13-1.93 (m, 4H), 1.68 (s, 1H), 1.29 (s, 1H), 1.15 (s, 4H).

Step 2

3-[(2,6-difluoro-3-methoxy-phenyl)methoxy]-5-[4-[(3S)-3-hydroxypyrrolidin-1-yl] butylcarbamoylamino] isothiazole-4-carboxamide

In a microwave tube, methyl 3-[(2,6-difluoro-3-methoxyphenyl)methoxy]-5-[4-[(3S)-3-hydroxylpyrrolidin-1-yl] butylaminoacylamino]isothiazol-4-carboxylate I441 (210 mg, 408.1 μmol) was dissolved in $NH_3$/MeOH (10 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(2,6-difluoro-3-methoxyphenyl)methoxy]-5-[4-[(3S)-3-hydroxylpyrrolidin-1-yl]butylcarbamate]isothiazol-4-formamide T104 (70 mg, 140.1 μmol, yield: 34.34%).
The product was verified by LCMS, H-NMR, C-NMR and F-NMR.
$^1$H NMR (400 MHz, $CD_3OD$) δ (ppm) 7.19-7.11 (m, 1H), 7.00-7.93 (m, 1H), 5.55 (s, 2H), 4.39 - 4.32 (m, 1H), 3.87 (s, 3H), 3.25 (t, J = 6.4 Hz, 2H), 2.91-2.85 (m, 1H), 2.83-2.76 (m, 1H), 2.68-2.60 (m, 1H), 2.56 (d, J = 7.2 Hz, 3H), 2.16-2.07 (m, 1H), 1.77-1.70 (m, 1H), 1.66-1.50 (m, 4H). $^{13}$C NMR (100 MHz, $CD_3OD$) δ (ppm) 170.36, 167.12, 162.90, 156.15, 145.89, 115.87, 111.50, 111.31, 111.27, 101.41, 71.33, 63.46, 59.47, 57.28, 53.76, 40.87, 40.44, 35.00, 28.74, 26.48.
$^{19}$F NMR (400 MHz, $CD_3OD$) δ (ppm) -127.69, -136.89.

Example 81: Synthesis of Compound T105

Synthesis Route:

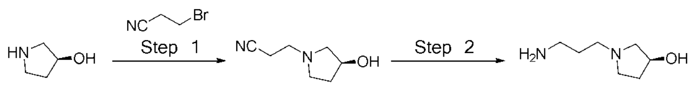


1442 1443

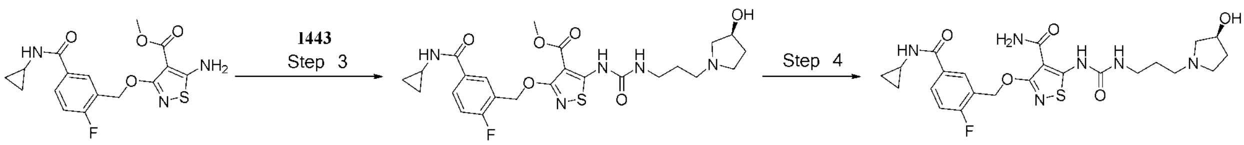


1376 1444 T105

Step 1

3-[(3S)-3-hydroxypyrrolidin-1-yl] propanenitrile

(3S)-pyrrolidin-3-ol (1 g, 11.48) was dissolved in acetonitrile (10 mL), followed by the addition of 3-bromopropionitrile (1.4 g, 10.33 mmol) and potassium carbonate (3.2 g, 22.96 mmol), and the reaction system was stirred at room temperature to react for 16 hours. The reaction mixture was poured into water (30 mL) and extracted with ethyl acetate (20 mL x 3), and the organic phases were combined, washed with saturated brine, dried with anhydrous sodium sulfate, and concentrated under reduced pressure to obtain a crude product, which was then purified by the flash column chromatography (DCM/MeOH = 1/0-10/1) to obtain a yellow oily product 3-[(3S)-3-hydroxylpyrrolidin-1-yl]propionitrile I442 (1.2 g, 8.56 mmol, yield: 74.58%).
The product was verified by LCMS and HNMR.
$^1$H NMR (400 MHz, $CDCl_3$) δ (ppm) 4.41-4.26 (m, 1H), 3.47 (d, J = 4.0 Hz, 1H), 3.03-2.87 (m, 1H), 2.86-2.70 (m, 3H), 2.59 (dd, J = 10.0, 5.2 Hz, 1H), 2.53 (t, J = 7.2 Hz, 2H), 2.36 (dd, J = 15.2, 8.8 Hz, 1H), 2.23-2.15 (m, 1H), 1.81-1.73 (m, 1H).

Step 2

(3S)-1-(3-aminopropyl) pyrrolidin-3-ol

3-[(3S)-3-hydroxylpyrrolidin-1-yl]propionitrile I442 (500 mg, 3.57 mmol) was dissolved in methanol (10 mL), followed by the addition of raney nickel (230.3 mg, 3.92 mmol), and in the atmosphere of hydrogen (20 psi), the reaction system was stirred at room temperature to react for 3 hours. The reaction mixture was filtered, and the filtrate was concentrated under reduced pressure to obtain a yellow oily liquid product (3S)-1-(3-aminopropyl)pyrrolidin-3-ol I443 (460 mg, 3.19 mmol, yield: 89.43%).
The product was verified by LCMS and H-NMR.
$^1$H NMR (400 MHz, $CDCl_3$) δ 4.33 (s, 1H), 3.00-2.60(m, 4H), 2.50 (s, 3H), 2.21 (s, 4H), 1.70 (s, 3H).

Step 3

Methyl 3-[[5-(cyclopropylcarbamoyl)-2-fluoro-phenyl]methoxy]-5-[3-[(3S)-3-hydroxyp yrro lidin-1-yl]propylcarbamoylamino] isothiazole-4-carboxylate

(3S)-1-(3-aminopropyl)pyrrolidin-3-ol I443 (138.1 mg, 957.91 μmol) was dissolved in anhydrous THF (5 mL) and then cooled to 0°C under the protection of $N_2$, followed by the addition of CDI (332.8 mg, 2.05 mmol), the mixture was stirred at room temperature to react for 1 hour, followed by the addition of DMSO(5 mL), the pressure was reduced to remove most of the THF, followed by the addition of methyl 5-amino-3-[[5-(cyclopropylcarbamoyl)-2-fluorophenyl] methoxy]isothiazol-4-carboxylate I376 (250 mg, 684.22 μmol) and potassium carbonate (283.7 mg, 2.05 mmol), and the reaction system was stirred at room temperature to react for 1 hour. After complete reaction, water (60 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a yellow solid product methyl 3-[[5-(cyclopropylcarbamoyl)-2-fluoro-phenyl]methoxy]-5-[3-[(3S)-3-hydroxylpyrrolidin-1-yl]propylcarbamate]isothiazol-4-carboxylate I444 (130 mg, 242.72 μmol, yield: 35.47%).
The product was verified by LCMS.

Step 4

3-[[5-(cyclopropylcarbamoyl)-2-fluoro-phenyl]methoxy]-5-[3-[(3S)-3-hydroxypyrrolidin-1-yl]propylcarbamoylamino]isothiazole-4-carboxamide

In a microwave tube, methyl 3-[[5-(cyclopropylcarbamoyl)-2-fluoro-phenyl]methoxy]-5-[3-[(3S)-3-hydroxylpyrrolidin-1-yl]propylcarbamate]isothiazol-4-carboxylate I444 (130 mg, 242.72 μmol) was dissolved in $NH_3$/MeOH (10 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[[5-(cyclopropylcarbamoyl)-2-fluorophenyl]methoxy]-5-[3-[(3S)-3-hydroxylpyrrolidin-1-yl]propylaminoacylamino]isothiazol-4-formamide T105 (40 mg, 76.84 μmol, yield: 31.66%).
The product was verified by LCMS, H-NMR, C-NMR and F-NMR.
$^1$H NMR (400MHz, $CD_3OD$): δ (ppm) 8.00 (dd, J = 7.2, 2.4 Hz, 1H), 7.88-7.82 (m, 1H), 7.27-7.20 (m, 1H), 5.57 (s, 2H), 4.38-4.32 (m, 1H), 3.28-3.24 (m, 2H), 2.86-2.80 (m, 2H), 2.78-2.73 (m, 1H), 2.62-2.50 (m, 4H), 2.16-2.09 (m, 1H), 1.80-1.69 (m, 3H), 0.84-0.76 (m, 2H), 0.66-0.57 (m, 2H). $^{13}$C NMR (100MHz, $CD_3OD$): δ (ppm) 170.36, 170.24, 167.15, 165.70, 163.18, 162.97, 156.11, 132.10, 132.06, 131.56, 131.51, 131.17, 131.08, 125.08, 124.93, 116.85, 116.63, 101.41, 98.73, 71.47, 65.16, 65.12, 63.53, 54.99, 53.77, 39.49, 35.15, 29.56, 24.08, 6.56.
$^{19}$F NMR (400MHz, $CD_3OD$): δ (ppm) -115.46.

Example 82: Synthesis of Compound T106

Synthesis Route:

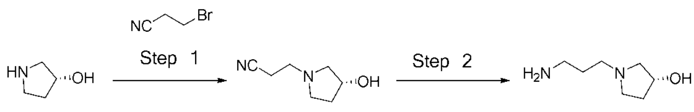


I445 I446

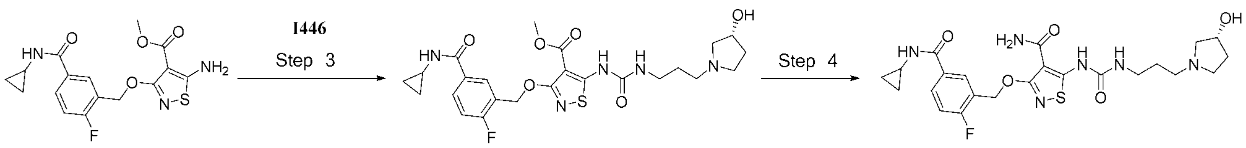


I376 I447 T106

Step 1

3-[(3R)-3-hydroxypyrrolidin-1-yl]propanenitrile

(3R)-pyrrolidin-3-ol (2 g, 22.96 mmol) was dissolved in acetonitrile (20 mL), followed by the addition of 3-bromopropionitrile (3.1 g, 22.96 mmol) and potassium carbonate (9.5 g, 68.87 mmol), and the reaction system was stirred at room temperature to react for 16 hours. The reaction mixture was filtered, and the filtrate was directly concentrated to obtain a crude product, which was then purified by the flash column chromatography (DCM/MeOH = 1/0-10/1) to obtain a yellow solid product 3-[(3R)-3-hydroxylpyrrolidin-1-yl]propionitrile I445 (2.8 g, 19.97 mmol, yield: 87.01%). The product was verified by LCMS and HNMR.

$^1$H NMR (400MHz, $CDCl_3$): δ 4.36-4.32 (m, 1H), 3.00-2.90 (m, 1H), 2.79-2.71 (m, 3H), 2.59 (dd, J=10.0 5.2 Hz 1H), 2.52 (t, J= 6.8 Hz 2H), 2.46 (s, 1H), 2.38-2.32 (m, 1H), 2.22-2.13 (m, 1H), 1.80-1.72 (m, 1H).

Step 2

(3R)-1-(3-aminopropyl)pyrrolidin-3-ol

3-[(3R)-3-hydroxylpyrrolidin-1-yl]propionitrile I445 (3.1 g, 22.11 mmol) was dissolved in methanol (30 mL), followed by the addition of raney nickel (868.3 mg, 14.79 mmol), and in the atmosphere of hydrogen (20 psi), the reaction system was stirred at room temperature to react for 16 hours. The reaction mixture was filtered, and the filtrate was concentrated under reduced pressure to obtain a green oily liquid product (3R)-1-(3-aminopropyl)pyrrolidin-3-ol I446 (3.1 g, 21.50 mmol, yield: 97.20%).

The product was verified by LCMS and H-NMR.

$^1$H NMR (400MHz, $CDCl_3$ ): δ 4.31 (s, 1H), 3.24 (s, 4H), 2.92-2.65 (m ,4H), 2.53-2.50 (m, 2H), 2.26-2.14 (m, 2H), 1.72-1.65 (m, 3H).

Step 3

Methyl3-[[5-(cyclopropylcarbamoyl)-2-fluoro-phenyl]methoxy]-5-[3-[(3R)-3-hydroxypyrrolidin-1-yl] propylcarbamoylamino] isothiazole-4-carboxylate

(3R)-1-(3-aminopropyl)pyrrolidin-3-ol I446 (148 mg, 1.03 mmol) was dissolved in anhydrous THF (10 mL) and then cooled to 0°C under the protection of $N_2$, followed by the addition of CDI (147.7 mg, 911.14 μmol), the mixture was stirred at room temperature to react for 1 hour, followed by the addition of DMSO(10 mL), the pressure was reduced to remove most of the THF, followed by the addition of methyl 5-amino-3-[[5-(cyclopropylcarbamoyl)-2-fluorophenyl] methoxy]isothiazol-4-carboxylate I376 (250 mg, 684.22 μmol) and potassium carbonate (283.7 mg, 2.05 mmol), and the reaction system was stirred at room temperature to react for 1 hour. After complete reaction, water (50 mL) was added to the reaction mixture, which was then extracted with ethyl acetate (30 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[[5-(cyclopropylcarbamoyl)-2-fluoro-phenyl]methoxy]-5-[3-[(3R)-3-hydroxylpyrrolidin-1-yl]propylaminoacylamino]isothiazol-4-carboxylate I447 (100 mg, 186.71 μmol, yield: 27.29%). The product was verified by LCMS and H-NMR.

$^1$H NMR (400MHz, $CDCl_3$ ): δ (ppm) 10.06 (s, 1H), 8.62 (s, 1H), 8.01-8.06 (m, 1H), 7.70-7.65 (m, 1H), 7.09 (t,

J=9.2 Hz 1H), 6.31 (s, 1h), 5.48 (s, 1H), 4.51 (s, 1H), 3.91 (s, 2H), 3.69 (s, 1H), 3.49 (s, 1H), 3.06-3.10 (m, 3H), 2.92-2.82 (m, 2H), 2.66-2.63 (m, 1H), 2.28-2.20 (m, 2H), 2.11 (d, J=7.2 Hz 1H), 1.89 (s, 1H), 1.77-1.71 (m, 3H), 0.88 (q, J=7.2 Hz 2H), 0.65-0.61 (m, 2H).

Step 4

3-[[5-(cyclopropylcarbamoyl)-2-fluoro-phenyl]methoxy]-5-[3-[(3R)-3-hydroxypyrrolidin-1-yl]propylcarbamoylamino]isothiazole-4-carboxamide

In a microwave tube, methyl 3-[[5-(cyclopropylcarbamoyl)-2-fluoro-phenyl]methoxy]-5-[3-[(3R)-3-hydroxylpyrrolidin-1-yl]propylaminoacylamino]isothiazol-4-carboxylate I447 (110 mg, 205.38 μmol) was dissolved in $NH_3$/MeOH (5 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[[5-(cyclopropylcarbamoyl)-2-fluoro-phenyl]methoxy]-5-[3-[(3R)-3-hydroxylpyrrolidin-1-yl]propylaminoacylamino]isothiazol-4-formamide T106 (29 mg, 61.75 μmol, yield: 23.46%). The product was verified by LCMS, H-NMR, C-NMR and F-NMR.

$^1H$ NMR (400MHz, $CD_3OD$): δ (ppm) 8.00 (dd, J=6.8, 2.4Hz 1H), 7.87-7.83 (m, 1H), 7.24 (t, J=9.6Hz 1H), 5.57 (s, 2H), 4.37-4.32 (m, 1H), 3.27 (t, J=7.2Hz 2H), 2.85-2.75 (m, 3H),2.61-2.53 (m, 4H), 2.17-2.08 (m, 1H),1.78-1.71 (m, 3H), 0.82-0.77 (m, 2H), 0.64-0.61 (m, 2H).

$^{13}C$ NMR (100 MHz, $CD_3OD$) δ (ppm) 170.36, 167.15, 165.71, 163.19, 162.98, 156.15, 132.08, 131.52, 131.17, 131.01, 125.09, 124.94, 116.85, 116.63, 71.38, 65.16, 63.48, 54.97, 53.78, 39.38, 35.06, 29.43, 24.07, 6.54.

$^{19}F$ NMR (400MHz, $CD_3OD$): δ (ppm) -115.45.

Example 83: Synthesis of Compound T107

Synthesis Route:

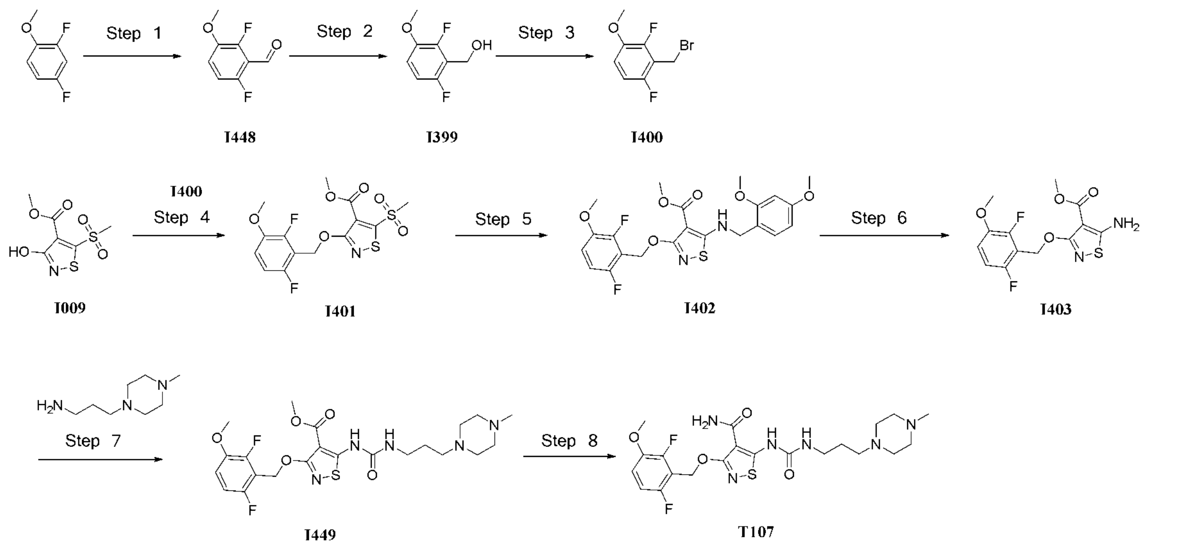

I448 I399 I400 I009 I401 I402 I403 I449 T107

Step 1

2,6-difluoro-3-methoxy-benzaldehyde

2,4-difluoro-1-metoxybenzene (23 g, 159.59 mmol) was dissolved in anhydrous THF (230 mL) and then cooled to -78°C under the protection of nitrogen, followed by the addition of n-BuLi (76.6 mL, 191.51 mmol, 2.5 mol/L n-hexane solution), the mixture was then stirred to react for 1 hour, followed by the addition of DMF (34.99 g, 478.77 mmol), and the mixture was then allowed to react at low temperature for about 1 hour. After complete reaction, the mixture was poured into saturated ammonium chloride solution (500 mL), and extracted with ethyl acetate (200 mL x 3), and the organic phases were combined, washed with saturated brine, dried with anhydrous sodium sulfate, and concentrated under reduced

pressure to obtain a yellow oily liquid product 2,6-difluoro-3-methoxybenzaldehyde I448 (23 g, 133.62 mmol, yield: 83.73%).

$^1$H NMR (400MHz, $CDCl_3$): δ (ppm) 10.35 (s, 1H), 7.20-7.13 (m, 1H), 6.95-6.89 (m, 1H), 3.90 (s, 3H).

Step 2

(2,6-difluoro-3-methoxy-phenyl)methanol

Under the protection of $N_2$, 2,6-difluoro-3-methoxybenzaldehyde I448 (23 g, 133.62 mmol) was dissolved in anhydrous ethanol(230 mL), followed by the addition of $NaBH_4$ (10.1 g, 267.24 mmol) in the ice water bath, and then, the mixture was stirred at room temperature to react for 0.5 hours. After complete reaction, the reaction mixture was quenched with water (500 mL) and extracted with ethyl acetate (100 mLx3), and the organic phases were combined, washed with saturated ammonium chloride and saturated brine (50 mL), dried with anhydrous sodium sulfate, and concentrated under reduced pressure to obtain a yellow oily liquid product (2,6-difluoro-3-methoxyphenyl)methanol I399 (23 g, 132.07 mmol, yield: 98.84%).

The product was verified by LCMS and H-NMR $^1$H NMR (400MHz, $CDCl_3$): δ (ppm) 6.85-6.80 (m, 2H), 4.78 (s, 2H), 3.84 (s, 3H), 1.94 (s, 1H).

Step 3

2-(bromomethyl)-1,3-difluoro-4-methoxy-benzene

(2,6-Difluoro-3-methoxyphenyl)methanol I399 (23 g, 132.07 mmol) was dissolved in DCM (230 mL) and then cooled to 0°C, followed by the addition of $PBr_3$ (42.9 g, 158.49 mmol), and the reaction system was stirred at room temperature to react for 1 hour. After complete reaction, the mixture was poured into water and extracted with dichloromethane, and the organic phases were combined, washed with saturated brine, dried with anhydrous sodium sulfate, and concentrated under reduced pressure to obtain a yellow oily liquid product 2-(bromomethyl)-1,3-difluoro-4-metoxybenzene I400 (17 g, 71.72 mmol, yield: 54.30%).

The product was verified by LCMS and H-NMR.

$^1$H NMR (400MHz, $CDCl_3$): δ (ppm) 6.92-6.81 (m, 2H), 4.53 (t, J=1.6 Hz, 2H), 3.87 (s, 3H).

Step 4

Methyl 3-[(2,6-difluoro-3-methoxy-phenyl)methoxy]-5-methylsulfonyl-isothiazole-4-carboxylate

Methyl 3-hydroxyl-5-(methylsulfonyl)isothiazol-4-carboxylate I009 (14 g, 59.01 mmol) and potassium carbonate (24.5 g, 177.03 mmol) were dissolved in DMF (140 mL), followed by the addition of 2-(bromomethyl)-1,3-difluoro-4-metoxybenzene I400 (16.8 g, 70.81 mmol), and the reaction system was stirred at room temperature to react for 1 hour. Water (600 mL) was added to the reaction mixture, which was then filtered, and the filter cake was dissolved in ethyl acetate, washed with water and saturated brine, dried with anhydrous sodium sulfate, and concentrated under reduced pressure to obtain a yellow solid product methyl 3-[(2,6-difluoro-3-methoxyphenyl)methoxy]-5-methylsulfonylisothiazol-4-carboxylate I401 (18 g, 45.76 mmol, yield: 77.54%).

The product was verified by LCMS.

Step 5

Methyl 3-[(2,6-difluoro-3-methoxy-phenyl)methoxy]-5-[(2,4-dimethoxyphenyl) methylamino]isothiazole-4-carboxylate

Methyl 3-[(2,6-difluoro-3-methoxyphenyl)methoxy]-5-methylsulfonylisothiazol-4-carboxylate I401 (15 g, 38.13 mmol) was dissolved in THF(300 mL), followed by the addition of 2,4-dimethoxybenzylamine (63.8 g, 381.31 mmol), and the reaction system was stirred at 65 °C to react for 1 hour. After the reaction was finished, the reaction mixture was added to water (500 mL) and then filtered, and the filter cake was dissolved in ethyl acetate, washed with saturated brine, dried with anhydrous sodium sulfate, and concentrated under reduced pressure to obtain a yellow solid product methyl 3-[(2,6-difluoro-3-methoxyphenyl)methoxy]-5-[(2,4-dimethoxyphenyl)methylamino]isothiazol-4-carboxylate I402 (17 g, 35.38 mmol, yield: 92.79%). The product was verified by LCMS and H-NMR.

$^1$H NMR (400MHz, $CDCl_3$): δ (ppm) 8.01 (s, 1H), 7.16 (d, J=8.4Hz, 1H), 6.95-6.89 (m, 1H), 6.86-6.81 (m, 1H), 6.47-6.41 (m, 2H), 5.46 (s, 2H), 4.26 (d, J=6.0Hz, 2H), 3.87 (s, 3H), 3.84 (s, 3H), 3.80 (s, 3H), 3.72 (s, 3H).

Step 6

Methyl 5-amino-3-[(2,6-difluoro-3-methoxy-phenyl)methoxy]isothiazole-4-carboxylate Methyl 3-[(2,6-difluoro-3-methoxyphenyl)methoxy]-5-[(2,4-dimethoxyphenyl)methylamino]isothiazol-4-carboxylate I402 (5 g, 10.41 mmol) was dissolved in DCM/$H_2O$ (200/40 mL), followed by the portionwise addition of DDQ (7.1 g, 31.22 mmol) at 0°C, and the reaction system was stirred at room temperature to react for 1 hour. After complete reaction, water (1000 mL) was added to the reaction mixture, which was then extracted with DCM (300 mL x2), the organic phases were combined, washed with saturated brine, dried with anhydrous sodium sulfate, and concentrated under reduced pressure to obtain a crude product, and the crude product was purified by chromatographic columns (neutral alumina, PE/EA = 100/0-3/1) to obtain a yellow solid product methyl 5-amino-3-[(2,6-difluoro-3-methoxyphenyl)methoxy]isothiazol-4-carboxylate I403 (2.7 g, 8.17 mmol, yield: 78.55%).

The product was verified by LCMS and H-NMR.

$^1$H NMR (400MHz DMSO-d6): $\delta$ (ppm) 7.89 (s, 2H), 7.27-7.21 (m, 1H), 7.12-7.07 (m, 1H), 5.32 (s, 2H), 3.84 (s, 3H), 3.62 (s, 3H).

Step 7

Methyl 3-[(2,6-difluoro-3-methoxy-phenyl)methoxy]-5-[3-(4-methylpiperazin-1-yl) propylcarbamoylamino]isothiazole-4-carboxylate

3-(4-methylpiperazin-1-yl)propyl-1-amine (178.5 mg, 1.14 mmol) was dissolved in anhydrous THF (10 mL) and then cooled to 0°C under the protection of $N_2$, followed by the addition of CDI (184.1 mg, 1.14 mmol), the mixture was stirred at room temperature to react for 1 hour, followed by the addition of DMSO(10 mL), the pressure was reduced to remove most of the THF, followed by the addition of methyl 5-amino-3-[(2,6-difluoro-3-methoxyphenyl)methoxy]isothiazol-4-carboxylate I403 (250 mg, 756.87 $\mu$mol) and potassium carbonate (313.8 mg, 2.27 mmol), and the reaction system was stirred at room temperature to react for 2 hours. After complete reaction, water (50 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[(2,6-difluoro-3-methoxyphenyl)methoxy]-5-[3-(4-methylpiperazin-1-yl)propylcarbamoylamino]isothiazol-4-carboxylate I449 (250 mg, 486.80 $\mu$mol, yield: 64.32%).

The product was verified by LCMS and H-NMR.

$^1$H NMR (400MHz, $CDCl_3$): $\delta$ (ppm) 10.33 (s, 1H), 6.95-6.89 (m, 1H), 6.86-6.81 (m, 1H), 5.47 (s, 2H), 3.86 (s, 3H), 3.75 (s, 3H), 3.30 (s, 5H), 2.61-2.57 (m, 6H), 2.39 (s, 3H), 1.80-1.76 (m, 4H).

Step 8

3-[(2,6-difluoro-3-methoxy-phenyl)methoxy]-5-[3-(4-methylpiperazin-1-yl) propylcarbamoylamino]isothiazole-4-carboxamide

In a microwave tube, methyl 3-[(2,6-difluoro-3-methoxyphenyl)methoxy]-5-[3-(4-methylpiperazin-1-yl)propylcarbamoylamino]isothiazol-4-carboxylate I449 (250 mg, 486.80 $\mu$mol) was dissolved in $NH_3$/MeOH (10 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[(2,6-difluoro-3-methoxyphenyl)methoxy]-5-[3-(4-methylpiperazin-1-yl)propylcarbamoylamino]isothiazol-4-formamide T107 (61 mg, 122.36 $\mu$mol, yield: 25.13%).

The product was verified by LCMS, H-NMR, C-NMR and F-NMR.

$^1$H NMR (400MHz, $CD_3OD$): $\delta$ (ppm) 7.18-7.12 (m, 1H), 6.99-6.94 (m, 1H), 5.55 (s, 2H), 3.87 (s, 2H), 3.26 (t, J=6.8 Hz, 2H), 2.52-2.42 (m, 11H), 2.29 (s, 3H), 1.78-1.71 (m, 2H).

$^{13}$C NMR (101 MHz, $CD_3OD$) $\delta$ (ppm) 170.32, 167.10, 162.89, 157.55, 157.50, 156.08, 155.14, 155.08, 153.62, 151.12, 151.05, 145.98, 145.87, 115.80, 115.70, 114.07, 113.92, 113.87, 113.71, 111.54, 111.49, 111.31, 111.26, 101.41, 98.64, 59.48, 57.27, 56.81, 55.61, 53.61, 45.90, 39.49, 27.66.

$^{19}$F NMR (400MHz, $CD_3OD$): $\delta$ (ppm) -127.69, -136.90.

Example 84: Synthesis of Compound T108

Synthesis Route:

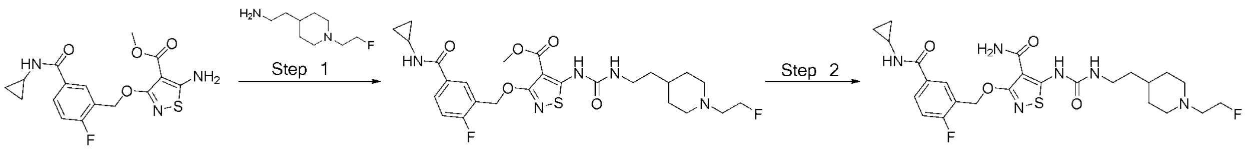


I376 I450 T108

Step 1

Methyl 3-[[5-(cyclopropylcarbamoyl)-2-fluoro-phenyl]methoxy]-5-[2-[1-(2-fluoroethyl)-4-piperidyl]ethylcarbamoylamino] isothiazole-4-carboxylate

2-[1-(2-fluoroethyl)-4-piperidyl]ethylamine (300.5 mg, 1.72 mmol) was dissolved in anhydrous THF (6mL) and then cooled to 0°C under the protection of $N_2$, followed by the addition of CDI (279.6 mg, 1.72 mmol), the mixture was stirred at room temperature to react for 1 hour, followed by the addition of DMSO(6 mL), the pressure was reduced to remove most of the THF, followed by the addition of methyl 5-amino-3-[[5-(cyclopropylcarbamoyl)-2-fluorophenyl] methoxy]isothiazol-4-carboxylate I376 (450 mg, 1.23 mmol) and potassium carbonate (510.6 mg, 3.69 mmol), and the reaction system was stirred at room temperature to react for 1 hour. After complete reaction, water (60 mL) was added to the reaction mixture, which was then extracted with ethyl acetate (20 mLx3), the organic phases were sequentially washed with water and saturated brine, dried with anhydrous sodium sulfate, and concentrated under reduced pressure to obtain a crude product, and the crude product was purified by the preparative thin-layer chromatography (DCM/MeOH = 10/1, 1% $NH_3.H_2O$) to obtain a white solid product methyl 3-[[5-(cyclopropylcarbamoyl)-2-fluoro-phenyl]methoxy]-5-[2-[1-(2-fluoroethyl)-4-piperidyl]ethylcarbamoylamino]isothiazol-4-carboxylate I450 (300 mg, 530.38$\mu$mol, yield: 43.06%).

The product was verified by LCMS and H-NMR.

$^1$H NMR (400MHz, $CDCl_3$): $\delta$ (ppm) 10.32 (s, 1H), 8.10-8.08 (m, 1H), 7.69-7.63 (m, 1H), 7.12-7.08 (m, 1H), 6.26 (s, 1H), 5.50 (s, 2H), 4.62 (t, *J* = 5.0 Hz, 1H), 4.50 (t, *J* = 5.0 Hz, 1H), 3.91 (s, 3H), 3.37-3.20 (m, 2H), 2.96 (d, *J* = 10.8 Hz, 2H), 2.92-2.86 (m, 1H), 2.71 (t, *J* = 5.0 Hz, 1H), 2.64 (t, *J* = 5.0 Hz, 1H), 2.05 (t, *J* = 10.1 Hz, 2H), 1.53-1.46 (m, 3H), 1.37-1.27(m, 4H), 0.90-0.85 (m, 2H), 0.64-0.59 (m, 2H).

Step 2

3-[[5-(cyclopropylcarbamoyl)-2-fluoro-phenyl]methoxy]-5-[2-[1-(2-fluoroethyl)-4-piperidyl]ethylcarbamoylamino]isothiazole-4-carboxamide

In a microwave tube, methyl 3-[[5-(cyclopropylcarbamoyl)-2-fluoro-phenyl]methoxy]-5-[2-[1-(2-fluoroethyl)-4-piperidyl]ethylcarbamoylamino]isothiazol-4-carboxylate I450 (450 mg, 795.57 $\mu$mol) was dissolved in $NH_3$/MeOH (10 mL,10 mol/L), and the mixture was sealed to react at 40 °C for 16 hours. The reaction mixture was concentrated under reduced pressure to obtain a crude product, and the crude product was purified by the preparative thin-layer chromatography (DCM/MeOH = 10/1, 1% $NH_3.H_2O$) to obtain a white solid product 3-[[5-(cyclopropylcarbamoyl)-2-fluoro-phenyl] methoxy]-5-[2-[1-(2-fluoroethyl)-4-piperidyl]ethylcarbamoylamino]isothiazol-4-formamide T108 (81 mg, 147.11$\mu$mol, yield: 18.49%).

The product was verified by LCMS, H-NMR and C-NMR.

$^1$H NMR (400MHz $CD_3OD$): $\delta$ (ppm) 8.01 (d, *J* = 7.0 Hz, 1H), 7.86 (s, 1H), 7.25 (t, *J* = 9.2 Hz, 1H), 5.58 (s, 2H), 4.67-4.58 (m, 1H), 4.54-4.47 (m, 1H), 3.26-3.24 (m, 2H), 3.01 (d, *J* = 11.0 Hz, 2H), 2.87-2.80 (m, 1H), 2.75 (t, *J* = 4.0 Hz, 1H), 2.68 (t, *J* = 4.8 Hz, 1H), 2.14 (t, *J* = 11.0 Hz, 2H), 1.94 (d, *J* = 1.0 Hz, 1H), 1.77 (d, *J* = 13.6 Hz, 2H), 1.50 (d, *J* = 6.6 Hz, 2H), 1.34-1.28 (m, 2H), 0.80 (d, J = 6.0 Hz, 2H), 0.63 (s, 2H).

$^{13}$C NMR (400MHz, $CD_3OD$): $\delta$ (ppm) 170.35, 170.20, 167.14, 165.68, 163.17, 162.96, 156.06, 132.08, 132.05, 131.57, 131.52, 131.16, 131.07, 125.07, 124.92, 116.84, 116.62, 98.72, 83.11, 81.45, 65.16, 65.13, 59.58, 59.38, 55.09, 38.67, 37.23, 33.94, 32.53, 24.10, 6.57.

Example 85: Synthesis of Compound T109

Synthesis Route:

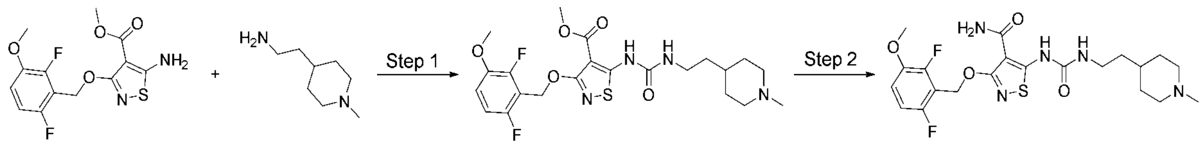

I403 I451 T109

Step 1

Methyl 3-[(2,6-difluoro-3-methoxy-phenyl)methoxy]-5-[2-(1-methyl-4-piperidyl) ethylcarbamoylamino]isothiazole-4-carboxylate

2-(1-Methyl-4-piperidyl)ethylamine (161.5 mg, 1.1 mmol) was dissolved in anhydrous THF (5 mL), followed by the addition of CDI (163.4 mg, 1.0 mmol) under the protection of $N_2$, the mixture was then stirred at 25°C to react for 1.0 hour, followed by the addition of DMSO (5 mL), the pressure was reduced to remove THF, followed by the addition of 5-amino-3-[(2,6-difluoro-3-methoxyphenyl)methoxy]isothiazol-4-methyl formate I403 (250.0 mg, 756.9 μmol) and potassium carbonate (313.8 mg, 2.3 mmol), and the reaction system was stirred at 25 °C to react for 1.0 hour. Water (30 mL) was poured into the reaction mixture, which was then subjected to liquid separation and extraction with ethyl acetate (10 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3-methoxyphenyl) methoxy]-5-[2-(1-methyl-4-piperidyl)ethylcarbamoylamino]isothiazol-4-carboxylate I451 (250.0 mg, 501.5 μmol, yield: 66.3%).

The product was verified by LCMS and H-NMR.

$^1H$ NMR (400MHz, $CDCl_3$): δ (ppm) 10.33 (s, 1H), 6.96-6.90 (m, 1H), 6.87-6.81 (m, 1H), 5.48 (s, 2H), 3.87 (s, 3H), 3.76 (s, 3H), 3.34 (d, J=6.4Hz, 2H), 3.03-2.96 (m, 4H), 2.39 (s, 3H), 2.13 (t, J=9.6Hz, 2H), 1.76 (d, J=10.0Hz, 2H), 1.45-1.41 (m, 4H).

Step 2

3-[(2,6-difluoro-3-methoxy-phenyl)methoxy]-5-[2-(1-methyl-4-piperidyl)ethylcarbamoylamino]isothiazole-4-carboxamide

Methyl 3-[(2,6-difluoro-3-methoxyphenyl)methoxy]-5-[2-(1-methyl-4-piperidyl)ethylcarbamoylamino]isothiazol-4-carboxylate I451 (250.0 mg, 501.5 μmol) was dissolved in $NH_3$/MeOH (10 mL, 10 mol/L). The mixture was sealed to react at 40 °C for 16 hours. The reaction mixture was concentrated under reduced pressure to obtain a crude product, and the crude product was purified by the column chromatography (DCM/MeOH = 10/1) to obtain a white solid product 3-[(2,6-difluoro-3-methoxyphenyl)methoxy]-5-[2-(1-methyl-4-piperidyl)ethylcarbamoylamino]isothiazol-4-formamide T109 (32.0 mg, 66.2 μmol, yield: 13.20%).

The product was verified by LCMS, H-NMR, C-NMR and F-NMR.

$^1H$ NMR (400MHz, $CD_3OD$): δ (ppm) 7.18-7.12 (m, 1H), 6.99-6.94 (m, 1H), 5.55 (s, 2H), 3.87 (s, 3H), 3.26 (t, J=7.2Hz, 2H), 2.90 (d, J=11.6Hz, 2H), 2.29 (s, 3H), 2.07 (t, J=10.8Hz, 2H), 1.77 (d, J=13.2Hz, 2H), 1.52-1.46 (m, 2H), 1.33-1.24 (m, 3H).

$^{13}C$ NMR (100MHz, $CD_3OD$): δ (ppm) 170.34, 167.12, 162.89, 157.49, 156.07, 155.08, 145.91, 115.81, 115.72, 114.06, 113.86, 111.55, 111.50, 111.32, 111.27, 99.62, 59.49, 57.28, 56.53, 46.15, 38.66, 37.16, 33.44, 32.55, 30.75.

$^{19}F$ NMR (400MHz, $CD_3OD$): δ (ppm) -127.69, -136.89.

Example 86: Synthesis of Compound T110

Synthesis Route:

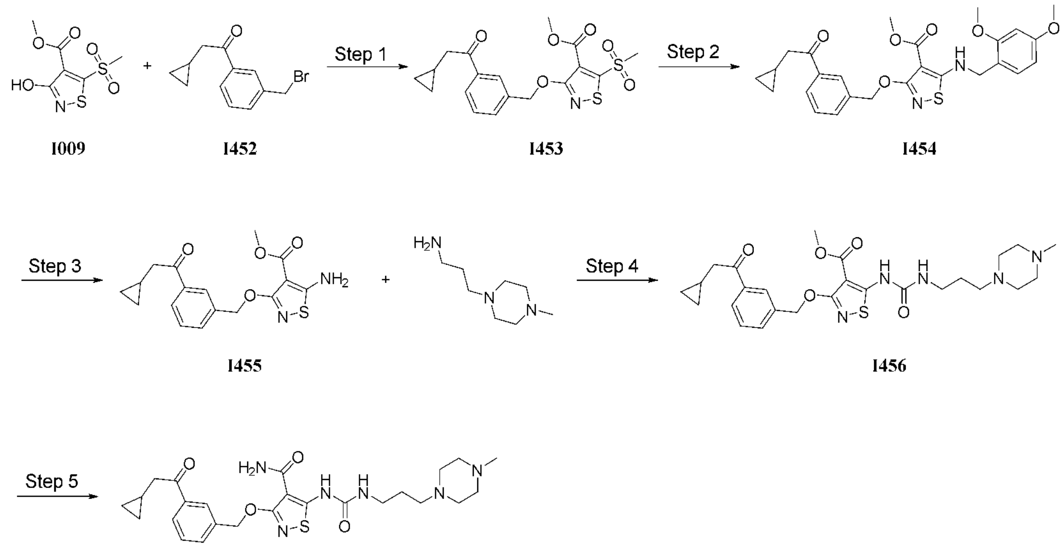


I009 I452 I453 I454

I455 I456

T110

Step 1

Methyl 3-[[3-(2-cyclopropylacetyl) phenyl] methoxy]-5-methylsulfonyl-isothiazole-4-carboxylate

At room temperature, methyl 3-hydroxyl-5-(methylsulfonyl)isothiazol-4-carboxylate I009 (1 g, 4.2 mmol) was dissolved in DMF (10 mL), followed by the addition of 1-[3-(bromomethyl)phenyl]-2-cyclopropyl-ethanone I452 (1.1 g, 4.2 mmol) and potassium carbonate (1.8 g, 12.6 mmol). The reaction system was stirred at 25 °C to react for 2.0 hours. The mixture was poured into water (100 mL) and filtered, and the filter cake was dissolved in ethyl acetate, dried with anhydrous sodium sulfate, and concentrated under reduced pressure to obtain a white solid product methyl 3-[[3-(2-cyclopropylacetyl)phenyl]methoxy]-5-methylsulfonylisothiazol-4-carboxylate I453 (700 mg, 1.7 mmol, yield: 40.56%).
The product was verified by LCMS and H-NMR.
$^1$H NMR (400MHz, $CDCl_3$): δ (ppm) 8.08 (s, 1H), 7.92 (d, J = 7.8 Hz, 1H), 7.65 (d, J = 7.6 Hz, 1H), 7.50 (t, J = 7.6 Hz, 1H), 5.54 (s, 2H), 3.99 (s, 3H), 3.49 (s, 3H), 2.90 (t, J = 4.8 Hz, 2H), 1.17 (s, 1H), 0.79 - 0.53 (m, 2H), 0.20 (q, J = 4.8 Hz, 2H).

Step 2

Methyl 3-[[3-(2-cyclopropylacetyl) phenyl] methoxy]-5-[(2,4-dimethoxyphenyl) methylamino] isothiazole-4-carboxylate

Methyl 3-[[3-(2-cyclopropylacetyl)phenyl]methoxy]-5-methylsulfonylisothiazol-4-carboxylate I453 (700 mg, 1.7 mmol) was dissolved in THF (10 mL), followed by the addition of 2,4-dimethoxybenzylamine (2.9 g, 17.1 mmol). The reaction system was stirred at 65 °C to react for 1.0 hour. The mixture was poured into water (50 mL) and filtered, and the filter cake was dissolved in ethyl acetate, dried with anhydrous sodium sulfate, and concentrated under reduced pressure to obtain a yellow solid product methyl 3-[[3-(2-cyclopropylacetyl)phenyl]methoxy]-5-[(2,4-dimethoxyphenyl)methylamino]isothiazol-4-carboxylate I454 (700 mg, 1.4 mmol, yield: 82.46%). The product was verified by LCMS and H-NMR.
$^1$H NMR (400MHz, $CDCl_3$): δ (ppm) 8.11 (t, J = 11.4 Hz, 2H), 7.88 (d, J = 7.8 Hz, 1H), 7.63 (d, J = 8.0 Hz, 1H), 7.46 (t, J = 7.6 Hz, 1H), 7.16 (d, J = 8.2 Hz, 1H), 6.48-6.42 (m, 2H), 5.47 (s, 2H), 4.27 (d, J = 6.0 Hz, 2H), 3.86 (s, 2H), 3.85 (s, 2H), 3.80 (s, 3H), 3.49 (d, J = 1.5 Hz, 2H), 2.90 (dd, J = 6.8, 2.8 Hz, 2H), 1.16 (d, J = 7.2 Hz, 1H), 0.64-0.55 (m, 2H), 0.23-0.16 (m, 2H).

Step 3

Methyl 5-amino-3-[[3-(2-cyclopropylacetyl) phenyl] methoxy] isothiazole-4-carboxylate

Methyl 3-[[3-(2-cyclopropylacetyl)phenyl]methoxy]-5-[(2,4-dimethoxyphenyl)methylamino]isothiazol-4-carboxylate I454 (700 mg, 1.4 mmol) was dissolved in DCM (20 mL) and water (4 mL), followed by the addition of DDQ (640.0 mg, 2.8 mmol) at room temperature. The reaction system was stirred at the room temperature of 25 °C to react for 2 hours. The reaction mixture was poured into water (30 mL), and extracted with DCM (20 mL x 3), the organic phases were combined, washed with saturated brine (30 mL), dried with anhydrous sodium sulfate, and concentrated under reduced pressure to

obtain a crude product, and the crude product was purified by chromatographic columns (PE/EA = 1/0-0/1) to obtain a yellow solid product methyl 5-amino-3-[[3-(2-cyclopropylacetyl)phenyl]methoxy]isothiazol-4-carboxylate I455 (300 mg, 866.1 μmol, yield: 61.44%).

The product was verified by LCMS and H-NMR.

$^1$H NMR (400MHz, $CDCl_3$): δ (ppm) 8.10 (d, J = 11.6 Hz, 1H), 7.90 (t, J = 9.6 Hz, 1H), 7.64 (d, J = 7.6 Hz, 1H), 7.52-7.41 (m, 1H), 6.47 (s, 1H), 5.55-5.45 (m, 2H), 3.93-3.78 (m, 3H), 2.90 (d, J = 6.8 Hz, 2H), 1.22-1.10 (m, 1H), 0.65-0.56 (m, 2H), 0.24-0.16 (m, 2H).

Step 4

Methyl 3-[[3-(2-cyclopropylacetyl) phenyl] methoxy]-5-[3-(4-methylpiperazin-1-yl) propylcarbamoylamino] isothiazole-4-carboxylate

3-(4-methylpiperazin-1-yl)propan-1-amine (158.9 mg, 1.0 mmol) was dissolved in THF (5 mL), followed by the addition of CDI (163.8 mg, 1.0 mmol) under the protection of $N_2$, and the mixture was stirred at 25 °C to react for 1 hour. DMSO(5 mL) was added, the pressure was reduced to remove THF, followed by the addition of methyl 5-amino-3-[[3-(2-cyclopropylacetyl)phenyl]methoxy]isothiazol-4-carboxylate I455 (250 mg, 721.7 μmol) and potassium carbonate (139.6 mg, 1.0 mmol), and the reaction system was stirred at 25 °C to react for 1 hour. Water (60 mL) was added to the reaction mixture, which was then subjected to liquid separation and extraction with ethyl acetate (20 mLx3). The organic phases were combined, washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a yellow solid product methyl 3-[[3-(2-cyclopropylacetyl)phenyl]methoxy]-5-[3-(4-methylpiperazin-1-yl)propylcarbamoylamino]isothiazol-4-carboxylate I456 (230 mg, 434.3 μmol, yield: 60.17%). The product was verified by LCMS and H-NMR.

$^1$H NMR (400MHz, $CDCl_3$): δ (ppm) 10.37 (s, 1H), 8.13 (s, 1H), 7.95 (s, 1H), 7.89 (d, J = 8.0 Hz, 1H), 7.63 (d, J = 8.0 Hz, 1H), 7.47 (t, J = 7.6 Hz, 1H), 5.49 (s, 2H), 4.78 (s, 3H), 3.92 (s, 3H), 3.44 (dd, J = 11.2, 5.6 Hz, 2H), 2.90 (d, J = 6.8 Hz, 2H), 2.79-2.41 (m, 9H), 2.36 (s, 3H), 1.22-1.12 (m, 1H), 0.65-0.53 (m, 2H), 0.23-0.12 (m, 2H).

Step 5

3-[[3-(2-cyclopropylacetyl)phenyl]methoxy]-5-[3-(4-methylpiperazin-1-yl) propylcarbamoylamino]isothiazole-4-carboxamide

Methyl 3-[[3-(2-cyclopropylacetyl)phenyl]methoxy]-5-[3-(4-methylpiperazin-1-yl)propylcarbamoylamino]isothiazol-4-carboxylate I456 (230 mg, 434.3 μmol) was dissolved in $NH_3$/MeOH (5 mL), and the mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a product 3-[[3-(2-cyclopropylacetyl)phenyl]methoxy]-5-[3-(4-methylpiperazin-1-yl)propylcarbamoylamino]isothiazol-4-formamide T110 (50 mg, 97.2 μmol, yield: 22.37%). The product was verified by LCMS, H-NMR and C-NMR.

$^1$H NMR (400MHz $CD_3OD$): δ (ppm) 7.91 (s, 1H), 7.77 (d, J = 7.6 Hz, 1H), 7.54 (d, J = 7.6 Hz, 1H), 7.34 (t, J = 7.6 Hz, 1H), 5.36 (s, 2H), 3.08 (t, J = 6.8 Hz, 1H), 2.75 (d, J = 6.8 Hz, 1H), 2.65-2.14 (m, 10H), 1.59-1.49 (m, 2H), 0.96-0.86 (m, 1H), 0.40-0.32 (m, 2H), 0.04-0.03 (m, 2H).

$^{13}$C NMR (101 MHz, $CD_3OD$) δ 202.21, 202.16, 170.30, 167.23, 163.23, 156.08, 138.59, 138.56, 134.05, 130.15, 129.27, 129.15, 98.82, 70.76, 56.84, 55.64, 53.66, 45.95, 44.37, 39.51, 27.67, 7.63, 4.98, 4.96.

Example 87: Synthesis of Compound T111

Synthesis Route:

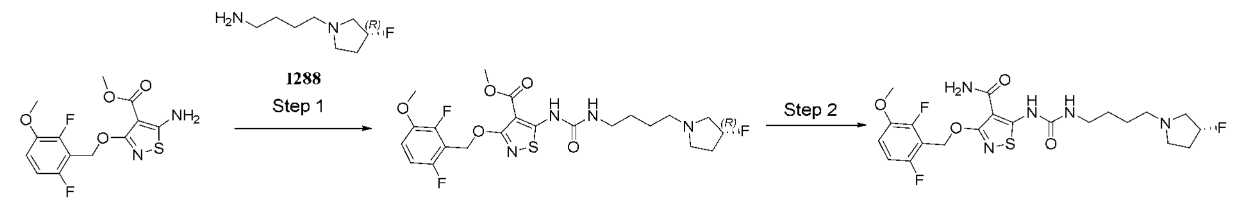


Step 1

Methyl 3-[(2,6-difluoro-3-methoxy-phenyl)methoxy]-5-[4-[(3R)-3-fluoropyrrolidin-1-yl]butylcarbamoylamino]isothiazole-4-carboxylate

4-[(3R)-3-fluoropyrrolidin-1-yl]butan-1-amine I288 (203.7 mg, 1.27 mmol) was dissolved in anhydrous THF (3 mL), followed by the addition of CDI (206.2 mg, 1.27 mmol) under the protection of $N_2$, the mixture was then stirred at 25°C to react for 1 hour, followed by the addition of DMSO(3 mL), the pressure was reduced to remove most of the THF, followed by the addition of methyl 5-amino-3-[(2,6-difluoro-3-methoxyphenyl)methoxy]isothiazol-4-carboxylate I403 (300 mg, 908.25 μmol) and potassium carbonate (376.6 mg, 2.72mmol), and the reaction system was stirred at 25 °C to react for 1 hour. Water (60 mL) was added to the reaction mixture, which was then subjected to liquid separation and extraction with ethyl acetate (20 mLx3), the organic phases were washed with saturated brine, dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3-methoxyphenyl) methoxy]-5-[4-[(3R)-3-fluoropyrrolidin-1-yl]butylcarbamylamino]isothiazol-4-carboxylate I457 (170 mg, 329.12μmol, yield: 36.24%).

The product was verified by LCMS and H-NMR.

$^1H$ NMR (400 MHz, $CDCl_3$) δ (ppm) 10.02 (s, 1H), 8.47 (s, 1H), 6.95-6.89 (m, 1H), 6.86-6.81 (m, 1H), 5.47 (s, 2H), 4.48 (s, 1H), 3.87 (s, 3H), 3.75 (s, 3H), 3.19-3.11 (m, 2H), 2.97 (d, J = 10.4 Hz, 2H), 2.62-2.54 (m, 1H), 2.50-2.46 (m, 1H), 2.35-2.32 (m, 2H), 2.18-2.11 (m, 2H), 1.92-1.87 (m, 2H), 1.77-1.64(m, 5H).

Step 2

3-[(2,6-difluoro-3-methoxy-phenyl)methoxy]-5-[4-[(3R)-3-fluoropyrrolidin-1-yl]butylc arbamoylamino]isothiazole-4-carboxamide

Methyl 3-[(2,6-difluoro-3-methoxyphenyl)methoxy]-5-[4-[(3R)-3-fluoropyrrolidin-1-yl]butylformamido]isothiazol-4-carboxylate I457 (300 mg, 580.80 μmol) was dissolved in $NH_3$/MeOH (3 mL, 10 mol/L). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3-methoxyphenyl)methoxy]-5-[4-[(3R)-3-fluoropyrrolidin-1-yl]butylcarbamylamino]isothiazol-4-formamide T111 (19 mg, 37.88 μmol, yield: 6.52%).

The product was verified by LCMS, H-NMR, C-NMR and F-NMR.

$^1H$ NMR (400 MHz, $CD_3OD$) δ (ppm) 7.18-7.12 (m, 1H), 6.99-6.94 (m, 1H), 5.55 (s, 2H), 5.25-5.09 (m, 1H), 3.87 (s, 3H), 3.25 (t, J = 6.2 Hz, 2H), 3.04-2.91 (m, 2H), 2.75-2.62 (m, 1H), 2.57-2.50 (m, 2H), 2.49-2.43 (m, 1H), 2.26-2.12 (m, 1H), 2.09-1.96 (m, 1H), 1.64-1.53 (m, 4H).

$^{13}C$ NMR (100 MHz, $CDCl_3$) δ (ppm) 169.53, 165.70, 161.42, 156.34, 156.28, 154.06, 144.44, 144.40, 144.33, 144.30, 114.22, 114.19, 114.13, 114.10, 112.95, 112.79, 112.75, 110.44, 110.40, 110.21, 110,17, 93.99,92.25, 60.39, 60.16, 58.49, 58.45 ,58.41, 56.85, 55.60, 52.16, 32.59, 32.37, 29.70, 27.75, 25.77.

$^{19}F$ NMR (100 MHz, $CD_3OD$) δ (ppm) -127.66. -136.86, -169.23

Example 88: Synthesis of Compound T112

Synthesis Route:

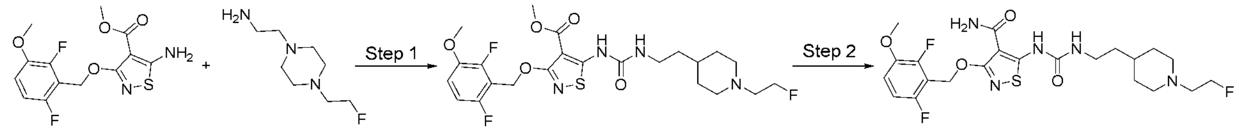

1403 1361 1458 T112

Step 1

Methyl 3-[(2,6-difluoro-3-methoxy-phenyl)methoxy]-5-[2-[1-(2-fluoroethyl)-4-piperidyl]ethylcarbamoylamino]isothiazole-4-carboxylate

2-[1-(2-fluoroethyl)-4-piperidyl]ethylamine I361 (184.7 mg, 1.1 mmol) was dissolved in anhydrous THF (5 mL), followed by the addition of CDI (152.5 mg, 1.1 mmol) under the protection of $N_2$, the mixture was then stirred at 25°C to react for 1.0 hour, followed by the addition of DMSO (5 mL), the pressure was reduced to remove THF, followed by the addition of 5-amino-3-[(2,6-difluoro-3-methoxyphenyl)methoxy]isothiazol-4-methyl formate I403 (250 mg, 756.9 μmol) and potassium carbonate (313.8 mg, 2.3 mmol), and the reaction system was stirred at 25 °C to react for 1.0 hour. Water (30 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-[(2,6-difluoro-3-methoxyphenyl) methoxy]-5-[2-[1-(2-fluoroethyl)-4-piperidyl]ethylcarbamylamino]isothiazol-4-carboxylate I458 (170 mg, 320.4 μmol, yield: 42.3%). The product was verified by LCMS.

Step 2

3-[(2,6-difluoro-3-methoxy-phenyl)methoxy]-5-[2-[1-(2-fluoroethyl)-4-piperidyl]ethylcarbamoylamino]isothiazole-4-carboxamide

Methyl 3-[(2,6-difluoro-3-methoxyphenyl)methoxy]-5-[2-[1-(2-fluoroethyl)-4-piperidyl]ethylcarbamylamino]isothiazol-4-carboxylate I458 (170 mg, 320.4 μmol) was dissolved in $NH_3$/MeOH (10 mL, 10 mol/L), followed by the dropwise addition of 5 drops of DMSO. The mixture was stirred at 40 °C to react for 16 hours. The reaction mixture was concentrated under reduced pressure to obtain a crude product, and the crude product was purified by the column chromatography (DCM/MeOH = 10/1) to obtain a white solid product 3-[(2,6-difluoro-3-methoxyphenyl)methoxy]-5-[2-[1-(2-fluoroethyl)-4-piperidyl]ethylcarbamylamino]isothiazol-4-formamide T112 (30 mg, 58.2 μmol, yield: 18.16%).

The product was verified by LCMS, H-NMR, C-NMR and F-NMR.

$^1$H NMR (400 MHz, $CDCl_3$) δ (ppm) 10.98 (s, 1H), 7.11 (s, 1H), 6.99-6.93 (m, 1H), 6.90-6.85 (m, 1H), 5.86 (t, J = 5.2 Hz, 1H), 5.58 (s, 2H), 5.53 (s, 1H), 4.62 (t, J = 4.8 Hz, 1H), 4.50 (t, J = 4.8 Hz, 1H), 3.88 (s, 3H), 3.33 (dd, J = 13.6, 6.8 Hz, 2H), 2.96 (d, J = 10.8 Hz, 2H), 2.72 (t, J = 5.2 Hz, 1H), 2.66-2.63 (m, 1H), 2.12 (s, 2H), 2.03 (t, J = 10.8 Hz, 2H), 1.69 (d, J = 9.2 Hz, 2H), 1.33 (s, 3H).

$^{13}$C NMR (100 MHz, $CDCl_3$) δ (ppm) 169.49, 165.83, 161.39, 153.93, 144.31, 114.22, 114.15, 112.71, 112.52, 110.42, 110.23, 82.61, 80.95, 58.55, 58.36, 56.83, 54.06, 40.90, 38.21, 36.28, 32.95, 31.83, 29.70.

$^{19}$F NMR (400 MHz, $CDCl_3$) δ (ppm) δ -124.85, -134.48.

Example 89: Synthesis of Compound T113

Synthesis Route:

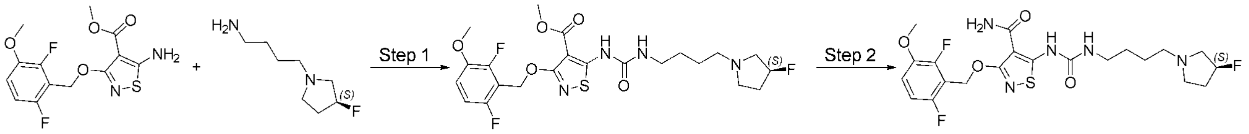

1403 1278 1459 T113

Step 1

Methyl 3-[(2,6-difluoro-3-methoxy-phenyl)methoxy]-5-[4-[(3S)-3-fluoropyrrolidin-1-yl]butylcarbamoylamino]isothiazole-4-carboxylate

4-[(3S)-3-fluoropyrrolidin-1-yl]butan-1-amine I278 (169.8 mg, 1.1 mmol) was dissolved in anhydrous THF (5 mL), followed by the addition of CDI (171.8 mg, 1.1 mmol) under the protection of $N_2$, the mixture was then stirred at 25°C to react for 1.0 hour, followed by the addition of DMSO (5 mL), the pressure was reduced to remove THF, followed by the addition of 5-amino-3-[(2,6-difluoro-3-methoxyphenyl)methoxy]isothiazol-4-methyl formate I403 (250 mg, 756.9 μmol) and potassium carbonate (313.8 mg, 2.3 mmol), and the reaction system was stirred at 25 °C to react for 1.0 hour. Water (30 mL) was poured into the reaction mixture, which was then subjected to liquid separation and extraction with ethyl acetate (10 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3-methoxyphenyl)methoxy]-5-[4-[(3S)-3-fluoropyrrolidin-1-yl]butylcarbamylamino]isothiazol-4-carboxylate I459 (220 mg, 425.92 μmol, yield: 56.27%).

The product was verified by LCMS and H-NMR.

$^1$H NMR (400MHz, $CDCl_3$): δ (ppm) 10.17 (s, 1H), 6.97-6.89 (m, 1H), 6.88 - 6.80 (m, 1H), 5.49 (s, 2H), 5.34 - 5.14 (m, 1H), 3.88 (s, 3H), 3.77(s, 3H), 3.41 - 3.24 (m, 2H), 3.01 - 2.85 (m, 2H), 2.78 - 2.65 (m, 1H), 2.61 - 2.42 (m, 3H), 2.26 - 2.10 (m, 2H), 1.73 - 1.60 (m, 6H).

Step 2

3-[(2,6-difluoro-3-methoxy-phenyl)methoxy]-5-[4-[(3S)-3-fluoropyrrolidin-1-yl]butylcarbamoylamino]isothiazole-4-carboxamide

Methyl 3-[(2,6-difluoro-3-methoxyphenyl)methoxy]-5-[4-[(3S)-3-fluoropyrrolidin-1-yl]butylcarbamylamino]isothiazol-4-carboxylate I459 (220 mg, 425.9 μmol) was dissolved in $NH_3$/MeOH (10 mL, 10 mol/L), followed by the dropwise addition of 5 drops of DMSO. The mixture was stirred at 40 °C to react for 16 hours. The reaction mixture was concentrated under reduced pressure to obtain a crude product, and the crude product was purified by the column chromatography (DCM/MeOH = 10/1) to obtain a white solid product 3-[(2,6-difluoro-3-methoxyphenyl)methoxy]-5-[4-[(3S)-3-fluoropyrrolidin-1-yl]butylcarbamylamino]isothiazol-4-formamide T113 (70 mg, 139.6 μmol, yield: 32.8%).

The product was verified by LCMS, H-NMR, C-NMR and F-NMR.

$^1$H NMR (400MHz, $CD_3OD$): δ (ppm) 7.19-7.11 (m, 1H), 7.01 - 6.91 (m, 1H), 5.55 (s, 2H), 5.26 - 5.08 (m, 1H), 3.87 (s, 3H), 3.25 (t, J = 6.4 Hz, 2H), 3.04 - 2.89 (m, 2H), 2.74 - 2.62 (m, 1H), 2.53 (d, J = 7.2 Hz, 2H), 2.49 - 2.41 (m, 1H), 2.27 - 2.13 (m, 1H), 2.07 - 1.94 (m, 1H), 1.67 - 1.50 (m, 4H).

$^{13}$C NMR (100MHz, $CD_3OD$): δ (ppm) 170.35, 167.12, 162.90, 157.50, 156.14, 155.15, 153.62, 151.13, 145.99, 145.88, 115.81, 115.74, 114.07, 113.91, 113.71, 111.54, 111.50, 111.31, 111.27, 98.64, 95.03, 93.29, 61.68, 61.45, 59.48, 57.28, 57.01, 53.41, 40.85, 33.42, 33.19, 28.69, 26.43. $^{19}$F NMR (400MHz, $CD_3OD$): δ (ppm) -127.67, -136.88, -169.17.

Example 90: Synthesis of Compound T114

Synthesis Route:

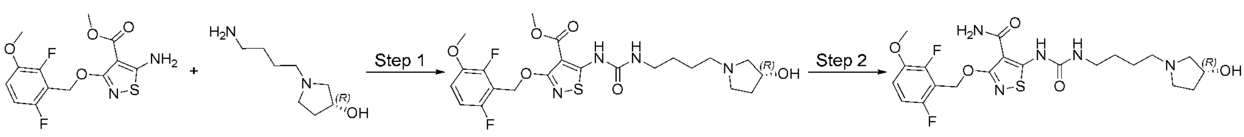


1403 1392 1460 T114

Step 1

Methyl 3-[(2,6-difluoro-3-methoxy-phenyl)methoxy]-5-[4-[(3R)-3-hydroxypyrrolidin-1-yl]butylcarbamoylamino]isothiazole-4-carboxylate

(3R)-1-(4-aminobutyl)pyrrolidin-3-ol I392 (201.2 mg, 1.27 mmol) was dissolved in anhydrous THF (3 mL), followed by the addition of CDI (206.2 mg, 1.27 mmol) under the protection of $N_2$, the mixture was then stirred at 25°C to

react for 1.0 hour, followed by the addition of DMSO (3 mL), the pressure was reduced to remove THF, followed by the addition of 5-amino-3-[(2,6-difluoro-3-methoxyphenyl)methoxy]isothiazol-4-methyl formate I403 (300 mg, 908.25μmol) and potassium carbonate (376.6 mg, 2.72 mmol), and the reaction system was stirred at 25 °C to react for 1.0 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3-methoxyphenyl)methoxy]-5-[4-[(3R)-hydroxylpyrrolidin-1-yl]butylcarbamylamino]isothiazol-4-carboxylate I460 (170 mg, 330.39μmol, yield: 36.38%). The product was verified by LCMS and H-NMR.

$^1$H NMR (400 MHz, $CDCl_3$) δ (ppm) 10.02 (s, 1H), 8.47 (s, 1H), 6.95-6.89 (m, 1H), 6.86- 6.81 (m, 1H), 5.47 (s, 2H), 4.48 (s, 1H), 3.87 (s, 3H), 3.75 (s, 3H), 3.19-3.11 (m, 2H), 2.97 (d, J = 10.4 Hz, 2H), 2.62-2.54 (m, 1H), 2.50-2.46 (m, 1H), 2.35-2.32 (m, 2H), 2.18-2.11 (m, 2H), 1.92-1.87 (m, 2H), 1.77-1.64(m, 5H).

Step 2

3-[(2,6-difluoro-3-methoxy-phenyl)methoxy]-5-[4-[(3R)-3-hydroxypyrrolidin-1-yl]butylcarbamoylamino]isothiazole-4-carboxamide

Methyl 3-[(2,6-difluoro-3-methoxyphenyl)methoxy]-5-[4-[(3R)-hydroxylpyrrolidin-1-yl]butylcarbamylamino]isothiazol-4-carboxylate I460 (200 mg, 388.70 μmol) was dissolved in $NH_3$/MeOH (3 mL, 10 mol/L). The mixture was stirred at 40 °C to react for 16 hours. The reaction mixture was concentrated under reduced pressure to obtain a crude product, and the crude product was purified by the column chromatography (DCM/MeOH = 10/1) to obtain a white solid product 3-[(2,6-difluoro-3-methoxyphenyl)methoxy]-5-[4-[(3R)-hydroxylpyrrolidin-1-yl]butylcarbamylamino]isothiazol-4-formamide T114 (47 mg, 94.09μmol, yield: 24.21%).

The product was verified by LCMS, H-NMR, C-NMR and F-NMR.

$^1$H NMR (400 MHz, $CD_3OD$) δ (ppm) 7.17-7.11 (m, 1H), 6.98-6.93 (m, 1H), 5.55 (s, 1H), 4.36-4.23 (m, 1H), 3.87 (s, 3H), 3.23 (t, *J* = 8.0 Hz, 2H), 2.84-2.80 (m, 1H), 2.72-2.68 (m, 1H), 2.59-2.43 (m, 4H), 2.16-2.07 (m, 1H), 1.74-1.67 (m, 1H), 1.61-1.51 (m, 4H).

$^{13}$C NMR (100 MHz, $CD_3OD$) δ(ppm) 170.37, 167.12, 162.89, 157.57, 157.51, 156.13, 155.15, 155.10, 153.64, 153.56, 151.14, 151.06, 145.99, 145.96, 145.89, 145.85 ,115.86, 115.83, 115.76, 115.73, 114.08, 113.88, 113.72, 111.54, 111.49, 111.31, 111.26, 98.61, 71.44, 63.52, 59.51, 59.46, 59.42, 57.33, 57.28, 53.75, 40.95, 35.10, 28.81, 26.71

$^{19}$F NMR (100 MHz, $CD_3OD$) δ(ppm) -127.62, -136.82

Example 91: Synthesis of Compound T115

Synthesis Route:

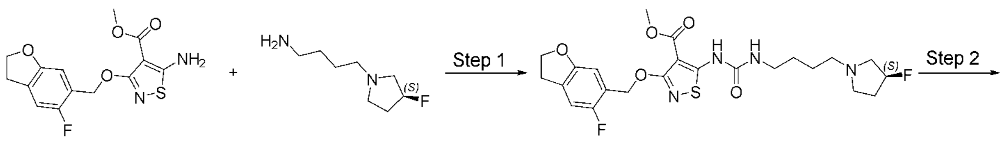


I311 I288 I461

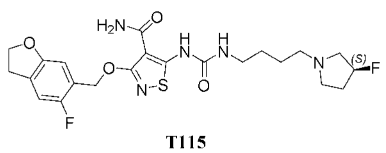

T115

Step 1

Methyl 3-[(5-fluoro-2,3-dihydrobenzofuran-6-yl)methoxy]-5-[4-[(3S)-3-fluoropyrrolidin-1-yl]butylcarbamoylamino]isothiazole-4-carboxylate

4-[(3S)-3-fluoropyrrolidin-1-yl]butan-1-amine I288 (242.1 mg, 1.51 mmol) was dissolved in anhydrous THF (6

mL), followed by the addition of CDI (244.9 mg, 1.51 mmol) under the protection of $N_2$, the mixture was then stirred at 25°C to react for 1.0 hour, followed by the addition of DMSO(6 mL), the pressure was reduced to remove THF, followed by the addition of methyl 5-amino-3-[(5-fluoro-2,3-dihydrobenzofuran-6-yl)methoxy]isothiazol-4-carboxylate I311 (350 mg, 1.08 mmol) and potassium carbonate (447.4 mg, 3.24 mmol), and the reaction system was stirred at 25 °C to react for 1.0 hour. Water (60 mL) was added to the reaction mixture, which was then subjected to liquid separation and extraction with ethyl acetate (20 mLx3), the organic phases were washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(5-fluoro-2,3-dihydrobenzofuran-6-yl)methoxy]-5-[4-[(3S)-3-fluoropyrrolidin-1-yl]butylcarbamylamino]isothiazol-4-carboxylate I461 (350 mg, 685.53 μmol, yield: 63.52%). The product was verified by LCMS and H-NMR.

$^1$H NMR (400MHz, $CDCl_3$): δ (ppm) 10.57(s, 1H), 7.52(s, 2H), 6.93 (d, J = 5.8 Hz, 1H), 6.88 (d, J = 9.0 Hz, 1H), 5.41 (s, 2H), 4.55 (t, J = 8.8 Hz, 2H), 3.85 (s, 3H), 3.34-3.24 (m, 2H), 3.18 (t, J = 8.8 Hz, 2H), 2.98-2.86 (m, 2H), 2.52 (d, J = 4.6 Hz, 2H), 2.54-2.40 (m, 2H), 2.25-2.10 (m, 3H), 1.61 (s, 4H).

Step 2

3-[(5-fluoro-2,3-dihydrobenzofuran-6-yl)methoxy]-5-[4-[(3S)-3-fluoropyrrolidin-1-yl]butylcarbamoylamino]isothiazole-4-carboxamide

Methyl 3-[(5-fluoro-2,3-dihydrobenzofuran-6-yl)methoxy]-5-[4-[(3S)-3-fluoropyrrolidin-1-yl]butylcarbamylamino]isothiazol-4-carboxylate I461 (300 mg, 587.6 μmol) was dissolved in $NH_3$/MeOH (6 mL, 10 mol/L). The mixture was stirred at 40 °C to react for 16 hours. The reaction mixture was concentrated under reduced pressure to obtain a crude product, and the crude product was purified by the column chromatography (DCM/MeOH = 10/1) to obtain a white solid product 3-[(5-fluoro-2,3-dihydrobenzofuran-6-yl)methoxy]-5-[4-[(3S)-3-fluoropyrrolidin-1-yl]butylcarbamylamino]isothiazol-4-formamide T115 (47 mg, 94.85 μmol, yield: 16.14%).

The product was verified by LCMS, H-NMR, C-NMR and F-NMR.

$^1$H NMR (400MHz, $CDCl_3$&$CD_3OD$): δ (ppm) 6.96 (d, J = 9.2 Hz, 1H), 6.79 (d, J = 5.6 Hz, 1H), 5.40 (s, 2H), 5.24-5.06 (m, 1H), 4.55 (t, J = 8.8 Hz, 1H), 3.26-3.16 (m, 4H), 3.04-2.90 (m, 2H), 2.72-2.60 (m, 1H), 2.59-2.49 (m, 2H), 2.48-2.42 (m, 1H), 2.26-1.95 (m, 2H), 1.66-1.49 (m, 4H).

$^{13}$C NMR (400MHz, $CDCl_3$& $CD_3OD$): δ (ppm) 169.52, 165.79, 161.67, 156.87, 156.06, 154.48, 154.01, 129-24, 129.15, 121.95, 121.78, 112.45, 112.20, 110.27, 110.23, 97.36, 94.25, 92.51, 71.69, 64.60, 64.56, 60.53, 60.31, 55.51, 52.13, 40.31, 32.63, 62.41, 29.99, 29.70, 27.86, 16.14

$^{19}$F NMR (400MHz, $CDCl_3$& $CD_3OD$): δ (ppm) -130.14, -168.77

Example 92: Synthesis of Compound T116

Synthesis Route:

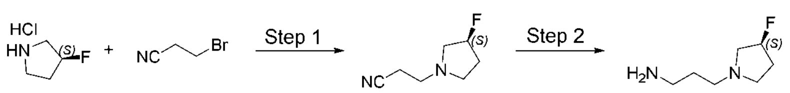


I462 I463

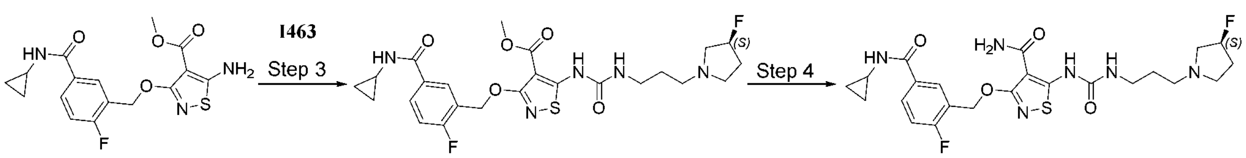


I376 T161 T116

Step 1

3-[(3S)-3-fluoropyrrolidin-1-yl] propanenitrile

(3S)-3-fluoropyrrolidine hydrochloride (5 g, 39.8 mmol) was dissolved in acetonitrile (48.7 mL), followed by the

addition of 3-bromopropionitrile (5.3 g, 39.82 mmol) and potassium carbonate (16.5 g, 119.5 mmol), and the reaction system was stirred at 60 °C to react for 16 hours. The reaction mixture was poured into water (50 mL) and extracted with ethyl acetate (50 mL x 3), the organic phases were combined, washed with saturated brine (200 mL), dried with anhydrous sodium sulfate, and concentrated under reduced pressure to obtain a crude product, and the crude product was purified by the neutral alumina column chromatography (DCM) to obtain a colorless oily product 3-[(3S)-3-fluoropyrrolidin-1-yl] propionitrile I462 (3.4 g, 23.9 mmol, yield: 60.06%). The product was verified by LCMS and H-NMR.

$^1$H NMR (400MHz, $CDCl_3$): δ (ppm) 5.28 - 5.06 (m, 1H), 3.00 - 2.73 (m, 5H), 2.64 - 2.44 (m, 3H), 2.26 - 1.97 (m, 2H).

Step 2

3-[(3S)-3-fluoropyrrolidin-1-yl]propan-1-amine

3-[(3S)-3-fluoropyrrolidin-1-yl]propionitrile I462 (3 g, 21.1 mmol) was dissolved in methanol (15 mL), followed by the addition of raney nickel (1.4 g, 23.2 mmol), in the atmosphere of hydrogen, and the reaction system was stirred at 25 °C to react for 3 hours. The reaction mixture was filtered, and the filtrate was concentrated under reduced pressure to obtain a colorless oily product 3-[(3S)-3-fluoropyrrolidin-1-yl]propylamine I463 (3 g, 20.52 mmol, yield: 97.24%).

The product was verified by LCMS and H-NMR.

$^1$H NMR (400MHz, $CDCl_3$): δ (ppm) 5.83 - 4.93 (m, 1H), 2.93 - 2.34 (m, 7H), 2.17 - 1.48 (m, 6H).

Step 3

Methyl 3-[[5-(cyclopropylcarbamoyl)-2-fluoro-phenyl]methoxy]-5-[3-[(3S)-3-fluoropyrrolidin-1-yl]propylcarbamoylamino]isothiazole-4-carboxylate

3-[(3S)-3-fluoropyrrolidin-1-yl]propylamine I463 (196.1 mg, 1.3 mmol) was dissolved in anhydrous THF (5 mL), followed by the addition of CDI (217.5 mg, 1.3 mmol) under the protection of $N_2$, the mixture was then stirred at 25°C to react for 1.0 hour, followed by the addition of DMSO (5 mL), the pressure was reduced to remove THF, followed by the addition of methyl 5-amino-3-[[5-(cyclopropylcarbamoyl)-2-fluoro-phenyl]methoxy]isothiazol-4-carboxylate I376 (350 mg, 957.9 μmol) and potassium carbonate (397.2 mg, 2.9 mmol), and the reaction system was stirred at 25 °C to react for 1.0 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-[[5-(cyclopropylcarbamoyl)-2-fluorophenyl]methoxy]-5-[3-[(3S)-3-fluoropyrrolidin-1-yl]propylcarbamoylamino]isothiazol-4-carboxylate T161 (175 mg, 325.5 μmol, yield: 33.98%).

The product was verified by LCMS.

Step 4

3-[[5-(cyclopropylcarbamoyl)-2-fluoro-phenylmethoxy]-5-[3-[(3S)-3-fluoropyrrolidin-1-yl]propylcarbamoylamino]isothiazole-4-carboxamide

Methyl 3-[[5-(cyclopropylcarbamoyl)-2-fluorophenyl]methoxy]-5-[3-[(3S)-3-fluoropyrrolidin-1-yl]propylcarbamoylamino]isothiazol-4-carboxylate I464 (175 mg, 325.5 μmol) was dissolved in $NH_3$/MeOH (10 mL, 10 mol/L) and DMSO (0.3 mL). The mixture was stirred at 40 °C to react for 16 hours. The reaction mixture was concentrated under reduced pressure to obtain a crude product, and the crude product was purified by the preparative thin-layer chromatography (DCM/MeOH = 10/1) to obtain a white solid product 3-[[5-(cyclopropylcarbamoyl)-2-fluorophenyl]methoxy]-5-[3-[(3S)-3-fluoropyrrolidin-1-yl]propylcarbamoylamino]isothiazol-4-formamide T116 (40 mg, 76.55 μmol, yield: 23.51%).

The product was verified by LCMS, H-NMR, C-NMR and F-NMR.

$^1$H NMR (400MHz $CD_3OD$): δ (ppm) 8.00 (dd, J = 7.2, 2.4 Hz, 1H), 7.88 - 7.82 (m, 1H), 7.24 (t, J = 7.2 Hz, 1H), 5.56 (s, 2H), 5.26-5.06 (m, 1H), 3.27 (t, J = 6.8 Hz, 2H), 3.02 - 2.88 (m, 2H), 2.86 - 2.79 (m, 1H), 2.72 - 2.64 (m, 2H), 2.64 - 2.53 (m, 2H), 2.46 - 2.36 (m, 1H), 2.27 - 2.11 (m, 1H), 2.09 - 1.91 (mf, 1H), 1.83 - 1.70 (m, 1H), 0.84 - 0.75 (m, 2H), 0.67 - 0.57 (m, 2H).

$^{13}$C NMR (101 MHz, $CD_3OD$) δ 170.35, 167.16, 165.71, 163.19, 162.97, 156.10, 132.09, 131.57, 131.52, 131.18, 131.08, 125.08, 124.93, 116.86, 116.63, 98.73, 95.18, 93.44, 65.16, 61.76, 61.53, 54.71, 53.40, 40.43, 39.41, 33.57, 33.34, 29.60, 24.07, 6.55.

$^{19}$F NMR (400 MHz, $CD_3OD$) δ (ppm) -115.43, -168.98.

Example 93: Synthesis of Compound T117

Synthesis Route:

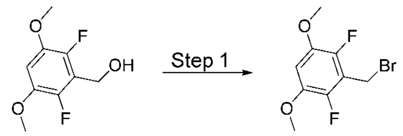


I465

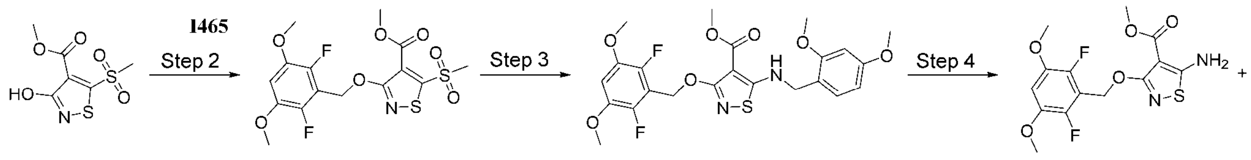


I009 I466 I467 I468

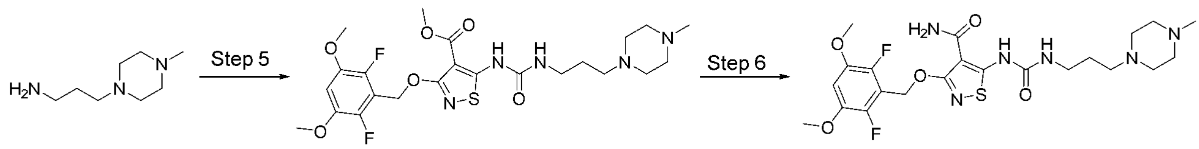


I469 T117

Step 1

3-(bromomethyl)-2,4-difluoro-1,5-dimethoxy-benzene

(2,6-difluoro-3.5-dimethoxyphenyl)methanol (1 g, 4.90 mmol) was dissolved in DCM (10 mL), followed by the addition of $PBr_3$ (2.6 g, 9.80 mmol) at 0°C, and the reaction system was stirred at room temperature to react for about 2 hours. The reaction mixture was poured into water (50 mL) and extracted with ethyl acetate (30 mL x 3), and the organic phases were combined, washed with saturated brine (40 mL), dried with anhydrous sodium sulfate, and concentrated under reduced pressure to obtain a yellow oily crude product 3-(bromomethyl)-2,4-difluoro-1,5-dimetoxybenzene I465 (1 g, 3.74 mmol, yield: 76.45%).

The product was verified by H-NMR

$^1H$ NMR (400MHz, $CDCl_3$): δ (ppm) 6.62 (t, J = 8.0 Hz, 1H), 4.53 (t, J = 1.4 Hz, 2H), 3.88 (s, 6H).

Step 2

Methyl 3-[(2,6-difluoro-3,5-dimethoxy-phenyl)methoxy]-5-methylsulfonyl-isothiazole-4-carboxylate

Methyl 3-hydroxyl-5-(methylsulfonyl)isothiazol-4-carboxylate I009 (740 mg, 3.12 mmol) was dissolved in DMF(10 mL), followed by the addition of 3-(bromomethyl)-2,4-difluoro-1,5-dimetoxybenzene I465 (1 g, 3.74 mmol) and potassium carbonate (1.3 g, 9.36 mmol), and the reaction system was stirred at 25 °C to react for 1 hour. The reaction mixture was poured into water (60 mL) and filtered, and the filter cake was dried to obtain a white solid product 3-[(2,6-difluoro-3,5-dimethoxyphenyl)methoxy]-5-methylsulfonylisothiazol-4-methyl formate I466 (1 g, 2.36 mmol, yield: 75.72%).

The product was verified by LCMS and H-NMR.

$^1H$ NMR (400MHz, $CDCl_3$): δ (ppm) 6.69 (t, J = 8.0 Hz, 1H), 5.56 (s, 2H), 3.89 (d, J = 3.0 Hz, 9H), 3.47 (s, 3H).

Step 3

Methyl 3-[(2,6-difluoro-3,5-dimethoxy-phenyl)methoxy]-5-[(2,4-dimethoxyphenyl)methylamino]isothiazole-4-carboxylate

3-[(2,6-difluoro-3,5-dimethoxyphenyl)methoxy]-5-methylsulfonylisothiazol-4-methyl formate I466 (1 g, 2.36 mmol) was dissolved in THF(4 mL), followed by the addition of 2,4-dimethoxybenzylamine (3.9 g, 23.62 mmol), and the reaction system was stirred at 65 °C to react for 1 hour. The mixture was poured into water (60 mL) and filtered, and the filter cake was dried to obtain a yellow solid product methyl 3-[(2,6-difluoro-3,5-dimethoxyphenyl)methoxy]-5-[(2,4-dimethoxyphenyl)methylamino]isothiazol-4-carboxylate I467 (700 mg, 1.37 mmol, yield: 58.06%).

The product was verified by LCMS and H-NMR.

$^1$H NMR (400MHz, $CDCl_3$): δ (ppm) 7.15 (d, J = 8.2 Hz, 1H), 6.65 (t, J = 8.0 Hz, 1H), 6.47 - 6.42 (m, 2H), 5.46 (s, 2H), 4.26 (d, J = 6.0 Hz, 2H), 3.88 (s, 6H), 3.83 (s, 3H), 3.80 (s, 3H), 3.71 (s, 3H).

Step 4

Methyl 5-amino-3-[(2,6-difluoro-3,5-dimethoxy-phenyl)methoxy]isothiazole-4-carboxylate

Methyl 3-[(2,6-difluoro-3,5-dimethoxyphenyl)methoxy]-5-[(2,4-dimethoxyphenyl)methylamino]isothiazol-4-carboxylate I467 (700 mg, 1.37 mmol) was dissolved in DCM/$H_2O$ (10/2 mL), followed by the addition of DDQ (622.5 mg, 2.74 mmol), and the reaction system was stirred at 25 °C to react for 2.0 hours. The reaction mixture was poured into water (50 mL), and extracted with DCM (30 mL x 3), the organic phases were combined, washed with saturated brine (40 mL), dried with anhydrous sodium sulfate, and concentrated under reduced pressure to obtain a crude product, and the crude product was purified by neutral alumina chromatographic columns (PE/EA = 10/1-0/1) to obtain a white solid product methyl 5-amino-3-[(2,6-difluoro-3,5-dimethoxyphenyl)methoxy]isothiazol-4-carboxylate I468 (200 mg, 555.04 μmol, yield: 40.48%).

The product was verified by LCMS.

Step 5

Methyl 3-[(2,6-difluoro-3,5-dimethoxy-phenyl)methoxy]-5-[3-(4-methylpiperazin-1-yl)propylcarbamoylamino]isothiazole-4-carboxylate

3-(4-methylpiperazin-1-yl)propylamine(122.2 mg, 777.06 μmol) was dissolved in anhydrous THF (8 mL), followed by the addition of CDI (126.0 mg, 777.06 μmol) under the protection of $N_2$, the mixture was then stirred at 25°C to react for 1.0 hour, followed by the addition of DMSO(8 mL), the pressure was reduced to remove THF, followed by the addition of methyl 5-amino-3-[(2,6-difluoro-3,5-dimethoxyphenyl)methoxy]isothiazol-4-carboxylate I468 (200 mg, 555.04 μmol) and potassium carbonate (230.1 mg, 1.67 mmol), and the reaction system was stirred at 25 °C to react for 1.0 hour. Water (60 mL) was poured into the reaction mixture, which was then subjected to liquid separation and extraction with ethyl acetate (30 mL x 3), the organic phases were washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3,5-dimethoxyphenyl)methoxy]-5-[3-(4-methylpiperazin-1-yl)propylcarbamylamino]isothiazol-4-carboxylate I469 (130 mg, 239.15 μmol, yield: 43.09%). The product was verified by LCMS and H-NMR.

$^1$H NMR (400MHz, $CDCl_3$): δ (ppm) 10.36 (s, 1H), 7.90 (s, 1H), 6.67 (t, J = 8.0 Hz, 1H), 5.49 (s, 2H), 3.89 (s, 6H), 3.76 (s, 3H), 3.45-3.41 (m, 2H), 2.83-2.37 (m, 10H), 2.34 (s, 3H).

Step 6

3-[(2,6-difluoro-3,5-dimethoxy-phenyl)methoxy]-5-[3-(4-methylpiperazin-1-yl)propylcarbamoylamino]isothiazole-4-carboxamide

Methyl 3-[(2,6-difluoro-3,5-dimethoxyphenyl)methoxy]-5-[3-(4-methylpiperazin-1-yl)propylcarbamylamino]isothiazol-4-carboxylate I469 (150 mg, 275.95 μmol) was dissolved in $NH_3$/MeOH (5 mL, 10 mol/L). The mixture was stirred at 40 °C to react for 16 hours. The reaction mixture was concentrated under reduced pressure to obtain a crude product, and the crude product was purified by the preparative thin-layer chromatography (DCM/MeOH = 10/1) to obtain a product 3-[(2,6-difluoro-3,5-dimethoxyphenyl)methoxy]-5-[3-(4-methylpiperazin-1-yl)propylcarbamylamino]isothiazol-4-formamide T117 (13 mg, 24.59 μmol, yield: 8.91%).

The product was verified by LCMS, H-NMR, C-NMR and F-NMR.

$^1$H NMR (400MHz, $CD_3OD$): δ (ppm) 6.92 (t, J = 8.4 Hz, 1H), 5.53 (s, 2H), 3.87 (s, 6H), 3.25 (t, J = 6.8 Hz, 2H), 2.85

- 2.28 (m, 10H), 2.27 (s, 3H), 1.72 (dd, J = 14.4, 7.1 Hz, 2H).

$^{13}C$ NMR (101 MHz, $CD_3OD$): δ (ppm) 170.32, 167.11, 162.89, 156.09, 147.03, 146.97, 145.26, 145.22, 145.15, 145.11, 144.61, 144.56, 114.46, 114.29, 114.12, 102.80, 98.62, 59.66, 59.62, 59.58, 57.61, 56.84, 55.62, 53.66, 45.95, 39.50, 27.65

$^{19}F$ NMR (101 MHz, $CD_3OD$): δ(ppm) -148.69

Example 94: Synthesis of Compound T118

Synthesis Route:

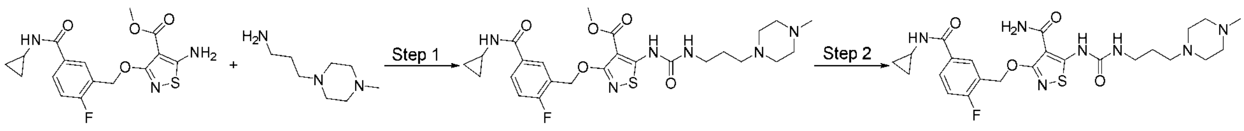


1376 1470 T118

Step 1

Methyl 3-[[5-(cyclopropylcarbamoyl)-2-fluoro-phenyl]methoxy]-5-[3-(4-methylpiperazin-1-yl)propylcarbamoylamino] isothiazole-4-carboxylate

3-(4-methylpiperazin-1-yl)propan-1-amine (180.8 mg, 1.2 mmol) was dissolved in anhydrous THF (10 mL), followed by the addition of CDI (186.4 mg, 1.2 mmol) under the protection of $N_2$, the mixture was then stirred at 25°C to react for 1.0 hour, followed by the addition of DMSO (10 mL), the pressure was reduced to remove THF, followed by the addition of methyl 5-amino-3-[[5-(cyclopropylcarbamoyl)-2-fluoro-phenyl]methoxy]isothiazol-4-carboxylate I376 (300 mg, 821.1 μmol) and potassium carbonate (340.4 mg, 2.5 mmol), and the reaction system was stirred at 25 °C to react for 1.0 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[[5-(cyclopropylcarbamoyl)-2-fluorophenyl]methoxy]-5-[3-(4-methylpiperazin-1-yl)propylcarbamylamino]isothiazol-4-carboxylate I470 (180 mg, 328.1 μmol, yield: 39.96%).

The product was verified by LCMS and HNMR.

$^1H$ NMR (400 MHz, $CDCl_3$) δ (ppm) 10.34 (s, 1H), 8.08 (d, J = 4.6 Hz, 2H), 7.68 (dd, J = 5.6, 3.0 Hz, 1H), 7.11 (t, J = 9.0 Hz, 1H), 6.25 (s, 1H), 5.51 (s, 2H), 3.93 (s, 3H), 3.44 (d, J = 5.2 Hz, 2H), 2.90 (dd, J = 6.8, 3.6 Hz, 1H), 2.68 - 2.45 (m, 8H), 2.37 (s, 3H), 1.73 (d, J = 5.8 Hz, 2H), 1.25 (s, 2H), 1.05 (s, 2H), 0.91 - 0.86 (m, 2H), 0.62 (t, J = 8.1 Hz, 2H).

Step 2

3-[[5-(cyclopropylcarbamoyl)-2-fluoro-phenyl]methoxy]-5-[3-(4-methylpiperazin-1-yl)propylcarbamoylamino]isothiazole-4-carboxamide

Methyl 3-[[5-(cyclopropylcarbamoyl)-2-fluorophenyl]methoxy]-5-[3-(4-methylpiperazin-1-yl)propylcarbamylamino]isothiazol-4-carboxylate I470 (180 mg, 328.1 μmol) was dissolved in $NH_3$/MeOH (10 mL, 10 mol/L). The mixture was stirred at 40 °C to react for 16 hours. The reaction mixture was concentrated under reduced pressure to obtain a crude product, and the crude product was purified by the preparative thin-layer chromatography (DCM/MeOH = 10/1) to obtain a white solid product 3-[[5-(cyclopropylcarbamoyl)-2-fluorophenyl]methoxy]-5-[3-(4-methylpiperazin-1-yl)propylcarbamylamino]isothiazol-4-formamide T118 (20 mg, 37.5 μmol, yield: 11.42%).

The product was verified by LCMS, H-NMR, C-NMR and F-NMR.

$^1H$ NMR (400 MHz, $CD_3OD$) δ (ppm) 8.00 (d, J = 4.8 Hz, 1H), 7.90 - 7.78 (m, 1H), 7.24 (t, J = 9.2 Hz, 1H), 5.56 (s, 2H), 3.26 (t, J = 6.8 Hz, 2H), 2.86 - 2.80 (m, 1H), 2.77 - 2.31 (m, 9H), 2.28 (s, 3H), 1.80 - 1.69 (m, 2H), 1.28 (s, 1H), 0.83 - 0.74 (m, 2H), 0.63 (s, 2H).

$^{13}C$ NMR (100 MHz, $CD_3OD$) δ (ppm) 170.36, 167.16, 165.71, 163.20, 162.98, 156.10, 132.08, 131.57, 131.52, 131.18, 131.08, 125.09, 124.94, 116.85, 116.63, 98.72, 65.16, 56.83, 55.63, 53.66, 45.95, 39.49, 27.66, 24.07, 6.54.

$^{19}F$ NMR (400 MHz, $CD_3OD$) δ (ppm) -115.45

Example 95: Synthesis of Compound T119

Synthesis Route:

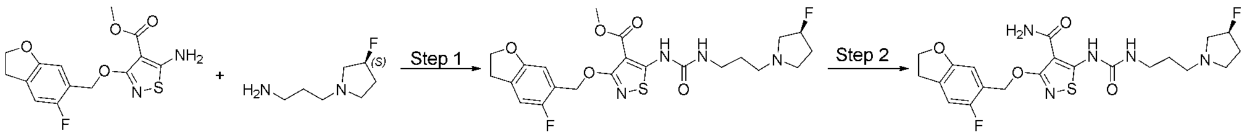


I311 I463 I471 T119

Step 1

Methyl 3-[(5-fluoro-2,3-dihydrobenzofuran-6-yl) methoxy]-5-[3-[(3S)-3-fluoropyrrolidin-1-yl] propylcarbamoylamino] isothiazole-4-carboxylate

3-[(3S)-3-fluoropyrrolidin-1-yl]propan-1-amine I463 (189.3 mg, 1.3 mmol) was dissolved in anhydrous THF (5 mL), followed by the addition of CDI (210 mg, 1.3 mmol) under the protection of $N_2$, the mixture was then stirred at 25°C to react for 1.0 hour, followed by the addition of DMSO(5 mL), the pressure was reduced to remove THF, followed by the addition of methyl 5-amino-3-[(5-fluoro-2,3-dihydrobenzofuran-6-yl)methoxy]isothiazol-4-carboxylate I311 (300 mg, 925 μmol) and potassium carbonate (383.5 mg, 2.77 mmol), and the reaction system was stirred at 25 °C to react for 1.0 hour. Water (60 mL) was added to the reaction mixture, which was then subjected to liquid separation and extraction with ethyl acetate (20 mLx3), the organic phases were washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(5-fluoro-2,3-dihydrobenzofuran-6-yl)methoxy]-5-[3-[(3S)-3-fluoropyrrolidin-1-yl]propylcarbamylamino] isothiazol-4-carboxylate I471 (150 mg, 302.1 μmol, yield: 32.66%). The product was verified by LCMS and H-NMR.

$^1$H NMR (400 MHz, $CDCl_3$) δ (ppm) 10.07 (s, 1H), 6.97 - 6.84 (m, 2H), 5.44 (s, 2H), 5.23 (d, J = 54.2 Hz, 1H), 4.57 (t, J = 8.8 Hz, 2H),3.87 (s, 3H), 3.69 - 3.49 (m, 1H), 3.46 - 3.28 (m, 1H), 3.20 (t, J = 8.6 Hz, 2H), 3.16 - 3.08 (m, 2H), 2.80 - 2.67 (m, 1H), 2.45 - 2.06 (m, 4H), 1.82 - 1.65 (m, 2H).

Step 2

3-[(5-fluoro-2,3-dihydrobenzofuran-6-yl)methoxy]-5-[3-[(3S)-3-fluoropyrrolidin-1-yl]propylcarbamoylamino]isothiazole-4-carboxamide

Methyl 3-[(5-fluoro-2,3-dihydrobenzofuran-6-yl)methoxy]-5-[3-[(3S)-3-fluoropyrrolidin-1-yl]propylcarbamylamino] isothiazol-4-carboxylate I471 (56 mg, 112.8 μmol) was dissolved in $NH_3$/MeOH (5 mL). The mixture was stirred at 40 °C to react for 16 hours. The reaction mixture was concentrated under reduced pressure to obtain a crude product, and the crude product was purified by the preparative thin-layer chromatography (DCM/MeOH = 10/1) to obtain a white solid product 3-[(5-fluoro-2,3-dihydrobenzofuran-6-yl)methoxy]-5-[4-[(3S)-3-fluoropyrrolidin-1-yl]butylcarbamylamino]isothiazol-4-formamide T119 (48 mg, 99.7 μmol, yield: 88.39%).

The product was verified by LCMS, H-NMR, C-NMR and F-NMR.

$^1$H NMR (400 MHz, $CD_3OD$) δ(ppm) 7.02 (d, J = 9.2 Hz, 1H), 6.82 (d, J = 6.0 Hz, 1H), 5.43 (s, 2H), 5.25-5.08 (m, 1H), 4.55 (t, J = 8.4 Hz, 2H), 3.26 (d, J = 6.8 Hz, 2H), 3.21 (t, J = 9.2 Hz, 2H), 3.01-2.87 (m, 2H), 2.72 - 2.61 (m, 1H), 2.58 - 2.50 (m, 2H), 2.46-2.38 (m, 1H), 2.26 - 2.15 (m, 1H), 2.06 - 1.96 (m, 1H), 1.81-1.71 (m, 2H).

$^{13}$C NMR (100 MHz, $CD_3OD$) δ (ppm) 174.06, 168.02, 164.33, 161.23, 155.67, 154.01, 129.72, 121.62, 112.49, 112.24, 109.85, 94.18, 92.46, 71.46, 63.78, 60.27, 60.04, 52.57, 51.82, 48.56, 48.43, 47.86, 47.65, 47.44, 32.32, 32.10, 29.29, 28.15, 20.72, 14.04.

$^{19}$F NMR (400 MHz, $CD_3OD$) δ (ppm) -131.15, -169.00.

Example 96: Synthesis of Compound T120

Synthesis Route:

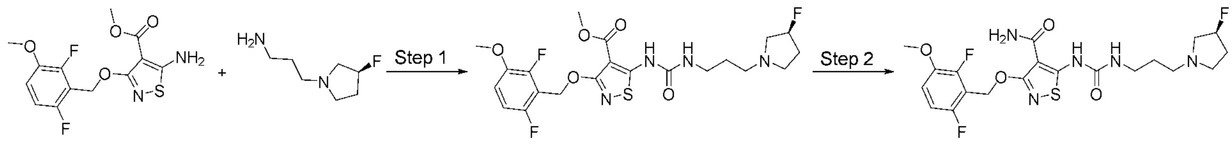


1403 1463 1472 T120

Step 1

Methyl 3-[(2,6-difluoro-3-methoxy-phenyl)methoxy]-5-[3-[(3S)-3-fluoropyrrolidin-1-yl]propylcarbamoylamino]isothiazole-4-carboxylate

3-[(3S)-3-fluoropyrrolidin-1-yl]propylamine I463 (232.38 mg, 1.59 mmol) was dissolved in anhydrous THF (5 mL), followed by the addition of CDI (257.72 mg, 1.59 mmol) under the protection of $N_2$, the mixture was then stirred at 25°C to react for 1.0 hour, followed by the addition of DMSO (5 mL), the pressure was reduced to remove THF, followed by the addition of 5-amino-3-[(2,6-difluoro-3-methoxyphenyl)methoxy]isothiazol-4-methyl formate I403 (350 mg, 1.06 mmol) and potassium carbonate (439.34 mg, 3.18 mmol), and the reaction system was stirred at 25 °C to react for 1.0 hour. Water (20 mL) was poured into the reaction mixture, which was then subjected to liquid separation and extraction with ethyl acetate (10 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3-methoxyphenyl)methoxy]-5-[3-[(3S)-3-fluoropyrrolidin-1-yl]propylcarbamylamino]isothiazol-4-carboxylate I472 (170 mg, 338.30 μmol, yield: 31.93%).

The product was verified by LCMS and HNMR.

$^1H$ NMR (400MHz, $CDCl_3$): δ (ppm) 10.04 (s, 1H), 6.81-6.93 (m, 2H), 5.48 (s, 2H), 5.15-5.30 (m, 1H), 3.87 (s, 3H), 3.76 (s, 3H), 3.57 (s, 1H), 3.30-3.35 (m, 1H), 3.02-3.14 (m ,2H), 2.60-2.76 (m, 3H), 2.32-2.48 (m, 2H), 2.03-2.27 (m, 2H), 1.71-1.76 (m, 2H).

Step 2

3-[(2,6-difluoro-3-methoxy-phenyl)methoxy]-5-[3-[(3S)-3-fluoropyrrolidin-1-yl]propylcarbamoylamino]isothiazole-4-carboxamide

Methyl 3-[(2,6-difluoro-3-methoxyphenyl)methoxy]-5-[3-[(3S)-3-fluoropyrrolidin-1-yl]propylcarbamylamino]isothiazol-4-carboxylate I472 (170 mg, 338.30 μmol) was dissolved in $NH_3$/MeOH (4 mL, 10 mol/L). The mixture was stirred at 40 °C to react for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3-methoxyphenyl)methoxy]-5-[3-[(3S)-3-fluoropyrrolidin-1-yl]propylcarbamylamino]isothiazol-4-formamide T120 (53 mg, 108.72 μmol, yield: 32.14%). The product was verified by LCMS, H-NMR, C-NMR and F-NMR.

$^1H$ NMR (400MHz, $CD_3OD$): δ (ppm) 7.12-7.18 (m, 1H), 6.94-6.99 (m, 1H), 5.55 (s, 2H), 5.37-5.50 (m, 1H), 3.86 (s,3H), 3.47-3.68 (m, 3H), 3.28-3.36 (m, 4H), 2.65 (s, 1H), 2.35-2.47 (m, 2H), 1.95-2.02 (m, 2H).

$^{13}C$ NMR (100MHz, $CD_3OD$): δ (ppm) 169.45, 166.84, 161.49, 156.42 156.36, 154.41, 153.99, 153.93, 150.08, 150.01, 144.40, 144.37, 144.29, 114.20, 114.11, 112.81, 112.65, 112.61, 112.45, 110.30, 110.26, 110.07, 110.03, 97.13, 93.98, 92.24, 59.56, 59.34, 58.35, 56.85, 52.65, 51.84, 37.71, 31.96, 31.74, 29.69, 26.85.

$^{19}F$ NMR (400MHz, $CD_3OD$): δ (ppm) -127.74, -136.93, -174.31.

Example 97: Synthesis of Compound T121

Synthesis Route:

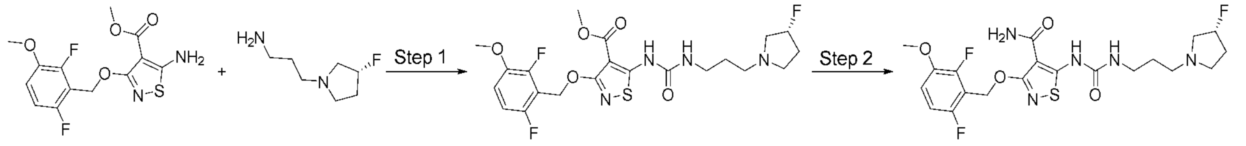


I403 I345 I473 T121

Step 1

Methyl 3-[(2,6-difluoro-3-methoxy-phenyl)methoxy]-5-[3-[(3R)-3-fluoropyrrolidin-1-yl]propylcarbamoylamino]isothiazole-4-carboxylate

3-[(3R)-3-fluoropyrrolidin-1-yl]propylamine I345 (167.3 mg, 1.14 mmol) was dissolved in anhydrous THF (3 mL), followed by the addition of CDI (164.7 mg, 1.14 mmol) under the protection of $N_2$, the mixture was then stirred at 25°C to react for 1.0 hour, followed by the addition of DMSO (3 mL), the pressure was reduced to remove most of the THF, followed by the addition of 5-amino-3-[(2,6-difluoro-3-methoxyphenyl)methoxy]isothiazol-4-methyl formate I403 (270 mg, 817.42 μmol) and potassium carbonate (338.9 mg, 2.45 mmol), and the reaction system was stirred at 25 °C to react for 1.0 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3-methoxyphenyl)methoxy]-5-[3-[(3R)-3-fluoropyrrolidin-1-yl]propylcarbamylamino]isothiazol-4-carboxylate I473 (270 mg, 537.31 μmol, yield: 65.73%).
The product was verified by LCMS and HNMR.
$^1$H NMR (400 MHz, $CDCl_3$) δ 10.04 (s, 1H), 6.95-6.89 (m, 1H), 6.86-6.81 (m, 1H), 5.48 (s, 2H), 5.30-5.13 (m, 1H), 3.87 (s, 3H), 3.77 (s, 3H), 3.64-3.53 (m, 1H), 3.39-3.29 (m, 1H), 3.16-2.96 (m, 2H), 2.76-2.69 (m, 1H), 2.65 (d, J = 5.4 Hz, 1H), 2.53-2.28 (m, 2H), 2.26-2.05 (m, 2H), 1.80-1.74 (m, 2H).

Step 2

3-[(2,6-difluoro-3-methoxy-phenyl)methoxy]-5-[3-[(3R)-3-fluoropyrrolidin-1-yl]propylcarbamoylamino]isothiazole-4-carboxamide:

Methyl 3-[(2,6-difluoro-3-methoxyphenyl)methoxy]-5-[3-[(3R)-3-fluoropyrrolidin-1-yl]propylcarbamylamino]isothiazol-4-carboxylate I473 (210 mg, 417.91 μmol) was dissolved in $NH_3$/MeOH (6 mL, 10 mol/L). The mixture was sealed to react at 40°C for 16 hours. The reaction mixture was concentrated under reduced pressure to obtain a crude product, and the crude product was purified by the preparative thin-layer chromatography (DCM/MeOH = 10/1) to obtain a product 3-[(2,6-difluoro-3-methoxyphenyl)methoxy]-5-[3-[(3R)-3-fluoropyrrolidin-1-yl]propylcarbamylamino]isothiazol-4-formamide T121 (72 mg, 147.69 μmol, yield: 35.34%).
The product was verified by LCMS, H-NMR, C-NMR and F-NMR.
$^1$H NMR (400 MHz, $CD_3OD$) δ (ppm) 7.18-7.12 (m, 1H), 6.99-6.94 (m, 1H), 5.55 (s, 2H), 5.24-5.08 (m, 1H), 3.86 (s, 3H), 3.26 (t, J = 8.0Hz, 2H), 3.00-2.88 (m, 2H), 2.72-2.58 (m, 1H), 2.55 (t, J = 7.8 Hz, 2H), 2.44-2.38 (m, 1H), 2.27-2.11 (m, 1H), 2.08-1.89 (m, 1H), 1.81-1.71 (m, 2H).
$^{13}$C NMR (400 MHz, $CD_3OD$): δ (ppm) 170.35, 167.20, 162.94, 157.54, 156.12, 145.94, 145.90, 145.83, 145.80, 115,85, 115.82, 115.75, 115.72, 113.86, 113.82, 113.66, 111.62, 111.57, 111.39, 111.34, 95.15, 93.40, 79.51, 79.19, 78.86, 61.83, 61.60, 59.61, 59.57, 59.53, 57.45, 54.77, 53.48, 39.48, 33.62, 33.40, 29.61.
$^{19}$F NMR (400 MHz, $CD_3OD$): δ(ppm) -127.71, -136.91, -169.00
Example 98: Synthesis of Compound T122

Synthesis Route:

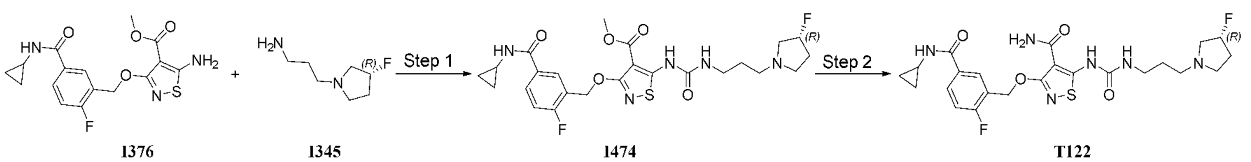


I376 I345 I474 T122

Step 1

Methyl 3-[[5-(cyclopropylcarbamoyl)-2-fluoro-phenyl]methoxy]-5-[3-[(3R)-3-fluoropyrrolidin-1-yl]propylcarbamoylamino]isothiazole-4-carboxylate

3-[(3R)-3-fluoropyrrolidin-1-yl]propylamine I345 (196.1 mg, 1.34 mmol) was dissolved in anhydrous THF (3 mL), followed by the addition of CDI (217.4 mg, 1.34 mmol) under the protection of $N_2$, the mixture was then stirred at 25°C to react for 1.0 hour, followed by the addition of DMSO (3 mL), the pressure was reduced to remove most of the THF, followed by the addition of methyl 5-amino-3-[[5-(cyclopropylcarbamoyl)-2-fluoro-phenyl]methoxy]isothiazol-4-carboxylate I376 (350 mg, 957.9 μmol) and potassium carbonate (397.2 mg, 2.9 mmol), and the reaction system was stirred at 25 °C to react for 1.0 hour. Water (50 mL) was poured into the reaction mixture, which was then subjected to liquid separation and extraction with ethyl acetate (30 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[[5-(cyclopropylcarbamoyl)-2-fluorophenyl]methoxy]-5-[3-[(3R)-3-fluoropyrrolidin-1-yl]propylcarbamoylamino]isothiazol-4-carboxylate I474 (350 mg, 651.07 μmol, yield: 67.97%).

The product was verified by LCMS and HNMR.

$^1H$ NMR (400 MHz, $CDCl_3$) δ 10.10 (s, 1H), 8.08 (dd, J= 6.8, 2.2 Hz, 1H), 7.72-7.66 (m, 1H), 7.52 (s, 1H), 7.07 (t, *J* = 9.0 Hz, 1H), 6.58 (s, 1H), 5.46 (s, 2H), 4.75 (s, 1H), 3.89 (s, 3H), 3.34-3.26 (m, 1H), 3.09-2.93 (m, 2H), 2.92-2.77 (m, 2H), 2.50-2.05 (m, 6H), 1.78-1.63 (m, 3H), 0.88-0.81 (m, 2H), 0.64-0.57 (m, 2H).

Step 2

3-[[5-(cyclopropylcarbamoyl)-2-fluoro-phenyl]methoxy]-5-[3-[(3R)-3-fluoropyrrolidin-1-yl]propylcarbamoylamino]isothiazole-4-carboxamide

Methyl 3-[[5-(cyclopropylcarbamoyl)-2-fluorophenyl]methoxy]-5-[3-[(3R)-3-fluoropyrrolidin-1-yl]propylcarbamoylamino]isothiazol-4-carboxylate I474 (350 mg, 651.07 μmol) was dissolved in $NH_3$/MeOH (6 mL, 10 mol/L). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[[5-(cyclopropylcarbamoyl)-2-fluorophenyl]methoxy]-5-[3-[(3R)-3-fluoropyrrolidin-1-yl]propylcarbamoylamino]isothiazol-4-formamide T122 (56 mg, 107.16 μmol, yield: 16.46%).

The product was verified by LCMS, H-NMR, C-NMR and F-NMR.

$^1H$ NMR (400 MHz, $CDCl_3$&$CD_3OD$ ) δ (ppm) 7.84 (d, J = 5.8 Hz, 1H), 7.77 (s, 1H), 7.09 (t, J = 8.9 Hz, 1H), 5.46 (s, 2H), 5.11 (d, J = 54.6 Hz, 1H), 3.33-3.25 (m, 2H), 2.97-2.86 (m, 2H), 2.84-2.77 (m, 1H), 2.64-2.56 (m, 1H), 2.54-2.39 (m, 3H), 2.36-2.25 (m, 1H), 2.19-1.94 (m, 3H), 0.79 (d, J = 6.4 Hz, 2H), 0.57 (s, 2H).

$^{13}C$ NMR (100 MHz, $CD_3OD$) δ(ppm) 169.24, 165.84, 164.35, 161.82, 161.50, 154.25, 130.68, 130.10, 123.17, 123.02, 116.00, 115.79, 9797.20, 94.07,92.32, 64.16, 60.65, 60.43, 60.30, 60.08, 54.24, 54.13, 53.26, 52.30, 38.57, 32.40, 32.18, 31.86, 29.63, 27.66, 26.79, 23.15, 23.01, 21.34, 11.20.

$^{19}F$ NMR (400 MHz, $CDCl_3$&$CD_3OD$) δ (ppm) -117.20, -171.14

Example 99: Synthesis of Compound T123

Synthesis Route:

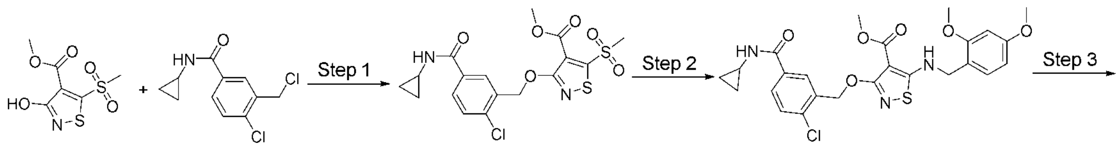


I009 I475 I476 I477

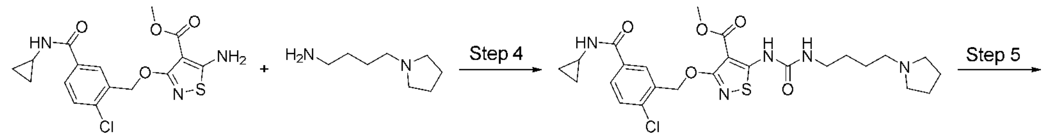


I478 I479

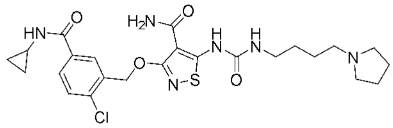

T123

Step 1

Methyl 3-[[2-chloro-5-(cyclopropylcarbamoyl)phenyl]methoxy]-5-methylsulfonyl-isothiazo le-4-carboxylate:

Methyl 3-hydroxyl-5-(methylsulfonyl)isothiazol-4-carboxylate I009 (1 g, 4.21 mmol) was dissolved in DMF(10 mL), followed by the addition of 4-chloro-3-chloromethyl-N-cyclopropyl-benzamide I475 (1.0 g, 4.21 mmol), NaI (63.2 mg, 421.49 μmol) and potassium carbonate (1.7 g, 12.64 mmol), and the reaction system was stirred at 25 °C to react for 1.0 hour. The reaction mixture was poured into water (50 mL) and filtered, and the filter cake was dissolved in ethyl acetate, dried with anhydrous sodium sulfate, and concentrated under reduced pressure to obtain a yellow solid product 3-[[2-chloro-5-(cyclopropylcarbamoyl)phenyl]methoxy]-5-methylsulfonylisothiazol-4-methyl formate I476 (1.2 g, 2.70 mmol, yield: 63.99%).

The product was verified by LCMS and H-NMR.

$^1$H NMR (400MHz, $CDCl_3$): δ (ppm) 8.12 (d, J = 2.0 Hz, 1H), 7.69 (dd, J = 8.4, 2.2 Hz, 1H), 7.45 (d, J = 8.4 Hz, 1H), 6.43 (s, 1H), 5.60 (s, 2H), 4.05 (s, 3H), 3.50 (s, 3H), 2.96-2.88 (m, 1H), 0.89 (q, J = 7.0 Hz, 1H), 0.68-0.62 (m, 1H).

Step 2

Methyl 3-[[2-chloro-5-(cyclopropylcarbamoyl)phenyl]methoxy]-5-[(2,4-dimethoxyphenyl) methylamino]isothiazole-4-carboxylate

3-[[2-chloro-5-(cyclopropylcarbamoyl)phenyl]methoxy]-5-methylsulfonylisothiazol-4-methyl formate I476 (1.2 g, 2.70 mmol) was dissolved in THF(15 mL), followed by the addition of 2,4-dimethoxybenzylamine (4.5 g, 26.97 mmol), and the reaction system was stirred at 65 °C to react for 1 hour. The mixture was poured into water (30 mL) and filtered, and the filter cake was dissolved in ethyl acetate, dried with anhydrous sodium sulfate, and concentrated under reduced pressure to obtain a white solid product methyl 3-[[2-chloro-5-(cyclopropylcarbamoyl)phenyl]methoxy]-5-[(2,4-dimethoxyphenyl)methylamino]isothiazol-4-carboxylate I477 (1.1 g, 2.07 mmol, yield: 76.66%).

The product was verified by LCMS and H-NMR.

$^1$H NMR (400 MHz, $CDCl_3$) δ (ppm) 8.19 (d, J = 2.0 Hz, 1H), 8.03 (t, J = 5.6 Hz, 1H), 7.65 (dd, J = 8.4, 2.2 Hz, 1H), 7.41 (d, J = 8.4 Hz, 1H), 7.17 (d, J = 8.2 Hz, 1H), 6.49-6.37 (m, 3H), 5.48 (s, 2H), 4.28 (d, J = 6.0 Hz, 2H), 3.92 (s, 3H), 3.86 (s, 3H), 3.81 (s, 3H), 2.95-2.87 (m, 1H), 0.87 (t, J = 6.2 Hz, 2H), 0.68-0.57 (m, 2H).

Step 3

Methyl 5-amino-3-[[2-chloro-5-(cyclopropylcarbamoyl)phenyl]methoxy]isothiazole-4-car boxylate

Methyl 3-[[2-chloro-5-(cyclopropylcarbamoyl)phenyl]methoxy]-5-[(2,4-dimethoxyphenyl)methylamino]isothiazol-4-carboxylate I477 (1 g, 1.88 mmol) was dissolved in DCM (20 mL), followed by the addition of DDQ (1.3 g, 5.64 mmol), and the reaction system was stirred at 25 °C to react for 1.0 hour. The reaction mixture was poured into water (50

mL), and extracted with DCM (50 mL x 3), the organic phases were combined, washed with saturated brine (30 mL), dried with anhydrous sodium sulfate, and concentrated under reduced pressure to obtain a crude product, and the crude product was purified by the preparative medium-pressure chromatography to obtain a white solid product methyl 5-amino-3-[[2-chloro-5-(cyclopropylcarbamoyl)phenyl]methoxy]isothiazol-4-carboxylate I478 (600 mg, 1.57 mmol, yield: 83.60%).

The product was verified by LCMS and HNMR.

$^1$H NMR (400MHz, DMSO-d6): δ (ppm) 8.57-8.54 (m, 1H), 8.17-8.16 (m, 1H), 7.99-7.93 (m, 2H), 7.79-7.76 (m, 1H), 7.59-7.56 (m, 1H), 5.40-5.38 (m, 2H), 3.80-3.79 (m, 3H), 2.88-2.83 (m, 1H), 0.72-0.68 (m, 2H), 0.58-0.53 (m, 2H).

Step 4

Methyl 3-[[2-chloro-5-(cyclopropylcarbamoyl)phenyl]methoxy]-5-(4-pyrrolidin-1-ylbutylcar bamoylamino)isothiazole-4-carboxylate

4-(pyrrolidin-1-yl)butan-1-amine (111.8 mg, 785.68 μmol) was dissolved in anhydrous THF (8 mL), followed by the addition of CDI (127.3 mg, 785.68 μmol) under the protection of $N_2$, the mixture was then stirred at 25°C to react for 1.0 hour, followed by the addition of DMSO (8 mL), the pressure was reduced to remove most of the THF, followed by the addition of methyl 5-amino-3-[[2-chloro-5-(cyclopropylcarbamoyl)phenyl]methoxy]isothiazol-4-carboxylate I478 (200 mg, 523.79 μmol) and potassium carbonate (216.9 mg, 1.57 mmol), and the reaction system was stirred at 25 °C to react for 1.0 hour. Water (60 mL) was poured into the reaction mixture, which was then subjected to liquid separation and extraction with ethyl acetate (50 mL x 3), the organic phases were combined, washed with saturated brine (50 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[[2-chloro-5-(cyclopropylcarbamoyl)phenyl]methoxy]-5-(4-pyrrolidin-1-ylbutylcarbamylamino)isothiazol-4-carboxylate I479 (100 mg, 181.80 μmol, yield: 34.71%).

The product was verified by LCMS and H-NMR.

$^1$H NMR (400MHz, $CD_3OD$): δ (ppm) 8.22 (d, J = 1.8 Hz, 1H), 7.70-7.67 (m, 1H), 7.49 (d, J = 8.4 Hz, 1H), 7.05 (s, 1H), 5.45 (s, 2H), 3.95 (s, 3H), 2.82-2.85 (m, 1H), 2.54 (dd, J = 19.8, 12.5 Hz, 7H), 1.82 (d, J = 3.4 Hz, 4H), 1.61-1.57 (m, 3H), 0.80 (dd, J = 7.0, 5.2 Hz, 2H), 0.66-0.62 (m, 2H).

Step 5

3-[[2-chloro-5-(cyclopropylcarbamoyl)phenyl]methoxy]-5-(4-pyrrolidin-1-ylbutylcarbamo ylamino)isothiazole-4-carboxamide

Methyl 3-[[2-chloro-5-(cyclopropylcarbamoyl)phenyl]methoxy]-5-(4-pyrrolidin-1-ylbutylcarbamylamino)isothiazol-4-carboxylate I479 (100 mg, 181.80 μmol) was dissolved in $NH_3$/MeOH (4 mL). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[[2-chloro-5-(cyclopropylcarbamoyl)phenyl]methoxy]-5-(4-pyrrolidin-1-ylbutylcarbamylamino)isothiazol-4-formamide T123 (22 mg, 41.12 μmol, yield: 22.62%).

The product was verified by LCMS, H-NMR and C-NMR.

$^1$H NMR (400MHz, $CD_3OD$): δ (ppm) 8.01 (d, J = 2.0 Hz, 1H), 7.79 (dd, J = 8.4, 2.2 Hz, 1H), 7.56 (d, J = 8.4 Hz, 1H), 5.61 (s, 2H), 3.26 (t, J = 6.4 Hz, 2H), 2.89-2.81 (m, 1H), 2.59 (dd, J = 20.8, 13.8 Hz, 6H), 1.85 (dd, J = 6.6, 3.4 Hz, 4H), 1.62 (d, J = 14.4 Hz, 4H), 0.85-0.78 (m, 2H), 0.68-0.62 (m, 2H).

$^{13}$C NMR (100MHz, $CD_3OD$): δ (ppm) 170.40, 170.24, 167.16, 162.93, 156.15, 138.33, 135.50, 134.65, 130.94, 130.73, 129.87, 98.71, 68.53, 57.04, 55.00, 40.88, 28.80, 26.66, 24.11, 22.43, 6.57

Example 100: Synthesis of Compound T124

Synthesis Route:

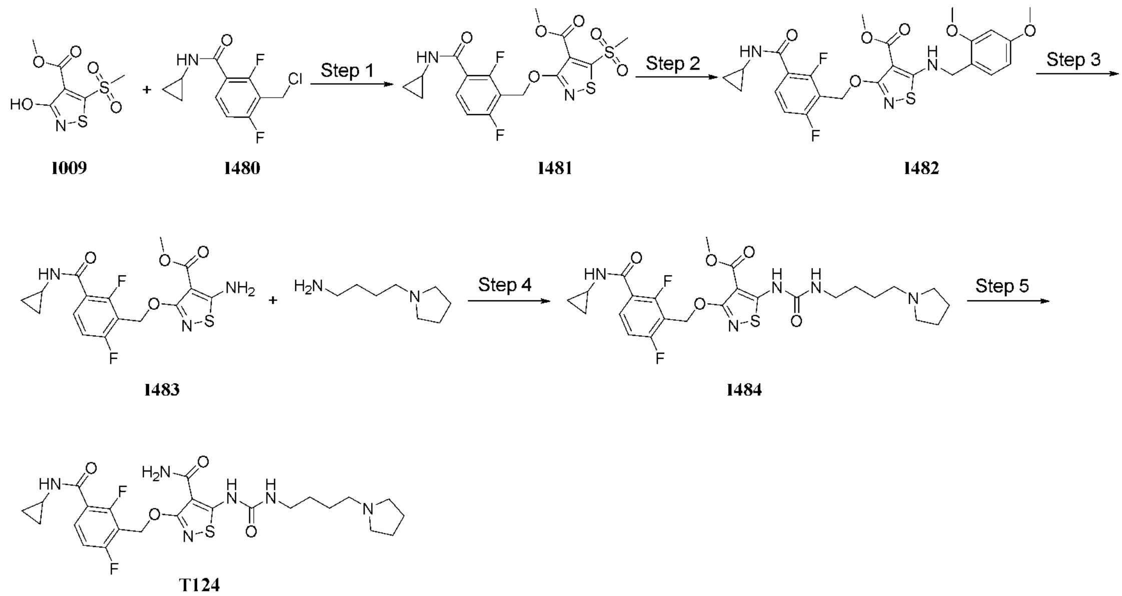


I009 I480 I481 I482

I483 I484

T124

Step 1

Methyl 3-[[3-(cyclopropylcarbamoyl)-2,6-difluoro-phenyl]methoxy]-5-methylsulfonyl-isothiazole-4-carboxylate

Methyl 3-hydroxyl-5-(methylsulfonyl)isothiazol-4-carboxylate I009 (900 mg, 3.8 mmol) was dissolved in DMF(10 mL), followed by the addition of 3-chloromethyl-N-cyclopropyl-2,4-difluorobenzamide I480 (1.3 g, 4.6 mmol) and potassium carbonate (1.6 g, 11.4 mmol), and the reaction system was stirred at 25 °C to react for 16 hours. The reaction mixture was poured into water (50 mL) and filtered, and the filter cake was dissolved in ethyl acetate, dried with anhydrous sodium sulfate, and concentrated under reduced pressure to obtain a yellow solid product 3-[[3-(cyclopropylcarbamoyl)-2,6-difluorophenyl]methoxy]-5-methylsulfonylisothiazol-4-methyl

formate I481 (900 mg, 2.02 mmol, yield: 53.1%).

The product was verified by LCMS and H-NMR.

$^1$H NMR (400MHz, $CDCl_3$): δ (ppm) 8.24 - 8.10 (m, 1H), 7.07 (t, J = 8.8 Hz, 1H), 6.75 (d, J = 10.8 Hz, 1H), 5.56 (s, 2H), 3.88 (s, 3H), 3.47 (s, 3H), 2.98 - 2.91 (m, 1H), 0.93 - 0.82 (m, 2H), 0.66 - 0.57 (m, 2H).

Step 2

Methyl 3-[[3-(cyclopropylcarbamoyl)-2,6-difluoro-phenyl]methoxy]-5-[(2,4-dimethoxyphenyl)methylamino]isothiazole-4-carboxylate:

3-[[3-(cyclopropylcarbamoyl)-2,6-difluorophenyl]methoxy]-5-methylsulfonylisothiazol-4-methyl formate I481 (900 mg, 2.0 mmol) was dissolved in THF(40 mL), followed by the addition of 2,4-dimethoxybenzylamine (3.4 g, 20.16 mmol), and the reaction system was stirred at 65 °C to react for 1 hour. The mixture was poured into water (50 mL) and filtered, and the filter cake was dissolved in ethyl acetate, dried with anhydrous sodium sulfate, and concentrated under reduced pressure to obtain a yellow solid product methyl 3-[[3-(cyclopropylcarbamoyl)-2,6-difluorophenyl]methoxy]-5-[(2,4-dimethoxyphenyl)methylamino]isothiazol-4-carboxylate I482 (900 mg, 1.7 mmol, yield: 83.7%).

The product was verified by LCMS and H-NMR.

$^1$H NMR (400MHz, $CDCl_3$): δ (ppm) 8.19 - 8.05 (m, 2H), 7.16 (d, J = 8.4 Hz, 1H), 7.04 (t, J = 8.6 Hz, 1H), 6.79 (d, J = 12.8 Hz, 1H), 6.49 - 6.41 (m, 2H), 5.46 (s, 2H), 4.27 (d, J = 6.0 Hz, 2H), 3.84 (s, 3H), 3.80 (s, 3H), 3.74 (s, 3H), 2.96 - 2.88 (m, 1H), 0.91 - 0.82 (m, 2H), 0.65 - 0.56 (m, 2H).

Step 3

Methyl 5-amino-3-[[3-(cyclopropylcarbamoyl)-2,6-difluoro-phenyl]methoxy]isothiazole-4-carboxylate

Methyl 3-[[3-(cyclopropylcarbamoyl)-2,6-difluorophenyl]methoxy]-5-[(2,4-dimethoxyphenyl)methylamino]isothiazol-4-carboxylate I482 (900 mg, 1.7 mmol) was dissolved in DCM/$H_2O$ (10/2 mL), followed by the addition of DDQ(1.2 g, 5.1 mmol), and the reaction system was stirred at 25 °C to react for 1.0 hour. The reaction mixture was

poured into water (20 mL), and extracted with DCM (10 mL x 3), the organic phases were combined, washed with saturated brine (50 mL), dried with anhydrous sodium sulfate, and concentrated under reduced pressure to obtain a crude product, and the crude product was purified by neutral alumina chromatographic columns (PE/EA = 10/1-1/1) to obtain a white solid product methyl 5-amino-3-[[3-(cyclopropylcarbamoyl)-2,6-difluorophenyl]methoxy]isothiazol-4-carboxylate I483 (360 mg, 939 μmol, yield: 55.7%).

The product was verified by LCMS and H-NMR.

$^1$H NMR (400MHz, $CDCl_3$): δ (ppm) 8.25-8.12 (m, 1H), 7.05 (t, J = 8.4 Hz, 1H), 6.78 (d, J = 12.8 Hz, 1H), 6.57 - 6.36 (m, 2H), 5.51 (s, 2H), 3.75 (s, 3H), 2.99 - 2.87 (m, 1H), 1.31 - 1.22 (m, 2H), 0.94-0.85 (m, 2H).

Step 4

Methyl 3-[[3-(cyclopropylcarbamoyl)-2,6-difluoro-phenyl]methoxy]-5-(4-pyrrolidin-1-ylbutylcarbamoylamino)isothiazole-4-carboxylate

4-(Pyrrolidin-1-yl)butan-1-amine (129.9 mg, 913 μmol) was dissolved in anhydrous THF (5 mL), followed by the addition of CDI (148 mg, 913 μmol) under the protection of $N_2$, the mixture was then stirred at 25°C to react for 1.0 hour, followed by the addition of DMSO (5 mL), the pressure was reduced to remove most of the THF, followed by the addition of methyl 5-amino-3-[[3-(cyclopropylcarbamoyl)-2,6-difluorophenyl]methoxy]isothiazol-4-carboxylate I483 (250 mg, 652.1 μmol) and potassium carbonate (270.4 mg, 2.0 mmol), and the reaction system was stirred at 25 °C to react for 1.0 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-[[3-(cyclopropylcarbamoyl)-2,6-difluorophenyl]methoxy]-5-(4-pyrrolidin-1-ylbutylcarbamylamino)isothiazol-4-carboxylate I484 (160 mg, 290.1 μmol, yield: 44.48%).

The product was verified by LCMS and H-NMR.

$^1$H NMR (400 MHz, $CDCl_3$) δ (ppm) 10.19 (s, 1H), 8.21 - 8.10 (m, 2H), 7.52 (s, 1H), 7.04 (d, J = 7.8 Hz, 1H), 6.80 (s, 1H), 5.49 (s, 2H), 3.76 (s, 3H), 3.31 (s, 2H), 2.93 (s, 1H), 2.66 - 2.47 (m, 8H), 1.87 (s, 4H), 1.68 (s, 4H), 1.25 (s, 2H), 0.88 (d, J = 6.0 Hz, 2H), 0.62 (s, 2H).

Step 5

3-[[3-(cyclopropylcarbamoyl)-2,6-difluoro-phenyl]methoxy]-5-(4-pyrrolidin-1-ylbutylcarbamoylamino)isothiazole-4-carboxamide

Methyl 3-[[3-(cyclopropylcarbamoyl)-2,6-difluorophenyl]methoxy]-5-(4-pyrrolidin-1-ylbutylcarbamylamino)isothiazol-4-carboxylate I484 (160 mg, 290.06 μmol) was dissolved in $NH_3$/MeOH (10 mL). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[[3-(cyclopropylcarbamoyl)-2,6-difluorophenyl]methoxy]-5-(4-pyrrolidin-1-ylbutylcarbamylamino)isothiazol-4-formamide T124 (41 mg, 76.41 μmol, yield: 26.34%).

The product was verified by LCMS, H-NMR, C-NMR and F-NMR.

$^1$H NMR (400MHz $CD_3OD$): δ (ppm) 7.74 (dd, J = 14.8, 8.4 Hz, 1H), 7.14 (t, J = 8.8 Hz, 1H), 5.59 (s, 2H), 3.24 (t, J = 7.2 Hz, 2H), 2.87-2.83 (m, 1H), 2.56-2.50 (m, 6H), 1.81 (s, 4H), 1.57 (d, J = 2.5 Hz, 4H), 0.83-0.79 (m, 2H), 0.64-0.60 (m, 2H).

$^{13}$C NMR (100 MHz, $CD_3OD$) δ (ppm) 170.44, 167.43, 167.07, 162.78, 159.18, 156.15, 133.24, 113.89, 113.12, 112.89, 98.57, 61.56, 58.98, 57.12, 54.99, 40.90, 28.84, 26.80, 24.14, 24.03, 20.87, 14.47, 6.63

$^{19}$F NMR (400 MHz, $CD_3OD$) δ (ppm) -111.42, -116.32.

Example 101: Synthesis of Compound T125

Synthesis Route:

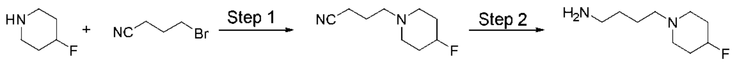


I485 I486

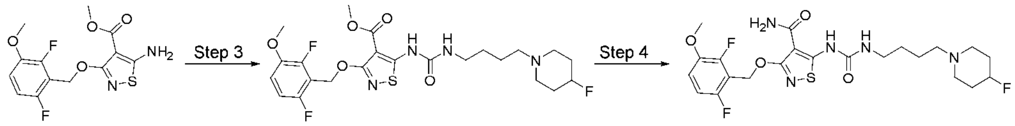


I403 I487 T125

Step 1

4-(4-fluoro-1-piperidyl)butanenitrile

4-Fluoropiperidine hydrochloride (900 mg, 8.7 mmol) was dissolved in acetonitrile (20.0 mL), followed by the addition of 4-bromobutyronitrile (1.3 g, 8.7 mmol) and potassium carbonate (3.6 g, 26.2 mmol), and the reaction system was stirred at 60 °C to react for 16 hours. The reaction mixture was poured into water (30 mL) and extracted with ethyl acetate (10 mL x 3), the organic phases were combined, washed with saturated brine (30 mL), dried with anhydrous sodium sulfate, and concentrated under reduced pressure to obtain a crude product, and the crude product was purified by the neutral alumina column chromatography (DCM) to obtain a colorless oily product 4-(4-fluoro-1-piperidyl)butyronitrile I485 (900 mg, 5.3 mmol, yield: 60.6%).

The product was verified by LCMS and H-NMR.

$^1$H NMR (400MHz, $CDCl_3$): δ (ppm) 4.77 - 4.58 (m, 1H), 2.61 - 2.52 (m, 2H), 2.49 - 2.41 (m, 4H), 2.40 - 2.32 (m, 2H), 1.97 - 1.77 (m, 6H).

Step 2

4-(4-fluoro-1-piperidyl)butan-1-amine

4-(4-Fluoro-1-piperidyl)butyronitrile I485 (900 mg, 5.3 mmol) was dissolved in methanol (10 mL), followed by the addition of raney nickel (341.4 mg, 5.8 mmol), and the reaction system was stirred at 25 °Cin the atmosphere of hydrogen to react for 16 hours. The reaction mixture was filtered, and the filtrate was concentrated under reduced pressure to obtain a brown oily product 4-(4-fluoro-1-piperidyl)butan-1-amine I486 (900 mg, 5.2 mmol, yield: 97.7%).

The product was verified by LCMS and H-NMR.

$^1$H NMR (400MHz, $CDCl_3$): δ (ppm) 4.79 - 4.58 (m, 1H), 2.70 (dd, J = 17.0, 10.1 Hz, 2H), 2.56 (d, J = 14.8 Hz, 4H), 2.47 - 2.29 (m, 4H), 2.02 - 1.81 (m, 4H), 1.66 - 1.41 (m, 4H).

Step 3

Methyl 3-[(2,6-difluoro-3-methoxy-phenyl)methoxy]-5-[4-(4-fluoro-1-piperidyl)butylcarbamoylamino]isothiazole-4-carboxylate

4-(4-Fluoro-1-piperidyl)butan-1-amine I486 (184.6 mg, 1.1 mmol) was dissolved in anhydrous THF (10 mL) followed by the addition of CDI (152.5 mg, 940.7 μmol) under the protection of $N_2$, the mixture was then stirred at 25°C to react for 1.0 hour, followed by the addition of DMSO (10 mL), the pressure was reduced to remove most of the THF, followed by the addition of 5-amino-3-[(2,6-difluoro-3-methoxyphenyl)methoxy]isothiazol-4-methyl formate I403 (250 mg, 756.9 μmol) and potassium carbonate (313.8 mg, 2.3 mmol), and the reaction system was stirred at 25 °C to react for 1.0 hour. Water (10 mL) was poured into the reaction mixture, which was then subjected to liquid separation and extraction with ethyl acetate (10 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-[(2,6-difluoro-3-methoxyphenyl)methoxy]-5-[4-(4-fluoro-1-piperidyl)butylcarbamylamino]isothiazol-4-carboxylate I487 (250 mg, 471.2 μmol, yield: 62.26%).

The product was verified by LCMS and H-NMR.

$^1$H NMR (400MHz, $CDCl_3$): δ (ppm) 10.30 (s, 1H), 6.97-6.90 (m, 1H), 6.88-6.82 (m, 1H), 5.48 (s, 2H), 3.89 (s, 3H), 3.77 (s, 2H), 3.32 (d, J = 4.8 Hz, 1H), 2.63 (s, 8H), 2.50-2.32 (m, 3H), 2.05-1.85 (m, 2H).

Step 4

3-[(2,6-difluoro-3-methoxy-phenyl)methoxy]-5-[4-(4-fluoro-1-piperidyl)butylcarbamoylamino]isothiazole-4-carboxamide

Methyl 3-[(2,6-difluoro-3-methoxyphenyl)methoxy]-5-[4-(4-fluoro-1-piperidyl)butylcarbamylamino]isothiazol-4-carboxylate I487 (250 mg, 471.2 μmol) was dissolved in $NH_3$/MeOH (5 mL, 10 mol/L), followed by the dropwise addition of 3 drops of DMSO. The mixture was sealed to react at 40 °C for 16 hours. The reaction mixture was concentrated under reduced pressure to obtain a crude product, and the crude product was purified by the column chromatography (DCM/MeOH = 10/1) to obtain a yellow solid product 3-[(2,6-difluoro-3-methoxyphenyl)methoxy]-5-[4-(4-fluoro-1-piperidyl)butylcarbamylamino]isothiazol-4-formamide T125 (25 mg, 48.5 μmol, yield: 10.3%).

The product was verified by LCMS, H-NMR, C-NMR and F-NMR.

$^1$H NMR (400MHz, $CD_3OD$): δ (ppm) 7.19 - 7.11 (m, 1H), 7.00 - 6.93 (m, 1H), 5.55 (s, 2H), 4.74 - 4.54 (m, 1H), 3.87 (s, 3H), 3.24 (t, J = 6.4 Hz, 2H), 2.66 - 2.57 (m, 2H), 2.48 - 2.34 (m, 4H), 1.97 - 1.77 (m, 4H), 1.63 - 1.51 (m, 4H).

$^{13}$C NMR (100MHz, $CD_3OD$): δ (ppm) 168.91, 165.69, 161.46, 156.11, 156.05, 154.68, 153.70, 153.64, 152.17, 152.10, 149.68, 149.60, 144.53, 144.50, 144.42, 144.39, 114.39, 114.30, 112.62, 112.47, 112.42, 112.26, 110.12, 110.08, 109.89, 109.85, 97.19, 88.45, 86.75, 58.12, 58.08, 58.04, 57.71, 55.86, 39.52, 39.03, 30.72, 30.53, 27.39, 23.49.

$^{19}$F NMR (400MHz, $CD_3OD$): δ (ppm) -127.68, -136.89.

Example 102: Synthesis of Compound T126

Synthesis Route:

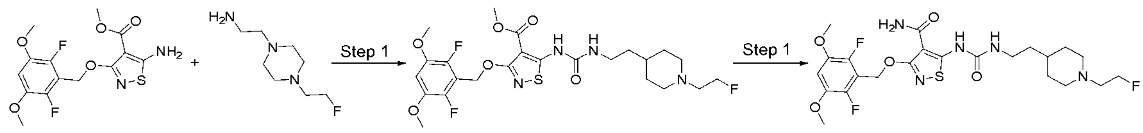


I468 I361 I488 T126

Step 1

Methyl 3-[(2,6-difluoro-3,5-dimethoxy-phenyl)methoxy]-5-[2-[1-(2-fluoroethyl)-4-piperidyl]ethylcarbamoylamino]isothiazole-4-carboxylate

2-[1-(2-fluoroethyl)-4-piperidyl]ethylamine I361 (108.8 mg, 624.42 μmol) was dissolved in anhydrous THF (5 mL), followed by the addition of CDI (101.3 mg, 624.42 μmol) under the protection of $N_2$, the mixture was then stirred at 25°C to react for 1.0 hour, followed by the addition of DMSO (5 mL), the pressure was reduced to remove most of the THF, followed by the addition of 5-amino-3-[(2,6-difluoro-3,5-dimethoxyphenyl)methoxy]isothiazol-4-methyl formate I468 (150.0 mg, 416.28 μmol) and potassium carbonate (172.6 mg, 1.25 mmol), and the reaction system was stirred at 25 °C to react for 1.0 hour. Water (20 mL) was poured into the reaction mixture, which was then subjected to liquid separation and extraction with ethyl acetate (10 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3,5-dimethoxyphenyl)methoxy]-5-[2-[1-(2-fluoroethyl)-4-piperidyl]ethylcarbamylamino]isothiazol-4-carboxylate I488 (100 mg, 178.39 μmol, yield: 42.85%).

The product was verified by LCMS and HNMR.

$^1$H NMR (400MHz, $CDCl_3$): δ (ppm) 10.33 (s, 1H), 6.66 (t, J=8.0 Hz, 1H), 5.49 (s, 1H), 5.30 (s, 1H), 4.64-4.61 (m, 1H), 4.52-4.50 (m, 1H), 3.89 (s, 6H), 3.78 (s, 3H), 3.38-3.33 (m, 2H), 2.96 (d, J=10.8 Hz, 2H), 2.73-2.71 (m ,1H), 2.66-2.64 (m, 1H), 2.08-2.03 (m, 2H), 1.51 (s, 3H), 1.34-1.25 (m, 4H).

Step 2

3-[(2,6-difluoro-3,5-dimethoxy-phenyl)methoxy]-5-[2-[1-(2-fluoroethyl)-4-piperidyl]ethylcarbamoylamino]isothiazole-4-carboxamide

Methyl 3-[(2,6-difluoro-3,5-dimethoxyphenyl)methoxy]-5-[2-[1-(2-fluoroethyl)-4-piperidyl]ethylcarbamylamino] isothiazol-4-carboxylate I488 (100 mg, 178.39 μmol) was dissolved in $NH_3$/MeOH (5 mL, 10 mol/L). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a

white solid product 3-[(2,6-difluoro-3,5-dimethoxyphenyl)methoxy]-5-[2-[1-(2-fluoroethyl)-4-piperidyl]ethylcarbamylamino]isothiazol-4-formamide T126 (20 mg, 36.66 μmol, yield: 20.55%).

The product was verified by LCMS, H-NMR, C-NMR and F-NMR.

$^1$H NMR (400MHz, $CDCl_3$): δ (ppm) 11.03 (s, 1H), 7.13 (s, 1H), 6.70 (t, J= 8.0 Hz, 1H), 5.61 (s, 2H), 5.46 (s, 1H), 4.66 (t, J= 4.8 Hz, 1H), 4.54 (t, J= 4.8 Hz, 1H), 3.92 (s, 6H), 3.38-3.33 (m, 2H), 2.98 (d, J=10.8 Hz, 2H), 2.75 (t, J= 4.8 Hz, 2H), 2.68 (t, J= 4.8 Hz ,2H), 2.07 (t, J=10.8 Hz, 2H), 1.91 (s, 4H), 1.72 (d, J=8.8 Hz, 2H), 1.53 (s, 2H).

$^{13}$C NMR (100MHz, $CD_3OD$): δ (ppm) 169.47, 165.81, 161.35, 153.93, 146.10, 143.67, 143.62, 143.56, 143.52, 113.37, 113.20, 113.03, 101.63, 97.42, 82.66, 81.00, 58.66, 58.57, 58.37, 57.25, 54.06, 38.24, 36.29, 32.98, 31.89, 29.69, 29.31, 14.10.

$^{19}$F NMR (400MHz, $CD_3OD$): δ (ppm) -145.76, -217.96.

Example 103: Synthesis of Compound T127

Synthesis Route:

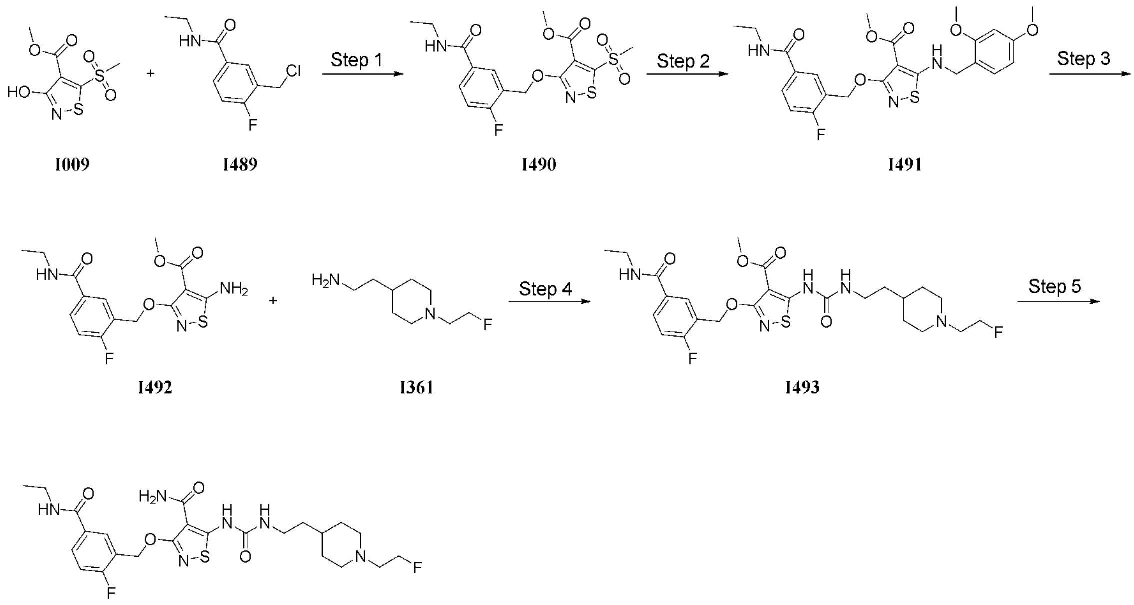


I009 I489 I490 I491

I492 I361 I493

T127

Step 1

Methyl 3-[[5-(ethylcarbamoyl)-2-fluoro-phenyl]methoxy]-5-methylsulfonyl-isothiazole-4-carboxylate

Methyl 3-hydroxyl-5-(methylsulfonyl)isothiazol-4-carboxylate I009 (800.0 mg, 3.37 mmol) was dissolved in DMF(10 mL), followed by the addition of 3-chloromethyl-N-ethyl-4-fluorobenzamide I489 (872.6 mg, 4.05 mmol) and potassium carbonate (1.4 g, 10.12 mmol), and the reaction system was stirred at 30 °C to react for 1.5 hours. The reaction mixture was poured into water (50 mL) and filtered, and the filter cake was dried to obtain a white solid product 3-[[5-(ethylcarbamoyl)-2-fluorophenyl]methoxy]-5-methylsulfonylisothiazol-4-methyl formate I490 (1.0 g, 2.40 mmol, yield: 71.21%).

The product was verified by LCMS and H-NMR.

$^1$H NMR (400MHz, $CDCl_3$): δ (ppm) 8.06-8.03 (m, 1H), 7.78-7.74 (m, 1H), 7.14 (t, *J* = 8.0 Hz, 1H), 6.24 (s, 1H), 5.57 (s, 2H), 3.99 (s, 3H), 3.53-3.46 (m, 5H), 1.26 (t, *J* = 8.2 Hz, 3H)

Step 2

Methyl 5-[(2,4-dimethoxyphenyl)methylamino]-3-[[5-(ethylcarbamoyl)-2-fluorophenyl]methoxy]isothiazole-4-carboxylate

3-[[5-(Ethylcarbamoyl)-2-fluorophenyl]methoxy]-5-methylsulfonylisothiazol-4-methyl formate I490 (1.0 g, 2.40 mmol) was dissolved in THF(12 mL), followed by the addition of 2,4-dimethoxybenzylamine (4.0 g, 24.01 mmol), and the reaction system was stirred at 65 °C to react for 1 hour. The mixture was poured into water (100 mL) and filtered, and the filter cake was dissolved in ethyl acetate, dried with anhydrous sodium sulfate, and concentrated under reduced pressure to obtain a yellow solid product methyl 5-[(2,4-dimethoxyphenyl)methylamino]-3-[[5-(ethylcarbamoyl)-2-fluorophenyl]methoxy]isothiazol-4-carboxylate I491 (1.0 g, 1.99 mmol, yield: 82.70%).

The product was verified by LCMS and H-NMR.

$^1H$ NMR (400MHz, $CDCl_3$ ): δ (ppm) 8.09 (d, J = 4.8 Hz, 1H), 7.75-7.71 (m, 1H), 7.17-7.08 (m, 2H), 6.48-6.43 (m, 2H), 5.49 (s, 2H), 4.27 (d, J = 6.0 Hz, 2H), 3.88 (s, 3H), 3.85 (s, 3H), 3.81 (s, 3H), 3.53-3.46 (m, 2H), 1.26 (t, J = 8.0 Hz, 3H)

Step 3

Methyl 5-amino-3-[[5-(ethylcarbamoyl)-2-fluoro-phenyl]methoxy]isothiazole-4-carboxylate

Methyl 5-[(2,4-dimethoxyphenyl)methylamino]-3-[[5-(ethylcarbamoyl)-2-fluorophenyl]methoxy]isothiazol-4-carboxylate I491 (1.0 g, 1.99 mmol) was dissolved in $DCM/H_2O$ (10/2 mL), followed by the addition of DDQ (450.8 mg, 1.99 mmol), and the reaction system was stirred at 25 °C to react for 1.0 hour. The reaction mixture was poured into water (50 mL), and extracted with DCM (30 mL x 3), the organic phases were combined, washed with saturated brine (30 mL), dried with anhydrous sodium sulfate, and concentrated under reduced pressure to obtain a crude product, and the crude product was purified by neutral alumina chromatographic columns (PE/EA = 10/1-0/1) to obtain a white solid product methyl 5-amino-3-[[5-(ethylcarbamoyl)-2-fluorophenyl]methoxy]isothiazol-4-carboxylate I492 (300.0 mg, 848.97 μmol, yield: 42.75%).

The product was verified by LCMS and H-NMR.

$^1H$ NMR (400MHz, $CDCl_3$): δ (ppm) 8.11 (d, J = 7.2 Hz, 1H), 7.77-7.70 (m, 1H), 7.11 (t, J = 9.0 Hz, 1H), 6.46 (s, 1H), 5.50 (s, 2H), 3.90 (s, 3H), 3.52-3.48 (m, 2H), 1.26 (t, J = 8.0Hz, 3H).

Step 4

Methyl 3-[[5-(ethylcarbamoyl)-2-fluoro-phenyl]methoxy]-5-[2-[1-(2-fluoroethyl)-4-piperidyl]ethylcarbamoylamino]isothiazole-4-carboxylate

2-[1-(2-Fluoroethyl)-4-piperidyl]ethylamine I361 (138.1 mg, 792.38 μmol) was dissolved in anhydrous THF (8 mL), followed by the addition of CDI (128.5 mg, 792.38 μmol) under the protection of $N_2$, the mixture was then stirred at 25°C to react for 1.0 hour, followed by the addition of DMSO(8 mL), the pressure was reduced to remove most of the THF, followed by the addition of methyl 5-amino-3-[[5-(ethylcarbamoyl)-2-fluorophenyl]methoxy]isothiazol-4-carboxylate I492 (200 mg, 565.98 μmol) and potassium carbonate (234.7 mg, 1.70 mmol), and the reaction system was stirred at 25 °C to react for 1.0 hour. Water (80 mL) was poured into the reaction mixture, which was then subjected to liquid separation and extraction with ethyl acetate (50 mLx3), the organic phases were combined, washed with saturated brine (50 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[[5-(ethylcarbamoyl)-2-fluorophenyl]methoxy]-5-[2-[1-(2-fluoroethyl)-4-piperidyl]ethylcarbamylamino]isothiazol-4-carboxylate I493 (200 mg, 361.26 μmol, yield: 63.83%).

The product was verified by LCMS and H-NMR.

$^1H$ NMR (400MHz, $CDCl_3$): δ (ppm) 10.32 (s, 1H), 8.10 (d, J = 4.8 Hz, 1H), 7.72 - 7.67 (m, 1H), 7.11 (s, 1H), 6.17 (s, 1H), 6.00 (s, 1H), 5.50 (s, 2H), 4.62 (t, J = 4.0 Hz 1H), 4.50 (t, J = 4.0 Hz 1H), 3.91 (s, 3H), 3.54 - 3.44 (m, 2H), 3.32 (dd, J = 13.8, 6.6 Hz, 2H), 2.95 (d, J = 11.4 Hz, 2H), 2.70 (t, J = 4.0 Hz 1H ), 2.03 (t, J = 10.8 Hz, 2H), 1.69 - 1.62 (m, 3H), 1.50 (s, 2H), 1.32 (s, 3H), 1.25 (t, J = 7.4 Hz, 3H).

Step 5

3-[[5-(ethylcarbamoyl)-2-fluoro-phenyl]methoxy]-5-[2-[1-(2-fluoroethyl)-4-piperidyl]ethylcarbamoylamino]isothiazole-4-carboxamide

Methyl 3-[[5-(ethylcarbamoyl)-2-fluorophenyl]methoxy]-5-[2-[1-(2-fluoroethyl)-4-piperidyl]ethylcarbamylamino]isothiazol-4-carboxylate I493 (150.0 mg, 270.94 μmol) was dissolved in $NH_3$/MeOH (6 mL). The mixture was sealed to

react at 40°C for 16 hours. The reaction mixture was concentrated under reduced pressure to obtain a crude product, and the crude product was purified by the preparative thin-layer chromatography (DCM/MeOH = 10/1) to obtain a white solid product 3-[[5-(ethylcarbamoyl)-2-fluorophenyl]methoxy]-5-[2-[1-(2-fluoroethyl)-4-piperidyl]ethylcarbamylamino]isothiazol-4-formamide T127 (26 mg, 48.27 μmol, yield: 17.82%). The product was verified by LCMS, H-NMR, C-NMR and F-NMR.

$^1$H NMR (400 MHz, $CDCl_3$) δ (ppm) 11.01 (s, 1H), 7.93 (d, J = 5.2 Hz, 1H), 7.79 (s, 1H), 7.15 (t, J = 8.8 Hz, 1H), 7.07 (s, 1H), 6.25 (s, 1H), 5.78 (s, 1H), 5.66 (s, 1H), 5.54 (s, 2H), 4.61 (t, J = 4.8 Hz, 1H), 4.49 (t, J = 4.8 Hz, 1H), 3.53-3.44 (m, 2H), 3.30 (d, J = 6.1 Hz, 2H), 2.93 (d, J = 10.6 Hz, 2H), 2.70 (t, J = 4.8 Hz, 1H), 2.63 (t, J = 4.8 Hz, 1H), 2.13-1.93(m, 5H), 1.65 (s, 2H), 1.47 (s, 2H), 1.34-1.21 (m, 3H).

$^{13}$C NMR (100 MHz, $CDCl_3$) δ (ppm) 169.36, 166.24, 165.79, 164.09, 161.56, 161.38, 154.17, 131.20, 131.17, 130.12, 130.07, 129.72, 129.68, 123.41, 123.26, 115.96, 115.74, 97.30, 82.56, 80.90, 63.98, 63.95, 58.51, 58.31, 53.98, 38.11, 36.16, 35.13, 32.94, 31.76, 29.68, 14.77

$^{19}$F NMR (400 MHz, $CDCl_3$) δ (ppm) -113.24, -217.86

Example 104: Synthesis of Compound T128

Synthesis Route:

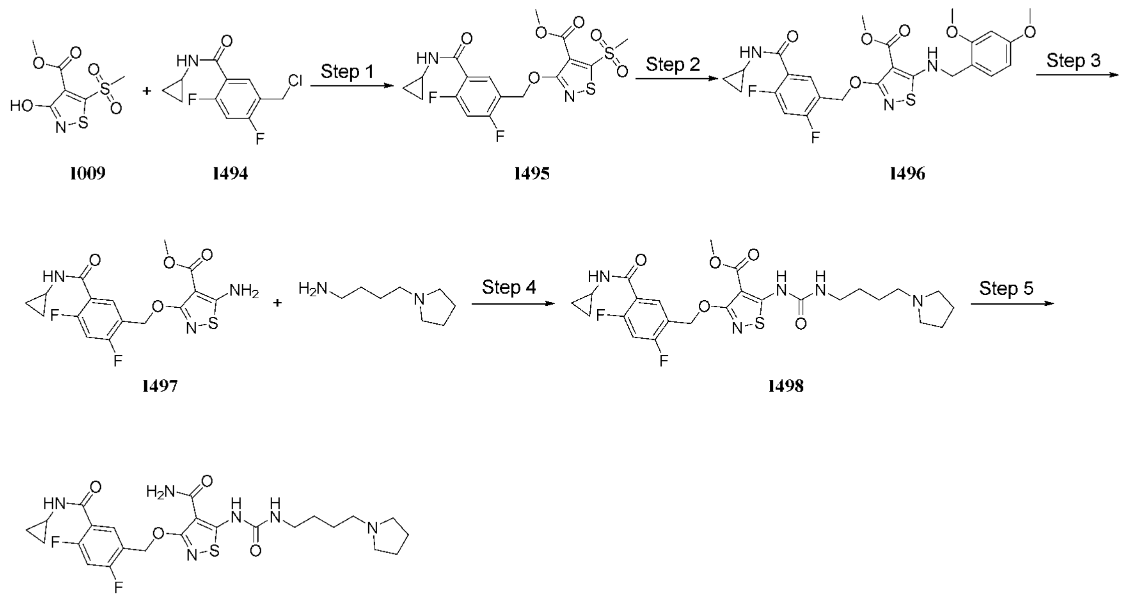


I009 I494 I495 I496 I497 I498 T128

Step 1

Methyl 3-[[5-(cyclopropylcarbamoyl)-2,4-difluoro-phenyl]methoxy]-5-methylsulfonyl-isothiazole-4-carboxylate

Methyl 3-hydroxyl-5-(methylsulfonyl)isothiazol-4-carboxylate I009 (2.2 g, 9.2 mmol) was dissolved in DMF(20 mL), followed by the addition of 5-chloromethyl-N-cyclopropyl-2,4-difluorobenzamide I494 (2.7 g, 11.0 mmol) and potassium carbonate (3.8 g, 27.5 mmol), and the reaction system was stirred at 25 °C to react for 1.0 hour. The reaction mixture was poured into water (100 mL) and filtered, and the filter cake was dissolved in ethyl acetate, dried with anhydrous sodium sulfate, and concentrated under reduced pressure to obtain a white solid product 3-[[5-(cyclopropylcarbamoyl)-2,4-difluorophenyl]methoxy]-5-methylsulfonylisothiazol-4-methyl formate I495 (3.5 g, 7.8 mmol, yield: 85.59%).

The product was verified by LCMS and H-NMR.

$^1$H NMR (400MHz, $CDCl_3$): δ (ppm) 8.37 (t, J = 8.6 Hz, 1H), 6.91 (dd, J = 11.6, 9.2 Hz, 1H), 6.73 (d, J = 12.4 Hz, 1H), 5.52 (s, 2H), 4.01 (s, 3H), 3.50 (s, 3H), 2.96-2.91 (m, 1H), 0.92-0.87 (m, 2H), 0.65-0.61 (m, 2H).

Step 2

Methyl 3-[[5-(cyclopropylcarbamoyl)-2,4-difluoro-phenyl]methoxy]-5-[(2,4-dimethoxyph enyl)methylamino]isothiazole-4-carboxylate

3-[[5-(cyclopropylcarbamoyl)-2,4-difluorophenyl]methoxy]-5-methylsulfonylisothiazol-4-methyl formate I495 (3.5 g, 7.8 mmol) was dissolved in THF(40 mL), followed by the addition of 2,4-dimethoxybenzylamine (13.1 g, 78.4 mmol), and the reaction system was stirred at 65 °C to react for 1 hour. The mixture was poured into water (100 mL) and filtered, and the filter cake was dissolved in ethyl acetate, dried with anhydrous sodium sulfate, and concentrated under reduced pressure to obtain a white solid product methyl 3-[[5-(cyclopropylcarbamoyl)-2,4-difluorophenyl]methoxy]-5-[(2,4-dimethoxyphenyl)methylamino]isothiazol-4-carboxylate I496 (3.3 g, 6.2 mmol, yield: 78.89%).

The product was verified by LCMS and H-NMR.

$^1$H NMR (400MHz, $CDCl_3$): δ (ppm) 8.44 (t, J = 8.6 Hz, 1H), 8.15 (t, J = 5.8 Hz, 1H), 7.16 (d, J = 8.2 Hz, 1H), 6.86 (dd, J = 11.6, 9.4 Hz, 1H), 6.70 (d, J = 11.8 Hz, 1H), 6.48-6.43 (m, 2H), 5.42 (s, 2H), 4.27 (d, J = 6.0 Hz, 2H), 3.91 (s, 3H), 3.85 (s, 3H), 3.80 (s, 3H), 2.95-2.90 (m, 1H), 0.87 (dd, J = 7.0, 5.6 Hz, 2H), 0.65-0.60 (m, 2H).

Step 3

Methyl 5-amino-3-[[5-(cyclopropylcarbamoyl)-2,4-difluoro-phenyl]methoxy]isothiazole-4-carboxylate

Methyl 3-[[5-(cyclopropylcarbamoyl)-2,4-difluorophenyl]methoxy]-5-[(2,4-dimethoxyphenyl)methylamino]isothiazol-4-carboxylate I496 (3.3 g, 6.2 mmol) was dissolved in DCM/$H_2O$ (100/20 mL), followed by the addition of DDQ(4.2 g, 18.6 mmol), and the reaction system was stirred at 25 °C to react for 1.0 hour. The reaction mixture was poured into water (100 mL) and extracted with ethyl acetate (100 mL x 3), the organic phases were combined, washed with saturated brine (50 mL), dried with anhydrous sodium sulfate, and concentrated under reduced pressure to obtain a crude product, and the crude product was purified by the preparative medium-pressure column chromatography to obtain a white solid product methyl 5-amino-3-[[5-(cyclopropylcarbamoyl)-2,4-difluorophenyl]methoxy]isothiazol-4-carboxylate I497 (2 g, 5.2 mmol, yield: 84.35%).

The product was verified by LCMS and HNMR.

$^1$H NMR (400MHz, DMSO-d6): δ (ppm) 8.42 (d, J = 4.0 Hz, 1H), 7.94 (s, 2H), 7.83 (t, J = 8.2 Hz, 1H), 7.40 (t, J = 10.2 Hz, 1H), 5.34 (s, 2H), 3.72 (s, 3H), 2.80-2.85(m, J = 7.4, 3.8 Hz, 1H), 0.70 (dd, J = 7.2, 2.4 Hz, 2H), 0.55-0.52 (m, 2H).

Step 4

Methyl 3-[[5-(cyclopropylcarbamoyl)-2,4-difluoro-phenyl]methoxy]-5-(4-pyrrolidin-1-ylbutylcarbamoylamino)isothiazole-4-carboxylate

4-(pyrrolidin-1-yl)butan-1-amine (167.0 mg, 1.2 mmol) was dissolved in anhydrous THF (10 mL), followed by the addition of CDI (169.0 mg, 1.2 mmol) under the protection of $N_2$, the mixture was then stirred at 25°C to react for 1.0 hour, followed by the addition of DMSO (10 mL), the pressure was reduced to remove most of the THF, followed by the addition of methyl 5-amino-3-[[5-(cyclopropylcarbamoyl)-2,4-difluorophenyl]methoxy]isothiazol-4-carboxylate I497 (300 mg, 782.5 μmol) and potassium carbonate (324.0 mg, 2.4 mmol), and the reaction system was stirred at 25 °C to react for 1.0 hour. Water (30 mL) was poured into the reaction mixture, which was then subjected to liquid separation and extraction with ethyl acetate (30 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[[5-(cyclopropylcarbamoyl)-2,4-difluorophenyl]methoxy]-5-(4-pyrrolidin-1-ylbutylcarbamylamino)isothiazol-4-carboxylate I498 (100 mg, 181.3 μmol, yield: 23.17%).

The product was verified by LCMS and H-NMR.

$^1$H NMR (400MHz, $CD_3OD$): δ (ppm) 8.04 (t, J = 8.2 Hz, 1H), 7.11 (t, J = 10.2 Hz, 1H), 5.43 (s, 2H), 3.92 (s, 3H), 3.28 (t, J = 6.4 Hz, 2H), 2.90-2.85 (m, 1H), 2.63-2.54 (m, 7H), 1.85 (s, 5H), 1.62 (d, J = 4.0 Hz, 2H), 0.88-0.79 (m, 2H), 0.69-0.59 (m, 2H).

Step 5

3-[[5-(cyclopropylcarbamoyl)-2,4-difluoro-phenyl]methoxy]-5-(4-pyrrolidin-1-ylbutylcar bamoylamino)isothiazole-4-carboxamide

Methyl 3-[[5-(cyclopropylcarbamoyl)-2,4-difluorophenyl]methoxy]-5-(4-pyrrolidin-1-ylbutylcarbamylamino)isothiazol-4-carboxylate I498 (80 mg, 145.0 μmol) was dissolved in $NH_3$/MeOH (8 mL). The mixture was sealed to react

at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[[5-(cyclopropylcarbamoyl)-2,4-difluorophenyl]methoxy]-5-(4-pyrrolidin-1-ylbutylcarbamylamino)isothiazol-4-formamide T128 (22 mg, 41.0 μmol, yield: 28.27%).

The product was verified by LCMS, H-NMR, C-NMR and F-NMR.

1H NMR (400MHz, $CD_3OD$): δ (ppm) 7.91 (t, J = 8.2 Hz, 1H), 7.15 (t, J = 10.2 Hz, 1H), 5.55 (s, 2H), 3.26 (t, J = 6.4 Hz, 2H), 2.92-2.81 (m, 1H), 2.59 (dd, J = 9.4, 3.8 Hz, 4H), 2.56-2.51 (m, 2H), 1.87-1.79 (m, 4H), 1.65-1.55 (m, 4H), 0.83-0.80 (m, 2H), 0.63 (dd, J = 3.8, 2.0 Hz, 2H).

$^{13}C$ NMR (100MHz, $CD_3OD$): δ (ppm) 169.00, 165.77, 161.42, 154.71, 132.51, 132.46, 132.41, 120.38, 120.23, 119.82, 119.72, 104.57, 104.30, 104.03, 97.27, 62.97, 62.94, 55.71, 53.56, 39.50, 27.43, 25.40, 22.73, 22.62, 5.23

$^{19}F$ NMR (400MHz, $CD_3OD$): δ (ppm) -110.90, -110.93, -111.15, -111.18

Example 105: Synthesis of Compound T129

Synthesis Route:

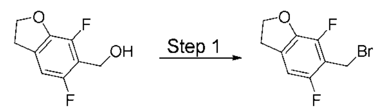


1499

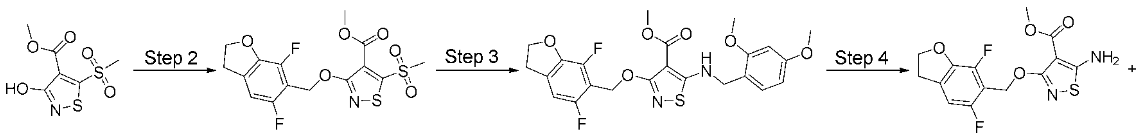


1009 1500 1501 1502

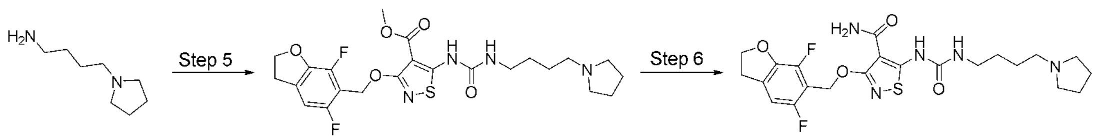


1503 T129

Step 1

6-(bromomethyl)-5,7-difluoro-2,3-dihydrobenzofuran

(5,7-difluoro-2, 3-dihydrobenzofuran-6-yl)methanol (2.7 g, 14.5 mmol) was dissolved in DCM (30 mL), followed by the addition of $PBr_3$ (5.9 g, 21.8 mmol), and the reaction system was stirred at room temperature to react for 1 hour. The reaction mixture was poured into water (50 mL) and extracted with DCM(20 mL x 3), and the organic phases were combined, washed with saturated brine (50 mL), dried with anhydrous sodium sulfate, and concentrated under reduced pressure to obtain a yellow oily crude product 6-(bromomethyl)-5,7-difluoro-2,3-dihydrobenzofuran I499 (2.5 g, 10.0 mmol, yield: 69.2%).

The product was verified by LCMS and H-NMR.

$^1H$ NMR (400MHz, $CDCl_3$): δ (ppm) 6.75 - 6.79 (m, 2H), 5.16 (d, J= 9.2 Hz, 1H), 4.64 -4.69 (m ,2H), 4.51 (s, 1H), 3.23 - 3.27 (m, 2H).

Step 2

methyl 3-[(5,7-difluoro-2,3-dihydrobenzofuran-6-yl)methoxy]-5-methylsulfonyl-isothiazole-4-carboxylate

Methyl 3-[(5,7-difluoro-2,3-dihydrobenzofuran-6-yl)methoxy]-5-methylsulfonylisothiazol-4-carboxylate

Methyl 3-hydroxyl-5-(methylsulfonyl)isothiazol-4-carboxylate I009 (2 g, 8.4 mmol) was dissolved in DMF(20 mL), followed by the addition of 6-(bromomethyl)-5,7-difluoro-2,3-dihydrobenzofuran I499 (2.5 g, 10.1 mmol) and potassium carbonate (3.5 g, 25.3 mmol), and the reaction system was stirred at 25 °C to react for 1 hour. The reaction mixture was poured into water (100 mL) and filtered, and the filter cake was dried to obtain a yellow solid product methyl 3-[(5,7-difluoro-2,3-dihydrobenzofuran-6-yl)methoxy]-5-methylsulfonylisothiazol-4-carboxylate I500 (2.2 g, 5.4 mmol, yield: 64.4%).

The product was verified by LCMS and H-NMR.

$^1$H NMR (400MHz, $CDCl_3$): δ (ppm) 6.79 (d, J=8.0 Hz, 1H), 5.51 (s, 2H), 4.67 - 4.71 (m, 2H), 3.84 - 3.90 (m, 3H), 3.47 (s, 3H), 3.24 - 3.30 (m, 2H).

Step 3

Methyl 3-[(5,7-difluoro-2,3-dihydrobenzofuran-6-yl)methoxy]-5-[(2,4-dimethoxyphenyl)methylamino]isothiazole-4-carboxylate

Methyl 3-[(5,7-difluoro-2,3-dihydrobenzofuran-6-yl)methoxy]-5-methylsulfonylisothiazol-4-carboxylate I500 (2.2 g, 5.4 mmol) was dissolved in THF(30 mL), followed by the addition of 2,4-dimethoxybenzylamine (9.1 g, 54.3 mmol), and the reaction system was stirred at 65 °C to react for 1 hour. The mixture was poured into water (100 mL) and filtered, and the filter cake was dissolved in ethyl acetate, dried with anhydrous sodium sulfate, and concentrated under reduced pressure to obtain a yellow solid product methyl 3-[(5,7-difluoro-2,3-dihydrobenzofuran-6-yl)methoxy]-5-[(2,4-dimethoxyphenyl)methylamino]isothiazol-4-carboxylate I501 (2.5 g, 5.1 mmol, yield: 93.5%).

The product was verified by LCMS and H-NMR.

$^1$H NMR (400MHz, $CDCl_3$): δ (ppm) 8.01 (s, 1H), 7.15 (d, J=8.0 Hz, 1H), 6.76 (d, J= 8.4 Hz, 1H), 6.42-6.46 (m, 2H), 5.42 (s, 2H), 4.64-4.69 (m, 2H), 4.26 (d, J=6.0 Hz, 2H), 3.83 (s, 3H), 3.80 (s, 3H), 3.72 (s, 3H), 3.23 - 3.28 (m, 2H).

Step 4

methyl 5-amino-3-[(5,7-difluoro-2,3-dihydrobenzofuran-6-yl)methoxy]isothiazole-4-carboxylate Methyl 5-amino-3-[(5,7-difluoro-2,3-dihydrobenzofuran-6-yl)methoxy]isothiazol-4-carboxylate Methyl 3-[(5,7-difluoro-2,3-dihydrobenzofuran-6-yl)methoxy]-5-[(2,4-dimethoxyphenyl)methylamino]isothiazol-4-carboxylate I501 (2.5 g, 5.1 mmol) was dissolved in DCM/$H_2O$ (80/16 mL), followed by the addition of DDQ (2.3 g, 10.2 mmol), and the reaction system was stirred at 25 °C to react for 1.0 hour. The reaction mixture was poured into water (100 mL), and extracted with DCM (100 mL x 3), the organic phases were combined, washed with saturated brine (500 mL), dried with anhydrous sodium sulfate, and concentrated under reduced pressure to obtain a crude product, and the crude product was purified by chromatographic columns (PE/EA = 1/1-3/1) to obtain a white solid product methyl 5-amino-3-[(5,7-difluoro-2,3-dihydrobenzofuran-6-yl)methoxy]isothiazol-4-carboxylate I502 (1.6 g, 4.7 mmol, yield: 92.1%). The product was verified by LCMS and H-NMR.

$^1$H NMR (400MHz, $CDCl_3$): δ (ppm) 6.77 (d, J=8.0 Hz, 1H), 6.40 (s, 2H), 5.44 (s, 2H), 4.65 - 4.70 (m, 2H), 3.76 (s, 3H), 3.24 - 3.28 (m, 2H).

Step 5

Methyl 3-[(5,7-difluoro-2,3-dihydrobenzofuran-6-yl)methoxy]-5-(4-pyrrolidin-1-ylbutylcarbamoylamino)isothiazole-4-carboxylate

4-(pyrrolidin-1-yl)butyl-1-amine (187.0 mg, 1.3 mmol) was dissolved in anhydrous THF (5 mL), followed by the addition of CDI (213.2 mg, 1.31 mmol) under the protection of $N_2$, the mixture was then stirred at 25°C to react for 1.0 hour, followed by the addition of DMSO(5 mL), the pressure was reduced to remove most of the THF, followed by the addition of methyl 5-amino-3-[(5,7-difluoro-2,3-dihydrobenzofuran-6-yl)methoxy]isothiazol-4-carboxylate I502 (300 mg, 876.38 μmol) and potassium carbonate (363.4 mg, 2.6 mmol), and the reaction system was stirred at 25 °C to react for 1.0 hour. Water (10 mL) was added to the reaction mixture, which was then extracted with ethyl acetate (10 mLx3) was subjected to liquid separation and extraction with, the organic phases were washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(5,7-difluoro-2,3-dihydrobenzofuran-6-yl)methoxy]-5-(4-pyrrolidin-1-ylbutylcarbamylamino)isothiazol-4-carboxylate I503 (300 mg, 587.6 μmol, yield: 67.1%).

The product was verified by LCMS and H-NMR.

$^1H$ NMR (400MHz, $CDCl_3$): δ (ppm) 10.21 (s, 1H), 7.89 (s, 1H), 6.77 (d, J=8.4 Hz, 1H), 5.44 (s, 2H), 4.65 - 4.70 (m, 3H), 3.77 (s, 2H), 3.21 - 3.30 (m, 4H), 2.62 (s, 1H), 2.47 - 2.55 (m, 6H), 1.84 (s, 4H).

Step 6

3-[(5,7-difluoro-2,3-dihydrobenzofuran-6-yl)methoxy]-5-(4-pyrrolidin-1-ylbutylcarbamoylamino)isothiazole-4-carboxamide

Methyl 3-[(5,7-difluoro-2,3-dihydrobenzofuran-6-yl)methoxy]-5-(4-pyrrolidin-1-ylbutylcarbamylamino)isothiazol-4-carboxylate I503 (300 mg, 587.6 μmol) was dissolved in $NH_3$/MeOH (5 mL), followed by the dropwise addition of 1 drop of DMSO. The mixture was sealed to react at 40°C for 16 hours. The reaction mixture was concentrated under reduced pressure to obtain a crude product, and the crude product was purified by the preparative thin-layer chromatography (DCM/MeOH = 10/1) to obtain a yellow solid product 3-[(5,7-difluoro-2,3-dihydrobenzofuran-6-yl)methoxy]-5-(4-pyrrolidin-1-ylbutylcarbamylamino)isothiazol-4-formamide I129 (45 mg, 90.8 μmol, yield: 15.5%).
The product was verified by LCMS, H-NMR, C-NMR and F-NMR.
$^1H$ NMR (400MHz, $CD_3OD$): δ (ppm) 6.95 (d, J = 8.4 Hz, 1H), 5.53 (s, 2H), 4.68 (t, J = 8.8 Hz, 2H), 3.30 - 3.25 (m, 4H), 2.75 (s, 4H), 2.70 - 2.65 (m, 2H), 1.89 (s, 4H), 1.68 - 1.59 (m, 4H).
$^{13}C$ NMR (100MHz, $CD_3OD$): δ (ppm) 168.89, 165.72, 161.56, 154.76, 132.61, 107.39, 72.64, 58.12, 55.48, 53.60, 39.35, 29.89, 27.28, 24.94, 22.70.
$^{19}F$ NMR (400MHz, $CD_3OD$): δ (ppm) -127.30, -142.59.

Example 106: Synthesis of Compound T130

Synthesis Route:

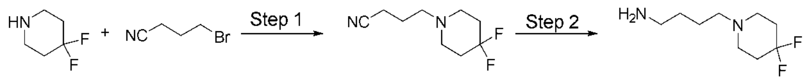


I504 I505

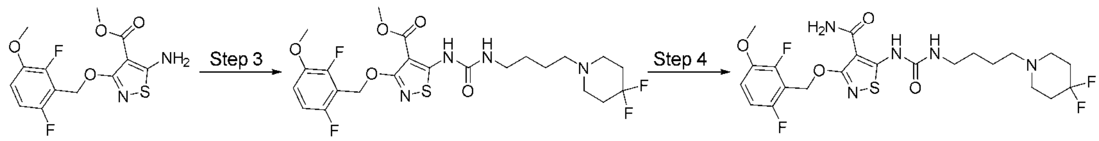


I403 I506 T130

Step 1

4-(4,4-difluoro-1-piperidyl)butanenitrile

4,4-Difluoropiperidine (4.5 g, 37.2 mmol) was dissolved in acetonitrile (50.0 mL), followed by the addition of 4-bromobutyronitrile (5.5 g, 37.2 mmol, 3.7 mL) and potassium carbonate (15.4 g, 111.5 mmol), and the reaction system was stirred at 25 °C to react for 16 hours. The reaction mixture was poured into water (100 mL) and extracted with ethyl acetate (100 mL x 3), the organic phases were combined, washed with saturated brine (200 mL), dried with anhydrous sodium sulfate, and concentrated under reduced pressure to obtain a crude product, and the crude product was purified by the neutral alumina column chromatography (DCM) to obtain a colorless oily product 4-(4,4-difluoro-1-piperidyl)butyronitrile I504 (6.5 g, 34.53 mmol, yield: 92.96%).
The product was verified by LCMS and H-NMR.
$^1H$ NMR (400MHz, $CDCl_3$): δ (ppm) 2.59 (t, J = 6.8 Hz, 1H), 2.55 - 2.49 (m, 4H), 2.43 (t, J = 6.8 Hz, 2H), 2.26 - 2.15 (m, 1H), 2.05 - 1.93 (m, 4H), 1.87 - 1.76 (m, 2H).

Step 2

4-(4,4-difluoro-1-piperidyl)butan-1-amine

4-(4,4-Difluoro-1-piperidyl)butyronitrile I504 (6.5 g, 34.5 mmol) was dissolved in methanol (500 mL), followed by the addition of raney nickel (2.2 g, 38.0 mmol), and the reaction system was stirred at 25 °C in the atmosphere of hydrogen (20 psi) to react for 16 hours. The reaction mixture was filtered, and the filtrate was concentrated under reduced pressure to obtain a colorless oily product 4-(4,4-difluoro-1-piperidyl)butan-1-amine I505 (6.5 g, 33.8 mmol, yield: 97.9%).

The product was verified by LCMS and H-NMR.

$^1$H NMR (400MHz, $CDCl_3$): δ (ppm) 4.74 (d, J = 30.8 Hz, 1H), 3.71 (s, 2H), 2.91 - 2.79 (m, 1H), 2.73 - 2.33 (m, 6H), 2.13 - 1.93 (m, 4H), 1.85 - 1.34 (m, 4H).

Step 3

Methyl 3-[(2,6-difluoro-3-methoxy-phenyl)methoxy]-5-[4-(4,4-difluoro-1-piperidyl)butylcarbamoylamino]isothiazole-4-carboxylate

4-(4,4-difluoro-1-piperidyl)butan-1-amine I505 (122.2 mg, 635.8 μmol) was dissolved in anhydrous THF (5 mL), followed by the addition of CDI (103.1 mg, 635.8 μmol) under the protection of $N_2$, the mixture was then stirred at 25°C to react for 1.0 hour, followed by the addition of DMSO (5 mL), the pressure was reduced to remove most of the THF, followed by the addition of 5-amino-3-[(2,6-difluoro-3-methoxyphenyl)methoxy]isothiazol-4-methyl formate I403 (150 mg, 454.1 μmol) and potassium carbonate (188.3 mg, 1.4 mmol), and the reaction system was stirred at 25 °C to react for 1.0 hour. Water (10 mL) was poured into the reaction mixture, which was then subjected to liquid separation and extraction with ethyl acetate (10 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-[(2,6-difluoro-3-methoxyphenyl)methoxy]-5-[4-(4,4-difluoro-1-piperidyl)butylaminoamino]isothiazol-4-carboxylate I506 (150 mg, 273.5 μmol, yield: 60.21%). The product was verified by LCMS and H-NMR.

$^1$H NMR (400MHz, $CDCl_3$): δ (ppm) 7.19-7.11 (m, 1H), 6.99-6.91 (m, 1H), 5.41 (s, 2H), 3.89 (s, 3H), 3.80 (s, 3H), 2.60 (s, 4H), 2.49-2.43 (m, 2H), 2.10-1.92 (m, 5H), 1.72-1.44 (m, 5H).

Step 4

3-[(2,6-difluoro-3-methoxy-phenyl)methoxy]-5-[4-(4,4-difluoro-1-piperidyl)butylcarbamoylamino]isothiazole-4-carboxamide

Methyl 3-[(2,6-difluoro-3-methoxyphenyl)methoxy]-5-[4-(4,4-difluoro-1-piperidyl)butylaminoamino]isothiazol-4-carboxylate I506 (150 mg, 273.5 μmol) was dissolved in $NH_3$/MeOH (5 mL, 10 mol/L), followed by the dropwise addition of 1 drop of DMSO. The mixture was sealed to react at 40°C for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3-methoxyphenyl)methoxy]-5-[4-(4,4-difluoro-1-piperidyl)butylaminoamino]isothiazol-4-formamide T130 (20 mg, 37.49 μmol, yield: 13.71%).

The product was verified by LCMS, H-NMR, C-NMR and F-NMR.

$^1$H NMR (400MHz, $CD_3OD$): δ (ppm) 7.21-7.13 (m, 1H), 7.03-6.95 (m, 1H), 5.57 (s, 2H), 3.89 (s, 3H), 3.29-3.24 (m, 2H), 2.62-2.58 (m, 4H), 2.49-2.44 (m, 4H), 2.03-1.96 (m, 4H), 1.63-1.56 (m, 4H).

$^{13}$C NMR (100MHz, $CD_3OD$): δ (ppm) 168.94, 165.71, 161.49, 158.70, 156.15, 154.73, 153.68, 144.58, 121.47, 119.08, 114.48, 112.65, 110.14, 110.10, 109.91, 109.86, 97.21, 58.06, 56.96, 56.81, 55.88, 49.72, 49.67, 49.62, 40.03, 39.48, 33.41, 33.18, 32.95, 30.29, 28.04, 27.28, 25.91, 23.82, 23.73, 19.68, 12.90.

$^{19}$F NMR (400MHz, $CD_3OD$): δ (ppm) -127.68, -136.89.

Example 107: Synthesis of Compound T131

Synthesis Route:

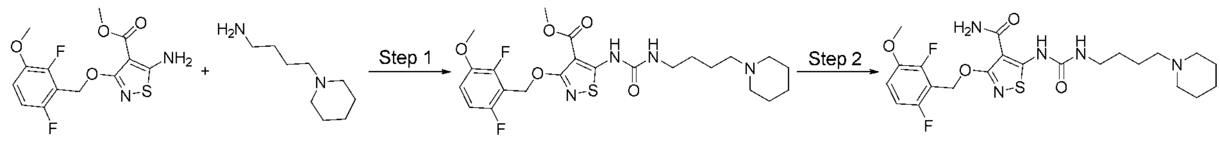


1403 I507 T131

Step 1

Methyl 3-[(2,6-difluoro-3-methoxy-phenyl)methoxy]-5-[4-(1-piperidyl)butylcarbamoyl amino]isothiazole-4-carboxylate

4-(1-Piperidyl)butan-1-amine (212.9 mg, 1.36 mmol) was dissolved in anhydrous THF (5 mL), followed by the addition of CDI (220.9 mg, 1.36 mmol) under the protection of $N_2$, the mixture was then stirred at 25°C to react for 1.0 hour, followed by the addition of DMSO (5 mL), the pressure was reduced to remove THF, followed by the addition of 5-amino-3-[(2,6-difluoro-3-methoxyphenyl)methoxy]isothiazol-4-methyl formate I403 (300 mg, 908.25 μmol) and potassium carbonate (376.6mg, 2.72 mmol), and the reaction system was stirred at 25 °C to react for 1 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3-methoxyphenyl) methoxy]-5-[4-(1-piperidyl)butylcarbamoylamino]isothiazol-4-carboxylate I507 (300.0 mg, 585.29 μmol, yield: 64.44%).
The product was verified by LCMS and H-NMR.
$^1$H NMR (400MHz, $CDCl_3$): δ (ppm) 10.26 (s, 1H), 6.91-6.93 (m, 1H), 6.82-6.87 (m, 1H), 5.49 (s, 2H), 3.88 (s, 3H), 3.76 (s, 3H), 3.31 (s, 2H), 2.40 (s, 3H), 2.31-2.34 (s, 2H), 1.60-1.63 (m, 8H), 1.35 (s, 2H).

Step 2

3-[(2,6-difluoro-3-methoxy-phenyl)methoxy]-5-[4-(1-piperidyl)butylcarbamoylamino] isothiazole-4-carboxamide

Methyl 3-[(2,6-difluoro-3-methoxyphenyl)methoxy]-5-[4-(1-piperidyl)butylcarbamoylamino]isothiazol-4-carboxylate I507 (400.0 mg, 780.38 μmol) was dissolved in $NH_3$/MeOH (10 mL, 10 mol/L). The mixture was sealed to react at 40 °C for 16 hours. The reaction mixture was concentrated under reduced pressure to obtain a crude product, and the crude product was purified by the column chromatography (DCM/MeOH = 10/1) to obtain a white solid product 3-[(2,6-difluoro-3-methoxyphenyl)methoxy]-5-[4-(1-piperidyl)butylcarbamoylamino]isothiazol-4-formamide T131 (30.0 mg, 60.29 μmol, yield: 7.73%).
The product was verified by LCMS, H-NMR, C-NMR and F-NMR.
$^1$H NMR (400MHz, $CD_3OD$): δ (ppm) 7.20-7.12(m, 1H), 7.01-6.96 (m, 1H), 5.57 (s, 2H), 3.88 (s, 3H), 3.26 (t, J = 6.4 Hz, 2H), 2.57-2.34 (m, 6H), 1.67-1.54 (m, 8H), 1.52-1.44 (m, 1H).
$^{13}$C NMR (100MHz, $CD_3OD$): δ (ppm) 168.92, 165.68, 161.46, 156.12, 156.06, 154.69, 153.70, 153.65, 152.18, 152.10, 149.68, 149.61, 144.54, 144.51, 144.43, 144.41, 114.38, 114.34, 114.27, 114.24, 112.64, 112.49, 112.43, 112.27, 110.12, 112.08, 109.89, 109.85, 97.21, 58.47, 58.11, 58.07, 58.03, 55.85, 53.95, 39.52
$^{19}$F NMR (400MHz, $CD_3OD$): δ (ppm) -127.64, -136.87

Example 108: Synthesis of Compound T132

Synthesis Route:

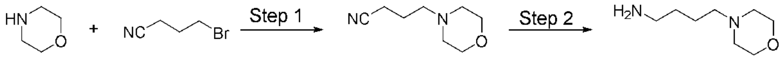


I508 I509

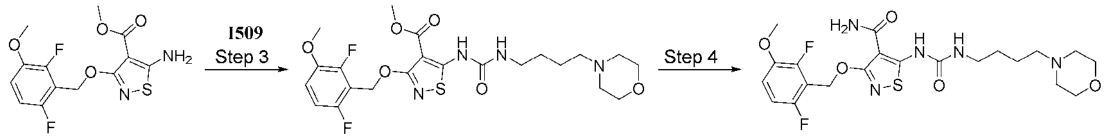


I403 I510 T132

Step 1

4-morpholinobutanenitrile

Morpholine (3.0 g, 34.44 mmol) was dissolved in acetonitrile (30.0 mL), followed by the addition of 4-bromobutyronitrile (6.1 g, 41.32 mmol) and potassium carbonate (14.3 g, 103.31 mmol), and the reaction system was stirred at 15 °C to react for 16 hours. The reaction mixture was poured into water (50 mL) and extracted with ethyl acetate (100 mL x 3), the organic phases were combined, washed with saturated brine (50 mL), dried with anhydrous sodium sulfate, and concentrated under reduced pressure to obtain a crude product, and the crude product was purified by the column chromatography (PE/EA = 1:0-1:1) to obtain a white solid product 4-morpholinylbutyronitrile I508 (5 g, 32.42 mmol, yield: 94.16%).

The product was verified by LCMS and H-NMR.

$^1$H NMR (400MHz, $CDCl_3$): $\delta$ (ppm) 3.71-3.66 (m, 4H), 2.46-2.39 (m, 8H), 1.83-1.78 (m, 2H).

Step 2

4-morpholinobutan-1-amine

4-Morpholinylbutyronitrile I508 (500.0 mg, 3.24 mmol) was dissolved in methanol (5 mL), followed by the addition of raney nickel (190.3 mg, 3.24 mmol), and the reaction system was stirred at 25 °C in the atmosphere of hydrogen (20 psi) to react for 16 hours. The reaction mixture was filtered, and the filtrate was concentrated under reduced pressure to obtain a brown oily product 4-morpholinyl-1-butylamine I509 (400 mg, 2.53 mmol, yield: 77.96%).

The product was verified by H-NMR.

$^1$H NMR (400 MHz, $CDCl_3$) $\delta$ 3.75-3.63 (m, 4H), 2.73-2.63 (m, 2H), 2.44 (s, 4H), 2.39-2.29 (m, 2H), 1.61-1.41 (m, 4H).

Step 3

Methyl 3-[(2,6-difluoro-3-methoxy-phenyl)methoxy]-5-(4-morpholinobutylcarbamoyla mino)isothiazole-4-carboxylate

4-Morpholinyl-1-butylamine I509 (134.1 mg, 847.70 $\mu$mol) was dissolved in anhydrous THF (6 mL), followed by the addition of CDI (122.0 mg, 847.70 $\mu$mol) under the protection of $N_2$, the mixture was then stirred at 25°C to react for 1.0 hour, followed by the addition of DMSO (6 mL), the pressure was reduced to remove THF, followed by the addition of 5-amino-3-[(2,6-difluoro-3-methoxyphenyl)methoxy]isothiazol-4-methyl formate I403 (200.0 mg, 605.50 $\mu$mol) and potassium carbonate (251.1 mg, 1.82 mmol), and the reaction system was stirred at 25 °C to react for 1.0 hour. Water (40 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product 3-[(2,6-difluoro-3-methoxyphenyl) methoxy]-5-(4-morpholinylbutylcarbamylamino)isothiazol-4-methyl formate I510 (200 mg, 388.70 $\mu$mol, yield: 64.19%).

The product was verified by LCMS and H-NMR.

$^1$H NMR (400MHz, $CDCl_3$): $\delta$ (ppm) 10.31 (s, 1H), 6.95-6.89 (m, 1H), 6.86-6.19 (m, 1H), 5.47 (s, 2H), 4.76 (s, 1H), 3.87 (s, 3H), 3.76 (s, 3H), 3.7-3.71 (m, 4H), 3.34-3.28 (m, 2H), 2.45 (d, J = 2.0 Hz, 4H), 2.38-2.33 (m, 2H), 1.58 (d, J = 12.8 Hz, 4H).

Step 4

3-[(2,6-difluoro-3-methoxy-phenyl)methoxy]-5-(4-morpholinobutylcarbamoylamin o)isothiazole-4-carboxamide

3-[(2,6-difluoro-3-methoxyphenyl)methoxy]-5-(4-morpholinylbutylcarbamylamino)isothiazol-4-methyl formate I510 (150.0 mg, 291.52 μmol) was dissolved in $NH_3$/MeOH (8 mL, 10 mol/L). The mixture was sealed to react at 40 °C for 16 hours. The reaction mixture was concentrated under reduced pressure to obtain a crude product, and the crude product was purified by the column chromatography (DCM/MeOH = 10/1) to obtain a white solid product 3-[(2,6-difluoro-3-methoxyphenyl)methoxy]-5-(4-morpholinylbutylcarbamylamino)isothiazol-4-formamide T132 (30 mg, 60.06 μmol, yield: 20.60%).

The product was verified by LCMS, H-NMR, C-NMR and F-NMR.

$^1$H NMR (400MHz, $CD_3OD$): δ (ppm) 7.20-7.14 (m, 1H), 7.01- 6.95 (m, 1H), 5.57 (s, 2H), 3.89 (s, 3H), 3.71 (t, J = 4.0 Hz, 4H), 3.28-3.23 (m, 2H), 2.50 (s, 4H), 2.44-2.38 (m, 2H), 1.63-1.55(m, 1H).

$^{12}$C NMR (100MHz, $CD_3OD$): δ (ppm) 168.93, 165.70, 161.48, 156.14, 156.09, 154.72, 153.73, 153.68, 452.21, 149.76, 149.68, 144.57, 144.54, 144.46, 144.43, 114.45, 114.42, 114.35, 114.32, 112.65, 112.49, 112.45, 112.29, 110.13, 110.07, 110.90, 110.86, 97.27, 66.11, 58.16, 58.10, 58.06, 58.02, 55.87, 53.25, 39.48, 27.25, 22.91

$^{19}$F NMR (400MHz, $CD_3OD$): δ (ppm)-127.68, -127.69, -136.89

Example 109: Synthesis of Compound T133

Synthesis Route:

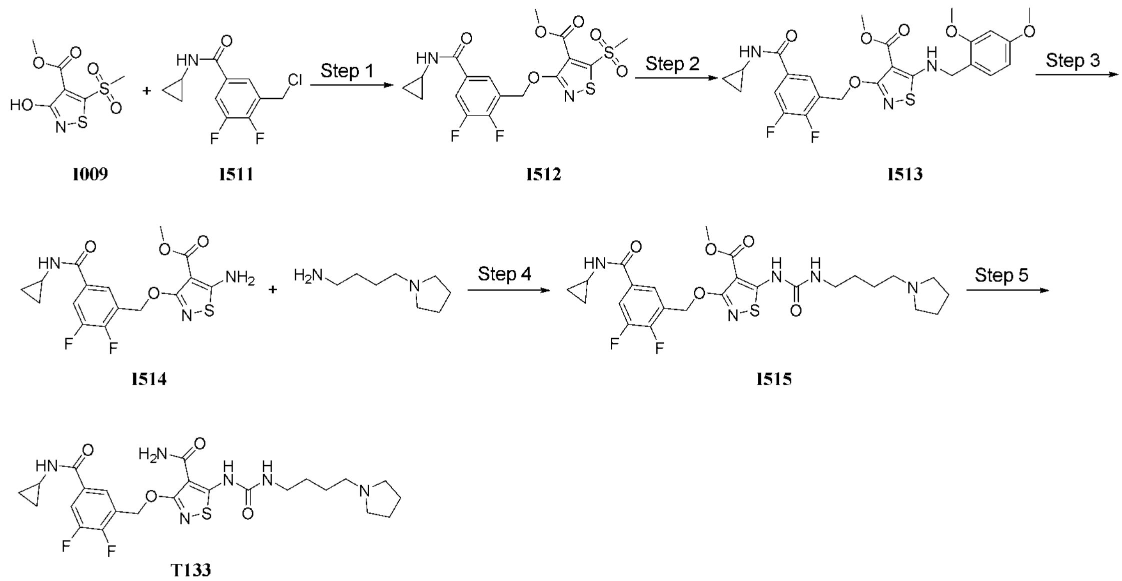


I009 I511 I512 I513

I514 I515

T133

Step 1

Methyl 3-[[5-(cyclopropylcarbamoyl)-2,3-difluoro-phenyl]methoxy]-5-methylsulfonyl-isothiazole-4-carboxylate

Methyl 3-hydroxyl-5-(methylsulfonyl)isothiazol-4-carboxylate I009 (2.5 g, 10.54 mmol) was dissolved in DMF(25 mL), followed by the addition of 3-chloromethyl-N-cyclopropyl-4,5-difluorobenzamide I511 (4.3 g, 14.75 mmol), NaI (157.9 mg, 1.05 mmol) and potassium carbonate (4.4 g, 31.61 mmol), and the reaction system was stirred at 25 °C to react for 1.5 hours. The reaction mixture was poured into water (100 mL) and filtered, and the filter cake was dissolved in ethyl acetate, dried with anhydrous sodium sulfate, and concentrated under reduced pressure to obtain a white solid product 3-[[5-(cyclopropylcarbamoyl)-2,3-difluorophenyl]methoxy]-5-methylsulfonylisothiazol-4-methyl formate I512 (3.5 g, 7.84 mmol, yield: 74.40%).

The product was verified by LCMS and H-NMR.

$^1$H NMR (400MHz, $CDCl_3$): δ (ppm) 7.78 (d, J = 5.4 Hz, 1H), 7.67-7.56 (m, 1H), 6.38 (s, 1H), 5.58 (s, 2H), 3.99 (s, 3H), 3.48 (s, 3H), 2.93 -2.86 (m, 1H), 0.91-0.86 (m, 2H), 0.66-0.60 (m, 2H).

Step 2

Methyl 3-[[5-(cyclopropylcarbamoyl)-2,3-difluoro-phenyl]methoxy]-5-[(2,4-dimethoxyphenyl)methylamino]isothiazole-4-carboxylate

3-[[5-(cyclopropylcarbamoyl)-2,3-difluorophenyl]methoxy]-5-methylsulfonylisothiazol-4-methyl formate I512 (3.5 g, 7.84 mmol) was dissolved in THF(40 mL), followed by the addition of 2,4-dimethoxybenzylamine (13.1 g, 78.4 mmol), and the reaction system was stirred at 65 °C to react for 1 hour. The mixture was poured into water (200 mL) and filtered, and the filter cake was dissolved in ethyl acetate, dried with anhydrous sodium sulfate, and concentrated under reduced pressure to obtain a yellow solid product methyl 3-[[5-(cyclopropylcarbamoyl)-2,3-difluorophenyl]methoxy]-5-[(2,4-dimethoxyphenyl)methylamino]isothiazol-4-carboxylate I513 (4 g, 7.50 mmol, yield: 95.63%).
The product was verified by LCMS and H-NMR.
$^1$H NMR (400MHz, $CDCl_3$): δ (ppm) 8.03 (t, J = 5.6 Hz, 1H), 7.82 (d, J = 5.2 Hz, 1H), 7.60-7.56 (m, 1H), 7.16 (d, J = 8.2 Hz, 1H), 6.48-6.43 (m, 2H), 6.33 (s, 1H), 5.48 (s, 2H), 4.27 (d, J = 6.0 Hz, 2H), 3.85 (d, J = 3.4 Hz, 6H), 3.80 (s, 3H), 2.91-2.87 (m, 1H), 0.90-0.85 (m, 2H), 0.64-0.60 m, 2H).

Step 3

Methyl 5-amino-3-[[5-(cyclopropylcarbamoyl)-2,3-difluoro-phenyl]methoxy]isothiazole-4-carboxylate

Methyl 3-[[5-(cyclopropylcarbamoyl)-2,3-difluorophenyl]methoxy]-5-[(2,4-dimethoxyphenyl)methylamino]isothiazol-4-carboxylate I513 (1.0 g, 1.87 mmol) was dissolved in DCM/$H_2O$ (10/2 mL), followed by the addition of DDQ (850.9 mg, 3.75 mmol), and the reaction system was stirred at 25 °C to react for 1.0 hour. The reaction mixture was poured into water (50 mL), and extracted with DCM (30 mL x 3), the organic phases were combined, washed with saturated brine (50 mL), dried with anhydrous sodium sulfate, and concentrated under reduced pressure to obtain a crude product, and the crude product was purified by chromatographic columns (PE/EA = 10/1-0/1) to obtain a yellow solid product methyl 5-amino-3-[[5-(cyclopropylcarbamoyl)-2,3-difluorophenyl]methoxy]isothiazol-4-carboxylate I514 (400.0 mg, 1.04 mmol, yield: 55.67%).
The product was verified by LCMS.

Step 4

Methyl 3-[[5-(cyclopropylcarbamoyl)-2,3-difluoro-phenyl]methoxy]-5-(4-pyrrolidin-1-ylbutylcarbamoylamino)isothiazole-4-carboxylate

4-(pyrrolidin-1-yl)butan-1-amine (77.9mg, 547.77 μmol) was dissolved in anhydrous THF (4 mL), followed by the addition of CDI (78.8 mg, 486.30 μmol) under the protection of $N_2$, the mixture was then stirred at 25°C to react for 1.0 hour, followed by the addition of DMSO(4 mL), the pressure was reduced to remove THF, followed by the addition of methyl 5-amino-3-[[5-(cyclopropylcarbamoyl)-2,3-difluorophenyl]methoxy]isothiazol-4-carboxylate I514 (150.0 mg, 391.27 μmol) and potassium carbonate (162.2 mg, 1.17 mmol), and the reaction system was stirred at 25 °C to react for 1.0 hour. Water (60 mL) was poured into the reaction mixture, which was then subjected to liquid separation and extraction with ethyl acetate (50 mL x 3), the organic phases were combined, washed with saturated brine (50 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[[5-(cyclopropylcarbamoyl)-2,3-difluorophenyl]methoxy]-5-(4-pyrrolidin-1-ylbutylcarbamylamino)isothiazol-4-carboxylate I515 (150.0 mg, 271.93 μmol, yield: 69.50%). The product was verified by LCMS and H-NMR.
$^1$H NMR (400 MHz, CDCl3) δ 10.20 (s, 1H), 8.25 (s, 1H), 7.83 (d, J = 5.2 Hz, 1H), 7.61-7.54 (m, 1H), 6.89 (s, 1H), 5.46 (s, 2H), 3.83 (s, 3H), 3.25 (s, 1H), 2.90-2.86 (m, 1H), 2.53 (d, J = 9.1 Hz, 4H), 2.48 (t, J = 5.8 Hz, 2H), 1.82 (t, J = 3.2 Hz, 4H), 1.61 (s, 4H), 0.87-0.79 (m, 2H), 0.69-0.57 (m, 2H).

Step 5

3-[[5-(cyclopropylcarbamoyl)-2,3-difluoro-phenyl]methoxy]-5-(4-pyrrolidin-1-ylbutylcar bamoylamino)isothiazole-4-carboxamide

Methyl 3-[[5-(cyclopropylcarbamoyl)-2,3-difluorophenyl]methoxy]-5-(4-pyrrolidin-1-ylbutylcarbamylamino)isothiazol-4-carboxylate I515 (150.0 mg, 271.93 μmol) was dissolved in $NH_3$/MeOH (6 mL). The mixture was sealed to react at 40 °C for 16 hours. The reaction mixture was concentrated under reduced pressure to obtain a crude product, and the crude product was purified by the preparative HPLC to obtain a white solid product 3-[[5-(cyclopropylcarbamoyl)-2,3-difluorophenyl]methoxy]-5-(4-pyrrolidin-1-ylbutylcarbamylamino)isothiazol-4-formamide

T133 (30.0 mg, 55.91 μmol, yield: 20.56%).

The product was verified by LCMS, H-NMR, C-NMR and F-NMR.

$^{1}H$ NMR (400MHz, $CD_3OD$): δ (ppm) 7.82 (d, *J* = 5.6 Hz, 1H), 7.77-7.70 (m, 1H), 5.59 (s, 2H), 3.23 (t, *J* = 6.4 Hz, 2H), 2.85-2.79 (m, 1H), 2.62-2.59 (m, 4H), 2.57-2.52 (m, 2H), 1.85-1.79 (m, 4H), 1.63-1.53 (m, 4H), 0.82-0.76 (m, 2H), 0.64-0.59 (m, 2H).

$^{12}C$ NMR (100MHz, $CD_3OD$): δ (ppm) 170.47, 168.89, 167.09, 162.75, 156,13, 153.56, 153.43, 152.65, 152.52, 151.03, 150.90, 150.18, 150.05, 132.39, 132.34, 132.29, 127.59, 127.48, 126.06, 126.12, 125.99, 117.89, 117.71, 98.69, 64.58, 57.09, 54.99, 40,91, 28.83, 26.76, 24.15, 6.53

$^{19}F$ NMR (400MHz, $CD_3OD$): δ (ppm) -139.15, -139-20, -140-05, -140.10

Example 110: Synthesis of Compound T134

Synthesis Route:

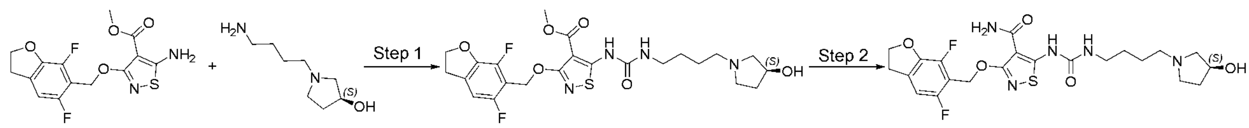


I502 I389 I516 T134

Step 1

Methyl 3-[(5,7-difluoro-2,3-dihydrobenzofuran-6-yl)methoxy]-5-[4-[(3S)-3-hydroxypyrrolidin-1-yl]butylcarbamoylamino] isothiazole-4-carboxylate

(3S)-1-(4-aminobutyl)pyrrolidin-3-ol I389 (97.1 mg, 613.5 μmol) was dissolved in anhydrous THF (5 mL), followed by the addition of CDI (99.5 mg, 613.5 μmol) under the protection of $N_2$, the mixture was then stirred at 25°C to react for 1.0 hour, followed by the addition of DMSO (5 mL), the pressure was reduced to remove THF, followed by the addition of 5-amino-3-[(5,7-difluoro-2,3-dihydrobenzofuran-6-yl)methoxy]isothiazol-4-methyl formate I502 (150 mg, 438.2 μmol) and potassium carbonate (181.7 mg, 1.3 mmol), and the reaction system was stirred at 25 °C to react for 1.0 hour. Water (30 mL) was poured into the reaction mixture, which was then subjected to liquid separation and extraction with ethyl acetate (10 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a yellow solid product methyl 3-[(5,7-difluoro-2,3-dihydrobenzofuran-6-yl)methoxy]-5-[4-[(3S)-3-hydroxylpyrrolidin-1-yl]butylcarbamylamino]isothiazol-4-carboxylate I516 (150 mg, 284.9 μmol, yield: 65.0%).

The product was verified by LCMS and H-NMR.

$^{1}H$ NMR (400MHz, $CDCl_3$): δ (ppm) 10.01 (s, 1H), 8.60 (s, 1H), 6.78 (d, J = 7.9 Hz, 1H), 5.44 (s, 2H), 4.68 (t, J = 8.8 Hz, 2H), 4.51 (s, 1H), 3.76 (s, 3H), 3.53 (s, 1H), 3.27 (t, J = 8.8 Hz, 1H), 3.18 (s, 2H), 3.01 (d, J = 10.0 Hz, 1H), 2.70-2.40 (m, 3H), 2.38 - 2.30 (m, 1H), 2.26 - 2.12 (m, 2H), 1.95 - 1.88 (m, 1H), 1.83 - 1.69 (m, 3H).

Step 2

3-[(5,7-difluoro-2,3-dihydrobenzofuran-6-yl)methoxy]-5-[4-[(3S)-3-hydroxypyrrolidin-1-yl]butylcarbamoylamino]isothiazole-4-carboxamide

Methyl 3-[(5,7-difluoro-2,3-dihydrobenzofuran-6-yl)methoxy]-5-[4-[(3S)-3-hydroxylpyrrolidin-1-yl]butylcarbamylamino]isothiazol-4-carboxylate I516 (150 mg, 284.9 μmol) was dissolved in $NH_3$/MeOH (3 mL). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[(5,7-difluoro-2,3-dihydrobenzofuran-6-yl)methoxy]-5-[4-[(3S)-3-hydroxylpyrrolidin-1-yl]butylcarbamylamino]isothiazol-4-formamide T134 (22 mg, 43.0 μmol, yield: 15.1%).

The product was verified by LCMS, H-NMR, C-NMR and F-NMR.

$^{1}H$ NMR (400MHz, $CD_3OD$): δ (ppm) 6.92 (d, J = 8.8 Hz, 1H), 5.50 (s, 2H), 4.66 (t, J = 8.8 Hz, 2H), 4.38 - 4.31 (m, 1H), 3.29 - 3.20 (m, 4H), 2.87-2.79 (m, 1H), 2.78 - 2.69 (m, 1H), 2.62-2.46 (m, 4H), 2.18-2.05 (m, 1H), 1.78-1.66 (m, 1H), 1.64-1.51 (m, 4H).

$^{13}C$ NMR (100MHz, $CD_3OD$): δ (ppm) 170.35, 167.23, 163.02, 157.85, 157.80, 156.14, 155.44, 155.39, 148.14, 148.06, 145.65, 145.58, 144.46, 144.35, 133.83, 133.78, 133.72, 133.67, 112.28, 112.13, 112.08, 111.92, 108.92, 108.88, 108.66, 108.63, 98.74, 79.49, 79.16, 78.84, 74.07, 71.45, 63.59, 59.67, 57.39, 53.85, 41.07, 35.17, 31.47, 28.86, 26.74.

$^{19}F$ NMR (400MHz, $CD_3OD$): δ (ppm) -127.28, -142.57.

Example 111: Synthesis of Compound T135

Synthesis Route:

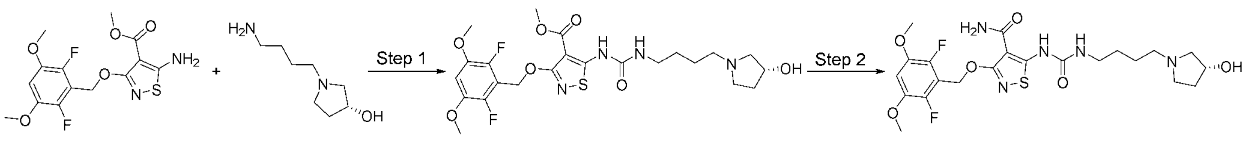


1468 1392 1517 T135

Step 1

Methyl 3-[(2,6-difluoro-3,5-dimethoxy-phenyl)methoxy]-5-[4-[(3R)-3-hydroxypyrrolidin-1-yl]butylcarbamoylamino]isothiazole-4-carboxylate

(3R)-1-(4-aminobutyl)pyrrolidin-3-ol I392 (300.0 mg, 1.90 mmol) was dissolved in anhydrous THF (5 mL), followed by the addition of CDI (300.0 mg, 1.85 mmol) under the protection of $N_2$, the mixture was then stirred at 25°C to react for 1.0 hour, followed by the addition of DMSO (5 mL), the pressure was reduced to remove THF, followed by the addition of methyl 5-amino-3-[(2,6-difluoro-3,5-dimethoxyphenyl)methoxy]isothiazol-4-carboxylate I468 (200.0 mg, 555.04 μmol) and potassium carbonate (400.0mg, 2.89 mmol), and the reaction system was stirred at 25 °C to react for 1.0 hour. Water (60 mL) was poured into the reaction mixture, which was then subjected to liquid separation and extraction with ethyl acetate (30 mL x 3), the organic phases were combined, washed with saturated brine (50 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3,5-dimethoxyphenyl)methoxy]-5-[4-[(3R)-hydroxylpyrrolidin-1-yl]butylcarbamylamino]isothiazol-4-carboxylate I517 (100.0 mg, 183.63 μmol, yield: 33.08%).

The product was verified by LCMS and H-NMR.

$^1H$ NMR (400MHz, $CDCl_3$ ): δ (ppm) 6.66 (s, 1H), 5.48 (s, 2H), 3.89 (d, J = 4.0 Hz, 6H), 3.76 (s, 3H), 3.22-3.12 (m, 2H), 3.00 (d, J = 10.4 Hz, 1H), 2.60-2.54 (m, 1H), 2.52-2.45 (m, 1H), 2.35 (dd, J = 10.4, 4.2 Hz, 1H), 2.25-2.12 (m, 2H), 1.95-1.87 (m, 2H), 1.69-1.64 (m,5H).

Step 2

3-[(2,6-difluoro-3,5-dimethoxy-phenyl)methoxy]-5-[4-[(3R)-3-hydroxypyrrolidin-1-yl]butylcarbamoylamino]isothiazole-4-carboxamide

Methyl 3-[(2,6-difluoro-3,5-dimethoxyphenyl)methoxy]-5-[4-[(3R)-hydroxylpyrrolidin-1-yl]butylcarbamylamino] isothiazol-4-carboxylate I517 (90.0 mg, 165.27 μmol) was dissolved in $NH_3$/MeOH (5 mL, 10 mol/L). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3,5-dimethoxyphenyl)methoxy]-5-[4-[(3R)-hydroxylpyrrolidin-1-yl]butylcarbamylamino]isothiazol-4-formamide T135 (15.43 mg, 29.14 μmol, yield: 17.63%).

The product was verified by LCMS, H-NMR, C-NMR and F-NMR.

$^1H$ NMR (400MHz, $CD_3OD$): δ (ppm) 6.92 (t, J = 8.4 Hz, 1H), 5.54 (s, 2H), 4.38-4.34 (m, 1H), 3.88 (s, 6H), 3.24 (t, J = 6.0 Hz, 2H), 2.92-79 (m, 2H), 2.70-2.54 (m, 4H), 2.17-2.07 (m, 1H), 1.78-1.70 (m, 1H), 1.58 (d, J = 3.6 Hz, 4H).

$^{13}C$ NMR (100 MHz, $CD_3OD$):170.34, 167.11, 162.90, 156.14, 147.04, 146.99, 145.26, 145.22, 145.16, 145.11, 144.63, 144.57, 114.46, 114.29, 114.12, 102.85, 101.41, 98.62, 71.33, 63.45, 59.62, 59.58, 57.62, 57.29, 53.75, 40.87, 34.99, 28.73, 26.47.

$^{19}F$ NMR (400MHz, $CD_3OD$): δ (ppm) -148.66

Example 112: Synthesis of Compound T136

Synthesis Route:

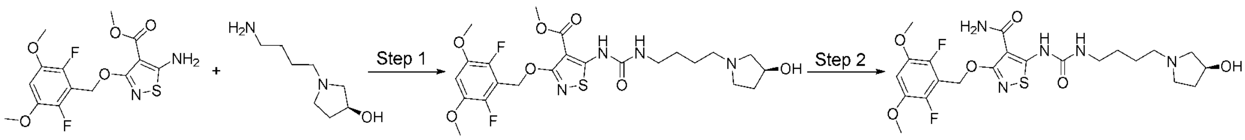


I468 I389 I518 T136

Step 1

Methyl 3-[(2,6-difluoro-3,5-dimethoxy-phenyl)methoxy]-5-[4-[(3S)-3-hydroxypyrrolidin-1-yl]butylcarbamoylamino]isothiazole-4-carboxylate

(3S)-1-(4-aminobutyl)pyrrolidin-3-ol I389 (300.0 mg, 1.90 mmol) was dissolved in anhydrous THF (6 mL), followed by the addition of CDI (300.0 mg, 1.85 mmol) under the protection of $N_2$, the mixture was then stirred at 25°C to react for 1.0 hour, followed by the addition of DMSO (6 mL), the pressure was reduced to remove THF, followed by the addition of methyl 5-amino-3-[(2,6-difluoro-3,5-dimethoxyphenyl)methoxy]isothiazol-4-carboxylate I468 (200.0 mg, 555.04 μmol) and potassium carbonate (230.1 mg, 1.67 mmol), and the reaction system was stirred at 30 °C to react for 1.0 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3,5-dimethoxyphenyl)methoxy]-5-[4-[(3S)-hydroxylpyrrolidin-1-yl]butylcarbamylamino]isothiazol-4-carboxylate I518 (130.0 mg, 238.72 μmol, yield: 43.01%). The product was verified by LCMS and H-NMR.

$^1$H NMR (400MHz, $CDCl_3$ ): δ (ppm) 10.01 (s, 1H), 8.47 (s, 1H), 6.66 (t, J = 8.0 Hz, 1H), 5.48 (s, 2H), 3.88 (s, 6H), 3.76 (s, 3H), 3.16 (d, J = 8.6 Hz, 2H), 3.00 (d, J = 10.4 Hz, 1H), 2.65-2.57 (m, 1H), 2.53-2.46 (m, 1H), 2.36 (dd, J = 10.4, 4.2 Hz, 1H), 2.24-2.14(m, 2H), 1.95-1.87 (m, 2H), 1.71-1.60 (m, 5H).

Step 2

3-[(2,6-difluoro-3,5-dimethoxy-phenyl)methoxy]-5-[4-[(3S)-3-hydroxypyrrolidin-1-yl]butylcarbamoylamino]isothiazole-4-carboxamide

Methyl 3-[(2,6-difluoro-3,5-dimethoxyphenyl)methoxy]]-5-[4-[(3S)-hydroxylpyrrolidin-1-yl]butylcarbamylamino] isothiazol-4-carboxylate I518 (120.0 mg, 220.36 μmol) was dissolved in $NH_3$/MeOH (8 mL, 10 mol/L). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3,5-dimethoxyphenyl)methoxy]-5-[4-[(3S)-hydroxylpyrrolidin-1-yl]butylcarbamylamino]isothiazol-4-formamide T136 (27.32 mg, 51.59 μmol, yield: 23.41%).

The product was verified by LCMS, H-NMR, C-NMR and F-NMR.

$^1$H NMR (400MHz, $CD_3OD$): δ (ppm) 6.93 (t, *J* = 8.4 Hz, 1H), 5.54 (s, 2H), 4.38-4.33 (m, 1H), 3.88 (s, 6H), 3.24 (t, *J* = 6.2 Hz, 2H), 2.89-2.85(m, 1H), 2.83-2.77 (m, 1H), 2.68-2.61 (m, 1H), 2.58-2.54 (m, 3H), 2.17-2.08 (m, 1H), 1.78-1.69 (m, 1H), 1.63-1.54 (m, 4H).

$^{13}$C NMR (100 MHz, $CD_3OD$):170.33, 167.11, 162.91, 156.18, 147.06, 147.00, 145.27, 145.23, 145.17, 145.12, 144.65, 144.59, 114.29, 102.91, 71.11, 63.32, 59.67, 59.62, 59.57, 57.68, 57.63, 57.18, 53.76, 53.71, 40.2, 34.79, 28.59, 26.00

$^{19}$F NMR (400MHz, $CD_3OD$): δ (ppm) -148.65

Example 113: Synthesis of Compound T137

Synthesis Route:

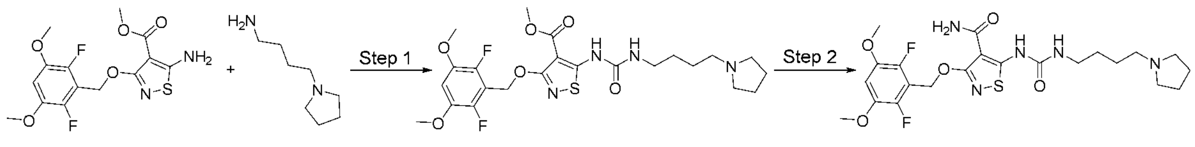


1468 I519 T137

Step 1

Methyl 3-[(2,6-difluoro-3,5-dimethoxy-phenyl)methoxy]-5-(4-pyrrolidin-1-ylbutylcarbamoylamino)isothiazole-4-carboxylate

4-(Pyrrolidin-1-yl)butan-1-amine (675.0 mg, 4.75 mmol) was dissolved in anhydrous THF (6 mL), followed by the addition of CDI (675.0 mg, 4.16 mmol) under the protection of $N_2$, the mixture was then stirred at 30°C to react for 1.0 hour, followed by the addition of DMSO(6 mL), the pressure was reduced to remove THF, followed by the addition of methyl 5-amino-3-[(2,6-difluoro-3,5-dimethoxyphenyl)methoxy]isothiazol-4-carboxylate I468 (450.0 mg, 1.25 mmol) and potassium carbonate (517.8 mg, 3.75 mmol), and the reaction system was stirred at 30 °C to react for 1.0 hour. Water (60 mL) was poured into the reaction mixture, which was then subjected to liquid separation and extraction with ethyl acetate (50 mL x 3), and the organic phases were washed with saturated brine (30 mL), dried with anhydrous sodium sulfate, and concentrated under reduced pressure to obtain a crude product. The crude product was purified by the preparative thin-layer chromatography (DCM/MeOH = 10/1) to obtain a white solid product methyl 3-[(2,6-difluoro-3,5-dimethoxy-phenyl) methoxy]-5-(4-pyrrolidin-1-ylbutylcarbamate)isothiazol-4-carboxylate I519 (250 mg, 472.98 μmol, yield: 37.87%).

The product was verified by LCMS and H-NMR.

$^1H$ NMR (400MHz, $CDCl_3$): δ (ppm) 6.66 (t, J = 8.0 Hz, 1H), 5.49 (s, 2H), 3.88 (s, 6H), 3.77 (s, 3H), 2.69 (s, 4H), 2.62-2.58 (m, 2H), 1.90 (s, 4H), 1.68 (s, 4H).

Step 2

3-[(2,6-difluoro-3,5-dimethoxy-phenyl)methoxy]-5-(4-pyrrolidin-1-ylbutylcarbamoylamino) isothiazole-4-carboxamide

Methyl 3-[(2,6-difluoro-3,5-dimethoxy-phenyl)methoxy]-5-(4-pyrrolidin-1-ylbutylcarbamate)isothiazol-4-carboxylate I519 (230.0 mg, 435.14 μmol) was dissolved in $NH_3$/MeOH (6 mL, 10 mol/L). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3,5-dimethoxy-phenyl)methoxy]-5-(4-pyrrolidin-1-ylbutylcarbamate)isothiazol-4-formamide T137 (74.64 mg, 145.34 μmol, yield: 33.40%).

The product was verified by LCMS, H-NMR, C-NMR and F-NMR.

$^1H$ NMR (400 MHz, $CDCl_3$): δ (ppm) 10.89 (s, 1H), 7.79 (s, 1H), 7.05 (s, 1H), 6.67 (t, J = 8.2 Hz, 1H), 5.58 (s, 2H), 5.47 (s, 1H), 3.89 (s, 6H), 3.30 (s, 2H), 2.60 (s, 4H), 2.53 (t, J = 6.0 Hz , 2H), 1.86 (s, 4H), 1.66 (s, 4H).

$^{13}C$ NMR (100 MHz, $CD_3OD$): δ (ppm)169.60, 165.78, 161.38, 154.15, 146.15, 146.10, 143.72, 143.66, 143.62, 143.55, 143.51, 129.56, 115.56, 113.48, 113.31, 113.14, 101.66, 97.27, 58.60, 58.56, 58.51, 57.27, 55.84, 54.02, 53.89, 40.53, 40.50, 40.46, 40.44, 19.69, 28.04, 26.58, 23.38, $^{19}F$ NMR (400MHz, $CD_3OD$): δ (ppm) -145.66

Example 114: Synthesis of Compound T138

Synthesis Route:

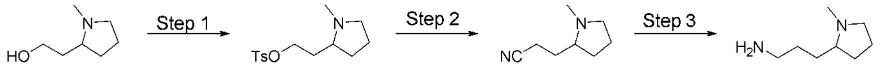


I520 I521 I522

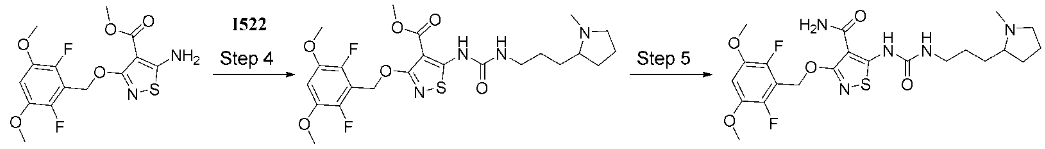


1468 1523 T138

Step 1

2-(1-methylpyrrolidin-2-yl)ethyl 4-methylbenzenesulfonate

2-(1-Methylpyrrolidin-2-yl) ethanol(19.0 g, 147.06 mmol) was dissolved in THF(100 mL), followed by the addition of sodium hydride (5.3 g, 220.59 mmol), and the mixture was then stirred at 0 °C to react for 0.5 hours. 4-Methylbenzene sulfonyl chloride (15.6 g, 220.59 mmol) was added, and the reaction system was stirred at 25°C to react for 16 hours. The reaction mixture was poured into water (100 mL), and extracted with ethyl acetate (100 mL x 3), and the organic phases were combined, washed with saturated brine (200 mL), dried with anhydrous sodium sulfate, and concentrated under reduced pressure to obtain a crude product. The crude product was purified by the silica-gel column chromatography (PE/EA = 0/1-DCM/MeOH = 10/1) to obtain a brown oily product 2-(1-methylpyrrolidin-2-yl)ethyl4-methylbenzene sulfonate I520 (32.0 g, 112.92 mmol, yield: 76.79%).
The product was verified by LCMS and H-NMR.
$^1$H NMR (400 MHz, $CDCl_3$) δ (ppm) 7.78 (d, J = 8.0 Hz, 2H), 7.16 (d, J = 7.8 Hz, 2H), 5.04-4.98 (m, 1H), 4.26-4.14 (m, 2H), 3.91-3.76(m 1H), 3.28- 3.12 (m, 2H), 3.24-3.16 (m, 2H), 3.03-2.88(m, 3H)2.69-2.49 (m, 2H), 2.50-2.36 (m, 2H), 2.34 (s, 3H).

Step 2

3-(1-methylpyrrolidin-2-yl)propanenitrile

2-(1-Methylpyrrolidin-2-yl)ethyl4-methylbenzene sulfonate I520 (10.0 g, 35.29 mmol) was dissolved in DMSO(100 mL), followed by the addition of sodium cyanide (1.9 g, 35.29 mmol), and the reaction system was stirred at 100 °C to react for 16 hours. The reaction mixture was poured into water (100 mL) and extracted with ethyl acetate (50 mL x 3), the organic phases were combined, washed with saturated brine (50 mL), dried with anhydrous sodium sulfate, and concentrated under reduced pressure to obtain a crude product, and the crude product was purified by the silica-gel column chromatography (DCM-DCM/MeOH = 1-10/1) to obtain a yellow oily product 3-(1-methylpyrrolidin-2-yl)propionitrile I521 (3.5 g, 25.32 mmol, yield: 71.76%).
The product was verified by LCMS and H-NMR.
$^1$H NMR (400MHz, $CDCl_3$): δ (ppm) 3.06-3.02 (m, 1H), 2.47-2.39 (m, 1H), 2.30 (s, 3H), 2.24-2.15 (m, 2H), 2.06-1.88 (m, 3H), 1.76-1.59 (m, 3H), 1.50-1.38 (m, 1H).

Step 3

3-(1-methylpyrrolidin-2-yl)propan-1-amine

3-(1-Methylpyrrolidin-2-yl)propionitrile I521 (700.0 mg, 5.06 mmol) was dissolved in methanol (10 mL), followed by the addition of raney nickel (700.0 mg, 5.06 mmol), and the reaction system was stirred at 25 °C in the atmosphere of hydrogen (20 psi) to react for 4 hours. The reaction mixture was filtered, and the filtrate was concentrated under reduced pressure to obtain a brown oily product 3-(1-methylpyrrolidin-2-yl)propyl-1-amine I522 (400 mg, 2.81 mmol, yield: 55.52%). The product was verified by LCMS and H-NMR.
$^1$H NMR (400MHz, $CDCl_3$): δ (ppm) 3.47 (d, J = 1.4 Hz, 1H), 3.11-3.01 (m, 1H), 2.76-2.67 (m, 2H), 2.30 (s, 3H), 2.13 (dd, J = 17.8, 9.4 Hz, 1H), 2.02-1.89 (m, 2H), 1.82-1.64 (m, 4H), 1.50-1.35 (m, 4H).

Step 4

Methyl 3-[(2,6-difluoro-3,5-dimethoxy-phenyl)methoxy]-5-[3-(1-methylpyrrolidin-2-yl)propylcarbamoylamino]isothiazole-4-carboxylate

3-(1-Methylpyrrolidin-2-yl)propyl-1-amine I522 (442.1 mg, 3.11 mmol) was dissolved in anhydrous THF (6 mL), followed by the addition of CDI (447.4 mg, 2.76 mmol) under the protection of $N_2$, the mixture was then stirred at 25°C to react for 1.0 hour, followed by the addition of DMSO(6 mL), the pressure was reduced to remove THF, followed by the addition of methyl 5-amino-3-[(2,6-difluoro-3,5-dimethoxyphenyl)methoxy]isothiazol-4-carboxylate I468 (280.0 mg, 777.06 μmol) and potassium carbonate (322.2 mg, 2.33 mmol), and the reaction system was stirred at 25 °C to react for 1.0 hour. Water (60 mL) was poured into the reaction mixture, which was then subjected to liquid separation and extraction with ethyl acetate (30 mL x 3), the organic phases were washed with saturated brine (50 mL), dried with anhydrous sodium sulfate, and concentrated under reduced pressure to obtain a crude product, and the crude product was purified by the preparative thin-layer chromatography (DCM/MeOH = 10/1) to obtain a white solid product 3-[(2,6-difluoro-3,5-dimethoxy-phenyl)methoxy]-5-[3-(1-methylpyrrolidin-2-yl)propylcarbamoylamino]isothiazol-4-methyl formate I523 (100 mg, 189.19 μmol, yield: 24.35%).

The product was verified by LCMS and H-NMR.

$^1H$ NMR (400MHz, $CDCl_3$): δ (ppm) 6.66 (t, J = 8.0 Hz, 1H), 5.49 (s, 2H), 3.89 (s, 6H), 3.77 (s, 3H), 3.44-3.39 (m, 1H), 3.11-3.06 (m, 2H), 2.31 (s, 3H), 2.20-2.13 (m, 2H), 2.08-2.03 (m, 2H), 1.98-1.85 (m, 6H).

Step 5

3-[(2,6-difluoro-3,5-dimethoxy-phenyl)methoxy]-5-[3-(1-methylpyrrolidin-2-yl)propylcarbamoylamino]isothiazole-4-carboxamide

3-[(2,6-difluoro-3,5-dimethoxy-phenyl)methoxy]-5-[3-(1-methylpyrrolidin-2-yl)propylcarbamoylamino]isothiazol-4-methyl formate I523 (100 mg, 189.19 μmol) was dissolved in $NH_3$/MeOH (8 mL, 10 mol/L). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3,5-dimethoxy-phenyl)methoxy]-5-[3-(1-methylpyrrolidin-2-yl)propylcarbamoylamino]isothiazol-4-formamide T138 (14.06 mg, 27.38 μmol, yield: 14.47%). The product was verified by LCMS, H-NMR, C-NMR and F-NMR.

$^1H$ NMR (400MHz, $CD_3OD$): δ (ppm) 6.93 (t, J = 8.4 Hz, 1H), 5.54 (s, 2H), 3.88 (s, 6H), 3.24 (t, J = 6.8 Hz, 2H), 3.09-3.03 (m, 1H), 2.34 (s, 3H), 2.29-2.21 (m, 1H), 2.18-2.12 (m, 1H), 2.07-2.02 (m, 1H), 1.79-1.72 (m, 3H), 1.61-1.42 (m, 3H), 1.30-1.24 (m, 1H).

$^{13}C$ NMR (100 MHz, $CD_3OD$): δ (ppm) 170.34, 167.12, 162.89, 156.13, 147.04, 146.98, 145.25, 145.21, 145.14, 145.10, 144.62, 144.56, 114.46, 114.29, 114.12, 102.82, 98.62, 67.57, 59.67, 59.63, 59.58, 57.99, 57.62, 41.29, 40.58, 31.58, 31.57, 22.45.

$^{19}F$ NMR (400MHz, $CD_3OD$): δ (ppm) -148.66.

Example 115: Synthesis of Compound T139

Synthesis Route:

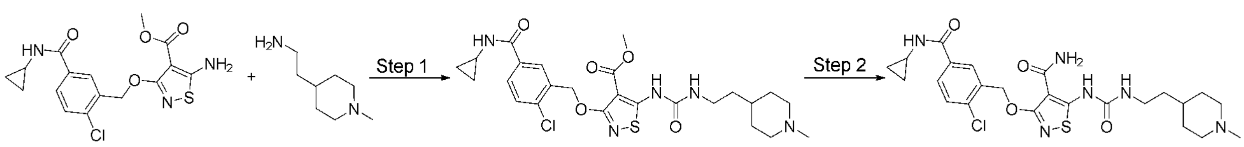


I478 I524 T139

Step 1

methyl 3-[[2-chloro-5-(cyclopropylcarbamoyl)phenyl]methoxy]-5-[2-(1-methyl-4-piperidyl)ethylcarbamoylamino]isothiazole-4-carboxylate

2-(1-methyl-4-piperidyl)ethylamine (104.3 mg, 733.30 μmol) was dissolved in anhydrous THF (5 mL), followed by the addition of CDI (118.9 mg, 733.30 μmol) under the protection of $N_2$, the mixture was then stirred at 25°C to react for

1.0 hour, followed by the addition of DMSO (5 mL), the pressure was reduced to remove THF, followed by the addition of methyl 5-amino-3-[[2-chloro-5-(cyclopropylcarbamoyl)phenyl]methoxy]isothiazol-4-carboxylate I478 (200 mg, 523.79 μmol) and potassium carbonate (217.2 mg, 1.57 mmol), and the reaction system was stirred at 25 °C to react for 1.0 hour. Water (30 mL) was poured into the reaction mixture, which was then subjected to liquid separation and extraction with ethyl acetate (10 mL x 3), the organic phases were combined, washed with saturated brine (30 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl3-[[2-chloro-5-(cyclopropylcarbamoyl)phenyl]methoxy]-5-[2-(1-methyl-4-piperidyl)ethylcarbamoylamino]isothiazol-4-methyl formate I524 (250 mg, 454.49 μmol, yield: 86.77%).

The product was verified by LCMS and H-NMR.

$^1$H NMR (400MHz, $CDCl_3$): δ (ppm) 10.48 (s, 1H), 8.28 (s, 1H), 7.80-7.69 (m, 3H), 5.48 (s, 2H), 3.89 (s, 3H), 3.27 (s, 2H), 2.91 (s, 1H), 2.25 (s, 2H), 1.95-1.85 (m, 2H), 1.69 (s, 2H), 1.35-1.15 (m, 5H), 0.82-0.81 (m, 2H), 0.65 (s, 2H).

Step 2

3-[[2-chloro-5-(cyclopropylcarbamoyl)phenyl]methoxy]-5-[2-(1-methyl-4-piperidyl)ethylcarbamoylamino]isothiazole-4-carboxamide

3-[[2-chloro-5-(cyclopropylcarbamoyl)phenyl]methoxy]-5-[2-(1-methyl-4-piperidyl)ethylcarbamoylamino]isothiazol-4-methyl formate I524 (250.0 mg, 454.49 μmol) was dissolved in $NH_3$/MeOH (5 mL). The mixture was sealed to react at 40 °C for 16 hours. The reaction mixture was concentrated under reduced pressure to obtain a crude product, and the crude product was purified by the high-performance liquid chromatography to obtain a white solid product 3-[[2-chloro-5-(cyclopropylcarbamoyl)phenyl]methoxy]-5-[2-(1-methyl-4-piperidyl)ethylcarbamoylamino]isothiazol-4-formamide T139 (32.0 mg, 66.2 μmol, yield: 13.20%).

The product was verified by LCMS, H-NMR and C-NMR.

$^1$H NMR (400MHz, $CD_3OD$): δ (ppm) 7.99 (d, J=2.0 Hz, 1H), 7.79-7.75 (m, 1H), 7.55 (d, J=8.4 Hz, 1H), 5.60 (s, 2H), 3.28-3.24 (m, 2H), 2.89-2.80 (m, 3H), 2.27 (s, 3H), 2.10-2.01 (m, 2H), 1.76 (d, J=13.0 Hz, 2H), 1.51-1.46 (m, 2H), 1.37-1.25 (m, 3H), 0.62-0.77 (m, 2H), 0.65-0.61 (m, 2H). $^{13}$C NMR (100MHz, $CD_3OD$): δ (ppm) 170.42, 170.26, 167.17, 162.93, 156.09, 138.32, 135.52, 134.68, 030.93, 130.73, 129.86, 98.71, 68.53, 56.63, 46.34, 38.67, 37.22, 33.59, 32.72, 24.09, 6.55.

Example 116: Synthesis of Compound T140

Synthesis Route:

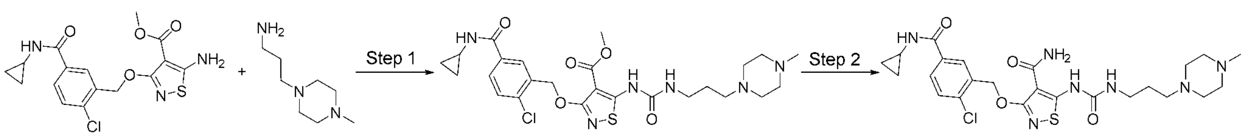


I478 I525 T140

Step 1

Methyl 3-[[2-chloro-5-(cyclopropylcarbamoyl)phenyl]methoxy]-5-[3-(4-methylpiperazin-1-yl)propylcarbamoylamino]isothiazole-4-carboxylate

3-(4-methylpiperazin-1-yl)propan-1-amine (115.3 mg, 733.30 μmol) was dissolved in anhydrous THF (5 mL), followed by the addition of CDI (118.9 mg, 733.30 μmol) under the protection of $N_2$, the mixture was then stirred at 25°C to react for 1.0 hour, followed by the addition of DMSO (5 mL), the pressure was reduced to remove THF, followed by the addition of methyl 5-amino-3-[[2-chloro-5-(cyclopropylcarbamoyl)phenyl]methoxy]isothiazol-4-carboxylate I478 (200 mg, 523.79 μmol) and potassium carbonate (217.2 mg, 1.57 mmol), and the reaction system was stirred at 25 °C to react for 1.0 hour. Water (60 mL) was poured into the reaction mixture, which was then subjected to liquid separation and extraction with ethyl acetate (20 mL x 3), the organic phases were combined, washed with saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl

3-[[2-chloro-5-(cyclopropylcarbamoyl)phenyl]methoxy]-5-[3-(4-methylpiperazin-1-yl)propylcarbamoylamino]isothiazol-4-carboxylate I525 (250 mg, 442.41 μmol, yield: 84.46%). The product was verified by LCMS and H-NMR.

$^1$H NMR (400 MHz, $CDCl_3$) δ (ppm) 10.41 (s, 1H), 7.85 (d, J = 39.4 Hz, 2H), 7.58 (d, J = 42.8 Hz, 1H), 7.46 (s, 1H), 5.40 (s, 2H), 3.91 (s, 3H), 3.05 (d, J = 136.6 Hz, 6H), 2.55 (d, J = 4.4 Hz, 4H), 2.35 (s, 3H), 2.21 (s, 2H), 1.65 (s, 2H), 0.73 (s, 2H), 0.58 (s, 2H).

Step 2

3-[[2-chloro-5-(cyclopropylcarbamoyl)phenyl]methoxy]-5-[3-(4-methylpiperazin-1-yl)propylcarbamoylamino]isothiazole-4-carboxamide

Methyl 3-[[2-chloro-5-(cyclopropylcarbamoyl)phenyl]methoxy]-5-[3-(4-methylpiperazin-1-yl)propylcarbamoylamino]isothiazol-4-carboxylate I525 (250 mg, 442.41 μmol) was dissolved in $NH_3$/MeOH (5 mL). The mixture was sealed to react at 40 °C for 16 hours. The reaction mixture was concentrated under reduced pressure to obtain a crude product, and the crude product was purified by the preparative thin-layer chromatography (DCM/MeOH = 10/1) to obtain a faint yellow solid product 3-[[2-chloro-5-(cyclopropylcarbamoyl)phenyl]methoxy]-5-[3-(4-methylpiperazin-1-yl)propylcarbamoylamino]isothiazol-4-formamide T140 (37.7 mg, 68.59 μmol, yield: 15.50%).

The product was verified by LCMS, H-NMR and C-NMR.

$^1$H NMR (400 MHz, DMSO_d6) δ (ppm) 8.00 (d, J = 2.0 Hz, 1H), 7.77 (d, J = 8.4 Hz, 1H), 7.53 (dd, J = 8.2, 3.6 Hz, 1H), 5.59 (s, 2H), 3.26 (m, J = 6.6 Hz, 2H), 2.86-2.81 (m, 1H), 2.81-2.33 (m, 10H), 2.30 (s, 3H), 1.79-1.70 (m, 2H), 0.83-0.76 (m, 2H), 0.63 (dd, J = 7.4, 4.0 Hz, 2H).

$^{13}$C NMR (100 MHz, $CD_3OD$): δ (ppm) 170.35, 170.21, 167.13, 162.92, 156.08, 138.31, 135.49, 134.64, 130.93, 130.73, 129.87, 98.74, 68.54, 56.77, 55.56, 53.54, 45.85, 39.49, 30.81, 27.65, 24.12, 23.76, 6.58.

Example 117: Synthesis of Compound T141

Synthesis Route:

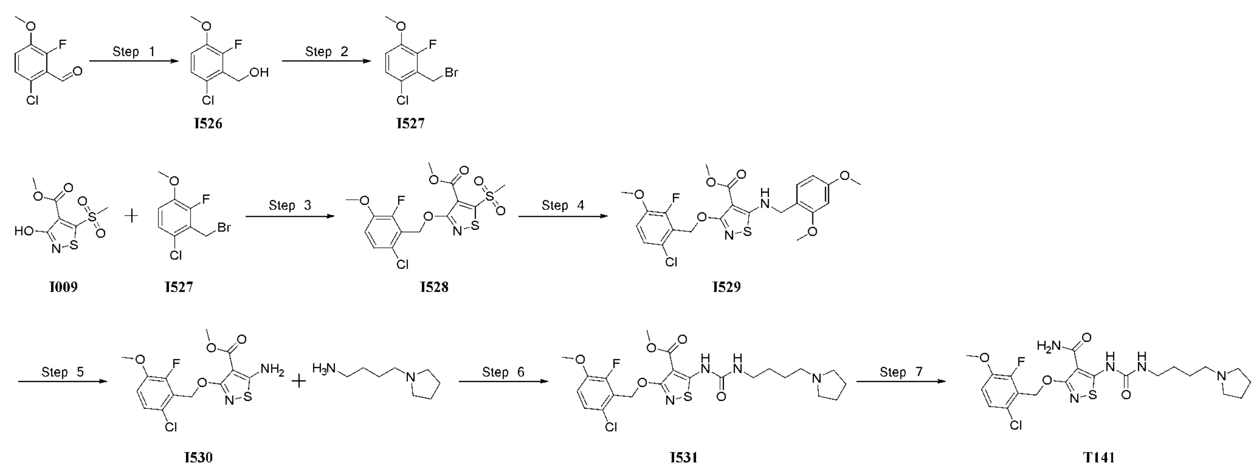


I526 I527 I009 I527 I528 I529 I530 I531 T141

Step 1

(6-chloro-2-fluoro-3-methoxy-phenyl)methanol

At room temperature, 6-chloro-2-fluoro-3-methoxybenzaldehyde (4.7 g, 24.92 mmol) was dissolved in ethanol (50 mL), followed by the addition of sodium borohydride (1.9 g, 49.85 mmol). The reaction system was stirred at 25°C to react for 1 h. The reaction mixture was poured into ammonium chloride aqueous solution (50 mL) and extracted with ethyl acetate (100 mL x 3), and the organic phases were combined, washed with saturated brine (50 mL x 3), dried with anhydrous sodium sulfate, and concentrated under reduced pressure to obtain a colorless oily liquid product (6-chloro-2-fluoro-3-methoxy)benzyl alcohol I526 (4.1 g, 21.51 mmol, yield: 86.31%).

The product was verified by LCMS and H-NMR.

For MS-ESI, the calculated value was [M+H]+ 191.0, and the measurement was 173.0 [M-OH]. $^1$H NMR

(400MHz, $CDCl_3$): δ (ppm) 7.13 (dd, J = 8.8, 2.0 Hz, 1H), 6.87 (t, J = 8.8 Hz, 1H), 4.85 (d, J = 2.4 Hz, 2H), 3.88 (s, 3H).

Step 2

2-(bromomethyl)-1-chloro-3-fluoro-4-methoxy-benzene

At room temperature, (6-chloro-2-fluoro-3-methoxy)benzyl alcohol I526 (4.1 g, 21.51 mmol) was dissolved in dichloromethane (50 mL), followed by the addition of phosphorus tribromide (8.7 g, 32.27 mmol). The reaction system was stirred at 25°C to react for 1 h. The reaction mixture was poured into water (50 mL), and extracted with dichloromethane (100 mL x 3), and the organic phases were combined, washed with saturated brine (50 mL), dried with anhydrous sodium sulfate, and concentrated under reduced pressure to obtain a yellow solid product (6-chloro-2-fluoro-3-methoxy)benzyl bromide I527 (4.5 g, 17.75 mmol, yield: 82.52%).

The product was verified by H-NMR.

$^1$H NMR (400MHz, $CDCl_3$): δ (ppm) 7.14 (dd, J = 8.8, 1.6 Hz, 1H), 6.89 (t, J = 8.8 Hz, 1H), 4.61 (d, J = 2.0 Hz, 2H), 3.88 (s, 3H).

Step 3

Methyl 3-[(6-chloro-2-fluoro-3-methoxy-phenyl)methoxy]-5-methylsulfonyl-isothiazole-4-carboxylate

At room temperature, methyl 3-hydroxyl-5-methylsulfonylisothiazol-4-carboxylate I009 (3.8 g, 16.14 mmol) was dissolved in N,N-dimethylformamide (50 mL), followed by the addition of (6-chloro-2-fluoro-3-methoxy)benzyl bromide I527 (4.5 g, 17.75 mmol) and potassium carbonate (6.7 g, 48.41 mmol). The reaction system was stirred at 25°C to react for 2 h. The reaction mixture was poured into water (100 mL), stirred and filtered, and the filter cake was dissolved in ethyl acetate (100 mL), dried with anhydrous sodium sulfate, and concentrated under reduced pressure to obtain a yellow solid product methyl 3-[(6-chloro-2-fluoro-3-methoxyphenyl)methoxy]-5-methylsulfonylisothiazol-4-carboxylate I528 (6 g, 14.64 mmol, yield: 90.72%).

The product was verified by LCMS and H-NMR.

For MS-ESI, the calculated value was [M+H]+ 409.8, and the measurement was 409.9.

$^1$H NMR (400MHz, $CDCl_3$): δ (ppm) 7.18 (dd, J = 8.8, 2.0 Hz, 1H), 6.95 (t, J = 8.8 Hz, 1H), 5.61 (d, J = 2.0 Hz, 2H), 3.90 (s, 3H), 3.88 (s, 3H), 3.48 (s, 3H).

Step 4

Methyl 3-[(6-chloro-2-fluoro-3-methoxy-phenyl)methoxy]-5-[(2,4-dimethoxyphenyl)methylamino]isothiazole-4-carboxylate

At room temperature, methyl 3-[(6-chloro-2-fluoro-3-methoxyphenyl)methoxy]-5-methylsulfonylisothiazol-4-carboxylate I528 (6 g, 14.64 mmol) was dissolved in tetrahydrofuran (60 mL), followed by the addition of 2,4-dimethoxybenzylamine (24.5 g, 146.40 mmol). The reaction system was stirred at 65°C to react for 2 h. The reaction mixture was poured into water (100 mL), stirred and filtered, and the filter cake was dissolved in ethyl acetate (100 mL), dried with anhydrous sodium sulfate, and concentrated under reduced pressure to obtain a yellow solid product methyl 3-[(6-chloro-2-fluoro-3-methoxyphenyl)methoxy]-5-[(2,4-dimetoxybenzene)methylamino]isothiazol-4-carboxylate I529 (5.4 g, 10.87 mmol, yield: 74.23%). The product was verified by LCMS and H-NMR.

For MS-ESI, the calculated value was [M+H]+ 496.9, and the measurement was 496.9.

$^1$H NMR (400MHz, $CDCl_3$): δ (ppm) 8.10 (t, J = 5.2 Hz, 1H), 7.17-7.13 (m, 2H), 6.91 (t, J = 8.8 Hz, 1H), 6.47-6.41 (m, 2H), 5.51 (d, J = 2.0 Hz, 2H), 4.27 (d, J = 6.0 Hz, 2H), 3.88 (s, 3H), 3.84 (s, 3H), 3.80 (s, 3H), 3.71 (s, 3H).

Step 5

Methyl 5-amino-3-[(6-chloro-2-fluoro-3-methoxy-phenyl)methoxy]isothiazole-4-carboxylate

At room temperature, methyl 3-[(6-chloro-2-fluoro-3-methoxyphenyl)methoxy]-5-[(2,4-dimetoxybenzene) methylamino]isothiazol-4-carboxylate I529 (2 g, 4.02 mmol) was dissolved in dichloromethane (20 mL) and water (4 mL) followed by the addition of 2,3-dichloro-5,6-dicyanobenzoquinone (913.6 mg, 4.02 mmol). The reaction system was stirred at 25°C to react for 1 hour. The reaction mixture was poured into water (50 mL) and extracted with dichloromethane (50 mL x 3), and the organic phases were combined, washed with saturated brine (50 mL), dried with anhydrous sodium sulfate, and concentrated under reduced pressure to obtain a crude product. The crude product was purified by

chromatographic columns (PE/EA = 10/1-5/1) to obtain a yellow solid product methyl 5-amino-3-[(6-chloro-2-fluoro-3-methoxyphenyl)methoxy]isothiazol-4-carboxylate I530 (1 g, 2.88 mmol, yield: 71.65%).

The product was verified by LCMS and H-NMR.

For MS-ESI, the calculated value was [M+H]+ 346.8, and the measurement was 346.9.

$^1$H NMR (400MHz $CD_3Cl$): δ (ppm) 7.17 (dd, J = 8.8, 2.0 Hz, 1H), 6.92 (t, J = 8.8 Hz, 1H), 6.42 (s, 2H), 5.53 (d, J = 2.0 Hz, 2H), 3.89 (s, 3H), 3.75 (s, 3H).

Step 6

Methyl 3-[(6-chloro-2-fluoro-3-methoxy-phenyl)methoxy]-5-(4-pyrrolidin-1-ylbutylcarbamoylamino)isothiazole-4-carboxylate

4-Pyrrolidin-1-ylbutyl-1-amine (205.1 mg, 1.44 mmol) was dissolved in tetrahydrofuran (5 mL), followed by the addition of N,N'-carbonyl diimidazole (207.6 mg, 1.44 mmol) under the protection of $N_2$, and the mixture was then stirred at 25°C to react for 1 hour. Dimethyl sulfoxide (5 mL) was added, the pressure was reduced to remove tetrahydrofuran, followed by the addition of methyl 5-amino-3-[(6-chloro-2-fluoro-3-methoxyphenyl)methoxy]isothiazol-4-carboxylate I530 (500 mg, 1.44 mmol) and potassium carbonate (199.3 mg, 1.44 mmol) , and the reaction system was stirred at 25°C to react for 1 hour. Water (60 mL) was added to the reaction mixture, which was then subjected to liquid separation and extraction with ethyl acetate (20 mLx3). The organic phases were combined, washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a yellow solid product methyl 3-[(6-chloro-2-fluoro-3-methoxyphenyl)methoxy]-5-(4-pyrrolidin-1-ylbutylamino)isothiazol-4-carboxylate I531 (400 mg, 776.70 μmol, yield: 53.87%).

The product was verified by LCMS and H-NMR.

For MS-ESI, the calculated value was [M+H]+ 515.0, and the measurement was 515.1.

$^1$H NMR (400MHz $CD_3OD$): δ (ppm) 10.21 (s, 1H), 8.05 (s, 1H), 7.16-7.14 (m, 1H), 6.91 (t, J = 8.8 Hz, 1H), 5.54 (d, J = 2.0 Hz, 2H), 3.89 (s, 3H), 3.77 (s, 3H), 3.32 (s, 2H),2.71 (s, 4H), 2.62 (s, 2H), 1.92 (s, 4H), 1.71 (s, 4H).

Step 7

3-[(6-chloro-2-fluoro-3-methoxy-phenyl)methoxy]-5-(4-pyrrolidin-1-ylbutylcarbamoylamino)isothiazole-4-carboxamide

Methyl 3-[(6-chloro-2-fluoro-3-methoxyphenyl)methoxy]-5-(4-pyrrolidin-1-ylbutylamino)isothiazol-4-carboxylate I531 (500 mg, 970.88 μmol) was dissolved in $NH_3$/MeOH (5 mL), and the mixture was stirred at 40°C to react for 16 hours. The reaction mixture was concentrated under reduced pressure to obtain a crude product, and the crude product was purified by the preparative thin-layer chromatography (DCM/MeOH = 10/1) to obtain a product 3-[(6-chloro-2-fluoro-3-methoxyphenyl)methoxy]-5-(4-pyrrolidin-1-ylbutylamino)isothiazol-4-formamide T141 (51.71 mg, 103.42 μmol, yield: 10.65%).

The product was verified by LCMS, H-NMR, C-NMR and F-NMR.

For MS-ESI, the calculated value was [M+H]+ 500.0, and the measurement was 500.1.

$^1$H NMR (400MHz $CD_3OD$): δ (ppm) 7.24 (d, J = 8.8 Hz, 1H), 7.14 (t, J = 8.8 Hz, 1H), 5.59 (s, 2H), 3.88 (s, 3H), 3.25 (t, J = 6.4 Hz, 2H), 2.62 (s, 4H), 2.56 (t, J = 6.8 Hz, 2H), 1.82 (s, 4H), 1.59 (s, 4H).

$^{13}$C NMR (100 MHz, $CD_3OD$) δ (ppm) 170.33, 167.09, 162.94, 156.14, 154.18, 151.68, 148.54, 148.43, 127.20, 126.16, 126.11, 123.30, 123.16m 115.98, 98.59, 62.43, 62.38, 57.04, 57.01, 55.00, 40.87, 28.80, 26.66, 24.14.

$^{19}$F NMR (400MHz $CD_3OD$): δ (ppm) -136.08

Example 118: Synthesis of Compound T142

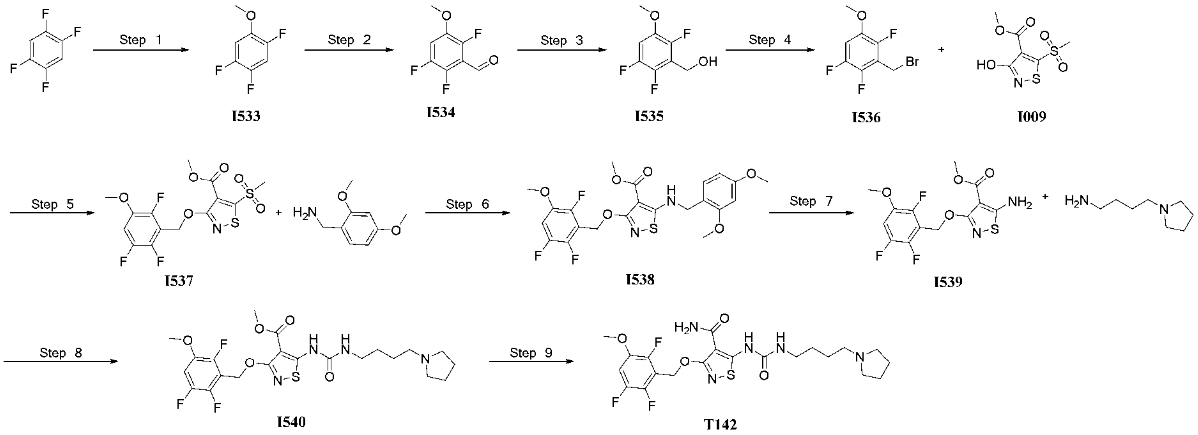


Step 1

1,2,4-trifluoro-5-methoxy-benzene

At room temperature, 1,2,4,5-tetrafluorobenzene (20 g, 133.27 mmol) and sodium methoxide (7.20 g, 133.27 mmol) were added to a 500 mL single-neck flask. The reaction system was stirred at 60 °C to react for 2 hours. The reaction mixture poured into dichloromethane (100 ml), and filtered under reduced pressure, and the filtrate was concentrated under reduced pressure to obtain a crude product 1,2,4-trifluoro-5-metoxybenzeneI533 (11 g, 67.86 mmol, yield: 50.92%).
The product was verified by H-NMR.
1H NMR (400MHz, $CDCl_3$): δ (ppm) 7.11-7.03 (m, s1H), 7.01-6.94 (m, 1H), 3.86 (s, 3H).

Step 2

2,3,6-trifluoro-5-methoxy-benzaldehyde

At room temperature, 1,2,4-trifluoro-5-metoxybenzeneI533 (10 g, 61.69 mmol) was dissolved in ultradry tetrahydrofuran (100 mL). N-butyllithium (24.7 mL, 61.69 mmol, 2.5 mol/L) was dropwise added to the reaction system at -78°C under the protection of nitrogen, and the reaction system ws then stirred to react for 1 hour. N,Ndimethylformamide (4.5 g, 61.69 mmol) was dropwise added at -78 °C. The reaction system was stirred at -78 °C for 1 hour. The reaction mixture was poured into saturated ammonium chloride (50 mL) and extracted with ethyl acetate (50 mL x 3), and the organic phases were combined, washed with saturated brine (50 mL), dried with anhydrous sodium sulfate, and concentrated under reduced pressure to obtain a crude product. The crude product was purified by chromatographic columns (PE/EA = 1/0-1/1) to obtain a yellow solid product 2,3,6-trifluoro-5-methoxybenzaldehydeI534 (10 g, 52.60 mmol, yield: 85.27%)
The product was verified by H-NMR.
$^1$H NMR (400 MHz, $CDCl_3$) δ (ppm) 10.33 (s, 1H), 6.95-6.79 (m, 1H), 3.82 (s, 3H).

Step 3

(2,3,6-trifluoro-5-methoxy-phenyl)methanol

At room temperature, 2,3,6-trifluoro-5-methoxybenzaldehydeI534 (10 g, 52.60 mmol) was dissolved in methanol (100 mL), followed by the addition of sodium borohydride (2.4 g, 63.11 mmol) at 0°C. The reaction system was stirred at 25°C to react for 1 hour. The reaction mixture was poured into 1N hydrochloric acid (100 mL) and extracted with ethyl acetate (50 mL x 3), and the organic phases were combined, washed with saturated brine (50 mL), dried with anhydrous sodium sulfate, and concentrated under reduced pressure to obtain a crude product. The crude product was purified by chromatographic columns (PE/EA = 1/0-3/1) to obtain a yellow solid product (2,3,6-trifluoro-5-methoxy-phenyl)methanol I535 (3.3 g, 17.18 mmol, yield: 32.65%)
The product was verified by H-NMR.
$^1$H NMR (400MHz, $CDCl_3$): δ (ppm) 6.82-6.75 (m, 1H), 4.81 (s, 2H), 3.85 (s, 3H).

Step 4

3-(bromomethyl)-1,2,4-trifluoro-5-methoxy-benzene

At room temperature, (2,3,6-trifluoro-5-methoxy-phenyl)methanol I535 (3.4 g, 17.70 mmol) was dissolved in dichloromethane (50 mL), followed by the addition of phosphorus tribromide (5.8 g, 21.24 mmol). The reaction system was stirred at 25°C to react for 1 hour. The reaction mixture was poured into water (30 mL), and extracted with dichloromethane (50 mL x 3), and the organic phases were combined, washed with saturated brine (30 mL), dried with anhydrous sodium sulfate, and concentrated under reduced pressure to obtain a crude product 3-(bromomethyl)-1,2,4-trifluoro-5-metoxybenzeneI536 (3.5 g, 13.72 mmol, yield: 77.55%) as a white solid.

The product was verified by H-NMR.

$^1$H NMR (400MHz, DMSO-$d_6$): δ (ppm) 7.56-7.44 (m, 1H), 4.68 (s, 2H), 3.85 (s, 3H).

Step 5

Methyl 5-methylsulfonyl-3-[(2,3,6-trifluoro-5-methoxy-phenyl)methoxy]isothiazole-4-carboxylate

At room temperature, methyl 3-hydroxyl-5-methylsulfonylisothiazol-4-carboxylate (3.8 g, 16.02 mmol) was dissolved in N,N-dimethylformamide (40 mL), followed by the addition of 3-(bromomethyl)-1,2,4-trifluoro-5-metoxybenzeneI536 (4.9 g, 19.22 mmol) and potassium carbonate (6.6 g, 48.05 mmol). The reaction system was stirred at 25°C to react for 1 hour. The reaction mixture was poured into water (200 mL), stirred and filtered, and the filter cake was dissolved in ethyl acetate (100 mL), dried with anhydrous sodium sulfate, and concentrated under reduced pressure to obtain a white solid methyl 5-methylsulfonyl-3-[(2,3,6-trifluoro-5-methoxyphenyl)methoxy]isothiazol-4-carboxylate I537 (3.4 g, 8.27 mmol, yield: 51.60%).

The product was verified by LCMS and H-NMR.

For MS-ESI, the calculated value was $[M+H]^+$ 412.0, and the measurement was 411.9.

$^1$H NMR (400MHz, DMSO_$d_6$): δ (ppm) 7.56-7.49 (m, 1H), 5.56 (s, 2H), 3.86 (s, 3H), 3.83 (s, 3H), 3.70 (s, 3H).

Step 6

Methyl 5-[(2,4-dimethoxyphenyl)methylamino]-3-[(2,3,6-trifluoro-5-methoxyphenyl)methoxy]isothiazole-4-carboxylate

Methyl 5-methylsulfonyl-3-[(2,3,6-trifluoro-5-methoxy-phenyl)methoxy]isothiazol-4-carboxylate I537 (3.8 g, 9.24 mmol) was dissolved in tetrahydrofuran (35 mL) , followed by the addition of 2,4-dimethoxybenzylamine (15.5 g, 92.37 mmol). The reaction system was stirred at 65°C to react for 1 hour. The reaction mixture was poured into ice water (50 mL), stirred and filtered, and the filter cake was dissolved in ethyl acetate (100 mL), dried with anhydrous sodium sulfate, and concentrated under reduced pressure to obtain a white solid methyl 5-[(2,4-dimethoxyphenyl)methylamino]-3-[(2,3,6-trifluoro-5-methoxy-phenyl)methoxy]isothiazol-4-carboxylate I538 (3.4 g, 6.82 mmol, yield: 73.84%).

The product was verified by LCMS and H-NMR.

For MS-ESI, the calculated value was $[M+H]^+$ 499.1, and the measurement was 500.0.

$^1$H NMR (400MHz, DMSO_$d_6$): δ (ppm) 8.41 (s, 1H), 7.51-7.44 (m,1H), 7.15 (d, J = 8.4 Hz, 1H), 6.58 (d, J = 2.4 Hz, 1H), 6.52-6.46 (m,1H), 5.36 (s, 2H), 4.27 (d, J = 4.2 Hz, 2H), 3.85 (s, 3H), 3.83 (s, 3H), 3.75 (s, 3H), 3.66 (s, 3H).

Step 7

Methyl 5-amino-3-[(2,3,6-trifluoro-5-methoxy-phenyl)methoxy]isothiazole-4-carboxylate

Methyl 5-[(2,4-dimethoxyphenyl)methylamino]-3-[(2,3,6-trifluoro-5-methoxyphenyl)methoxy]isothiazol-4-carboxylate I538 (3 g, 6.02 mmol) was dissolved in dichloromethane (60 mL) and water (12 mL), followed by the addition of 2,3-dichloro-5,6-dicyanobenzoquinone (2.7 g, 12.04 mmol at room temperature). The reaction system was stirred at 25°C to react for 1 h. The reaction mixture was poured into water (100 mL) and extracted wiith dichloromethane (50mL x 3), and the organic phases were combined, washed with saturated brine (100 mL), dried with anhydrous sodium sulfate, and concentrated under reduced pressure to obtain a crude product. The crude product was purified by chromatographic columns (PE/EA = 10/1-3/1) to obtain a yellow solid product methyl 5-amino-3-[(2,3,6-trifluoro-5-methoxy-phenyl) methoxy]isothiazol-4-carboxylate I539 (1.8 g, 5.17 mmol, yield: 85.87%).

The product was verified by LCMS and H-NMR.

For MS-ESI, the calculated value was $[M+H]^+$ 349.0, and the measurement was 349.0.

$^1$H NMR (400MHz, $CDCl_3$): δ (ppm) 6.87-6.81 (m, 1H), 6.42 (s, 2H), 5.50 (s, 2H), 3.87 (s, 3H), 3.77 (s, 3H).

Step 8

Methyl 5-(4-pyrrolidin-1-ylbutylcarbamoylamino)-3-[(2,3,6-trifluoro-5-methoxyphenyl)methoxy]isothiazole-4-carboxylate

4-Pyrrolidin-1-ylbutyl-1-amine (171.5 mg, 1.21 mmol) was dissolved in tetrahydrofuran (10 mL), followed by the addition of carbonyl diimidazole (173.6 mg, 1.21 mmol) under the protection of nitrogen, and the mixture was then stirred at 25 °C to react for 1 hour. Dimethyl sulfoxide (10 mL) was added, rotary evaporaton was carried out under reduced pressure to remove tetrahydrofuran, followed by the addition of methyl 5-amino-3-[(2,3,6-trifluoro-5-methoxyphenyl)methoxy] isothiazol-4-carboxylate I539 (300 mg, 861.33 μmol) and potassium carbonate (357.1 mg, 2.58 mmol), and the reaction system was stirred at 25°C to react for 1 hour. Water (30 mL) was added to the reaction mixture, which was then subjected to liquid separation and extraction with ethyl acetate (20 mLx3). The organic phases were combined, washed with saturated brine (50 mL), dried with anhydrous sodium sulfate, and concentrated under reduced pressure to obtain a crude product. The crude product was purified by the preparative thin-layer chromatography (DCM/MeOH = 10/1) to obtain a yellow solid product methyl 5-(4-pyrrolidin-1-ylbutylcarbamylamino)-3-[(2,3,6-trifluoro-5-methoxy-phenyl)methoxy]isothiazol-4-carboxylate I540 (250 mg, 484.00 μmol, yield: 56.19%)

The product was verified by LCMS and H-NMR.

For MS-ESI, the calculated value was $[M+H]^+$ 517.2, and the measurement was 517.1.

$^1H$ NMR (400MHz, $CDCl_3$): δ (ppm) 6.91-6.89 (m, 1H), 5.47 (s, 1H), 3.88 (m, 3H), 3.78 (s, 3H), 3.25 (s, 2H), 2.59-2.53 (m, 6H), 1.80 (s, 4H), 1.59 (s, 4H).

Step 9

5-(4-pyrrolidin-1-ylbutylcarbamoylamino)-3-[(2,3,6-trifluoro-5-methoxyphenyl)methoxy]isothiazole-4-carboxamide

At room temperature, methyl 5-(4-pyrrolidin-1-ylbutylcarbamylamino)-3-[(2,3,6-trifluoro-5-methoxy-phenyl) methoxy]isothiazol-4-carboxylate I540 (250 mg, 484.00 μmol) was dissolved in the solution of ammonia gas in methanol (5 mL, 10 mol/L). The reaction system was stirred at 40°C to react for 16 h. The reaction mixture was concentrated under reduced pressure to obtain a crude product. The crude product was purified by the preparative thin-layer chromatography (DCM/MeOH = 10/1) to obtain a white solid product 5-(4-pyrrolidin-1-ylbutylcarbamylamino)-3-[(2,3,6-trifluoro-5-methoxy-phenyl)methoxy]isothiazol-4-formamide T142 (32.46 mg, 64.72 μmol, yield: 13.37%)

The product was verified by LCMS, H-NMR, C-NMR and F-NMR.

For MS-ESI, the calculated value was $[M+H]^+$ 502.2, and the measurement was 502.0.

$^1H$ NMR (400MHz, $CDCl_3$): δ (ppm) 10.93 (s, 1H), 7.79 (s, 1H), 7.00 (s, 1H), 6.90-6.83 (m, 1H), 5.60 (s, 2H), 5.47 (s, 1H), 3.87 (s, 3H), 3.31 (s, 2H), 2.69 (s, 4H), 2.61 (s, 2H), 1.91 (s, 4H), 1.69 (s, 4H).

$^{13}C$ NMR (100 MHz, $CDCl_3$) δ (ppm) 169.75, 165.72, 161.12, 153.97, 147.86, 147.36, 145.38, 143.96, 114.26, 114.09, 113.93, 103.18, 102.96, 97.15, 58.22, 56.91, 55.89, 53.89, 40.59, 29.70, 28.20, 26.83, 23.39.

$^{19}F$ NMR (400 MHz, $CD_3OD$): δ (ppm) -142.73, -142.77, -143.32, -143.36, -143.38, -143.42, - 152.66, -152.72.

Example 119: Synthesis of Compound T145

Synthesis Route:

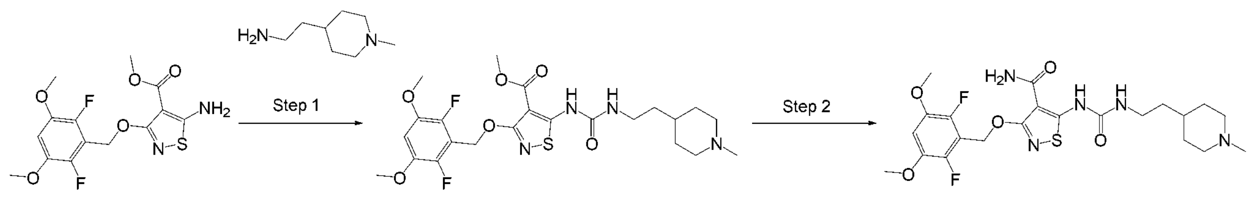


I468 I532 T145

Step 1

Methyl 3-[(2,6-difluoro-3,5-dimethoxy-phenyl)methoxy]-5-[2-(1-methyl-4-piperidyl)ethylcarbamoylamino]isothiazole-4-carboxylate

2-(1-methyl-4-piperidyl)ethylamine (165.8 mg, 1.17 mmol) was dissolved in anhydrous THF (6 mL) and cooled to

0°C under the protection of $N_2$, followed by the addition of CDI (407 mg, 2.5 mmol), the mixture was then stirred at room temperature to react for 1 hour, followed by the addition of DMSO(6 mL), the pressure was reduced to remove THF, followed by the addition of methyl 5-amino-3-[(2,6-difluoro-3,5-dimethoxy-phenyl)methoxy]isothiazol-4-carboxylate I468 (300.0 mg, 832.56 μmol) and potassium carbonate (345.2 mg, 2.50 mmol), and the reaction system was stirred at 25°C to react for 1 hour. After complete reaction, water (60 mL) was added to the reaction mixture, which was then extracted with ethyl acetate (20 mLx3), the organic phases were washed saturated brine (20 mL), dried with anhydrous sodium sulfate, and concentrated under reduced pressure to obtain a crude product, and the crude product was purified by the preparative thin-layer chromatography (DCM/MeOH = 10/1) to obtain a white solid product methyl 3-[(2,6-difluoro-3,5-dimethoxy-phenyl)methoxy]-5-[2-(1-methyl-4-piperidylethylcarbamoylamino] isothiazol-4-carboxylate I094 (300 mg, 567.57 μmol, yield: 68.17%).

The product was verified by LCMS and H-NMR.

1H NMR (400 MHz, $CDCl_3$) δ (ppm) 10.30 (s, 1H), 6.66 (t, J = 8.0 Hz, 1H), 5.59 (s, 1H), 5.49 (s, 2H), 3.88 (s, 6H), 3.77 (s, 3H), 3.37-3.31 (m, 2H), 2.83 (d, J = 10.8 Hz, 2H), 2.24 (s, 3H), 1.90-1.88 (m, 2H), 1.70-1.68 (m, 2H), 1.53-1.51 (m, 2H), 1.30-1.25 (m, 3H).

Step 2

3-[(2,6-difluoro-3,5-dimethoxy-phenyl)methoxy]-5-[2-(1-methyl-4-piperidyl)ethylcarbamoylamino]isothiazole-4-carboxamide

In a microwave tube, methyl 3-[(2,6-difluoro-3,5-dimethoxy-phenyl)methoxy]-5-[2-(1-methyl-4-piperidylethylcarbamoylamino] isothiazol-4-carboxylate I094 (300.0 mg, 567.57 μmol) was dissolved in $NH_3$/MeOH (8 mL, 9.5 mol/L), and the mixture was sealed to react at 40°C for 16 hours. The reaction mixture was concentrated under reduced pressure to obtain a crude product, and the crude product was purified by the preparative thin-layer chromatography (DCM/MeOH = 10/1) to obtain a white solid product 3-[(2,6-difluoro-3,5-dimethoxy-phenyl)methoxy]-5-[2-(1-methyl-4-piperidyl)ethylcarbamoylamino] isothiazol-4-formamide T145 (54.88 mg, 106.86 μmol, yield: 18.83%).

The product was verified by LCMS, H-NMR, C-NMR and F- NMR.

$^1$H NMR (400MHz, $CD_3OD$): δ (ppm) 6.93 (t, J = 8.2 Hz, 1H), 5.54 (s, 2H), 3.88 (s, 6H), 3.28-3.24 (m, 2H), 2.87 (d, J = 10.9 Hz, 2H), 2.26 (s, 3H), 2.03 (t, J = 11.6 Hz, 2H), 1.75 (d, J = 12.8 Hz, 2H), 1.49-1.48 (m, 2H), 1.38-1.22 (m, 3H).

$^{13}$C NMR (100MHz, $CDCl_3$): δ (ppm) 169.48,169.31, 153.95, 153.92, 153.87, 165.78, 161.36, 146.09, 146.04, 143.67, 143.36, 143.52, 113.37, 113.20, 113.03, 101.57, 58.66, 57.24, 55.72, 46.35,38.39, 38.35, 38.27, 38.21, 38.18, 38.15, 38.08, 38.05, 36.32, 32.65, 32.07.

$^{19}$F NMR (400MHz, $CD_3OD$): δ (ppm) -144.72.

Example 120: Synthesis of Compound T148

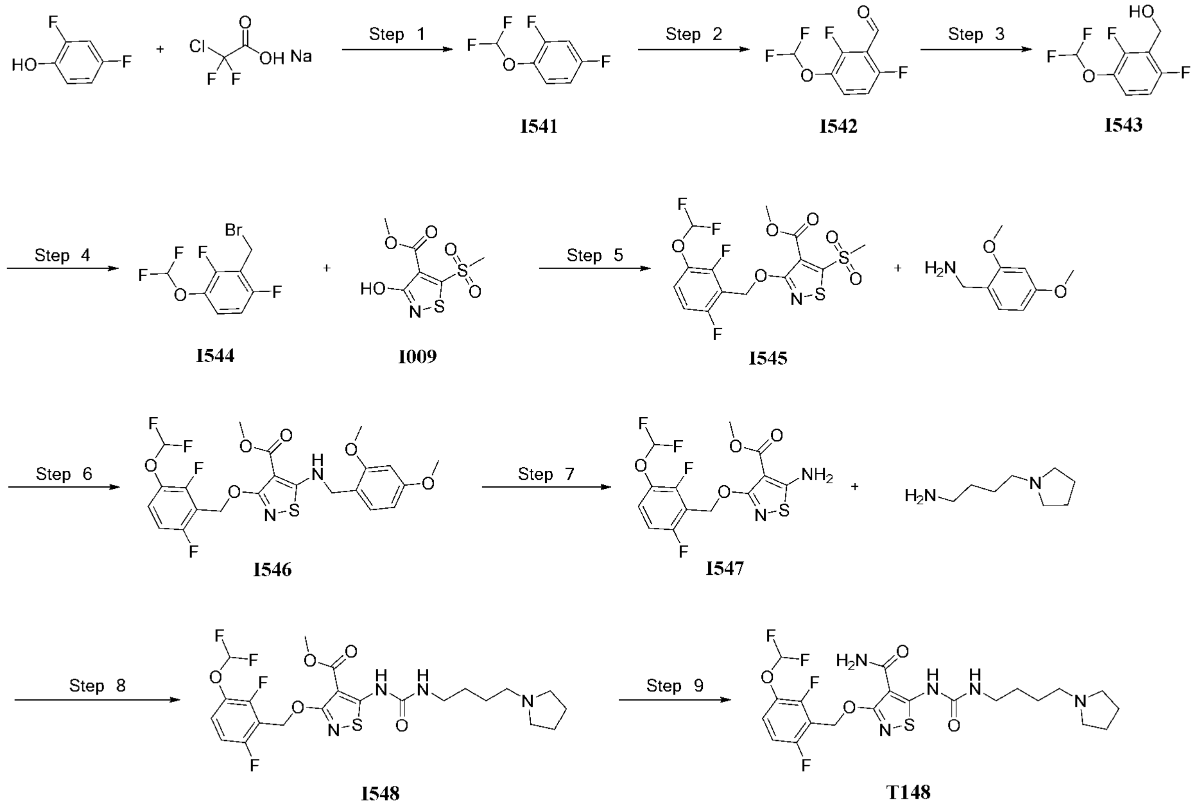


I541 I542 I543 I544 I009 I545 I546 I547 I548 T148

Step 1

1-(difluoromethoxy)-2,4-difluoro-benzene

At room temperature, 2,4-difluorophenol (13.5 g, 103.77 mmol) was dissolved in N,Ndimethylformamide (150 mL), followed by the addition of 2-chloro-2,2-sodium difluoroacetate (19 g, 124.53 mmol) and potassium carbonate (43 g, 311.32 mmol). The reaction system was stirred at 100°C to react for 2 h. The reaction mixture was poured into water (500 mL) and extracted with ethyl acetate (200 mL x 3), and the organic phases were combined, washed with saturated brine (500 mL), dried with anhydrous sodium sulfate, and concentrated under reduced pressure to obtain a brown oily crude product was 1-(difluoromethoxy)-2,4-difluorobenzeneI541 (9 g, 49.97 mmol, yield: 48.16%).

The product was verified by H-NMR.

$^1$H NMR (400MHz, $CDCl_3$): δ (ppm) 7.37-7.3 (m, 1H), 6.95-6.91(m, 2H), 6.69-6.32 (m,1H).

Step 2

3-(difluoromethoxy)-2,6-difluoro-benzaldehyde

1-(difluoromethoxy)-2,4-difluorobenzeneI541 (10 g, 55.52 mmol) was dissolved in tetrahydrofuran (100 mL), followed by the addition of n-butyllithium (2.5 M, 33.3 mL) under the protection of nitrogen, and the mixture was then stirred at -78 °C to react for 0.5 hours. N,N-dimethylformamide (8.1 g, 111.05 mmol) was added, and the reaction system was stirred at -78°C to react for 1.5 hours. Saturated ammonium chloride aqueous solution (500 mL) was added to the reaction mixture, which was then subjected to liquid separation and extraction with ethyl acetate (200 mLx3). The organic phases were combined, washed with saturated brine (500 mL), dried with anhydrous sodium sulfate, and concentrated under reduced pressure to obtain a crude product3-(difluoromethoxy)-2,6-difluorobenzaldehydeI542 (3 g, 14.42 mmol, yield: 25.96%). The product was verified by H-NMR.

$^1$H NMR (400 MHz, $CDCl_3$) δ (ppm) 10.34 (s, 1H), 7.50 (dd, J = 14.0, 8.8 Hz, 1H), 7.01 (t, J = 9.2 Hz, 1H), 6.56 (t, J = 72.4 Hz, 1H).

Step 3

[3-(difluoromethoxy)-2,6-difluoro-phenyl]methanol

At room temperature, 3-(difluoromethoxy)-2,6-difluorobenzaldehydeI542 (3 g, 14.42 mmol) was dissolved in methanol (30 mL), followed by the addition of sodium borohydride (654.4 mg, 17.30 mmol). The reaction system was stirred at 25°C to react for 1 h. The reaction mixture was poured into 1N dilute hydrochloric acid (20 mL) and extracted with ethyl acetate (15 mL x 3), and the organic phases were combined, washed with saturated brine (20 mL), dried with anhydrous sodium sulfate, and concentrated under reduced pressure to obtain a crude product. The crude product was purified by chromatographic columns (PE/EA = 1/0-3/1) to obtain a yellow oily product [3-(difluoromethoxy)-2,6-difluorophenyl]methanol I543 (2.5 g, 11.90 mmol, yield: 82.53%).
The product was verified by H-NMR.
$^1$H NMR (400MHz, $CDCl_3$): δ (ppm) 7.24-7.18 (m, 1H), 6.90 (t, J = 8.8 Hz, 1H), 6.51 (t, J = 73.2 Hz, 1H), 4.80 (s, 2H).

Step 4

3-(bromomethyl)-1-(difluoromethoxy)-2,4-difluoro-benzene

At room temperature, [3-(difluoromethoxy)-2,6-difluorophenyl]methanol I543 (2.5 g, 11.90 mmol) was dissolved in dichloromethane (30 mL), followed by the addition of phosphorus tribromide (4.8 g, 17.85 mmol). The reaction system was stirred at 25°C to react for 1 h. The reaction mixture was poured into water (50 mL), and extracted with dichloromethane (10 mL x 3), and the organic phases were combined, washed with saturated brine (30 mL), dried with anhydrous sodium sulfate, and concentrated under reduced pressure to obtain a yellow oily crude product 3-(bromomethyl)-1-(difluoromethoxy)-2,4-difluorobenzeneI544 (2.5 g, 9.16 mmol, yield: 76.96%).
The product was verified by H-NMR.
$^1$H NMR (400MHz, $CDCl_3$): δ (ppm) 7.31-7.19 (m, 1H), 6.95-6.88 (m, 1H), 6.52 (t, J = 72.8 Hz, 1H), 4.52 (s, 2H).

Step 5

methyl 3-[[3-(difluoromethoxy)-2,6-difluoro-phenyl]methoxy]-5-methylsulfonyl-isothiazole-4-carboxylate

3-[[3-(Difluoromethoxy)-2,6-difluoro-phenyl]methoxy]-5-methylsulfonylisothiazol-4-methyl formate

At room temperature, methyl 3-hydroxyl-5-methylsulfonylisothiazol-4-carboxylate I009 (1.7 g, 7.17 mmol) was dissolved in DMF (20 mL), followed by the addition of 3-(bromomethyl)-1-(difluoromethoxy)-2,4-difluorobenzeneI544 (2.4 g, 8.60 mmol) and potassium carbonate (3 g, 21.50 mmol). The reaction system was stirred at 25°C to react for 1 h. The reaction mixture was poured into water (100 mL) and then filtered, and the filter cake was dissolved in ethyl acetate (20 mL), 用 dried with anhydrous sodium sulfate, and concentrated under reduced pressure to obtain a yellow crude product 3-[[3-(difluoromethoxy)-2,6-difluoro-phenyl]methoxy]-5-methylsulfonylisothiazol-4-methyl formateI545 (1.8 g, 4.19 mmol, yield: 58.51%).
The product was verified by LCMS and H-NMR.
For MS-ESI, the calculated value was $[M+H]^+$ 430.0, and the measurement was 429.8.
$^1$H NMR (400MHz, $CDCl_3$): δ (ppm) 7.33-7.29 (m, 1H), 6.94 (t, J = 8.8 Hz, 1H), 6.54 (t, J = 72.8 Hz, 1H), 5.57 (s, 2H), 3.89 (s, 3H), 3.48 (s, 3H).

Step 6

Methyl 3-[[3-(difluoromethoxy)-2,6-difluoro-phenyl]methoxy]-5-[(2,4-dimethoxyphenyl)methylamino]isothiazole-4-carboxylate

At room temperature, 3-[[3-(difluoromethoxy)-2,6-difluoro-phenyl]methoxy]-5-methylsulfonylisothiazol-4-methyl formate I545 (1.8 g, 4.19 mmol) was dissolved in tetrahydrofuran (20 mL), followed by the addition of (2,4-dimethoxyphenyl)methylamine (7 g, 41.92 mmol). The reaction system was stirred at 65°C to react for 1 h. The reaction mixture was poured into water (50 mL) and then filtered, and the filter cake was dissolved in ethyl acetate (30 mL), dried with anhydrous sodium sulfate, and concentrated under reduced pressure to obtain a yellow crude product methyl 3-[[3-(difluoromethoxy)-2,6-difluorophenyl]methoxy]-5-[(2,4-dimethoxyphenyl)methylamino]isothiazol-4-carboxylate I546 (1.3 g, 2.52 mmol, yield: 60.04%) The product was verified by LCMS and H-NMR.
For MS-ESI, the calculated value was $[M+H]^+$ 517.1, and the measurement was 517.0. $^1$H NMR (400MHz,

$CDCl_3$): δ (ppm) 8.10 (s, 1H), 7.16 (d, J=8.0 Hz, 1H), 6.90 (t, J = 8.8 Hz, 1H), 6.70-6.33 (m, 3H), 5.47 (s, 2H), 4.26 (d, J=6.0 Hz, 2H), 3.84 (s, 3H), 3.80 (s, 3H), 3.72 (s, 3H).

Step 7

Methyl 5-amino-3-[[3-(difluoromethoxy)-2,6-difluoro-phenyl]methoxy]isothiazole-4-carboxylate

At room temperature, methyl 3-[[3-(difluoromethoxy)-2,6-difluorophenyl]methoxy]-5-[(2,4-dimethoxyphenyl) methylamino]isothiazol-4-carboxylate I546 (1.3 g, 2.52 mmol) was dissolved in dichloromethane (20 mL) and water (4 mL), followed by the addition of 2,3-dichloro-5,6-dicyanobenzoquinone (1.7 g, 7.55 mmol). The reaction system was stirred at 25°C to react for 1 h. The reaction mixture was poured into water (50 mL) and extracted with dichloromethane (20 mL x 3), and the organic phases were combined, washed with saturated brine (50 mL), dried with anhydrous sodium sulfate, and concentrated under reduced pressure to obtain a crude product. The crude product was purified by chromatographic columns (PE/EA = 1/0-3/1) to obtain a yellow solid product methyl 5-amino-3-[[3-(difluoromethoxy)-2,6-difluoro-phenyl]methoxy]isothiazol-4-carboxylate I547 (800 mg, 2.18 mmol, yield: 86.77%)

The product was verified by LCMS and H-NMR.

For MS-ESI, the calculated value was $[M+H]^+$ 367.0, and the measurement was 367.0.

$^1H$ NMR (400MHz, $CDCl_3$): δ (ppm) 7.21-7.15 (m, 1H), 6.86-6.82 (m, 1H), 6.64-6.27 (m, 3H), 5.42 (s, 2H), 3.69 (s, 3H).

Step 8

Methyl 3-[[3-(difluoromethoxy)-2,6-difluoro-phenyl]methoxy]-5-(4-pyrrolidin-1-ylbutylcarbamoylamino)isothiazole-4-carboxylate

4-Pyrrolidin-1-butyl-1-amine (291.25 mg, 2.05 mmol) was dissolved in tetrahydrofuran (8 mL), followed by the addition of carbonyl diimidazole (332 mg, 2.05 mmol) under the protection of nitrogen, and the mixture was stirred at 25 °C to react for 1 hour. Dimethyl sulfoxide (8 mL) was added, rotary evaporaton was carried out under reduced pressure to remove tetrahydrofuran, followed by the addition of methyl 5-amino-3-[[3-(difluoromethoxy)-2,6-difluorophenyl]methoxy] isothiazol-4-carboxylate I547 (500 mg, 1.37 mmol) and potassium carbonate (566 mg, 4.10 mmol), and the reaction system was stirred at 25 °C to react for 1 hour. Water (30 mL) was added to the reaction mixture, which was then subjected to liquid separation and extraction with ethyl acetate (20 mLx3). The organic phases were combined, washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a yellow solid product methyl 3-[[3-(difluoromethoxy)-2,6-difluoro-phenyl]methoxy]-5-(4-pyrrolidin-1-ylbutylcarbamylamino)isothiazol-4-carboxylate I548 (300 mg, 561.25 μmol, yield: 41.12%). The product was verified by LCMS and H-NMR.

For MS-ESI, the calculated value was $[M+H]^+$ 535.2, and the measurement was 535.0.

$^1H$ NMR (400MHz, $CDCl_3$): δ (ppm) 10.20 (s, 1H), 7.87 (s, 1H), 7.25-7.22 (m, 1H), 6.93-6.89 (m, 1H), 6.71-6.34 (m, 1H), 5.49 (s, 2H), 3.77 (s, 3H), 3.31 (s, 2H), 2.59 (s, 4H), 1.86 (s, 6H), 1.65 (s, 4H).

Step 9

3-[[3-(difluoromethoxy)-2,6-difluoro-phenyl]methoxy]-5-(4-pyrrolidin-1-ylbutylcarbamoylamino)isothiazole-4-carboxamide

Methyl 3-[[3-(difluoromethoxy)-2,6-difluoro-phenyl]methoxy]-5-(4-pyrrolidin-1-ylbutylcarbamylamino)isothiazol-4-carboxylate I548 (300 mg, 561.25 μmol) was dissolved in $NH_3$/MeOH (10 mol/L, 5 mL), and the mixture was stirred at 40 °C to react for 16 hours. The reaction mixture was concentrated under reduced pressure to obtain a crude product. The crude product was purified by the preparative thin-layer chromatography (DCM/MeOH = 10/1) to obtain a white solid product 3-[[3-(difluoromethoxy)-2,6-difluoro-phenyl]methoxy]-5-(4-pyrrolidin-1-ylbutylcarbamylamino)isothiazol-4-formamide T148 (53.52 mg, 103.02 μmol, yield: 18.36%).

The product was verified by LCMS, H-NMR, C-NMR and F-NMR.

For MS-ESI, the calculated value was $[M+H]^+$ 520.2, and the measurement was 520.0.

$^1H$ NMR (400MHz, $CD_3OD$): δ (ppm) 7.39 (dd, J = 14.4, 9.2 Hz, 1H), 7.08 (t, J=8.8Hz, 1H), 6.83 (t, J=73.2Hz, 1H), 5.59 (s, 2H), 3.24 (t, J=6.0Hz, 2H), 2,60 (s, 4H), 2.53 (t, J=6.4Hz, 2H), 1,82 (s, 4H), 1.58 (s, 4H).

$^{13}C$ NMR (100 MHz, $CD_3OD$): δ (ppm) 170.46, 167.08, 162.71, 161.26, 158,74, 156.03, 136.16, 125.54, 125.44, 120.25, 117.64, 114.83, 112.66, 112.62, 112.42, 112.38, 98.63, 59.19, 57.04, 40.88, 28.79, 26.63, 24.15.

$^{19}F$ NMR (400 MHz, $CD_3OD$): δ (ppm) -84.20, -84.21, -118.80, -118.81, -131.27, -131.28.

Example 121: Synthesis of Compound T149

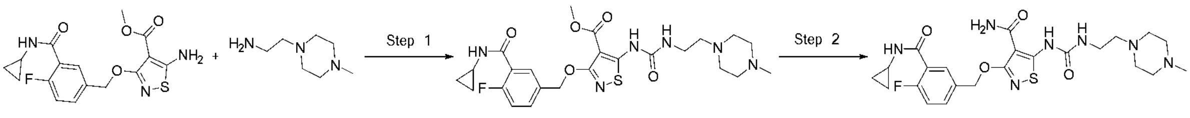


I341 I549 T149

Step 1

Methyl 3-[[3-(cyclopropylcarbamoyl)-4-fluoro-phenyl]methoxy]-5-[2-(4-methylpiperazin-1-yl)ethylcarbamoylamino]isothiazole-4-carboxylate

At room temperature, 2-(4-methylpiperazin-1-yl)ethylamine (118 mg, 821.07 μmol) was dissolved in tetrahydrofuran (4 mL), followed by the addition of N,N'-carbonyl diimidazole (133 mg, 821.07 μmol) under the protection of nitrogen, and the mixture was stirred at 25 °C to react for 1 hour. Dimethyl sulfoxide (4 mL) was added, the pressure was reduced to remove tetrahydrofuran, followed by the addition of methyl 5-amino-3-[[3-(cyclopropylcarbamyl)-4-fluorophenyl]methoxy]isothiazol-4-carboxylate I341 (200 mg, 547.38 μmol) and anhydrous potassium carbonate (227 mg, 1.64 mmol), and the reaction system was stirred at 25 °C to react for 1 hour. Water (40 mL) was added to the reaction mixture, and then extracted with ethyl acetate (20 mLx3) for liquid seperation. The organic phases were combined, washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a white solid product methyl 3-[[3-(cyclopropylcarbamyl)-4-fluoro-phenyl]methoxy]-5-[2-(4-methylpiperazin-1-yl)ethylcarbamylamino]isothiazol-4-carboxylate I549 (130 mg, 243.17 μmol, yield: 44.42%)

The product was verified by LCMS and HNMR.

For MS-ESI, the calculated value was $[M+H]^+$ 535.2, and the measurement was 535.0.

$^1$H NMR (400MHz, $CDCl_3$): δ (ppm) 10.33 (s, 1H), 8.27 (dd, J = 7.6, 2.0 Hz, 1H), 7.55 -7.51 (m, 1H), 7.13-7.10 (m, 1H), 6.83 (d, *J*= 13.2 Hz, 1H), 5.88 (s, 1H), 5.43 (s, 2H), 3.96 (s, 3H), 3.44-3.40 (m, 2H), 2.96-2.93 (m, 1H), 2.62 -2.42 (m, 10H), 2.32 (s, 3H), 0.91-0.86 (m, 2H), 0.65-0.61 (m, 2H).

Step 2

3-[[3-(cyclopropylcarbamoyl)-4-fluoro-phenyl]methoxy]-5-[2-(4-methylpiperazin-1-yl)ethylcarbamoylamino]isothiazole-4-carboxamide

At room temperature, methyl 3-[[3-(cyclopropylcarbamyl)-4-fluoro-phenyl]methoxy]-5-[2-(4-methylpiperazin-1-yl)ethylcarbamylamino]isothiazol-4-carboxylate I549 (130 mg, 243.17 μmol) was dissolved in the solution of ammonia in methanol (6 mL, 7mol/L), and the mixture was stirred at 40 °C to react for 16 hours. The reaction mixture was concentrated under reduced pressure to obtain a crude product. The crude product was purified by the preparative thin-layer chromatography (DCM/MeOH = 10/1) to obtain a white solid product 3-[[3-(cyclopropylcarbamyl)-4-fluoro-phenylmethoxy]-5-[2-(4-methylpiperazin-1-yl)ethylcarbamylamino]isothiazol-4-formamide T149 (20.93 mg, 40.28 μmol, yield: 16.57%). The product was verified by LCMS, H-NMR, C-NMR and F-NMR.

For MS-ESI, the calculated value was $[M+H]^+$ 520.2, and the measurement was 520.1. $^1$H NMR (400MHz, $CD_3OD$): δ (ppm) 7.78 (dd, *J* = 6.8, 2.4 Hz, 1H), 7.66-7.62 (m, 1H), 7.23-7.18 (m, 1H), 5.47 (s, 2H), 3.36 (t, *J* = 6.8 Hz, 2H), 2.89-2.83 (m, 1H), 2.72-2.34 (m, 10H), 2.28 (s, 3H), 0.83-0.76 (m, 2H), 0.64-0.60 (m, 2H).

$^{19}$F NMR (400MHz, $CD_3OD$): δ (ppm) -116.89

$^{13}$C NMR (400MHz, $CD_3OD$): δ (ppm) 170.27, 168.11, 167.16, 163.13, 162.28, 159.79, 156.12, 134.40, 134.36, 134.16, 134.07, 131.45, 131.42, 124.75, 124.61, 117.63, 117.40, 98.83, 70.08, 58.36, 55.68, 53.62, 45.98, 38.25, 24.04, 6.69

Example 122: Synthesis of Compound T150

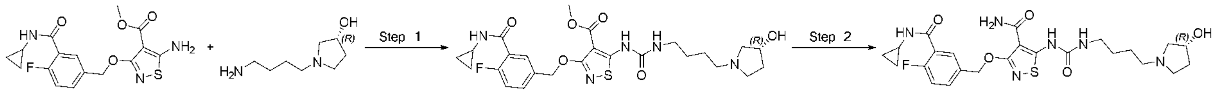


I341 I550 T150

Step 1

Methyl 3-[[3-(cyclopropylcarbamoyl)-4-fluoro-phenyl]methoxy]-5-[4-[(3R)-3-hydroxypyrrolidin-1-yl]butylcarbamoylamino]isothiazole-4-carboxylate

(3R)-1-(4-aminobutyl)pyrrolidin-3-ol (130 mg, 821.07 μmol) was dissolved in tetrahydrofuran (6 mL), followed by the addition of N,N'-carbonyl diimidazole (133 mg, 821.07 μmol) under the protection of nitrogen, and the mixture was stirred at 25 °C to react for 1 hour. Dimethyl sulfoxide (6 mL) was added, the pressure was reduced to remove tetrahydrofuran, followed by the addition of methyl 5-amino-3-[[3-(cyclopropylcarbamyl)-4-fluoro-phenyl]methoxy]isothiazol-4-carboxylate I341 (200 mg, 547.38 μmol) and anhydrous potassium carbonate (227 mg, 1.64 mmol), and the reaction system was stirred at 25 °C to react for 1 hour. Water (60 mL) was added to the reaction mixture, which was then subjected to liquid separation and extraction with ethyl acetate (20 mLx3). The organic phases were combined, washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a white solid product methyl 3-[[3-(cyclopropylcarbamyl)-4-fluoro-phenyl]methoxy]-5-[4-[(3R)-3-hydroxylpyrrolidin-1-yl]butylcarbamylamino]isothiazol-4-carboxylate I550 (140 mg, 254.72 μmol, yield: 46.54%)

The product was verified by LCMS and HNMR.

For MS-ESI, the calculated value was $[M+H]^+$ 550.2, and the measurement was 550.0.

$^1$H NMR (400MHz, $CDCl_3$ ): δ (ppm) 10.02 (s, 1H), 8.71 (s, 1H), 8.26 (dd, J = 7.2, 2.0 Hz, 1H), 7.53-7.50 (m, 1H), 7.12-7.07 (m, 1H), 6.82 (d, J = 13.2 Hz, 1H), 5.41 (s, 2H), 4.53 (s, 1H), 3.93 (s, 3H), 3.54 (s, 1H), 3.21-3.14 (m, 2H), 3.02 (d, J = 10.4 Hz, 1H), 2.96-2.93 (m, 1H), 2.65-2.57 (m, 1H), 2.53-2.46 (m, 1H), 2.36 (dd, J = 10.4, 4.0 Hz, 1H), 2.28-2.12 (m, 2H), 1.97-1.92 (m, 2H), 1.73-1.62 (m, 3H), 0.91-0.86 (m, 2H), 0.65-0.61 (m, 2H).

Step 2

3-[[3-(cyclopropylcarbamoyl)-4-fluoro-phenyl]methoxy]-5-[4-[(3R)-3-hydroxypyrrolidin-1-yl]butylcarbamoylamino]isothiazole-4-carboxamide

Methyl 3-[[3-(cyclopropylcarbamyl)-4-fluoro-phenyl]methoxy]-5-[4-[(3R)-3-hydroxylpyrrolidin-1-yl]butylcarbamylamino]isothiazol-4-carboxylate I550 (150 mg, 272.92 μmol) was dissolved in $NH_3$/MeOH (6 mL, 7mol/L), and the mixture was stirred at 40 °C to react for 16 hours. The reaction mixture was concentrated under reduced pressure to obtain a crude product. The crude product was purified by the preparative thin-layer chromatography (DCM/MeOH = 10/1) to obtain a white solid product 3-[[3-(cyclopropylcarbamyl)-4-fluorophenyl]methoxy]-5-[4-[(3R)-3-hydroxylpyrrolidin-1-yl]butylcarbamylamino]isothiazol-4-formamide T150 (32.57 mg, 60.92 μmol, yield: 22.32%)

The product was verified by LCMS, H-NMR, C-NMR and F-NMR.

For MS-ESI, the calculated value was $[M+H]^+$ 535.2, and the measurement was 535.1.

$^1$H NMR (400 MHz, $CD_3OD$) δ(ppm) 7.78 (dd, J = 6.8, 2.0 Hz, 1H), 7.66-7.62 (m, 1H), 7.20 (t, J = 10.4 Hz, 1H), 5.47 (s, 2H), 4.40- 4.37 (m, 1H), 3.24 (t, *J* = 6.4 Hz, 2H), 2.95-2.83 (m, 3H), 2.75 -2.64 (m, 2H), 2.19-2.10 (m, 1H), 1.79-1.76 (m, 1H), 1.59 (s, 4H), 0.83-0.79 (m, 2H), 0.64-0.60 (m, 2H).

$^{19}$F NMR (400 MHz, $CD_3OD$) δ(ppm) -116.84

$^{13}$C NMR (400 MHz, $CD_3OD$) δ(ppm) 170.30, 168.16, 167.21, 163.15, 162.28, 159.79, 156.16, 134.40, 134.37, 134.15, 134.06, 131.42, 131.39, 124.77, 124.62, 117.63, 117.40, 98.80, 71.18, 70.07, 63.36, 57.22, 53.76, 40.80, 34.87, 28.64, 26.18, 24.02, 6.67

Example 123: Synthesis of Compound T151

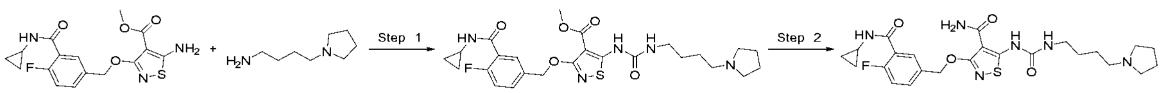


I341 I551 T151

Step 1

Methyl 3-[[3-(cyclopropylcarbamoyl)-4-fluoro-phenyl]methoxy]-5-(4-pyrrolidin-1-ylbutylcarbamoylamino)isothiazole-4-carboxylate

4-pyrrolidin-1-ylbutan-1-amine (146 mg, 1.03 mmol) was dissolved in tetrahydrofuran (6 mL), followed by the addition of N,N'-carbonyl diimidazole (166 mg, 1.03 mmol) under the protection of nitrogen, and the mixture was stirred at 25°C to react for 1 hour. Dimethyl sulfoxide (6 mL) was added, the pressure was reduced to remove tetrahydrofuran, followed by the addition of methyl 5-amino-3-[[3-(cyclopropylcarbamyl)-4-fluoro-phenyl]methoxy]isothiazol-4-carboxylate I341 (250 mg, 684.22 μmol) and anhydrous potassium carbonate (284 mg, 2.05 mmol), and the reaction system was stirred at 25 °C to react for 1 hour. Water (60 mL) was added to the reaction mixture, which was then subjected to liquid separation and extraction with ethyl acetate (20 mLx3). The organic phases were combined, washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a yellow solid product methyl 3-[[3-(cyclopropylcarbamyl)-4-fluorophenyl]methoxy]-5-(4-pyrrolidin-1-ylbutylcarbamylamino)isothiazol-4-carboxylate I551 (150 mg, 281.10 μmol, yield: 41.08%).

The product was verified by LCMS and HNMR.

For MS-ESI, the calculated value was $[M+H]^+$ 534.2, and the measurement was 534.0.

$^1H$ NMR (400MHz, $CDCl_3$ ): δ (ppm) 10.19 (s, 1H), 8.26 (d, J = 5.6 Hz, 1H), 7.55-7.52 (m, 1H), 7.12-7.07 (m, 2H), 6.82 (d, J = 10.8 Hz, 1H), 5.43 (s, 2H), 3.92 (s, 3H), 3.30 (m, 2H), 2.96-2.93 (m, 1H), 2.61 (s, 4H), 2.54 (t, J = 6.0 Hz, 2H), 1.89 (s, 4H), 1.68 (s, 4H), 0.91-0.86 (m, 2H), 0.65-0.61 (m, 2H).

Step 2

3-[[3-(cyclopropylcarbamoyl)-4-fluoro-phenylmethoxy]-5-(4-pyrrolidin-1-ylbutylcarbamoylamino)isothiazole-4-carboxamide

Methyl 3-[[3-(cyclopropylcarbamyl)-4-fluorophenyl]methoxy]-5-(4-pyrrolidin-1-ylbutylcarbamylamino)isothiazol-4-carboxylate I551 (150 mg, 281.10 μmol) was dissolved in the solution of ammonia in methanol (6 mL,7mol/L), and the mixture was stirred at 40°C to react for 16 hours. The reaction mixture was concentrated under reduced pressure to obtain a crude product. The crude product was purified by the preparative thin-layer chromatography (DCM/MeOH = 10/1) to obtain a yellow solid product 3-[[3-(cyclopropylcarbamyl)-4-fluorophenyl]methoxy]-5-(4-pyrrolidin-1-ylbutylcarbamylamino)isothiazol-4-formamide T151.

The product was verified by LCMS, H-NMR, C-NMR and F-NMR.

For MS-ESI, the calculated value was $[M+H]^+$ 519.2, and the measurement was 519.0.

$^1H$ NMR (400MHz, $CD_3OD$): δ (ppm) 7.77 (dd, *J* = 6.4, 2.0 Hz, 1H), 7.66-7.62 (m, 1H), 7.20 (t, J = 10.0 Hz, 1H), 5.47 (s, 2H), 3.24 (t, J = 6.4 Hz, 2H), 2.89-2.83 (m, 1H), 2.60 (s, 4H), 2.54 (t, J = 7.2 Hz, 2H), 1.82 (s, 4H), 1.58 (s, 4H), 0.83-0.78 (m, 2H), 0.64-0.60 (m, 2H).

$^{19}F$ NMR (400MHz, $CD_3OD$): δ (ppm) -116.87

$^{13}C$ NMR (400MHz, $CD_3OD$): δ (ppm) 170.32, 168.13, 167.20, 163.14, 162.28, 159.79, 156.13, 134.40, 134.37, 134.14, 134.05, 131.43, 131.40, 124.76, 124.61, 117.62, 117.39, 98.78, 70.07, 57.09, 54.99, 40.92, 28.83, 26.75, 24.16, 24.04, 6.68.

Example 124: Synthesis of Compound T152

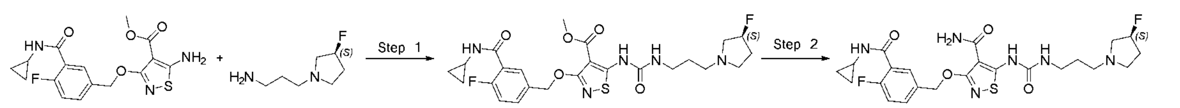


I341 I552 T152

Step 1

Methyl 3-[[3-(cyclopropylcarbamoyl)-4-fluoro-phenyl]methoxy]-5-[3-[(3S)-3-fluoropyrrolidin-1-yl]propylcarbamoylamino]isothiazole-4-carboxylate

3-[(3S)-3-fluoropyrrolidin-1-yl]propyl-1-amine (210 mg, 1.44 mmol) was dissolved in tetrahydrofuran (6 mL),

followed by the addition of N,N'-carbonyl diimidazole (233 mg, 1.44 mmol) under the protection of nitrogen, and the mixture was stirred at 25 °C to react for 1 hour. Dimethyl sulfoxide (6 mL) was added, the pressure was reduced to remove tetrahydrofuran, followed by the addition of methyl 5-amino-3-[[3-(cyclopropylcarbamyl)-4-fluorophenyl]methoxy]isothiazol-4-carboxylate I341 (350 mg, 957.91 μmol) and anhydrous potassium carbonate (397 mg, 2.87 mmol), and the reaction system was stirred at 25°C to react for 1 hour. Water (60 mL) was added to the reaction mixture, which was then subjected to liquid separation and extraction with ethyl acetate (20 mLx3). The organic phases were combined, washed with saturated brine (20 mL), dried with anhydrous sodium sulfate, and concentrated under reduced pressure to obtain a crude product. The crude product was purified by the preparative thin-layer chromatography (DCM/MeOH = 10/1) to obtain a yellow solid product methyl 3-[[3-(cyclopropylcarbamyl)-4-fluoro-phenyl]methoxy]-5-[3-[(3S)-3-fluoropyrrolidin-1-yl]propylcarbamylamino]isothiazol-4-carboxylate I552 (200 mg, 372.04 μmol, yield: 38.84%) The product was verified by LCMS and HNMR.

For MS-ESI, the calculated value was $[M+H]^+$ 538.2, and the measurement was [M+H]+538.4.

$^1H$ NMR (400 MHz, $CDCl_3$) δ(ppm) 10.06 (s, 1H), 8.25 (dd, *J* =2.0, 7.6Hz, 1H), 7.56-7.52 (m, 1H), 7.42-7.34 (m, 1H), 7.12-7.05 (m, 1H), 6.81 (d, *J*= 13.2 Hz, 1H), 5.42 (s, 2H), 5.30-5.17 (m, 1H), 3.92 (s, 3H), 3.64-3.57 (m, 1H), 3.39-3.30 (m, 1H), 3.16-3.03 (m, 2H), 2.97-2.91 (m, 1H), 2.79-2.72 (m, 1H), 2.67-2.65 (m, 1H), 2.52-2.35 (m, 2H), 2.24-2.11 (m, 3H), 1.80-1.72 (m, 2H), 0.91-0.86 (m, 2H), 0.65-0.61 (m, 2H).

Step 2

3-[[3-(cyclopropylcarbamoyl)-4-fluoro-phenylmethoxy]-5-[3-[(3S)-3-fluoropyrrolidin-1-yl]propylcarbamoylamino]isothiazole-4-carboxamide

Methyl 3-[[3-(cyclopropylcarbamyl)-4-fluoro-phenyl]methoxy]-5-[3-[(3S)-3-fluoropyrrolidin-1-yl]propylcarbamylamino]isothiazol-4-carboxylate I552 (270 mg, 502.25 μmol) was dissolved in the solution of ammonia in methanol (6 mL), and the mixture was stirred at 40 °C to react for 16 hours. The reaction mixture was concentrated under reduced pressure to obtain a crude product. The crude product was purified by the preparative thin-layer chromatography (DCM/MeOH = 10/1) to obtain a yellow solid product 3-[[3-(cyclopropylcarbamyl)-4-fluoro-phenyl]methoxy]-5-[3-[(3S)-3-fluoropyrrolidin-1-yl]propylcarbamylamino]isothiazol-4-formamide T152 (51.37 mg, 98.30 μmol, yield: 19.57%).

The product was verified by LCMS, H-NMR, C-NMR and F-NMR.

For MS-ESI, the calculated value was $[M+H]^+$ 523.2, and the measurement was $[M+H]^+$ 523.1. $^1H$ NMR (400 MHz, $CD_3OD$) δ(ppm) 7.78 (dd, *J* = 6.8, 2.4 Hz, 1H), 7.66-7.62 (m, 1H), 7.23-7.18 (m, 1H), 5.48 (s, 2H), 5.28-5.12 (m, 1H), 3.29-3.26(m, 2H), 3.10-2.98 (m, 2H), 2.89-2.84 (m, 1H), 2.83-2.71 (m, 1H), 2.65 (t, *J*= 7.6 Hz, 1H), 2.58-2.52 (m, 1H), 2.29-2.14 (m, 1H), 2.12-1.99 (m, 1H), 1.83-1.75 (m, 2H), 0.83-0.79 (m, 2H), 0.64-0.60 (m, 2H).

$^{19}F$ NMR (400 MHz, $CD_3OD$) δ(ppm) -116.88, -169.68.

$^{13}C$ NMR (100 MHz, $CD_3OD$) δ(ppm) 170.22, 168.06, 167.17, 163.13, 162.28, 159.79, 156.10, 134.37, 134.34, 134.16, 134.07, 131.47, 131.44, 124.70, 124.56, 117.64, 117.41, 98.83, 95.05, 93.31, 70.11, 61.69, 61.46, 54.88, 54.72, 53.44, 39.28, 33.45, 33.23, 29.36, 24.07, 6.73. Example 125: Synthesis of Compound T153

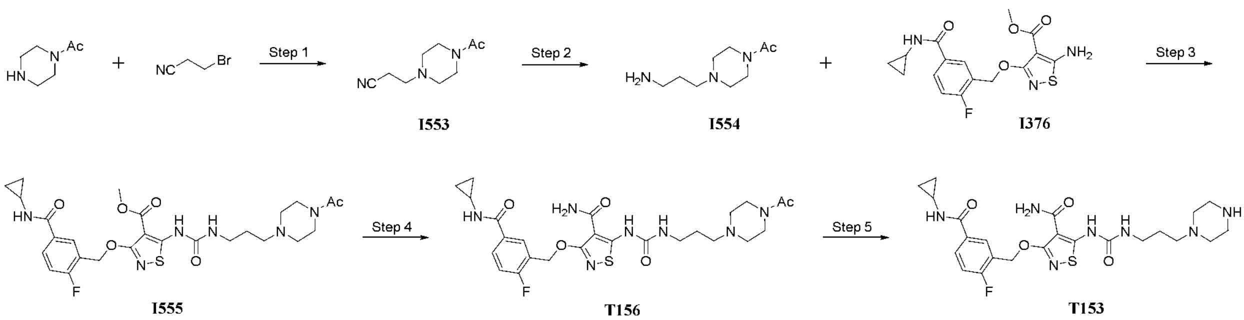

I553
I554
I376
I555
T156
T153

Step 1

3-(4-acetylpiperazin-1-yl)propanenitrile

N-acetylpiperazine (24 g, 187.25 mmol) was dissolved in acetonitrile (240.00 mL), followed by the addition of 3-bromopropionitrile (25.1 g, 187.25 mmol) and potassium carbonate (77.6 g, 561.75 mmol) at 0 °C, and the mixture was stirred at 60 °C to react for 16 hours. The reaction mixture was concentrated under reduced pressure to obtain a crude product. The crude product was purified by the column chromatography (petroleum ether/ethyl acetate = 10/1-0/1) to

obtain a white solid product 3-(4-acetylpiperazin-1-yl)propionitrile I553 (11 g, 60.69 mmol, yield: 32.41%). The product was verified by LCMS and H-NMR.

For MS-ESI, the calculated value was $[M+H]^+$ 182.1, and the measurement was 182.1.

$^1$H NMR (400MHz, $CDCl_3$): δ (ppm) 3.65 (t, *J* = 5.2 Hz, 2H), 3.50 (t, *J*= 4.8 Hz, 2H), 2.72 (t, *J* = 6.8 Hz, 2H), 2.55-2.51 (m, 4H), 2.48 (t, *J* =5.2 Hz, 2H), 2.10 (s, 3H).

Step 2

1-(4-(3-aminopropyl)piperazin-1-yl)ethan-1-one

At room temperature, 3-(4-acetylpiperazin-1-yl)propionitrile I553 (5 g, 27.59 mmol) was dissolved in methanol (50 mL), followed by the addition of raney nickel (5 g). The reaction system was stirred at 25 °C in the atmosphere of hydrogen to react for 3 hours. The reaction mixture was filtered with diatomite, and the filtrate was concentrated under reduced pressure to obtain a yellow oily product 1-(4-(3-aminopropyl)piperazin-1-yl)ethan-1-oneI554 (2.7 g, 14.57 mmol, yield: 52.82%).

The product was verified by LCMS and H-NMR.

For MS-ESI, the calculated value was $[M+H]^+$ 186.2, and the measurement was 186.1.

$^1$H NMR (400MHz, $CDCl_3$): δ (ppm) 3.57 (t, *J* = 5.2 Hz,2H), 3.43-3.41 (m, 2H), 2.74-2.59 (m, 2H), 2.40-2.35 (m, 6H), 2.04 (s, 3H), 1.66-1.57 (m, 2H).

Step 3

Methyl 5-[3-(4-acetylpiperazin-1-yl)propylcarbamoylamino]-3-[[5-(cyclopropylcarbamoyl)-2-fluoro-phenyl]methoxy]isothiazole-4-carboxylate

At room temperature, 1-(4-(3-aminopropyl)piperazin-1-yl)ethan-1-oneI554 (342 mg, 1.85 mmol) was dissolved in tetrahydrofuran (5 mL), followed by the addition of N,N'-carbonyl diimidazole (266 mg, 1.85 mmol). The reaction system was stirred at 25°C to react for 1 h. The mixture was spin-dried and dissolved in the solution of dimethyl sulfoxide (5 mL), followed by the addition of methyl 5-amino-3-[[5-(cyclopropylcarbamyl)-2-fluoro-phenyl]methoxy]isothiazol-4-carboxylate I376 (450 mg, 1.23 mmol) and potassium carbonate (510 mg, 3.69 mmol). The reaction system was stirred at 25°C to react for 3 hours. The reaction mixture was poured into water (30 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane /methanol = 10/1) to obtain a yellow solid product methyl 5-[3-(4-acetylpiperazin-1-yl)propylcarbamylamino]-3-[[5-(cyclopropylcarbamyl)-2-fluorophenyl]methoxy]isothiazol-4-carboxylate I555 (350 mg, 606.97 μmol, yield: 49.28%).

The product was verified by LCMS and H-NMR.

For MS-ESI, the calculated value was $[M+H]^+$ 577.2, and the measurement was 577.1.

$^1$H NMR (400MHz, $CDCl_3$): δ (ppm) 10.34 (s, 1H), 8.09 (dd, *J* = 6.8, 4.4 Hz, 1H), 7.70-73.66 (m, 1H), 7.11 (t, *J* = 8.8 Hz, 1H), 6.93 (s, 1H), 6.24 (s, 1H), 3.92 (s, 3H), 3.70-3.65 (m, 2H), 3.53 (t, *J* =4.0 Hz, 2H), 3.46 (dd, *J* = 11.6, 6.0 Hz, 2H), 2.91-2.89 (m, 1H), 2.56-2.46 (m, 6H), 2.11 (s, 3H), 1.80-1.77 (m, 3H), 1.29-1.25 (m, 1H), 0.91-0.86 (m, 2H), 0.64-0.60 (m, 2H).

Step 4

5-[3-(4-acetylpiperazin-1-yl)propylcarbamoylamino]-3-[[5-(cyclopropylcarbamoyl)-2-fluorophenyl]methoxy]isothiazole-4-carboxamide

At room temperature, methyl 5-[3-(4-acetylpiperazin-1-yl)propylcarbamylamino]-3-[[5-(cyclopropylcarbamyl)-2-fluoro-phenyl]methoxy]isothiazol-4-carboxylate I555 (350 mg, 606.97 μmol) was dissolved in the solution of ammonia methanol (5 mL), and the reaction system was stirred at 60°C to react for 16 hours. The reaction mixture was concentrated under reduced pressure to obtain a crude product. The crude product was purified by the preparative plate (dichloromethane /methanol = 10/1) to obtain a yellow solid product 5-[3-(4-acetylpiperazin-1-yl)propylcarbamylamino]-3-[[5-(cyclopropylcarbamyl)-2-fluoro-phenyl]methoxy]isothiazol-4-formamide T156 (110 mg, 195.86 μmol, yield: 32.27%).

The product was verified by LCMS and H-NMR.

For MS-ESI, the calculated value was $[M+H]^+$ 562.2, and the measurement was 562.0.

$^1$H NMR (400MHz, $CDCl_3$): δ (ppm) 11.07 (s, 1H), 7.94 (dd, *J* = 6.8, 2.4 Hz, 1H), 7.79-7.75 (m, 2H), 7.15 (d, *J* = 8.8

Hz, 1H), 7.10-7.00 (m, 2H), 6.70 (s, 1H), 5.83 (s, 1H), 3.59 (s, 2H), 3.49-3.47 (m, 2H), 3.42-3.40 (m, 2H), 2.92-2.89 (m, 1H), 2.51-2.39 (m, 6H), 2.09 (s, 3H), 1.86-1.74 (m, 2H), 1.42-1.25 (m, 2H), 0.98-0.84 (m, 2H), 0.67-0.62 (m, 2H).

Step 5

3-[[5-(cyclopropylcarbamoyl)-2-fluoro-phenyl]methoxy]-5-(3-piperazin-1-ylpropylcarbamoylamino)isothiazole-4-carboxamide

At room temperature, 5-[3-(4-acetylpiperazin-1-yl)propylcarbamylamino]-3-[[5-(cyclopropylcarbamyl)-2-fluorophenyl]methoxy]isothiazol-4-formamide T156 (110 mg, 195.86 μmol) was dissolved in ethanol (5 mL), followed by the addition of sodium hydroxide (23.5 mg, 587.58 μmol). The reaction system was stirred at 80°C to react for 16 h. The reaction mixture was concentrated under reduced pressure to obtain a crude product. The crude product was purified by the high-pressure preparative liquid chromatography to obtain a white solid product 3-[[5-(cyclopropylcarbamyl)-2-fluorophenyl]methoxy]-5-(3-piperazin-1-ylpropylcarbamylamino)isothiazol-4-formamide T153 (21.7 mg, 41.67 μmol, yield: 21.27%). The product was verified by LCMS, H-NMR, C-NMR and F-NMR.

For MS-ESI, the calculated value was $[M+H]^+$ 520.2, and the measurement was 520.2.

$^1$H NMR (400MHz, CD3OD): δ (ppm) 8.00 (d, *J* = 6.0 Hz, 1H), 7.84 (s, 1H), 7.22 (t, *J* = 9.2 Hz, 1H), 5.54 (s, 2H), 3.30 (s, 1H), 3.25 (t, *J* = 6.4 Hz, 2H), 2.83 (s, 4H), 2.44-2.38 (m, 6H), 1.73 (s, 2H), 0.78 (d, *J* = 6.0 Hz, 2H), 0.62 (s, 2H).

$^{13}$C NMR (101 MHz, $CD_3OD$) δ 172.90, 172.75, 169.68, 168.21, 165.69, 165.51, 158.66, 134.09, 134.04, 133.69, 133.60, 127.45, 119.37, 119.15, 101.25, 87.68, 57.35, 42.04.29.98, 26.62, 9.09 $^{19}$F NMR (377 MHz, $CD_3OD$) δ -111.37

Example 126: Synthesis of Compound T155

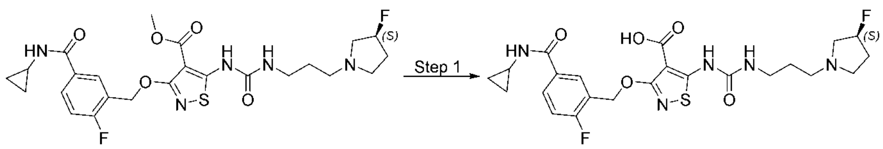


I464 T155

3-[[5-(cyclopropylcarbamoyl)-2-fluoro-phenylmethoxy]-5-[3-[(3S)-3-fluoropyrrolidin-1-yl]propylcarbamoylamino]isothiazole-4-carboxylic acid

At room temperature, methyl 3-[[5-(cyclopropylcarbamyl)-2-fluoro-phenyl]methoxy]-5-[3-[(3S)-3-fluoropyrrolidin-1-yl]propylcarbamylamino]isothiazol-4-carboxylate I464 (500 mg, 930.10 μmol) was dissolved in tetrahydrofuran (5 mL), followed by the addition of potassium trimethyl silanolate (358 mg, 2.79 mmol). The reaction system was stirred at 60°C to react for 16 h. The reaction mixture was concentrated under reduced pressure to obtain a crude product. The crude product was purified by the high-pressure preparative liquid chromatography to obtain a yellow solid product 3-[[5-(cyclopropylcarbamyl)-2-fluoro-phenylmethoxy]-5-[3-[(3S)-3-fluoropyrrolidin-1-yl]propylcarbamylamino]isothiazol-4-carboxylic acidT155 (93.09 mg, 177.80 μmol, yield: 19.12%).

The product was verified by LCMS, H-NMR and F-NMR.

For MS-ESI, the calculated value was $[M+H]^+$ 524.2, and the measurement was 524.0.

$^1$H NMR (400MHz, DMSO_$d_6$ ): δ (ppm) 10.49 (s, 1H), 10.24-10.13 (m, 1H), 8.48 (d, *J*= 4.0 Hz, 1H), 8.31 (s, 1H), 8.01 (dd, *J* = 6.8, 1.6 Hz, 1H), 7.86-7.83 (m, 1H), 7.32 (t, *J* = 9.2 Hz, 1H), 5.54-5.50 (m, 1H), 5.40 (s, 2H), 3.92-3.85 (m, 1H), 3.72 (s, 1H), 3.51-3.20 (m, 6H), 2.86-2.79 (m, 1H), 2.33 (s, 1H), 2.15 (s, 1H), 1.88-1.81 (m, 2H), 0.71-0.62 (m, 2H), 0.58-0.54 (m, 2H).

$^{19}$F NMR (400MHz, DMSO_$d_6$): δ (ppm) -114.68, -171.22, -172.15.

Example 127: Synthesis of Compound T156

Refer to Example 125.

Example 128: Synthesis of Compound T157

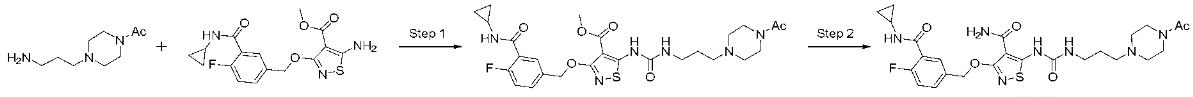


I554 I341 I556 T157

Step 1

5-[3-(4-acetylpiperazin-1-yl)propylcarbamoylamino]-3-[[3-(cyclopropylcarbamoyl)-4-fluorophenyl]methoxy]isothiazole-4-carboxylate

At room temperature, 1-[4-(3-aminopropyl)piperazin-1-yl]ethanone I554 (2.3 g, 12.32 mmol) was dissolved in tetrahydrofuran (30 mL), followed by the addition of N,N'-carbonyl diimidazole (2.0 g, 12.32 mmol), and the reaction system was stirred at 25 °C to react for 2 hours. Tetrahydrofuran was spun away, the reaction mixture was then dissolved in dimethyl sulfoxide (30 mL), followed by the addition of methyl 5-amino-3-[[3-(cyclopropylcarbamyl)-4-fluorophenyl] methoxy]isothiazol-4-carboxylate I341 (3 g, 8.21 mmol) and potassium carbonate (3.4 g, 24.60 mmol), and the reaction system was stirred at 25 °C to react for 14 hours. The reaction mixture was poured into water (100 mL) and extracted with ethyl acetate (50 mL x 3), and the organic phases were combined, washed with saturated brine (50 mL), dried with anhydrous sodium sulfate, and concentrated under reduced pressure to obtain a crude product. The crude product was purified by chromatographic columns (dichloromethane /methanol = 50/1-30/ 1) to obtain a white solid product methyl 5-[3-(4-acetylpiperazin-1-yl)propylcarbamylamino]-3-[[3-(cyclopropylcarbamyl)-4-fluoro-phenyl]methoxy]isothiazol-4-carboxylate I556 (2 g, 3.47 mmol, yield: 42.24%).

The product was verified by LCMS and H-NMR.

For MS-ESI, the calculated value was $[M+H]^+$ 577.2, and the measurement was 577.2.

$^1$H NMR (400MHz, DMSO-$d_6$): δ (ppm) 10.39 (s, 1H), 8.41 (d, *J* = 3.6 Hz, 1H), 8.15-8.13 (m, 1H), 7.67-7.65 (m, 1H), 7.56-7.55 (m, 1H), 7.31-7.26 (m, 1H), 5.36 (s, 2H), 3.82 (s, 3H), 3.42-3.39 (m, 4H), 3.20-3.15 (m, 2H), 2.86-2.82 (m, 1H), 2.36-2.27 (m, 6H), 1.98 (s, 3H), 1.64-1.60 (m, 2H), 0.72-0.67 (m, 2H), 0.55-0.51(m, 2H).

Step 2

5-[3-(4-acetylpiperazin-1-yl)propylcarbamoylamino]-3-[[3-(cyclopropylcarbamoyl)-4-fluorophenyl]methoxy]isothiazole-4-carboxamide

At room temperature, methyl 5-[3-(4-acetylpiperazin-1-yl)propylcarbamylamino]-3-[[3-(cyclopropylcarbamyl)-4-fluoro-phenyl]methoxy]isothiazol-4-carboxylate I556 (1 g, 1.73 mmol) was dissolved in the solution of ammonia in methanol (10 mL). The reaction system was stirred at 70°C to react for 16 h. The reaction mixture was concentrated under reduced pressure to obtain a crude product. The crude product was purified by the high-pressure preparative liquid chromatography to obtain a white solid product 5-[3-(4-acetylpiperazin-1-yl)propylcarbamylamino]-3-[[3-(cyclopropylcarbamyl)-4-fluoro-phenyl]methoxy]isothiazol-4-formamide T157 (28.7 mg, 51.14 μmol, yield: 2.95%).

The product was verified by LCMS, H-NMR and F-NMR.

For MS-ESI, the calculated value was $[M+H]^+$ 562.2, and the measurement was 562.0.

$^1$H NMR (400MHz, $CD_3OD$): δ (ppm) 7.79 (dd, *J* = 6.8, 2.4 Hz, 1H), 7.66-7.62 (m, 1H), 7.22 (dd, *J*= 10.4, 8.8 Hz, 1H), 5.48 (s, 2H), 3.59-3.53 (m, 4H), 3.27 (d, *J*= 6.8 Hz, 2H), 2.88-2.84 (m, 1H), 2.50-2.42 (m, 6H), 2.08 (s, 3H), 1.79-1.74 (m, 2H), 0.83-0.78 (m, 2H), 0.64-0.60 (m, 2H).

$^{19}$F NMR (400MHz, $CD_3OD$): δ (ppm) -116.94.

Example 129: Synthesis of Compound T158

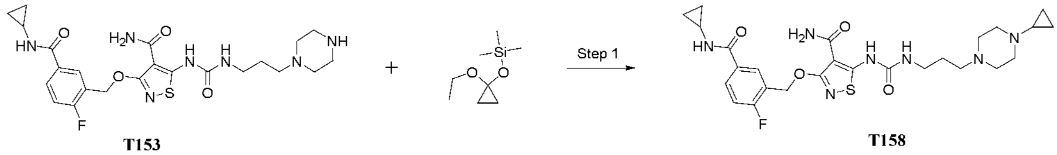


T153 T158

3-[[5-(cyclopropylcarbamoyl)-2-fluoro-phenyl]methoxy]-5-[3-(4-cyclopropylpiperazin-1-yl)propylcarbamoylamino]isothiazole-4-carboxamide

At room temperature, 3-[[5-(cyclopropylcarbamyl)-2-fluoro-phenyl]methoxy]-5-(3-piperazin-1-ylpropylcarbamylamino)isothiazol-4-formamide T153 (270 mg, 519.64 μmol) was dissolved in methanol (3 mL), followed by the addition of (1-ethoxycyclopropoxy)-trimethylsilane (317 mg, 1.82 mmol), cyanosodium borohydride (147 mg, 2.34 mmol) and glacial acetic acid (312 mg, 5.20 mmol). The reaction system was stirred at 25°C to react for 20 minutes, then warmed to 60°C and stirred for 16 hours. The reaction mixture was poured into water (10 mL), regulated with 2N sodium hydroxide aqueous solution to pH = 9-10, and then extracted with ethyl acetate (10 mL x 3), and the organic phases were combined, washed with saturated brine (30 mL), dried with anhydrous sodium sulfate, and concentrated under reduced pressure to obtain a crude product. The crude product was purified by the high-pressure preparative liquid chromatography to obtain a white solid product 3-[[5-(cyclopropylcarbamyl)-2-fluoro-phenyl]methoxy]-5-[3-(4-cyclopropylpiperazin-1-yl)propylcarbamylamino]isothiazol-4-formamide T158 (14.91 mg, 26.64 μmol, yield: 5.13%).

The product was verified by LCMS, H-NMR and F-NMR.

For MS-ESI, the calculated value was $[M+H]^+$ 560.2, and the measurement was 560.1.

$^1H$ NMR (400MHz, $CD_3OD$): δ (ppm) 8.01 (dd, J=6.8, 2.4 Hz, 1H), 7.87-7.83 (m, 1H), 7.27-7.22 (m, 1H), 5.57 (s, 2H), 3.26-3.25 (m, 2H), 2.85-2.81 (m, 1H), 2.68-2.42 (m, 10H), 1.77-1.73 (m, 2H), 1.68-1.65 (m, 1H), 0.80-0.77 (m, 2H), 0.64-0.60 (m, 2H), 0.49-0.46 (m, 2H), 0.42-0.40 (m, 2H).

$^{19}F$ NMR (400MHz, $CD_3OD$): δ (ppm) -115.45.

Example 130: Synthesis of Compound T159

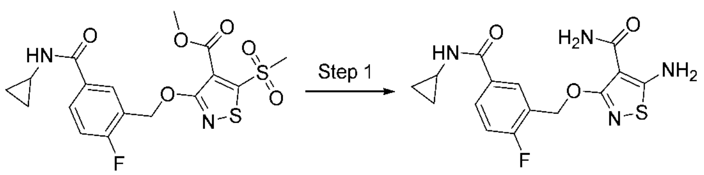


I374 T159

5-amino-3-[[5-(cyclopropylcarbamoyl)-2-fluoro-phenyl]methoxy]isothiazole-4-carboxamide

At room temperature, 3-[[5-(cyclopropylcarbamyl)-2-fluoro-phenyl]methoxy]-5-methylsulfonyl-isothiazol-4-methyl formate I374 (300 mg, 0.70 mmol) was dissolved in the solution of ammonia in methanol (5 mL, 7mol/L). The reaction system was stirred at 120°C to react for 16 h. The reaction mixture was concentrated under reduced pressure to obtain a crude product. The crude product was purified by the high-pressure preparative liquid chromatography to obtain a white solid product 5-amino-3-[[5-(cyclopropylcarbamyl)-2-fluoro-phenyl]methoxy]isothiazol-4-formamide T159 (23.11 mg, 65.96 μmol, yield: 9.42%).

The product was verified by LCMS, H-NMR and F-NMR.

For MS-ESI, the calculated value was $[M+H]^+$ 351.1, and the measurement was 351.0.

$^1H$ NMR (400MHz, $CD_3OD$): δ (ppm) 8.00 (dd, *J* =6.8, 2.0 Hz, 1H), 7.87-7.83 (m, 1H), 7.23 (m, 1H), 5.53 (s, 2H), 2.86-2.80 (m, 1H), 0.82-0.77 (m, 2H), 0.65-0.61 (m, 2H). $^{19}F$ NMR (400MHz, $CD_3OD$): δ (ppm) -115.49.

Example 131: Synthesis of Compound T160

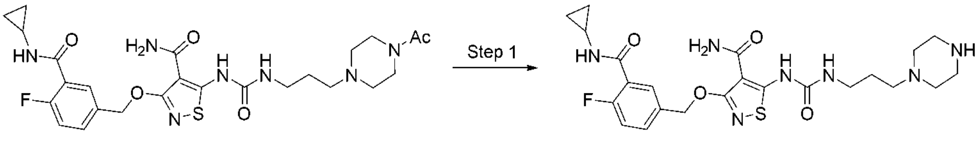


T157 T160

3-[[3-(cyclopropylcarbamoyl)-4-fluoro-phenyl]methoxy]-5-(3-piperazin-1-ylpropylcarbamoylamino)isothiazole-4-carboxamide

At room temperature, 5-[3-(4-acetylpiperazin-1-yl)propylcarbamylamino]-3-[[3-(cyclopropylcarbamyl)-4-fluoro-phenyl]methoxy]isothiazol-4-formamide T157 (300 mg, 0.53 mmol) was dissolved in ethanol (10 mL) and water (2 mL), followed by the addition of NaOH (64.1 mg, 1.60 mmol). The reaction system was stirred at 80°C to react for 16 h. The reaction mixture was concentrated under reduced pressure to obtain a crude product. The crude product was purified by the high-pressure preparative liquid chromatography to obtain a white solid product 3-[[3-(cyclopropylcarbamyl)-4-fluoro-phenyl]methoxy]-5-(3-piperazin-1-ylpropylcarbamylamino)isothiazol-4-formamide T160 (12.08 mg, 23.25 μmol, yield: 4.35%).
The product was verified by LCMS, H-NMR, C-NMR and F-NMR.
For MS-ESI, the calculated value was $[M+H]^+$ 520.2, and the measurement was 520.1.
$^1H$ NMR (400MHz, $CD_3OD$): δ (ppm) 7.79 (dd, *J* = 6.8, 2.4 Hz, 1H), 7.66-7.62 (m, 1H), 7.22 (dd, *J*= 10.4, 8.8 Hz, 1H), 5.48 (s, 2H), 3.26 (t, *J* = 6.8 Hz, 2H), 2.89-2.85 (m, 5H), 2.47-2.39 (m, 6H), 1.74-1.71 (m, 2H), 0.82-0.78 (m, 2H), 0.64-0.60 (m, 2H).
$^{13}C$ NMR (101 MHz, $CD_3OD$) δ170.30, 168.15, 167.21, 163.15, 162.28, 159.79, 156.10, 134.41, 134.37, 134.14, 131.39, 124.78, 124.63, 117.62, 117.39, 98.80, 70.07, 57.45, 54.54, 46.03, 39.48, 27.47, 24.03, 6.67.
$^{19}F$ NMR (400MHz, $CD_3OD$): δ (ppm) -116.92.

Example 132: Synthesis of Compound T161

Refer to Example 92 for the synthesis of Compound T161.

Example 133: Synthesis of Compound T162

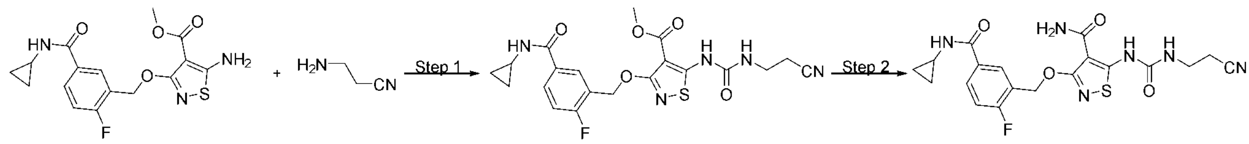


I376 I557 T162

Step 1

Methyl 5-(2-cyanoethylcarbamoylamino)-3-[[5-(cyclopropylcarbamoyl)-2-fluorophenyl]methoxy]isothiazole-4-carboxylate

But-3-en-1-amine (227 mg, 3.28 mmol) was dissolved in tetrahydrofuran (10 mL), followed by the addition of N,N'-carbonyl diimidazole (473 mg, 3.28 mmol) under the protection of nitrogen, and the mixture was stirred at 25 °C to react for 1 hour. Dimethyl sulfoxide (10 mL) was added, the pressure was reduced to remove tetrahydrofuran, followed by the addition of anhydrous potassium carbonate (1.1 g, 8.21 mmol) and methyl 5-amino-3-[[5-(cyclopropylcarbamyl)-2-fluoro-phenyl]methoxy]isothiazol-4-carboxylate I376 (1 g, 2.74 mmol), and the reaction system was stirred at 25 °C to react for 1 hour. Water (100 mL) was added to the reaction mixture, which was then subjected to liquid separation and extraction with ethyl acetate (50 mLx3). The organic phases were combined, washed with saturated brine (100 mL), dried with anhydrous sodium sulfate, and concentrated under reduced pressure to obtain a crude product. The crude product was purified by the preparative thin-layer chromatography (dichloromethane /methanol = 10/1) to obtain a yellow solid product methyl 5-(2-cyanoethylcarbamylamino)-3-[[5-(cyclopropylcarbamyl)-2-fluoro-phenyl]methoxy]isothiazol-4-carboxylate I557 (600 mg, 1.30 mmol, yield: 47.51%).
The product was verified by LCMS and HNMR.
For MS-ESI, the calculated value was $[M+H]^+$ 462.1, and the measurement was 462.0.
$^1H$ NMR (400MHz, $CDCl_3$): δ (ppm) 10.52 (s, 1H), 8.09 (d, *J* = 4.8 Hz, 1H), 7.70-7.66 (m, 1H), 7.48-7.41 (m, 1H), 7.10 (t, *J* = 8.6 Hz, 1H), 6.33 (s, 1H), 5.49 (s, 2H), 3.92 (s, 3H), 3.59-3.55 (m, 2H), 2.92-2.89 (m, 1H), 2.68 (t, *J* = 6.2 Hz, 2H), 0.91-0.86 (m, 2H), 0.65-0.61 (m, 2H).

Step 2

5-(2-cyanoethylcarbamoylamino)-3-[[5-(cyclopropylcarbamoyl)-2-fluorophenyl]methoxy]isothiazole-4-carboxamide

Methyl 5-(2-cyanoethylcarbamylamino)-3-[[5-(cyclopropylcarbamyl)-2-fluorophenyl]methoxy]isothiazol-4-carboxylate I557 (500 mg, 1.08 mmol) was dissolved in the solution of ammonia in methanol (6 mL, 7mol/L), and the mixture was stirred at 40 °C to react for 16 hours. The reaction mixture was concentrated under reduced pressure to obtain a crude product. The crude product was purified by the high-pressure preparative chromatography to obtain a white solid product 5-(2-cyanoethylcarbamylamino)-3-[[5-(cyclopropylcarbamyl)-2-fluorophenyl]methoxy]isothiazol-4-formamide T162 (7.2 mg, 16.13 μmol, yield: 1.49%)

The product was verified by LCMS, H-NMR and F-NMR.

For MS-ESI, the calculated value was $[M+H]^+$ 447.1, and the measurement was 447.0.

$^1$H NMR (400MHz, $CD_3OD$&$CDCl_3$): δ (ppm) 8.00 (d, *J* = 6.0 Hz, 1H), 7.90-7.81 (m, 1H), 7.24 (t, *J* = 9.2 Hz, 1H), 5.57 (s, 2H), 3.49 (t, *J* = 6.2 Hz, 2H), 2.86-2.80 (m, 1H), 2.71 (t, *J* = 6.2 Hz, 2H), 0.84-0.76 (m, 2H), 0.66-0.60 (m, 2H).

$^{19}$F NMR (400MHz, $CD_3OD$&$CDCl_3$): δ (ppm) -116.92.

Example 134: Synthesis of Compound T163

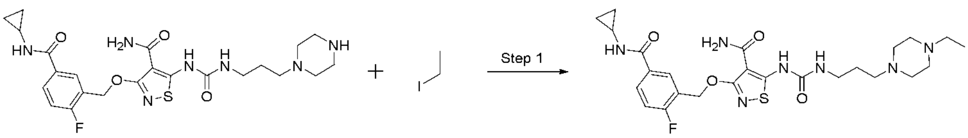


T153 T163

3-[[5-(cyclopropylcarbamoyl)-2-fluoro-phenyl]methoxy]-5-[3-(4-ethylpiperazin-1-yl)propylcarbamoylamino]isothiazole-4-carboxamide

At room temperature, 3-[[5-(cyclopropylcarbamyl)-2-fluoro-phenyl]methoxy]-5-(3-piperazin-1-ylpropylcarbamylamino)isothiazol-4-formamide T153 (300 mg, 577.38 μmol) was dissolved in 1,4-dioxane (5 mL), followed by the addition of iodoethane (108 mg, 0.69 mmol), potassium iodide (144 mg, 0.87 mmol) and cesium carbonate (376 mg, 1.15 mmol). The reaction system was stirred at 30°C to react for 3 h. The reaction mixture was concentrated under reduced pressure to obtain a crude product. The crude product was purified by the high-pressure preparative liquid chromatography to obtain a white solid product 3-[[5-(cyclopropylcarbamyl)-2-fluorophenyl]methoxy]-5-[3-(4-ethylpiperazin-1-yl)propylcarbamylamino]isothiazol-4-formamide T163 (35.29 mg, 64.44 μmol, yield: 11.16%).

The product was verified by LCMS, H-NMR, C-NMR and F-NMR.

For MS-ESI, the calculated value was $[M+H]^+$ 548.2, and the measurement was 548.2.

$^1$H NMR (400MHz, $CD_3OD$): δ (ppm) 8.00 (dd, *J* =6.8, 2.0Hz, 1H), 7.87-7.83 (m, 1H), 7.24 (t, *J* = 9.2 Hz, 1H), 5.57 (s, 2H), 3.28-3.25(m, 2H), 2.85-2.81 (m, 1H), 2.76-2.40 (m, 12H), 1.76-1.73(m, 2H), 1.09 (t, *J* = 7.2 Hz, 3H), 0.82-0.77 (m, 2H), 0.64-0.61 (m, 2H).

$^{13}$C NMR (100 MHz, $CD_3OD$) δ (ppm) 170.38, 170.17, 167.14, 165.67, 163.16, 162.98, 156.11, 131.57, 131.52, 131.17, 131.08, 124.92, 116.84, 116.62, 98.73, 65.17, 39.56, 27.69, 24.12, 11.81, 6.59.

$^{19}$F NMR (400MHz, $CD_3OD$): δ (ppm) -115.45.

Example 135: Synthesis of Compound T164

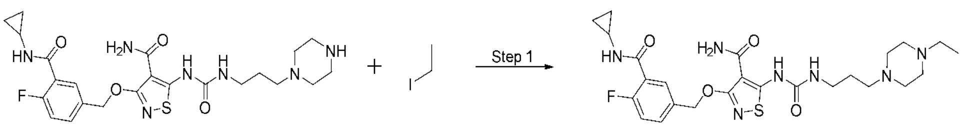


T160 T164

3-[[3-(cyclopropylcarbamoyl)-4-fluoro-phenyl]methoxy]-5-[3-(4-ethylpiperazin-1-yl)propylcarbamoylamino]isothiazole-4-carboxamide

At room temperature, 3-[[3-(cyclopropylcarbamyl)-4-fluoro-phenyl]methoxy]-5-(3-piperazin-1-ylpropylcarbamylamino)isothiazol-4-formamide T160 (120 mg, 0.23 mmol) was dissolved in 1,4-dioxane (5 mL), followed by the addition of iodoethane (54 mg, 0.35 mmol), potassium iodide (19 mg, 0.12 mmol) and cesium carbonate (151 mg, 0.46 mmol). The reaction system was stirred at 25°C to react for 3 h. The reaction mixture was concentrated under reduced pressure to obtain a crude product. The crude product was purified by the high-pressure preparative liquid chromatography to obtain a white solid product 3-[[3-(cyclopropylcarbamyl)-4-fluorophenyl]methoxy]-5-[3-(4-ethylpiperazin-1-yl)propylcarbamylamino]isothiazol-4-formamide

T164 (15.21 mg, 27.77 μmol, yield: 12.03%).

The product was verified by LCMS, H-NMR, C-NMR and F-NMR.

For MS-ESI, the calculated value was $[M+H]^+$ 548.2, and the measurement was 548.2.

$^1$H NMR (400MHz, $CD_3OD$): δ (ppm) 7.78 (dd, *J* = 7.2, 2.4 Hz, 1H), 7.65-7.62 (m, 1H), 7.21 (dd, *J*= 10.0, 8.4 Hz, 1H), 5.46 (s, 2H), 3.26 (t, *J*= 6.8 Hz, 2H), 2.89-2.83 (m, 1H), 2.72-2.40 (m, 11H), 1.77-1.70(m, 2H), 1.09 (t, *J* = 7.2 Hz, 3H), 0.83-0.78 (m, 2H), 0.64-0.60 (m, 2H).

$^{13}$C NMR (100 MHz, $CD_3OD$) δ (ppm) 170.32, 168.13, 167.20, 163.15, 162,28, 159.79, 156.10, 134.13, 134.04, 131.42, 131.39, 124.64, 117.62, 117.38, 101.41, 98.79, 70.06, 27.68, 24.03, 11.78, 6.66.

$^{19}$F NMR (400MHz, $CD_3OD$): δ (ppm) -116.84.

Example 136: Synthesis of Compound T165

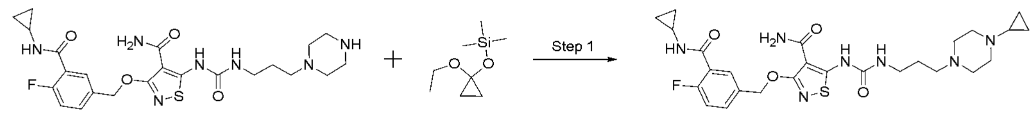


T160

T165

3-[[3-(cyclopropylcarbamoyl)-4-fluoro-phenyl]methoxy]-5-[3-(4-cyclopropylpiperazin-1-yl)propylcarbamoylamino]isothiazole-4-carboxamide

At room temperature, 3-[[3-(cyclopropylcarbamyl)-4-fluoro-phenyl]methoxy]-5-(3-piperazin-1-ylpropylcarbamylamino)isothiazol-4-formamide T160 (100 mg, 0.12 mmol) was dissolved in methanol (2 mL), followed by the addition of (1-ethoxycyclopropoxy)-trimethylsilane (151 mg, 0.87 mmol), glacial acetic acid (116 mg, 1.92 mmol) and sodium borohydride acetate (42 mg, 0.67 mmol). The reaction system was stirred at 25°C to react for 0.5 h. The reaction system was stirred at 60°C to react for 2.5 h. The reaction mixture was poured into water (10 mL), regulated with 2N sodium hydroxide aqueous solution to the pH of 8-9, and extracted with ethyl acetate (10 mL x 3), and the organic phases were combined, washed with saturated brine (30 mL), dried with anhydrous sodium sulfate, and concentrated under reduced pressure to obtain a crude product. The crude product was purified by the high-pressure preparative liquid chromatography to obtain a white solid product 3-[[3-(cyclopropylcarbamyl)-4-fluoro-phenyl]methoxy]-5-[3-(4-cyclopropylpiperazin-1-yl)propylcarbamylamino]isothiazol-4-formamide T165 (31.09 mg, 55.55 μmol, yield: 28.86%).

The product was verified by LCMS, H-NMR, C-NMR and F-NMR.

For MS-ESI, the calculated value was $[M+H]^+$ 560.2, and the measurement was 560.2.

$^1$H NMR (400MHz, $CD_3OD$): δ (ppm) 7.79 (dd, *J*= 6.8, 2.4 Hz, 1H), 7.65-7.61 (m, 1H), 7.21 (dd, *J*= 10.4, 8.4 Hz, 1H), 5.46 (s, 2H), 3.25 (t, *J* = 6.8 Hz, 2H), 2.89-2.83 (m, 1H), 2.66 (s, 4H), 2.47-2.39 (m, 6H), 1.77-1.70 (m, 2H), 1.68-1.63 (m, 1H), 0.83-0.78 (m, 2H), 0.65-0.61 (m, 2H), 0.50-0.45 (m, 2H), 0.40-0.37 (m, 2H).

$^{13}$C NMR (101 MHz, $CD_3OD$) δ170.30, 168.10, 167.19, 163.15, 162,28, 159.79, 156.08, 134.14, 134.05, 131.44, 131.41, 124.61, 117.63, 117.40, 101.41, 98.79, 70.08, 39.57, 39.46, 27.68, 24.05, 6.69, 5.79.

$^{19}$F NMR (400MHz, $CD_3OD$): δ (ppm) -116.74.

Example 137: Synthesis of Compound T166

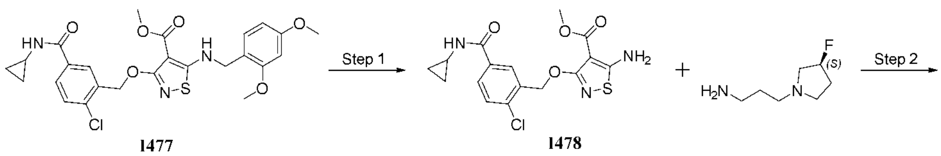


I477 I478

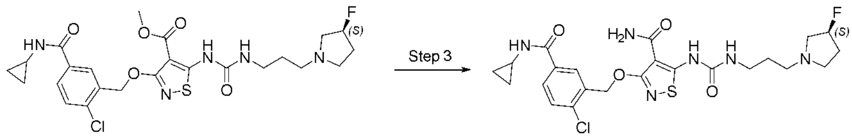


I558 T166

Step 1

Methyl 5-amino-3-[[2-chloro-5-(cyclopropylcarbamoyl)phenyl]methoxy]isothiazole-4-carboxylate

At room temperature, methyl 3-[[2-chloro-5-(cyclopropylcarbamyl)phenyl]methoxy]-5-[(2,4-dimethoxyphenyl) methylamino]isothiazol-4-carboxylate I477 (1.7 g, 3.20 mmol) was dissolved in dichloromethane (20 mL), followed by the addition of trifluoroacetic acid (1.8 g, 15.98 mmol, 1.2 mL). The reaction system was stirred at 30°C to react for 1 h. The reaction mixture was regulated with 2 mol/L sodium hydroxide solution to the pH of 8 for solid precipitation and then filtered, and the filter cake was dried to obtain a yellow solid product methyl 5-amino-3-[[2-chloro-5-(cyclopropylcarbamyl)phenyl] methoxy]isothiazol-4-carboxylate I478 ( 1 g, 2.62 mmol, yield: 81.96%).

The product was verified by LCMS and H-NMR.

For MS-ESI, the calculated value was $[M+H]^+$ 382.1, and the measurement was 382.0.

$^1H$ NMR (400MHz, DMSO_$d_6$): δ (ppm) 8.54 (d, *J* =4.0 Hz, 1H), 8.17 (s, 1H), 7.96 (s, 2H), 7.78 (dd, *J* = 8.0, 1.6 Hz, 1H), 7.57 (d, *J* = 8.0 Hz, 1H), 5.39 (s, 2H), 3.79 (s, 3H), 2.88-2.83 (m, 1H), 0.72-0.68 (m, 2H), 0.58-0.55 (m, 2H).

Step 2

Methyl 3-[[2-chloro-5-(cyclopropylcarbamoyl)phenylmethoxy]-5-[3-[(3S)-3-fluoropyrrolidin-1-yl]propylcarbamoylamino] isothiazole-4-carboxylate

At room temperature, (S)-3-(3-fluoropyrrolidin-1-yl)propyl-1-amine (287 mg, 1.96 mmol) was dissolved in tetrahydrofuran (5 mL), followed by the addition of N,N'-carbonyl diimidazole (318 mg, 1.96 mmol). The reaction system was stirred at 30°C to react for 1 h. The reaction mixture was subjected to rotary evaporation under reduced pressure, and then dissolved in dimethyl sulfoxide (5 mL), followed by the addition of methyl 5-amino-3-[[2-chloro-5-(cyclopropylcarbamyl)phenyl]methoxy]isothiazol-4-carboxylate I478 (500 mg, 1.31 mmol) and potassium carbonate (181 mg, 1.31 mmol). The reaction system was stirred at 30°C to react for 1 h. The reaction mixture was poured into water (20 mL) and extracted with ethyl acetate (30 mL x 3), and the organic phases were combined, washed with saturated brine (20 mL), dried with anhydrous sodium sulfate, and concentrated under reduced pressure to obtain a crude product. The crude product was purified by chromatographic columns ( petroleum ether/ethyl acetate = 5/1-1/1) to obtain a white solid product methyl 3-[[2-chloro-5-(cyclopropylcarbamyl)phenyl]methoxy]-5-[3-[(3S)-3-fluoropyrrolidin-1-yl]propylcarbamylamino] isothiazol-4-carboxylate I558 ( 300 mg, 541.48 μmol, yield: 41.35%).

The product was verified by LCMS and H-NMR.

For MS-ESI, the calculated value was $[M+H]^+$ 554.2, and the measurement was 554.0.

$^1H$ NMR (400MHz, DMSO_$d_6$): δ (ppm) 10.44 (s, 1H), 8.55 (d, *J* = 4.0 Hz, 1H), 8.22-8.17 (m, 3H), 7.79 (d, *J* = 8.8 Hz, 1H), 7.65 (s, 1H), 7.58 (d, *J* = 8.4 Hz, 1H), 7.02 (s, 2H), 5.42 (s, 2H), 5.26-5.12(m, 2H), 3.90 (s, 3H), 2.87-2.76 (m, 5H), 2.28-2.26 (m, 1H), 1.66-1.61 (m, 2H), 0.71-0.68 (m, 2H), 0.59-0.57 (m, 2H).

Step 3

3-[[2-chloro-5-(cyclopropylcarbamoyl)phenyl]methoxy]-5-[3-[(3S)-3-fluoropyrrolidin-1-yl]propylcarbamoylamino]isothiazole-4-carboxamide

At room temperature, methyl 3-[[2-chloro-5-(cyclopropylcarbamyl)phenyl]methoxy]-5-[3-[(3S)-3-fluoropyrrolidin-1-yl]propylcarbamylamino]isothiazol-4-carboxylate I558 (300 mg, 541.48 μmol) was dissolved in the solution of

ammonia methanol (5 mL). The reaction system was stirred at 40°C to react for 16 h. The reaction mixture was concentrated under reduced pressure to obtain a crude product. The crude product was purified by the high-pressure preparative liquid chromatography to obtain a white solid product 3-[[2-chloro-5-(cyclopropylcarbamyl)phenyl]methoxy]-5-[3-[(3S)-3-fluoropyrrolidin-1-yl]propylcarbamylamino]isothiazol-4-formamide T166 ( 3.85 mg, 7.14 μmol, yield: 1.32%). The product was verified by LCMS, H-NMR and F-NMR.

For MS-ESI, the calculated value was $[M+H]^+$ 539.2, and the measurement was 539.1.

$^1$H NMR (400MHz, $CD_3OD$): δ (ppm) 7.99 (d, *J* = 2.0 Hz, 1H), 7.78 (dd, *J* = 8.4, 2.0 Hz, 1H), 7.55 (d, *J*= 8.4 Hz, 1H), 5.61 (s, 2H), 5.25-5.22 (m, 1H), 3.29-3.26 (m, 2H), 3.01-2.89 (m, 2H), 2.85-2.81 (m, 1H), 2.73-2.64 (m, 1H), 2.57 (t, *J* = 7.6 Hz, 2H), 2.47-2.41 (m, 1H), 2.24-2.13 (m, 1H), 2.06-1.97 (m, 1H), 1.81-1.73 (m, 2H), 0.82-0.77 (m, 2H), 0.65-0.61 (m, 2H).

$^{19}$F NMR (400MHz, $CD_3OD$): δ (ppm) -169.15.

Example 138: Synthesis of Compound T167

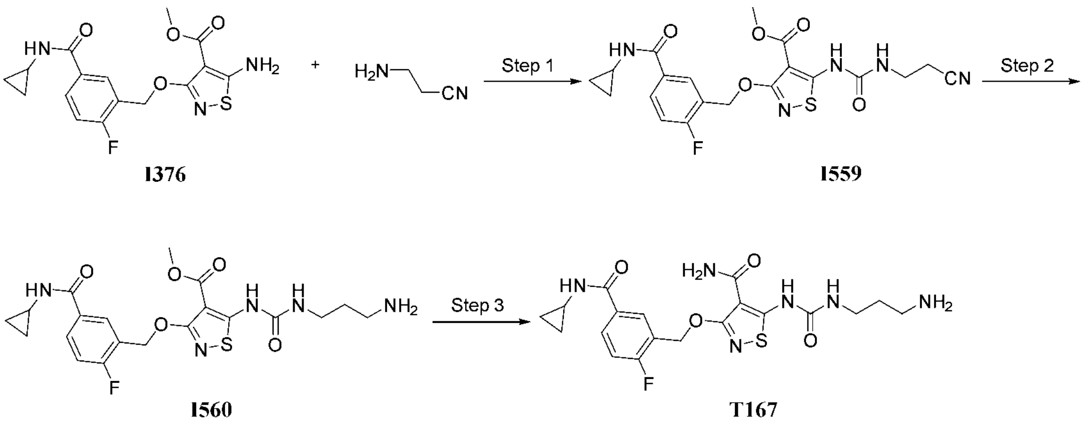


I376 I559 I560 T167

Step 1

Methyl 5-(2-cyanoethylcarbamoylamino)-3-[[5-(cyclopropylcarbamoyl)-2-fluorophenyl]methoxy]isothiazole-4-carboxylate

But-3-en-1-amine (227 mg, 3.28 mmol) was dissolved in tetrahydrofuran (10 mL), followed by the addition of N,N'-carbonyl diimidazole (473 mg, 3.28 mmol) under the protection of nitrogen, and the mixture was stirred at 25 °C to react for 1 hour. Dimethyl sulfoxide (10 mL) was added, the pressure was reduced to remove tetrahydrofuran, followed by the addition of anhydrous potassium carbonate (1.1 g, 8.21 mmol) and methyl 5-amino-3-[[5-(cyclopropylcarbamyl)-2-fluoro-phenyl]methoxy]isothiazol-4-carboxylate I376 (1 g, 2.74 mmol), and the reaction system was stirred at 25 °C to react for 1 hour. Water (100 mL) was added to the reaction mixture, which was then subjected to liquid separation and extraction with ethyl acetate (50 mL x 3). The organic phases were combined, washed with saturated brine (100 mL), dried with anhydrous sodium sulfate, and concentrated under reduced pressure to obtain a crude product. The crude product was purified by the preparative thin-layer chromatography (dichloromethane /methanol = 10/1) to obtain a yellow solid product methyl 5-(2-cyanoethylcarbamylamino)-3-[[5-(cyclopropylcarbamyl)-2-fluoro-phenyl]methoxy]isothiazol-4-carboxylate I559 (600 mg, 1.30 mmol, yield: 47.51%).

The product was verified by LCMS and HNMR.

For MS-ESI, the calculated value was $[M+H]^+$ 462.13, and the measurement was 462.0.

$^1$H NMR (400 MHz, $CDCl_3$) δ (ppm) 8.12-8.06 (m, 1H), 7.78-7.71 (m, 1H), 7.12 (t, *J* = 9.0 Hz, 1H), 5.49 (s, 2H), 4.02 (s, 3H), 3.55 (t, *J*= 6.4 Hz, 2H), 3.38-3.32 (m, 1H), 2.66 (s, 2H), 0.88-0.83 (m, 2H), 0.66-0.62 (m, 2H).

Step 2

Methyl 5-(3-aminopropylcarbamoylamino)-3-[[5-(cyclopropylcarbamoyl)-2-fluorophenyl]methoxy]isothiazole-4-carboxylate

Methyl 5-(2-cyanoethylcarbamylamino)-3-[[5-(cyclopropylcarbamyl)-2-fluorophenyl]methoxy]isothiazol-4-carboxylate I559 (200 mg, 433.40 μmol) was dissolved in methanol (3 mL), followed by the addition of raney nickel (25

mg) at room temperature, and the reaction system was stirred at 25°C to react for 5 hours. The reaction mixture was filtered with diatomite, and the filtrate was concentrated under reduced pressure to obtain a yellow solid product methyl 5-(3-aminopropylcarbamylamino)-3-[[5-(cyclopropylcarbamyl)-2-fluorophenyl]methoxy]isothiazol-4-carboxylate I560 (160 mg, 343.72 μmol, yield: 79.31%).

The product was verified by LCMS.

For MS-ESI, the calculated value was $[M+H]^+$ 466.16, and the measurement was 466.0.

Step 3

5-(3-aminopropylcarbamoylamino)-3-[[5-(cyclopropylcarbamoyl)-2-fluorophenyl]methoxy]isothiazole-4-carboxamide

Methyl 5-(3-aminopropylcarbamylamino)-3-[[5-(cyclopropylcarbamyl)-2-fluorophenyl]methoxy]isothiazol-4-carboxylate I560 (200 mg, 429.65 μmol) was dissolved in the solution of ammonia in methanol (5 mL), and the mixture was stirred at 40°C to react for 16 hours. The reaction mixture was concentrated under reduced pressure to obtain a crude product. The crude product was purified by the high-pressure preparative chromatography to obtain a yellow solid product 5-(3-aminopropylcarbamylamino)-3-[[5-(cyclopropylcarbamyl)-2-fluorophenyl]methoxy]isothiazol-4-formamide T167 (14.81 mg, 32.88 μmol, yield: 7.65%)

The product was verified by LCMS, H-NMR, C-NMR and F-NMR.

For MS-ESI, the calculated value was $[M+H]^+$ 451.16, and the measurement was 450.9.

$^1$H NMR (400MHz, $CD_3OD$&$CDCl_3$): δ (ppm) 8.00 (d, *J* = 6.0 Hz, 1H), 7.88-8.82 (m, 1H), 7.22 (t, *J= 9.2* Hz, 1H), 5.56 (s, 2H), 3.31-3.27(m, 2H), 2.85-2.81 (m, 1H), 2.73 (t, *J* = 7.0 Hz, 2H), 1.75-1.69 (m, 2H), 0.83-0.78 (m, 2H), 0.65-0.62 (m, 2H).

$^{13}$C NMR (100MHz, $CD_3OD$&$CDCl_3$): δ (ppm) 170.41, 170.27, 167.20, 165.72, 163.20, 163.00, 156.33, 132.09, 132.05, 131.63, 131.59, 131.21, 131.12,125.00, 124.85, 116.90, 116.67, 65.20, 39.58, 38.50, 33.29, 24.13, 6.66.

$^{19}$F NMR (400MHz, $CD_3OD$&$CDCl_3$): δ (ppm) -115.06.

Example 139: Synthesis of Compound T168

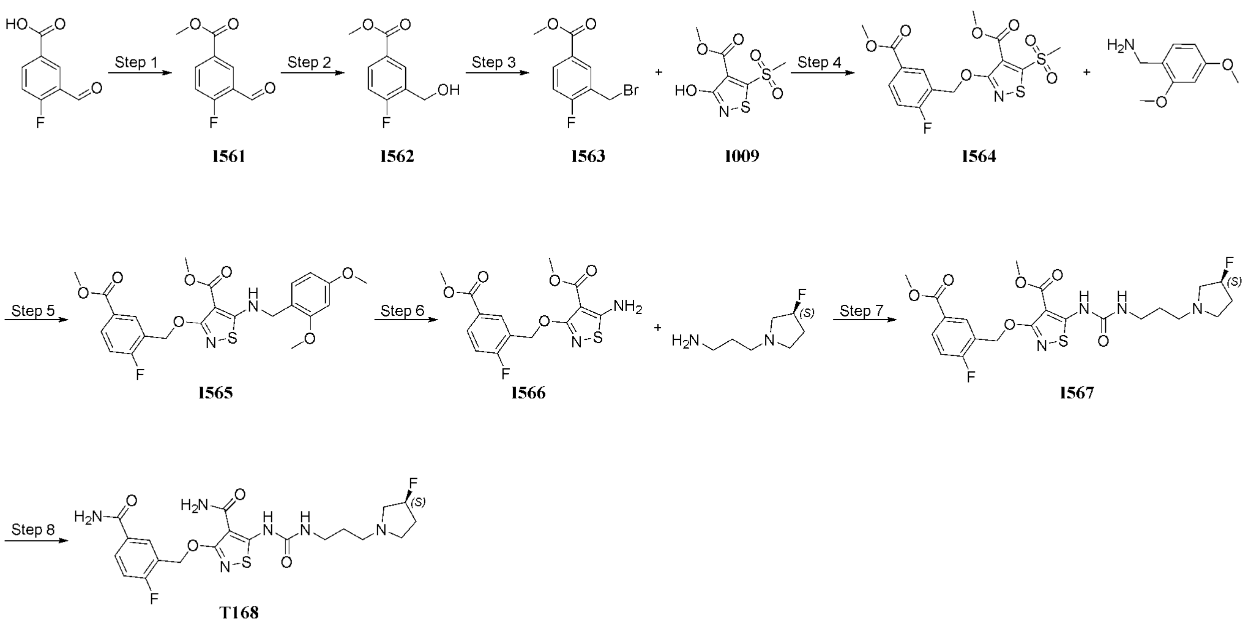


I561 I562 I563 I009 I564 I565 I566 I567 T168

Step 1

Methyl 4-fluoro-3-formyl-benzoate

At room temperature, 4-fluoro-3-formylbenzoic acid (1.5 g, 8.92 mmol) was dissolved in methanol (15 mL), followed by the addition of concentrated sulfuric acid (875 mg, 8.92 mmol). The reaction system was stirred at 70°C to react for 16 h. The reaction mixture was poured into water (30 mL) and extracted with ethyl acetate (10 mL x 3), and the organic

phases were combined, washed with saturated brine (30 mL), dried with anhydrous sodium sulfate, and concentrated under reduced pressure to obtain a colorless oily crude product was 4-fluoro-3-formylmethyl benzoateI561 (1 g, 5.49 mmol, yield: 61.53%).

The product was verified by H-NMR.

$^1$H NMR (400MHz, $CDCl_3$): δ (ppm) 10.37 (s, 1H), 8.57 (dd, *J* = 6.8, 2.4 Hz, 1H), 8.33-8.29 (m, 1H), 7.29-7.25 (m, 1H), 3.95 (s, 3H).

Step 2

Methyl 4-fluoro-3-(hydroxymethyl)benzoate

At room temperature, 4-fluoro-3-formylmethyl benzoateI561 (800 mg, 4.39 mmol) was dissolved in methanol (10 mL), followed by the addition of sodium borohydride (249 mg, 6.59 mmol). The reaction system was stirred at 0°C to react for 1 h. The reaction mixture was pouredi into saturated ammonium chloride aqueous solution (30 mL) and extracted with ethyl acetate (10 mL x 3), and the organic phases were combined, washed with saturated brine (30 mL), dried with anhydrous sodium sulfate, and concentrated under reduced pressure to obtain a colorless oily crude product was 4-fluoro-3-(hydroxymethyl)methyl benzoateI562 (800 mg, 4.34 mmol, 98.91% yield).

The product was verified by LCMS and H-NMR.

For MS-ESI, the calculated value was $[M+H]^+$ 185.1, and the measurement was 185.0.

$^1$H NMR (400MHz, $CDCl_3$): δ (ppm) 8.17 (dd, *J* = 7.2, 2.0 Hz, 1H), 8.01-7.97 (m, 1H), 7.11 (t, *J* = 9.2 Hz, 1H), 4.80 (s, 2H), 3.92 (s, 3H).

Step 3

Methyl 3-(bromomethyl)-4-fluoro-benzoate

At room temperature, 4-fluoro-3-(hydroxymethyl)methyl benzoateI562 (800 mg, 4.34 mmol) was dissolved in dichloromethane (10 mL), followed by the addition of phosphorus tribromide (1.4 g, 5.21 mmol). The reaction system was stirred at 25°C to react for 1 h. The reaction mixture was poured into water (10 mL) and extracted with dichloromethane (10 mL x 3), and the organic phases were combined, washed with saturated brine (30 mL), dried with anhydrous sodium sulfate, and concentrated under reduced pressure to obtain a white solid crude product was 3-(bromomethyl)-4-fluoro-methyl benzoateI563 (800 mg, 3.24 mmol, yield: 74.54%).

The product was verified by LCMS and H-NMR.

For MS-ESI, the calculated value was $[M+H]^+$247.0/249.0, and the measurement was 247.0/248.9. $^1$H NMR (400MHz, $CDCl_3$ ): δ (ppm) 8.12 (dd, *J* =7.2, 2.2 Hz, 1H), 8.74-8.04 (m, 1H), 7.17-7.13 (m, 1H), 4.52 (s, 1H), 3.92 (s, 1H).

Step 4

Methyl 3-[(2-fluoro-5-methoxycarbonyl-phenyl)methoxy]-5-methylsulfonyl-isothiazole-4-carboxylate

At room temperature, methyl 3-hydroxyl-5-methylsulfonyl-isothiazol-4-carboxylate I009 (600 mg, 2.53 mmol) was dissolved in N,N-dimethylformamide (10 mL), followed by the addition of 3-(bromomethyl)-4-fluoro-methyl benzoateI563 (687 mg, 2.78 mmol) and potassium carbonate (1.1 g, 7.59 mmol). The reaction system was stirred at 25°C to react for 1 h. The reaction mixture was poured into water (30 mL) and then filtered, and the filter cake was dissolved in ethyl acetate, dried with anhydrous sodium sulfate, and concentrated under reduced pressure to obtain a yellow solid product methyl 3-[(2-fluoro-5-methoxycarbonyl-phenyl)methoxy]-5-methylsulfonyl-isothiazol-4-carboxylate I564 (600 mg, 1.49 mmol, yield: 58.81%).

The product was verified by LCMS and H-NMR.

For MS-ESI, the calculated value was $[M+H]^+$ 404.0, and the measurement was 403.9.

$^1$H NMR (400MHz, DMSO_$d_6$): δ (ppm) 8.25 (dd, *J* = 7.2, 2.4 Hz, 1H), 8.06-8.02 (m, 1H), 7.46-7.42 (m, 1H), 5.60 (s, 2H), 3.91 (s, 3H), 3.87 (s, 3H), 3.63 (s, 3H).

Step 5

Methyl 5-[(2,4-dimethoxyphenyl)methylamino]-3-[(2-fluoro-5-methoxycarbonyl-phenyl)methoxy]isothiazole-4-carboxylate

At room temperature, methyl 3-[(2-fluoro-5-methoxycarbonyl-phenyl)methoxy]-5-methylsulfonyl-isothiazol-4-carboxylate I564 (600 mg, 1.49 mmol) was dissolved in tetrahydrofuran (10 mL), followed by the addition of 2,4-dimetoxybenzenemethylamine (746 mg, 4.46 mmol). The reaction system was stirred at 65°C to react for 1 h. The reaction mixture was poured into water (30 mL) and then filtered, and the filter cake was dissolved in ethyl acetate, dried with anhydrous sodium sulfate, and concentrated under reduced pressure to obtain a yellow solid product methyl 5-[(2,4-dimethoxyphenyl)methylamino]-3-[(2-fluoro-5-methoxycarbonylphenyl)methoxy]isothiazol-4-carboxylate I565 (600 mg, 1.22 mmol, yield: 82.24%).

The product was verified by LCMS and H-NMR.

For MS-ESI, the calculated value was $[M+H]^+$ 491.1, and the measurement was 491.0.

$^1$H NMR (400MHz, DMSO_$d_6$): δ (ppm) 8.46 (t, *J* = 5.6 Hz, 1H), 8.27 (dd, *J* = 6.8, 2.0 Hz, 1H), 8.01-7.97 (m, 1H), 7.39 (t, *J*= 9.6 Hz, 1H), 7.17 (d, *J*= 8.4 Hz, 1H), 6.60 (d, *J* = 2.0 Hz, 1H), 5.42 (s, 2H), 4.29 (d, *J* = 6.0 Hz, 2H), 3.87 (s, 3H), 3.83 (s, 3H), 3.79 (s, 3H), 3.75 (s, 3H).

Step 6

Methyl 5-amino-3-[(2-fluoro-5-methoxycarbonyl-phenyl)methoxy]isothiazole-4-carboxylate

At room temperature, methyl 5-[(2,4-dimethoxyphenyl)methylamino]-3-[(2-fluoro-5-methoxycarbonylphenyl) methoxy]isothiazol-4-carboxylate I565 (500 mg, 1.02 mmol) was dissolved in dichloromethane (5 mL), followed by the addition of trifluoroacetic acid (581 mg, 5.10 mmol). The reaction system was stirred at 25°C to react for 3 h. The reaction mixture was regulated with 2N sodium hydroxide solution to the pH of 8 for solid precipitation and then filtered, and the filter cake was dried to obtain a white solid crude product methyl 5-amino-3-[(2-fluoro-5-methoxycarbonyl-phenyl)methoxy] isothiazol-4-carboxylate I566 (340 mg, 999.04 μmol, yield: 98.01%).

The product was verified by LCMS and H-NMR.

For MS-ESI, the calculated value was $[M+H]^+$ 341.1, and the measurement was 341.0.

$^1$H NMR (400MHz, DMSO_$d_6$): δ (ppm) 8.28 (dd, *J*= 7.2, 2.0 Hz, 1H), 8.01-7.96 (m, 3H), 7.40 (t, *J*= 9.6 Hz, 1H), 5.42 (s, 2H), 3.87 (s, 3H), 3.77 (s, 3H).

Step 7

Methyl 3-[(2-fluoro-5-methoxycarbonyl-phenyl)methoxy]-5-[3-[(3S)-3-fluoropyrrolidin-1-yl]propylcarbamoylamino]isothiazole-4-carboxylate

3-[(3S)-3-fluoropyrrolidin-1-yl]propyl-1-amine (206 mg, 1.41 mmol) was dissolved in tetrahydrofuran (5 mL), followed by the addition of N,N'-carbonyl diimidazole (229 mg, 1.41 mmol) under the protection of $N_2$, and the mixture was stirred at 25°C to react for 1 hour. Dimethyl sulfoxide (5 mL) was added, the pressure was reduced to remove tetrahydrofuran, followed by the addition of methyl 5-amino-3-[(2-fluoro-5-methoxycarbonyl-phenyl)methoxy]isothiazol-4-carboxylate I566 (400 mg, 1.18 mmol) and potassium carbonate (162 mg, 1.18 mmol), and the reaction system was stirred at 25°C to react for 1 hour. Water (10 mL) was added to the reaction mixture, which was then subjected to liquid separation and extraction with ethyl acetate (10 mLx3). The organic phases were combined, washed with saturated brine (20 mL), dried with anhydrous sodium sulfate, and concentrated under reduced pressure to obtain a crude product. The crude product was purified by the preparative thin-layer chromatography (dichloromethane /methanol = 10/1) to obtain a yellow solid product methyl 3-[(2-fluoro-5-methoxycarbonyl-phenyl)methoxy]-5-[3-[(3S)-3-fluoropyrrolidin-1-yl]propylcarbamylamino]isothiazol-4-carboxylate I567 (200mg, 390.22 μmol, yield: 33.20%).

The product was verified by LCMS.

For MS-ESI, the calculated value was $[M+H]^+$ 513.2, and the measurement was 513.0.

Step 8

3-[(5-carbamoyl-2-fluoro-phenyl)methoxy]-5-[3-[(3S)-3-fluoropyrrolidin-1-yl]propylcarbamoylamino]isothiazole-4-carboxamide

At room temperature, methyl 3-[(2-fluoro-5-methoxycarbonyl-phenyl)methoxy]-5-[3-[(3S)-3-fluoropyrrolidin-1-yl]propylcarbamylamino]isothiazol-4-carboxylate I567 (200 mg, 390.22 μmol) was dissolved in the solution of ammonia in methanol (2 mL, 7mol/L). The reaction system was stirred at 60°C to react for 16 h. The reaction mixture was concentrated under reduced pressure to obtain a crude product. The crude product was purified by the high-pressure preparative liquid chromatography to obtain a white solid product 3-[(5-carbamyl-2-fluoro-phenyl)methoxy]-5-[3-[(3S)-3-fluoropyrrolidin-1-yl]propylcarbamylamino]isothiazol-4-formamide T168 (4.99 mg, 10.34 μmol, yield: 2.65%).

The product was verified by LCMS, H-NMR and F-NMR.

For MS-ESI, the calculated value was $[M+H]^+$ 483.2, and the measurement was 483.0.

$^1H$ NMR (400MHz, $CD_3OD$): δ (ppm) 8.08 (dd, *J* = 7.2, 2.4 Hz, 1H), 7.95-7.91 (m, 1H), 7.26 (t, *J* = 9.2 Hz, 1H), 5.58 (s, 2H), 5.25-5.08 (m, 1H), 3.27 (t, *J* = 6.8 Hz, 2H), 3.00-2.88 (m, 2H), 2.72-2.54 (m, 3H), 2.45-2.39 (m, 1H), 2.27-2.11(m, 1H), 2.07-1.94 (m, 1H), 1.80-1.73 (m, 2H).

$^{19}F$ NMR (400MHz, $CD_3OD$): δ (ppm) -115.14, -169.06.

Example 140: Synthesis of Compound T169

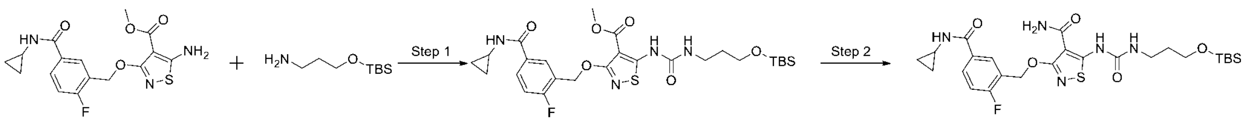


I376 I568 T169

Step 1

Methyl 5-[3-[tert-butyl(dimethyl)silyl]oxypropylcarbamoylamino]-3-[[5-(cyclopropylcarbamoyl)-2-fluoro-phenyl]methoxy]isothiazole-4-carboxylate

At room temperature, 3-[tert-butyl(dimethyl)silicyl]oxypropyl-1-amine (389 mg, 2.05 mmol) was dissolved in tetrahydrofuran (5 mL), followed by the addition of N,N'-carbonyl diimidazole (333 mg, 2.05 mmol). The reaction system was stirred at 30°C to react for 1 h. The mixture was spun under reduced pressure to remove the solvent, and then dissolved in dimethyl sulfoxide (5 mL), followed by the addition of methyl 5-amino-3-[[5-(cyclopropylcarbamyl)-2-fluorophenyl]methoxy]isothiazol-4-carboxylate I376 (500 mg, 1.37 mmol) and potassium carbonate (567 mg, 4.11 mmol). The reaction system was stirred at 30°C to react for 1 h. The reaction mixture was poured into water (10 mL) and extracted with ethyl acetate (10 mL x 3), and the organic phases were combined, washed with saturated brine (30 mL), dried with anhydrous sodium sulfate, and concentrated under reduced pressure to obtain a crude product. The crude product was purified by chromatographic columns (petroleum ether/ethyl acetate = 5/1-1/1) to obtain a white solid product methyl 5-[3-[tert-butyl(dimethyl)silicyl]oxypropylcarbamylamino]-3-[[5-(cyclopropylcarbamyl)-2-fluoro-phenyl]methoxy]isothiazol-4-carboxylate I568 ( 300 mg, 516.58 μmol, yield: 37.75%). The product was verified by LCMS and H-NMR.

For MS-ESI, the calculated value was $[M+H]^+$ 581.2, and the measurement was 581.1.

$^1H$ NMR (400MHz, DMSO_$d_6$): δ (ppm) 10.36 (s, 1H), 8.45 (d, *J* = 4.0 Hz, 1H), 8.11-8.07 (m, 2H), 7.82-7.79 (m, 1H), 7.28 (t, *J* = 9.6 Hz, 1H), 5.37 (s, 2H), 3.80 (s, 3H), 3.60 (t, *J* = 6.4 Hz, 2H), 3.19-3.12 (m, 2H), 2.82-2.78(m, 1H), 1.64-1.58 (m, 2H), 0.83 (s, 9H), 0.68-0.63 (m, 2H), 0.54-0.51 (m, 2H), 0.07 (m, 6H).

Step 2

5-[3-[tert-butyl(dimethyl)silyl]oxypropylcarbamoylamino]-3-[[5-(cyclopropylcarbamoyl)-2-fluoro-phenyl]methoxy]isothiazole-4-carboxamide

At room temperature, methyl 5-[3-[tert-butyl(dimethyl)silicyl]oxypropylcarbamylamino]-3-[[5-(cyclopropylcarbamyl)-2-fluoro-phenyl]methoxy]isothiazol-4-carboxylate I568 (600 mg, 1.03 mmol) was dissolved in the solution of ammonia methanol (6 mL). The reaction system was stirred at 40°C to react for 16 h. The reaction mixture was concentrated under reduced pressure to obtain a crude product. The crude product was purified by the high-pressure preparative chromatography to obtain a white solid product 5-[3-[tert-butyl(dimethyl)silicyl]oxypropylcarbamylamino]-3-[[5-(cyclopropylcarbamyl)-2-fluoro-phenyl]methoxy]isothiazol-4-formamide T169 ( 314.23 mg, 0.56 mmol, yield: 53.76%).

The product was verified by LCMS, H-NMR and F-NMR.

For MS-ESI, the calculated value was [M+H]+566.2, and the measurement was 566.2.

$^1H$ NMR (400MHz, $CD_3OD$): δ (ppm) 7.94 (d, *J*= 6.0 Hz, 1H), 7.83-7.75 (m, 1H), 7.17 (t, *J*= 9.2 Hz, 1H), 5.50 (s, 2H), 3.65 (t, *J* = 6.0 Hz, 2H), 3.25-3.24 (m, 2H), 2.77-2.75 (m, 1H), 1.70-1.67 (m, 2H), 0.84 (s, 9H), 0.73 (d, *J* = 6.0 Hz, 2H), 0.59-0.56 (m, 2H), 0.00 (s, 6H).

$^{19}F$ NMR (400MHz, $CD_3OD$): δ (ppm) -115.59.

Example 141: Synthesis of Compound T170

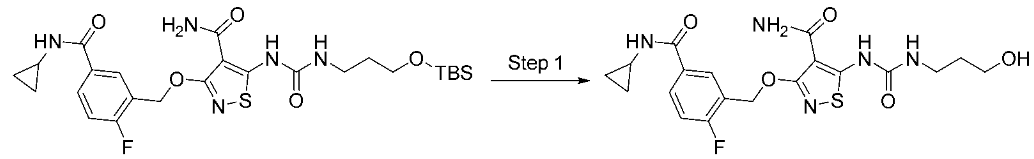


T169 T170

3-[[5-(cyclopropylcarbamoyl)-2-fluoro-phenyl]methoxy]-5-(3-hydroxypropylcarbamoylamino)isothiazole-4-carboxamide

At room temperature, 5-[3-[tert-butyl(dimethyl)silicyl]oxypropylcarbamylamino]-3-[[5-(cyclopropylcarbamyl)-2-fluoro-phenyl]methoxy]isothiazol-4-formamide T169 (50 mg, 88.38 μmol) was dissolved in tetrahydrofuran (1 mL), followed by the addition of tetrabutylammonium fluoride (132.57 μL, 132.57 μmol, 1 mol/L) at 0 °C. The reaction system was stirred at 0°C to react for 1 hour, and then stirred at 30 °C to react for 2 hours. The reaction mixture was concentrated under reduced pressure to obtain a crude product. The crude product was purified by the high-pressure preparative liquid chromatography to obtain a white solid product 3-[[5-(cyclopropylcarbamyl)-2-fluoro-phenyl]methoxy]-5-(3-hydroxypropylcarbamylamino)isothiazol-4-formamide T170( 17.69 mg, 39.18 μmol, yield: 44.33%).

The product was verified by LCMS, H-NMR and F-NMR.

For MS-ESI, the calculated value was $[M+H]^+$ 452.1, and the measurement was 452.1.

$^1$H NMR (400MHz, DMSO_$d_6$): δ (ppm) 11.01 (s, 1H), 8.03 (d, *J*= 5.6 Hz, 1H), 7.91-7.87 (m, 1H), 7.35 (t, *J* = 9.2 Hz, 1H), 5.49 (s, 2H), 4.46 (t, *J* = 5.2 Hz, 1H), 3.44 (dd, *J*= 11.6, 6.0 Hz, 2H), 3.16 (t, *J*= 6.8 Hz, 2H), 2.83-2.80 (m, 1H), 1.62-1.57(m, 2H), 0.71-0.67(m, 2H), 0.57-0.56 (d, *J* = 2.8 Hz, 2H).

$^{19}$F NMR (400MHz, $CD_3OD$): δ (ppm) -117.65.

Example 142: Synthesis of Compound T171

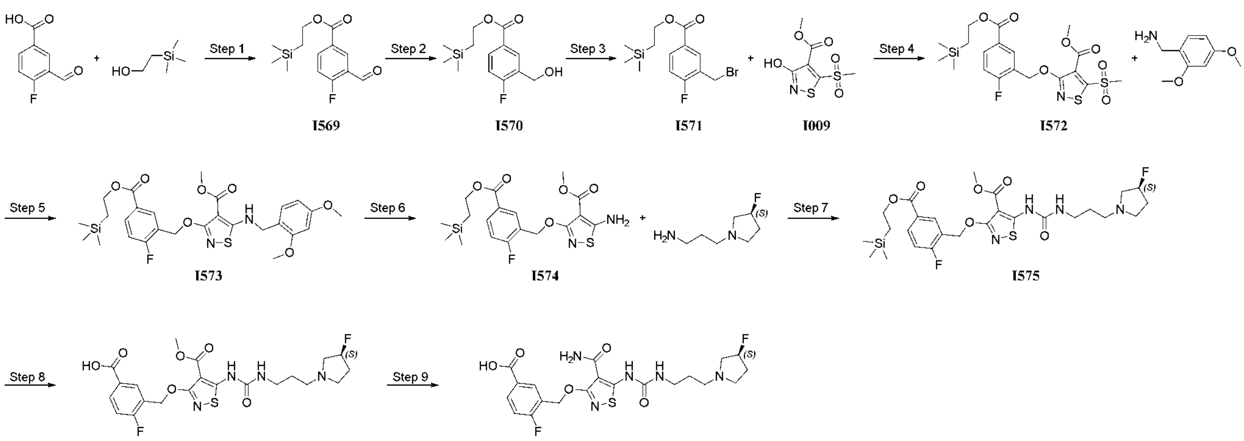


I569 I570 I571 I009 I572 I573 I574 I575 I576 T171

Step 1

2-trimethylsilylethyl 4-fluoro-3-formyl-benzoate

At room temperature, 4-fluoro-3-formylbenzoic acid (2.5 g, 14.87 mmol) was dissolved in dichloromethane (30 mL), followed by the addition of 2-trimethylsilyl ethanol (5.3 g, 44.61 mmol) and 4-dimethylaminopyridine (181 mg, 1.49 mmol). The reaction system was stirred at 25°C to react for 0.5 h. N,N'-dicyclohexylcarbodiimide (3.1 g, 14.87 mmol) was added, and the reaction system was stirred at 25 °C to react for 3 hours. The reaction mixture was filtered, and the filtrate was concentrated under reduced pressure to obtain a crude product. The crude product was purified by chromatographic columns (petroleum ether/ethyl acetate = 100/1-10/1) to obtain a yellow oily product 2-trimethylsilylethyl-4-fluoro-3-formylbenzoateI569 (3.8 g, 14.16 mmol, yield: 95.23%). The product was verified by H-NMR.

$^1H$ NMR (400MHz, $CDCl_3$): δ (ppm) 10.37 (s, 1H), 8.56 (dd, *J*= 6.8, 2.4 Hz, 1H), 8.31-8.27 (m, 1H), 7.26-7.23 (m, 1H), 4.46-4.41 (m, 2H), 1.17-1.11 (m, 2H), 0.09 (s, 9H).

Step 2

2-trimethylsilylethyl 4-fluoro-3-(hydroxymethyl)benzoate

At room temperature, 2-trimethylsilylethyl-4-fluoro-3-formylbenzoateI569 (4.3 g, 16.02 mmol) was dissolved in methanol (50 mL), followed by the addition of sodium borohydride (909 mg, 24.04 mmol). The reaction system was stirred at 0°C to react for 1 h. The reaction mixture was poured into saturated ammonium chloride aqueous solution (200 mL) and extracted with ethyl acetate (100 mL x 3), and the organic phases were combined, washed with saturated brine (300 mL), dried with anhydrous sodium sulfate, and concentrated under reduced pressure to obtain a yellow oily crude product 2-trimethylsilylethyl-4-fluoro-3-(hydroxymethyl)benzoateI570 (4 g, 14.79 mmol, yield: 92.33%).
The product was verified by H-NMR.
$^1H$ NMR (400MHz, $CDCl_3$): δ (ppm) 8.15 (dd, *J*= 7.2, 1.6 Hz, 1H), 8.00-7.96 (m, 1H), 7.09 (m, *J*= *9.2* Hz, 1H), 4.79 (s, 2H), 4.43-4.38 (m, 2H), 1.15-1.11 (m, 2H), 0.08 (s, 9H).

Step 3

2-trimethylsilylethyl 3-(bromomethyl)-4-fluoro-benzoate

At room temperature, 2-trimethylsilylethyl-4-fluoro-3-(hydroxymethyl)benzoateI570 (1 g, 3.70 mmol) was dissolved in dichloromethane (10 mL), followed by the addition of phosphorus tribromide (1.5 g, 5.55 mmol). The reaction system was stirred at 25°C to react for 1 h. The reaction mixture was poured into water (20 mL), and extracted with dichloromethane (10 mL x 3), and the organic phases were combined, washed with saturated brine (30 mL), dried with anhydrous sodium sulfate, and concentrated under reduced pressure to obtain a white solid crude product 2-trimethylsilylethyl-3-(bromomethyl)-4-fluoro-benzoateI571 (1 g, 3.00 mmol, yield: 81.13%).
The product was verified by H-NMR.
$^1H$ NMR (400MHz, $CDCl_3$ ): δ (ppm) 8.15-8.11 (m, 1H), 8.00-7.94 (m, 1H), 7.15-7.06 (m, 1H), 4.48 (d, *J*= 4.4 Hz, 2H), 4.39-4.34 (m, 2H), 1.12-1.07 (m, 2H), 0.05-0.04 (m, 9H).

Step 4

Methyl 3-[[2-fluoro-5-(2-trimethylsilylethoxycarbonyl)phenyl]methoxy]-5-methylsulfonylisothiazole-4-carboxylate

At room temperature, 2-trimethylsilylethyl-3-(bromomethyl)-4-fluoro-benzoateI571 (1 g, 3.00 mmol) was dissolved in N,N-dimethylformamide (10 mL), followed by the addition of methyl 3-hydroxyl-5-methylsulfonyl-isothiazol-4-carboxylate I009(712 mg, 3.00 mmol) and potassium carbonate (1.2 g, 9.00 mmol). The reaction system was stirred at 25°C to react for 1 h. The reaction mixture was poured into water (30 mL) and then filtered, and the filter cake was dissolved in ethyl acetate, dried with anhydrous sodium sulfate, and concentrated under reduced pressure to obtain a yellow solid product 3-[[2-fluoro-5-(2-trimethylsilylethoxycarbonyl)phenyl]methoxy]-5-methylsulfonyl-isothiazol-4-methyl formateI572 (550 mg, 1.12 mmol, yield: 37.44%).
The product was verified by LCMS and H-NMR.
For MS-ESI, the calculated value was $[M+1]^+$490.0, and the measurement was 507.0$[M+1+NH3]^+$. $^1H$ NMR (400MHz, DMSO-$d_6$): δ (ppm) 8.27 (dd, *J* = 6.8, 2.0 Hz, 1H), 8.06-8.03 (m, 1H), 7.19-7.13 (m, 1H), 5.56 (s, 2H), 4.44-4.38 (m, 2H), 3.99 (s, 3H), 3.50 (s, 3H), 1.15-1.11 (m, 2H), 0.08 (s, 9H).

Step 5

Methyl 5-[(2,4-dimethoxyphenyl)methylamino]-3-[[2-fluoro-5-(2-trimethylsilylethoxycarbonyl)phenyl]methoxy]isothiazole-4-carboxylate

At room temperature, 3-[[2-fluoro-5-(2-trimethylsilylethoxycarbonyl)phenyl]methoxy]-5-methylsulfonyl-isothiazol-4-methyl formate I572 (550 mg, 1.12 mmol) was dissolved in tetrahydrofuran (10 mL), followed by the addition of (2,4-dimethoxyphenyl)methylamine (1.9 g, 11.23 mmol). The reaction system was stirred at 65°C to react for 1 h. The reaction mixture was poured into water (30 mL) and then filtered, and the filter cake was dissolved in ethyl acetate, dried with anhydrous sodium sulfate, and concentrated under reduced pressure to obtain a yellow solid product methyl 5-[(2,4-dimethoxyphenyl)methylamino]-3-[[2-fluoro-5-(2-trimethylsilylethoxycarbonyl)phenyl]methoxy]isothiazol-4-carboxylate

I573 (500 mg, 866.99 μmol, yield: 77.18%).

The product was verified by LCMS and H-NMR.

For MS-ESI, the calculated value was $[M+H]^+$ 577.2, and the measurement was 577.1.

$^1$H NMR (400MHz, DMSO_$d_6$): δ (ppm) 8.45 (s, 1H), 8.23 (dd, *J* = 6.8, 2.0 Hz, 1H), 8.01-7.91 (m, 2H), 7.41-7.35 (m, 1H), 7.16 (d, *J*= 8.4 Hz, 1H), 6.59 (d, *J*= 2.0 Hz, 1H), 6.52-6.45 (m, 2H), 5.42 (s, 2H), 4.40-4.34 (m, 2H), 3.82 (s, 3H), 3.78 (s, 3H), 3.75 (s, 3H), 1.10-1.06 (m, 2H), 0.04-0.03 (m, 9H).

Step 6

Methyl 5-amino-3-[[2-fluoro-5-(2-trimethylsilylethoxycarbonyl)phenyl]methoxy]isothiazole-4-carboxylate

At room temperature, methyl 5-[(2,4-dimethoxyphenyl)methylamino]-3-[[2-fluoro-5-(2-trimethylsilylethoxycarbonyl)phenyl]methoxy]isothiazol-4-carboxylate I573 (500 mg, 866.99 μmol) was dissolved in dichloromethane (5 mL), followed by the addition of trifluoroacetic acid (494 mg, 4.33 mmol). The reaction system was stirred at 25°C to react for 3 h. The reaction mixture was regulated with 2 mol/L sodium hydroxide solution to the pH of 8 for soid precipitation and then filtered, and the filter cake was dried to obtain a white solid crude product methyl 5-amino-3-[[2-fluoro-5-(2-trimethylsilylethoxycarbonyl)phenyl]methoxy]isothiazol-4-carboxylate I574 (300 mg, 703.34 μmol, yield: 81.12%).

The product was verified by LCMS and H-NMR.

For MS-ESI, the calculated value was $[M+1]^+$ 427.1, and the measurement was 427.0.

$^1$H NMR (400MHz, DMSO_$d_6$): δ (ppm) 8.24 (dd, *J*= 7.2 Hz, 1H), 7.97-7.93 (m, 2H), 7.41-7.37 (m, 1H), 6.51-6.44 (m, 1H), 5.42 (s, 2H), 4.40-4.36 (m, 2H), 3.76 (s, 3H), 1.11-1.07 (m, 2H), 0.06-0.03 (m, 9H).

Step 7

Methyl 5-[3-[(3S)-3-fluoropyrrolidin-1-yl]propylcarbamoylamino]-3-[[2-fluoro-5-(2-trimethylsilylethoxycarbonyl)phenyl]methoxy]isothiazole-4-carboxylate

3-[(3S)-3-fluoropyrrolidin-1-yl]propyl-1-amine (154 mg, 1.06 mmol) was dissolved in tetrahydrofuran (5 mL), followed by the addition of N,N'-carbonyl diimidazole (171 mg, 1.06 mmol) under the protection of nitrogen, and the mixture was stirred at 25 °C to react for 1 hour. Dimethyl sulfoxide (5 mL) was added, the pressure was reduced to remove tetrahydrofuran, followed by the addition of methyl 5-amino-3-[[2-fluoro-5-(2-trimethylsilylethoxycarbonyl)phenyl]methoxy]isothiazol-4-carboxylate I574 (300 mg, 703.34 μmol) and potassium carbonate (292 mg, 2.11 mmol), and the reaction system was stirred at 25 °C to react for 1 hour. Water (10 mL) was added to the reaction mixture, which was then subjected to liquid separation and extraction with ethyl acetate (10 mLx3). The organic phases were combined, washed with saturated brine (20 mL), dried with anhydrous sodium sulfate, and concentrated under reduced pressure to obtain a crude product. The crude product was purified by the preparative thin-layer chromatography (dichloromethane /methanol = 10/1) to obtain a yellow solid product methyl 5-[3-[(3S)-3-fluoropyrrolidin-1-yl]propylcarbamyl]-3-[[2-fluoro-5-(2-trimethylsilylethoxycarbonyl)phenyl]methoxy]isothiazol-4-carboxylate I575 (50 mg, 83.51 μmol, yield: 11.87%).

The product was verified by LCMS.

For MS-ESI, the calculated value was $[M+H]^+$ 599.2, and the measurement was 599.1.

Step 8

4-fluoro-3-[[5-[3-[(3S)-3-fluoropyrrolidin-1-yl]propylcarbamoylamino]-4-methoxycarbonyl-isothiazol-3-yl]oxymethyl]benzoic acid

At room temperature, methyl 5-[3-[(3S)-3-fluoropyrrolidin-1-yl]propylcarbamyl]-3-[[2-fluoro-5-(2-trimethylsilylethoxycarbonyl)phenyl]methoxy]isothiazol-4-carboxylate I575 (50 mg, 83.51 μmol) was dissolved in tetrahydrofuran (2 mL), followed by the addition of tetrabutylammonium fluoride (1 mol/L, 125.26 μL). The reaction system was stirred at 25°C to react for 3 h. The reaction mixture was concentrated under reduced pressure to obtain a yellow solid crude product4-fluoro-3-[[5-[3-[(3S)-3-fluoropyrrolidin-1-yl]propylcarbamylamino]-4-methoxycarbonyl-isothiazol-3-yl]oxymethyl]benzoic acid I576 (30 mg, 60.18 μmol, yield: 72.06%).

The product was verified by LCMS.

For MS-ESI, the calculated value was $[M+H]^+$ 499.1, and the measurement was 499.0.

Step 9

3-[[4-carbamoyl-5-[3-[(3S)-3-fluoropyrrolidin-1-yl]propylcarbamoylamino]isothiazol-3-yl]oxymethyl]-4-fluoro-benzoic acid

4-fluoro-3-[[5-[3-[(3S)-3-fluoropyrrolidin-1-yl]propylcarbamylamino]-4-methoxycarbonyl-isothiazol-3-yl]oxymethyl]benzoic acid I576 (30 mg, 60.18 μmol) was dissolved in the solution of ammonia in methanol (2 mL, 7 mol/L), and the mixture was stirred at 60 °C to react for 16 hours. The reaction mixture was concentrated under reduced pressure to obtain a crude product. The crude product was purified by the high-pressure preparative liquid chromatography to obtain a white solid product 3-[[4-carbamyl-5-[3-[(3S)-3-fluoropyrrolidin-1-yl]propylcarbamylamino]isothiazol-3-yl]oxymethyl]-4-fluoro-benzoic acid T171 (3.06 mg, 6.33 μmol, yield: 10.52%).

The product was verified by LCMS and H-NMR.

For MS-ESI, the calculated value was $[M+H]^+$ 484.1, and the measurement was 484.0.

$^1$H NMR (400MHz, $CD_3OD$&$CDCl_3$): δ (ppm) 8.20 (d, *J* =6.4 Hz, 1H), 8.09-8.05 (m, 1H), 7.22 (t, *J*= 9.2 Hz, 1H), 5.58 (s, 2H), 5.44 (s, 0.5H), 5.35-5.32 (m, 0.5H), 3.24-3.20 (m, 2H), 3.12-3.08 (m, 2H), 1.93 (s, 2H), 1.70-1.60 (m, 2H), 1.46-1.40 (m, 1H), 1.19 (t, *J* =14.0 Hz, 1H), 1.04 (t, *J*= 14.8 Hz, 2H).

Example 143: Synthesis of Compound T173

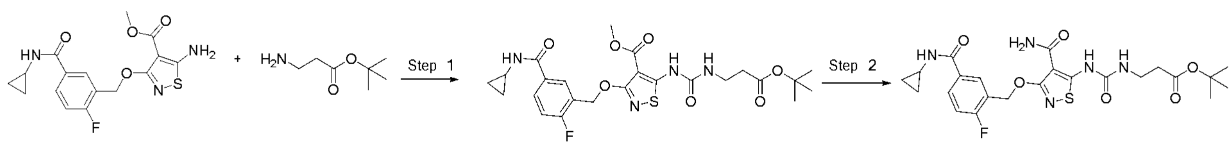


I376 I577 T173

Step 1

Methyl 5-[(3-tert-butoxy-3-oxo-propyl)carbamoylamino]-3-[[5-(cyclopropylcarbamoyl)-2-fluoro-phenyl]methoxy]isothiazole-4-carboxylate

Tert-butyl 3-aminopropanoate (477 mg, 3.28 mmol) was dissolved in tetrahydrofuran (10 mL), followed by the addition of N,N'-carbonyl diimidazole (394 mg, 2.43 mmol) under the protection of nitrogen, and the mixture was stirred at 25 °C to react for 1 hour. Dimethyl sulfoxide (10 mL) was added, the pressure was reduced to remove tetrahydrofuran, followed by the addition of methyl 5-amino-3-[[5-(cyclopropylcarbamyl)-2-fluoro-phenyl]methoxy]isothiazol-4-carboxylate I376 (1 g, 2.74 mmol) and anhydrous potassium carbonate (1.1 g, 8.21 mmol), and the reaction system was stirred at 30°C to react for 16 hours. Water (100 mL) was added to the reaction mixture, which was then subjected to liquid separation and extraction with ethyl acetate (30 mLx3). The organic phases were combined, washed with saturated brine (30 mL), dried with anhydrous sodium sulfate, and concentrated under reduced pressure to obtain a crude product. The crude product was purified by the preparative thin-layer chromatography (dichloromethane /anhydrous methanol = 10/1) to obtain a yellow oily product methyl 5-[(3-tert-butoxy-3-oxo-propyl)carbamylamino]-3-[[5-(cyclopropylcarbamyl)-2-fluoro-phenyl]methoxy]isothiazol-4-carboxylate I577 (300 mg, 559.10 μmol, yield: 20.43%)

The product was verified by LCMS and H-NMR.

For MS-ESI, the calculated value was [M+H]+ 537.2, and the measurement was 537.0. $^1$H NMR (400MHz, $CDCl_3$): δ (ppm) 10.31 (s, 1H), 8.12-8.08 (m, 1H), 7.71-7.69 (m, 1H), 7.11 (t, *J* = 8.8Hz, 1H), 6.35 (s, 1H), 6.20 (s, 1H), 5.50 (s, 2H), 3.92 (s, 3H), 3.58-3.54(m, 2H), 2.93-2.90 (m, 1H), 2.54 (t, *J* = 6.0Hz, 2H), 1.48 (s, 9H), 0.90-0.86 (m, 2H), 0.66-0.62 (m, 2H).

Step 2

Tert-butyl 3-[[4-carbamoyl-3-[[5-(cyclopropylcarbamoyl)-2-fluoro-phenyl]methoxy]isothiazol-5-yl]carbamoylamino]propanoate

Methyl 5-[(3-tert-butoxy-3-oxo-propyl)carbamylamino]-3-[[5-(cyclopropylcarbamyl)-2-fluorophenyl]methoxy] isothiazol-4-carboxylate I577 (300 mg, 559.10 μmol) was dissolved in the solution of ammonia in methanol (4 mL, 7mol/L), and the mixture was stirred at 40°C to react for 16 hours. The reaction mixture was concentrated under reduced pressure to obtain a crude product. The crude product was purified by the preparative thin-layer chromatography (dichloromethane /anhydrous methanol = 10/1) to obtain a white solid product tert-butyl 3-[[4-carbamyl-3-[[5-(cyclopropylcarbamyl)-2-fluoro-phenyl]methoxy]isothiazol-5-yl]carbamylamino]propanoate

T173 (100 mg, 191.73 μmol, 34.29% yield).

The product was verified by LCMS, H-NMR and F-NMR.

For MS-ESI, the calculated value was [M+H]+ 522.2, and the measurement was 522.0.

$^{1}$H NMR (400MHz, $CD_3OD$): δ (ppm) 8.00 (dd, *J* = 6.8, 2.4 Hz, 1H), 7.84-7.84 (m, 1H), 7.24 (t, *J*= *9.2* Hz, 1H), 5.57 (s, 2H), 3.45 (t, *J*= 6.8 Hz, 2H), 2.85-2.81 (m, 1H), 2.47 (t, *J*= 6.4 Hz, 2H), 1.45 (s, 9H), 0.82-0.77 (m, 2H), 0.64-0.61 (m, 2H).

$^{19}$F NMR (400MHz, $CD_3OD$): δ (ppm) -115.46.

Example 144: Synthesis of Compound T172

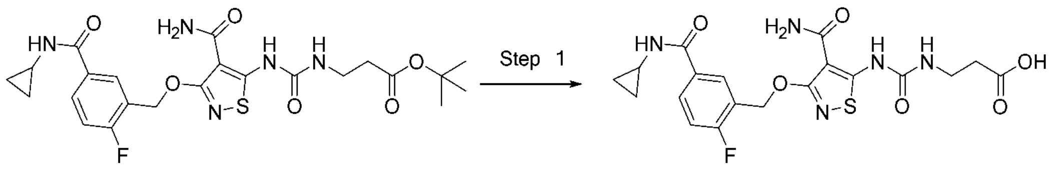


T173 T172

3-[[4-carbamoyl-3-[[5-(cyclopropylcarbamoyl)-2-fluoro-phenyl]methoxy]isothiazol-5-yl]carbamoylamino]propanoic acid

Tert-butyl 3-[[4-carbamyl-3-[[5-(cyclopropylcarbamyl)-2-fluoro-phenyl]methoxy]isothiazol-5-yl]carbamylamino] propanoateT173 (100 mg, 191.73 μmol) was dissolved in dichloromethane (3 mL), followed by the addition of trifluoroacetic acid (0.5 mL), and the mixture was stirred at 25°C to react for 16 hours. The reaction mixture was concentrated under reduced pressure to obtain a crude product. The crude product was purified by the high-pressure preparative chromatography to obtain a white solid product 3-[[4-carbamyl-3-[[5-(cyclopropylcarbamyl)-2-fluorophenyl]methoxy] isothiazol-5-yl]carbamylamino]propionic acid T172 (18.91 mg, 40.63 μmol, yield: 21.19%).

The product was verified by LCMS, H-NMR and F-NMR.

For MS-ESI, the calculated value was [M+H]+ 466.1, and the measurement was 465.9.

$^{1}$H NMR (400MHz, $CD_3OD$): δ (ppm) 8.51 (d, *J*= 1.6 Hz, 1H), 8.04 (d, *J* =8.4 Hz, 1H), 7.87-7.80 (m, 2H), 7.66 (d, *J* =8.0 Hz, 1H), 7.57-7.50 (m, 2H), 7.38-7.35 (m, 1H), 7.25 (dd, *J* =8.8, 2.8 Hz, 1H), 7.18-7.13 (m, 2H), 3.69 (s, 2H), 3.33-3.29 (m, 2H), 2.58 (s, 4H), 1.85-1.81 (m, 4H), 1.14 (t, *J* = 7.2Hz, 3H).

$^{19}$F NMR (400MHz, $CD_3OD$): δ (ppm) -115.65.

Test Examples:

Compound T001 used in the following examples is a positive compound PAN90806, of which the commercially available product is used.

Example 1: Assay of Ability of Compounds to Inhibit Tyrosine Kinases

This assay directly monitored the ability of compounds to inhibit kinase phosphorylation by measuring non-phosphorylated and phosphorylated substrates in kinase reactions by using Mobility Shift Assays/IMAP™ analysis. The reagents and instruments used are shown in the table below.

Table 1. Reagents and Instruments

| Reagent | Supplier | Catalog No. |
|---|---|---|
| FLT1 (VEGFR1) | Invitrogen | PR6731B |
| KDR (VEGFR2) | Carna | 08-191 |
| FLT4 (VEGFR3) | Invitrogen | PV4129 |
| TIE2 | BPS | 40270 |
| EGFR | Carna | 08-115 |
| ATP | Sigma | 2383-5G |
| $MgCl_2$ | Sigma | M2670-500g |

(continued)

| Reagent | Supplier | Catalog No. |
|---|---|---|
| DTT | Sigma | D0632-10G |
| EGTA | Sigma | E3889-25G |
| DMSO | Sigma | D2650 |
| Staurosporine | MCE | HY-15141 |
| Plate Reader | Caliper Life Sciences | CaliperEZ Reader II |

Experimental Procedures:

Add the kinase and DTT to 1x kinase buffer to prepare a 2.5x kinase solution; transfer 10 μL of 2.5x kinase solution to a 384-well reaction plate and add 1x kinase buffer to negative control wells. Incubate the plate for 10 min at room temperature; add FAM-labeled peptides, $MgCl_2$, ATP and other reagents to 1x kinase buffer to prepare a 2.5x substrate solution; transfer 10 μL of the above 2.5x substrate solution to the 384-well reaction plate; incubate the plate at 28°C for different times, and add 25 μL of stop solution to the 384-well reaction plate to stop the reaction. Read the conversion ratio data on CaliperEZ Reader II. Convert conversion rates into inhibition ratio data.

$$\%Inhibition = \frac{Max - Conversion}{Max - Min} \times 100\%$$

"Min" indicates the readout of the control well without the addition of enzymes; and "Max" indicates the readout of the control well with the addition of DMSO. Fit $IC_{50}$ values with XLFit excel add-in version 5.4.0.8.

$$Y = Bottom + \frac{Top - Bottom}{1 + \frac{IC_{50}}{X}^{(LogIC_{50} - X)Hill\ Slope}}$$

X indicates the log value of compound concentration, and Y indicates the inhibition ratio (%inhibition).

The $IC_{30}$ results of the compounds prepared in the synthesis examples for VEGFR2 are shown in the table below:

Table 2. Experimental results

| No. | VEGFR2 IC50 (nM) | No. | VEGFR2 IC50 (nM) | No. | VEGFR2 IC50 (nM) | No. | VEGFR2 IC50 (nM) | No. | VEGFR2 IC50 (nM) |
|---|---|---|---|---|---|---|---|---|---|
| T001 | 8.03 | T062 | 58.77 | T090 | 3.55 | T120 | 47.00 | T149 | 2.35 |
| T002 | 129.90 | T063 | 62.99 | T091 | 3.03 | T121 | 40.00 | T150 | 1.20 |
| T003 | 112.00 | T064 | 43.26 | T092 | 17.25 | T122 | 2.55 | T151 | 1.75 |
| T004 | 163.09 | T065 | 9.95 | T093 | 12.44 | T123 | 0.63 | T152 | 3.53 |
| T006 | 47.40 | T066 | 77.85 | T094 | 9.10 | T124 | 2.20 | T153 | 0.66 |
| T007 | 42.99 | T067 | 8.86 | T095 | 4.70 | T125 | 13.50 | T156 | 1.44 |
| T009 | 217.00 | T068 | 170.90 | T098 | 1.56 | T126 | 2.70 | T157 | 2.50 |
| T030 | 34.83 | T069 | 2.77 | T101 | 4.85 | T127 | 3.00 | T158 | 1.05 |
| T032 | 56.81 | T070 | 12.02 | T102 | 5.50 | T128 | 1.20 | T159 | 41.67 |
| T033 | 51.86 | T071 | 4.36 | T103 | 4.96 | T129 | 11.95 | T160 | 1.41 |
| T042 | 14.23 | T073 | 2.73 | T104 | 16.44 | T130 | 41.00 | T162 | 3.05 |
| T044 | 127.97 | T074 | 26.46 | T105 | 1.60 | T131 | 17.40 | T163 | 0.32 |
| T045 | 22.14 | T075 | 22.99 | T106 | 1.64 | T132 | 14.75 | T164 | 1.25 |
| T046 | 61.98 | T076 | 12.64 | T107 | 8.57 | T133 | 0.50 | T165 | 1.46 |

(continued)

| No. | VEGFR2 IC50 (nM) | No. | VEGFR2 IC50 (nM) | No. | VEGFR2 IC50 (nM) | No. | VEGFR2 IC50 (nM) | No. | VEGFR2 IC50 (nM) |
|---|---|---|---|---|---|---|---|---|---|
| T049 | 19.83 | T077 | 7.01 | T108 | 0.72 | T134 | 6.60 | T166 | 0.45 |
| T050 | 3.68 | T078 | 2.09 | T109 | 13.77 | T135 | 1.90 | T167 | 0.95 |
| T052 | 8.17 | T079 | 4.47 | T111 | 15.05 | T136 | 1.80 | T168 | 8.45 |
| T053 | 10.40 | T080 | 7.03 | T112 | 14.57 | T137 | 2.10 | T169 | 16.39 |
| T054 | 17.70 | T081 | 15.98 | T113 | 14.66 | T138 | 2.60 | T170 | 1.12 |
| T055 | 15.69 | T082 | 24.04 | T114 | 12.81 | T139 | 0.41 | T172 | 2.60 |
| T056 | 4.69 | T083 | 12.71 | T115 | 7.20 | T140 | 0.44 | T173 | 1.97 |
| T057 | 151.60 | T084 | 14.09 | T116 | 1.14 | T145 | 1.39 | | |
| T058 | 20.95 | T085 | 1.70 | T117 | 2.54 | T146 | 10.53 | | |
| T061 | 44.72 | T087 | 1.66 | T118 | 1.15 | T142 | 6.60 | | |
| | | T089 | 6.77 | T119 | 10.00 | T148 | 16.00 | | |

The $IC_{30}$ results of the compounds prepared in the synthesis examples for VEGFR1 are shown in the table below:

Table 3. Experimental results

| No. | VEGFR1 $IC_{50}$ (nM) | No. | VEGFR1 $IC_{50}$ (nM) | No. | VEGFR1 $IC_{50}$ (nM) | No. | VEGFR1 $IC_{50}$ (nM) | No. | VEGFR1 $IC_{50}$ (nM) |
|---|---|---|---|---|---|---|---|---|---|
| T001 | 37.43 | T056 | 3.33 | T073 | 5.99 | T082 | 27.75 | T116 | 1.21 |
| T042 | 34.26 | T058 | 29.98 | T075 | 43.97 | T083 | 25.61 | T149 | 2.39 |
| T049 | 21.56 | T065 | 6.55 | T076 | 26.66 | T084 | 30.11 | T151 | 1.53 |
| T050 | 6.08 | T066 | 37.95 | T077 | 33.00 | T085 | 4.95 | T152 | 2.25 |
| T052 | 42.06 | T067 | 9.43 | T078 | 5.72 | T087 | 1.11 | | |
| T053 | 14.66 | T069 | 5.48 | T079 | 11.84 | T089 | 13.48 | | |
| T054 | 34.28 | T070 | 9.14 | T080 | 13.47 | T090 | 7.73 | | |
| T055 | 7.70 | T071 | 13.41 | T081 | 21.86 | T091 | 5.87 | | |

The $IC_{30}$ results of the compounds prepared in the synthesis examples for VEGFR3 are shown in the table below:

Table 4. Experimental results

| No. | VEGFR3 $IC_{50}$ (nM) | No. | VEGFR3 $IC_{50}$ (nM) | No. | VEGFR3 $IC_{50}$ (nM) | No. | VEGFR3 $IC_{50}$ (nM) | No. | VEGFR3 $IC_{50}$ (nM) |
|---|---|---|---|---|---|---|---|---|---|
| T001 | 53.65 | T063 | 39.08 | T076 | 23.81 | T084 | 37.09 | T151 | 3.91 |
| T042 | 41.95 | T065 | 24.09 | T077 | 43.99 | T085 | 10.58 | T152 | 12.83 |
| T050 | 16.07 | T067 | 30.13 | T078 | 16.81 | T087 | 0.80 | | |
| T052 | 18.70 | T069 | 14.59 | T079 | 21.37 | T089 | 9.90 | | |
| T053 | 17.44 | T070 | 50.08 | T080 | 26.13 | T090 | 13.01 | | |
| T054 | 38.84 | T071 | 18.70 | T081 | 23.90 | T091 | 6.72 | | |
| T055 | 39.34 | T073 | 10.65 | T082 | 48.53 | T116 | 4.80 | | |
| T056 | 13.21 | T075 | 48.78 | T083 | 22.30 | T149 | 10.00 | | |

The $IC_{50}$ results of the compounds prepared in the synthesis examples for TIE2 are shown in the table below:

Table 5. Experimental results

| No. | TIE2 $IC_{50}$ (nM) | No. | TIE2 $IC_{50}$ (nM) | No. | TIE2 $IC_{50}$ (nM) | No. | TIE2 $IC_{50}$ (nM) | No. | TIE2 $IC_{50}$ (nM) |
|---|---|---|---|---|---|---|---|---|---|
| T001 | 88.80 | T060 | 3248.92 | T085 | 4587.60 | T113 | 162.53 | T140 | 988.00 |
| T002 | 2661.00 | T061 | 238.96 | T086 | 895.48 | T114 | 187.30 | T146 | 739.50 |
| T006 | 1408.98 | T062 | 1675.51 | T087 | 1424.47 | T115 | 117.00 | T153 | 1005.81 |
| T007 | 474.85 | T063 | 1004.81 | T088 | 6395.94 | T116 | 2360.19 | T155 | > 60000.00 |
| T009 | 4731.00 | T064 | 1603.45 | T089 | 134.82 | T118 | 1079.00 | T156 | 2194.33 |
| T030 | 282.18 | T065 | 5430.85 | T090 | 121.24 | T119 | 205.00 | T157 | 4279.50 |
| T032 | 637.66 | T066 | 10000.00 | T091 | 139.61 | T120 | 397.00 | T158 | 5384.12 |
| T033 | 1461.69 | T067 | 6698.62 | T092 | 595.77 | T121 | 230.00 | T159 | 48585.33 |
| T042 | 448.37 | T068 | 1609.30 | T093 | 181.31 | T122 | 2099.00 | T160 | 4267.76 |
| T044 | 1635.04 | T069 | 3513.55 | T094 | 413.00 | T123 | 973.00 | T162 | 7360.42 |
| T045 | 230.01 | T070 | 7797.58 | T098 | 4905.15 | T124 | 2890.67 | T163 | 2686.20 |
| T046 | 467.00 | T071 | 147.15 | T101 | 163.07 | T125 | 160.50 | T164 | 5712.17 |
| T049 | 7560.42 | T072 | 1670.01 | T102 | 142.20 | T127 | 241.00 | T165 | 3933.47 |
| T051 | 3211.00 | T076 | 231.97 | T103 | 94.53 | T128 | 1176.00 | T166 | 1737.83 |
| T052 | 100.00 | T077 | 240.84 | T104 | 228.63 | T130 | 471.00 | T167 | 1110.91 |
| T053 | 258.02 | T078 | 4055.57 | T105 | 2735.46 | T131 | 183.33 | T168 | 5129.84 |
| T054 | 157.28 | T079 | 115.14 | T106 | 2622.16 | T132 | 163.00 | T169 | > 60000.00 |
| T055 | 3362.23 | T080 | 88.04 | T107 | 124.08 | T133 | 1099.00 | T170 | 2469.96 |
| T056 | 2251.55 | T081 | 177.46 | T108 | 621.48 | T134 | 117.50 | T171 | 5845.30 |
| T057 | 2872.27 | T082 | 203.12 | T109 | 137.95 | T135 | 97.00 | T172 | 3262.20 |
| T058 | 4897.97 | T083 | 164.49 | T111 | 223.10 | T137 | 85.00 | T173 | 1728.42 |
| T059 | 1946.95 | T084 | 221.00 | T112 | 129.18 | T139 | 366.00 | | |

The $IC_{30}$ results of the compounds prepared in the synthesis examples for EGFR are shown in the table below:

Table 6. Experimental results

| No. | EGFR $IC_{50}$ (nM) | No. | EGFR $IC_{50}$ (nM) | No. | EGFR $IC_{50}$ (nM) | No. | EGFR $IC_{50}$ (nM) | No. | **EGFR** $IC_{50}$ (nM) |
|---|---|---|---|---|---|---|---|---|---|
| T001 | 3562.93 | T069 | > 60000.00 | T090 | 13947.77 | T118 | 8957.50 | T140 | 15482.00 |
| T006 | 35000.00 | T070 | > 60000.00 | T091 | 9886.30 | T119 | 19894.00 | T145 | > 60000.00 |
| T030 | 21762.72 | T071 | 15219.78 | T093 | 14583.19 | T120 | 40703.00 | T146 | 26101.00 |
| T032 | 39597.82 | T072 | 43499.70 | T094 | > 60000.00 | T121 | 17181.00 | T142 | 30965.00 |
| T033 | 54931.70 | T073 | 4554.48 | T095 | 11367.00 | T122 | 5681.50 | T148 | 4011.00 |
| T042 | 33041.81 | T074 | 62717.09 | T098 | 16501.50 | T123 | 26861.00 | T149 | 11978.00 |
| T044 | 10036.80 | T075 | 24319.06 | T101 | 9159.17 | T124 | > 60000.00 | T150 | 7417.00 |
| T053 | 11369.03 | T076 | 43615.00 | T102 | 9892.58 | T125 | 9706.50 | T151 | 10726.00 |
| T055 | 49680.50 | T077 | 51974.20 | T103 | 5493.38 | T126 | > 60000.00 | T152 | 23954.00 |
| T056 | 50602.22 | T078 | 16122.22 | T104 | 18908.86 | T129 | 15624.50 | T155 | > 60000.00 |
| T057 | > 60000.00 | T079 | 5136.78 | T105 | 11944.00 | T130 | 13713.00 | T157 | 15201.40 |
| T058 | > 60000.00 | T080 | 8045.56 | T106 | 20142.30 | T131 | 15000.75 | T158 | 9843.39 |

(continued)

| No. | EGFR $IC_{50}$ (nM) | No. | EGFR $IC_{50}$ (nM) | No. | EGFR $IC_{50}$ (nM) | No. | EGFR $IC_{50}$ (nM) | No. | **EGFR** $IC_{50}$ (nM) |
|---|---|---|---|---|---|---|---|---|---|
| T059 | > 60000.00 | T081 | 19615.00 | T107 | 9137.01 | T132 | 19176.00 | T159 | 8772.25 |
| T060 | > 60000.00 | T082 | 15467.00 | T109 | 7453.92 | T133 | 21466.00 | T163 | 11187.40 |
| T062 | > 60000.00 | T083 | 18126.10 | T111 | 19839.62 | T134 | 20601.00 | T164 | 12209.10 |
| T063 | > 60000.00 | T084 | 15751.69 | T112 | 10173.90 | T135 | > 60000.00 | T165 | 31589.80 |
| T064 | > 60000.00 | T085 | > 60000.00 | T113 | 11815.34 | T136 | > 60000.00 | T166 | 15635.00 |
| T065 | 60000.00 | T086 | 6829.90 | T114 | 13449.74 | T137 | > 60000.00 | T169 | > 60000.00 |
| T066 | > 60000.00 | T087 | 15168.58 | T115 | 8220.00 | T138 | > 60000.00 | T171 | 29131.50 |
| T067 | > 60000.00 | T088 | > 60000.00 | T116 | 17952.95 | T139 | 10054.00 | T173 | 5912.60 |
| T068 | 4407.81 | T089 | 15106.75 | T117 | > 60000.00 | | | | |

Example 2: Assay of Ability of Compounds to Inhibit VEGFR2 Phosphorylation in HUVEC Cells VEGF is a highly potent growth factor capable of promoting angiogenesis and vascular permeability. Vascular endothelial growth factor receptor 2 (VEGFR2) is a primary receptor for VEGF-induced endothelial cell signaling. After ligand binding, VEGFR2 is autophosphorylated and activated. The main function of signaling from VEGFR2 is to induce vascularization during development and after tissue injury, and to bypass blocked blood vessels. Pathologically, VEGF and VEGFR2 are involved in tumor angiogenesis and vascular leakage. They are considered to be main participants in many different cancers and ocular diseases that accompany pathological neovascularization. This assay measured the ability of the compounds to inhibit VEGF-induced VEGFR2 autophosphorylation (pVEGFR2) in HUVEC cells by using ELISA.

The reagents and instruments used are shown in the table below.

Table 7. Reagents and instruments

| Reagent or Instrument | Supplier | Catalog or model number |
|---|---|---|
| Endothelial Cell Growth Base Media | R&D | 390598 |
| Endothelial Cell Growth Supplement (50X) | R&D | 390599 |
| Fetal bovine serum | Invitrogen | 10099141C |
| DPBS | Corning | 21-031-CV |
| Trypsin-EDTA (0.05%), phenol red | Invitrogen | 25300062 |
| Human VEGF-165 Recombinant Protein | CST | 48143 |
| Phosphatase Inhibitor Cocktail 3 | Sigma | P0044 |
| Phosphatase Inhibitor Cocktail 2 | Sigma | P5726 |
| PathScan® Phospho-VEGFR-2 (Tyr1175) Sandwich ELISA Antibody Pair | CST | 7824S |
| Protease Inhibitor | Roche | 4693159001 |
| 10X Cell Lysis Buffer | CST | 9803S |
| Tween® 20 | Sigma | P1379 |
| Bovine Serum Albumin (BSA) | Sigma | B2064-100G |
| TMB Substrate | CST | 7004P4 |
| STOP Solution | CST | 7002P4 |
| Microplate Reader | Molecular Devices | SpectraMax® 340 PC 384 |

Experimental procedures:

Culture cells in a cell culture flask to reach a fusion degree of 60%-80% before cell inoculation. Dilute the cell

suspension to $4.5\times10^6$ cells/mL with a pre-warmed complete medium. Add 80 μL of the cell suspension to all wells of a 96-well cell plate (Corning-3599); and incubate the plate overnight at 37 °C in the presence of 5% $CO_2$. Dissolve the compound powder to 10 mM with DMSO and store it in a nitrogen cabinet. Compound dilution plate: Add a reference compound Ponatinib or a compound to be tested to a 96 V-well plate (Axygen-WIPP02280) at concentrations achieved as follows: performing 3-fold gradient dilution starting from 5 mM (5 μL of 10 mM compound solution plus 5 μL of DMSO) for a total of 8 concentration points. For the 100% inhibition control group, the concentration of the reference compound Ponatinib in the V-well plate was 2.5 mM (3 μL of 10 mM compound solution plus 9 μL of DMSO). Transfer 20 μL of the diluted compound from the intermediate plate to a cell plate. Then, incubate the cell plate for 1 h at 37 °C in the presence of 5% $CO_2$. After the compound was treated for 1 h, add 25 μL of 250 ng/ml human VEGF at a final concentration of 50 ng/mL to a 96-well plate; and incubate the plate for 5 min at 37 °C in the presence of 5% $CO_2$, centrifuge the plate for 10 min at 3,000 rpm, remove the medium and wash the cells once with 300 μL of pre-chilled DPBS per well. Then, centrifuge the plate for 10 min at 4,000 rpm, remove DPBS, and add 110 μL of pre-chilled 1X cell lysis buffer per well to the 96-well plate. Shake the plate for 40 min at 4 °C. Then, freeze the plate in a refrigerator at -80°C. On the next day, remove the plate from the refrigerator at -80°C, thaw the plate, centrifuge the plate for 10 min at 3500 rpm, and add 100 μL of the supernatant per well to an ELISA plate. Seal the plate and incubate it for 2 h at 37 °C. Wash the wells 4 times with 1x wash buffer. Add 100 μL of recombinant detection antibody per well. Seal the plate, incubate the plate for 1 h at 37 °C, and repeat the step of plate washing. Add 100 μL of secondary antibody per well. Seal the plate, incubate the plate for 30 min at 37 °C, and repeat the step of plate washing. Add 100 μL of TMB (protected from light) per well. Seal the plate, incubate the plate for 30 min at 37 °C, and add 100 μL of the stop solution per well. Gently shake for 30 sec, measure the absorbance with a SpectraMax® 340 PC 384 Absorbance Microplate Reader within 30 min, and read an optical density value at 450 nm. Conduct data analysis using XL Fit software:

$$\text{Inhibition ratio(\%Inhibition)} = \frac{\text{Mean value of negative control group} - \text{Sample value}}{\text{Mean value of negative control group} - \text{Mean value of positive control group}}$$

"Human VEGF-stimulated cells with DMSO" was taken as the negative control group (0% inhibition rate), "Human VEGF-stimulated cells with 5 μM Ponatinib" was taken as the positive control group (100% inhibition rate), and all other values were expressed as percentages (inhibition rate) of this control.

Conduct curve fitting using the 4 Parameter Logistic Model or Sigmoidal Dose-Response Model to obtain $IC_{50}$ values.

$$Y = Bottom + \frac{Top - Bottom}{1 + 10^{(LogIC_{50} - X)\times Hill\ Slope}}$$

X indicates the log value of compound concentration, Y indicates the inhibition rate (%inhibition), Top indicates the readout of the negative control wells, and Bottom indicates the readout of the positive control well.

Table 8. Experimental results

| No. | pVEGFR 2 $IC_{50}$ (nM) | No. | pVEGFR 2 $IC_{50}$ (nM) | No. | pVEGFR 2 $IC_{50}$ (nM) | No. | pVEGFR 2 $IC_{50}$ (nM) | No. | pVEGFR 2 $IC_{50}$ (nM) |
|---|---|---|---|---|---|---|---|---|---|
| T00 1 | 171.67 | T09 5 | 93.00 | T11 6 | 61.00 | T12 8 | 4.90 | T14 9 | 52.25 |
| T04 2 | 22.00 | T09 8 | 132.00 | T11 8 | 48.25 | T12 9 | 71.00 | T15 1 | 68.40 |
| T07 8 | 56.50 | T10 1 | 103.00 | T12 3 | 0.78 | T13 1 | 19.00 | | |
| T08 7 | 30.00 | T10 4 | 34.50 | T12 4 | 16.25 | T13 4 | 16.00 | | |
| T09 1 | 87.00 | T11 4 | 32.50 | T12 6 | 27.00 | T14 5 | 2.20 | | |

Example 3: Assay of Inhibitory Effect of Compounds Against VEGF-induced HUVEC Cell Proliferation

In this experiment, human umbilical vein endothelial cells (HUVEC, Lonza) were used. HUVEC cells are primary cells isolated from umbilical veins. This cell line is a model system to study the functions of endothelial cells. Endothelial cell proliferation in response to VEGF is crucial to the pathological angiogenesis associated with conditions such as tumor

development and ocular neovascular diseases.

In this assay, a luciferase bioluminescence assay (CellTiter GLU™, Promega) is used to determine the inhibitory activity of the compounds against VEGF-induced HUVEC cell proliferation and viability. CellTiter GLO™ is a homogeneous method for quantifying the count of viable cells in culture based on ATP, an indicator of metabolically active cells. In this assay, the compound was incubated with HUVEC cells for 72 hours, and then the inhibitory activity of the resulting compound agasint VEGF-induced HUVEC cell proliferation was measured on the EnVision plate reader.

The reagents and instruments used are shown in the table below.

Table 9. Reagents and instruments

| Reagent or Instrument | Supplier | Catalog or model number |
|---|---|---|
| HUVEC | Lonza | C2519A |
| EGMTM-2 Endothelial Cell Growth Medium-2 BulletKit™ | Lonza | CC-3162 |
| RPMI1640 | Gibco | A10491-01 |
| Penicillin-Streptomycin | Gibco | 15140-122 |
| Fetal Bovine Serum | Gibco | 10099-141C |
| Phosphate Buffered Saline (PBS) | Gibco | 10010-031 |
| TrypLE | Gibco | 12604-021 |
| DMSO | Sigma | D8418-1L |
| Ki8751 | Selleck | S1363 |
| rhVEGF | R&D | 293-VE |
| CelltiterGlo assay kit (CTG) | Promega | G7573 |
| Envision | PerkinElmer | 2105 |

Experimental procedures:

With the RPMI-1640 medium (plus 5% FBS, 1% Penicillin-Streptomycin) used for the HUVEC cell proliferation experiment, add a cell suspension to a 96-well plate at 100 μl per well, i.e., 4000 HUVEC cells/well, and incubate them overnight in a 5% $CO_2$ incubator at 37°C (serum starvation). After 24 hours, add 96 μl of the experimental medium to the cell wells, and then add 2 μl of the compound to the cell wells; and after 1 hour following the addition of the compound, add 2 μl of 2 μg/ml VEGF to the cell wells. Add 0.1% DMSO and 20 ng/ml VEGF to cell wells to serve as high-reading control wells. Take cell-free wells with only medium as low-reading control wells. The detected final concentrations of the test compound were 1000 μM, 333 μM, 111 μM, 37 μM, 12.3 μM, 4.12 μM, 1.37 μM, 0.46 μM, 0.15 μM and 0 μM. After 72 hours of incubation of the cell plate in the incubator, hold the cell plate to be tested at room temperature and discard 100 μl of medium per well. Add 100 μl of CTG reagent (CellTiter Glo kit) per well, oscillate the cell plate in a fast oscillator for 3 minutes, and keep the cell plate at room temperature in the dark for 30 minutes. Read the chemiluminescence signal value with the Envision instrument. Calculate $IC_{50}$ using GraphPad Prism 8 software, and obtain the $IC_{50}$ (median inhibitory concentration) of the compound using the following nonlinear fitting formula of 4 Parameter Logistic Model or Sigmoidal Dose-Response Model:

$$Y = Bottom + \frac{Top - Bottom}{1 + 10^{(LogIC_{50} - X) \times Hill\ Slope}}$$

X indicates the log value of compound concentration, Y indicates the inhibition rate (%inhibition), Top indicates the readout of the high-reading control well.

$$\text{Inhibition ratio(\%inhibition)} = \frac{\text{Readout of high} - \text{reading control well} - \text{Readout of compound well}}{\text{Readout of high} - \text{reading control well} - \text{Readout of low} - \text{reading control well}} \times 100\%$$

The $IC_{30}$ results of the compounds prepared in the synthesis examples against HUVEC cell proliferation are shown in the table below:

Table 10. Experimental results

| No. | HUVEC $IC_{50}$ (nM) | No. | HUVEC $IC_{50}$ (nM) | No. | HUVEC $IC_{50}$ (nM) | No. | HUVEC $IC_{50}$ (nM) | No. | HUVEC $IC_{50}$ (nM) |
|---|---|---|---|---|---|---|---|---|---|
| T001 | 76.85 | T079 | 57.68 | T101 | 48.61 | T114 | 19.00 | T123 | 17.09 |
| T042 | 79.88 | T087 | 45.38 | T103 | 66.00 | T115 | 36.00 | T124 | 43.60 |
| T053 | 69.72 | T090 | 46.85 | T104 | 35.50 | T116 | 21.00 | T126 | 22.00 |
| T064 | 79.21 | T091 | 35.95 | T111 | 32.00 | T118 | 39.00 | | |
| T071 | 74.50 | T093 | 55.77 | T112 | 36.00 | T119 | 67.00 | | |
| T073 | 26.85 | T095 | 55.77 | T113 | 27.00 | T122 | 44.00 | | |

Example 4: Solubility Test for Compounds

The reagents used are shown in the table below.

Table 11. Reagents

| Reagent or Instrument | Supplier | Catalog or model number |
|---|---|---|
| Trisodium citrate dihydrate | Sinopharm Chemical Reagent Co., Ltd. | 10019418 |
| Citric acid monohydrate | Sinopharm Chemical Reagent Co., Ltd. | 10007118 |
| Methanol | ASTOON | AT-3292 |
| Acetonitrile | ASTOON | AT-3022 |
| Verapamil hydrochloride | Aladdin | V111249 |
| Glibenclamide | Aladdin | G127198 |
| DMSO | Aladdin | D103273 |
| Water | Self-prepared deionized water | - |

Experimental procedures:

Accurately weigh 3 portions of about 2 mg of the compound, and add appropriate volumes of buffers with the pHs of 2.0, 6.5, and 7.4 respectively to obtain a solution with a concentration of 2 mg/mL. After sonication for 10 min, fix them on a shaker and shaking for 8 h at room temperature. After shaking, sonicate for 10 min and centrifuge for 15 min at 13,000 rpm. Pipette 0.1 mL of supernatant into a new tube, shake and rinse it with 5 mL, and discard the liquid. Transfer 0.5 mL of the supernatant again to a new tube, centrifuge it for 15 min at 13,000 rpm, then collect the supernatant (if necessary, dilute with water) and analyze it by LC-MS. The sample concentrations at 3~5 points are subjected to fitting and reticle for quantification.

The solubility of the compounds prepared in the synthesis examples is shown in the table below.

Table 12. Experimental results

| No. | Solubility (μg/mL) | No. | Solubility (μg/mL) | No. | Solubility (μg/mL) | No. | Solubility (μg/mL) | No. | Solubility (μg/mL) |
|---|---|---|---|---|---|---|---|---|---|
| T001 | 124.50 | T042 | 820.00 | T067 | 977.00 | T116 | 1085.50 | T133 | 757.00 |
| T006 | 1171.00 | T049 | 2314.00 | T069 | 633.00 | T118 | 2053.00 | T149 | 1580.00 |
| T007 | 608.00 | T055 | 144.00 | T070 | 927.00 | T122 | 1280.00 | T151 | 1345.00 |
| T022 | 1288.00 | T056 | 2001.00 | T072 | 2058.50 | T124 | 1949.00 | T152 | 815.00 |
| T027 | 478.00 | T064 | 400.00 | T074 | 133.00 | T127 | 1639.00 | | |

(continued)

| No. | Solubility (μg/mL) | No. | Solubility (μg/mL) | No. | Solubility (μg/mL) | No. | Solubility (μg/mL) | No. | Solubility (μg/mL) |
|---|---|---|---|---|---|---|---|---|---|
| T030 | 1518.00 | T065 | 782.00 | T078 | 800.00 | T131 | 278.00 | | |

Example 5: Assay of Binding Capacity of Compounds to Melanin

Pigmented eye tissues contain melanin in intracellular melanosomes. Drugs bind to melanin to varying degrees, ranging from non-binding to extensive binding. Therefore, melanin binding is an important feature that affects drug delivery and biodistribution in the eye. The binding capacity of the compound to the melanin must be considered in the development of ophthalmic drugs.

Reagent:

Melanin was derived from the melanin of cuttlefish (Sepia officinalis) and was commercially available from Sigma Aldrich (St Louis, MO).

Experimental procedures:

First, prepare 1 mg/mL melanin suspension with PBS (pH 7.4). As a washing step for the matrix, centrifuge the suspended particles for 5 min at 3000 rpm. Resuspend the resulting particles in a fresh PBS buffer, heat to 37 °C over continuous shaking and incubate it for 1 h. Accurately pipette 2 μL of the test stock solution (0.1 mM) into 98 μL of melanin suspension (1 mg/mL) to achieve a final incubation concentration of 2 μM. Similarly, accurately pipette 2 μL of the stock solution (0.1 mM) to 98 μL of PBS buffer (containing 0.1% BSA) as a blank control sample. Incubate the samples in a shaker (80-90 rpm) for 2 h at 37°C. Then, centrifuge the samples at room temperature. Pipette 50 μL of the supernatant and add 50 μL of precipitant (containing an internal standard). After protein precipitation of all the above samples, centrifuge the samples to collect the supernatants, dilute the supernatants with water (if necessary) and inject them for analysis. At the same time, measure the positive controls, including the high-binding compounds Sunitinib and chloroquine and the low-binding compound celecoxib. Establish an LC-MS/MS method for determining compound concentrations, and calibrate the peak area ratio of analyte/internal standard (Analyte/AIS) in the measured sample by the dilution factor to take it as an alternative concentration for the sample for data processing. The binding rate ($F_b$) of a compound in melanin was calculated as:

$$Fb = 100 - \frac{C_{test}}{C_{control}} \times 100\%$$

$C_{test}$ was the concentration of the compound in the melanin-incubated sample or the peak area ratio of the test compound to the internal standard. $C_{control}$ was the concentration of the compound in the blank control sample or the peak area ratio of the test compound to the internal standard. Percentage of free drug ($F_u$%) = 100 - $F_b$.

The binding rates of the compounds of the present invention to melanin are shown in the table below:

Table 13. Experimental results

| No. | Fu% | No. | Fu% | No. | Fu% | No. | Fu% | No. | Fu% |
|---|---|---|---|---|---|---|---|---|---|
| T001 | 1.29 | T078 | 2.41 | T095 | 4.69 | T110 | 4.85 | T126 | 4.95 |
| T027 | 4.21 | T079 | 5.26 | T096 | 7.92 | T111 | 4.49 | T127 | 3.62 |
| T030 | 2.32 | T080 | 5.82 | T097 | 8.17 | T112 | 2.22 | T129 | 8.89 |
| T053 | 2.25 | T081 | 6.32 | T098 | 6.66 | T113 | 2.68 | T130 | 7.09 |
| T056 | 1.83 | T082 | 7.57 | T099 | 8.84 | T114 | 3.33 | T131 | 6.29 |
| T058 | 2.25 | T083 | 7.49 | T100 | 5.44 | T115 | 1.48 | T132 | 3.63 |
| T062 | 1.95 | T085 | 6.12 | T101 | 7.02 | T116 | 3.76 | T134 | 4.01 |
| T063 | 8.71 | T087 | 3.74 | T102 | 2.35 | T117 | 2.07 | T135 | 2.00 |

(continued)

| No. | Fu% | No. | Fu% | No. | Fu% | No. | Fu% | No. | Fu% |
|---|---|---|---|---|---|---|---|---|---|
| T064 | 2.69 | T088 | 2.07 | T103 | 2.29 | T118 | 11.10 | T136 | 2.85 |
| T071 | 2.31 | T089 | 2.35 | T104 | 3.22 | T119 | 2.02 | T137 | 4.46 |
| T072 | 1.96 | T090 | 2.50 | T105 | 3.16 | T120 | 1.73 | T138 | 3.98 |
| T074 | 4.62 | T091 | 2.86 | T106 | 1.72 | T122 | 3.72 | T140 | 7.26 |
| T075 | 3.91 | T092 | 3.10 | T107 | 4.03 | T123 | 2.98 | T145 | 4.37 |
| T076 | 3.11 | T093 | 5.31 | T108 | 2.71 | T124 | 7.07 | | |
| T077 | 3.85 | T094 | 4.81 | T109 | 4.35 | T125 | 4.02 | | |

The test results show that the compounds of the present invention improve the percentage of the free drug, reduce the rate of binding to the melanin, and are able to interact with receptors in the eye tissues to increase the bioavailability. Comparatively, PAN90806 (T001) shows a lower percentage of free drug with respect to the melanin.

Example 7: Maximal Tolerated Dose (MTD) of Compounds Administered as Eye Drops

MTD is generally defined as a dose with which an "acceptable" level of toxicity is achieved during a particular study, or beyond which an animal is at an "unacceptable" risk of toxicity. It is sometimes referred to as the "minimum toxic dose". We used male Dutch black-banded rabbits to assess the MTD of the compounds administered in eye drops.

Experimental procedures:

Male Dutch black-banded rabbits (weighing 1.5 kg to 2.5 kg) were purchased from an eligible supplier. In this experimental protocol, the experimental procedures designed for application to animals were conducted after the approval of the Institutional Animal Care and Use Committee (IACUC). Any operation performed on the experimental animals were in compliance with the relevant requirements of IACUC experimental operations.

A total of 8 rabbits animals with good health conditions and normal eyes (without abnormalities such as trauma, congestion, redness and swelling) were randomly divided into 4 groups. These rabbits were binocularly administrated with (50 μl in each lower eyelid): the positive compound PAN90806 (T001) at a dose of 100 μg/eye, or 175 μg/eye; and T093 at a dose of 175 μg/eye, or 250 μg/eye. Eye drop administration was conducted for two consecutive days, 3 times a day, at an interval of 6 ± 0.5 hours. Before administration, the status of the dosing preparation was inspected, and its homogeneity was ensured by means of vortexing, stirring, or shaking. Before each administration, the states of rabbit eyes were observed, including but not limited to eye color, substances around the eyes, conjunctival swelling, eye exudate, discharge, cornea, pupillary reflex, etc. Subsequently, the toxicity risk assessment for six consecutive days of eye drop administration was further conducted. A total of 8 rabbits were randomly divided into 2 groups. The rabbits were binocularly administered with (50 μl in each lower eyelid): the positive compound PAN90806 at a dose of 100 μg/eye; and T093 at a dose of 250 mcg/eye. Eye drop administration was conducted 3 times a day, at an interval of 6 ± 0.5 hours. In addition, similar method was used to test the MTDs of T078 and T116 after three consecutive days of eye drop administration at a dose of 1000 μg/eye.

Experimental results:

On the second day of eye drop administration of the positive compound PAN90806 (175 μg/eye), one of the rabbits showed a reddish left eyelid. On the third day of eye drop administration, one rabbit showed reddish left and right eyelids, and one rabbit showed a red left eyelid with white intraocular discharge and a red right eyelid with yellow intraocular discharge. No abnormalities were observed in the eyes of any rabbit in the two dose groups (175 μg/eye and 250 μg/eye) of T093. Therefore, on the third day of eye drop administration, the MTD of PAN90806 was 100 μg/eye, and the MTD of T093 was 250 μg/eye or higher.

The results of toxicity risk assessment for six consecutive days of eye drop administration showed that on the seventh day of eye drop administration with the positive compound PAN90806 (100 μg/eye), ocular toxicity (corneal opacity and conjunctival swelling, or corneal edema and conjunctival hyperemia, or ocular discharge) was observed in all the 4 rabbits. In the T093 (250 μg/eye) group, no abnormalities were observed in the eyes of all rabbits. Therefore, on the eighth day of eye drop administration, the MTD of PAN90806 was 50 μg/eye, and the MTD of T093 was 250 μg/eye or higher. Based on this, the dose was selected for the subsequent animal efficacy test, with 250 μg/eye for T093 and 50

μg/eye for the positive compound PAN90806.

T078 and T116 were administered as eye drops at a dose of 1000 μg/eye for three consecutive days, and the results of toxicity risk assessment showed that no abnormalities were observed in the eyes of all rabbits. Therefore, the MTDs of T078 and T116 on the fourth day of eye drop administration were 1000 μg/eye or higher.

Described above provide only preferred embodiments of the present invention, which are not intended to limit the present invention. Any modification, equivalent replacement or the like made within the spirit and principle of the present invention shall be regarded as falling within the protection scope of the present invention.

The foregoing embodiments and methods described in the present invention may vary depending on the ability, experience and preferences of those skilled in the art.

The method steps are listed only in a certain order in the present invention, which, however, does not impose any limitation on the order of the method steps.

## Claims

1. A compound or a pharmaceutically acceptable salt, stereoisomer, ester, prodrug, solvate, and deuterated compound thereof, wherein said compound has a structure of:

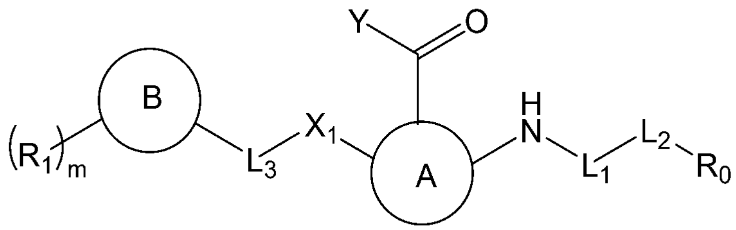

(I)

wherein,

Ring A is a 5-7 membered heteroaromatic ring;
Ring B is a $C_6$-$C_{10}$ aromatic ring or a 5-10 membered heterocyclic ring; optionally, said $C_6$-$C_{10}$ aromatic ring or 5-10 membered heterocyclic ring may be fused with a $C_6$-$C_{10}$ aromatic ring, a $C_5$-$C_8$ aliphatic ring, or a 5-10 membered heterocyclic ring;
$X_1$ is O or S;
Y is -N($C_0$-$C_{10}$ alkyl)($C_0$-$C_{10}$ alkyl) or -O($C_0$-$C_{10}$ alkyl);
$L_1$ is selected from: a single bond, -C(O)-, -C(O)O-, -C(O)$NR_3$-, -C(O)N($R_3$)O-, -$S(O)_2$-, - $S(O)_2NR_3$-, -S(O)-, -S(O)$NR_3$-, -Cy-; -Cy- is selected from: substituted or unsubstituted cycloalkylene, substituted or unsubstituted arylene, and substituted or unsubstituted heterocyclylene;
$L_2$ is a single bond or alkylene, wherein one or more methylene units in alkylene are optionally and independently substituted by a group selected from:

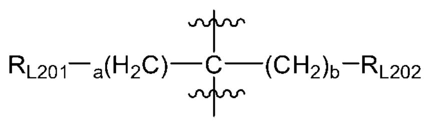

,

N($R_4$)-, -N($R_4$)C(O)-, -C(O)N($R_4$)-, -N($R_4$)$S(O)_2$-, -$S(O)_2$N($R_4$)-, -O-, -C(O)-, -OC(O)-, -C(O)O-, -S-, -S(O)- or -$S(O)_2$; wherein, a and b are independently integers from 0 to 10, and $R_{L201}$ and $R_{L202}$ are independently selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, -$OR_{L203}$, -C(O)$R_{L203}$, -C(O)$OR_{L203}$, - $NR_{L204}$C(O)$OR_{L203}$, -OC(O)$R_{L203}$, -$NR_{L204}SO_2R_{L203}$, -$SO_2NR_{L203}R_{L204}$, -$NR_{L204}$C(O)$R_{L203}$, - C(O)$NR_{L203}R_{L204}$, -$NR_{L203}R_{L204}$, -$SR_{L203}$, -S(O)$R_{L203}$, -$S(O)_2R_{L203}$, -$SO_3H$, $C_3$-$C_6$ cycloalkyl, cycloalkyl alkyl, heterocyclyl, heterocyclylalkyl; wherein, $R_{L203}$ and $R_{L204}$ are independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl;
$L_3$ is alkylene, wherein one or more methylene units in alkylene are optionally and independently substituted by a

group selected from:

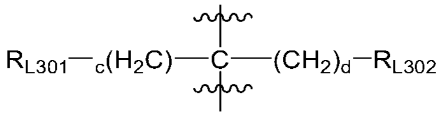

,

$-N(R_5)-$, $-N(R_5)C(O)-$, $-C(O)N(R_5)-$, $-N(R_5)S(O)_2-$, $-S(O)_2N(R_5)-$, -O-, -C(O)-, -OC(O)-, -C(O)O-, -S-, -S(O)- or $-S(O)_2$;

wherein, c and d are independently integers from 0 to 10, and $R_{L301}$ and $R_{L302}$ are independently selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, $-OR_{L303}$, $-C(O)R_{L303}$, $-C(O)OR_{L303}$, $-NR_{L304}C(O)OR_{L303}$, $-OC(O)R_{L303}$, $-NR_{L304}SO_2R_{L303}$, $-SO_2NR_{L303}R_{L304}$, $-NR_{L304}C(O)R_{L303}$, $-C(O)NR_{L303}R_{L304}$, $-NR_{L303}R_{L304}$, $-SR_{L303}$, $-S(O)R_{L303}$, $-S(O)_2R_{L303}$, $-SO_3H$, $C_3$-$C_6$ cycloalkyl, cycloalkyl alkyl, heterocyclyl, heterocyclylalkyl; wherein, $R_{L303}$ and $R_{L304}$ are independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl;

$R_3$ to $R_5$ are each independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl, wherein said alkyl, cycloalkyl, or heterocyclyl is optionally substituted by a group selected from: halogen, cyano, nitro, azido, -OR', -C(O)R', -C(O)OR', -OC(O)R', -NR'C(O)OR", $-NR'SO_2R"$, $-SO_2NR'R"$, - NR'C(O)R", -C(O)NR'R", -NR'R", -SR', -SOR', $-SO_2R'$, $-SO_3H$, $C_1$-$C_{10}$ alkyl, $C_1$-$C_{10}$ haloalkyl, $C_1$-$C_{10}$ silyl, $C_3$-$C_{10}$ cycloalkyl, phenyl, 4-10 membered heterocyclyl;

$R_1$ is one or more independent substituents on Ring B, each $R_1$ is independently selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, $-OR_{101}$, $-C(O)R_{101}$, $-C(O)OR_{101}$, $-NR_{102}C(O)OR_{101}$, $-OC(O)R_{101}$, $-NR_{102}SO_2R_{101}$, $-SO_2NR_{101}R_{102}$, $-NR_{102}C(O)R_{101}$, $-C(O)NR_{101}R_{102}$, $-NR_{101}R_{102}$, $-S(O)_jR_{101}$, where j is an integer from 0 to 2, $-SO_3H$, $-NR_{102}(CR_{103}R_{104})_tOR_{101}$, $-(CH_2)_t(C_6$-$C_{10}$ aryl), $-SO_2(CH_2)_t(C_6$-$C_{10}$ aryl), $-S(CH_2)_t(C_6$-$C_{10}$ aryl), $-O(CH_2)_t(C_6$-$C_{10}$ aryl), $-(CH_2)_t$(4-10 membered heterocyclyl), - $SO_2(CH_2)_t$(4-10 membered heterocyclyl), $-S(CH_2)_t$(4-10 membered heterocyclyl), $-O(CH_2)_t$(4-10 membered heterocyclyl), $-(CH_2)_t(C_3$-$C_{10}$ cycloalkyl), $-SO_2(CH_2)_t(C_3$-$C_{10}$ cycloalkyl), - $S(CH_2)_t(C_3$-$C_{10}$ cycloalkyl), $-O(CH_2)_t(C_3$-$C_{10}$ cycloalkyl), where t is an integer from 0 to 5; wherein, said $C_1$-$C_{10}$ alkyl, $C_6$-$C_{10}$ aryl, or 4-10 membered heterocyclyl is optionally substituted by a group selected from: halogen, cyano, nitro, azido, -OR', -C(O)R', -C(O)OR', -OC(O)R', - NR'C(O)OR", $-NR'SO_2R"$, $-SO_2NR'R"$, -NR'C(O)R", -C(O)NR'R", -NR'R", -SR', -SOR', $-SO_2R'$, $-SO_3H$, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl;

$R_{101}$ to $R_{104}$ are each independently selected from: H, substituted or unsubstituted alkyl, substituted or unsubstituted cycloalkyl, substituted or unsubstituted cycloalkyl alkyl, substituted or unsubstituted heterocyclyl, substituted or unsubstituted heterocyclylalkyl, and $C_1$-$C_{10}$ silyl; wherein said alkyl, cycloalkyl, or heterocyclyl is optionally substituted by a group selected from: halogen, cyano, nitro, azido, -OR', -C(O)R', -C(O)OR', -OC(O)R', -NR'C(O)OR", $-NR'SO_2R"$, - $SO_2NR'R"$, -NR'C(O)R", -C(O)NR'R", -NR'R", -SR', -SOR', $-SO_2R'$, $-SO_3H$, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl;

m is an integer from 1 to 5 (for example, 1, 2, 3, 4, or 5, where valency permits);

$R_0$ is selected from: H, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, $-OR_{001}$, $-C(O)R_{001}$, $-C(O)OR_{001}$, $-NR_{002}C(O)OR_{001}$, - $OC(O)R_{001}$, $-NR_{002}SO_2R_{001}$, $-SO_2NR_{001}R_{002}$, $-NR_{002}C(O)R_{001}$, $-C(O)NR_{001}R_{002}$, $-NR_{001}R_{002}$, - $S(O)_iR_{002}$ (where i is an integer from 0 to 2), $-SO_3H$, $-NR_{002}(CR_{003}R_{004})_tOR_{001}$, and

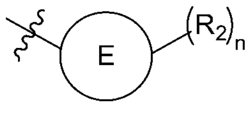

;

wherein, Ring E is a $C_6$-$C_{10}$ aromatic ring or a 4-10 membered heterocyclic ring; optionally, said $C_6$-$C_{10}$ aromatic ring or 4-10 membered heterocyclic ring may be fused with a $C_6$-$C_{10}$ aromatic ring, a $C_5$-$C_8$ aliphatic ring, or a 4-10 membered heterocyclic ring;

$R_2$ is selected from: H, =O, $C_1$-$C_{10}$ alkyl, $C_2$-$C_{10}$ alkenyl, $C_2$-$C_{10}$ alkynyl, halogen, cyano, nitro, trifluoromethyl, trifluoromethoxy, azido, $-OR_{201}$, $-C(O)R_{201}$, $-C(O)OR_{201}$, $-NR_{202}C(O)OR_{201}$, - $OC(O)R_{201}$, $-NR_{202}SO_2R_{201}$, $-SO_2NR_{201}R_{202}$, $-NR_{202}C(O)R_{201}$, $-C(O)NR_{201}R_{202}$, $-NR_{201}R_{202}$, - $S(O)_iR_{201}$, where i is an integer from 0 to 2, $-SO_3H$, $-(CH_2)_j(C_6$-$C_{10}$ aryl), $-SO_2(CH_2)_j(C_6$-$C_{10}$ aryl), $-S(CH_2)_j(C_6$-$C_{10}$ aryl), $-O(CH_2)_j(C_6$-$C_{10}$ aryl), $-(CH_2)_j$(4-10

membered heterocyclyl), - $SO_2(CH_2)_j$(4-10 membered heterocyclyl), -$S(CH_2)_j$(4-10 membered heterocyclyl), -$O(CH_2)_j$(4-10 membered heterocyclyl), -$(CH_2)_j$($C_3$-$C_{10}$ cycloalkyl), -$SO_2(CH_2)_j$($C_3$-$C_{10}$ cycloalkyl), - $S(CH_2)_j$ ($C_3$-$C_{10}$ cycloalkyl), and -$O(CH_2)_j$($C_3$-$C_{10}$ cycloalkyl), where j is an integer from 0 to 5;

wherein, said $C_1$-$C_{10}$ alkyl, $C_6$-$C_{10}$ aryl, or 4-10 membered heterocyclyl is optionally substituted by a group selected from: halogen, cyano, nitro, azido, -OR', -C(O)R', -C(O)OR', -OC(O)R', - NR'C(O)OR", -NR'$SO_2$R", -$SO_2$NR'R", -NR'C(O)R", -C(O)NR'R", -NR'R", -SR', -SOR', -$SO_2$R', -$SO_3$H, $C_3$-$C_{10}$ cycloalkyl, phenyl, and 4-10 membered heterocyclyl;

n is an integer from 1 to 5 (for example, 1, 2, 3, 4, or 5, where valency permits);

$R_{001}$ to $R_{004}$ are each independently selected from: H, substituted or unsubstituted alkyl, substituted or unsubstituted cycloalkyl, substituted or unsubstituted cycloalkyl alkyl, substituted or unsubstituted heterocyclyl, substituted or unsubstituted heterocyclylalkyl, and $C_1$-$C_{10}$ silyl, wherein said alkyl, cycloalkyl, or heterocyclyl is optionally substituted by a group selected from: halogen, cyano, nitro, azido, -OR', -C(O)R', -C(O)OR', -OC(O)R', -NR'C(O)OR", -NR'$SO_2$R", - $SO_2$NR'R", -NR'C(O)R", -C(O)NR'R", -NR'R", -SR', -SOR', -$SO_2$R', -$SO_3$H, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl;

$R_{201}$ to $R_{204}$ are each independently selected from: H, substituted or unsubstituted alkyl, substituted or unsubstituted cycloalkyl, substituted or unsubstituted cycloalkyl alkyl, substituted or unsubstituted heterocyclyl, substituted or unsubstituted heterocyclylalkyl, and $C_1$-$C_{10}$ silyl, wherein said alkyl, cycloalkyl, or heterocyclyl is optionally substituted by a group selected from: halogen, cyano, nitro, azido, -OR', -C(O)R', -C(O)OR', -OC(O)R', -NR'C(O)OR", -NR'$SO_2$R", - $SO_2$NR'R", -NR'C(O)R", -C(O)NR'R", -NR'R", -SR', -SOR', -$SO_2$R', -$SO_3$H, $C_1$-$C_{10}$ alkyl, $C_1$-$C_{10}$ haloalkyl, $C_1$-$C_{10}$ silyl, $C_3$-$C_{10}$ cycloalkyl, phenyl, and 4-10 membered heterocyclyl; and R' and R" are independently selected from: H, substituted or unsubstituted alkyl, substituted or unsubstituted cycloalkyl, substituted or unsubstituted cycloalkyl alkyl, substituted or unsubstituted heterocyclyl, and substituted or unsubstituted heterocyclylalkyl.

**2.** The compound according to claim 1, wherein Ring A is

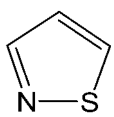 , 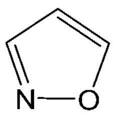 , 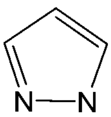 ,

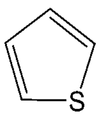 , 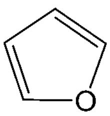 , 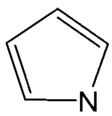 , 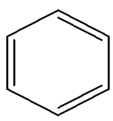 , 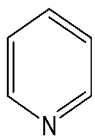 , 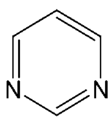 , or 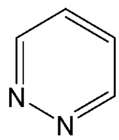 ,

particularly

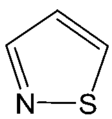 ;

and preferably, the

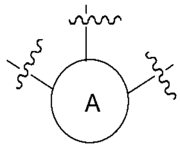

moiety has a structure of:

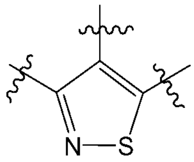, 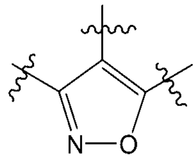,

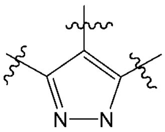, 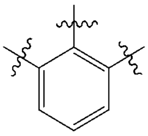, 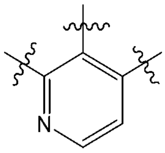, 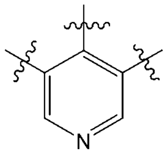, 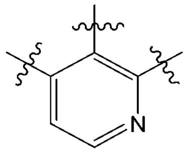, 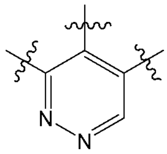,

or

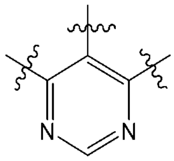,

particularly

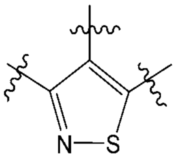.

3. The compound according to claim 1 or 2, wherein Ring B is a benzene ring, a benzo-fused aliphatic ring, or a heterocyclic ring; preferably, Ring B is selected from:

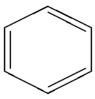, 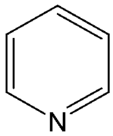, 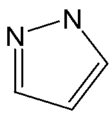, 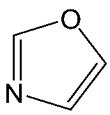, 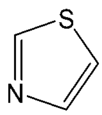,

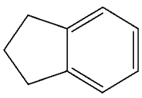, 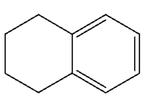, 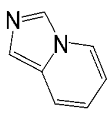, 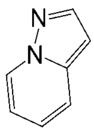, 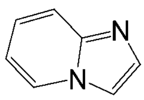, 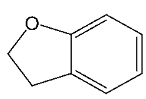, 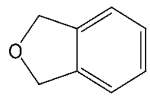,

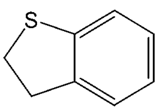, 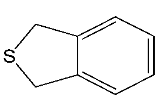, 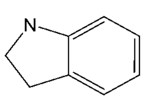, 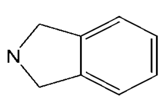, 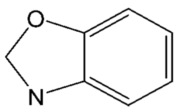, 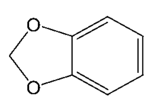,

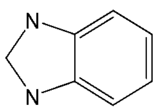, 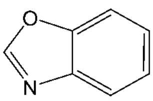, 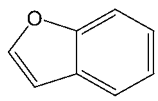, 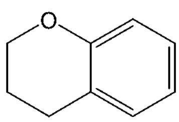, 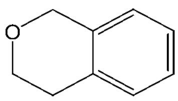, and 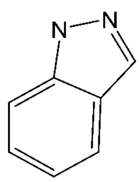;

and more preferably, the

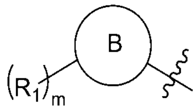

moiety is selected from the following structures:

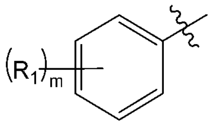 , 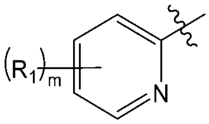 , 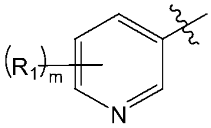 , 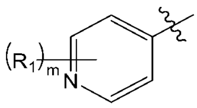 , 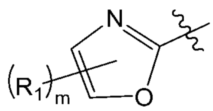 ,

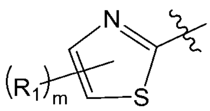 , 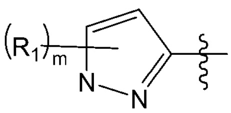 , 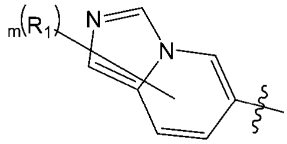 , 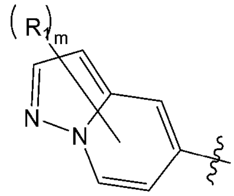 ,

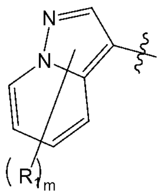 , 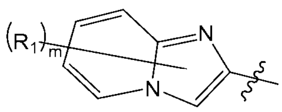 , 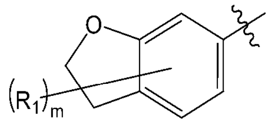 , 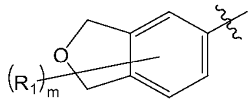 ,

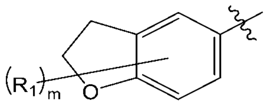 , 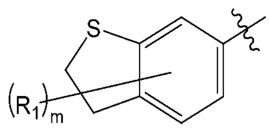 , 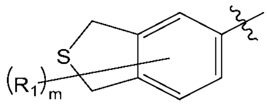 , 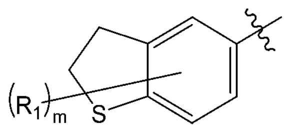 ,

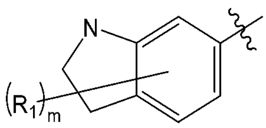 , 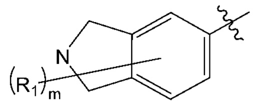 , 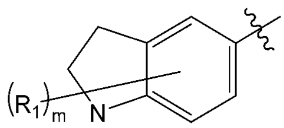 , 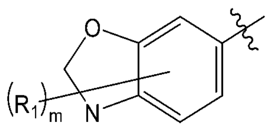 ,

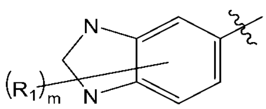 , 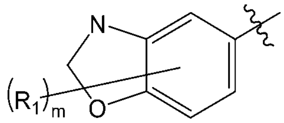 , 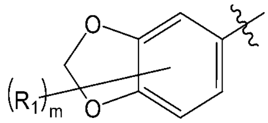 , 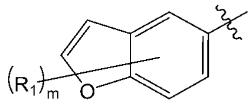 ,

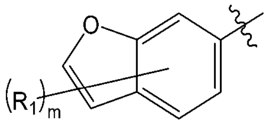 , 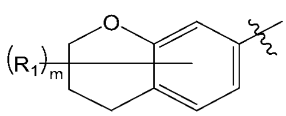 , 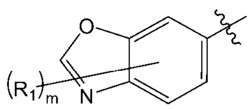 , 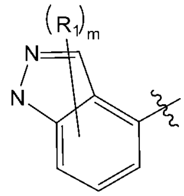 ,

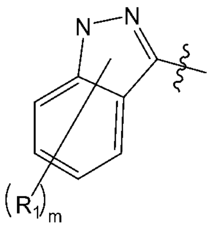 , 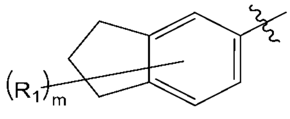 , and 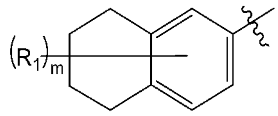 .

4. The compound according to any one of claims 1 to 3, wherein each $R_1$ is independently selected from: $C_1$-$C_6$ alkyl, halogen, $C_1$-$C_6$ haloalkyl, cyano, nitro, azido, $-OR_{101}$, $-C(O)R_{101}$, - $C(O)OR_{101}$, $-NHC(O)OR_{101}$, $-OC(O)R_{101}$, $-NHSO_2R_{101}$, $-SO_2NR_{101}R_{102}$, $-NHC(O)R_{101}$, - $C(O)NR_{101}R_{102}$, $-NR_{101}R_{102}$, $-SR_{101}$, $-S(O)_2R_{101}$, $-SO_3H$, $-(CH_2)_t$(phenyl), $-(CH_2)_t$(4-10 membered heterocyclyl), and $-(CH_2)_t(C_3$-$C_{10}$ cycloalkyl), where t is an integer form 0 to 5; preferably, $R_{101}$ and $R_{102}$ are independently selected from: H, $C_1$-$C_6$ alkyl (for example, $-CH_3$,

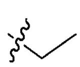 , 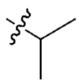 , 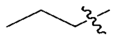 , 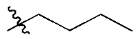 , or 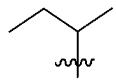 ),

$C_1$-$C_6$ haloalkyl (for example, $-CHF_2$, $-CH_2F$, $-CF_3$, $-CH_2$-$CH_2F$, $-CH_2$-$CHF_2$, $-CH_2$-$CF_3$, $-CH_2CH_2$-$CF_3$, and $-CH_2CH_2CH_2$-$CF_3$), $C_1$-$C_6$ hydroxyl-substituted alkyl (for example,

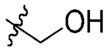 , 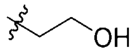 , or 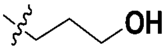

), $C_1$-$C_6$ alkoxy-substituted alkyl (for example,

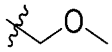 , 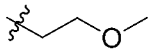 , or 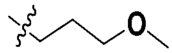

), $C_1$-$C_6$ amino-substituted alkyl (for example,

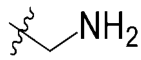 , 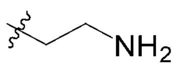 , or 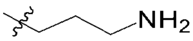

), $C_1$-$C_6$ alkylamino-substituted alkyl (for example,

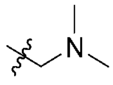 , 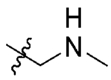 , or 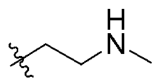

), $C_3$-$C_{10}$ cycloalkyl (for example,

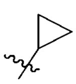 , 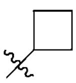 , 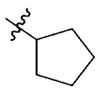 , 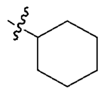 , 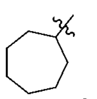 , 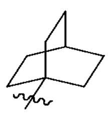 , 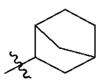 , 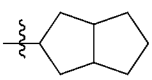 , 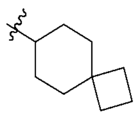 ,

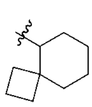 , or 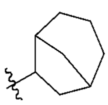

), $C_4$-$C_{10}$ cycloalkyl alkyl (for example,

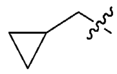, 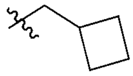, 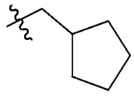,

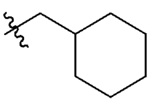, or 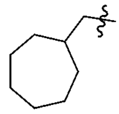

), substituted or unsubstituted 4-10 membered heterocyclyl (for example,

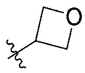, 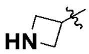, 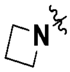, 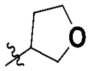, 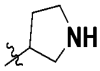, 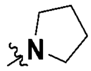, 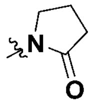, 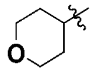, 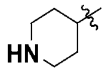, 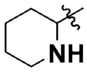,

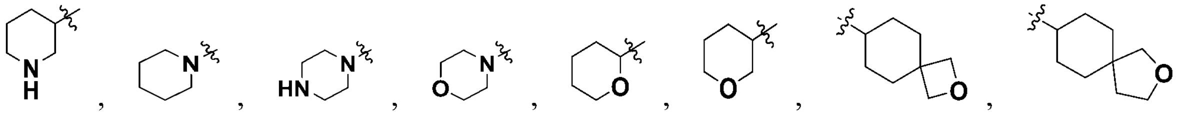,

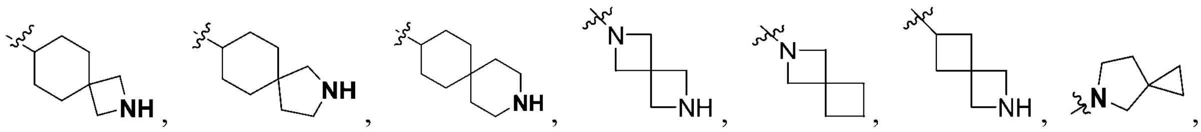,

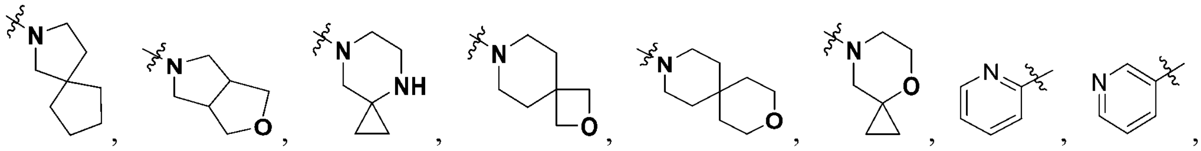,

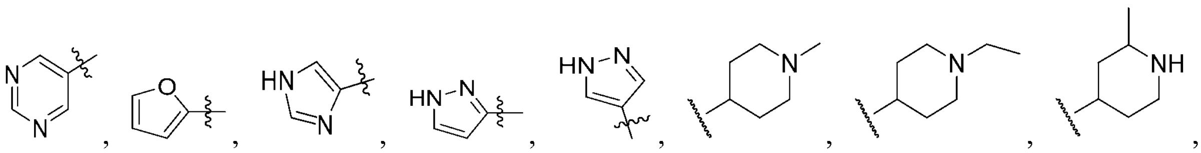,

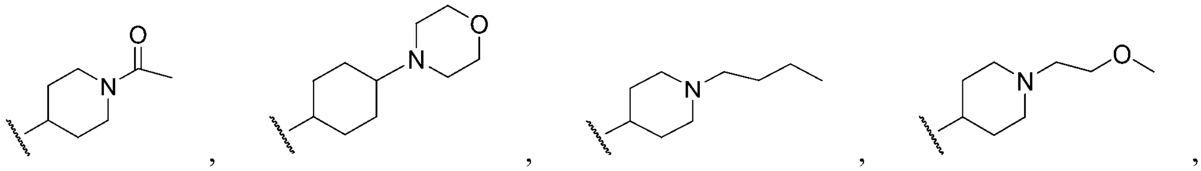,

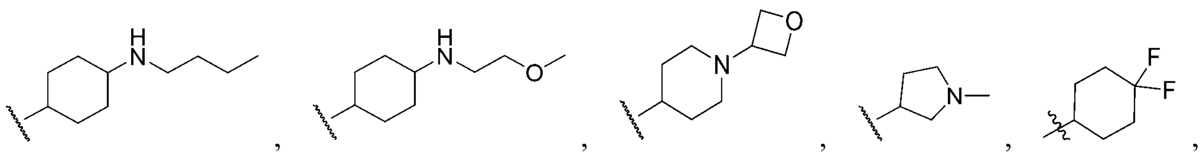,

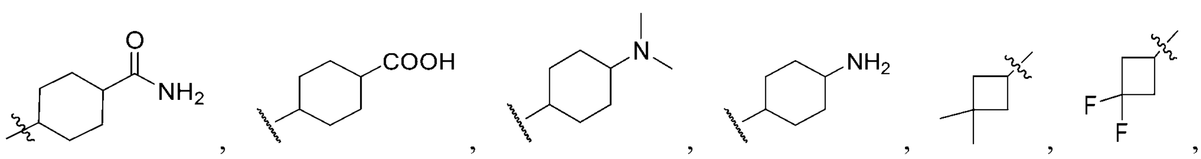,

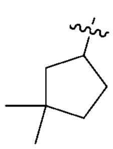, 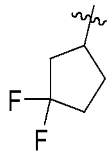, 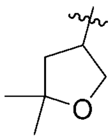, or 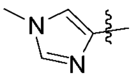), 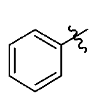, 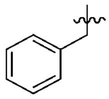,

$C_1$-$C_{10}$ silyl-substituted alkyl (for example,

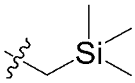, 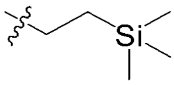, or 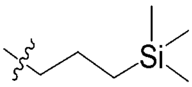

), and $C_1$-$C_{10}$ silyl (for example,

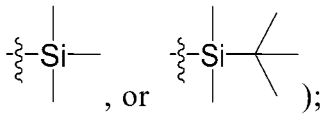, or );

more preferably, each $R_1$ is independently selected from: H, methyl, ethyl, n-propyl, isopropyl, $-CF_3$, $-CHF_2$, $-CH_2F$,

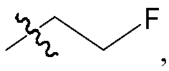, 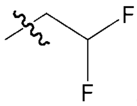, 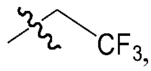,

F, Cl, Br, I, cyano, nitro, azido, -OH,

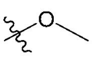, 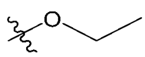, 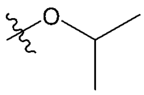, 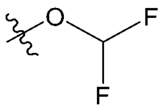, 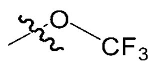, 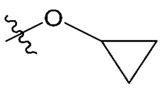, 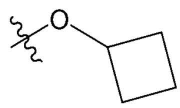,

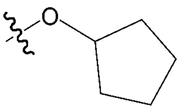, 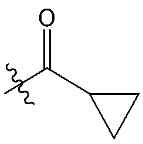, 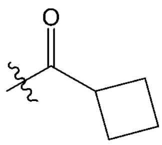, 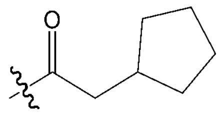, 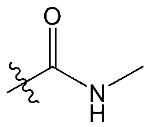, 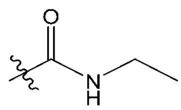,

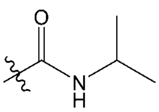, 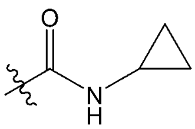, 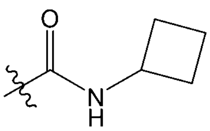, 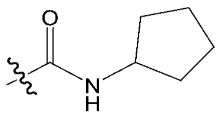, 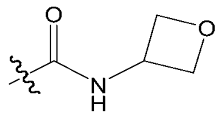,

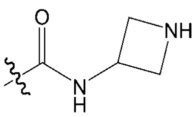, 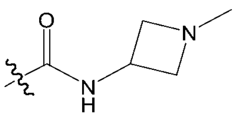, 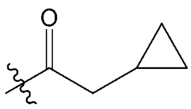, 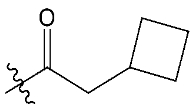, 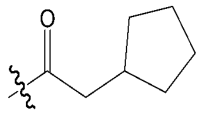,

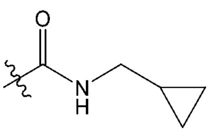, 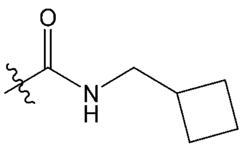, 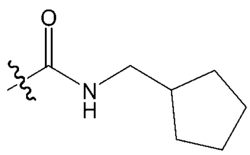, 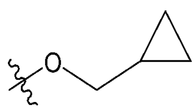,

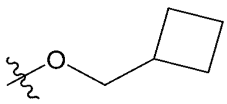, 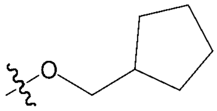, 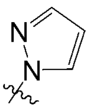, 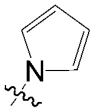, 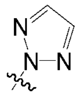, 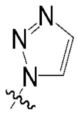, 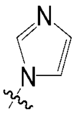, 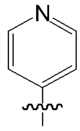,

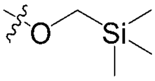, 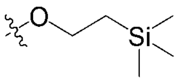, 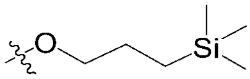, 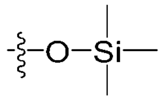, and 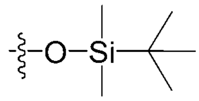.

**5.** The compound according to any one of claims 1 to 4, wherein $R_0$ is

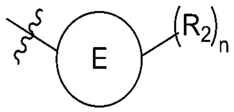,

Ring E is a 4-10 membered heterocyclic ring, particularly a 4-8 membered saturated heterocyclic ring; preferably, Ring E is selected from:

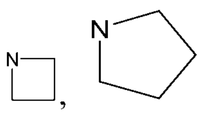, 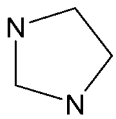, 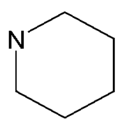, 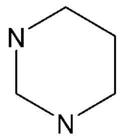, 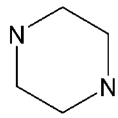,

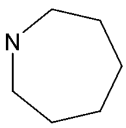, 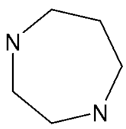, 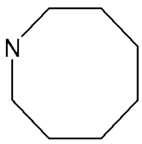, 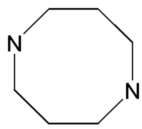, 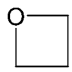, 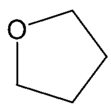, 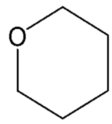, 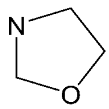,

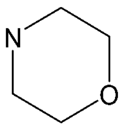, 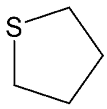, 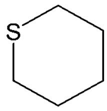, 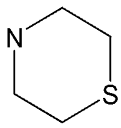, 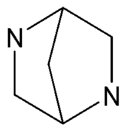, 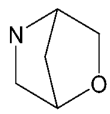, 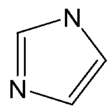, 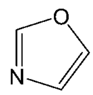, 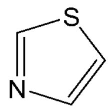,

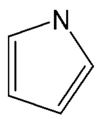, 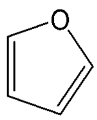, and 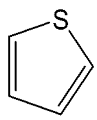;

and more preferably, the

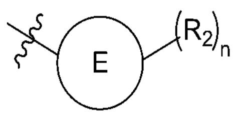

moiety is

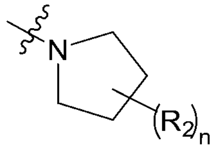 , 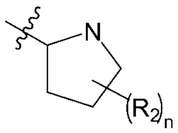 ,

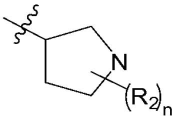 , 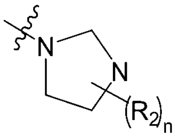 , 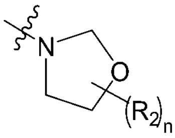 , 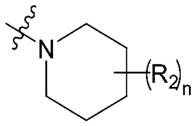 , 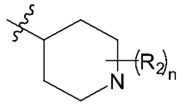 ,

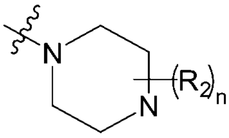 , 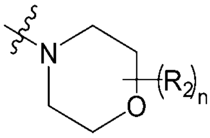 , or 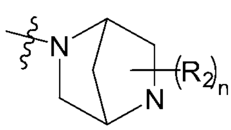 .

**6.** The compound according to any one of claims 1 to 5, wherein $R_2$ is selected from: H, =O, $C_1$-$C_6$ alkyl, $C_1$-$C_6$ haloalkyl, halogen, cyano, nitro, azido, $-OR_{201}$, $-C(O)R_{201}$, $-C(O)OR_{201}$, - $NHC(O)OR_{201}$, $-OC(O)R_{201}$, $-NHSO_2R_{201}$, $-SO_2NR_{201}R_{202}$, $-NHC(O)R_{201}$, $-C(O)NR_{201}R_{202}$, - $NR_{201}R_{202}$, $-SR_{201}$, $-S(O)_2R_{201}$, $-SO_3H$, $-(CH_2)_j$(phenyl), $-(CH_2)_j$(4-10 membered heterocyclyl), and $-(CH_2)_j$($C_3$-$C_{10}$ cycloalkyl), where j is an integer from 0 to 5; preferably, $R_{201}$ and $R_{202}$ are independently selected from: H, $C_1$-$C_6$ alkyl (for example, $-CH_3$,

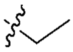 , 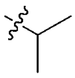 , 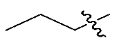 , 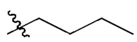 , or 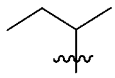

), $C_1$-$C_6$ haloalkyl (for example, $-CHF_2$, $-CH_2F$, $-CF_3$, $-CH_2$-$CH_2F$, $-CH_2$-$CHF_2$, $-CH_2$-$CF_3$, $-CH_2CH_2$-$CF_3$, and $-CH_2CH_2CH_2$-$CF_3$), $C_1$-$C_6$ hydroxyl-substituted alkyl (for example,

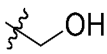 , 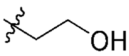 , or 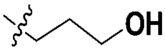

), $C_1$-$C_6$ alkoxy-substituted alkyl (for example,

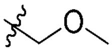 , 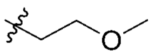 , or 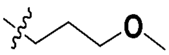

), $C_1$-$C_6$ amino-substituted alkyl (for example,

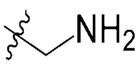 , 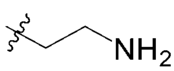 , or 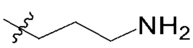

), $C_1$-$C_6$ alkylamino-substituted alkyl (for example,

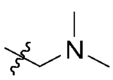 , 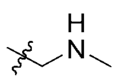 , or 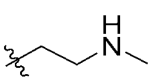

), $C_3$-$C_{10}$ cycloalkyl (for example,

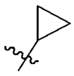, 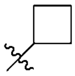, 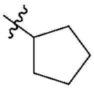, 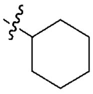, 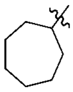, 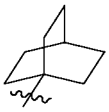, 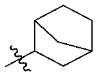, 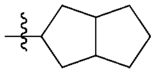, 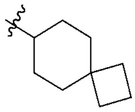,

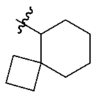, or 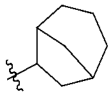

), $C_4$-$C_{10}$ cycloalkyl alkyl (for example,

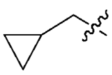, 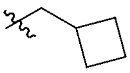, 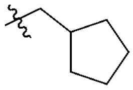,

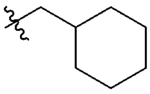, or 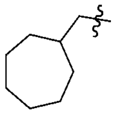

), and substituted or unsubstituted 4-10 membered heterocyclyl (for example,

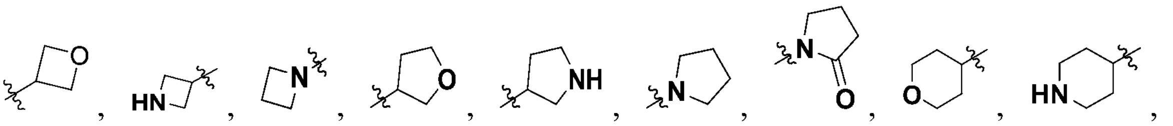

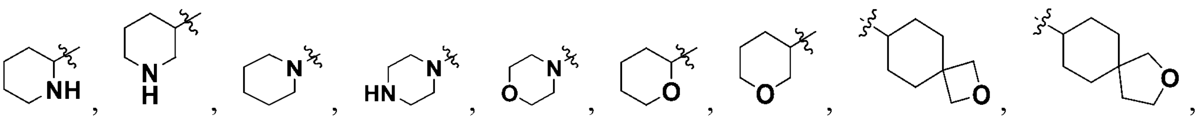

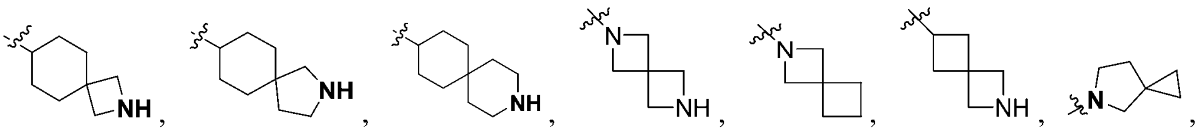

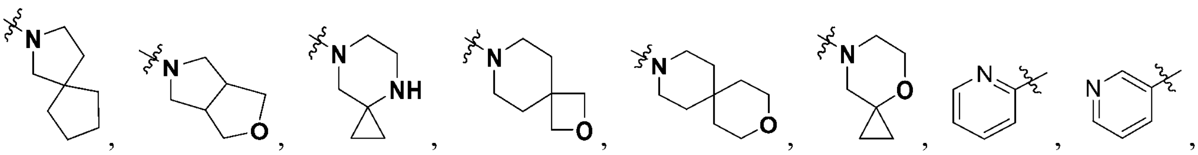

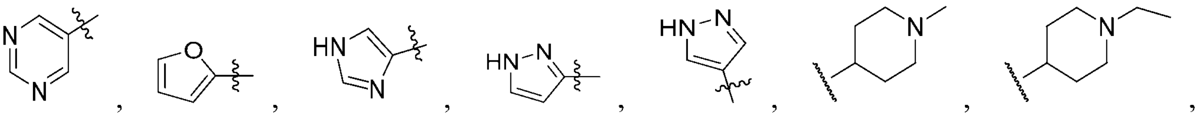

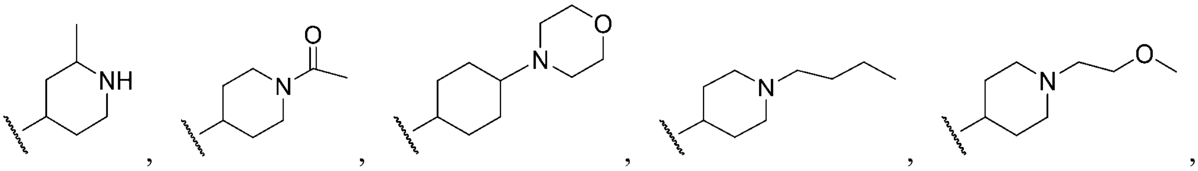

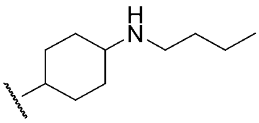, 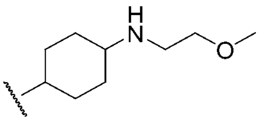, 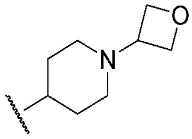, 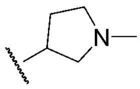, 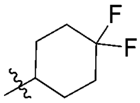,

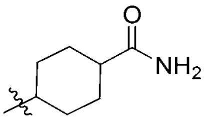, 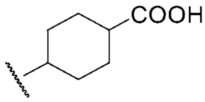, 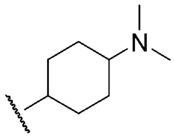, 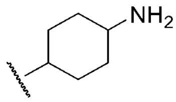, 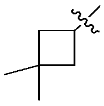, 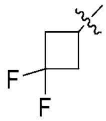,

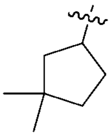, 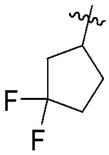, 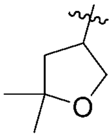, 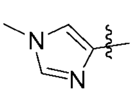), 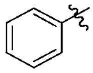, or 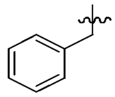;

more preferably, $R_2$ is selected from: H, methyl, ethyl, n-propyl, isopropyl, $-CF_3$, $-CHF_2$, $-CH_2F$,

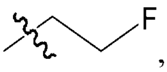, 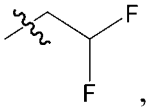, 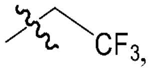

F, Cl, Br, I, cyano, nitro, azido, hydroxyl, methoxy, ethoxy,

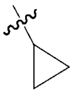, 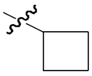, 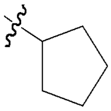, 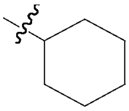, 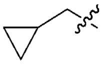, 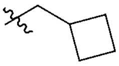, 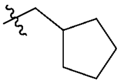, 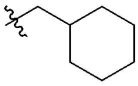, 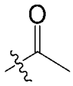,

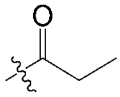, and 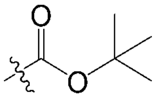;

and

further preferably, $R_0$ is selected from:

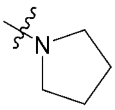 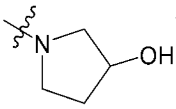 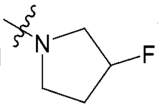 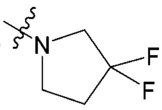 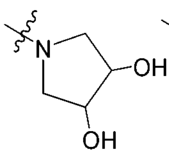 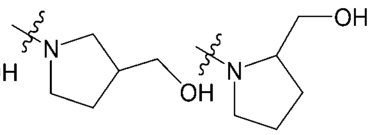

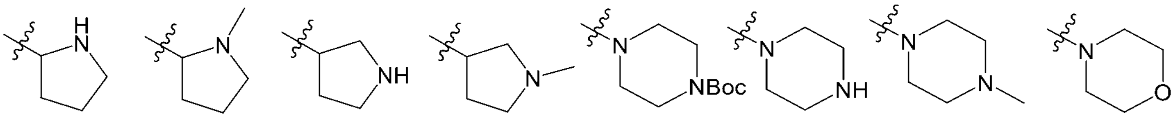

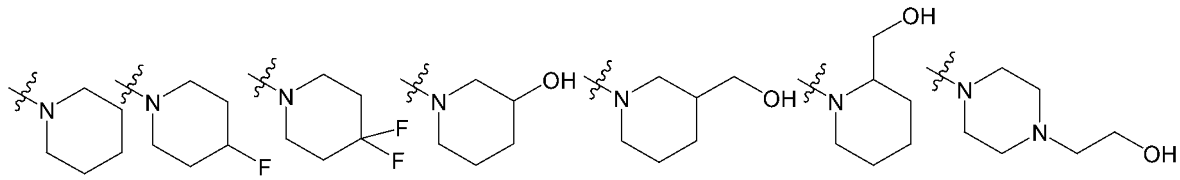

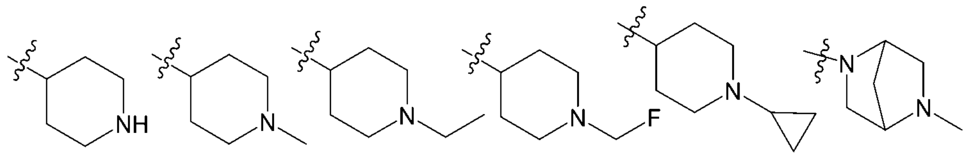

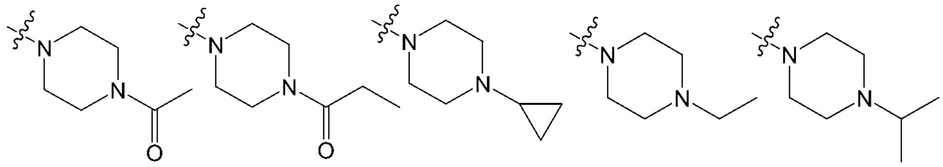
.

7. The compound according to any one of claims 1 to 4, wherein $R_0$ is $-NR_{001}R_{002}$, wherein $R_{001}$ and $R_{002}$ are independently selected from: H, $C_1$-$C_6$ alkyl, $C_1$-$C_6$ haloalkyl, $C_1$-$C_6$ hydroxyl-substituted alkyl, $C_1$-$C_6$ alkoxy-substituted alkyl, $C_1$-$C_6$ amino-substituted alkyl, $C_1$-$C_6$ alkylamino-substituted alkyl, $C_3$-$C_6$ cycloalkyl, and $C_4$-$C_{10}$ cycloalkyl alkyl; preferably, $R_0$ is selected from:

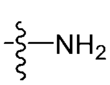, 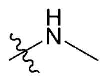, 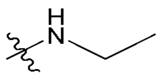, 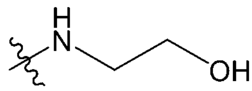,

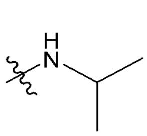, 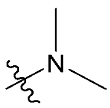, 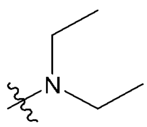, 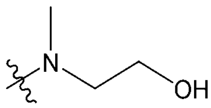, 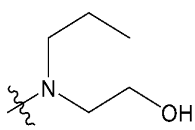, and 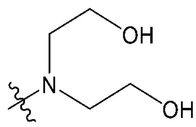;

or,

$R_0$ is selected from: H, cyano, -O($C_0$-$C_{10}$ alkyl), -O($C_1$-$C_{10}$ silyl), -S($C_0$-$C_{10}$ alkyl), -C(O)($C_0$-$C_{10}$ alkyl), -C(O)O($C_0$-$C_{10}$ alkyl), -OC(O)($C_0$-$C_{10}$ alkyl), -N($C_0$-$C_{10}$ alkyl)$SO_2$($C_0$-$C_{10}$ alkyl), - $SO_2$N($C_0$-$C_{10}$ alkyl)($C_0$-$C_{10}$ alkyl), -N($C_0$-$C_{10}$ alkyl)C(O)($C_0$-$C_{10}$ alkyl), -C(O)N($C_0$-$C_{10}$ alkyl)($C_0$-$C_{10}$ alkyl), and -$SO_2$($C_0$-$C_{10}$ alkyl), wherein said alkyl is optionally substituted by a group selected from: halogen, cyano, hydroxyl, amino, $C_1$-$C_6$ alkoxy, $C_1$-$C_6$ alkylamino, and $C_3$-$C_6$ cycloalkyl; and
preferably, $R_0$ is selected from: H, cyano, -OH,

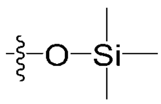, 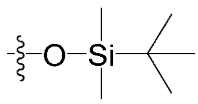, 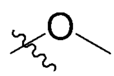,

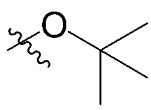, 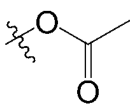, 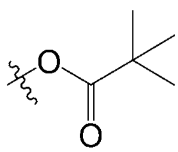,

-COOH, and

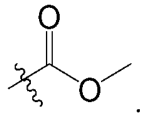.

8. The compound according to any one of claims 1 to 7, wherein $L_1$ is -C(O)NH-, -Cy-, a single bond or -C(O)-; preferably, -Cy- is selected from:

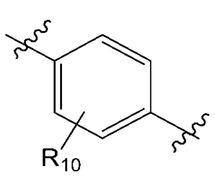, 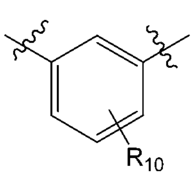, 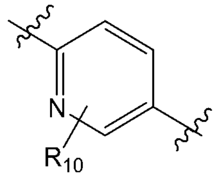,

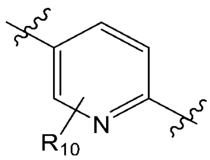, 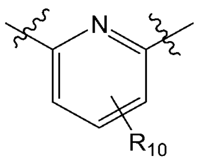, 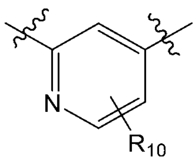, 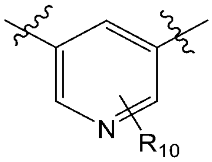, 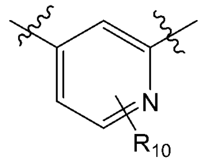,

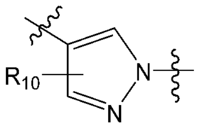, 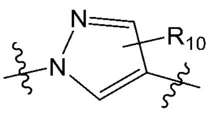, 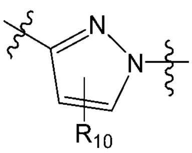, 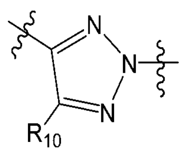, 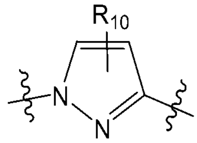,

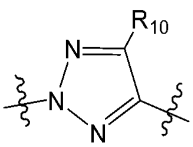, 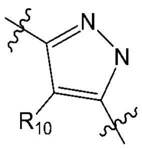, 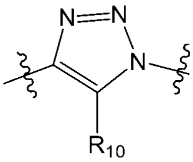, 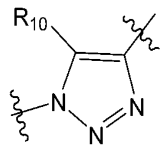, 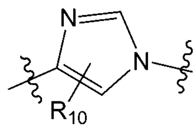, 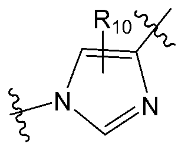,

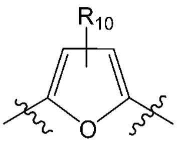, 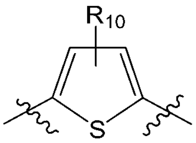, 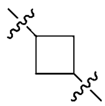, 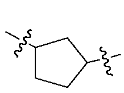, 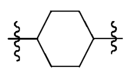, 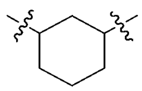, 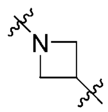,

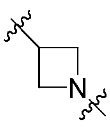, 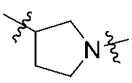, 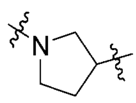, 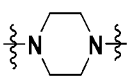, 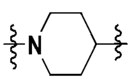, 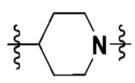, and 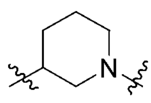;

each $R_{10}$ is independently selected from: H, halogen, $C_1$-$C_6$ alkyl, $C_1$-$C_6$ haloalkyl, $C_1$-$C_6$ alkoxy-substituted alkyl, $C_1$-$C_6$ alkylamino-substituted alkyl, $C_3$-$C_6$ cycloalkyl, $C_4$-$C_{10}$ cycloalkyl alkyl, and 4-10 membered heterocyclyl; and more preferably, -Cy- is selected from:

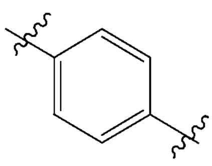, 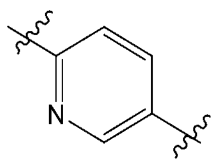, 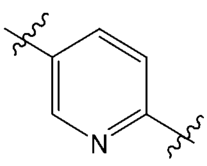,

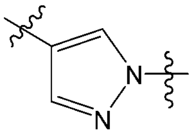, 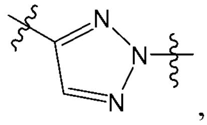, 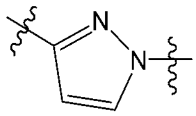, and 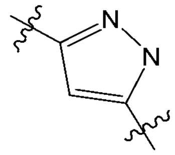.

**9.** The compound according to any one of claims 1 to 8, wherein $L_2$ is a single bond or $C_2$-$C_{10}$ alkylene, wherein one or more methylene units in alkylene are optionally and independently substituted by a group selected from: -N($R_4$)-, -N($R_4$)C(O)-, -C(O)N($R_4$)-, -N($R_4$)S$(O)_2$-, - S$(O)_2$N($R_4$)-, -O-, -C(O)-, -OC(O)-, -C(O)O-, -S-, -S(O)- or -S$(O)_2$, wherein one or more H atoms in alkylene are optionally and independently substituted by the following groups: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, and $C_4$-$C_{10}$ cycloalkyl alkyl;

preferably, $L_2$ is $C_2$-$C_6$ alkylene, wherein one or more methylene units in alkylene are optionally and independently substituted by a group selected from: -NH-, -O-, -C(O)-, -OC(O)-, -C(O)O-, and -S-; and
more preferably, $L_2$ is selected from: a single bond,

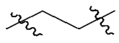, 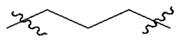, 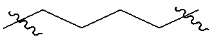,

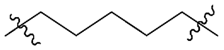, 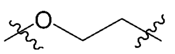, 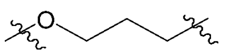, 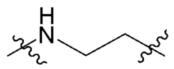, 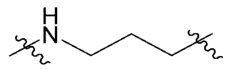,

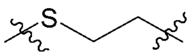, and 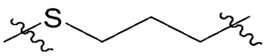.

**10.** The compound according to any one of claims 1 to 9, wherein $L_3$ is $C_1$-$C_6$ alkylene, wherein one or more methylene units in alkylene are optionally and independently substituted by a group selected from: -NH-, -O-, -C(O)-, -OC(O)-, -C(O)O-, -S-, wherein one or more H atoms are optionally and independently substituted by the following groups: H, $C_1$-$C_6$ alkyl, halogen, cyano, nitro, trifluoromethyl, trifluoromethoxy, azido, hydroxyl, $C_1$-$C_6$ alkoxy, $C_3$-$C_6$ cycloalkyl, or $C_4$-$C_{10}$ cycloalkyl alkyl; and
preferably, $L_3$ is methylene.

**11.** The compound according to claim 1, wherein said compound is selected from:

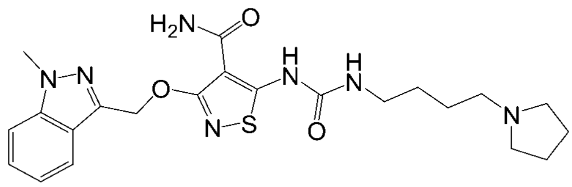

**T002**

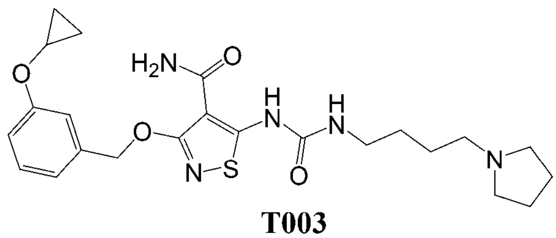

**T003**

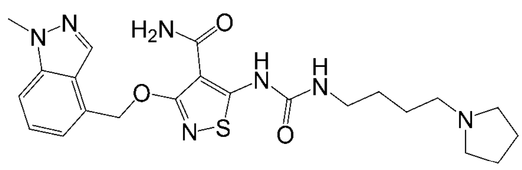

**T004**

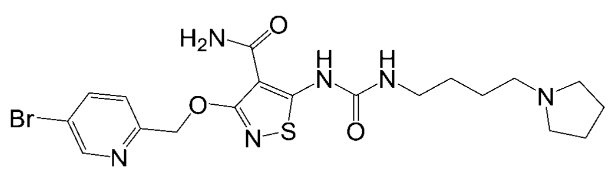

**T005**

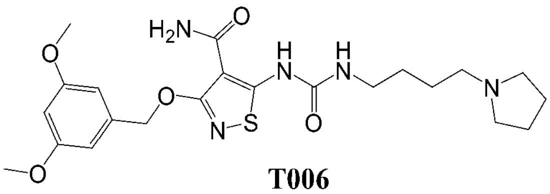

**T006**

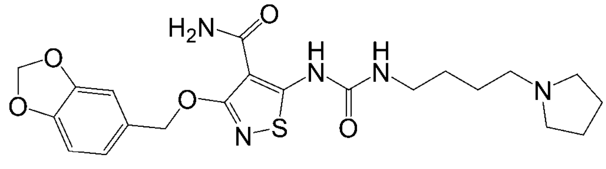

**T007**

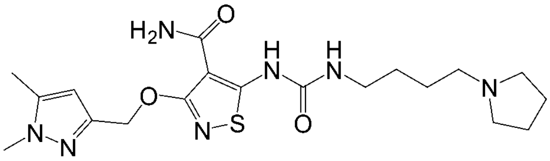

**T009**

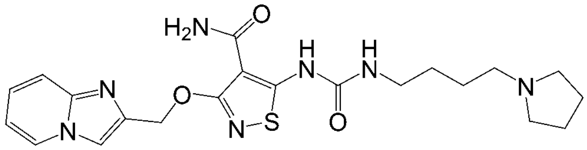

**T013**

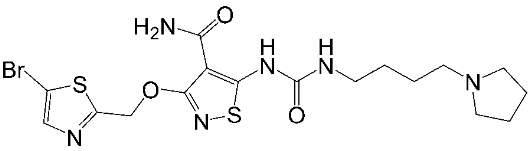

**T015**

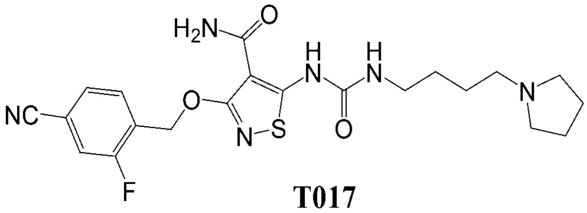

**T017**

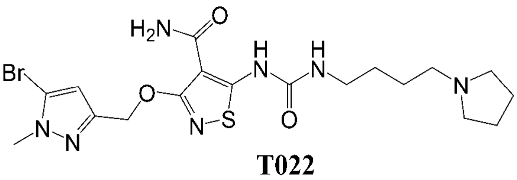

**T022**

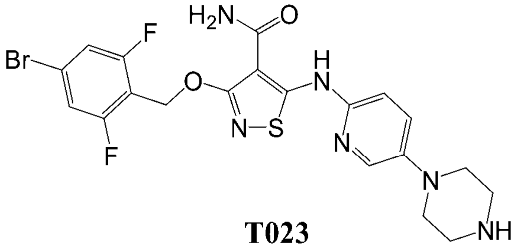

**T023**

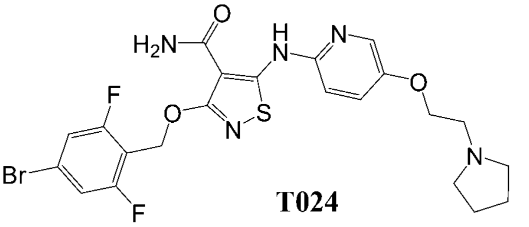

**T024**

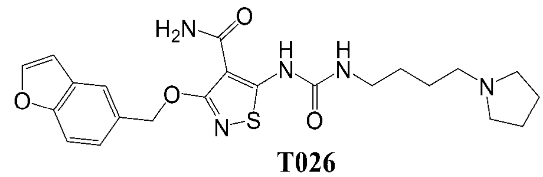

**T026**

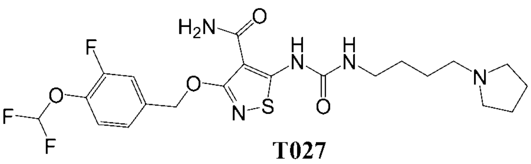

**T027**

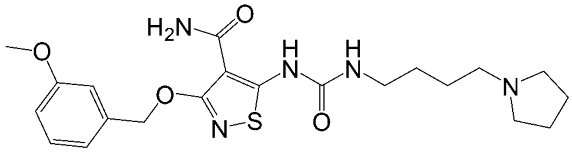

**T028**

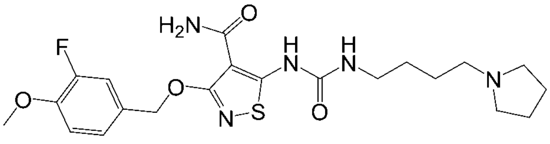

**T029**

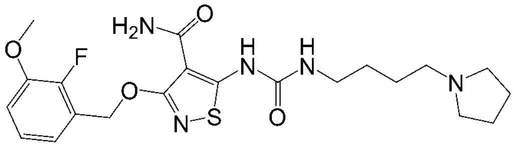

**T030**

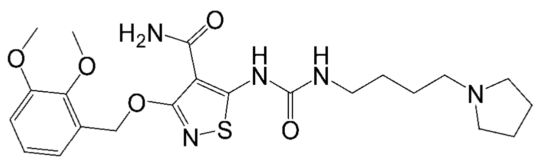

**T032**

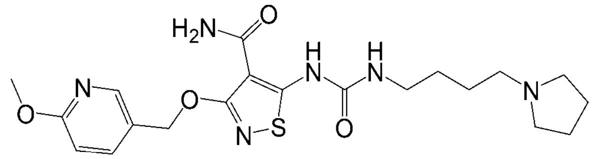

**T033**

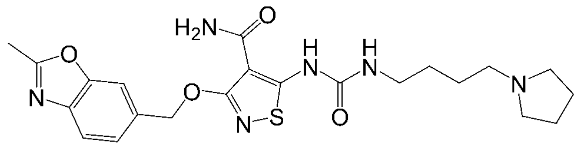

**T035**

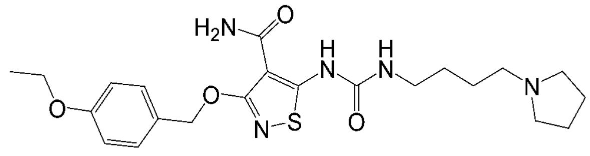

**T036**

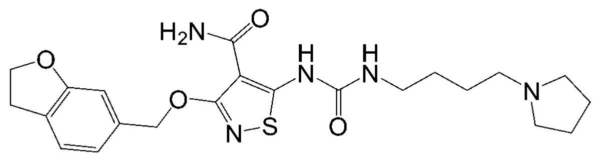

**T042**

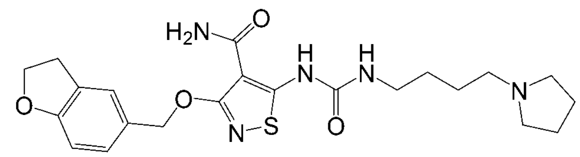

**T044**

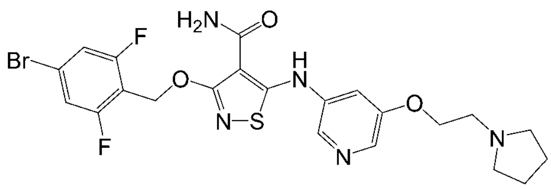

**T045**

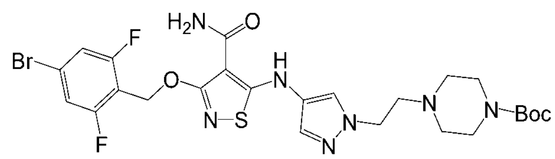

**T046**

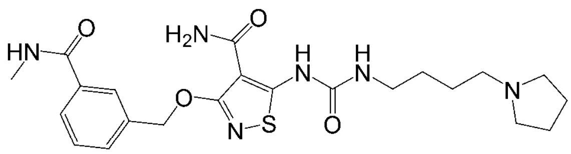

**T049**

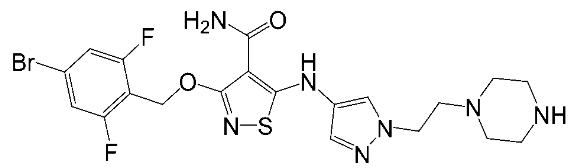

**T050**

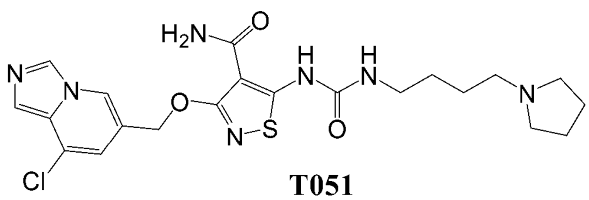

**T051**

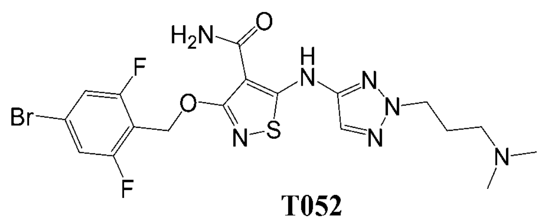

**T052**

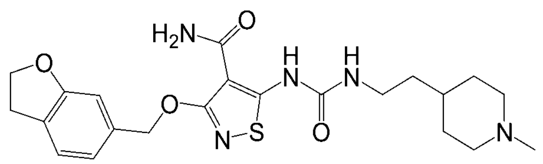

**T053**

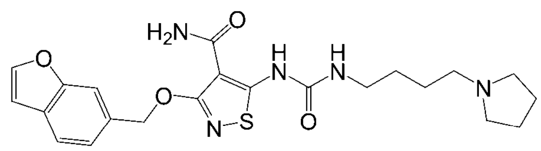

**T054**

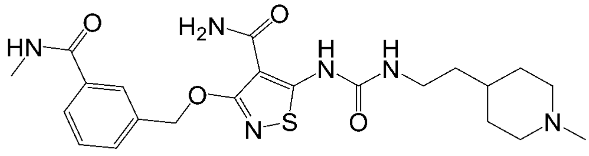

**T055**

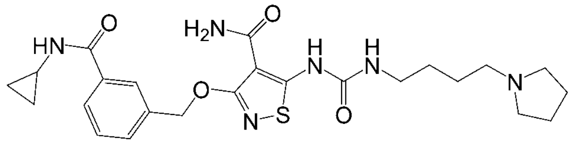

**T056**

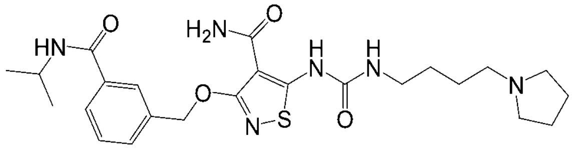

**T057**

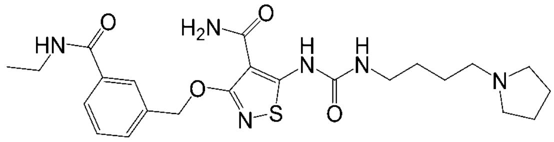

**T058**

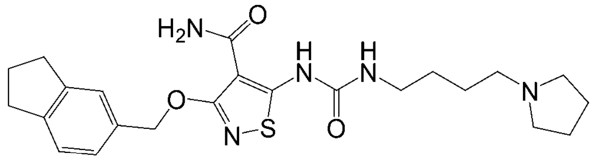

**T061**

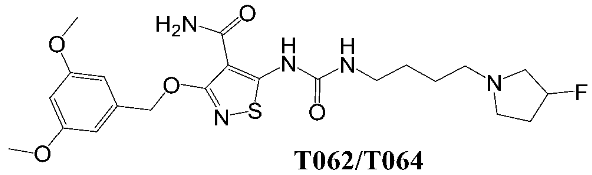

**T062/T064**

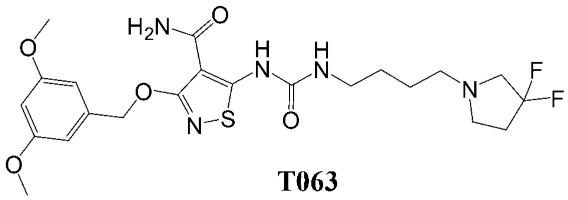

**T063**

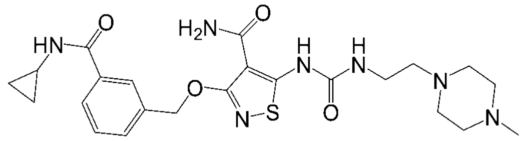

**T065**

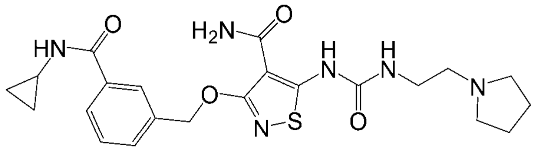

**T066**

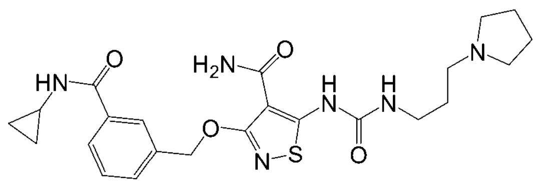

**T067**

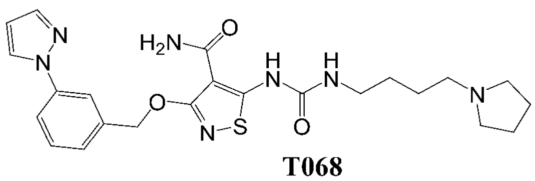

**T068**

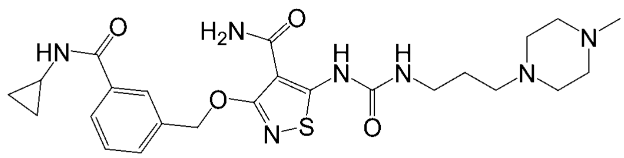

**T069**

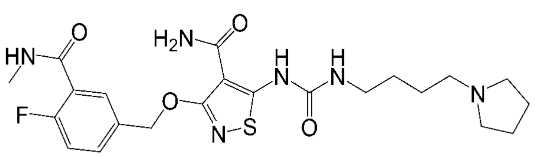

**T070**

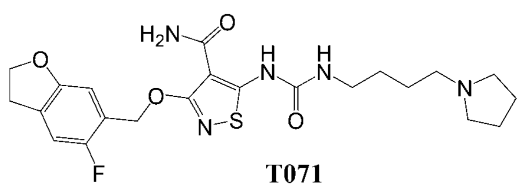

**T071**

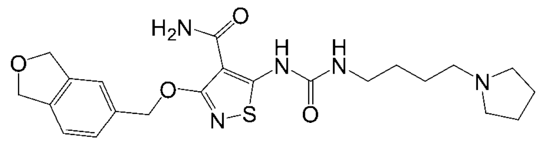

**T072**

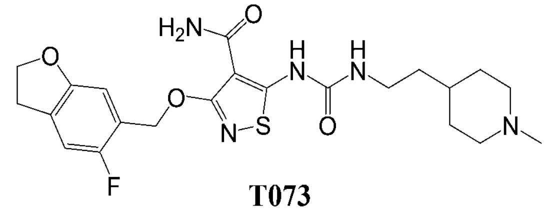

**T073**

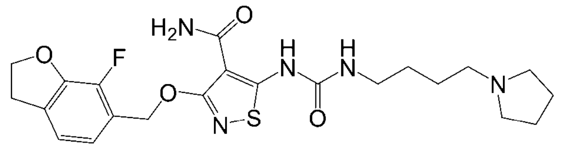

**T074**

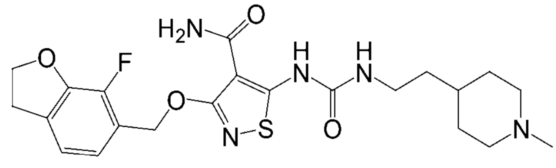

**T075**

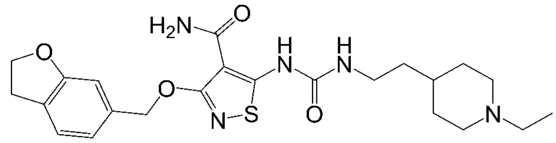

**T076**

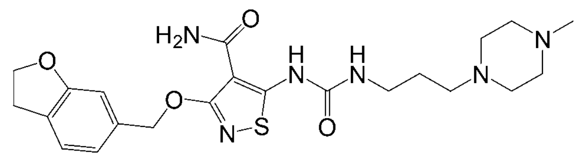

**T077**

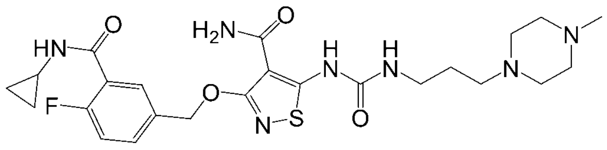

**T078**

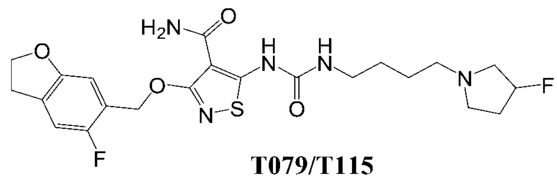

**T079/T115**

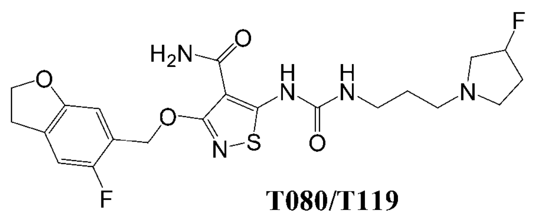

**T080/T119**

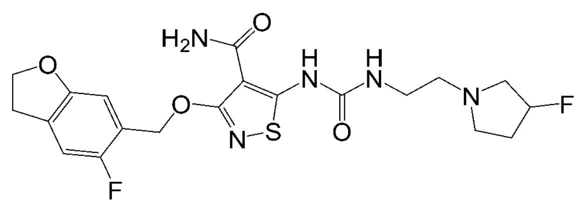

**T081**

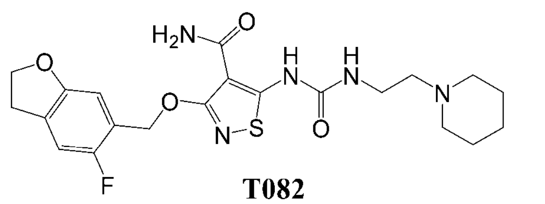

**T082**

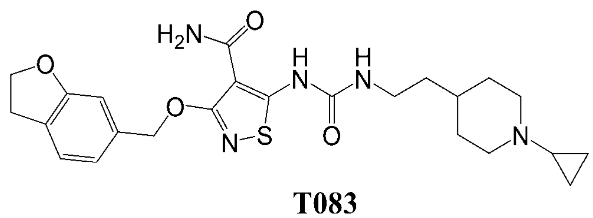

**T083**

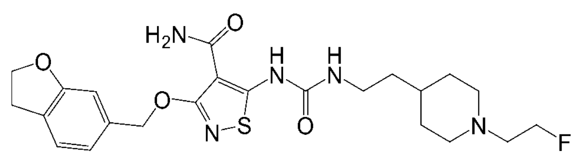

**T084**

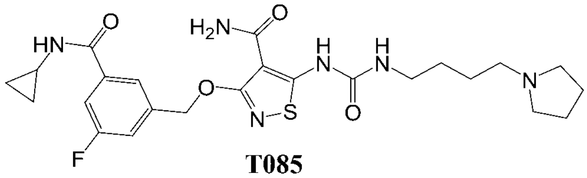

**T085**

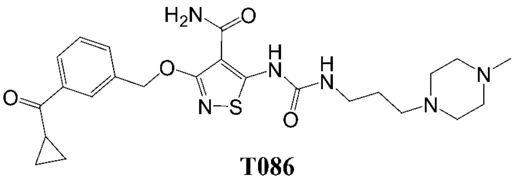

T086

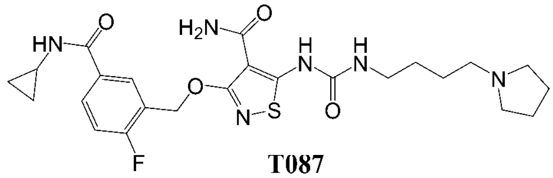

T087

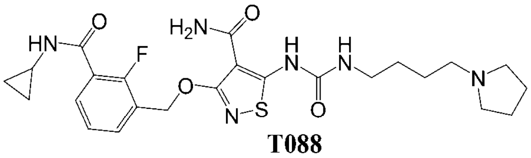

T088

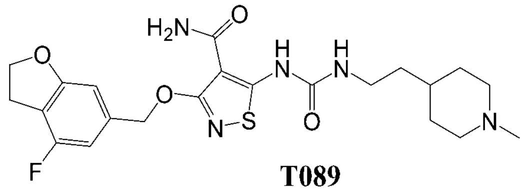

T089

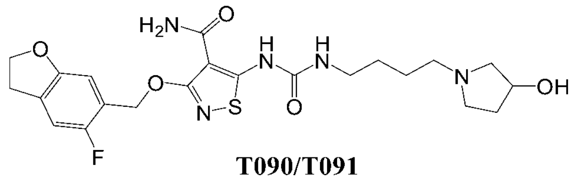

T090/T091

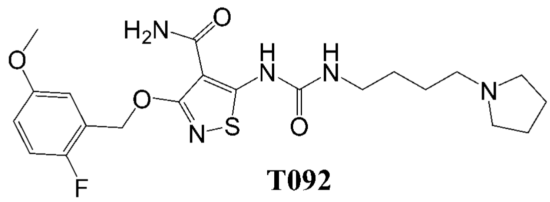

T092

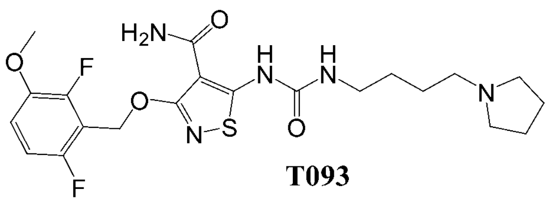

T093

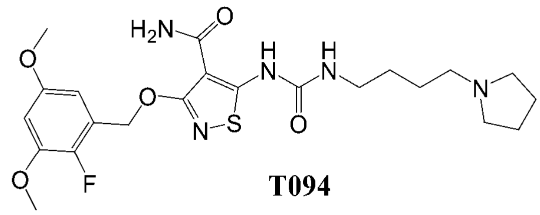

T094

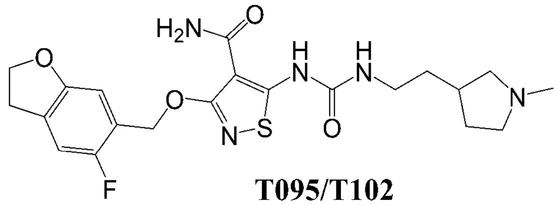

T095/T102

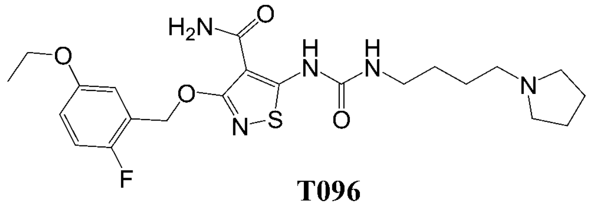

T096

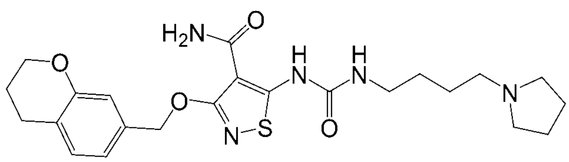

T097

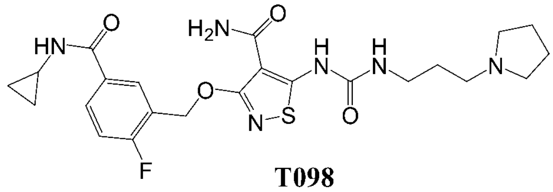

T098

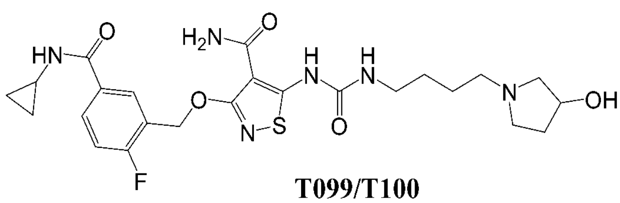

T099/T100

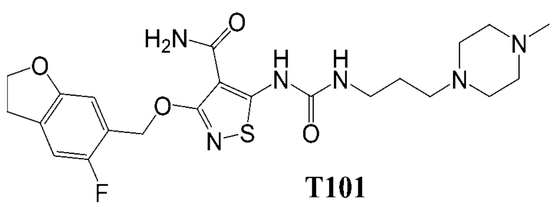

T101

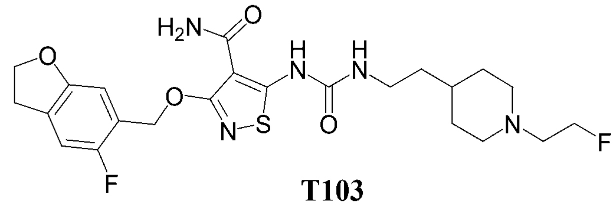

**T103**

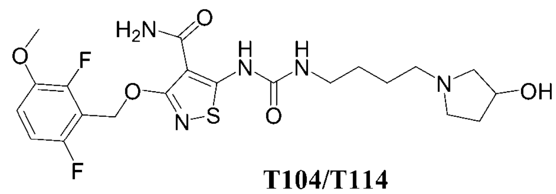

**T104/T114**

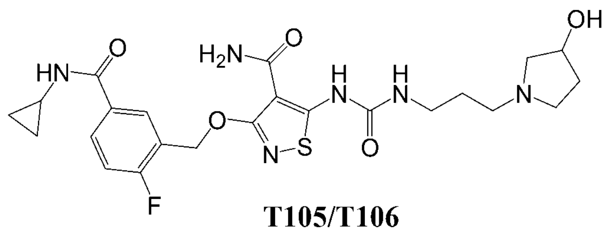

**T105/T106**

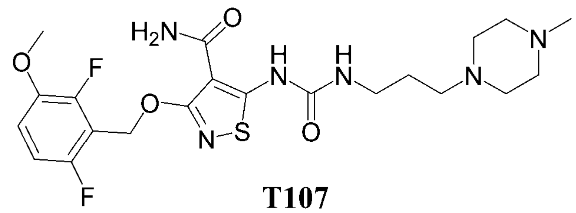

**T107**

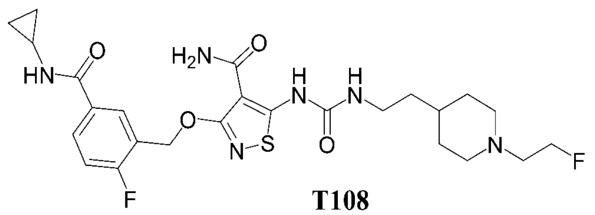

**T108**

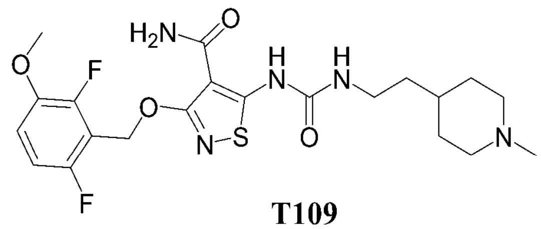

**T109**

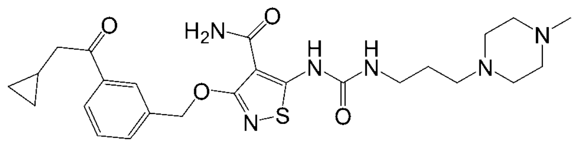

**T110**

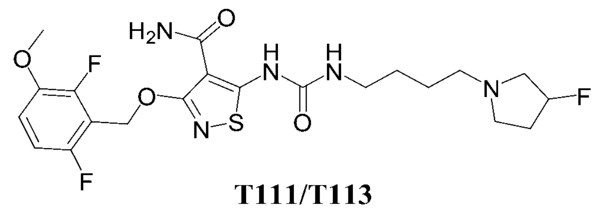

**T111/T113**

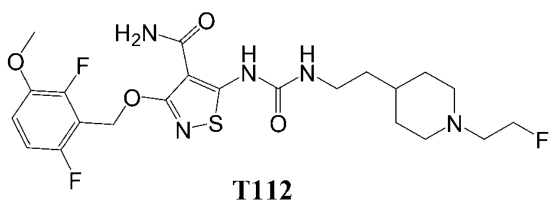

**T112**

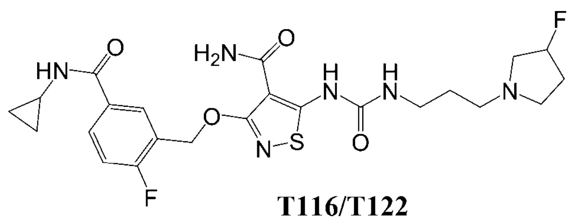

**T116/T122**

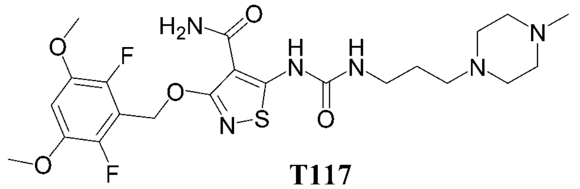

**T117**

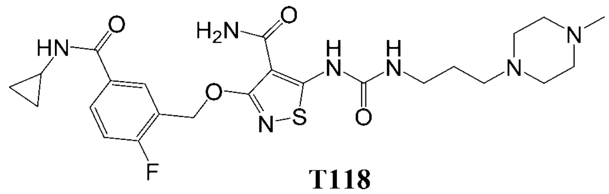

**T118**

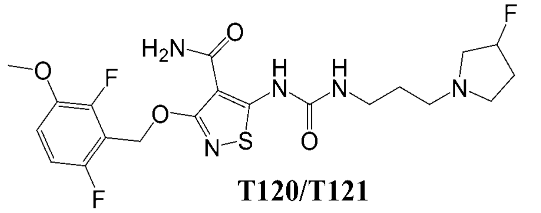

**T120/T121**

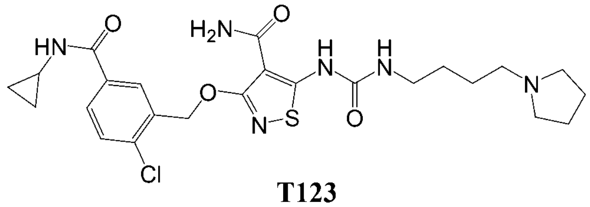

**T123**

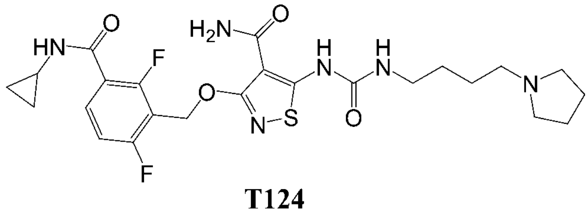

**T124**

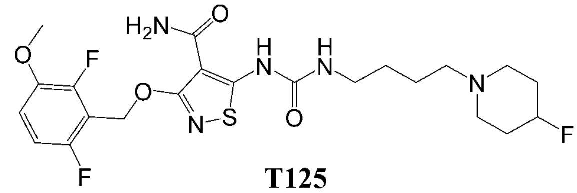

**T125**

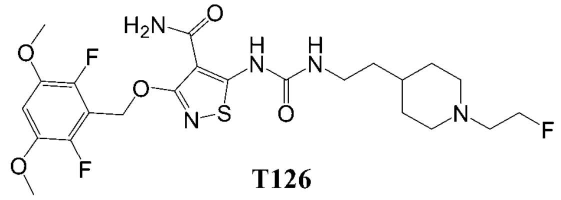

**T126**

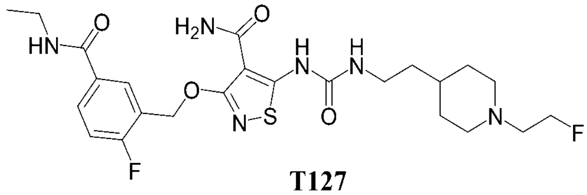

**T127**

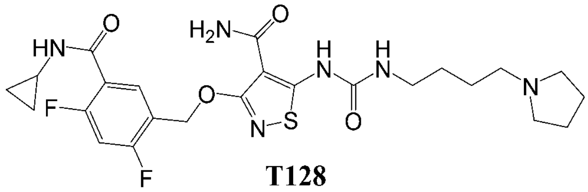

**T128**

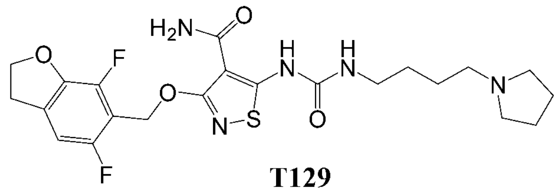

**T129**

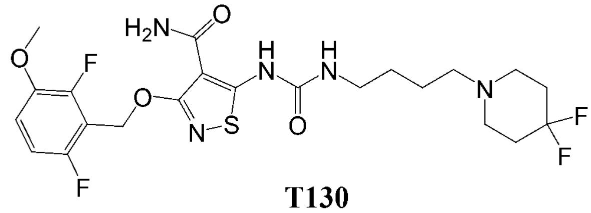

**T130**

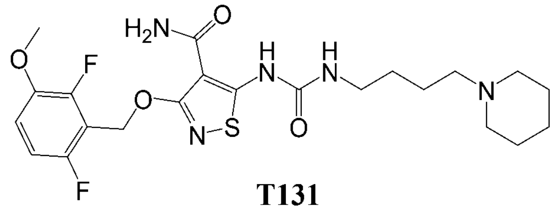

**T131**

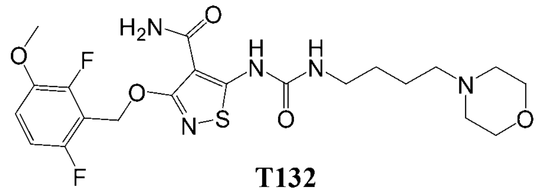

**T132**

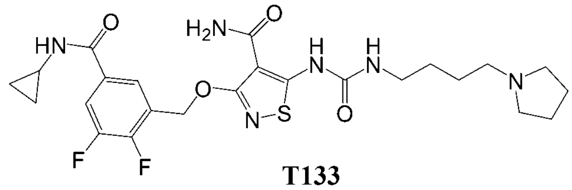

**T133**

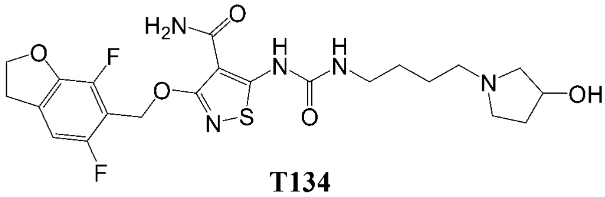

**T134**

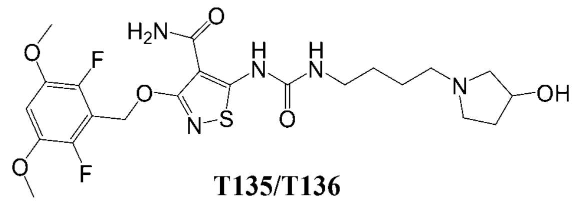

**T135/T136**

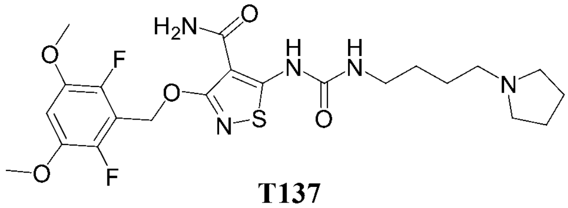

**T137**

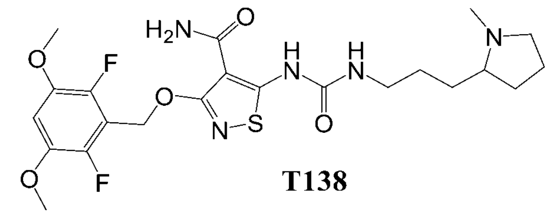

**T138**

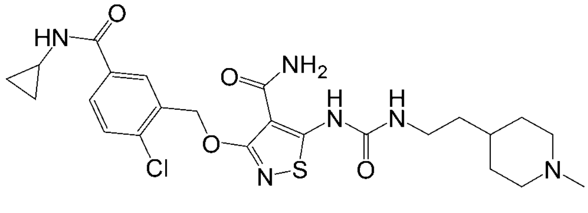

**T139**

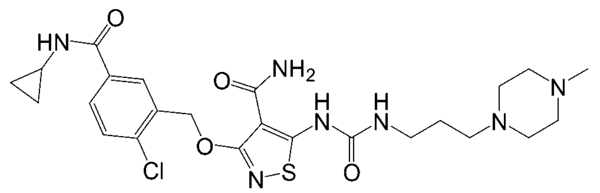

**T140**

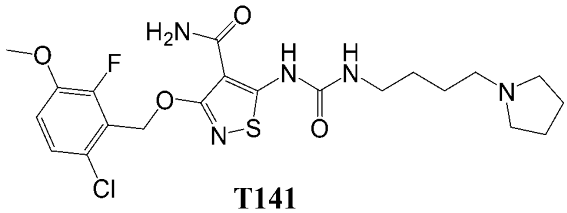

**T141**

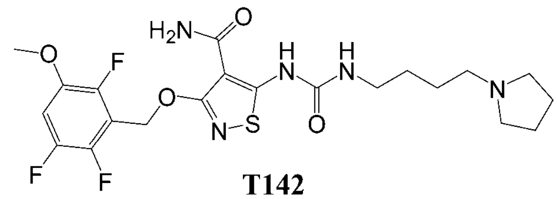

**T142**

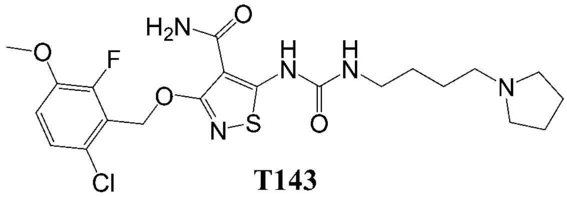

**T143**

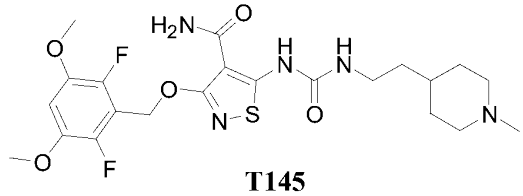

**T145**

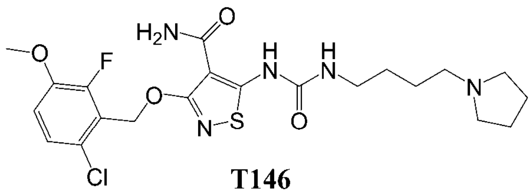

**T146**

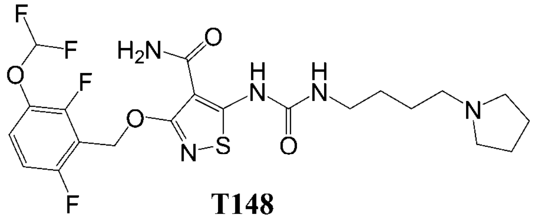

**T148**

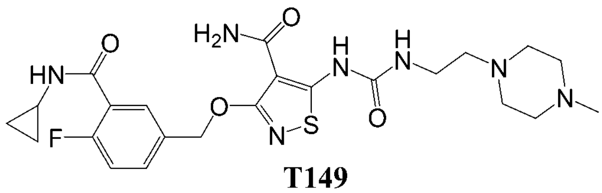

**T149**

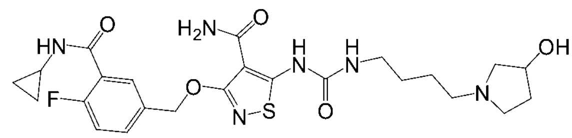

**T150**

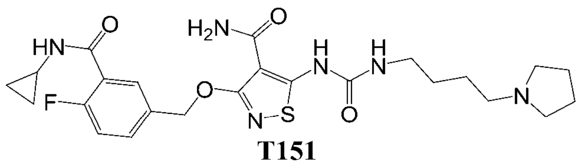

**T151**

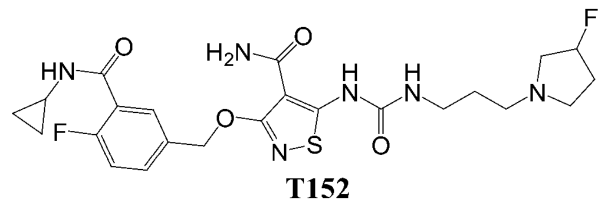

**T152**

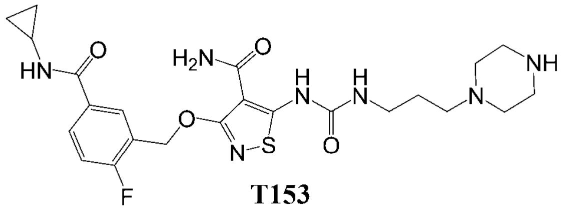

**T153**

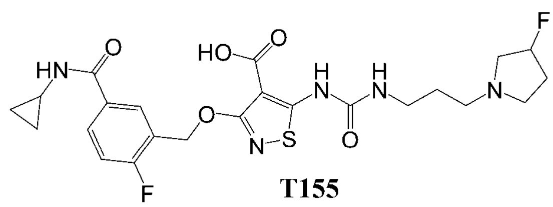

**T155**

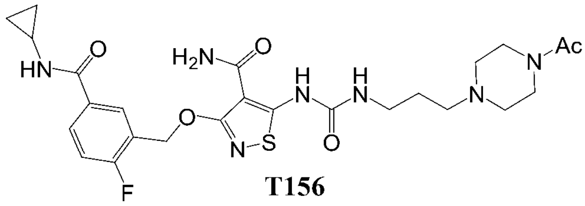

T156

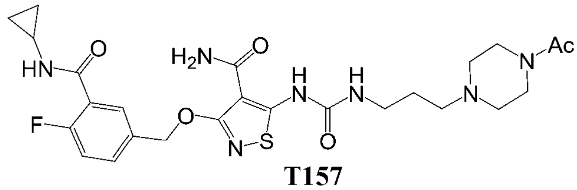

T157

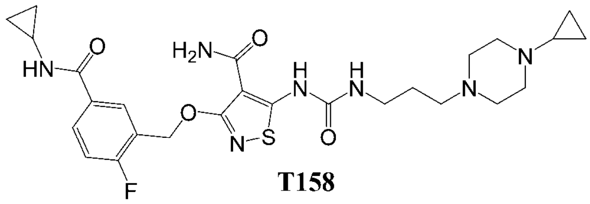

T158

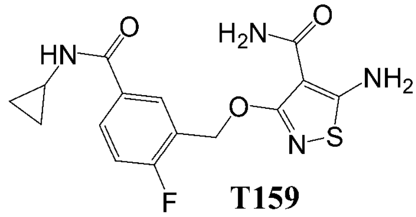

T159

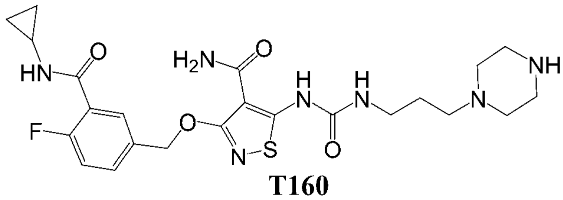

T160

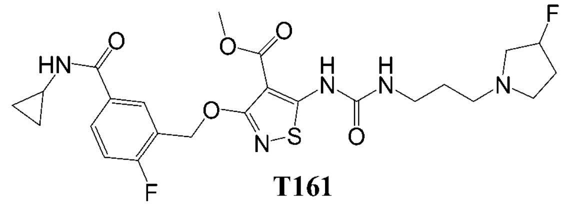

T161

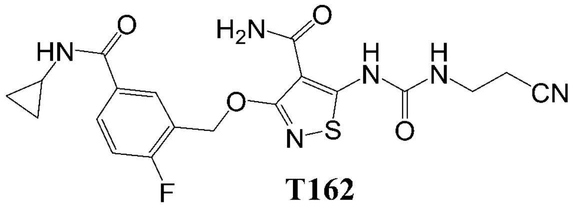

T162

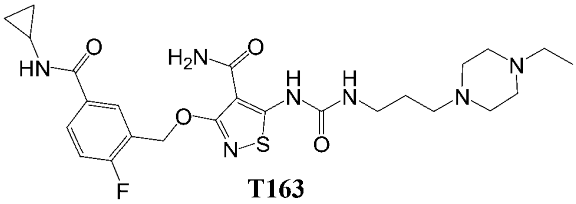

T163

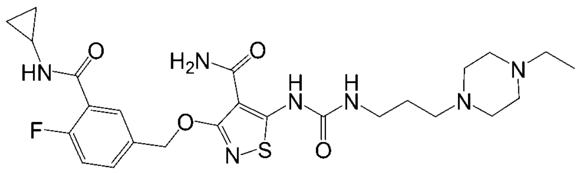

T164

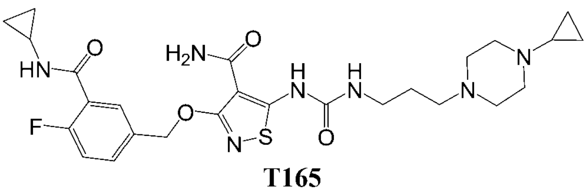

T165

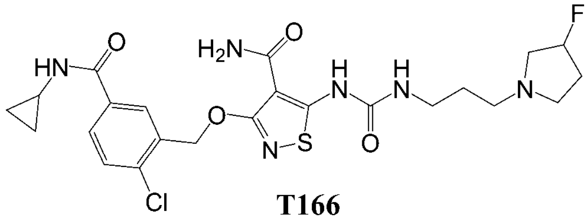

T166

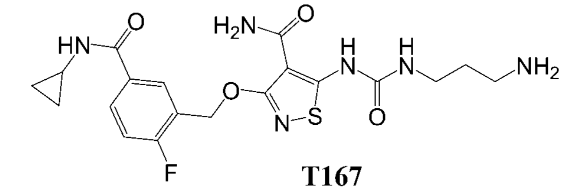

T167

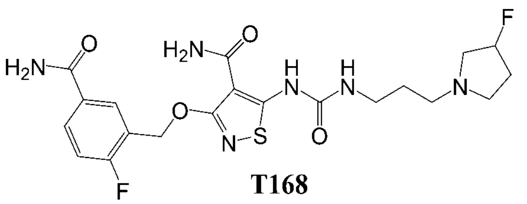

T168

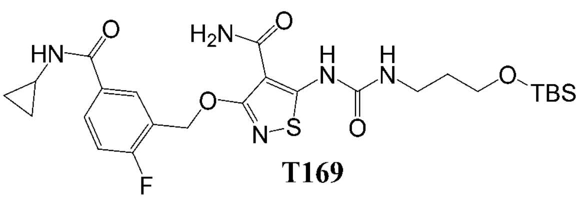

T169

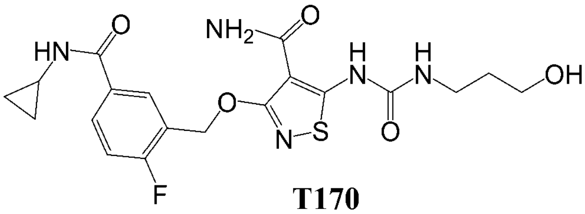

T170

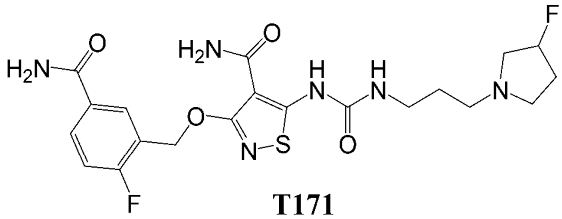

T171

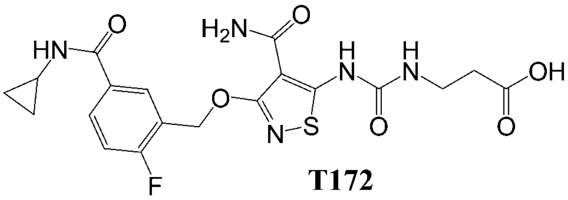

T172

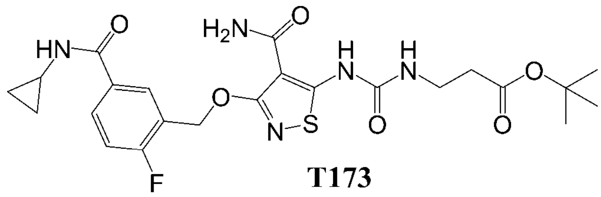

T173

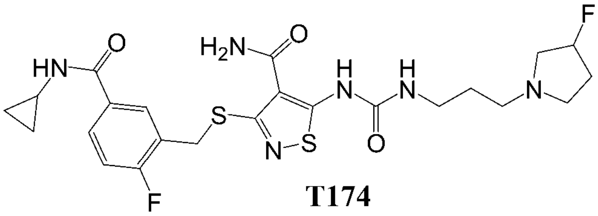

T174

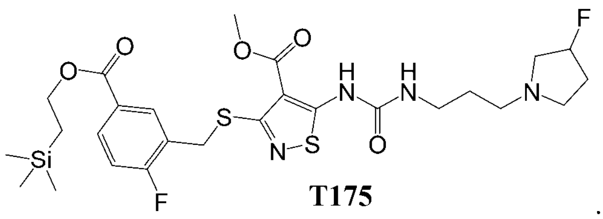

T175

.

**12.** The compound according to claim 1, wherein said stereoisomer is selected from:

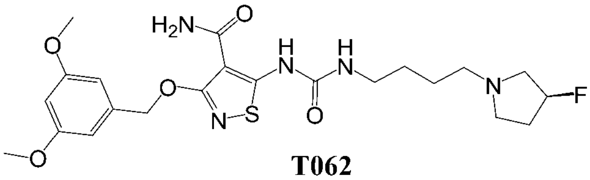

T062

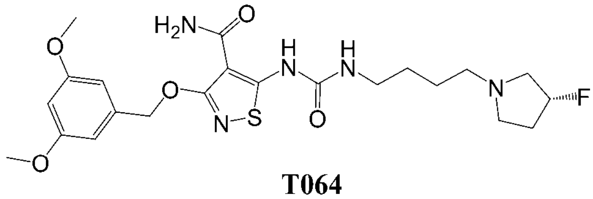

T064

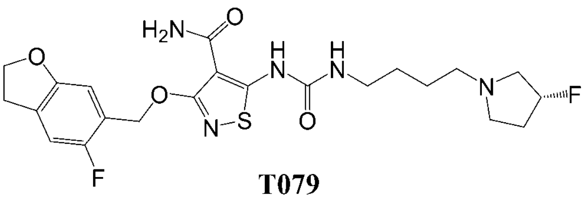

T079

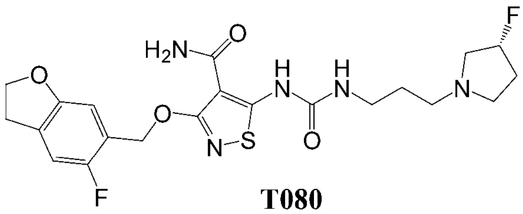

T080

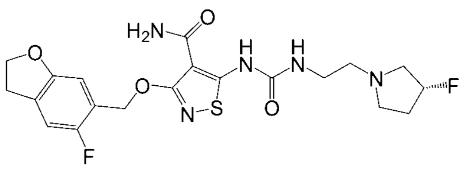

T081

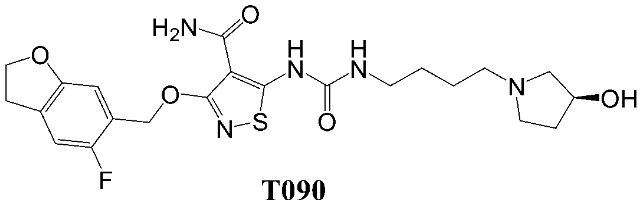

T090

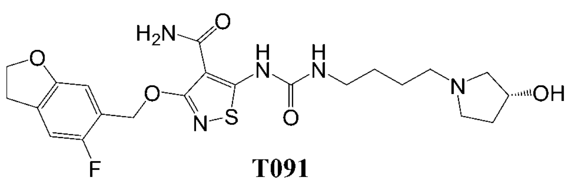

T091

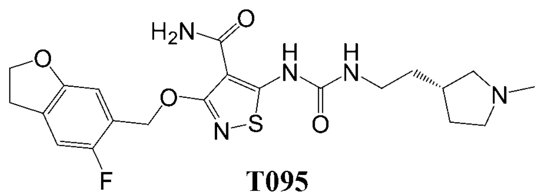

T095

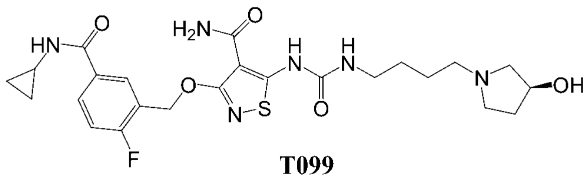

**T099**

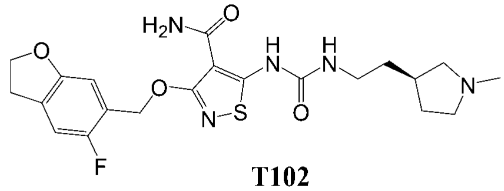

**T102**

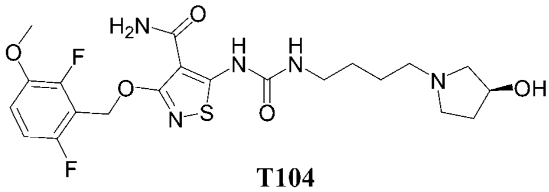

**T104**

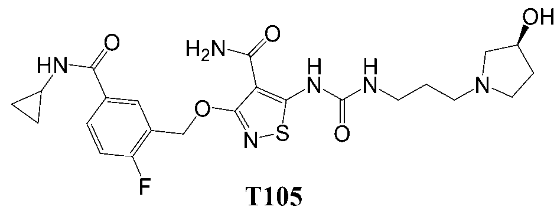

**T105**

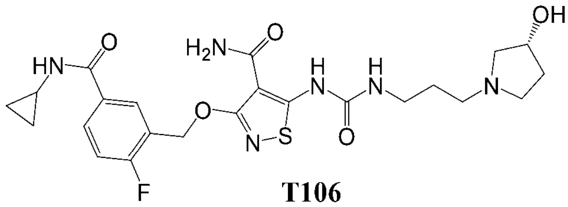

**T106**

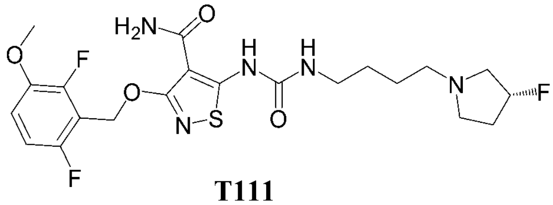

**T111**

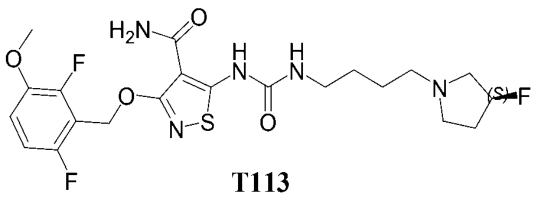

**T113**

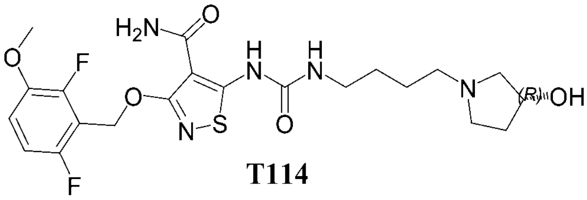

**T114**

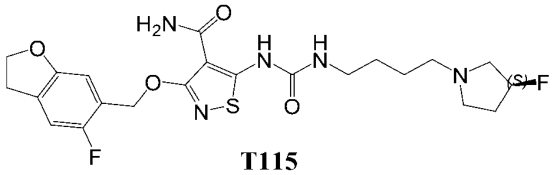

**T115**

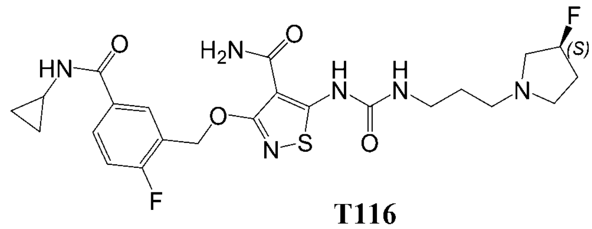

**T116**

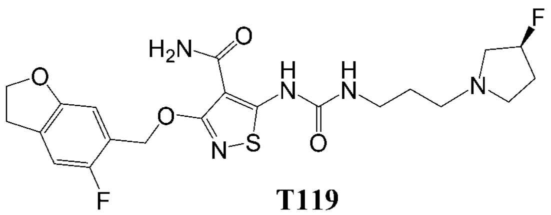

**T119**

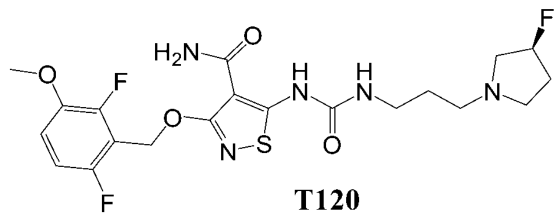

**T120**

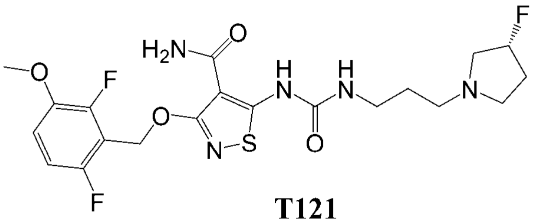

**T121**

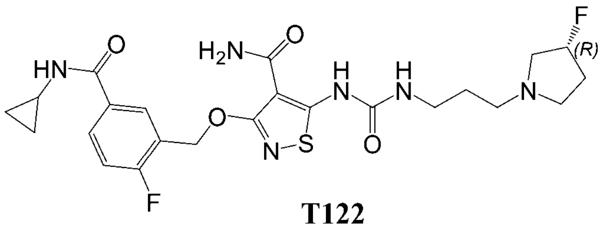

**T122**

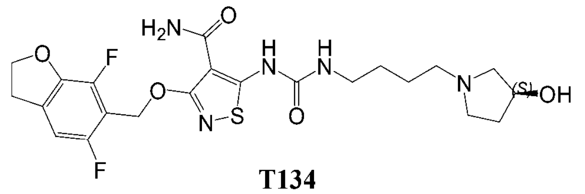

T134

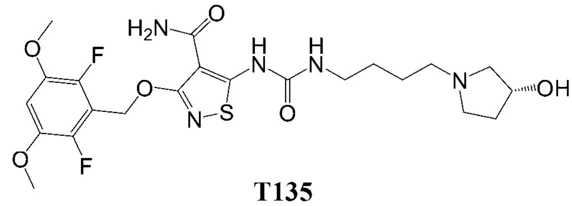

T135

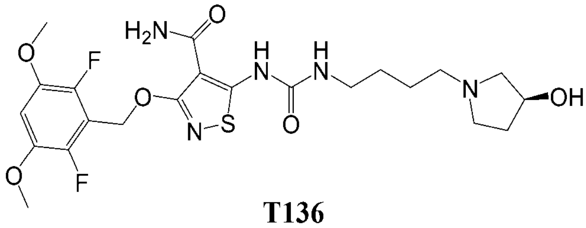

T136

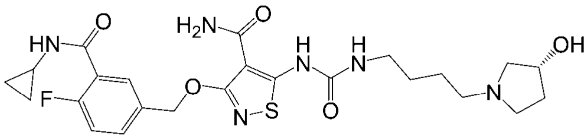

T150

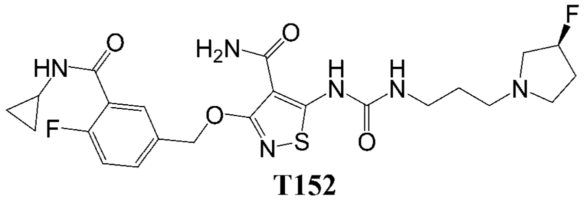

T152

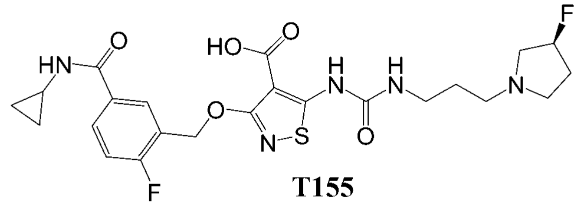

T155

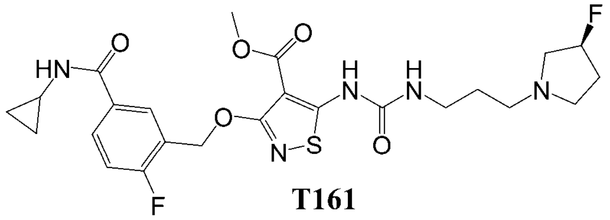

T161

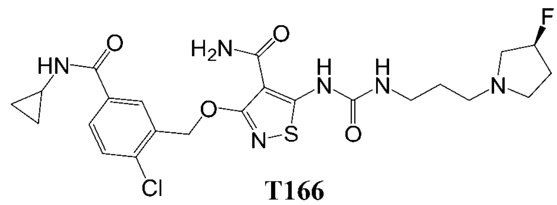

T166

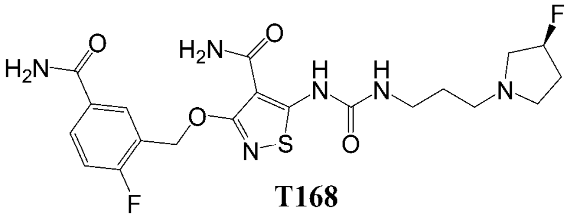

T168

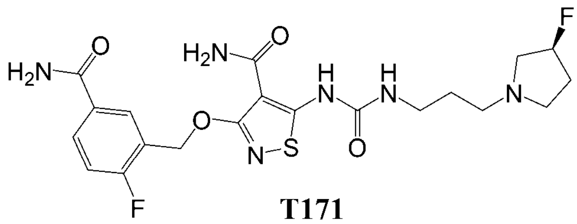

T171

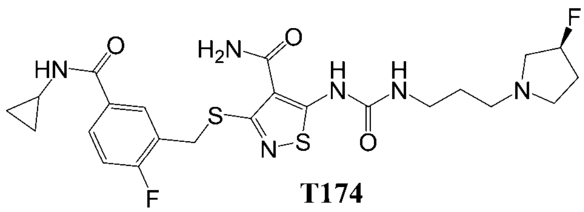

T174

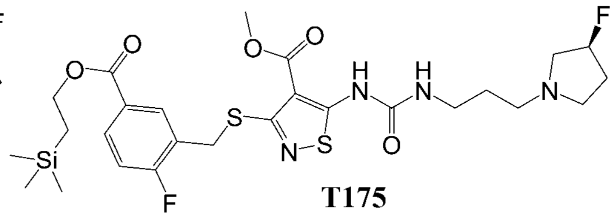

T175

.

**13.** The compound according to claim 1, wherein $L_1$ is -C(O)NR$_3$- or a single bond, the

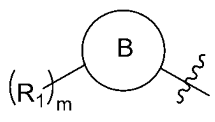

moiety is

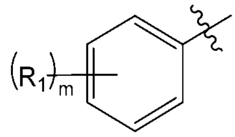

,

wherein, $R_1$ contains at least one of the following groups: $C_1$-$C_6$ haloalkyl, cyano, nitro, azido, $-OR_{101}$, $-C(O)R_{101}$, $-C(O)OR_{101}$, $-NHC(O)OR_{101}$, - $OC(O)R_{101}$, $-NHSO_2R_{101}$, $-SO_2NR_{101}R_{102}$, $-NHC(O)R_{101}$, $-C(O)NR_{101}R_{102}$, $-NR_{101}R_{102}$, $-SR_{101}$, - $S(O)_2R_{101}$, $-SO_3H$, $-(CH_2)_t$(phenyl), $-(CH_2)_t$(4-10 membered heterocyclyl), and $-(CH_2)_t$($C_3$-$C_{10}$ cycloalkyl), where t is an integer from 0 to 5; preferably, the

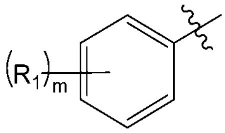

moiety has a structure of:

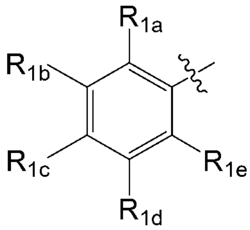

,

wherein, $R_{1b}$ is selected from: $C_1$-$C_6$ haloalkyl, cyano, nitro, azido, $-OR_{101}$, $-C(O)R_{101}$, $-C(O)OR_{101}$, $-NHC(O)OR_{101}$, $-OC(O)R_{101}$, $-NHSO_2R_{101}$, - $SO_2NR_{101}R_{102}$, $-NHC(O)R_{101}$, $-C(O)NR_{101}R_{102}$, $-NR_{101}R_{102}$, $-SR_{101}$, $-S(O)_2R_{101}$, $-SO_3H$, - $(CH_2)_t$(phenyl), $-(CH_2)_t$(4-10 membered heterocyclyl), and $-(CH_2)_t$($C_3$-$C_{10}$ cycloalkyl); more preferably, $R_{1b}$ is selected from: $C_1$-$C_6$ haloalkyl, cyano, nitro, azido, $-OR_{101}$, $-C(O)R_{101}$, - $NHC(O)R_{101}$, $-C(O)NHR_{101}$, $-NHR_{101}$, $-SR_{101}$, $-(CH_2)_t$(4-8 membered heterocyclyl), and - $(CH_2)_t$($C_3$-$C_6$ cycloalkyl), where t is an integer from 0 to 5;

$R_{1c}$ is selected from: H, F, $C_1$-$C_6$ alkyl, cyano, nitro, azido, $-OR_{101}$, $-C(O)R_{101}$, $-C(O)OR_{101}$, - $NHC(O)OR_{101}$, $-OC(O)R_{101}$, $-NHSO_2R_{101}$, $-SO_2NR_{101}R_{102}$, $-NHC(O)R_{101}$, $-C(O)NR_{101}R_{102}$, - $NR_{101}R_{102}$, $-SR_{101}$, $-S(O)_2R_{101}$, $-SO_3H$, $-(CH_2)_t$(phenyl), $-(CH_2)_t$(4-10 membered heterocyclyl), and $-(CH_2)_t$($C_3$-$C_{10}$ cycloalkyl);

$R_{1a}$, $R_{1d}$, and $R_{1e}$ are selected from: H, $C_1$-$C_6$ alkyl (for example, methyl, ethyl, n-propyl, or isopropyl), halogen (for example, F, Cl, Br, or I), $C_1$-$C_6$ haloalkyl, cyano, nitro, azido, $-OR_{101}$, - $C(O)R_{101}$, $-C(O)OR_{101}$, $-NHC(O)OR_{101}$, $-OC(O)R_{101}$, $-NHSO_2R_{101}$, $-SO_2NR_{101}R_{102}$, - $NHC(O)R_{101}$, $-C(O)NR_{101}R_{102}$, $-NR_{101}R_{102}$, $-SR_{101}$, $-S(O)_2R_{101}$, $-SO_3H$, $-(CH_2)_t$(phenyl), - $(CH_2)_t$(4-10 membered heterocyclyl), and $-(CH_2)_t$($C_3$-$C_{10}$ cycloalkyl); or, $L_1$ is $-C(O)NR_3-$, Ring B is a monocyclic heterocyclic ring, for example, the

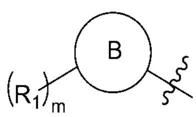

moiety is

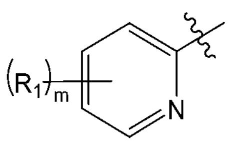 , 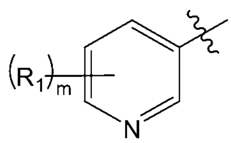 , 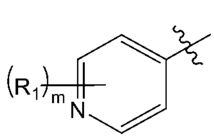 , 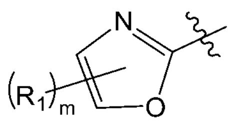 ,

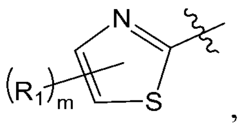 , or 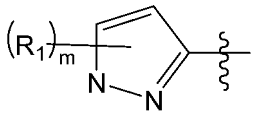 ;

or, $L_1$ is -C(O)$NR_3$-, Ring B is a fused bicyclic ring, for example, the

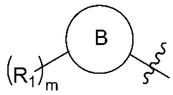

moiety is

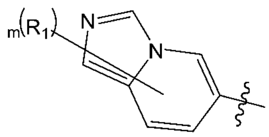 , 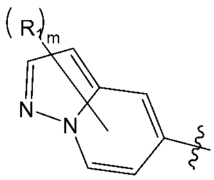 , 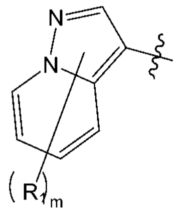 ,

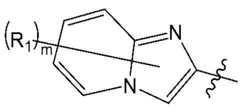 , 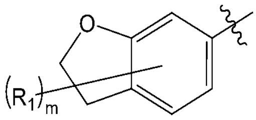 , 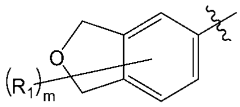 , 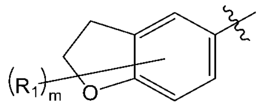 ,

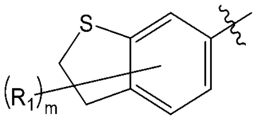 , 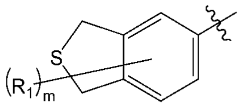 , 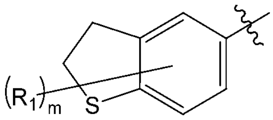 , 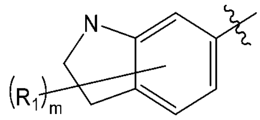 ,

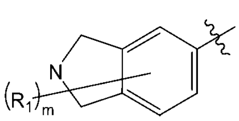 , 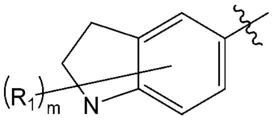 , 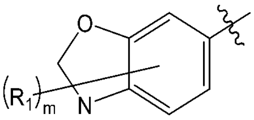 , 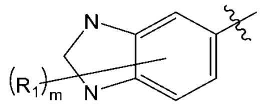 ,

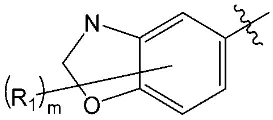 , 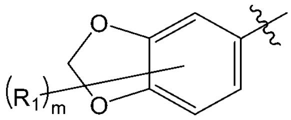 , 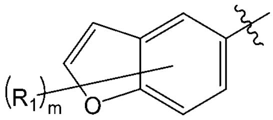 ,

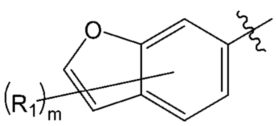 , 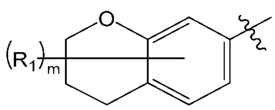 , 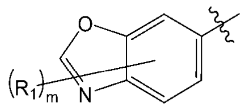 , 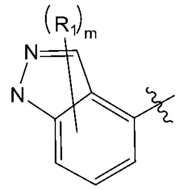 ,

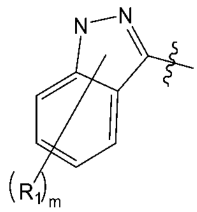, 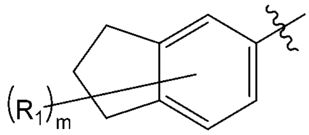, or 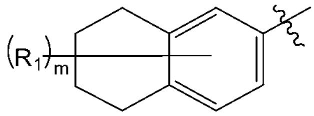;

or, Li is -Cy-.

**14.** A pharmaceutical composition, comprising the compound according to any one of claims 1 to 13 or a pharmaceutically acceptable salt, stereoisomer, ester, prodrug, solvate, and deuterated compound thereof, and one or more pharmaceutically acceptable excipients; and
preferably, said pharmaceutical composition is an ophthalmic formulation, for example, eye drops or ophthalmic ointment.

**15.** Use of the compound according to any one of claims 1 to 13 or a pharmaceutically acceptable salt, stereoisomer, ester, prodrug, solvate, and deuterated compound thereof in preparation of a medicament for preventing and/or treating a protein tyrosine kinase-medicated proliferative disease.

**16.** The use according to claim 15, wherein said disease is cancer, particularly malignant tumor, preferably selected from: breast cancer, lung cancer, adenocarcinoma, colorectal cancer, kidney cancer, liver cancer, pancreatic cancer, ovarian cancer, prostate cancer, glioma, glioblastoma, myeloma, agnogenic myeloid metaplasia, mesothelioma, myelodysplastic syndrome, and hematologic malignancy; or
said disease is an ocular disease, preferably selected from: diabetic retinopathy, comprising simple or background diabetic retinopathy, proliferative diabetic retinopathy, and diabetic macular edema; age-related macular degeneration, comprising neovascular or wet/exudative age-related macular degeneration, dry age-related macular degeneration, and geographic atrophy; pathological choroidal neovascularization, i.e., high myopia, trauma, sickle cell anemia, ocular histoplasmosis, angioid streaks, traumatic choroidal rupture, optic nerve drusen, and certain retinal dystrophies; pathological retinal neovascularization (i.e., sickle cell retinopathy, Eales disease, ocular ischemic syndrome, carotid cavernous fistula, familial exudative vitreoretinopathy, hyperviscosity syndrome, idiopathic occlusive arteritis, birdshot retinochoroidopathy, retinal vasculitis, sarcoidosis, or toxoplasmosis); uveitis; retinal vein occlusion (central or branch); ocular trauma; postsurgical edema; postsurgical neovascularization; cystoid macular edema; ocular ischemia; retinopathy of prematurity; Coat's disease; sickle cell retinopathy and/or neovascular glaucoma.

**INTERNATIONAL SEARCH REPORT**

International application No.
**PCT/CN2023/142499**

**A. CLASSIFICATION OF SUBJECT MATTER**

C07D417/12(2006.01)i; A61K31/425(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, EXTXT, DWPI, STN(REG, CAPLUS), CNKI, 谷歌学术, GOOGLE SCHOLAR: 苏州必扬医药, 胡齐悦, 胡伟民, 刘苏星, 异噻唑, 脲, 甲酰胺, 吡咯, 吡嗪, 酪氨酸激酶, 癌症, isothiazol?, urea, carboxamide, pyrrole, tyrosine kinase, VEGFR?, TIE2, EGFR, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| X | ACS. "RN number: 2436737-00-5" *STN REG*, 29 June 2020 (2020-06-29), see pages 1-9 | 1-10 |
| X | CN 1303380 A (PFIZER PRODUCTS INC.) 11 July 2001 (2001-07-11) see claims 1 and 10-17, and description, page 1 and embodiments | 1-10, 13-16 |
| X | CN 1476439 A (PFIZER PRODUCTS INC.) 18 February 2004 (2004-02-18) see claims 1-18 | 1-10, 13-16 |
| X | WO 2004011461 A1 (PFIZER PRODUCTS INC. et al.) 05 February 2004 (2004-02-05) see claims 1-15, and embodiments | 1-10, 13-16 |
| X | WO 2004067530 A1 (PFIZER PRODUCTS INC. et al.) 12 August 2004 (2004-08-12) see claims 1-15 | 1-10, 13-16 |
| X | WO 2005081997 A2 (THE SCRIPPS RESEARCH INSTITUTE et al.) 09 September 2005 (2005-09-09) see claims 1-26 | 1-10, 13-16 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 March 2024** | **12 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No.
**PCT/CN2023/142499**

**C. DOCUMENTS CONSIDERED TO BE RELEVANT**

| Category* | Citation of document, with indication, where appropriate, of the relevant passages | Relevant to claim No. |
|---|---|---|
| X | LIPPA B. et al. "Discovery of novel isothiazole inhibitors of the TrkA kinase: Structure-activity relationship, computer modeling, optimization, and identification of highly potent antagonists" *Bioorganic & Medicinal Chemistry Letters,* Vol. 16, No. 13, 24 April 2006 (2006-04-24), ISSN: 0960-894X, see pages 3444-3448, abstract, and tables 1-2 | 1-10 |
| X | WO 2005110406 A2 (PFIZER PRODUCTS INC. et al.) 24 November 2005 (2005-11-24) see claims 1-15 | 1-10, 13-16 |
| X | WO 2006026034 A2 (PFIZER PRODUCTS INC. et al.) 09 March 2006 (2006-03-09) see claim 1, and corresponding embodiments | 1-10, 13-16 |
| A | CN 1303380 A (PFIZER PRODUCTS INC.) 11 July 2001 (2001-07-11) see claims 1 and 10-17, and description, page 1 and embodiments | 11-12 |

Form PCT/ISA/210 (second sheet) (July 2022)

**INTERNATIONAL SEARCH REPORT**
**Information on patent family members**

International application No.
**PCT/CN2023/142499**

| Patent document cited in search report | Publication date (day/month/year) | Patent family member(s) | Publication date (day/month/year) |
|---|---|---|---|
| CN 1303380 A | 11 July 2001 | NO 20006071 D0 | 30 November 2000 |
| | | NO 20006071 L | 09 May 2005 |
| | | PT 1084114 E | 31 December 2004 |
| | | SK 17782000 A3 | 06 August 2001 |
| | | SK 286405 B6 | 05 September 2008 |
| | | PA 8472301 A1 | 29 September 2000 |
| | | ZA 993752 B | 04 December 2000 |
| | | GEP 20084337 B | 25 March 2008 |
| | | TW 561154 B | 11 November 2003 |
| | | TR 200003478 T2 | 21 March 2001 |
| | | SA 99200217 B1 | 06 May 2006 |
| | | BR 9910900 A | 13 February 2001 |
| | | BR 9910900 B1 | 17 September 2013 |
| | | YU 70100 A | 15 July 2004 |
| | | CZ 20004451 A3 | 16 May 2001 |
| | | CZ 298559 B6 | 07 November 2007 |
| | | NZ 507009 A | 28 November 2003 |
| | | GT 199900070 A | 10 November 2000 |
| | | AR 016725 A1 | 25 July 2001 |
| | | OA 11504 A | 17 May 2004 |
| | | HN 1999000080 A | 29 September 1999 |
| | | CA 2475113 A1 | 09 December 1999 |
| | | CA 2475113 C | 18 March 2008 |
| | | PE 20000568 A1 | 08 July 2000 |
| | | TNSN 99106 A1 | 10 November 2005 |
| | | ES 2226368 T3 | 16 March 2005 |
| | | CA 2333703 A1 | 09 December 1999 |
| | | CA 2333703 C | 14 June 2005 |
| | | EP 1084114 A1 | 21 March 2001 |
| | | EP 1084114 B1 | 08 September 2004 |
| | | US 2008300249 A1 | 04 December 2008 |
| | | US 7790902 B2 | 07 September 2010 |
| | | US 2003149048 A1 | 07 August 2003 |
| | | US 7405218 B2 | 29 July 2008 |
| | | ID 27006 A | 22 February 2001 |
| | | BG 104998 A | 31 July 2001 |
| | | BG 65104 B1 | 28 February 2007 |
| | | IL 138776 A0 | 31 October 2001 |
| | | HU 0102422 A2 | 29 May 2002 |
| | | HU 0102422 A3 | 28 June 2002 |
| | | HRP 20000835 A2 | 31 December 2001 |
| | | HRP 20000835 B1 | 31 January 2008 |
| | | JP 2002517384 A | 18 June 2002 |
| | | JP 3735254 B2 | 18 January 2006 |
| | | IS 5690 A | 27 October 2000 |
| | | IS 2269 B | 15 July 2007 |
| | | US 6235764 B1 | 22 May 2001 |
| | | ZA 9903752 B | 04 December 2000 |
| | | CO 5011121 A1 | 28 February 2001 |
| | | UA 60365 C2 | 15 October 2003 |

Form PCT/ISA/210 (patent family annex) (July 2022)

**INTERNATIONAL SEARCH REPORT**
**Information on patent family members**

International application No.
**PCT/CN2023/142499**

| Patent document cited in search report | Publication date (day/month/year) | Patent family member(s) | Publication date (day/month/year) |
|---|---|---|---|
| | | AU 3342199 A | 20 December 1999 |
| | | JP 2005002122 A | 06 January 2005 |
| | | SI 1084114 T1 | 28 February 2005 |
| | | AP 9901560 A0 | 30 June 1999 |
| | | AP 1309 A | 14 September 2004 |
| | | WO 9962890 A1 | 09 December 1999 |
| | | UY 25619 A1 | 23 February 2000 |
| | | ATE 275553 T1 | 15 September 2004 |
| | | PL 344691 A1 | 19 November 2001 |
| | | PL 198151 B1 | 30 May 2008 |
| | | HK 1036982 A1 | 25 January 2002 |
| | | MA 26638 A1 | 20 December 2004 |
| | | MY 130668 A | 31 July 2007 |
| | | KR 20010052516 A | 25 June 2001 |
| | | KR 100392434 B1 | 22 July 2003 |
| | | US 2001020034 A1 | 06 September 2001 |
| | | US 6548526 B2 | 15 April 2003 |
| | | EA 200001146 A1 | 23 April 2001 |
| | | EA 004935 B1 | 28 October 2004 |
| | | DE 69920009 D1 | 14 October 2004 |
| | | DE 69920009 T2 | 15 September 2005 |
| CN 1476439 A | 18 February 2004 | UY 27039 A1 | 31 July 2002 |
| | | SK 5862003 A3 | 04 May 2004 |
| | | DK 1337521 T3 | 29 January 2007 |
| | | TWI 287542 B | 01 October 2007 |
| | | HN 2001000268 A | 30 January 2002 |
| | | HRP 20030408 A2 | 31 August 2003 |
| | | ZA 200303341 B | 30 April 2004 |
| | | EP 1337521 A1 | 27 August 2003 |
| | | EP 1337521 B1 | 27 September 2006 |
| | | ES 2271086 T3 | 16 April 2007 |
| | | PL 362079 A1 | 18 October 2004 |
| | | KR 20050116401 A | 12 December 2005 |
| | | OA 12532 A | 02 June 2006 |
| | | AP 200102358 A0 | 31 December 2001 |
| | | AU 2002214204 B2 | 24 May 2007 |
| | | AR 031512 A1 | 24 September 2003 |
| | | TNSN 01167 A1 | 10 November 2005 |
| | | US 2002151573 A1 | 17 October 2002 |
| | | US 6831091 B2 | 14 December 2004 |
| | | AP 200102358 D0 | 31 December 2001 |
| | | DOP 2001000288 A | 15 December 2002 |
| | | UA 74221 C2 | 15 November 2005 |
| | | AU 1420402 A | 11 June 2002 |
| | | MXPA 03004714 A | 19 August 2003 |
| | | JP 2004514714 A | 20 May 2004 |
| | | MA 26960 A1 | 20 December 2004 |
| | | BG 107752 A | 30 January 2004 |
| | | HK 1059085 A1 | 18 June 2004 |
| | | MY 136686 A | 28 November 2008 |

Form PCT/ISA/210 (patent family annex) (July 2022)

**INTERNATIONAL SEARCH REPORT**
**Information on patent family members**

International application No.
**PCT/CN2023/142499**

| Patent document cited in search report | Publication date (day/month/year) | Patent family member(s) | Publication date (day/month/year) |
|---|---|---|---|
| | | SV 2002000748 A | 02 December 2002 |
| | | DE 60123461 D1 | 09 November 2006 |
| | | DE 60123461 T2 | 15 February 2007 |
| | | IL 155371 A0 | 23 November 2003 |
| | | GT 200100237 A | 03 July 2002 |
| | | WO 0244158 A1 | 06 June 2002 |
| | | EE 200300247 A | 15 October 2003 |
| | | BR 0115621 A | 02 September 2003 |
| | | PA 8533801 A1 | 31 October 2002 |
| | | YU 36403 A | 17 August 2006 |
| | | PT 1337521 E | 29 December 2006 |
| | | CA 2430065 A1 | 06 June 2002 |
| | | CA 2430065 C | 20 May 2008 |
| | | IS 6788 A | 14 April 2003 |
| | | KR 20030059275 A | 07 July 2003 |
| | | JP 2008056692 A | 13 March 2008 |
| | | JP 4971946 B2 | 11 July 2012 |
| | | PE 20020591 A1 | 06 July 2002 |
| | | ECSP 034628 A | 25 July 2003 |
| | | CZ 20031315 A3 | 14 April 2004 |
| | | CR 6963 A | 25 November 2003 |
| | | SI 1337521 T1 | 31 December 2006 |
| | | ATE 340786 T1 | 15 October 2006 |
| | | GEP 20053652 B | 10 November 2005 |
| | | NZ 525788 A | 26 November 2004 |
| | | EA 200300424 A1 | 30 October 2003 |
| | | EA 005859 B1 | 30 June 2005 |
| | | NO 20032388 D0 | 27 May 2003 |
| | | NO 20032388 L | 18 July 2003 |
| | | NO 325187 B1 | 11 February 2008 |
| | | HU 0302553 A2 | 28 November 2003 |
| | | HU 0302553 A3 | 30 March 2009 |
| WO 2004011461 A1 | 05 February 2004 | CA 2493701 A1 | 05 February 2004 |
| | | JP 2005536523 A | 02 December 2005 |
| | | JP 4511352 B2 | 28 July 2010 |
| | | US 2004152691 A1 | 05 August 2004 |
| | | US 7276602 B2 | 02 October 2007 |
| | | BR 0312798 A | 03 May 2005 |
| | | EP 1527071 A1 | 04 May 2005 |
| | | AU 2003281664 A1 | 16 February 2004 |
| | | MXPA 05000950 A | 16 May 2005 |
| WO 2004067530 A1 | 12 August 2004 | JP 2006516603 A | 06 July 2006 |
| | | US 2004147574 A1 | 29 July 2004 |
| | | MXPA 05005664 A | 27 July 2005 |
| | | EP 1590347 A1 | 02 November 2005 |
| | | CA 2513140 A1 | 12 August 2004 |
| | | BRPI 0407055 A | 17 January 2006 |
| WO 2005081997 A2 | 09 September 2005 | WO 2005081997 A3 | 11 May 2006 |
| | | WO 2005082001 A2 | 09 September 2005 |
| | | WO 2005082001 A3 | 02 March 2006 |

Form PCT/ISA/210 (patent family annex) (July 2022)

**INTERNATIONAL SEARCH REPORT**
**Information on patent family members**

International application No.
**PCT/CN2023/142499**

| Patent document cited in search report | Publication date (day/month/year) | Patent family member(s) | Publication date (day/month/year) |
|---|---|---|---|
| WO 2005110406 A2 | 24 November 2005 | TW 200538104 A | 01 December 2005 |
| | | AR 049500 A1 | 09 August 2006 |
| | | US 2005256177 A1 | 17 November 2005 |
| | | WO 2005110406 A3 | 10 August 2006 |
| WO 2006026034 A2 | 09 March 2006 | JP 2012153705 A | 16 August 2012 |
| | | JP 5498524 B2 | 21 May 2014 |
| | | ZA 200701016 B | 28 May 2008 |
| | | RU 2007106847 A | 27 August 2008 |
| | | RU 2349588 C2 | 20 March 2009 |
| | | JP 2008510841 A | 10 April 2008 |
| | | JP 5100390 B2 | 19 December 2012 |
| | | KR 20070070158 A | 03 July 2007 |
| | | KR 100893977 B1 | 20 April 2009 |
| | | HK 1111359 A1 | 08 August 2008 |
| | | US 2009023924 A1 | 22 January 2009 |
| | | US 8884025 B2 | 11 November 2014 |
| | | PT 1784183 E | 09 March 2012 |
| | | TW 200621241 A | 01 July 2006 |
| | | TWI 361688 B | 11 April 2012 |
| | | NO 20071319 L | 29 May 2007 |
| | | EP 1784183 A2 | 16 May 2007 |
| | | EP 1784183 A4 | 09 December 2009 |
| | | EP 1784183 B1 | 25 January 2012 |
| | | CA 2578023 A1 | 09 March 2006 |
| | | CA 2578023 C | 15 May 2012 |
| | | DK 1784183 T3 | 02 April 2012 |
| | | PL 1784183 T3 | 31 July 2012 |
| | | IL 181063 A0 | 04 July 2007 |
| | | AR 050613 A1 | 08 November 2006 |
| | | AU 2005280451 A1 | 09 March 2006 |
| | | AU 2005280451 B2 | 05 February 2009 |
| | | ATE 542533 T1 | 15 February 2012 |
| | | ES 2378994 T3 | 19 April 2012 |
| | | US 2015065726 A1 | 05 March 2015 |
| | | US 9371298 B2 | 21 June 2016 |
| | | WO 2006026034 A3 | 29 June 2006 |
| | | MX 2007002246 A | 20 April 2007 |
| | | BRPI 0514667 A | 17 June 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WADA, E et al.** *Seikagaku*, 1994, vol. 66, 15
- **GANNES, LZ et al.** *Comp Biochem Physiol Mol Integr Physiol*, 1998, vol. 119, 725
- Stedman's Medical Dictionary. Williams & Wilkins: Philadelphia, 1990